(19) Europäisches Patentamt
European Patent Office
Office européen des brevets

(11) EP 3 481 823 B1

(12) # EUROPÄISCHE PATENTSCHRIFT

(45) Veröffentlichungstag und Bekanntmachung des Hinweises auf die Patenterteilung:
**28.10.2020 Patentblatt 2020/44**

(21) Anmeldenummer: **17733874.6**

(22) Anmeldetag: **04.07.2017**

(51) Int Cl.:
***C07D 471/04*** $^{(2006.01)}$

(86) Internationale Anmeldenummer:
**PCT/EP2017/066632**

(87) Internationale Veröffentlichungsnummer:
**WO 2018/011017 (18.01.2018 Gazette 2018/03)**

(54) **7-SUBSTITUIERTE 1-PYRIDYL-NAPHTHYRIDIN-3-CARBONSÄUREAMIDE UND IHRE VERWENDUNG**

7-SUBSTITUTED 1-PYRIDYL-NAPHTHYRIDINE-3-CARBOXYLIC ACID AMIDES AND USE THEREOF

AMIDES DE L'ACIDE 1-PYRIDYL-NAPHTHYRIDIN-3-CARBOXYLIQUE SUBSTITUÉS EN POSITION 7, ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.07.2016 EP 16178835**
**06.12.2016 EP 16202510**

(43) Veröffentlichungstag der Anmeldung:
**15.05.2019 Patentblatt 2019/20**

(73) Patentinhaber:
- **Bayer Pharma Aktiengesellschaft**
  **13353 Berlin (DE)**
- **Bayer Aktiengesellschaft**
  **51371 Leverkusen (DE)**

(72) Erfinder:
- **TELLER, Henrik**
  **18258 Schwaan (DE)**
- **BOULTADAKIS ARAPINIS, Melissa**
  **40215 Düsseldorf (DE)**
- **VAKALOPOULOS, Alexandros**
  **40721 Hilden (DE)**
- **REBSTOCK, Anne-Sophie**
  **69410 Champagne au Mont d'Or (FR)**
- **STRAUB, Alexander**
  **42113 Wuppertal (DE)**
- **TINEL, Hanna**
  **42113 Wuppertal (DE)**
- **BRECHMANN, Markus**
  **San Francisco, CA 94114 (US)**
- **WITTWER, Matthias, Beat**
  **4125 Riehen (CH)**
- **KULLMANN, Maximilian, Andreas**
  **42799 Leichlingen (DE)**
- **MÜNTER, Klaus**
  **42489 Wülfrath (DE)**
- **MONDRITZKI, Thomas**
  **45130 Essen (DE)**
- **MARQUARDT, Tobias**
  **42115 Wuppertal (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 1 650 192** **WO-A1-2005/026165**
**WO-A1-2005/049602** **WO-A1-2016/071212**

- **TINGTING ZHANG ET AL: "Synthesis, antimycobacterial and antibacterial activity of fluoroquinolone derivatives containing an 3-alkoxyimino-4-(cyclopropylanimo)methylpy rrolidine moiety", EUROPEAN JOURNAL OF MEDICINAL CHEMISTRY, Bd. 104, 30. September 2015 (2015-09-30), Seiten 73-85, XP055405419, FR ISSN: 0223-5234, DOI: 10.1016/j.ejmech.2015.09.030 Gefunden im Internet: URL:http://dx.doi.org/10.1016/j.ejmech.201 5.09.>**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Anmeldung betrifft neue 7-substituierte 1-Pyridyl-naphthyridin-3-carbonsäureamide, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen und/oder Nierenerkrankungen.

Muskarinerge Rezeptoren, sind membranständige Rezeptoren, die als endogenen Liganden den Neurotransmitter Acetylcholin (ACh) binden (Acetylcholinrezeptoren), aber auch von Muskarin aktiviert werden können. Diese G-Protein-gekoppelte Rezeptoren gibt es als fünf Subtypen (M1-M5), die in fast allen Geweben im menschlichen Organismus exprimiert werden. Man findet sie sowohl im zentralen als auch im peripheren Nervensystem sowie in vielen Organen des vegetativen Nervensystems.

Der M2-Typ (M2R) wird überwiegend im Herzen exprimiert. Auf der zellulären Ebene bewirkt eine M2R Stimulation durch den Agonisten Acetylcholin eine Hemmung der Adenylcyclase und eine Aktivierung des einwärtsgleichrichtenden Kaliumkanals (IKACh-Kanal, GIRK engl.: G protein activated inwardly rectifying K+ channel; auch Kir3.x). Hierdurch vergrößert sich die Kaliumleitfähigkeit, was zu einer Hyperpolarisation der Muskelzellen führt. Dementsprechend werden die Zellen schwerer depolarisierbar, was zu einer negativ chronotropen und dromotropen Wirkung führt, so dass die Herzfrequenz sinkt. Der M2R ist der Hauptmediator der parasympathischen Kontrolle der Herzfunktion, die durch den Nervus vagus gesteuert wird. Dabei vermindert der rechte Nervus vagus über den Sinusknoten die Herzfrequenz, der linke erhöht über den Atrioventrikularknoten (AV-Knoten) überwiegend die atrioventrikuläre Überleitungszeit. Insgesamt überwiegt im Vergleich zum Sympathikus der Einfluss des Vagus auf die Ruhefrequenz des Herzens. Die Auswirkungen einer Stimulation von M2R sind somit entgegengesetzt zu denen der beta-adrenergen Stimulation..

Die Aktivierung des M2-Rezeptors durch den endogenen Agonisten Acetylcholin, aber auch durch synthetische Analoga wie Carbachol, Oxotremorin-M oder Iperoxo (Schräge et al., Biochem. Pharmacol. 2014, 90(3), 307-319) erfolgt durch die Bindung des Agonisten an die sog. orthosterische Bindestelle des Rezeptors und einer hierdurch ausgelösten Konformationsänderung des Rezeptors bzw. Stabilisierung der aktiven Rezeptorkonformation. Zu den klassischen natürlich vorkommenden Muskarin-Rezeptor-Agonisten gehören neben dem endogenen Agonisten Acetylcholin (ACh) verschiedene pflanzliche Alkaloide, wie Arecolin, Muscarin, sowie auch Pilocarpin (Neubig et al., Pharmacol Rev., 2003, 55, 597-606). Die orthosterische Bindungstelle aller muskarinergen Acetylcholin-Rezeptoren ist evolutionär stark konserviert und weist eine hohe Sequenz- und Strukturhomologie zwischen den verschiedenen Subtypen auf. Daher sind viele der bekannten Agonisten unselektiv gegenüber den verschiedenen Subtypen der muskarinergen Acetylcholin-Rezeptoren (Kruse et al., Mol Pharmacol., 2013, 84(4), 528-540). Der M2R weist neben einer orthosterischen auch eine allosterische Bindungstelle auf (Gregory et al., Current Neuropharmacol., 2007, 5(3), 157-167). Der älteste bekannte allosterische Modulator ist das Gallamin (Clark and Mitchelson, Br. J. Pharmac., 1976, 58, 323-331).

Allosterische Modulatoren weisen gegenüber klassischen, orthosterischen Liganden deutliche Unterschiede auf. Der allosterische Modulator selbst hat keinen direkten Einfluss auf die Rezeptoraktivierung. Durch die Bindung des Allosters kommt es vielmehr zu einer Modulation der Bindungsaffinität und/oder Effektivität des orthosterischen Agonisten. Die Wirkung eines allosterischen Modulators kann sich also nur in der Anwesenheit des endogenen Liganden entfalten. Daraus ergibt sich eine räumliche und zeitliche Spezifität der allosterischen Wirkung (Conn et al., Nat. Rev. Drug Disc., 2009, 8, 41-54; Conn et al, Nat. Rev. Drug. Disc., 2014, 13, 692-708). Darüber hinaus ist der Effekt eines allosterischen Modulators selbstlimitierend, wenn er in hohen Konzentrationen die Bindung des Agonisten stabilisiert. Hieraus wiederum resultiert grundsätzlich ein günstigeres sicherheitspharmakologisches Profil im Vergleich zu Agonisten, da toxische Effekte bedingt durch eine Rezeptorüberaktivierung begrenzt sind (Christopoulos, Mol. Pharmacol., 2014, 86, 463-478).

Die als Kooperativität bezeichnete gegenseitige Beeinflussung von allosterischen und orthosterischen Liganden hinsichtlich Affinität und intrinsischer Aktivität wird von beiden Liganden bestimmt. Im Falle eines positiven allosterischen Modulators des M2R werden die Effekte des ACh (orthosterischer Ligand) verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen (Wang et al., J. Pharmacol. Exp. Therap., 2009, 331, 340-348). Daraus ist im Falle des positiven allosterischen Modulators des M2R ein vorteilhaftes Wirkungsprofil, reduziertes Nebenwirkungsrisiko und ein Ansatzpunkt für die Entwicklung subtypselektiver Liganden gegenüber einem vollen Agonisten zu erwarten.

Von dem positiv allosterischen M4R und M2R-Liganden LY2119620 (3-Amino-5-chlor-*N*-cyclopropyl-4-methyl-6-[2-(4-methylpiperazin-1-yl)-2-oxoethoxy]thieno[2,3-b]pyridin-2-carboxamid) wurde die Kristallstruktur im Komplex mit dem M2R veröffentlicht. Die allosterische Bindungsstelle des M2R befindet sich räumlich benachbart, aber klar abgegrenzt zur orthosterischen Bindungsstelle und zeigt im Vergleich mit den anderen muskarinergen Rezeptor-Subtypen eine geringere Konservierung bzw. weist größere Sequenzunterschiede auf (Kruse et al., Nature, 2013, 504, 101-106). LY2119620 wurde als ein unselektiver M2R/M4R positiv allosterischer Modulator beschrieben (Croy et al., Molecular Pharmacology, July 2014 86, 1, 106-115; Schober et al., Molecular Pharmacology, July 2014 86, 1, 116-123).

Der M2R spielt als ein Bestandteil des autonomen Nervensystems eine wichtige Rolle in der Pathogenese und Progression von kardiovaskulären Erkrankungen. Autonomes Ungleichgewicht gekennzeichnet durch vagale (parasympathische) Abschwächung und eine Dominanz des sympathischen Nervensystems steht im engen Zusammenhang mit einer erhöhten Morbidität und Mortalität. Die klinische und prognostische Bedeutung des autonomen Ungleichgewichts ist gut dokumentiert in diversen Herz-Kreislauf-Erkrankungen, einschließlich Herzinsuffizienz (HF), Herzrhythmusstörungen, Ischämie /Reperfusion (I / R), Hypertension (He et al., Br. J. Pharmacol. 2014**,** Epub) und chronischer Nierenerkrankung (Ranpuria et al., Nephrol Dial Transplant. 2008, 23(2), 444-4499). Besonders bei Patienten mit Komorbiditäten wie Diabetes kann die autonome Imbalance zu gesteigerter Morbidität und Mortalität beitragen (Vinik et al., Diabet Med., 2011, 28(6), 643-651). Barorezeptor-Reflex-Funktionsstörungen, wie hypertensive Krisen oder Bluthochdruck-Variabilität, als Anzeichen einer Störung des autonomen Nervensystems, begleiten oft die akute Phase des ischämischen oder hämorrhagischen Schlaganfalls (Sykora et al., Stroke, 2009, 40(12), 678-682).

Die oft beobachtete Komorbidität zwischen kardiovaskulären und psychischen Erkrankungen, wie zwischen Herzinsuffizienz und Depression, basiert wahrscheinlich auf gemeinsamen Pathomechanismen, die mit der autonomen Imbalance einhergehen (Halaris et al., Mod Trends Pharmacopsychiatri., 2013, 28, 144-161). Chronischer Stress verschiebt das homöostatische Gleichgewicht des autonomen Nervensystems. Der verminderte Vagotonus trägt zu einem pro-inflammatorischen Status bei, wobei die Neurotransmitter-Regulierung, insbesondere die serotonerge Transmission, beeinträchtigt ist. Mit der autonomen Dysregulation wurden auch andere psychische Erkrankungen in Zusammenhang gebracht, wie z.B. die Aufmerksamkeitsdefizit-/Hyperaktivitätsstörung (ADHS), die sich durch Enthemmung, mangelhafte emotionale Selbstkontrolle, Unaufmerksamkeit und Hyperaktivität kennzeichnet (Rash and Aguirre-Camacho, Atten Defic Hyperact Disord., 2012, 4(4), 167-177).

Eine Stärkung der parasympathischen Aktivität durch einen positiven allosterischen Modulator einschließlich zu erwartender antiinflammatorischer Effekte, Erhöhung des Stickstoffmonoxids (NO), Regulierung des Redox-Zustands, Verbesserung der mitochondrialen Funktion und der Calcium-Regulation könnte daher ein neues Therapieprinizip insbesondere bei Herz-Kreislauferkrankungen darstellen. Es gibt zahlreiche Hinweise, dass die Modulation der parasympathischen Aktivität als potenzielles Therapieziel bei chronischer Herzinsuffizienz in Betracht gezogen werden kann. Vagusnervstimulation bei Hunden mit abgeheiltem Myokardinfarkt konnte die Inzidenz des plötzlichen Herztodes sowie bei chronisch herzinsuffizienten Ratten die Mortalität signifikant senken (De Ferrari, J. Cardiovasc. Transl. Res., 2014, 7(3), 310-320). In einem Hundemodell mit Herzinsuffizienz (LVEF 35%) und implantiertem Vagusstimulator konnte nachgewiesen werden, dass bei der Therapiegruppe im Vergleich zur Sham-Gruppe eine signifikante Verbesserung der linksventrikulären Ejektionsfraktion (LVEF) und Reduktion der endsystolischen und -diastolischen Volumina (LVESV; LVEDV) auftrat ebenso wie eine signifikante Reduktion der Herzfrequenz innerhalb von 3 Monaten. Der beschriebene Effekt der VNS war additiv zur Betablockergabe (De Ferrari, J. Cardiovasc. Transl. Res., 2014, 7(3), 310-320). Die Plasmalevel für TNF-$\alpha$ und IL-6 als auch deren myokardiale Proteinexpression konnte durch eine Vagusstimulation in diesem Tiermodel gesenkt werden, was dafür spricht, dass eine Stärkung des parasympatischen Nervensystems neben den Effekten auf LV-Remodeling auch positive Effekte auf proinflammatorische Zytokine hat.

Basierend auf den experimentellen präklinischen Daten erfolgten mittlerweile die ersten klinischen Studien zur vagalen Stimulation bei Patienten mit chronischer Herzinsuffizienz, wie bereits in der Behandlung der Epilepsie und Depression etabliert. Der Effekt der Stärkung des Parasympatikus über direkte Vagusnervstimulation (VNS) wurde in einer nicht randomisierten Beobachtungsstudie mit 32 Patienten mit einer linksventrikulären (LV) systolischen Dysfunktion bewertet, wobei die Ergebnisse darauf hindeuteten, daß eine Vagusreizung die Lebensqualität, die körperliche Belastbarkeit und das LV-Remodeling günstig beeinflusst (De Ferrari GM et al., Eur. Heart J., 2011, 32, 847-855) In der multizentrischen Open-Label-Machbarkeitsstudie ANTHEM-HF wurden die Sicherheit, Verträglichkeit und Wirksamkeit der VagusStimulation bei Patienten mit chronischer stabiler, symptomatischer Herzinsuffizienz mit reduzierter Auswurffraktion (HFrEF) zusätzlich zur Standardtherapie geprüft (Premchand RK et al., J. Card. Fail., 2014, 20(11), 808-816). Die in dieser Studie angewandte kontinuierliche Vagusnervstimulation führte zu einer Verbesserung der Auswurffraktion, Herzfrequenzvariabilität, NYHA-Klasse und Lebensqualität. Die erste Placebo-kontrollierte klinische Studie NECTAR-HF konnte hingegen keine signifikante Wirkung der Vagusnervstimulation auf die Herzfunktion von HF Patienten nach 6 Monaten zeigen (Zannad et al., Eur. Heart J., 2015, 36(7), 425-433). Lediglich die Lebensqualität konnte verbessert werden. Die INOVATE-HF Studie mit 650 HF Patienten konnte keine Effekte dieser Therapie in Bezug auf die Mortalität und Hospitalisierung zeigen. (Gold et al., JAm Coll Cardiol., 2016, Mar 29. pii: S0735-1097(16)32404-4. doi: 10.1016/j.jacc.2016.03.525). Lebensqualität und Gehstrecke konnten signifikant verbessert werden.

Neben dem Infektionsrisiko und den potentiellen Risiken eines chirurgischen Eingriffs ist eine Therapie mittels elektrischer Stimulierung des Vagusnervs limitiert durch Nebenwirkungen wie: Dysphonie, Husten und Mund-/Rachenschmerzen (Premchand RK et al., J. Card. Fail., 2014, 20(11), 808-816). Eine medikamentöse Stärkung des parasympatischen Nervensystems durch eine direkte Wirkung auf den M2R könnte eine neue Therapieoption darstellen.

Vorhofflimmern ist die häufigste anhaltende Herzrhythmusstörung und ihre Prävalenz nimmt mit dem Lebensalter zu (Chen et al., Circ. Res., 2014, 114(9), 1500-1515). Oft sind Vorhofflimmern und Herzinsuffizienz miteinander vergesellschaftet und verstärken sich gegenseitig. So nimmt die Prävalenz von Vorhofflimmern mit dem klinischen

Schweregrad der Herzinsuffizienz zu (Maisel and Stevenson, Am. J. Cardiol., 2003, 91, (suppl) 2D-8D). Klinische Daten legen nahe, dass Patienten, bei denen eine Herzinsuffizienz von Vorhofflimmern begleitet wird, eine schlechte Prognose haben. Sowohl die Letalität (Gesamtletalität, plötzlicher Tod und Pumpversagen) als auch die Morbidität (Hospitalisierung) erwies sich bei dieser Patientengruppe als signifikant erhöht.

Bei der Therapie des Vorhofflimmerns werden zwei Behandlungsstrategien unterschieden: die sog. Frequenzkontrolle mit Einstellung und möglichst Normalisierung der Kammerfrequenz im Rahmen des Vorhofflimmerns und die sog. Rhythmuskontrolle, welche Maßnahmen umfasst, mit denen ein Sinusrhythmus hergestellt oder erhalten werden soll. Eine effektive Behandlung besteht aus einer Kombination von nichtmedikamentösen sowie medikamentösen oder interventionellen Maßnahmen (Levalter T, Fortbildungsprogramm Pharmazie, 2011, 5, 106-127).

Zur medikamentösen Rhythmuskontrolle nach Kardioversion werden Betablocker, Klasse-I- und Klasse-III-Antiarrhythmika nach Maßgabe der kardialen Grunderkrankung und dem Ausmaß der linksventrikulären Pumpfunktionseinschränkung eingesetzt. Bei Patienten mit permanentem Vorhofflimmern als auch bei oligosymptomatischen (oft älteren) Patienten mit persistierendem oder paroxysmalem Vorhofflimmern ist die reine Frequenzkontrolle unter Beibehaltung und Belassen des Vorhofflimmerns oft die Therapie der Wahl. Eingesetzt werden primär Medikamente, die einen Einfluss auf die Refraktärperiode bzw. die Leitungskapazität des AV-Knotens haben. Prinzipiell könnte diese Wirkung durch eine Stimulation des M2R, der physiologisch die Schlüsselrolle an dieser Stelle spielt, z.B. mit Hilfe eines positiven allosterischen Modulators erreicht werden. Bis jetzt stehen Betablocker, Digitalis, Calciumantagonisten sowie in Einzelfällen Amiodaron zur Verfügung, die unter Berücksichtigung des Lebensstils, der kardialen Grunderkrankung bzw. etwaiger Begleiterkrankungen individualisiert zum Einsatz kommen. Insbesondere bei Patienten mit reduzierter linksventrikulärer Pumpfunktion und schwerer Herzinsuffizienz sind allerdings die Möglichkeiten der medikamentösen Therapie unzureichend. Calciumantagonisten sind kontraindiziert in dieser Patientengruppe. Therapie mit Digoxin führt, wie die neuesten Studien gezeigt haben, zu einer erhöhten Mortalität von Patienten mit Vorhofflimmern (Leong-Sit and Tang, Curr. Opin. Cardiol., 2015, Epub). Für Betablocker wurde in einer Metaanalyse eine fehlende Wirksamkeit bei Patienten mit Vorhofflimmern und Herzinsuffizienz (Leong-Sit and Tang, Curr. Opin. Cardiol., 2015, Epub). Entsprechend hoch ist der medizinische Bedarf nach neuen effizienten und sicheren Therapien zur Frequenzkontrolle. Dies könnte durch eine medikamentöse Stimulation des M2R erreicht werden.

Die Aufgabe der vorliegenden Erfindung liegt in der Identifizierung und Bereitstellung neuer Substanzen, die potente, positiv allosterische Modulatoren des muskarinergen M2 Rezeptors darstellen und sich als solche zur Behandlung und/oder Prävention insbesondere von kardiovaskulären Erkrankungen und/oder Nierenerkrankungen eignen.

1-Benzyl-substituierte 4-Oxo-1,4-Dihydrochinoline-3-carbonsäuren sind als allosterische Modulatoren des M1-Muskarinrezeptors zur Behandlung von neurodegenerativen Erkrankungen wie Alzheimer und Schizophrenie beschrieben (Scammells et al., ACS Chem. Neurosci., 2013, 4(7), 1026-1048; Mistry et al., J. Med. Chem. 2013, 56, 5151-5172). Unter anderem aus EP 0945435 B1 sind Pyridoncarbonsäurederivate bekannt, welche eine antibakterielle Antivität aufweisen. In WO 2002/085886-A2, WO 2003/050107-A1 und WO 2005/026145-A2 werden 7-Piperidino-substituierte Chinolon-Carbonsäurederivate, sowie in WO 2005/026165-A1 und WO 2005/049602-A1 verschiedene 7-Pyrrolidino-substituierte Chinolon-Carbonsäurederivate sowie in EP 1650192-A1 spezielle 7-Azetidinyl-Chinolon-Carbonsäurederivate mit einer antimikrobiellen / antibakteriellen Aktivität beansprucht. Aus WO 2005/009971-A1 und JP 2005012561 sind Chinolonderivate bekannt, welche als Thrombozytenaggregationshemmer eingesetzt werden können. In WO 2015/189560-A1 werden 1,4-Dihydrochinolinderivate als NPRC-Agonisten zur Behandlung von kardiovaskulären Erkrankungen offenbart. Chinolon-Carbonsäurederivate als MCT-Modulatoren werden in WO 2016/081464-A1 zur Behandlung von insbesondere Tumor-Erkrankungen und inflammatorischen Prozessen beschrieben.

Die vorliegende Erfindung betrifft Verbindungen der allgemeinen Formel (I)

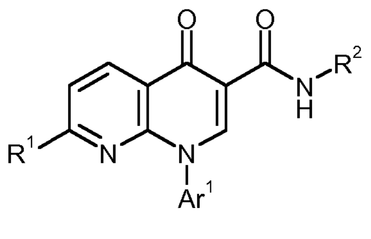

(I),

in welcher

$R^1$ für $NR^3R^4$ steht,
worin
$R^3$ Wasserstoff, Methyl, $(C_2-C_4)$-Alkyl oder $(C_3-C_6)$-Cycloalkyl bedeutet,
wobei $(C_2-C_4)$-Alkyl mit Hydroxy oder bis zu dreifach mit Fluor substituiert sein kannund

$R^4$ $(C_1\text{-}C_6)$-Alkyl, $(C_3\text{-}C_6)$-Cycloalkyl, 3- bis 6-gliedriges gesättigtes Heterocyclyl oder $(C_1\text{-}C_4)$-Alkylsulfonyl bedeutet,

wobei $(C_1\text{-}C_6)$-Alkyl, $(C_3\text{-}C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Hydroxycarbonyl, Oxo, Methoxy, Difluormethoxy, Trifluormethoxy, Cyano und weiterhin bis zu vierfach mit Fluor, substituiert sein können,oder

$R^3$ und $R^4$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder $SO_2$ als Ringglieder enthalten kann,

wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe $(C_1\text{-}C_4)$-Alkyl, Difluormethyl, Trifluormethyl, Hydroxy, Hydroxycarbonyl, Oxo, $(C_1\text{-}C_3)$-Alkoxy, Difluormethoxy, Trifluormethoxy, Cyano, $(C_1\text{-}C_3)$-Alkoxycarbonyl, Aminocarbonyl, Mono$(C_1\text{-}C_3)$-alkylaminocarbonyloxy, -NHC(=O)$R^{14A}$, -$CH_2$NHC(=O)$R^{14B}$, und weiterhin bis zu vierfach mit Fluor, substituiert sein können, worin

$R^{14A}$ und $R^{14b}$ unabhängig voneinander $(C_1\text{-}C_3)$-Alkyl oder Cyclopropyl darstellen,und

worin $(C_1\text{-}C_4)$-Alkyl ein- oder zweifach, gleich oder verschieden, mit Hydroxy, $(C_1\text{-}C_3)$-Alkoxy, und bis zu vierfach mit Fluor, substituiert sein kann,

$R^2$ für eine Gruppe der Formel

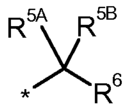
,
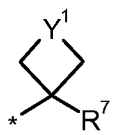
oder
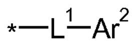

steht, worin

* die Verknüpfungsstelle mit dem N-Atom der Amid-Gruppierung markiert,

$R^{5A}$ Wasserstoff oder $(C_1\text{-}C_4)$-Alkyl bedeutet,

$R^{5B}$ Wasserstoff, $(C_1\text{-}C_4)$-Alkyl, Cyclopropyl, Monofluormethyl, Difluormethyl, Trifluormethyl, Pentafluorethyl, Methoxymethyl oder Trifluormethoxymethyl bedeutet,

$R^6$ bis zu fünffach mit Fluor substituiertes $(C_1\text{-}C_4)$-Alkyl oder bis zu vierfach mit Fluor substituiertes $(C_3\text{-}C_5)$-Cycloalkyl bedeutet,

$Y^1$ $-(CH_2)_k-$, $-CF_2-$, $-O-CH_2-$, $-CH_2-O-$ oder $-CH_2-O-CH_2-$ bedeutet,

worin

k für 0, 1, 2 oder 3 steht,

$R^7$ Wasserstoff, bis zu fünffach mit Fluor substituiertes $(C_1\text{-}C_2)$-Alkyl oder Trifluormethoxymethyl bedeutet,

$L^1$ eine Bindung oder eine Gruppe der Formel $-C(R^{8A}R^{8B})-(C(R^{9A}R^{9}B))_m-$ bedeutet, worin

m 0 oder 1 darstellt,

$R^{8A}$ Wasserstoff oder Methyl darstellt,

$R^{8b}$ Wasserstoff, Methyl, Trifluormethyl, Pentafluorethyl oder Trifluormethoxymethyl darstellt,

$R^{9A}$ und $R^{9B}$ unabhängig voneinander Wasserstoff oder Methyl darstellen,

$Ar^2$ Phenyl bedeutet,

wobei Phenyl ein- bis dreifach, gleich oder verschieden, mit Fluor, Chlor, $(C_1\text{-}C_3)$-Alkyl, Difluormethoxymethyl, Trifluormethoxymethyl und/oder Trifluormethyl substituiert sein kann,

oder

für einen 5- bis 10-gliedrigen bicyclischen oder tricyclischen Carbocyclus steht,

wobei der 5- bis 10-gliedrige bicyclische oder tricyclische Carbocyclus bis zu dreifach, gleich oder verschieden, mit $(C_1\text{-}C_3)$-Alkyl, Trifluormethyl, und weiterhin bis zu vierfach mit Fluor substituiert sein kann,

für einen über ein Ring-Kohlenstoffatom angebundenen Pyridinring steht,

wobei der Pyridinring ein oder zweifach mit Fluor, Chlor, Cyano, Methyl oder Trifluormethyl substituiert sein kann,

sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze,

Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Bei einem geeigneten pharmazeutisch unbedenklichen Salz der Verbindungen der vorliegenden Erfindung kann es sich beispielsweise um ein Säureadditionssalz einer Verbindung der vorliegenden Erfindung mit einem ausreichend basischen Stickstoffatom in einer Kette oder einem Ring handeln, wie ein Säureadditionssalz mit einer anorganischen Säure oder "Mineralsäure", beispielsweise Salzsäure, Bromwasserstoffsäure, Iodwasserstoffsäure, Schwefelsäure, Sulfaminsäure, Dischwefelsäure, Phosphorsäure oder Salpetersäure, oder mit einer organischen Säure, wie beispielsweise Ameisensäure, Essigsäure, Acetessigsäure, Brenztraubensäure, Trifluoressigsäure, Propionsäure, Buttersäure, Hexansäure, Heptansäure, Undecansäure, Laurylsäure, Benzoesäure, Salicylsäure, 2-(4-Hydroxybenzoyl)benzoesäure, Camphersäure, Zimtsäure, Cyclopentanpropionsäure, Diglukonsäure, 3-Hydroxy-2-naphthoesäure, Nicotinsäure, Pamoasäure, Pektinsäure, 3-Phenylpropionsäure, Pivalinsäure, 2-Hydroxyethansulfonsäure, Itaconsäure, Trifluormethansulfonsäure, Dodecylschwefelsäure, Ethansulfonsäure, Benzolsulfonsäure, para-Toluolsulfonsäure, Methansulfonsäure, 2-Naphthalinsulfonsäure, Naphthalindisulfonsäure, Camphersulfonsäure, Zitronensäure, Weinsäure, Stearinsäure, Milchsäure, Oxalsäure, Malonsäure, Bernsteinsäure, Äpfelsäure, Adipinsäure, Alginsäure, Maleinsäure, Fumarsäure, D-Glukonsäure, Mandelsäure, Ascorbinsäure, Glukoheptansäure, Glycerophosphorsäure, Asparaginsäure, Sulfosalicylsäure oder Thiocyansäure.

Ein anderes geeignetes pharmazeutisch unbedenkliches Salz einer ausreichend sauren Verbindung der vorliegenden Erfindung ist außerdem ein Alkalisalz, beispielsweise ein Natrium- oder Kaliumsalz, ein Erdalkalisalz, beispielsweise ein Calcium-, Magnesium- oder Strontiumsalz, oder ein Aluminium- oder Zinksalz oder ein Ammoniumsalz, das abgeleitet ist von Ammoniak oder einem organischen primären, sekundären oder tertiären Amin mit 1 bis 20 Kohlenstoffatomen, wie Ethylamin, Diethylamin, Triethylamin, Ethyldiisopropylamin, Monoethanolamin, Diethanolamin, Triethanolamin, Dicyclohexylamin, Dimethylaminoethanol, Diethylaminoethanol, Tris(hydroxymethyl)aminomethan, Procain, Dibenzylamin, *N*-Methylmorpholin, Arginin, Lysin, 1,2-Ethylendiamin, *N*-Methylpiperidin, *N*-Methylglucamin, *N,N*-Dimethylglucamin, *N*-Ethylglucamin, 1,6-Hexandiamin, Glucosamin, Sarcosin, Serinol, 2-Amino-1,3-propandiol, 3-Amino-1,2-propandiol, 4-Amino-1,2,3-butantriol, oder ein Salz mit einem quartären Ammoniumion mit 1 bis 20 Kohlenstoffatomen, wie Tetramethylammonium, Tetraethylammonium, Tetra(*n*-propyl)ammonium, Tetra(*n*-butyl)ammonium, *N*-Benzyl-*N,N,N*-trimethylammonium, Cholin oder Benzalkonium.

Weiterhin ist dem Fachmann bekannt, dass Säureadditionssalze der beanspruchten Verbindungen durch Umsetzung der Verbindungen mit der entsprechenden anorganischen oder organischen Säure nach einer Reihe bekannter Methoden hergestellt werden können. Alternativ dazu werden Alkali- und Erdalkalisalze der sauren erfindungsgemäßen Verbindungen hergestellt, indem man die erfindungsgemäßen Verbindungen nach verschiedenen bekannten Methoden mit der entsprechenden Base umsetzt.

Die vorliegende Erfindung schließt alle möglichen Salze der Verbindungen der vorliegenden Erfindung als einzelne Salze oder als Gemisch dieser Salze in einem beliebigen Verhältnis ein.

Wenn im vorliegenden Text, insbesondere im Experimentellen Teil, für die Synthese von Zwischenprodukten und Beispielen der vorliegenden Erfindung eine Verbindung als Salzform mit der entsprechenden Base oder Säure erwähnt wird, ist die exakte stöchiometrische Zusammensetzung der Salzform, wie sie durch das jeweilige Herstellungs- und/oder Reinigungsverfahren erhalten wurde, in den meisten Fällen nicht bekannt. Sofern nicht anders angegeben, bedeuten Zusätze zu chemischen Namen oder Strukturformeln, die sich auf Salze beziehen, wie beispielsweise "Hydrochlorid", "Trifluoracetat", "Natriumsalz" oder "x HCl", "x $CF_3COOH$", "x $Na^+$" eine Salzform, wobei die Stöchiometrie dieses Salzes nicht angegeben ist. Dies gilt entsprechend für Fälle, in denen Synthesezwischenprodukte oder Beispielverbindungen oder Salze davon nach dem beschriebenen Herstellungs- und/oder Reinigungsverfahren als Solvate, beispielsweise als Hydrate, erhalten wurden.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren).

Die vorliegende Erfindung umfasst deshalb die Enantiomere und Diastereomere sowie ihre jeweiligen Mischungen. Aus solchen Mischungen von Enantiomeren und/ oder Diastereomeren lassen sich die stereoisomer einheitlichen Bestandteile in bekannter Weise isolieren. Vorzugsweise werden hierfür chromatographische Verfahren angewandt, insbesondere die HPLC-Chromatographie an achiralen bzw. chiralen Trennphasen. Im Falle von Carbonsäuren als Zwischen- oder Endprodukten kann alternativ auch eine Trennung über diastereomere Salze mit Hilfe chiraler Amin-Basen erfolgen.

Der Begriff "enantiomerenrein" wird im Rahmen der vorliegenden Erfindung dahingehend verstanden, dass die betreffende Verbindung hinsichtlich der Absolutkonfiguration der chiralen Zentren in einem Enantiomerenüberschuss von mehr als 95%, bevorzugt von mehr als 98% vorliegt. Der Enantiomerenüberschuss (engl. *enantiomeric excess,* ee-Wert) wird hierbei durch Auswertung des Chromatogramms einer HPLC-Analyse an chiraler Phase nach der folgenden Formel berechnet:

$$ee = \left| \frac{\text{Enantiomer 1 (Flächenprozent)} - \text{Enantiomer 2 (Flächenprozent)}}{\text{Enantiomer 1 (Flächenprozent)} + \text{Enantiomer 2 (Flächenprozent)}} \right| \text{ x } 100\%.$$

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ("unnatürlicher Anteil") ausgetauscht ist. Unter dem Ausdruck "unnatürlicher Anteil" ist ein Anteil eines derartigen Isotops zu verstehen, der höher als dessen natürliche Häufigkeit ist. Die in diesem Zusammenhang anzuwendenden natürlichen Häufigkeiten von Isotopen finden sich in "Isotopic Compositions of the Elements 1997", Pure Appl. Chem., 70(1), 217-235, 1998. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Im Hinblick auf die Behandlung und/oder Prophylaxe der hier angegebenen Störungen enthalten die isotopischen Variante(n) der Verbindungen der allgemeinen Formel (I) vorzugsweise Deuterium ("deuteriumhaltige Verbindungen der allgemeinen Formel (I)"). Isotopische Varianten der Verbindungen der allgemeinen Formel (I), in die ein oder mehrere radioaktive Isotope, wie $^{3}H$ oder $^{14}C$, eingebaut sind, sind beispielsweise bei Arzneistoff- und/oder Substratsgewebeverteilungsstudien von Nutzen. Diese Isotope sind wegen ihrer leichten Einbaubarkeit und Nachweisbarkeit besonders bevorzugt. In eine Verbindung der allgemeinen Formel (I) können Positronen emittierende Isotope wie $^{18}F$ oder $^{11}C$ eingebaut werden. Diese isotopischen Varianten der Verbindungen der allgemeinen Formel (I) eignen sich zur Verwendung bei in-vivo-Bildgebungsanwendungen. Deuteriumhaltige und $^{13}C$-haltige Verbindungen der allgemeinen Formel (I) können im Rahmen präklinischer oder klinischer Studien bei Massenspektrometrie-Analysen verwendet werden (H. J. Leis et al., Curr. Org. Chem., 1998, 2, 131). Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Isotopische Varianten der Verbindungen der allgemeinen Formel (I) können im Allgemeinen nach dem Fachmann bekannten Verfahren wie in den hier beschriebenen Schemata und/oder Beispielen beschrieben hergestellt werden, indem man ein Reagenz durch eine isotopische Variante des Reagenzes, vorzugsweise ein deuteriumhaltiges Reagenz, ersetzt. Je nach den gewünschten Deuterierungsstellen kann in einigen Fällen Deuterium aus $D_2O$ entweder direkt in die Verbindungen oder in Reagenzien, die für die Synthese derartiger Verbindungen verwendet werden können, eingebaut werden (Esaki et al., Tetrahedron, 2006, 62, 10954; Esaki et al., Chem. Eur. J., 2007, 13, 4052). Ein nützliches Reagenz zum Einbau von Deuterium in Moleküle ist auch Deuteriumgas. Eine schnelle Route zum Einbau von Deuterium ist die katalytische Deuterierung von olefinischen Bindungen (H. J. Leis et al., Curr. Org. Chem., 1998, 2, 131; J. R. Morandi et al., J. Org. Chem., 1969, 34(6), 1889) und acetylenischen Bindungen (N. H. Khan, J. Am. Chem. Soc., 1952, 74(12), 3018; S. Chandrasekhar et al., Tetrahedron, 2011, 52, 3865). Zum direkten Austausch von Wasserstoff gegen Deuterium in funktionelle Gruppen enthaltenden Kohlenwasserstoffen können auch Metallkatalysatoren (d.h. Pd, Pt und Rh) in Gegenwart von Deuteriumgas verwendet werden (J. G. Atkinson et al., US-Patent 3966781). Verschiedene deuterierte Reagenzien und Synthesebausteine sind im Handel von Firmen wie beispielsweise C/D/N Isotopes, Quebec,

Kanada; Cambridge Isotope Laboratories Inc., Andover, MA, USA; und CombiPhos Catalysts, Inc., Princeton, NJ, USA, erhältlich. Weitere Informationen bezüglich des Standes der Technik im Hinblick auf Deuterium-Wasserstoff-Austausch finden sich beispielsweise in Hanzlik et al., J. Org. Chem., 1990, 55, 3992-3997; R. P. Hanzlik et al., Biochem. Biophys. Res. Commun., 1989, 160, 844; P. J. Reider et al., J. Org. Chem., 1987, 52, 3326-3334; M. Jarman et al., Carcinogenesis ,1993, 16(4), 683-688; J. Atzrodt et al., Angew. Chem., Int. Ed. 2007, 46, 7744; K. Matoishi et al., 2000, J. Chem. Soc, Chem. Commun., 1519-1520; K. Kassahun et al., WO 2012/112363.

Der Begriff "deuteriumhaltige Verbindung der allgemeinen Formel (I)" ist definiert als eine Verbindung der allgemeinen Formel (I), in der ein oder mehrere Wasserstoffatome durch ein oder mehrere Deuteriumatome ersetzt sind und in der die Häufigkeit von Deuterium in jeder deuterierten Position der Verbindung der allgemeinen Formel (I) höher ist als die natürliche Häufigkeit von Deuterium, die etwa 0,015% beträgt. Insbesondere ist in einer deuteriumhaltigen Verbindung der allgemeinen Formel (I) die Häufigkeit von Deuterium in jeder deuterierten Position der Verbindung der allgemeinen Formel (I) höher als 10%, 20%, 30%, 40%, 50%, 60%, 70% oder 80%, vorzugsweise höher als 90%, 95%, 96% oder 97%, noch weiter bevorzugt höher als 98% oder 99% in dieser Position bzw. diesen Positionen. Es versteht sich, dass die Häufigkeit von Deuterium in jeder deuterierten Position von der Häufigkeit von Deuterium in anderen deuterierten Positionen unabhängig ist.

Durch den selektiven Einbau eines oder mehrerer Deuteriumatome in eine Verbindung der allgemeinen Formel (I) können die physikalisch-chemischen Eigenschaften (wie beispielsweise Acidität [A. Streitwieser et al., J. Am. Chem. Soc., 1963, 85, 2759; C. L. Perrin et al., J. Am. Chem. Soc., 2007, 129, 4490], Basizität [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], Lipophilie [B. Testa et al., Int. J. Pharm., 1984, 19(3), 271]) und/oder das Stoffwechselprofil des Moleküls verändert und Veränderungen des Verhältnisses von Stammverbindung zu Metaboliten oder der gebildeten Mengen von Metaboliten verursacht werden. Derartige Veränderungen können zu bestimmten therapeutischen Vorteilen führen und daher unter bestimmten Umständen bevorzugt sein. Es ist über verringerte Stoffwechselraten und Stoffwechselumschaltung, bei der sich das Verhältnis von Metaboliten ändert, berichtet worden (D. J. Kushner et al., Can. J. Physiol. Pharmacol., 1999, 77, 79; A. E. Mutlib et al., Toxicol. Appl. Pharmacol., 2000, 169, 102). Diese Veränderungen der Exposition gegenüber Stammverbindung und Metaboliten können wichtige Konsequenzen hinsichtlich Pharmakodynamik, Verträglichkeit und Wirksamkeit einer deuteriumhaltigen Verbindung der allgemeinen Formel (I) haben. In einigen Fällen wird durch den Deuteriumersatz die Bildung eines unerwünschten oder toxischen Metaboliten verringert oder eliminiert und die Bildung eines gewünschten Metaboliten verstärkt (z.B. Nevirapin: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In anderen Fällen besteht der Haupteffekt der Deuterierung darin, die Geschwindigkeit der systemischen Clearance zu verringern. Dadurch wird die biologische Halbwertszeit der Verbindung erhöht. Zu den potentiellen klinischen Vorteilen gehören die Fähigkeit zur Aufrechterhaltung einer ähnlichen systemischen Exposition mit verringerten Spitzenspiegeln und erhöhten Talspiegeln. Dies könnte je nach der Pharmakokinctik/Pharmakodynamik-Bczichung der jeweiligen Verbindung zu geringeren Nebenwirkungen und erhöhter Wirksamkeit führen. Beispiele für diesen Deuterium-Effekt sind Indiplon (A. J. Morales et al., Abstract 285, The 15th North American Meeting of the International Society of Xenobiotics, San Diego, CA, 12.-16. Oktober, 2008), ML-337 (C. J. Wenthur et al., J. Med. Chem, 2013, 56, 5208) und Odanacatib (K. Kassahun et al., WO2012/112363). Es ist auch noch über weitere Fälle berichtet worden, bei denen verringerte Verstoffwechslungsraten zu einer Erhöhung der Arzneistoffexposition ohne Änderung der Rate der systemischen Clearance führen (z.B. Rofecoxib: F. Schneider et al., Arzneim. Forsch. Drug. Res., 2006, 56, 295; Telaprevir: F. Maltais et al., J. Med. Chem., 2009, 52, 7993). Deuterierte Arzneistoffe, die diesen Effekt zeigen, können verringerte Dosierungsanforderungen haben (z.B. geringere Zahl von Dosen oder niedrigere Dosierung zur Erzielung der gewünschten Wirkung) und/oder zu geringeren Metabolitenbelastungen führen.

Eine Verbindung der allgemeinen Formel (I) kann mehrere potenzielle Angriffsstellen für die Verstoffwechslung aufweisen. Zur Optimierung der oben beschriebenen Effekte auf die physikalisch-chemischen Eigenschaften und das Stoffwechselprofil können deuteriumhaltige Verbindungen der allgemeinen Formel (I) mit einem bestimmten Muster eines oder mehrerer Deuterium-Wasserstoff-Austausche gewählt werden. Insbesondere ist/sind das Deuteriumatom/die Deuteriumatome deuteriumhaltiger Verbindung(en) der allgemeinen Formel (I) an ein Kohlenstoffatom gebunden und/oder steht/stehen in den Positionen der Verbindung der allgemeinen Formel (I), bei denen es sich um Angriffsstellen für Verstoffwechslungsenzyme wie z.B. Cytochrom $P_{450}$ handelt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylsulfonyl, Alkylaminocarbonyloxy und Alkoxycarbonyl stehen für einen linearen oder verzweigten Alkylrest mit in der Regel 1 bis 6, vorzugsweise 1 bis 4 oder 1 bis 3 Kohlenstoffatomen, beispielhaft und vorzugsweise für Methyl, Ethyl, n-Propyl, Isopropyl, tert.-Butyl, iso-Butyl, (2-Methyl-prop-1-yl), n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylaminocarbonyloxy steht für einen Alkylaminocarbonyloxyrest mit einem oder zwei (unabhängig voneinander gewählten) Alkylsubstituenten. ($C_1$-$C_3$)-Alkylaminocarbonyloxy steht beispielsweise für einen Monoalkylaminocarbonyloxyrest mit 1 bis 3 Kohlenstoffatomen oder für einen Dialkylaminocarbonyloxyrest mit jeweils 1 bis 3 Kohlenstoffatomen pro Alkylsubstituent. Beispielhaft und vorzugsweise seien genannt: Methylaminocarbonyloxy, Ethylaminocarbonyloxy, n-Propylaminocarbonyloxy, Isopropylaminocarbonyloxy, tert.-Butylaminocarbonyloxy, n-Pentylaminocarbonyloxy, n-Hexylaminocarbonyloxy, *N,N*-Dimethylaminocarbonyloxy, *N,N*-Diethylaminocarbonyloxy, *N*-Ethyl-*N*-methylaminocarbonyloxy, *N*-Methyl-*N*-n-propylaminocarbonyloxy, *N*-Isopropyl-*N*-n-propylaminocarbonyloxy, *N*-tert.-Butyl-*N*-methylaminocarbonyl, *N*-Ethyl-*N*-n-pentylamino-carbonyl und *N*-n-Hexyl-*N*-methylaminocarbonyloxy.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Carbocyclus steht im Rahmen der Erfindung für einen mono-, poly- oder spirocyclischen, vorzugsweise mono-, oder bicyclischen gesättigten Carbocyclus mit insgesamt 3 bis 6 Ringatomen. Ein monocyclischer, gesättigter Carbocyclus wird synonym mit Cycloalkyl bezeichnet. Beispielhaft seien genannt: Cyclopropyl, Cyclobutyl, Cyclopentyl, Cyclohexyl, Cycloheptyl, Cyclopentenyl, Cyclohexenyl, Cyclohexadienyl, Cycloheptenyl, Cycloheptadienyl, Spiro[2.3]hexyl, Spiro[2.4]heptyl, Spiro[2.5]oktyl, Bicyclo[1.1.1]pentyl, Bicyclo[2.2.1]heptyl, Bicyclo[2.2.2]oktyl, Tricyclo[3.3.1.13,7]decyl. Bevorzugt ist monocyclisches Cycloalkyl mit 3 bis 5 Kohlenstoffatomen. Beispielhaft und bevorzugt seien genannt: Cyclopropyl, Cyclobutyl, Cyclopentyl oder Bicyclo[1.1.1]pent-1-yl.

Heterocyclyl steht für einen mono-, poly- oder spirocyclischen, vorzugsweise mono-, bi- oder spirocyclischen nicht-aromatischen heterocyclischen Rest mit in der Regel 3 bis 10 Ringatomen und bis zu 3, vorzugsweise bis zu 2 Heteroatomen und/oder Heterogruppen aus der Reihe N, O, S, SO, $SO_2$.,. Die Heterocyclyl-Reste können gesättigt oder partiell ungesättigt sein. Bevorzugt sind 4- bis 6-gliedrige monocyclische gesättigte Heterocyclylreste mit einem Stickstoffatom sowie solche mit einem weiteren Heteroatom aus der Reihe N oder O, sowie 6- bis 7-gliedrige bi- oder spirocyclische gesättigte Heterocyclylreste mit einem Stickstoffatom. Beispielhaft und vorzugsweise seien genannt: Azetidinyl, Pyrrolidinyl, Piperidinyl, Piperazinyl, Oxazolidinyl, Imidazolidinyl, Morpholinyl, Tetrahydropyrimidin, Azaspiro[2.4]heptyl oder Azabicyclo[3.1.0]hexyl.

Halogen steht für Fluor, Chlor, Brom und Iod, vorzugsweise für Chlor.

In der Formel der Gruppe, für die $R^1$, $R^2$, $Ar^1$, $Ar^2$, $Y^1$, bzw. $Y^2$ stehen kann, steht der Endpunkt der Linie, an dem das Zeichen $\#^1$, $\#^2$, $\#^3$; *, ** und *** steht, nicht für ein Kohlenstoffatom beziehungsweise eine $CH_2$-Gruppe, sondern ist Bestandteil der Bindung zu dem jeweils bezeichneten Atom, an das $R^1$, $R^2$, $Ar^1$, $Ar^2$, $Y^1$, bzw. $Y^2$ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der Formel (I), in welcher

$R^1$ für $NR^3R^4$ steht,
worin
$R^3$ Wasserstoff, Methyl oder ($C_2$-$C_4$)-Alkyl bedeutet, und
$R^4$ bis zu vierfach mit Fluor substituiertes ($C_1$-$C_6$)-Alkyl bedeutet,
wobei ($C_1$-$C_6$)-Alkyl mit Oxo substituiert sein kann,oder
$R^3$ und $R^4$ zusammen mit dem Stickstoffatom, an das sie gebunden sind, einen gesättigten 4- bis 6-gliedrigen monocyclischen oder 6- bis 9-gliedrigen bicyclischen Heterocyclus bilden, der ein oder zwei weitere, gleiche oder verschiedene Heteroatome aus der Reihe N und/oder O als Ringglieder enthalten kann,

wobei der 4- bis 6-gliedrige monocyclische und der 6- bis 9-gliedrige bicyclische Heterocyclus jeweils mit 1 bis 5 Substituenten unabhängig voneinander ausgewählt aus der Gruppe ($C_1$-$C_4$)-Alkyl, Difluormethyl, Trifluormethyl, Hydroxy, Oxo, ($C_1$-$C_3$)-Alkoxy, Difluormethoxy, Trifluormethoxy und weiterhin bis zu vierfach mit Fluor, substituiert sein können,
worin ($C_1$-$C_4$)-Alkyl ein- oder zweifach, gleich oder verschieden, mit Hydroxy, ($C_1$-$C_3$)-Alkoxy, und bis zu vierfach mit Fluor, substituiert sein kann,

$R^2$ für eine Gruppe der Formel

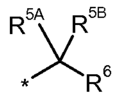 , 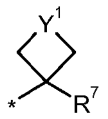 , 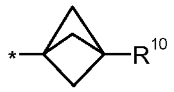 oder 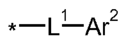

steht, worin
* die Verknüpfungsstelle mit dem N-Atom der Amid-Gruppierung markiert,
$R^{5A}$ Wasserstoff oder ($C_1$-$C_4$)-Alkyl bedeutet,
$R^{5B}$ Methyl, Monofluormethyl, Difluormethyl, Trifluormethyl, Pentafluorethyl oder Trifluormethoxymethyl bedeutet, und
$R^6$ bis zu fünffach mit Fluor substituiertes ($C_1$-$C_4$)-Alkyl oder bis zu vierfach mit Fluor substituiertes ($C_3$-$C_5$)-Cycloalkyl bedeutet,
$Y^1$ -$(CH_2)_k$- bedeutet,
worin
k für 1 oder 2 steht,
$R^7$ bis zu fünffach mit Fluor substituiertes ($C_1$-$C_2$)-Alkyl bedeutet,
$R^{10}$ Wasserstoff, Fluor oder Trifluormethyl bedeutet,
$L^1$ eine Bindung oder eine Gruppe der Formel -$CR^{8A}R^{8B}$- bedeutet,
worin
$R^{8A}$ Wasserstoff darstellt,
$R^{8b}$ Methyl, Trifluormethyl, Pentafluorethyl oder Trifluormethoxymethyl darstellt,
$Ar^2$ Phenyl bedeutet,
wobei Phenyl ein- bis dreifach, gleich oder verschieden, mit Fluor, Chlor, ($C_1$-$C_3$)-Alkyl, und/oder Trifluormethyl substituiert sein kann,

$Ar^1$ für einen über ein Ring-Kohlenstoffatom angebundenen Pyridinring steht,
wobei stituiert der Pyridinring ein oder zweifach mit Fluor, Chlor, Cyano, Methyl oder Trifluormethyl subsein kann,

sowie ihre Salze, Solvate und Solvate der Salze.

Bevorzugt im Rahmen der vorliegenden Erfindung sind Verbindungen der Formel (I), in welcher in welcher

$R^1$ für $NR^3R^4$ steht,
worin
$R^3$ Wasserstoff oder Methyl bedeutet, und
$R^4$ Methyl oder 2-Fluorethyl bedeutet,oder
für einen über ein Stickstoff-Atom gebundenen Heterocyclus der Formel

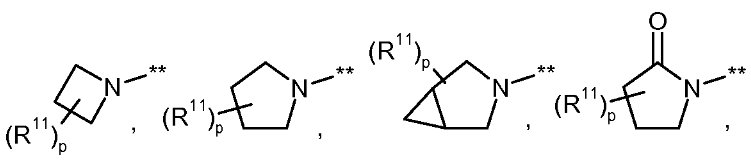

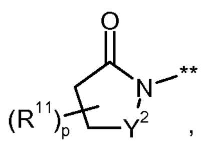 , 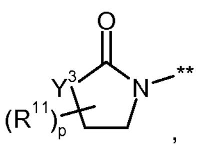 , 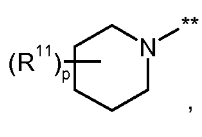 ,

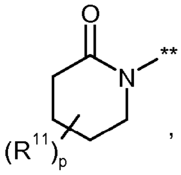,
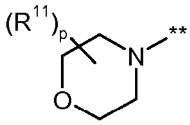
oder
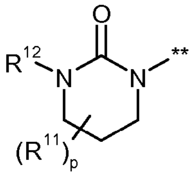

steht, worin

** die Verknüpfungsstelle mit dem Rest des Moleküls markiert,

$Y^2$ eine Gruppe der Formel

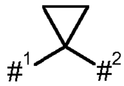

darstellt, worin

#1 die Verknüpfungsstelle mit dem N-Atom des Pyrrolidinonrings markiert, und

#2 die Verknüpfungsstelle mit dem C-Atom des Pyrrolidinonrings markiert,

$Y^3$ -N($R^{12}$)- oder eine Gruppe der Formel

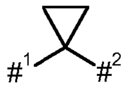

darstellt, worin

#1 und #2 jeweils die Verknüpfungsstelle mit dem C-Atom des Pyrrolidinonrings markieren,

$R^{11}$ Fluor, ($C_1$-$C_4$)-Alkyl, Difluormethyl, Trifluormethyl, Hydroxy, Hydroxymethyl, Methoxy, Difluormethoxy oder Trifluormethoxy darstellt,

p die Zahl 0, 1, 2, 3 oder 4 darstellt,

wobei im Fall, dass die Substituenten $R^{11}$ mehrfach auftreten, deren Bedeutungen jeweils gleich oder verschieden sein können,

$R^{12}$ Wasserstoff oder 2-Hydroxyethyl darstellt,

$R^2$ für eine Gruppe der Formel

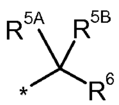,
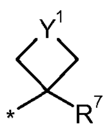,
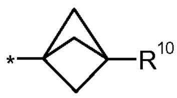
oder
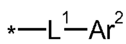

steht, worin

* die Verknüpfungsstelle mit dem N-Atom der Amid-Gruppierung markiert,

$R^{5A}$ Wasserstoff oder Methyl bedeutet,

$R^{5B}$ Methyl, Monofluormethyl, Difluormethyl, Trifluormethyl, Pentafluorethyl oder Trifluormethoxymethyl bedeutet, und

$R^6$ Methyl, Ethyl, Trifluormethyl, 2,2,2-Trifluorethyl, tert-Butyl, iso-Butyl oder Cyclopropyl bedeutet,

$Y^1$ $-(CH_2)_k-$ bedeutet,

worin

k für 1 oder 2 steht,

$R^7$ Trifluormethyl bedeutet,

$R^{10}$ Wasserstoff, Fluor oder Trifluormethyl bedeutet,

$L^1$ eine Bindung oder eine Gruppe der Formel $-CR^{8A}R^{8B}$ bedeutet,

worin

$R^{8A}$ Wasserstoff darstellt,

$R^{8b}$ Trifluormethyl darstellt,

$Ar^2$ Phenyl bedeutet,

wobei Phenyl ein- oder zweifach, gleich oder verschieden, mit Fluor und/oder Chlor substituiert sein kann,

$Ar^1$ für eine Gruppe der Formel

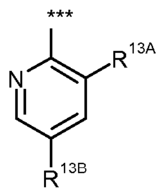 oder 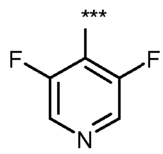

steht, worin
*** die Verknüpfungsstelle mit dem N-Atom markiert,
$R^{13A}$ für Fluor oder Chlor steht,
$R^{13B}$ für Fluor oder Wasserstoff steht,

sowie ihre Salze, Solvate und Solvate der Salze.

Besonders bevorzugt im Rahmen der vorliegenden Erfindung sind Verbindungen der Formel (I), in welcher in welcher

$R^1$ für einen über ein Stickstoff-Atom gebundenen Heterocyclus der Formel

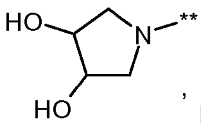, 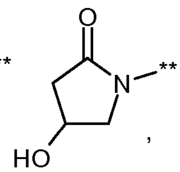, 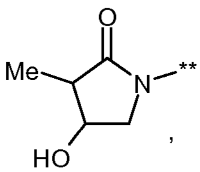, 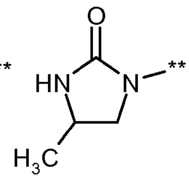 oder 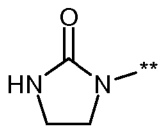

steht, worin
** die Verknüpfungsstelle mit dem Rest des Moleküls markiert,

$R^2$ für eine Gruppe der Formel

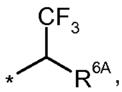, 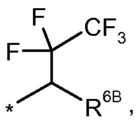, 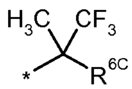, 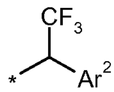 oder 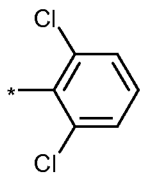

steht, worin
* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl oder Ethyl bedeutet,
$R^{6C}$ Trifluormethyl oder Cyclopropyl bedeutet,
$Ar^2$ für eine Gruppe der Formel

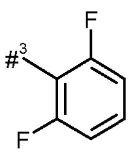 oder 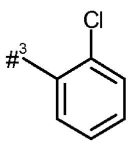

steht, worin
$\#^3$ jeweils die Verknüpfungsstelle markiert,

$Ar^1$ für eine Gruppe der Formel

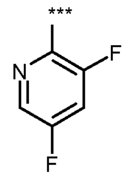

steht, worin
*** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

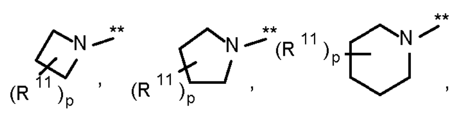

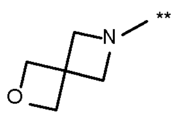

steht, worin
** die Verknüpfungsstelle mit dem Rest des Moleküls markiert,
$R^{11}$ Fluor, $(C_1-C_4)$-Alkyl, Difluormethyl, Trifluormethyl, Hydroxy, Hydroxymethyl, Methoxy, Difluormethoxy oder Trifluormethoxy darstellt,
p die Zahl 0, 1, 2, 3 oder 4 darstellt,
wobei im Fall, dass die Substituenten $R^{11}$ mehrfach auftreten, deren Bedeutungen jeweils gleich oder verschieden sein können,

$R^2$ für eine Gruppe der Formel

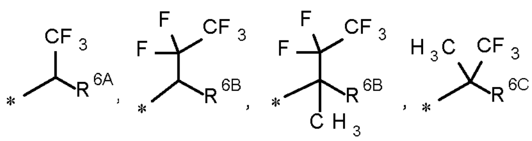

steht, worin
* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl, Ethyl, tert-Butyl oder Cyclopropyl bedeutet,
$R^{6C}$ Trifluormethyl oder Cyclopropyl bedeutet,

$Ar^1$ für eine Gruppe der Formel

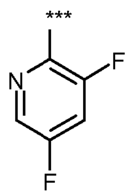

steht, worin
*** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

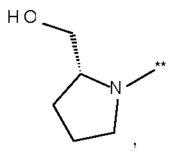, 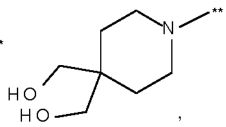, 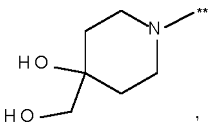, 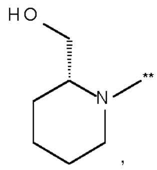,

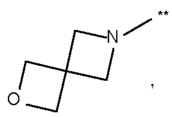, 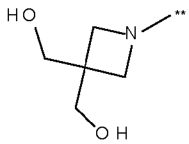

steht, worin

** die Verknüpfungsstelle mit dem Rest des Moleküls markiert,

$R^2$ für eine Gruppe der Formel

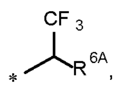, 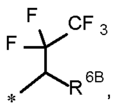, 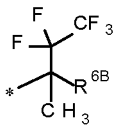

steht, worin

* die Verknüpfungsstelle mit dem N-Atom der Amid-Gruppierung markiert,

$R^{6A}$ Trifluormethyl bedeutet,

$R^{6B}$ Methyl bedeutet,

$Ar^1$ für eine Gruppe der Formel

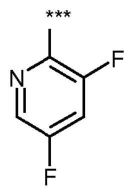

steht, worin

*** die Verknüpfungsstelle mit dem N-Atom markiert,

sowie ihre Salze, Solvate und Solvate der Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

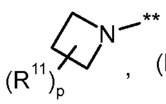, 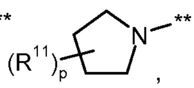, 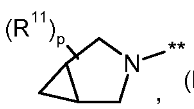, 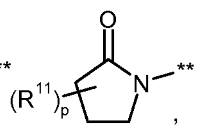,

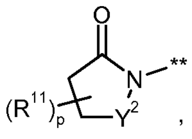, 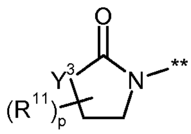 oder 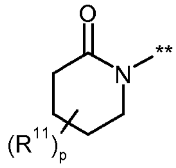

steht, worin
** die Verknüpfungsstelle mit dem Rest des Moleküls markiert,
$Y^2$ eine Gruppe der Formel

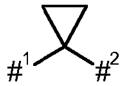

darstellt, worin
$\#^1$ die Verknüpfungsstelle mit dem N-Atom des Pyrrolidinonrings markiert, und
$\#^2$ die Verknüpfungsstelle mit dem C-Atom des Pyrrolidinonrings markiert,
$Y^3$ -N($R^{12}$)- oder eine Gruppe der Formel

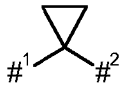

darstellt, worin
$\#^1$ und $\#^2$ jeweils die Verknüpfungsstelle mit dem C-Atom des Pyrrolidinonrings markieren,
$R^{11}$ Fluor, ($C_1$-$C_4$)-Alkyl, Difluormethyl, Trifluormethyl, Hydroxy, Hydroxymethyl, Methoxy, Difluormethoxy oder Trifluormethoxy darstellt,
p die Zahl 0, 1, 2, 3 oder 4 darstellt,
wobei im Fall, dass die Substituenten $R^{11}$ mehrfach auftreten, deren Bedeutungen jeweils gleich oder verschieden sein können,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

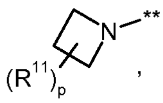, 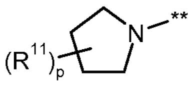 oder 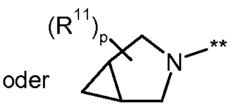

steht, worin
** die Verknüpfungsstelle mit dem Rest des Moleküls markiert,
$R^{11}$ Fluor, ($C_1$-$C_4$)-Alkyl, Difluormethyl, Trifluormethyl, Hydroxy, Hydroxymethyl, Methoxy, Difluormethoxy oder Trifluormethoxy darstellt,
p die Zahl 0, 1, 2, 3 oder 4 darstellt,
wobei im Fall, dass die Substituenten $R^{11}$ mehrfach auftreten, deren Bedeutungen jeweils gleich oder verschieden sein können,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

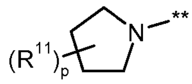

oder

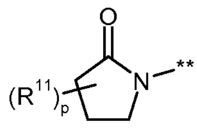

steht, worin

** die Verknüpfungsstelle mit dem Rest des Moleküls markiert,

$R^{11}$ Fluor, $(C_1-C_4)$-Alkyl, Difluormethyl, Trifluormethyl, Hydroxy, Hydroxymethyl, Methoxy, Difluormethoxy oder Trifluormethoxy darstellt,

p die Zahl 0, 1, 2, 3 oder 4 darstellt,

wobei im Fall, dass die Substituenten $R^{11}$ mehrfach auftreten, deren Bedeutungen jeweils gleich oder verschieden sein können,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

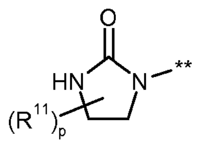

steht, worin

** die Verknüpfungsstelle mit dem Rest des Moleküls markiert,

$R^{11}$ Fluor, $(C_1-C_4)$-Alkyl, Difluormethyl, Trifluormethyl, Hydroxy, Hydroxymethyl, Methoxy, Difluormethoxy oder Trifluormethoxy darstellt,

p die Zahl 0, 1, 2, 3 oder 4 darstellt,

wobei im Fall, dass die Substituenten $R^9$ mehrfach auftreten, deren Bedeutungen jeweils gleich oder verschieden sein können,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

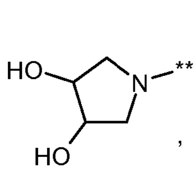

,

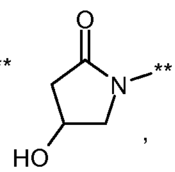

,

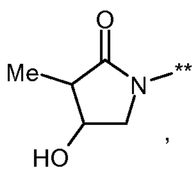

,

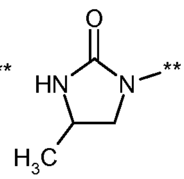

oder

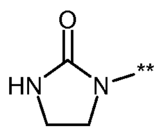

steht, worin

** die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

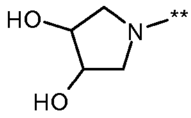 oder 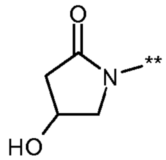

steht, worin

** die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^1$ für einen über ein Stickstoff-Atom gebundenen Heterocyclus der Formel

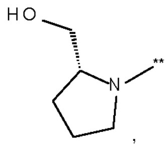, 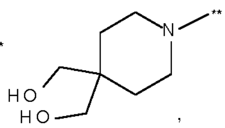, 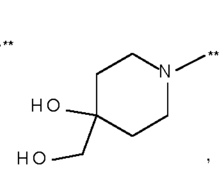, 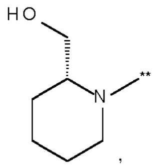,

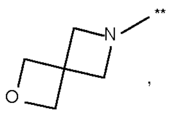, 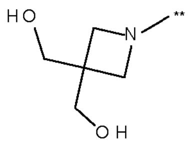

steht, worin

** die Verknüpfungsstelle mit dem Rest des Moleküls markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

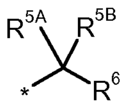 oder 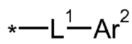

steht, worin

* die Verknüpfungsstelle mit dem N-Atom der Amid-Gruppierung markiert,

$R^{5A}$ Wasserstoff oder Methyl bedeutet,

$R^{5B}$ Methyl, Monofluormethyl, Difluormethyl, Trifluormethyl, Pentafluorethyl oder Trifluormethoxymethyl bedeutet, und

$R^6$ Methyl, Ethyl, Trifluormethyl, 2,2,2-Trifluorethyl, tert-Butyl, iso-Butyl oder Cyclopropyl bedeutet,

$L^1$ eine Bindung oder eine Gruppe der Formel $-CR^{8A}R^{8B}-$ bedeutet,

worin

$R^{8A}$ Wasserstoff darstellt,

$R^{8b}$ Trifluormethyl darstellt,

$Ar^2$ Phenyl bedeutet,

wobei Phenyl ein- oder zweifach, gleich oder verschieden, mit Fluor und/oder Chlor substituiert sein kann,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I),

in welcher

$R^2$ für eine Gruppe der Formel

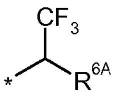, 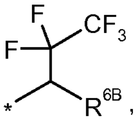, 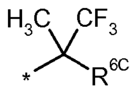, 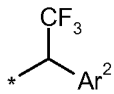 oder 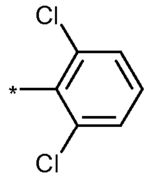

steht, worin
* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl oder Ethyl bedeutet,
$R^{6C}$ Trifluormethyl oder Cyclopropyl bedeutet,
$Ar^2$ für eine Gruppe der Formel

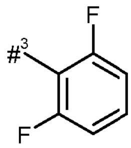 oder 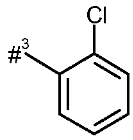

steht, worin
$\#^3$ jeweils die Verknüpfungsstelle markiert,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

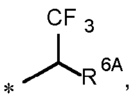, 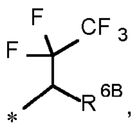, 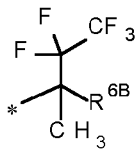, 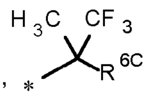

steht, worin
* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl, Ethyl, tert-Butyl oder Cyclopropyl bedeutet,
$R^{6C}$ Trifluormethyl oder Cyclopropyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

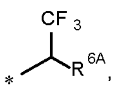, 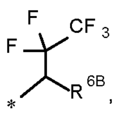, 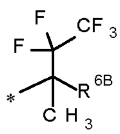

steht, worin

* die Verknüpfungsstelle mit dem N-Atom der Amid-Gruppierung markiert,
$R^{6A}$ Trifluormethyl bedeutet,
$R^{6B}$ Methyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

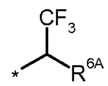

steht, worin
* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

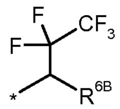

steht, worin
* die Verknüpfungsstelle mit dem N-Atom der Amid-Gruppierung markiert,
$R^{6B}$ Methyl oder Ethyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$R^2$ für eine Gruppe der Formel

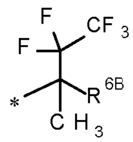

steht, worin
* die Verknüpfungsstelle mit dem N-Atom der Amid-Gruppierung markiert,
$R^{6B}$ Methyl bedeutet,

sowie ihre N-Oxide, Salze, Solvate, Salze der N-Oxide und Solvate der N-Oxide und Salze.

Eine weitere besondere Ausführungsform der vorliegenden Erfindung umfasst Verbindungen der Formel (I), in welcher

$Ar^1$ für eine Gruppe der Formel

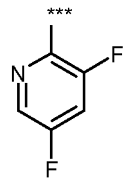

steht, worin
*** die Verknüpfungsstelle mit dem N-Atom markiert,

sowie ihre N-Oxide, Salze, Solvate, Salze der N-Oxide und Solvate der N-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche und Ausführungsformen.

Die als bevorzugt, besonders bevorzugt und ganz besonders bevorzugt genannten Restedefinitionen gelten sowohl für die Verbindungen der Formel (I) als auch in entsprechender Weise für alle Zwischenprodukte.

Weiterer Gegenstand der Erfindung ist ein Verfahren zur Herstellung von erfindungsgemäßen Verbindungen der Formel (I), dadurch gekennzeichnet, dass man

[A] eine Verbindung der Formel (II)

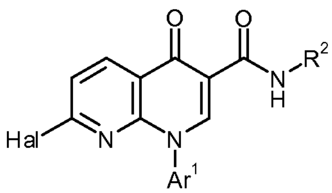
(II),

in welcher $R^2$ und $Ar^1$ die oben angegebenen Bedeutungen haben,
und
Hal für Fluor, Chlor, Brom oder Iod, bevorzugt für Chlor, steht,

mit einer Verbindung der Formel (III)

$R^1$-H (III),

in welcher $R^1$ die oben angegebene Bedeutung hat,
zum erfindungsgemäßen Carbonsäureamid der Formel (I)

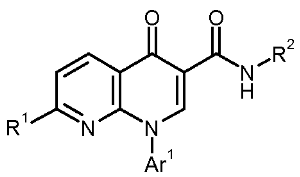
(I),

in welcher $R^1$, $R^2$ und $Ar^1$ die oben angegebenen Bedeutungen haben, umsetzt,
oder
[B] eine Verbindung der Formel (IV)

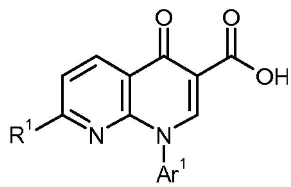

(IV),

in welcher $R^1$ und $Ar^1$ die oben angegebenen Bedeutungen haben,
mit einer Verbindung der Formel (V)

$R^2$-$NH_2$ (V),

in welcher $R^2$ die oben angegebene Bedeutung hat,
zum erfindungsgemäßen Carbonsäureamid der Formel (I)

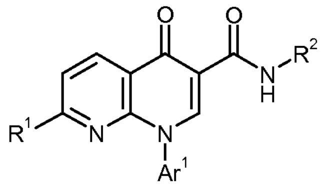

(I),

in welcher $R^1$, $R^2$ und $Ar^1$ die oben angegebenen Bedeutungen haben,
umsetzt,

und gegebenenfalls die so erhaltenen Verbindungen der Formel (I) in ihre Enantiomere und/oder Diastereomere trennt und/oder mit den entsprechenden (i) Lösungsmitteln und/oder (*ii*) Basen oder Säuren in ihre Solvate, Salze und/oder Solvate der Salze überführt.

Die Umsetzung (II) + (III) → (I) kann über eine nukleophile Substitutionsreaktion oder eine Übergangsmetall-vermittelte Kupplungsreaktion erfolgen.

Die nukleophile Substitutionsreaktion wird vorzugsweise in Gegenwart einer Base durchgeführt. Als Basen für den Verfahrensschritt (II) + (III) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Cäsiumcarbonat, Alkali-Alkoholate wie Lithium-, Natrium- oder Kalium-tert.-butylat, Alkalihydride wie Natrium- oder Kaliumhydrid, oder organische Amine wie *N,N*-Diisopropylethylamin (DIPEA), 1,5-Diazabicyclo[4.3.0]non-5-en (DBN) und 1,8-Diazabicyclo[5.4.0]undec-7-en (DBU). Bevorzugt wird *N,N*-Diisopropylethylamin (DIPEA) verwendet. Die Reaktion erfolgt im Allgemeinen in einem Temperaturbereich von 0°C bis +100°C, bevorzugt bei +23°C bis +80°C.

Inerte Lösungsmittel für den Verfahrensschritt (II) + (III) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid (DMF), *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt wird Dimethylformamid (DMF) oder *N*-Methylpyrrolidon (NMP) verwendet.

Die Übergangsmetall-vermittelte Kupplungsreaktion für den Verfahrensschritt (II) + (III) → (I) wird in einer bevorzugten Ausführungsform in Gegenwart eines Palladiumkatalysators durchgeführt. Geeignete Palladium-Katalysatoren sind beispielsweise Palladium(II)acetat, Palladium(II)chlorid, Bis(triphenylphosphin)palladium(II)chlorid, Bis(acetonitril)palladium(II)chlorid, Tetrakis(triphenylphosphin)-palladium(0), Bis(dibenzylidenaceton)palladium(0), Tris(dibenzylidenaceton)dipalladium(0) oder [1,1'-Bis(diphenylphosphino)ferrocen]palladium(II)chlorid, gegebenenfalls in Kombination mit einem geeigneten Phosphin-Liganden wie zum Beispiel Triphenylphosphin, Tri-tert.-butylphosphin, 2-Dicyclohexylphosphino-2',4',6'-triisopropylbiphenyl (X-Phos), 2-Dicyclohexylphosphino-2',6'-dimethoxybiphenyl (S-Phos), 1,2,3,4,5-Pentaphenyl-1'-(di-*tert*.-butylphosphino)ferrocen (Q-Phos), 4,5-Bis(diphenylphosphino)-9,9-dimethylxanthen (Xantphos), 2,2'-Bis(diphenylphosphino)-1,1'-binaphthyl (BINAP), 2-Dicyclohexylphosphino-2'-(*N,N*-dimethylamino)biphenyl oder 2-Di-*tert*.-butylphosphino-2'-(*N,N*-dimethylamino)biphenyl.

Die Palladium-katalysierte Kupplungsreaktion (II) + (III) → (I) wird in der Regel in Gegenwart einer Base durchgeführt. Als solche eignen sich insbesondere Alkalicarbonate wie Natrium-, Kalium- oder Cäsiumcarbonat, Alkaliphosphate wie Natrium- oder Kaliumphosphat, Alkalifluoride wie Kalium- oder Cäsiumfluorid, oder Alkali-tert.-butylate wie Natrium- oder Kalium-tert.-butylat. Die Umsetzung erfolgt in einem inerten Lösungsmittel wie beispielsweise Toluol, 1,2-Dimethoxyethan, Tetrahydrofuran, 1,4-Dioxan, Dimethylsulfoxid (DMSO), *N,N*-Dimethylformamid (DMF), *N,N*-Dimethylacetamid (DMA) oder Mischungen hiervon in einem Temperaturbereich von +80°C bis +200°C, bevorzugt bei +80°C bis +150°C, wobei ein Erhitzen mittels Mikrowellenapparatur von Vorteil sein kann.

Bevorzugt wird für diese Kupplungsreaktion ein Katalysator/Ligand/Base-System bestehend aus Palladium(II)acetat, 4,5-Bis(diphenylphosphino)-9,9-dimethylxanthen (Xantphos) und Cäsium- oder Kaliumcarbonat eingesetzt und 1,4-Dioxan als Lösungsmittel verwendet.

Die Kupplungsreaktion (II) + (III) → (I) kann in einer weiteren bevorzugten Ausführungsform auch mit Hilfe eines Kupfer(I)-Katalysators, wie Kupfer(I)oxid, -bromid oder -iodid, in Gegenwart eines Kupfer-Liganden, wie trans-*N,N'*-Dimethyl-1,2-cyclohexandiamin, 8-Hydroxychinolin oder 1,10-Phenanthrolin, und einer anorganischen oder organischen Carbonat-Base, wie Kalium-, Cäsium- oder Bis(tetraethylammonium)carbonat, durchgeführt werden. Als inerte Lösungsmittel für diese Umsetzung eignen sich insbesondere Toluol, Xylol, 1,4-Dioxan, Acetonitril, Dimethylsulfoxid (DMSO), *N,N*-Dimethylformamid (DMF) oder Gemische hiervon, gegebenenfalls unter Zusatz von Wasser. Bevorzugt wird ein System bestehend aus Kupfer(I)iodid, trans-*N,N'*-Dimethyl-1,2-cyclohexandiamin und Kaliumcarbonat in Dimethylformamid eingesetzt. Die Reaktion erfolgt im Allgemeinen in einem Temperaturbereich von +50°C bis +200°C, bevorzugt bei +60°C bis +150°C.

Die Kupplungsreaktion (IV) + (V) → (I) [Amid-Bildung] kann entweder auf direktem Weg mit Hilfe eines Kondensations- oder Aktivierungsmittels oder über die Zwischenstufe eines aus (IV) erhältlichen Carbonsäurechlorids, Carbonsäureesters oder Carbonsäureimidazolids erfolgen.

Als solche Kondensations- oder Aktivierungsmittel eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), Chlorameisensäureisopropylester oder Chlorameisensäureisobutylester, 1,2-Oxazolium-Verbindungen wie 2-Ethyl-5-phenyl-1,2-oxazolium-3-sulfat oder 2-*tert.*-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-*N,N*,2-trimethylprop-1-en-1-amin, 1,3,5-Triazin-Derivate wie 4-(4,6-Dimethoxy-1,3,5-triazin-2-yl)-4-methylmorpholiniumchlorid, Phosphor-Verbindungen wie *n*-Propanphosphonsäureanhydrid (T3P, PPACA), Cyanophosphonsäurediethylester, Diphenylphosphorylazid (DPPA), Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N,N,N',N'*-tetramethyluroniumtetrafluoroborat (TBTU), *O*-(Benzotriazol-1-yl)-*N,N,N',N'*-tetramethyluronium-hexafluorophosphat (HBTU), *O*-(1*H*-6-Chlorbenzotriazol-1-yl)-1,1,3,3-tetramethyluronium-tetrafluoroborat (TCTU), *O*-(7-Azabenzotriazol-l-yl)-*N,N,N',N'*-tetramethyluronium-hexafluorophosphat (HATU) oder 2-(2-Oxo-1-(2*H*)-pyridyl)-1,1,3,3-tetramethyluronium-tetrafluoroborat (TPTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, N-Methylmorpholin (NMM), *N*-Methylpiperidin (NMP), *N,N*-Diisopropylethylamin (DIPEA), Pyridin oder 4-*N,N*-Dimethylaminopyridin (DMAP). Als Kondensations- oder Aktivierungsmittel bevorzugt eingesetzt wird *O*-(7-Azabenzotriazol-l-yl)-*N,N,N',N'*-tetramethyluronium-hexafluorophosphat (HATU) in Kombination mit *N,N*-Diisopropylethylamin (DIPEA) sowie *n*-Propanphosphonsäureanhydrid (T3P, PPACA) in Kombination mit *N,N*-Diisopropylethylamin (DIPEA).

Die Verbindungen der Formel (II) können dadurch hergestellt werden, dass eine Carbonsäure-Verbindung der Formel (VI)

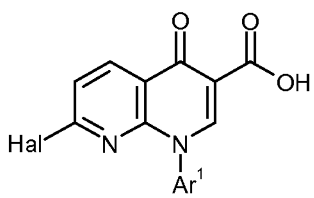 (VI),

in welcher Hal und $Ar^1$ die oben angegebenen Bedeutungen haben,
mit einer Verbindung der Formel (V)

$R^2$-$NH_{22}$ (V),

in welcher $R^2$ die oben angegebene Bedeutung hat,

zum erfindungsgemäßen Carbonsäureamid der Formel (II)

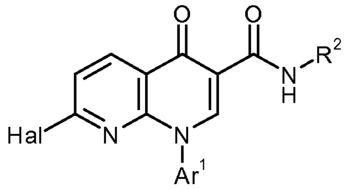

(II),

in welcher Hal, $R^1$, $R^2$ und $Ar^1$ die oben angegebenen Bedeutungen haben,
umgesetzt wird.

Die Kupplungsreaktion (VI) + (V) → (II) [Amid-Bildung] kann entweder auf direktem Weg mit Hilfe eines Kondensations- oder Aktivierungsmittels oder über die Zwischenstufe eines aus (VI) erhältlichen Carbonsäurechlorids, Carbonsäureesters oder Carbonsäureimidazolids analog zu den bereits zur Umsetzung (IV) + (V) → (I) beschriebenen Bedingungen und Reagenzien erfolgen.

Wird bei der Kupplungsreaktion zu (II) HATU als Aktivierungmittel eingesetzt, ist es möglich, dass entweder ein einzelnes, definiertes Produkt der allgemeinen Formel (II) erhalten wird, oder aber ein Gemisch mit einem "HATU-Addukt". Unter einem "HATU-Addukt" wird vorliegend eine Pseudohalogenid-Verbindung bezeichnet, wobei der Substituent Hal in der allgemeinen Formel (II) mit der Gruppe 3*H*-[1,2,3]Triazolo[4,5-b]pyridin-3-ol, auch als 1-Hydroxy-7-azabenzotriazol bezeichnet, ersetzt ist. Ein derartiges Gemisch einer Halogenverbindung der allgemeinen Formel (II) und einem "HATU-Addukt" kann analog zur beschriebenen Umsetzung ebenfalls direkt als Edukt für die Folgereaktion (nach (I) oder (VIII)) eingesetzt werden.

Bei zweistufiger Reaktionsführung über die aus (VI) erhältlichen Carbonsäurechloride oder Carbonsäureimidazolide wird die Kupplung mit der Amin-Komponente (V) in Gegenwart einer üblichen Base durchgeführt, wie beispielsweise Natrium- oder Kaliumcarbonat, Triethylamin, DIPEA, *N*-Methylmorpholin (NMM), *N*-Methylpiperidin (NMP), Pyridin, 2,6-Dimethylpyridin, 4-*N,N*-Dimethylaminopyridin (DMAP), 1,8-Diazabicyclo[5.4.0]undec-7-en (DBU), 1,5-Diazabicyclo[4.3.0]non-5-en (DBN), Natrium- oder Kaliummethanolat, Natrium- oder Kaliumethanolat, Natrium- oder Kalium-tert.-butylat oder Natrium- oder Kaliumhydrid.

Die Carbonsäureimidazolide selbst sind nach bekanntem Verfahren durch Umsetzung von (VI) mit N,N'-Carbonyldiimidazol (CDI) bei erhöhter Temperatur (+60°C bis +150°C) in einem entsprechend höhersiedenden Lösungsmittel wie N,N-Dimethylformamid (DMF) erhältlich. Die Herstellung der Carbonsäurechloride geschieht auf übliche Weise durch Behandlung von (VI) mit Thionylchlorid oder Oxalsäuredichlorid in einem inerten Lösungsmittel wie Dichlormethan oder THF.

Inerte Lösungsmittel für die genannten Kupplungsreaktionen sind - je nach eingesetztem Verfahren - beispielsweise Ether wie Diethylether, Diisopropylether, Methyl-tert.-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Ethylacetat, Acetonitril, Butyronitril, Pyridin, Dimethylsulfoxid (DMSO), *N,N*-Dimethylformamid (DMF), *N,N'*-Dimethylpropylenharnstoff (DMPU) oder *N*-Methylpyrrolidinon (NMP). Auch können Gemische solcher Lösungsmittel eingesetzt werden. Bevorzugt wird *N,N*-Dimethylformamid (DMF) und Dichlormethan (DCM) in Kombination mit Triethylamin verwendet. Die Kupplungen werden im Allgemeinen in einem Temperaturbereich von 0°C bis +130°C, bevorzugt bei +20°C bis +30°C durchgeführt.

Die Verbindungen der Formel (IV) können in Abhängigkeit vom jeweiligen Substitutionsmuster dadurch hergestellt werden, dass man entweder

[C] eine Verbindung der Formel (VII)

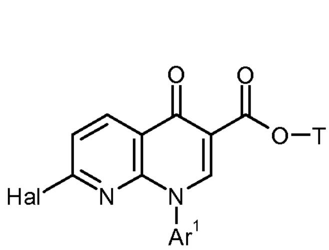

(VII),

in welcher Hal und $Ar^1$ die oben angegebenen Bedeutungen haben,
und

T für ($C_1$-$C_4$)-Alkyl oder Benzyl steht,

in einem ersten Schritt mit einer Verbindung der Formel (III)

$R^1$-H (III),

in welcher $R^1$ die oben angegebene Bedeutung hat,
zu einer Verbindung der Formel (VIII)

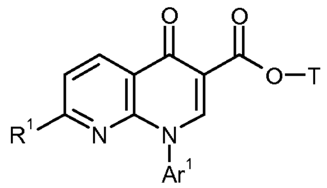

(VIII),

in welcher T, $R^1$ und $Ar^1$ die oben angegebenen Bedeutungen haben,
umsetzt, und gegebenenfalls in einem zweiten Schritt den Ester-Rest T zur erfindungsgemäßen Carbonsäure der Formel (IV) abspaltet,

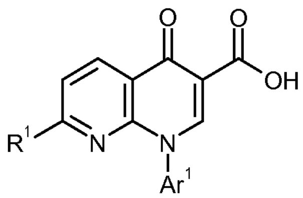

(IV),

in welcher $R^1$ und $Ar^1$ die oben angegebenen Bedeutungen haben,
oder

[D] eine Verbindung der Formel (VI)

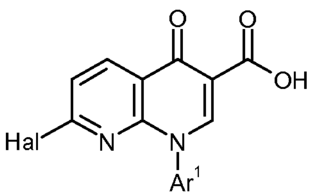

(VI),

in welcher Hal und Ar die oben angegebenen Bedeutungen haben, mit einer Verbindung der Formel (III)

$R^1$-H (III),

in welcher $R^1$ die oben angegebene Bedeutung hat,
zur erfindungsgemäßen Carbonsäure der Formel (IV),

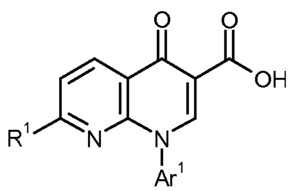

(IV),

in welcher $R^1$ und $Ar^1$ die oben angegebenen Bedeutungen haben,

umsetzt.

Die Umsetzung (VII) + (III) → (VIII) [Weg C] oder die Umsetzung (VI) + (III) → (IV) [Weg D] kann jeweils über eine nukleophile Substitutionsreaktion oder eine Übergangsmetall-vermittelte Kupplungsreaktion analog zu den bereits zur Umsetzung (II) + (III) → (I) beschriebenen Bedingungen und Reagenzien erfolgen.

In einer bevorzugten Ausführungsform wird die Umsetzung nach Weg C als nukleophile Substitutionsreaktion in Gegenwart einer Base durchgeführt, bevorzugt wird *N,N*-Diisopropylethylamin (DIPEA) verwendet. Bevorzugt wird Dimethylformamid (DMF), *N*-Methylpyrrolidon (NMP) oder Acetonitril als Lösungsmittel verwendet.

In einer bevorzugten Ausführungsform wird die Umsetzung nach Weg D als Übergangsmetall-vermittelte Kupplungsreaktion in Gegenwart eines geeigneten Palladiumkatalysators durchgeführt. Bevorzugt wird ein System bestehend aus Palladium(II)-acetat in Kombination mit 4,5-Bis(diphenylphosphino)-9,9-dimethylxanthen (Xantphos), Cäsium- oder Kaliumcarbonat und 1,4-Dioxan als Lösungsmittel verwendet.

Die Abspaltung der Ester-Gruppe T im Verfahrensschritt (VIII) → (IV) wird nach üblichen Methoden durchgeführt, indem man den Ester in einem inerten Lösungsmittel mit einer Säure oder Base behandelt, wobei bei letzterer Variante das zunächst entstehende Salz der Carbonsäure durch nachfolgende Behandlung mit Säure in die freie Carbonsäure überführt wird. Im Falle der tert.-Butylester erfolgt die Esterspaltung vorzugsweise mit einer Säure. Benzylester können alternativ auch durch Hydrierung (Hydrogenolyse) in Gegenwart eines geeigneten Katalysators, wie beispielsweise Palladium auf Aktivkohle, abgespalten werden.

Als Lösungsmittel eignen sich für diese Reaktionen Wasser und die für eine Esterspaltung üblichen organischen Lösungsmittel. Hierzu zählen insbesondere Alkohole wie Methanol, Ethanol, *n*-Propanol, Isopropanol, *n*-Butanol oder *tert.*-Butanol, Ether wie Diethylether, Tetrahydrofuran, 1,4-Dioxan oder 1,2-Dimethoxyethan, oder andere Lösungsmittel wie Dichlormethan, Acetonitril, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Tetrahydrofuran verwendet.

Als Basen sind die für eine Hydrolyse-Reaktion üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt wird wässrige Salzsäure (18 proz.) in einem Wasser/Tetrahydrofuran-Gemisch eingesetzt.

Die Esterspaltung wird in der Regel in einem Temperaturbereich von -20°C bis +100°C, bevorzugt bei 23°C bis +120°C durchgeführt.

Die Verbindungen der Formel (VI) sowie der Formel (VIII) können in Abhängigkeit vom jeweiligen Substitutionsmuster dadurch hergestellt werden, dass man in Analogie zu bekannten Verfahren (s. z.B. EP 0607825 A1, S. 25-26) ein 2,6-Dichlornicotinoyl-Acrylsäureester-Derivat der Formel (IX)

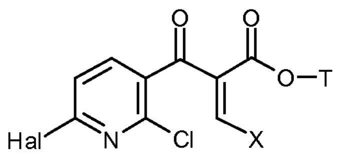

(IX),

in welcher Hal und T die oben angegebenen Bedeutungen haben,
und

X für eine Abgangsgruppe wie Dimethylamino, Methoxy oder Ethoxy steht, sowie

in einer ersten Stufe, bevorzugt in Gegenwart einer geeigneten Base, mit einer Aminopyridin-Verbindung der Formel (X)

$Ar^1$-$NH_2$ (X),

in welcher $Ar^1$ die oben angegebene Bedeutungen hat, umsetzt,
und dann in einem zweiten Schritt in Gegenwart einer geeigneten Base zur Ester-Verbindung der Formel

(VII)

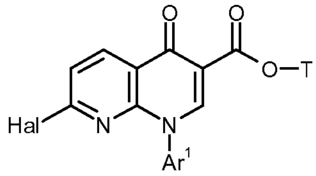

(VII),

in welcher Hal, Ar[1] und T die oben angegebene Bedeutung haben,
umsetzt, sowie dann gegebenenfalls unter Hydrolyse-Bedingungen in einem weiteren Schritt die Ester-Verbindung (VII) in die Carbonsäure-Verbindung (VI)

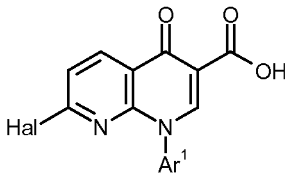

(VI),

in welcher Hal und Ar[1] die oben angegebenen Bedeutungen haben,
unter den in der Literatur bekannten Reaktionsbedingungen überführt.

Die Verbindungen der Formel (IX) sind literaturbekannt (siehe z.B. EP 0607825 A1) oder können in Analogie zu literaturbekannten Verfahren hergestellt werden.

Die Verbindungen der Formel (III), (V) und (X) sind kommerziell erhältlich oder als solche in der Literatur beschrieben, oder sie können auf für den Fachmann offenkundigem Wege in Analogie zu in der Literatur publizierten Metho-den hergestellt werden. Zahlreiche ausführliche Vorschriften sowie Literaturangaben zur Her-stellung der jeweiligen Ausgangsmaterialien befinden sich auch im Experimentellen Teil im Abschnitt zur Herstellung der Ausgangsverbindungen und Intermediate.

Die Auftrennung von Stereoisomeren (Enantio- und/oder Diastereomeren) der erfindungsgemäßen Verbindungen der Formel (I) läßt sich nach üblichen, dem Fachmann geläufigen Methoden erreichen. Vorzugsweise werden hierfür chromatographische Verfahren an achiralen bzw. chiralen Trennphasen angewandt.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Intermediate (II), (IV), oder (VIII) erfolgen, welche dann in separierter Form gemäß der zuvor beschriebenen Reaktionssequenz weiter umgesetzt werden. Für eine solche Auftrennung der Stereoisomere von Intermediaten werden gleichfalls bevorzugt chromatographische Verfahren an achiralen bzw. chiralen Trennphasen angewandt. Alternativ kann auch eine Trennung über diastereomere Salze der Carbonsäuren der Formel (IV) mit chiralen Amin-Basen erfolgen.

Die Herstellung der erfindungsgemäßen Verbindungen kann durch die folgenden Reaktions-schemata beispielhaft veranschaulicht werden:

## Schema 1

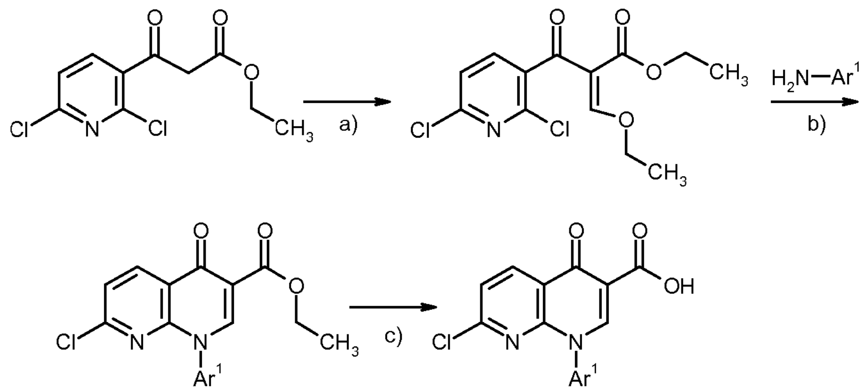


[a): Triethylorthoformiat, Acetanhydrid ; b): DIPEA, DCM, dann $K_2CO_3$; c): 18-proz. Salzsäure, THF, Wasser].

**Schema 2**

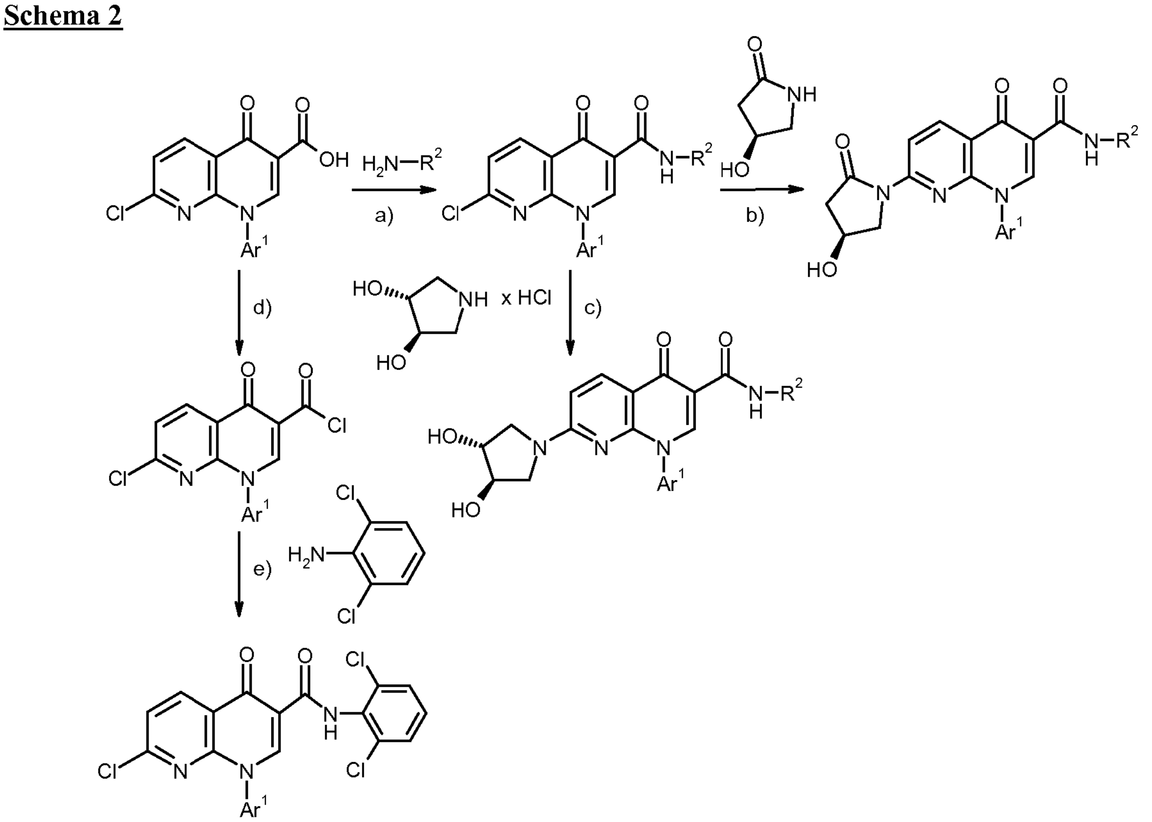


[a): HATU, DIPEA, DMF oder T3P, DIPEA, EtOAc; b): $Pd(OAc)_2$, Xantphos, $K_2CO_3$, 1,4-Dioxan; c): DIPEA, DMF; d): $(COCl)_2$, kat. DMF, THF; e): NaH, DMF oder Triethylamin, DCM].

**Schema 3**

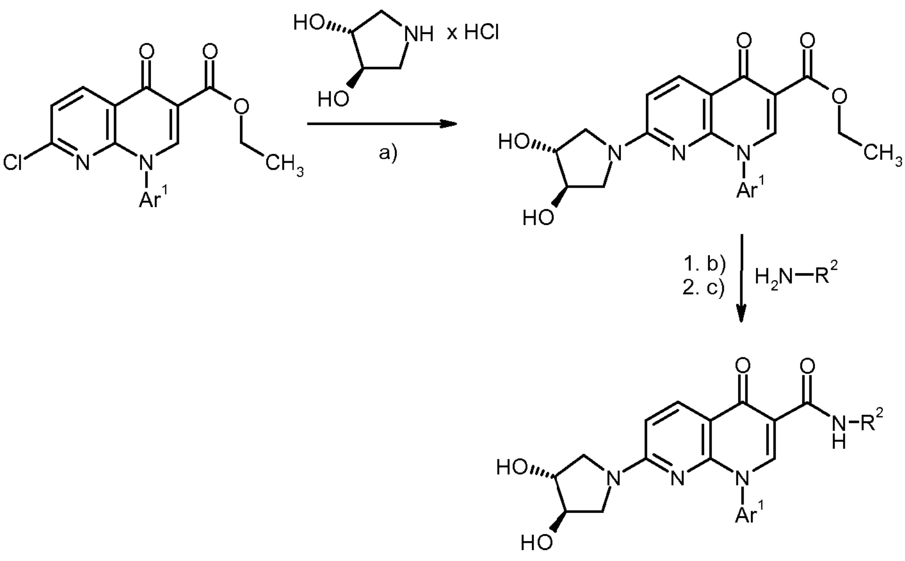


[a): DIPEA, DMF; b): wässr. LiOH, THF oder 18-proz. Salzsäure, THF, Wasser; c): HATU, DIPEA, DMF, RT.]

**Schema 4**

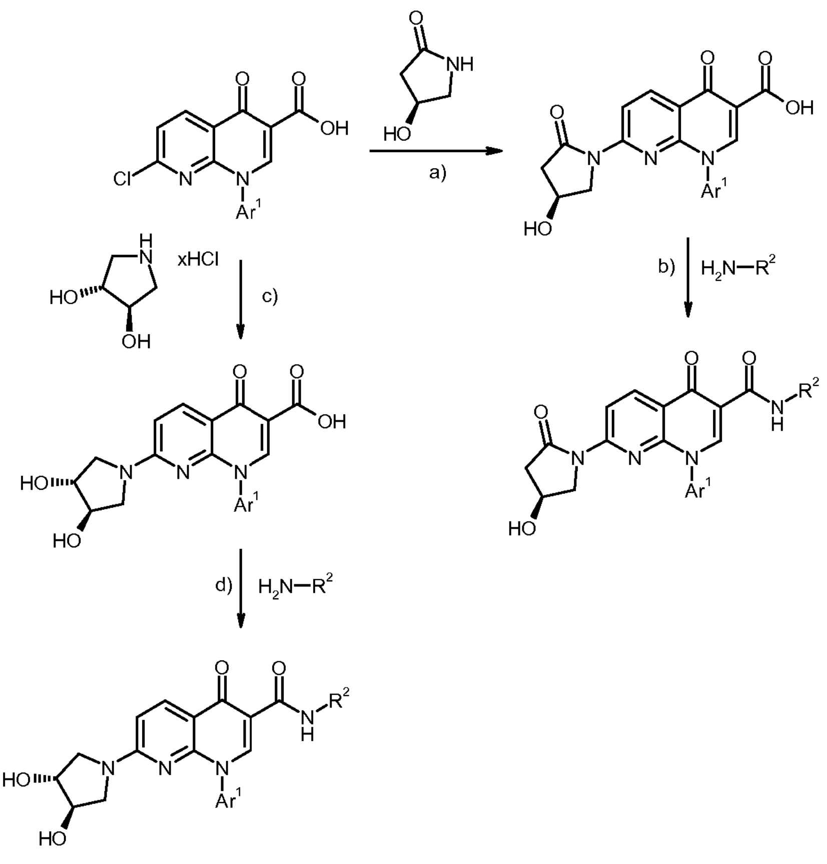


[a): $Pd(OAc)_2$, Xantphos, $K_2CO_3$, 1,4-Dioxan; b) HATU, DIPEA, DMF c) DIPEA, DMF.]

Weitere erfindungsgemäße Verbindungen der Formel (I) können, falls zweckmäßig, auch durch Umwandlungen von funktionellen Gruppen einzelner Reste und Substituenten, insbesondere den unter $R^1$ und $R^2$ aufgeführten, hergestellt werden, wobei von anderen, nach obigen Verfahren erhaltenen Verbindungen der Formel (I) oder deren Vorstufen ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen, Herstellungs- und Additionsreaktionen von Metallorganylen (z.B. Grignard-Verbindungen oder Lithiumorganylen), Oxidations- und Reduktionsreaktionen, Hydrierung, Halogenierung (z.B. Fluorierung, Bromierung), Dehalogenierung, Aminierung, Alkylierung und Acylierung, die Bildung von Carbonsäureestern, Carbonsäureamiden und Sulfonamiden, die Esterspaltung und -hydrolyse sowie die Einführung und Entfernung temporärer Schutzgruppen.

Die Erfindung betrifft in einem weiteren Aspekt Intermediate der allgemeinen Formel (II)

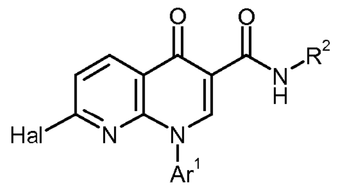

(II),

in welcher $R^2$ und $Ar^1$ die oben für Verbindungen der Formel (I) angegebenen Bedeutungen haben und

Hal für Fluor, Chlor, Brom oder Iod, bevorzugt für Chlor, steht.

Die Erfindung betrifft in einem weiteren Aspekt Intermediate der allgemeinen Formel (IV)

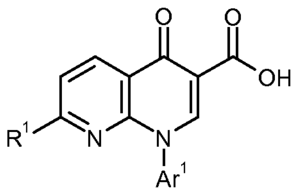

(IV),

in welcher $R^1$ und Ar die oben für Verbindungen der Formel (I) angegebenen Bedeutungen haben.

Die Erfindung betrifft in einem weiteren Aspekt die Verwendung einer Verbindung der allgemeinen Formel (II)

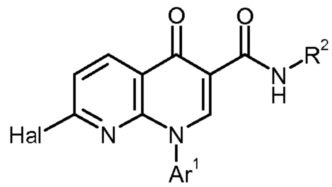

(II),

in welcher $R^2$ und $Ar^1$ die oben für Verbindungen der Formel (I) angegebenen Bedeutungen haben und

Hal für Fluor, Chlor, Brom oder Iod, bevorzugt für Chlor, steht.

oder
einer Verbindung der allgemeinen Formel (IV)

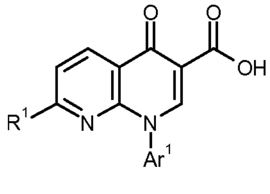

(IV),

in welcher $R^1$ und $Ar^1$ die oben für Verbindungen der Formel (I) angegebenen Bedeutungen haben,
zur Herstellung einer Verbindung der allgemeinen Formel (I) wie oben definiert.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren. Die erfindungsgemäßen Verbindungen besitzen wertvolle pharmakologische Eigenschaften und können zur Behandlung und/ oder Prophylaxe von Erkrankungen bei Menschen und Tieren verwendet werden.

Die erfindungsgemäßen Verbindungen stellen positive allosterische Modulatoren des muskarinergen M2-Rezeptors dar und eignen sich daher zur Behandlung und/oder Prävention von Erkrankungen und pathologischen Prozessen, insbesondere kardiovaskulärer Erkrankungen und/oder Nierenerkrankungen, bei denen im Zuge einer Fehlregulation des autonomen Nervensystems bzw. eines Ungleichgewichtes zwischen der Aktivität des sympathischen und parasympathischen Teils des autonomen Nervensystems der M2-Rezeptor involviert ist.

Gegenstand der vorliegenden Erfindung sind positive allosterische Modulatoren des muskarinergen M2-Rezeptors. Allosterische Modulatoren weisen gegenüber klassischen, orthosterischen Liganden deutliche Unterschiede auf. Der Effekt eines allosterischen Modulators ist selbstlimitierend, wenn er in hohen Konzentrationen die Bindung des Agonisten stabilisiert. Darüber hinaus kann sich die Wirkung eines allosterischen Modulators nur in der Anwesenheit des endogenen Liganden entfalten. Der allosterische Modulator selbst hat keinen direkten Einfluss auf die Rezeptoraktivierung. Daraus ergibt sich eine räumliche und zeitliche Spezifität der allosterischen Wirkung. Die als Kooperativität bezeichnete gegenseitige Beeinflussung von allosterischen und orthosterischen Liganden hinsichtlich Affinität und intrinsischer Aktivität wird von den beiden Liganden bestimmt. Im Falle eines positiven allosterischen Modulators werden die Effekte des orthosterischen Liganden verstärkt (positive Kooperativität). Aufgrund ihrer Fähigkeit, Rezeptorkonformationen in Gegenwart eines orthosterischen Liganden zu modulieren, können allosterische Liganden eine Feinabstimmung pharmakologischer Effekte hervorrufen.

Im Sinne der vorliegenden Erfindung sind unter Erkrankungen des Herzkreislauf-Systems beziehungsweise kardiovaskulären Erkrankungen beispielsweise die folgenden Erkrankungen zu verstehen: akute und chronische Herzinsuffizienz, arterielle Hypertonie, koronare Herzerkrankung, stabile und instabile Angina pectoris, myokardiale Ischämie, Myokardinfarkt, Schock, Atherosklerose, Herzhypertrophie, Herzfibrose, atriale und ventrikuläre Arrhythmien, Tachykardie, transitorische und ischämische Attacken, Hirnschlag, Präeklampsie, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädi-gungen (Vasculitis), Reperfusionsschäden, arterielle und venöse Thrombosen, Mikroalbuminurie, Herzmuskelschwäche, endotheliale Dysfunktion, periphere und kardiale Gefäßerkrankungen, periphere Durchblutungsstörungen, Herzinsuffizienz-bedingtes Ödem, erhöhte Spiegel von Fibrinogen und von LDL geringer Dichte sowie erhöhte Konzentrationen von Plasminogenaktivator-Inhibitor 1 (PAI 1).

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, Herzinsuffizienz bei erhaltener systolischer Pumpfunktion (HFpEF), diastolische Herzinsuffizienz, sowie Herzinsuffizienz bei reduzierter systolischer Pumpfunktion (HfrEF), systolische Herzinsuffizienz.

Im Sinne der vorliegenden Erfindung umfasst der Begriff atriale und ventrikuläre Arrhythmien auch spezifischere oder verwandte Krankheitsformen wie: Vorhofflimmern, paroxysmales Vorhofflimmern intermittierendes Vorhofflimmern, permanentes Vorhofflimmern, Vorhofflattern, Sinusarrhythmie, Sinustachykardie, passive Heterotopie, aktive Heterotopie, Ersatzsystolen, Extrasystolen, Reizleitungsstörungen, Sick-sinus-Syndrom, hypersensitiver Karotissinus, Tachykardien, AV-Knoten Reentrytachykardie, atriventrikuläre Reentrytachykardie, WPW-Syndrom (Wolff-Parkinson-White), Mahaim-Tachykardie, verborgene akzessorische Leitungsbahn, permanente junktionale Re-entrytachykardie, fokale atriale Tachykardie, junktional ektope Tachykardie, atriale Reentrytachykardie, Kammertachykardie, Kammerflattern, Kammerflimmern, plötzlicher Herztod.

Im Sinne der vorliegenden Erfindung umfasst der Begriff koronare Herzerkrankung auch spezifischere oder verwandte Krankheitsformen wie: ischämische Herzkrankheit, stabile Angina pectoris, akutes Koronarsyndrom, instabile Angina pectoris, NSTEMI (nicht-ST-Streckenhebungsinfarkt), STEMI (ST-Streckenhebungsinfarkt), ischämische Herzmuskelschädigung, Herzrhythmusstörungen, und Myokardinfarkt.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem

Nierenversagen.

Im Sinne der vorliegenden Erfindung umfasst der Begriff akute Niereninsuffizienz akute Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytisch-urämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplant-atabstoßung, Immunkomplex-induzierte Nierenerkrankungen, tubuläre Dilatation, Hyper-phosphatämie und/ oder akute Nierenerkrankungen, die durch die Notwendigkeit zur Dialyse charakterisiert werden können, sowie bei Teilresektionen der Niere, Dehydratation durch forcierte Diurese, unkontrolliertem Blutdruckanstieg mit maligner Hypertonie, Harnwegs-obstruktion und -infekt und Amyloidose sowie Systemerkrankungen mit glomerulärer Beteiligung, wie rheumatologisch-immunologische Systemerkrankungen, wie beispielsweise Lupus erythematodes, Nierenarterienthrombose, Nierenvenenthrombose, Analgetikanephropathie und renal-tubuläre Azidose, sowie Röntgen-Kontrastmittel- sowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membrano-proliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephro-pathie, Pyelonephritis, Nierenzysten, Nephrosklerose, hypertensive Nephrosklerose und nephrotisches Syndrom, welche diagnostisch beispielsweise durch abnorm verminderte Kreatinin- und/ oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/ oder die Notwendigkeit zur Dialyse charakterisiert werden können, sowie bei Nierenzellkarzinomen, nach Teilresektionen der Niere, Dehydratation durch forcierte Diurese, unkontrollierter Blutdruckanstieg mit maligner Hypertonie, Harnwcgsobstruktion und -infekt und Amyloidose sowie Systemerkrankungen mit glomerulärer Beteiligung, wic rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renal-tubuläre Azidose zu verstehen. Weiterhin Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/ oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von pulmonaler arterieller Hypertonie (PAH) und anderen Formen der pulmonalen Hypertonie (PH), der chronisch-obstruktiven Lungenerkrankung (COPD), des akuten Atemwegssyndroms (ARDS), der akuten Lungenschädigung (ALI), der alpha-1-Antitrypsin-Defizienz (AATD), der Lungenfibrose, des Lungenemphysems (z.B. durch Zigarettenrauch induziertes Lungenemphysem), der zystischen Fibrose (CF), von akutem Koronarsyndrom (ACS), Herzmuskelentzündungen (Myokarditis) und anderen autoimmunen Herzerkrankungen (Perikarditis, Endokarditis, Valvolitis, Aortitis, Kardiomyopathien), kardiogenem Schock, Aneurysmen, Sepsis (SIRS), multiplem Organversagen (MODS, MOF), entzündlichen Erkrankungen der Niere, chronischen Darmentzündungen (IBD, Crohn’s Disease, UC), Pankreatitis, Peritonitis, rheumatoiden Erkrankungen, entzündlichen Hauterkrankungen sowie entzündlichen Augenerkrankungen.

Die erfindungsgemäßen Verbindungen können weiterhin verwendet werden zur Behandlung und/ oder Prophylaxe von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, idiopathischer interstitieller Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lemleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre

Demenz, Schädel-Hirn-Trauma, Schlaganfall, Demenz, die nach Schlaganfällen auftritt ("post stroke dementia"), posttraumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, bipolarer Störung, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Behandlung und/oder Prophylaxe von urologischen Erkrankungen wie: Harninkontinenz, insbesondere Streßinkontinenz, Dranginkontinenz, Reflexinkontinenz und Überlaufinkontinenz, Detrusor-hyperaktivität, neurogene Detrusorhyperaktivität, idiopathische Detrusorhyperaktivität, benigne Prostatahyperplasie (BPH-Syndrom), Symptome des unteren Harntraktes (LUTS = lower urinary tract symptoms).

Die erfindungsgemäßen Verbindungen eignen sich weiterhin zur Behandlung und/oder Prävention von gastroenterologischen Erkrankungen, wie Ösophaguskrankheiten, Erbrechen, Achalasie, gastroösophageale Refluxkranheit, Magenkrankheiten, wie Gastritis, Darmerkrankungen, wie Diarrhö, Ostipation, Malassimilationssyndrom, Gallensäureverlustsyndrom, Morbus Crohn, Colitis ulcerosa, mikroskopische Colitis, und Reizdarmsyndrom.

Die erfindungsgemäßen Verbindungen eignen sich weiterhin zur Behandlung und/oder Prävention von Schmerzzuständen, wie beispielsweise Menstruationstörungen, Dysmenorrhoe, Endometriose, Frühgeburt, Tokolyse.

Aufgrund ihres biochemischen und pharmakologischen Eigenschaftsprofils eignen sich die erfindungsgemäßen Verbindungen insbesondere zur Behandlung und/oder Prävention von Herzinsuffizienz, koronarer Herzerkrankung, atrialen und ventrikulären Arrhythmien, Niereninsuffizienz und Nephropathien.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Die erfindungsgemäßen Verbindungen eignen sich weiterhin zur Behandlung und/oder Prävention von ophthalmologischen Erkrankungen, wie beispielsweise von Glaukom, altersbedingter Makuladegeneration (AMD), trockener (nicht-exsudativer) AMD, feuchter (exsudativer, neovaskulärer) AMD, choroidaler Neovaskularisation (CNV), diabetischer Retinopathie, atrophischen Veränderungen des retinalen Pigmentepithels (RPE), hypertrophischen Veränderungen des retinalen Pigmentepithels, Makulaödem, diabetischem Makulaödem, Netzhautvenenverschluss, choroidalem Netzhautvenenverschluss, Makulaödem auf grund von Netzhautvenenverschluss, Angiogenese an der Vorderseite des Auges wie kornealer Angiogenese beispielsweise nach Keratitis, Hornhauttransplantation oder Keratoplastik, kornealer Angiogenese auf grund von Hypoxie (durch extensives Tragen von Kontaktlinsen), Pterygium conjunctivae, subretinalem Ödem und intraretinalem Ödem. Des weiteren eignen sich die erfindungsgemäßen Verbindungen zur Behandlung und/oder Prävention von erhöhtem und hohem Augeninnendruck als Folge von traumatischem Hyphaema, periorbitalem Ödem, postoperativer viskoelastischer Retention oder intraokularer Entzündung.

Außerdem sind die erfindungsgemäßen Verbindungen zur Behandlung und/ oder Prophylaxe von Hepatitis, Neoplasma, Osteoporose, Glaukom und Gastroparese geeignet.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerz, Neuralgien und Tinnitus eingesetzt werden.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:

- den Blutdruck senkende Wirkstoffe, beispielhaft und vorzugsweise aus der Gruppe der Calcium-Antagonisten, Angiotensin AII-Antagonisten, ACE-Inhibitoren, NEP-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
- antiarrhythmische Wirkstoffe, wie beispielsweise und vorzugsweise Natriumkanalblocker, Betarezeptorenblocker, Kaliumkanalblocker, Kalziumantagonisten, If-Kanalblocker, Digitalis, Parasympatholytika (Vagoliytika), Sympathomimetika und andere Antiarrhythmika wie Adenosin, Adenosinrezeptor Agonisten sowie Vernakalant.
- positiv-inotrope Wirkstoffe, wie z.B. Herzglykoside (Dogoxin), beta-adrenerge und dopaminerge Agonisten, wie Isoprenalin, Adrenalin, Noradrenalin, Dopamin oder Dobutamin;
- Vasopressin-Rezeptor-Antagonisten, wie beispielsweise und vorzugsweise Conivaptan, Tolvaptan, Lixivaptan, Mozavaptan, Satavaptan, SR-121463, RWJ 676070 oder BAY 86-8050, sowie die in WO 2010/105770, WO2011/104322 und WO 2016/071212 beschriebenen Verbindungen;
- Natriuretische Peptide, wie z.B. atriales natriuretisches Peptid (ANP), natriuretisches Peptid Typ B (BNP, Nesiritid) natriuretisches Peptid Typ C (CNP) oder Urodilatin;
- Aktivatoren des kardialen Myosins, wie z.B. Omecamtiv Mecarbil (CK-1827452);
- Calcium-Sensitizer, wie z.B. Levosimendan
- den Energiestoffwechsel des Herzens beeinflussende Verbindungen, wie beispielhaft und vorzugsweise Etomoxir, Dichloracetat, Ranolazine oder Trimetazidine, volle oder partielle Adenosin A1 Receptor Agonisten wie GS-9667 (früher bekannt als CVT-3619), Capadenoson, Neladenoson und BAY 1067197;
- Verbindungen die die Herzfrequenz beinflussen, wie z.B. Ivabradin
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, Udenafil, Desantafil, Avanafil, Mirodenafil, Lodenafil or PF-00489791;
- antithrombotisch wirkende Mittel, beispielhaft und vorzugsweise aus der Gruppe der Thrombozytenaggregationshemmer, der Antikoagulantien oder der profibrinolytischen Substanzen;
- bronchodilatorisch wirkende Mittel, beispielhaft und vorzugsweise aus der Gruppe der beta-adrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason; sowie die nichtsteroidale anti-inflammatorische Wirkstoffe (NSAIDs),

wie insbesondere Acetylsalicylsäure (Aspirin), Ibuprofen and Naproxen, 5-Aminosalicylic Säure-Derivate, Leukotrien-Antagonists , TNF-alpha-Inhibitoren und Chemokin-Receptor Antagonisten, wie CCR1, 2 und/or 5 Inhibitoren;

- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-$\delta$-Agonisten, Cholesterin-Absorptionshemmer, Lipase-Inhibitoren, polymeren Gallensäureadsorber, Gallensäure-Reabsorptionshemmer und Lipoprotein(a)-Antagonisten.
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Chymase, Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12) und Neutrophil-Elastase (HNE), wie z.B. Sivelestat oder DX-890;
- Verbindungen, die die Bindung von Serotonin an dessen Rezeptor blockieren, beispielhaft und vorzugsweise Antagonisten des 5-$HT_{2b}$-Rezeptors;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- NO-unabhängige, jedoch Häm-abhängige Stimulatoren der löslichen Guanylatcyclase, wie insbesondere die in WO 00/06568, WO 00/06569, WO 02/42301, WO 03/095451, WO 2011/147809, WO 2012/004258, WO 2012/028647 und WO 2012/059549 beschriebenen Verbindungen;
- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
- Verbindungen, die die Synthese von cGMP steigern, wie beispielsweise sGC Modulatoren, wie beispielhaft und vorzugsweise Riociguat, Cinaciguat, Vericiguat oder BAY 1101042
- Prostacyclin-Analoga, wie beispielhaft und vorzugsweise Iloprost, Beraprost, Treprostinil oder Epoprostenol;
- Verbindungen, die die lösliche Epoxidhydrolase (sEH) inhibieren, wie beispielsweise *N,N'*-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff.
- den Glucosestoffwechsel verändernde Wirkstoffe, wie beispielsweise Insuline, Biguanide, Thiazolidinedione,Sulfonylharnstoffe, Acarbose, DPP4 Inhibitoren, GLP-1 Analoga oder SGLT-1 Inhibitor.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafarnib, Nintedanib, Dasatinib, Nilotinib, Bosutinib, Axitinib, Telatinib, Imatinib, Brivanib, Pazopanib, Pegaptinib, Pelitinib, Semaxanib, Sorafenib, Regorafenib, Sunitinib, Tandutinib, Tipifarnib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Dabigatran, Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Edoxaban (DU-176b), Apixaban, Otamixaban, Fidexaban, Razaxaban, Fondaparinux, Idraparinux, PMD-3112, Y*N*-150, KFA-1982, EMD-503982, MC*N*-17, mLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika ver-standen.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, Irbesartan, Olmesartan, Eprosartan oder Azilsartan oder einem dualen Angiotensin AII-Antagonisten/NEP-Inhibitor, wie beispielsweise und vorzugsweise Entresto (LCZ696, Valsartan/Sacubitril), verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan, Avosentan, Macitentan, Atrasentan oder Sitaxsentan, verabreicht.

Bei einer bevorzugten Ausführungsform der Erfindung werden die erfindungsgemäßen Verbindungen in Kombination mit einem TGFbeta Antagonisten, wie beispielhaft und vorzugsweise Pirfenidone oder Fresolimumab, verabreicht.

Bei einer bevorzugten Ausführungsform der Erfindung werden die erfindungsgemäßen Verbindungen in Kombination mit einem TNFalpha Antagonisten, wie beispielhaft und vorzugsweise Adalimumab, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit HIF-PH Inhibitoren, wie beispielhaft und vorzugsweise Molidustat oder Roxadustat verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, Finerenon verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-$\delta$-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), Anacetrapib, JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-$\delta$-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung werden die erfindungsgemäßen Verbindungen in Kombination mit sGC Modulatoren, wie beispielhaft und vorzugsweise Riociguat, Cinaciguat, Vericiguat oder BAY 1101042 verabreicht.

Bei einer bevorzugten Ausführungsform der Erfindung werden die erfindungsgemäßen Verbindungen in Kombination mit einem den Glucosestoffwechsel verändernden Wirkstoff, wie beispielhaft und vorzugsweise Insulin, einem Sulfonylharnstoff, Acarbose, DPP4 Inhibitoren, GLP-1 Analoga oder SGLT-1 Inhibitor, verabreicht.

Besonders bevorzugt sind Kombinationen der erfindungsgemäßen Verbindungen mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten,

Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

## A. BEISPIELE

**Abkürzungen und Akronyme:**

| | |
|---|---|
| AAV | allgemeine Arbeitsvorschrift |
| abs. | absolut |
| aq. | wässrig, wässrige Lösung |
| br. | breit (bei NMR-Signal) |
| Bsp. | Beispiel |
| Bu | Butyl |
| c | Konzentration |
| ca. | circa, ungefähr |
| cat. | katalytisch |
| CDI | Carbonyldiimidazol |
| CI | chemische Ionisation (bei MS) |
| d | Dublett (bei NMR) |
| d | Tag(e) |
| DCM | Dichlormethan |
| dd | Dublett von Dublett (bei NMR) |
| de | Diastereomerenüberschuss |
| DEA | Diethylamin |
| dest. | destilliert |
| DIPEA | N,N - Diisopropylethylamin |

(fortgesetzt)

| | |
|---|---|
| DMAP | 4-N,N-Dimethylaminopyridin |
| DMF | N,N - Dimethylformamid |
| DMSO | Dimethylsulfoxid |
| dt | Dublett von Triplett (bei NMR) |
| d. Th. | der Theorie (bei chemischer Ausbeute) |
| ee | Enantiomerenüberschuss |
| EI | Elektronenstoß-Ionisation (bei MS) |
| ent | enantiomerenrein, Enantiomer |
| Äq. | Äquivalent(e) |
| ESI | Elektrospray-Ionisation (bei MS) |
| Et | Ethyl |
| GC | Gaschromatographie |
| GC/MS | Gaschromatographie-gekoppelte Massenspektrometrie |
| h | Stunde(n) |
| HATU | O-(7-Azabenzotriazol-1-yl)-N,N,N',N'-tetramethyluroniumhexafluorophosphat; |
| HPLC | Hochdruck-, Hochleistungsflüssigchromatographie |
| konz. | konzentriert (bei Lösung) |
| LC | Flüssigchromatographie |
| LC/MS | Flüssigchromatographie-gekoppelte Massenspektrometrie |
| Lit. | Literatur(stelle) |
| m | Multiplett (bei NMR) |
| M | molar (bei Lösung) |
| Me | Methyl |
| min | Minute(n) |
| MS | Massenspektrometrie |
| NMR | Kernresonanzspektrometrie |
| q (oder quart) | Quartett (bei NMR) |
| qd | Quartett von Dublett (bei NMR) |
| quant. | quantitativ (bei chemischer Ausbeute) |
| quintt | Quinttett (bei NMR) |
| rac | racemisch, Racemat |
| RP | reverse phase (Umkehrphase, bei HPLC) |
| RT | Raumtemp eratur |
| Rt | Retentionszeit (bei HPLC, LC/MS) |
| s | Singulett (bei NMR) |
| sept | Septett (bei NMR) |
| SFC | superkritische Flüssigchromatographie |
| t | Triplett (bei NMR) |
| tBu | tert.-Butyl |

(fortgesetzt)

| | |
|---|---|
| td | Triplett von Dublett (bei NMR) |
| THF | T etrahydro furan |
| TFA | Trifluoressigsäure |
| UV | Ultraviolett - Spektrometrie |
| vgl. | vergleiche |
| v/v | Volumen zu Volumen-Verhältnis (einer Lösung) |
| Xantphos | 9,9-Dimethyl-4,5-bis-(diphenylphosphino)-xanthen |
| zus. | zusammen |

## HPLC- und LC/MS-Methoden:

Methode 1:

Instrument: Waters ACQUITY SQD UPLC System; Säule: Waters Acquity UPLC HSS T3 1.8 μ 50 x 1 mm; Eluent A: 1l Wasser + 0.25 ml 99%ige Ameisensäure , Eluent B: 1l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

Methode 2:

Gerätetyp MS: Thermo Scientific FT-MS; Gerätetyp UHPLC+: Thermo Scientific UltiMate 3000; Säule: Waters, HSST3, 2.1 x 75 mm, C18 1.8 μm; Eluent A: 1l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.

Methode 3:

Instrument: Waters ACQUITY SQD UPLC System; Säule: Waters Acquity UPLC HSS T3 1.8 μ 50 x 1 mm; Eluent A: 1l Wasser + 0.25 ml 99%ige Ameisensäure , Eluent B: 1l Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

Methode 4:

Instrument: Thermo DFS, Trace GC Ultra; Säule: Restek RTX-35, 15 m x 200 μm x 0.33 μm; konstanter Fluss mit Helium: 1.20 ml/min; Ofen: 60°C; Inlet: 220°C; Gradient: 60°C, 30°C/min → 300°C (3.33 min halten).

Methode 5:

Instrument MS: Waters (Micromass) Quattro Micro; Instrument Waters UPLC Acquity; Säule : Waters BEH C18 1.7 μ 50 x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A → 2.51 min 10% A → 3.0 min 10% A; Ofen: 40°C; Fluss: 0.5 ml/min; UV-Detektion: 210 nm.

## Weitere Angaben:

Die Prozentangaben in den folgenden Beispiel- und Testbeschreibungen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Bei Aufreinigungen von erfindungsgemäßen Verbindungen per präparativer HPLC nach den beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen ausreichend basische bzw. säure Funktionalitäten enthalten. Ein solches Salz kann durch verschiedene dem Fachmann bekannte Methoden in die entsprechende

freie Base bzw. Säure überführt werden.

Reinheitsangaben beziehen sich in der Regel auf entsprechende Peak-Integrationen im LC/MS-Chromatogramm, können aber zusätzlich auch unter Zuhilfenahme des 1H-NMR-Spektrums ermittelt worden sein. Wenn keine Reinheit angegeben ist, handelt es sich in der Regel um eine 100%-Reinheit laut automatischer Peak-Integration im LC/MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

Die nachfolgenden Beschreibungen der Kopplungsmuster von 1H-NMR-Signalen wurden teilweise direkt den Vorschlägen des ACD SpecManagers (ACD/Labs Release 12.00, Product version 12.5) entnommen und nicht notwendigerweise streng hinterfragt. Teilweise wurden die Vorschläge des SpecManagers manuell angepasst. Manuell angepasste bzw. zugewiesene Beschreibungen orientieren sich in der Regel an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt. Stark verbreiterte Signale - z.B. verursacht durch schnelle Rotation von Molekülteilen oder aufgrund von austauschenden Protonen - wurden ebenfalls tentativ zugeordnet (oft als breites Multiplett oder breites Singulett bezeichnet) oder sind nicht aufgeführt.

Schmelzpunkte und Schmelzbereiche, soweit angegeben, sind nicht korrigiert.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

## Allgemeine Arbeitsvorschriften

### AAV1

Eine Lösung der entsprechenden Carbonsäure (1 Äq.) in DMF (0.08-0.12M) wurde mit N,N-Diisopropylethylamin (1.4-1.5 Äq. bzw. 2.4-3.0 Äq. wenn das Amin als Hydrochlorid eingesetzt wurde) und HATU (1.0-1.65 Äq.) versetzt und die Mischung für 30 min bei RT gerührt. Anschließend wurde das entsprechende Amin (1.04-1.5 Äq.) zugegeben und die Mischung für 0.15-2h bei Raumtemperatur nachgerührt. Die Reaktion wurde dann durch die Zugabe von Wasser und 1M wässriger Salzsäure beendet. Der Niederschlag wurde abfiltriert, in DCM aufgenommen, über Magnesiumsulfat getrocknet, filtriert und das Lösungsmittel unter reduziertem Druck entfernt. Alternativ wurde nach dem Ansäuern mit Essigsäureethylester extrahiert, die vereinigten organischen Phasen über Magnesiumsulfat oder Natriumsulfat getrocknet, filtriert und das Lösungsmittel unter reduziertem Druck entfernt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung wenig mit Acetonitril, Wasser und Ameisensäure verdünnt und die Rohlösung mittels RP-HPLC (Wasser-Acetonitril-Gradient) gereinigt.

### AAV2

Kalium- oder Cäsiumcarbonat (1.5-2.5 Äq.) wurden im Reaktionsgefäß im Vakuum ausgeheizt. Es wurde auf RT abgekühlt und mit Argon geflutet. Palladiumacetat (0.1-0.36 Äq.), 9,9-Dimethyl-4,5-bis-(diphenylphosphino)-xanthen (Xantphos, 0.18-0.36 Äq.) und Dioxan (0.04-0.12M) wurden zugegeben und die Suspension wurde für 10 min bei Raumtemperatur im Argonstrom entgast. Anschließend wurde das entsprechende Amid (1.0-10 Äq.) und das entsprechende 7-Chlor-4-oxo-1,4-dihydro-1,8-naphthyridin (1.0 Äq.) zugegeben. Es wurde für 1h (oder bis zum vollständigen Umsatz nach analytischer HPLC oder Dünnschichtchromatographie mit entsprechenden Laufmittelgemischen) bei 80-110°C gerührt. Es wurde auf RT abgekühlt und alle flüchtigen Komponenten unter reduziertem Druck entfernt oder alternativ das Reaktionsgemisch auf Wasser gegossen, der pH-Wert mit 1M wässriger Salzsäure auf pH 1 eingestellt, mit Essigsäureethylester extrahiert, die vereinigten organischen Phasen mit gesättigter, wässriger Natriumchlorid-Lösung gewaschen, über Magnesiumsulfat getrocknet, filtriert und das Lösungsmittel unter reduziertem Druck entfernt. Das Rohprodukt wurde anschließend entweder mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung wenig mit Acetonitril, Wasser und Ameisensäure verdünnt und die Rohlösung mittels RP-HPLC (Wasser-Acetonitril-Gradient) gereinigt.

AAV3

Eine Lösung des entsprechenden 7-Chlor-4-oxo-1,4-dihydro-1,8-naphthyridins in DMF (0.10-0.22M) wurde nacheinander mit dem entsprechenden Amin (1.2 Äq.) und DIPEA (1.5-3.5 eq) versetzt. Die Reaktionslösung wurde über Nacht bei RT gerührt. Das Rohprodukt wurde anschließend entweder nach wääsriger Aufarbeitung und Extraktion mit entsprechendem organischen Lösungsmittel mittels Normalphasen-Chromatographie (Eluent: Cyclohexan-Essigsäureethylester-Gemische oder Dichlormethan-Methanol-Gemische) oder präparativer RP-HPLC (Wasser-Acetonitril-Gradient) gereinigt. Alternativ wurde die Reaktionsmischung wenig mit Acetonitril, Wasser und Ameisensäure verdünnt und die Rohlösung mittels RP-HPLC (Wasser-Acetonitril-Gradient) gereinigt.

**AUSGANGSVERBINDUNGEN UND INTERMEDIATE:**

**Beispiel 1A**

3-(1-{[(Benzyloxy)carbonyl]amino}cyclopropyl)-3-oxopropansäureethylester

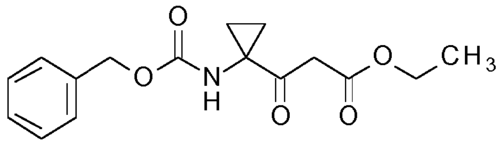

Eine Lösung aus 10.0 g (42.5 mmol) 1-{[(Benzyloxy)carbonyl]amino}cyclopropancarbonsäure in 316 ml THF wurde mit 5.48 g (33.8 mmol) Carbonyldiimidazol (CDI) versetzt und für 2.5 h bei RT nachgerührt. Anschließend wurden unter Eisbabkühlung 5.79 g (34.0 mmol) Kalium-3-ethoxy-3-oxopropanoat und 2.93 g (30.8 mmol) Magnesiumchlorid zugegeben. Nach vollständiger Zugabe wurde über Nacht bei 50°C nachgerührt. Das Lösungsmittel wurde unter reduziertem Druck entfernt, der Rückstand mit Essigsäureethylester und gesättigter wässriger Ammoniumchlorid-Lösung aufgenommen und die Phasen getrennt. Die organische Phase wurde mit gesättigter wässriger Natriumchlorid-Lösung gewaschen, über Magnesiumsulfat getrocknet, filtriert und das Lösungsmittel unter reduziertem Druck entfernt. Der Rückstand wurde mittels Normalphasenchromatographie (Essigsäureethylester-Cyclohexan-Gradient) gereinigt und 7.38 g (57 % d. Th., 100% Reinheit) der Titelverbindung erhalten.
LC-MS (Methode 1): Rt = 0.86 min; MS (ESIpos): m/z = 306 [M+H]+.
$^1$H-NMR (400 MHz, DMSO-d6): $\delta$ [ppm] = 8.16 (s, 1H), 7.31-7.40 (m, 5H), 5.06 (s, 2H), 4.07 (q, 2H), 3.60 (s, 2H), 1.36-1.47 (m, 2H), 1.12-1.20 (m, 5H).

**Beispiel 1B**

3-(1-{[(Benzyloxy)carbonyl]amino}cyclopropyl)-2,2-dimethyl-3-oxopropansäureethylester

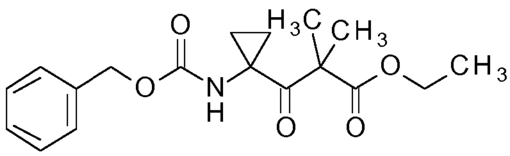

Die Gesamtmenge wurde auf zwei Mikrowellenvials aufgeteilt. Eine Suspension aus 1.50 g (4.91 mmol) 3-(1-{[(Benzyloxy)carbonyl]amino}cyclopropyl)-3-oxopropansäureethylester, 917 $\mu$l (14.7 mmol) Iodmethan und 1.36 g (9.83 mmol) Kaliumcarbonat in 21 ml Aceton wurde für 16h bei 60°C in der Mikrowelle zur Reaktion gebracht. Anschließend wurden weitere 459 $\mu$l (7.37 mmol) Iodmethan zugegeben und für 1h bei 60°C und 3h bei 120°C in der Mikrowelle zur Reaktion gebracht. Die Reaktionmischung wurde auf Wasser gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und in fünf Läufen mittels präparativer HPLC (Säule: Chromatorex C18, 10 $\mu$m, 125*40 mm, Lösungsmittel: Acetonitril, Wasser, 0.1% Ameisensäure) getrennt. Es wurden 471 mg (27%

d. Th., 94% Reinheit) der Titelverbindung erhalten.
LC-MS (Methode 2): Rt = 1.91 min; MS (ESIpos): m/z = 334 [M+H]+.
$^1$H-NMR (500 MHz, DMSO-d6): δ [ppm] = 7.89 (s, 1H), 7.29-7.42 (m, 5H), 5.00 (s, 2H), 3.99 (q, 2H), 1.37 (q, 2H), 1.25 (s, 6H), 1.08-1.15 (m, 5H).

**Beispiel 1C**

3-(1-{[(Benzyloxy)carbonyl]amino}cyclopropyl)-3-hydroxypivalonsäureethylester (Racemat)

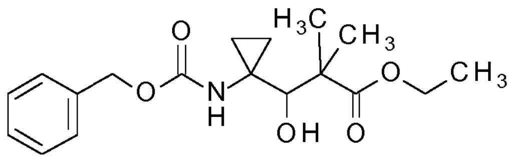

Zu einer Lösung von 465 mg (1.40 mmol) 3-(1-{[(Benzyloxy)carbonyl]amino}cyclopropyl)-2,2-dimethyl-3-oxopropansäureethylester in 7.3 ml Methanol wurden bei -30°C 73.9 mg (1.95 mmol) Natriumborhydrid gegeben. Es wurde für 2h bei -30°C nachgerührt und anschließend auf RT erwärmt und eine weitere Stunde gerührt. Die Reaktionslösung wurde auf gesättigte wässrige Ammoniumchlorid-Lösung gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in etwas Acetonitril aufgenommen, durch einen Milliporefilter filtriert und in zwei Läufen mittels präparativer HPLC (Säule: Chromatorex C18, 10 μm, 125*40 mm, Lösungsmittel: Acetonitril, Wasser, 0.1% Ameisensäure) gereinigt. Es wurden 330 mg (71% d. Th., 100% Reinheit) der Titelverbindung erhalten.
LC-MS (Methode 1): Rt = 0.94 min; MS (ESIpos): m/z = 336 [M+H]+.
$^1$H-NMR (400 MHz, DMSO-d6): δ [ppm] = 7.25-7.45 (m, 6H), 4.84-5.06 (m, 3H), 3.80-4.06 (m, 3H), 1.05-1.16 (m, 9H), 0.78-0.87 (m, 2H), 0.54-0.62 (m, 1H), 0.27-0.41 (m, 1H).

**Beispiel 1D**

7-Hydroxy-6,6-dimethyl-4-azaspiro[2.4]heptan-5-on (Racemat)

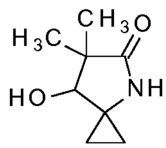

Zu einer Lösung von 325 mg (969 μmol) 3-(1-{[(Benzyloxy)carbonyl]amino}cyclopropyl)-3-hydroxy-pivalonsäureethylester in 4.9 ml Methanol wurden 24.3 mg Palladium auf Kohle (10%) gegeben und es für 6h bei Normaldruck und Raumtemperatur hydriert. Anschließend wurde die Reaktionsmischung durch einen Milliporefilter filtriert und das Lösungsmittel unter reduziertem Druck entfernt. Es wurden 150 mg (99 % d. Th., Reinheit 95%) der Titelverbindung erhalten.
$^1$H-NMR (400 MHz, DMSO-d6): δ [ppm] = 7.53 (br s, 1H), 5.03 (d, 1H), 3.66 (d, 1H), 1.04 (s, 3H), 0.94 (s, 3H), 0.84-0.91 (m, 1H), 0.56-0.69 (m, 2H), 0.43-0.51 (m, 1H).

**Beispiel 2A**

4- {[(Benzyloxy)carbonyl]amino}-3-oxobutansäureethylester

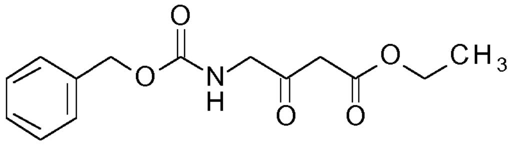

Eine Lösung aus 15.0 g (71.7 mmol) N-[(Benzyloxy)carbonyl]glycin in 534 ml THF wurde mit 9.24 g (57.0 mmol) Carbonyldiimidazol (CDI) versetzt und für 2.5h bei RT nachgerührt. Anschließend wurden unter Eisbadkühlung 9.76 g (57.4 mmol) Kalium-3-ethoxy-3-oxopropanoat und 4.95 g (52.0 mmol) Magnesiumchlorid zugegeben. Nach vollständiger Zugabe wurde für 48h bei 50°C nachgerührt. Das Lösungsmittel wurde unter reduziertem Druck entfernt, der Rückstand mit Essigsäureethylester und gesättigter wässriger Ammoniumchlorid-Lösung aufgenommen und die Phasen getrennt. Die organische Phase wurde mit gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Essigsäureethylester-Cyclohexan-Gradient) gereinigt und 12.7 g (60% d. Th., 95% Reinheit) der Titelverbindung erhalten.
LC-MS (Methode 1): Rt = 0.83 min; MS (ESIneg): m/z = 278 [M-H]-.
$^1$H-NMR (400 MHz, DMSO-d6): δ [ppm] = 7.56 (br t, 1H), 7.25-7.41 (m, 5H), 5.04 (s, 2H), 4.09 (q, 2H), 3.97 (d, 2H), 3.60 (s, 2H), 1.19 (t, 3H).

## Beispiel 2B

4-{[(Benzyloxy)carbonyl]amino}-2-methyl-3-oxobutansäureethylester (Racemat)

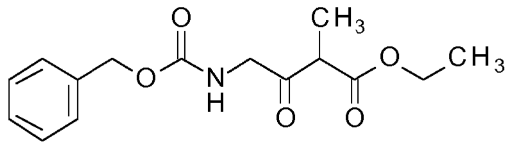

Eine Suspension aus 1.00 g (3.58 mmol) 4-{[(Benzyloxy)carbonyl]amino}-3-oxobutansäureethylester, 669 µl (10.7 mmol) Iodmethan und 990 mg (7.16 mmol) Kaliumcarbonat in 15 ml Aceton wurde für 2h bei 50°C in der Mikrowelle zur Reaktion gebracht. Es wurde für weitere 2h bei 45°C unter Reaktionskontrolle in der Mikrowelle bestrahlt. Die Reaktionsmischung wurde auf Wasser gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und in zwei Läufen mittels präparativer HPLC (Säule: Chromatorex C18, 10 µm, 125*40 mm, Lösungsmittel: Acetonitril, Wasser, 0.1% Ameisensäure) getrennt. Es wurden 536 mg (51% d. Th., 100% Reinheit) der Titelverbindung erhalten.
LC-MS (Methode 1): Rt = 0.87 min; MS (ESIneg): m/z = 292 [M-H]-.
$^1$H-NMR (400 MHz, DMSO-d6): δ [ppm] = 7.57 (br t, 1H), 7.24-7.40 (m, 5H), 5.04 (s, 2H), 4.09 (q, 2H), 4.03 (d, 2H), 3.80 (q, 1H), 1.22-1.09 (m, 6H).

## Beispiel 2C

4-{[(Benzyloxy)carbonyl]amino}-3-hydroxy-2-methylbutansäureethylester (Diastereomerengemisch)

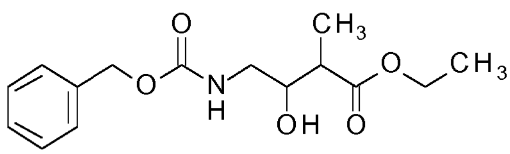

Zu einer Lösung von 533 mg (1.82 mmol) 4-{[(Benzyloxy)carbonyl]amino}-2-methyl-3-oxobutansäureethylester in 9.2 ml Methanol wurden bei -78°C 96.2 mg (2.54 mmol) Natriumborhydrid gegeben. Es wurde langsam unter Reaktionskontrolle auf -15°C erwärmt. Bei -15°C wurde die Reaktion durch Zugabe von gesättigter wässriger Ammoniumchlorid-Lösung beendet. Es wurde dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in etwas Acetonitril aufgenommen und in zwei Läufen mittels präparativer HPLC (Säule: Chromatorex C18, 10 µm, 125*40 mm, Lösungsmittel: Acetonitril, Wasser, 0.1% Ameisensäure) gereinigt. Es wurden 398 mg (74% d. Th., 100% Reinheit) der Titelverbindung erhalten.
LC-MS (Methode 1): Rt = 0.80 min; MS (ESIpos): m/z = 296 [M+H]+.
$^1$H-NMR (400 MHz, DMSO-d6): δ [ppm] = 7.26-7.45 (m, 5H), 7.20-7.25 (m, 0.3H), 7.11 (br t, 0.7H), 5.01 (s, 2H), 4.90-4.97 (m, 1H), 3.98-4.08 (m, 2H), 3.81-3.88 (m, 0.3H), 3.63-3.71 (m, 0.7H), 3.11-3.20 (m, 0.7H), 2.93-3.07 (m, 1.3H), 2.40-2.49 (m, 1H), 1.17 (t, 3H), 1.00-1.05 (m, 3H).

**Beispiel 2D**

4-Hydroxy-3-methylpyrrolidin-2-on (Diastereomerengemisch)

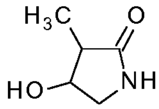

Zu einer Lösung von 397 mg (1.34 mmol) 4-{[(Benzyloxy)carbonyl]amino}-3-hydroxy-2-methylbutansäureethylester in 7.2 ml Methanol wurden 40 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Es wurden 211 mg (quantitativ) der Titelverbindung erhalten, welche ohne weitere Aufreinigung im nächsten Schritt eingesetzt wurde.

**Beispiel 3A**

2-Dibenzylamino-propansäureethylester (Racemat)

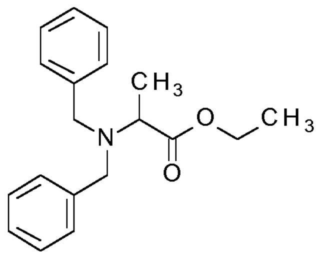

Eine Lösung von 12.0 g (66.3 mmol) 2-Brompropansäureethylester in 17.5 ml Ethanol wurde mit 14.4 g (72.9 mmol) Dibenzylamin versetzt und über Nacht unter Rückfluss erhitzt. Anschließend wurde das Lösungsmittel unter reduziertem Druck entfernt und der Rückstand mit 1M wässriger NatriumhydroxidLösung versetzt. Es wurde dreimal mit Dichlormethan extrahiert und die vereinigten organischen Phasen mit gesättigter wässriger Natriumchlorid-Lösung gewaschen. Über Natriumsulfat getrocknet, filtriert und das Lösungsmittel unter reduziertem Druck entfernt. Der Rückstand wurde mittels Normalphasen-chromatographie (Essigsäureethylester-Cyclohexan-Gradient) gereinigt. Es wurden 10 g (50% d. Th., 97% Reinheit) der Titelverbindung erhalten.
LC-MS (Methode 1): Rt = 1.28 min; MS (ESIpos): m/z = 298 [M+H]+.
$^1$H-NMR (400 MHz, DMSO-d6): δ [ppm] = 7.20-7.37 (m, 10H), 4.06-4.20 (m, 2H), 3.74 (d, 2H), 3.58 (d, 2H), 3.32-3.40 (m, 1H), 1.22-1.28 (m, 6H).

**Beispiel 3B**

4-(Dibenzylamino)-3-oxopentansäureethylester (Racemat)

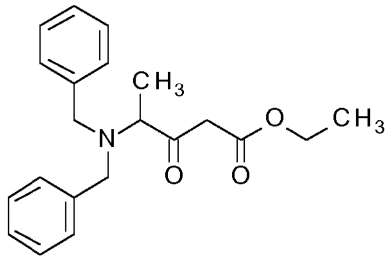

Eine Lösung aus 168 ml (168 mmol, 1M in THF) Bis-(trimethylsilyl)-lithiumamid verdünnt mit 75 ml THF wurde auf -78°C abgekühlt und mit 6.91 ml (70.6 mmol) Essigsäureethylester versetzt. Es wurde 30 min bei -78°C nachgerührt. Anschließend wurde eine Lösung von 10.0 g (33.6 mmol) 2-Dibenzylamino-propansäureethylester in 37 ml THF langsam zugetropft. Es wurde für 2h bei -78°C und 1h bei 0°C nachgerührt. Die Reaktion wurde durch Zugabe von gesättigter wässriger Ammoniumchlorid-Lösung beendet. Es wurde dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde mittels Normalphasenchromatographie (Essigsäureethylester-Cyclohexan-Gradient) gereinigt. Es wurden 9.89 g (87% d. Th., 100% Reinheit) der Titelverbindung erhalten.
LC-MS (Methode 2): Rt = 2.42 min; MS (ESIpos): m/z = 340 [M+H]+.
$^1$H-NMR (400 MHz, DMSO-d6): δ [ppm] = 7.24-7.40 (m, 10H), 3.99 (q, 2H), 3.68-3.77 (m, 1H), 3.53-3.64 (m, 3H), 3.37-3.49 (m, 3H), 1.07-1.13 (m, 6H).

**Beispiel 3C**

1-[2-(Dibenzylamino)propanoyl]cyclopropancarbonsäureethylester (Racemat)

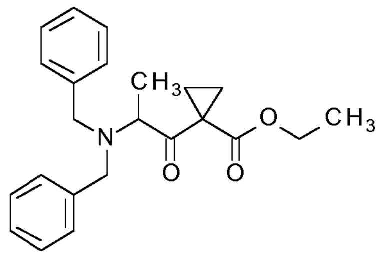

Eine Suspension aus 2.00 g (5.89 mmol) 4-(Dibenzylamino)-3-oxopentansäureethylester, 1.12 ml (12.9 mmol) 1,2-Dibromethan und 1.63 g (11.8 mmol) Kaliumcarbonat wurden in 6 ml Aceton über Nacht unter Rückfluss erhitzt. Anschließend wurden weitere 0.56 ml (1.1 Äq.) 1,2-Dibromethan und 814 mg Kaliumcarbonat hinzugegeben und für 48h unter Rückfluss erhitzt. Die Reaktionsmischung wurde auf Wasser gegossen und dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril aufgenommen, durch einen Milliporefilter filtriert und in fünf Läufen mittels präparativer HPLC (Säule: Chromatorex C18, 10 μm, 125*40 mm, Lösungsmittel: Acetonitril, Wasser, 0.1% Ameisensäure) gereinigt. Es wurden 926 mg (41% d. Th., 96% Reinheit) der Titelverbindung erhalten.
LC-MS (Methode 1): Rt = 1.29 min; MS (ESIpos): m/z = 366 [M+H]+.
$^1$H-NMR (400 MHz, DMSO-d6): δ [ppm] = 7.22-7.36 (m, 10H), 4.24 (q, 1H), 3.92 (dq, 1H), 3.71 (dq, 1H), 3.51 (s, 4H), 1.50 (qd, 2H), 1.07-1.17 (m, 5H), 0.95 (t, 3H).

**Beispiel 3D**

1-[2-(Dibenzylamino)-1-hydroxypropyl]cyclopropancarbonsäureethylester (Diastereomerengemisch)

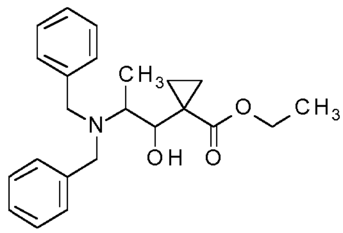

Zu einer Lösung von 925 mg (2.53 mmol) 1-[2-(Dibenzylamino)propanoyl]cyclopropancarbonsäure-ethylester in 13 ml Methanol wurden bei -78°C 134 mg (3.54 mmol) Natriumborhydrid gegeben. Es wurde langsam unter Reaktionskontrolle auf -15°C erwärmt. Bei -15°C wurde die Reaktion durch Zugabe von gesättigter wässriger Ammoniumchlorid-Lösung beendet. Es wurde dreimal mit Essigsäureethylester extrahiert. Die vereinigten organischen Phasen wurden mit gesättigter wässriger Natriumchlorid-Lösung gewaschen, über Natriumsulfat getrocknet, filtriert und das Lösungsmittel unter reduziertem Druck entfernt. Der Rückstand wurde in etwas Acetonitril aufgenommen und in drei Läufen mittels präparativer HPLC (Säule: Chromatorex C18, 10 μm, 125*40 mm, Lösungsmittel: Acetonitril, Wasser, 0.1% Ameisensäure) gereinigt. Es wurden 628 mg (66% d. Th., 98% Reinheit) der Titelverbindung erhalten.
LC-MS (Methode 1): Rt = 0.70 min; MS (ESIpos): m/z = 368 [M+H]+.
$^1$H-NMR (400 MHz, DMSO-d6): δ [ppm] = 7.17-7.38 (m, 10H), 4.29 (d, 1H), 3.76-3.89 (m, 4H), 3.54 (br dd, 1H), 3.40 (d, 2H), 3.09 (quintt, 1H), 1.06-1.12 (m, 1H), 0.91-1.03 (m, 7H), 0.80-0.88 (m, 1H), 0.65-0.72 (m, 1H).

**Beispiel 3E**

7-Hydroxy-6-methyl-5-azaspiro[2.4]heptan-4-on (Diastereomerengemisch)

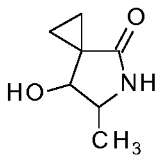

Zu einer Lösung von 626 mg (1.70 mmol) 1-[2-(Dibenzylamino)-1-hydroxypropyl]cyclopropancarbonsäureethylester in 12.7 ml Methanol wurden 63.5 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Es wurden 243 mg (96% d. Th., 95% Reinheit) der Titelverbindung erhalten.
LC-MS (Methode 5): Rt = 0.49 min; MS (ESIpos): m/z = 142 [M+H]+.
$^1$H-NMR (400 MHz, DMSO-d6): δ [ppm] = 7.58 (br s, 1H), 4.86 (d, 1H), 3.91 (t, 1H), 3.69 (quintt, 1H), 1.07 (d, 3H), 0.86-0.93 (m, 1H), 0.67-0.79 (m, 3H).

**Beispiel 4A**

7-Chlor-1-(3,5-difluorpyridin-2-yl)-4-oxo-1,4-dihydro-1,8-naphthyridin-3-carbonsäureethylester

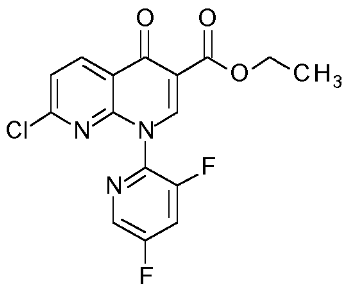

Eine Lösung aus 43.7 g (137 mmol) 2-[(2,6-Dichlorpyridin-3-yl)carbonyl]-3-ethoxyacrylsäureethylester (CAS 157373-27-8) und 25.0 g (192 mmol) 3,5-Difluorpyridin-2-amin in 216 ml DCM wurde mit 167 ml (961 mmol) DIPEA versetzt und für 4h bei RT gerührt. Anschließend wurden 19.0 g (137 mmol) Kaliumcarbonat zugegeben und für 2d unter Rückfluss erhitzt. Die Mischung wurde mit DCM verdünnt und dreimal mit 1M wässriger Salzsäure gewaschen. Die organische Phase wurde über Natriumsulfat getrocknet, filtriert und das Lösungsmittel unter reduziertem Druck entfernt. Es wurde mit Ether verdünnt und der Niederschlag abgesaugt. Die Prozedur wurde mit der Mutterlauge wiederholt und der gewonne Feststoff mit der Hauptfraktion vereinigt. Es wurden 26.6 g (53% d. Th., 100% Reinheit) der Titelverbindung erhalten.
LC-MS (Methode 2): Rt = 1.67 min; MS (ESIpos): m/z = 366 [M+H]+.
$^1$H-NMR (400 MHz, DMSO-d6): $\delta$ [ppm] = 8.89 (s, 1H), 8.66 (d, 1H), 8.62 (d, 1H), 8.39 (ddd, 1H), 7.68 (d, 1H), 4.26 (q, 2H), 1.28 (t, 3H).

## Beispiel 4B

7-Chlor-1-(3,5-difluorpyridin-2-yl)-4-oxo-1,4-dihydro-1,8-naphthyridin-3-carbonsäure

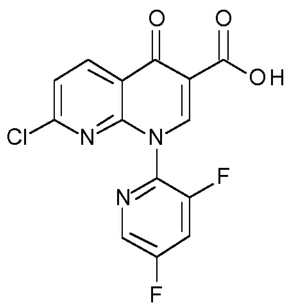

26.6 g (72.9 mmol) 7-Chlor-1-(3,5-difluorpyridin-2-yl)-4-oxo-1,4-dihydro-1,8-naphthyridin-3-carbonsäureethylester wurden in 218 ml Wasser vorgelegt, mit 218 ml 36 proz. wässriger Salzsäure und 218 ml THF versetzt und 3h bei 120°C nachgerührt. Das Reaktionsgemisch wurde auf RT abgekühlt und mit 1450 ml Wasser versetzt. Der Niederschlag wurde abgesaugt, mit Wasser nachgewaschen und am Hochvakuum getrocknet. Es wurden 24.3 g (97% d. Th., 99% Reinheit) der Titelverbindung erhalten.
LC-MS (Methode 2): Rt = 1.61 min; MS (ESIpos): m/z = 338 [M+H]+.
$^1$H-NMR (400 MHz, DMSO-d6): $\delta$ [ppm] = 13.86 (s, 1H), 9.18 (s, 1H), 8.78 (d, 1H), 8.67 (d, 1H), 8.42 (ddd, 1H), 7.82 (d, 1H).

## Beispiel 4C

7-Chlor-1-(3,5-difluorpyridin-2-yl)-4-oxo-N-[(2R)-1,1,1-trifluorbutan-2-yl]-1,4-dihydro-1,8-naphthyridin-3 -carbonsäureamid

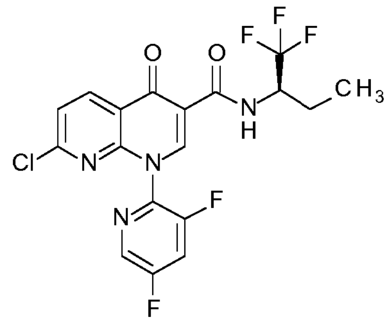

Gemäß allgemeiner Arbeitsvorschrift 1 wurden 200 mg (592 µmol) 7-Chlor-1-(3,5-difluorpyridin-2-yl)-4-oxo-1,4-dihydro-1,8-naphthyridin-3-carbonsäure (Beispiel 4B) mit 145 mg (888 µmol) (2R)-1,1,1-Trifluorbutan-2-amin-Hydrochlorid in Gegenwart von 270 mg (711 µmol) HATU und 413 µl (2.40 mmol) DIPEA in 3 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 197 mg (74% d. Th., 100% Reinheit) der Titelverbindung erhalten.
LC-MS (Methode 1): Rt = 1.15 min; MS (ESIpos): m/z = 447 [M+H]+.
$^1$H-NMR (400 MHz, DMSO-d6): δ [ppm] = 9.89 (d, 1H), 9.10 (s, 1H), 8.75 (d, 1H), 8.67 (d, 1H), 8.40 (ddd, 1H), 7.78 (d, 1H), 4.71-4.85 (m, 1H), 1.83-1.96 (m, 1H), 1.60-1.74 (m, 1H), 0.98 (t, 3H).

## Beispiel 5A

7-Chlor-1-(3,5-difluorpyridin-2-yl)-4-oxo-N-[(2S)-1,1,1-trifluorbutan-2-yl]-1,4-dihydro-1,8-naphthyridin-3 -carbonsäureamid

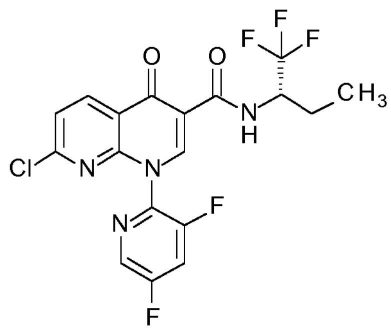

Gemäß allgemeiner Arbeitsvorschrift 1 wurden 400 mg (1.19 mmol) 7-Chlor-1-(3,5-difluorpyridin-2-yl)-4-oxo-1,4-dihydro-1,8-naphthyridin-3-carbonsäure (Beispiel 4B) mit 290 mg (1.78 mmol) (2S)-1,1,1-Trifluorbutan-2-amin in Gegenwart von 540 mg (1.42 mmol) HATU und 0.83 ml (4.7 mmol) DIPEA in 6 ml DMF zur Reaktion gebracht. Die Mischung wurde anschließend mit 20 ml Wasser und 30 ml Essigsäureethylester verdünnt. Die Phasen wurden getrennt und die wässrige Phase dreimal mit 20 ml Essigsäureethylester extrahiert. Die vereinigten organischen Phasen wurden mit 20 ml 1M wässriger Salzsäure/gesättigter wässriger Natriumchloridlösung (1:1) und dreimal mit 15 ml gesättigter wässriger Natriumchlorid-Lösung gewaschen. Über Natriumsulfat getrocknet, filtriert und das Lösungsmittel unter reduziertem Druck entfernt. Das Rohprodukt wurde in wenig Acetonitril gelöst und in zwei Läufen mittels präparativer HPLC (Säule: Chromatorex C18, 10 µm, 125*40 mm, Lösungsmittel: Acetonitril, Wasser, 0.1% Ameisensäure) gereinigt und 367 mg (69% d. Th., 100% Reinheit) der Titelverbindung erhalten.
LC-MS (Methode 2): Rt = 2.19 min; MS (ESIpos): m/z = 447 [M+H]+.
$^1$H-NMR (400 MHz, DMSO-d6): δ [ppm] = 9.89 (d, 1H), 9.10 (s, 1H), 8.75 (d, 1H), 8.67 (d, 1H), 8.40 (ddd, 1H), 7.78 (d, 1H), 4.72-4.84 (m, 1H), 1.84-1.95 (m, 1H), 1.62-1.73 (m, 1H), 0.98 (brt, 3H).

**Beispiel 6A**

7-Chlor-1-(3,5-difluorpyridin-2-yl)-4-oxo-N-[(2S)-1,1,1-trifluorpropan-2-yl]-1,4-dihydro-1,8-naphthyridin-3-carbonsäureamid

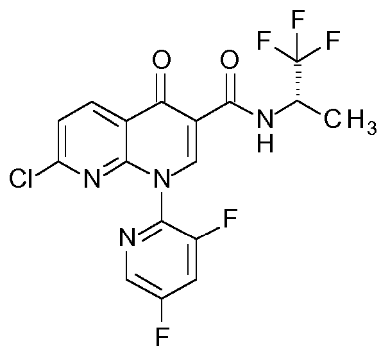

Gemäß allgemeiner Arbeitsvorschrift 1 wurden 200 mg (592 μmol) 7-Chlor-1-(3,5-difluorpyridin-2-yl)-4-oxo-1,4-dihydro-1,8-naphthyridin-3-carbonsäure (Beispiel 4B) mit 133 mg (888 μmol) (2S)-1,1,1-Trifluorpropan-2-amin-Hydrochlorid in Gegenwart von 270 mg (711 μmol) HATU und 0.41 ml (2.4 mmol) DIPEA in 3 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 180 mg (70% d. Th., 100% Reinheit) der Titelverbindung erhalten.
LC-MS (Methode 1): Rt = 1.15 min; MS (ESIpos): m/z = 433 [M+H]+.
$^1$H-NMR (500 MHz, DMSO-d6): δ [ppm] = 9.94 (d, 1H), 9.10 (s, 1H), 8.74 (d, 1H), 8.67 (d, 1H), 8.40 (ddd, 1H), 7.78 (d, 1H), 4.89-4.98 (m, 1H), 1.40 (d, 3H).

**Beispiel 7A**

7-Chlor-N-[1-cyclopropyl-2,2,2-trifluorethyl]-1-(3,5-difluorpyridin-2-yl)-4-oxo-1,4-dihydro-1,8-naphthyridin-3 -carbonsäureamid (Racemat)

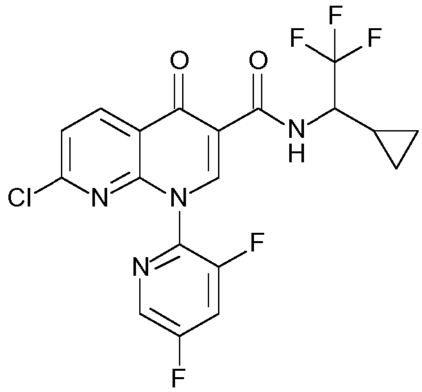

Gemäß allgemeiner Arbeitsvorschrift 1 wurden 100 mg (296 μmol) 7-Chlor-1-(3,5-difluorpyridin-2-yl)-4-oxo-1,4-dihydro-1,8-naphthyridin-3-carbonsäure (Beispiel 4B) mit 67.6 mg (385 μmol) 1-Cyclopropyl-2,2,2-trifluorethanamin-Hydrochlorid (Racemat) in Gegenwart von 135 mg (355 μmol) HATU und 206 μl (1.20 mmol) DIPEA in 1.5 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 102 mg (75% d. Th., 100% Reinheit) der Titelverbindung erhalten.

LC-MS (Methode 1): Rt = 1.16 min; MS (ESIpos): m/z = 459 [M+H]+.
$^{1}$H-NMR (400 MHz, DMSO-d6): δ [ppm] = 10.04 (d, 1H), 9.09 (s, 1H), 8.75 (d, 1H), 8.67 (d, 1H), 8.40 (ddd, 1H), 7.78 (d, 1H), 4.34-4.48 (m, 1H), 1.18-1.30 (m, 1H), 0.50-0.72 (m, 3H), 0.33 (br. s., 1H).

**Beispiel 8A**

7-Chlor-N-[(1R)-1-cyclopropyl-2,2,2-trifluorethyl]-1-(3,5-difluorpyridin-2-yl)-4-oxo-1,4-dihydro-1,8-naphthyridin-3 -carbonsäureamid

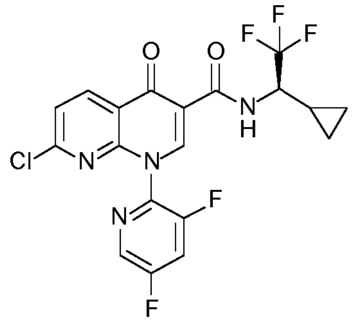

Gemäß allgemeiner Arbeitsvorschrift 1 wurden 200 mg (592 μmol) 7-Chlor-1-(3,5-difluorpyridin-2-yl)-4-oxo-1,4-dihydro-1,8-naphthyridin-3-carbonsäure (Beispiel 4B) mit 135 mg (770 μmol) (1R)-1-Cyclopropyl-2,2,2-trifluorethanamin-Hydrochlorid in Gegenwart von 270 mg (711 μmol) HATU und 0.41 ml (2.4 mmol) DIPEA in 2.7 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 180 mg (66% d. Th., 100% Reinheit) der Titelverbindung erhalten.
LC-MS (Methode 2): Rt = 2.22 min; MS (ESIpos): m/z = 459 [M+H]+.
$^{1}$H-NMR (400 MHz, DMSO-d6): δ [ppm] = 10.04 (d, 1H), 9.09 (s, 1H), 8.75 (d, 1H), 8.67 (d, 1H), 8.40 (ddd, 1H), 7.78 (d, 1H), 4.34-4.47 (m, 1H), 1.19-1.31 (m, 1H), 0.52-0.71 (m, 3H), 0.33 (br. s., 1H).

**Beispiel 9A**

7-Chlor-N-[(1 S)-1-cyclopropyl-2,2,2-trifluorethyl]-1-(3,5-difluorpyridin-2-yl)-4-oxo-1,4-dihydro-1,8-naphthyridin-3 -carbonsäureamid

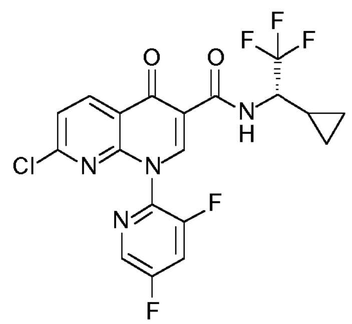

Gemäß allgemeiner Arbeitsvorschrift 1 wurden 600 mg (1.78 mmol) 7-Chlor-1-(3,5-difluorpyridin-2-yl)-4-oxo-1,4-dihydro-1,8-naphthyridin-3-carbonsäure (Beispiel 4B) mit 468 mg (2.67 mmol) (1S)-1-Cyclopropyl-2,2,2-trifluorethanamin-Hydrochlorid in Gegenwart von 675 mg (1.77 mmol) HATU und 0.74 ml (4.3 mmol) DIPEA in 18 ml DMF zur Reaktion gebracht. Die Mischung wurde anschließend mit 20 ml Wasser und 30 ml Essigsäureethylester verdünnt. Die Phasen wurden getrennt und die wässrige Phase dreimal mit 20 ml Essigsäureethylester extrahiert. Die vereinigten

organischen Phasen wurden mit 20 ml 1M wässriger Salzsäure/gesättigter wässriger Natriumchloridlösung (1:1) und dreimal mit 15 ml gesättigter wässriger Natriumchlorid-Lösung gewaschen, über Natriumsulfat getrocknet, filtriert und das Lösungsmittel unter reduziertem Druck entfernt. Das Rohprodukt wurde in wenig Dichlormethan gelöst und mittels Normalphasenchromatographie (Essigsäureethylester-Cyclohexan-Gradient) gereinigt und 384 mg (47% d. Th., 100% Reinheit) der Titelverbindung erhalten.
LC-MS (Methode 1): Rt = 1.21 min; MS (ESIpos): m/z = 459 [M+H]+.
$^1$H-NMR (500 MHz, DMSO-d6): δ [ppm] = 10.04 (br d, 1H), 9.09 (br s, 1H), 8.75 (d, 1H), 8.66 (d, 1H), 8.39 (ddd, 1H), 7.78 (d, 1H), 4.41 (sxt, 1H), 1.21-1.28 (m, 1H), 0.63-0.70 (m, 1H), 0.54-0.63 (m, 2H), 0.29-0.38 (m, 1H).

**Beispiel 10A**

7-Chlor-1-(3,5-difluorpyridin-2-yl)-N-(1,1,1,3,3,3-hexaf|uorpropan-2-yl)-4-oxo-1,4-dihydro-1,8-naphthyridin-3-carbonsäureamid

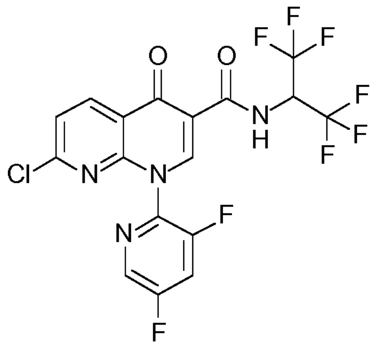

Zu einer Lösung aus 800 mg (2.37 mmol) 7-Chlor-1-(3,5-difluorpyridin-2-yl)-4-oxo-1,4-dihydro-1,8-naphthyridin-3-carbonsäure (Beispiel 4B), 435 mg (2.61 mmol) 1,1,1,3,3,3-Hexafluorpropan-2-amin und 1.24 ml (7.11 mmol) DIPEA in 21 ml Essigsäureethylester wurden 4.15 ml (7.11 mmol) 2,4,6-Tripropyl-1,3,5,2,4,6-trioxatriphosphorinan-2,4,6-trioxid (T3P, 50% in DMF) zugetropft. Es wurde über Nacht bei 80°C nachgerührt. Die Reaktionsmischung wurde auf Wasser und Essigsäureethylester gegossen und die Phasen getrennt. Die organische Phase wurde dreimal mit Wasser und einmal mit gesättigter wässriger Natriumchlorid-Lösung gewaschen, über Natriumsulfat getrocknet, filtriert und das Lösungsmittel unter reduziertem Druck entfernt. Der Rückstand wurde in wenig Acetonitril gelöst, über einen Milliporefilter filtriert und in drei Läufen mittels präparativer HPLC (Säule: Chromatorex C18, 10 μm, 125*40 mm, Lösungsmittel: Acetonitril, Wasser, 0.1% Ameisensäure) gereinigt. Es wurden 785 mg (68% d. Th., 100% Reinheit) der Titelverbindung erhalten.
LC-MS (Methode 2): Rt = 2.29 min; MS (ESIpos): m/z = 487 [M+H]+.
$^1$H-NMR (400 MHz, DMSO-d6): δ [ppm] = 10.77 (d, 1H), 9.19 (s, 1H), 8.78 (d, 1H), 8.68 (d, 1H), 8.41 (ddd, 1H), 7.81 (d, 1H), 6.35-6.50 (m, 1H).

**Beispiel 11A**

7-Chlor-1-(3,5-difluorpyridin-2-yl)-4-oxo-1,4-dihydro-1,8-naphthyridin-3-carbonsäurechlorid

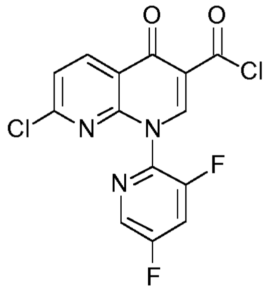

Zu einer Lösung von 1.00 g (2.96 mmol) 7-Chlor-1-(3,5-difluorpyridin-2-yl)-4-oxo-1,4-dihydro-1,8-naphthyridin-3-carbonsäure (Beispiel 4B) in 23.7 ml THF wurden 639 µl (8.77 mmol) Thionylchlorid gegeben und eine Stunde bei RT nachgerührt. Es wurde eine Stunde unter Rückfluss erhitzt und anschließend alle flüchtigen Komponenten unter reduziertem Druck entfernt. Das Rohprodukt wurde ohne weitere Aufarbeitung im nächsten Schritt eingesetzt (quantitative Umsetzung wurde angenommen).

**Beispiel 11B**

7-Chlor-N-(2,6-dichlorphenyl)-1-(3,5-difluorpyridin-2-yl)-4-oxo-1,4-dihydro-1,8-naphthyridin-3-carbonsäureamid

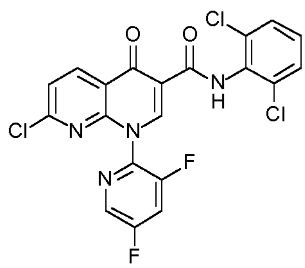

Zu einer Lösung von 1.05 g (2.95 mmol) 7-Chlor-1-(3,5-difluorpyridin-2-yl)-4-oxo-1,4-dihydro-1,8-naphthyridin-3-carbonsäurechlorid in 62 ml Dichlormethan wurden bei RT 1.23 ml (8.85 mmol) Triethylamin und 573 mg (3.54 mmol) 2,6-Dichloranilin gegeben. Es wurde für 30 min bei RT und über Nacht bei 50°C nachgerührt. Die Reaktionsmischung wurde eingeengt und in Dichlormethan aufgenommen, zweimal mit 1M wässriger Salzsäure gewaschen, Magnesiumsulfat getrocknet, filtriert und das Lösungsmittel unter reduziertem Druck entfernt. Das Rohprodukt wurde mittels Normalphasenchromatographie (Essigsäureethylester-Cyclohexan / 50:50) gereinigt. Es wurden 412 mg (29% d. Th., 100% Reinheit) der Titelverbindung erhalten.
LC-MS (Methode 1): Rt = 1.17 min; MS (ESIpos): m/z = 481 [M+H]+.
$^1$H-NMR (400 MHz, DMSO-d6): δ [ppm] = 11.34 (s, 1H), 9.17 (s, 1H), 8.81 (d, 1H), 8.67 (d, 1H), 8.40 (ddd, 1H), 7.80 (d, 1H), 7.60 (d, 2H), 7.40 (t, 1H).

**Beispiel 12A**

7-Chlor-1-(3,5-difluorpyridin-2-yl)-4-oxo-N-[1-(trifluormethoxy)butan-2-yl]-1,4-dihydro-1,8-naphthyridin-3-carbonsäureamid (Racemat)

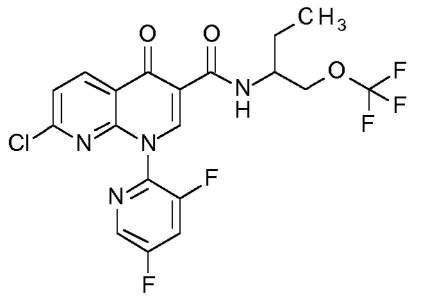

Gemäß allgemeiner Arbeitsvorschrift 1 wurden 200 mg (585 µmol) 7-Chlor-1-(3,5-difluorpyridin-2-yl)-4-oxo-1,4-dihydro-1,8-naphthyridin-3-carbonsäure (Beispiel 4B) mit 170 mg (878 µmol) 1-(Trifluormethoxy)butan-2-amin-Hydrochlorid (Racemat) in Gegenwart von 267 mg (702 µmol) HATU und 408 µl (2.34 mmol) DIPEA in 2.5 ml DMF zur Reaktion gebracht. Es wurde für 10 min nachgerührt. Die Rohlösung wurde mittels präparativer HPLC (Säule: Chromatorex C18, 10 µm, 125*40 mm, Lösungsmittel: Acetonitril, Wasser, 0.1% Ameisensäure) gereinigt und 148 mg (52% d. Th., 98% Reinheit) der Titelverbindung erhalten.
LC-MS (Methode 2): Rt = 2.24 min; MS (ESIpos): m/z = 477 [M+H]+.
$^1$H-NMR (500 MHz, DMSO-d6): δ [ppm] = 9.61 (d, 1H), 9.05 (s, 1H), 8.74 (d, 1H), 8.66 (d, 1H), 8.39 (ddd, 1H), 7.75 (d, 1H), 4.15-4.27 (m, 3H), 1.55-1.74 (m, 2H), 0.94 (t, 3H).

**Beispiel 13A**

7-Chlor-1-(3,5-difluorpyridin-2-yl)-4-oxo-N-[(2S)-1,1,1-trifluor-3,3-dimethylbutan-2-yl]-1,4-dihydro-1,8-naphthyridin-3-carbonsäureamid

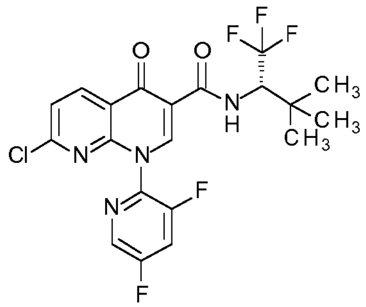

Gemäß allgemeiner Arbeitsvorschrift 1 wurden 600 mg (1.78 mmol) 7-Chlor-1-(3,5-difluorpyridin-2-yl)-4-oxo-1,4-dihydro-1,8-naphthyridin-3-carbonsäure (Beispiel 4B) mit 331 mg (2.13 mmol) (S)-2,2-Dimethyl-l-trifluormethyl-propylamin in Gegenwart von 811 mg (2.13 mmol) HATU und 929 µl (5.33 mmol) DIPEA in 6 ml DMF zur Reaktion gebracht. Es wurde für 5 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 719 mg (85% d. Th., 100% Reinheit) der Titelverbindung erhalten.
LC-MS (Methode 2): Rt = 2.38 min; MS (ESIpos): m/z = 475 [M+H]+.
$^1$H-NMR (400 MHz, DMSO-d6): δ [ppm] = 10.25 (d, 1H), 9.12 (s, 1H), 8.78 (d, 1H), 8.67 (d, 1H), 8.40 (ddd, 1H), 7.78 (d, 1H), 4.67 (quintt, 1H), 1.10 (s, 9H).

**Beispiel 14A**

N-Benzyl-1,1,1,2,2-pentafluorpentan-3-amin (Racemat)

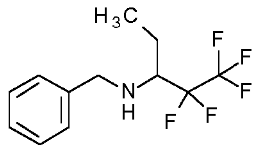

Zu einer Lösung von 2.00 g (11.4 mmol) 1,1,1,2,2-Pentafluorpentan-3-on in 10 ml Dichlormethan wurden bei 0°C 5.03 ml (17.0 mmol) Titantetraisopropoxid und 2.48 ml (22.7 mmol) Benzylamin gegeben. Es wurde 90 min bei RT nachgerührt bevor erneut auf 0°C abgekühlt wurde. Anschließend wurden 2.00 g (31.8 mmol) Natriumcyanoborhydrid, 36 ml Methanol und 3A Molsieb zugegeben. Es wurde auf RT erwärmt und 2d nachgerührt. Die Reaktionslösung wurde anschließend mit wenig Wasser und Essigsäureethylester versetzt und filtriert. Das Filtrat wurde zweimal mit gesättigter wässriger Natriumhydrogencarbonat-Lösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mittels Normalphasenchromatographie (Essigsäureethylester/Cyclohexan 1/20) gereinigt und 989 mg (25% d. Th., 76% Reinheit) der Titelverbindung erhalten.
LC-MS (Methode 1): Rt = 1.27 min; MS (ESIpos): m/z = 268 [M+H]+.
$^1$H-NMR (400 MHz, DMSO-d6): $\delta$ [ppm] = 7.21-7.36 (m, 5H), 3.73-3.85 (m, 2H), 3.05-3.20 (m, 1H), 1.63-1.75 (m, 1H), 1.49-1.61 (m, 1H), 1.15-1.20 (m, 1H), 0.96 (t, 3H).

**Beispiel 14B**

1,1,1,2,2-Pentafluorpentan-3-amin-Hydrochlorid (Racemat)

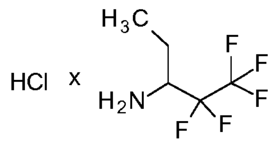

Zu einer Lösung von 980 mg (2.75 mmol, 75% Reinheit) N-Benzyl-1,1,1,2,2-pentafluorpentan-3-amin in 11.3 ml Methanol wurden 75 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Die Vorlage mit dem abdestillierten Lösungsmittel wurde anschließend in einen Kolben überführt und mit 4N Salzsäure in Dioxan versetzt und erneut eingeengt. Der Rückstand wurde mit Ether verrührt, der Niederschlag abgesaugt und im Hochvakuum getrocknet. Es wurden 379 mg (65% d. Th., 100% Reinheit) der Titelverbindung erhalten.
$^1$H-NMR (400 MHz, DMSO-d6): $\delta$ [ppm] = 8.97 (br. s, 3H), 4.16-4.28 (m, 1H), 1.67-1.94 (m, 2H), 1.05 (t, 3H).

**Beispiel 15A**

7-Chlor-1-(3,5-difluorpyridin-2-yl)-4-oxo-N-[1,1,1,2,2-pentafluorpentan-3-yl]-1,4-dihydro-1,8-naphthyridin-3 -carbonsäureamid (Racemat)

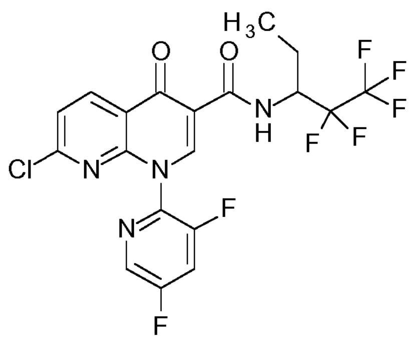

Gemäß allgemeiner Arbeitsvorschrift 1 wurden 200 mg (592 μmol) 7-Chlor-1-(3,5-difluorpyridin-2-yl)-4-oxo-1,4-dihydro-1,8-naphthyridin-3-carbonsäure (Beispiel 4B) mit 152 mg (711 μmol) 1,1,1,2,2-Pentafluorpentan-3-amin-Hydrochlorid in Gegenwart von 270 mg (711 μmol) HATU und 413 μl (2.37 mmol) DIPEA in 2 ml DMF zur Reaktion gebracht. Es wurde für 5 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 248 mg (84% d. Th., 100% Reinheit) der Titelverbindung erhalten.
LC-MS (Methode 2): Rt = 2.32 min; MS (ESIpos): m/z = 497 [M+H]+.
$^1$H-NMR (400 MHz, DMSO-d6): δ [ppm] = 9.93 (d, 1H), 9.11 (s, 1H), 8.75 (d, 1H), 8.66 (d, 1H), 8.40 (ddd, 1H), 7.78 (d, 1H), 4.84-4.97 (m, 1H), 1.88-1.99 (m, 1H), 1.63-1.75 (m, 1H), 0.97 (t, 3H).

**Beispiel 16A**

7-Chlor-1-(3-chlor-5-fluorpyridin-2-yl)-4-oxo-1,4-dihydro-1,8-naphthyridin-3-carbonsäureethylester

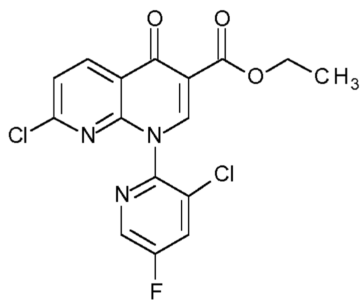

Eine Lösung aus 1.55 g (4.87 mmol) 2-[(2,6-Dichlorpyridin-3-yl)carbonyl]-3-ethoxyacrylsäureethylester (CAS 157373-27-8) und 1.00 g (6.82 mmol) 3-Chlor-5-fluorpyridin-2-amin in 7.7 ml DCM wurde mit 5.94 ml (34.1 mmol) DIPEA versetzt und für 4h bei RT gerührt. Anschließend wurden 674 mg (4.87 mmol) Kaliumcarbonat zugegeben und für 72h unter Rückfluss erhitzt. Die Mischung wurde mit Dichlormethan verdünnt und zweimal mit 1M wässriger Salzsäure, einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mittels Normalphasenchromatographie (Dichlormethan-Essigsäureethylester Gradient) gereinigt und 698 mg (37% d. Th., 100% Reinheit) der Titelverbindung erhalten.
LC-MS (Methode 1): Rt = 0.91 min; MS (ESIpos): m/z = 382 [M+H]+.
$^1$H-NMR (400 MHz, DMSO-d6): δ [ppm] = 8.93 (s, 1H), 8.76 (d, 1H), 8.63 (d, 1H), 8.54 (dd, 1H), 7.67 (d, 1H), 4.25 (q, 2H), 1.28 (t, 3H).

**Beispiel 16B**

7-Chlor-1-(3-chlor-5-fluorpyridin-2-yl)-4-oxo-1,4-dihydro-1,8-naphthyridin-3-carbonsäure

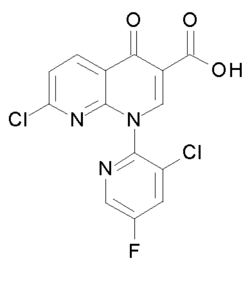

695 mg (1.82 mmol) 7-Chlor-1-(3-chlor-5-fluorpyridin-2-yl)-4-oxo-1,4-dihydro-1,8-naphthyridin-3-carbonsäureethylester wurden in 5.4 ml Wasser vorgelegt, mit 5.4 ml 36 proz. wässriger Salzsäure und 5.4 ml THF versetzt und 4h

bei 120°C nachgerührt. Das Reaktionsgemisch wurde auf RT abgekühlt und mit 36 ml Wasser versetzt. Der Niederschlag wurde abgesaugt, mit Wasser nachgewaschen und am Hochvakuum getrocknet. Es wurden 614 mg (95% d. Th., 100% Reinheit) der Titelverbindung erhalten.
LC-MS (Methode 1): Rt = 0.96 min; MS (ESIpos): m/z = 354 [M+H]+.
$^1$H-NMR (400 MHz, DMSO-d6): δ [ppm] = 13.89 (s, 1H), 9.27 (s, 1H), 8.75-8.81 (m, 3H), 8.56 (dd, 1H), 7.81 (d, 1H).

**Beispiel 16C**

7-Chlor-1-(3-chlor-5-fluorpyridin-2-yl)-4-oxo-N-[(2R)-1,1,1-trifluorbutan-2-yl]-1,4-dihydro-1,8-naphthyridin-3-carbonsäureamid

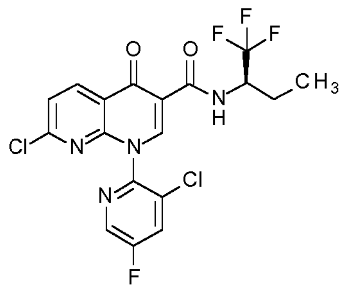

Gemäß allgemeiner Arbeitsvorschrift 1 wurden 150 mg (424 μmol) 7-Chlor-1-(3-chlor-5-fluorpyridin-2-yl)-4-oxo-1,4-dihydro-1,8-naphthyridin-3-carbonsäure (Beispiel 16B) mit 104 mg (635 μmol) (2R)-1,1,1-Trifluorbutan-2-amin-Hydrochlorid in Gegenwart von 193 mg (508 μmol) HATU und 295 μl (1.69 mmol) DIPEA in 2 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 150 mg (77% d. Th., 100% Reinheit) der Titelverbindung erhalten.
LC-MS (Methode 2): Rt = 2.26 min; MS (ESIpos): m/z = 463 [M+H]+.
$^1$H-NMR (400 MHz, DMSO-d6): δ [ppm] = 9.90 (d, 1H), 9.14 (d, 1H), 8.72-8.80 (m, 2H), 8.54 (dd, 1H), 7.77 (d, 1H), 4.72-4.84 (m, 1H), 1.83-1.96 (m, 1H), 1.62-1.74 (m, 1H), 0.98 (t, 3H).

**Beispiel 17A**

7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid

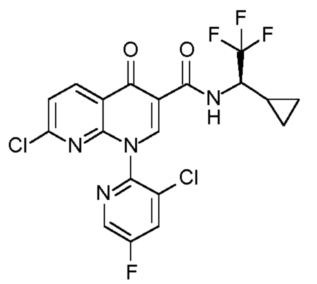

Gemäß allgemeiner Arbeitsvorschrift 1 wurden 150 mg (424 μmol) 7-Chlor-1-(3-chlor-5-fluorpyridin-2-yl)-4-oxo-1,4-dihydro-1,8-naphthyridin-3-carbonsäure (Beispiel 16B) mit 112 mg (635 μmol) (1R)-1-Cyclopropyl-2,2,2-trifluorethanamin-Hydrochlorid in Gegenwart von 193 mg (508 μmol) HATU und 295 μl (1.69 mmol) DIPEA in 2 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 159 mg (79% d. Th., 100% Reinheit)

der Titelverbindung erhalten.
LC-MS (Methode 1): Rt = 1.19 min; MS (ESIpos): m/z = 475 [M+H]+.
$^1$H-NMR (400 MHz, DMSO-d6): δ [ppm] = 10.06 (dd, 1H), 9.14 (d, 1H), 8.72-8.79 (m, 2H), 8.54 (ddd, 1H), 7.77 (d, 1H), 4.33-4.46 (m, 1H), 1.19-1.30 (m, 1H), 0.52-0.71 (m, 3H), 0.29-0.39 (m, 1H).

**Beispiel 18A**

7-Chlor-1-(3-chlorpyridin-2-yl)-4-oxo-1,4-dihydro-1,8-naphthyridin-3-carbonsäureethylester

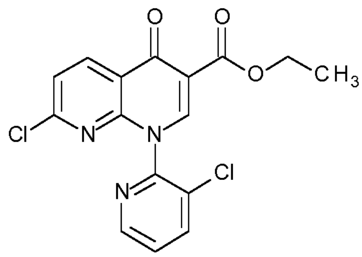

Eine Lösung aus 6.07 g (19.1 mmol) 2-[(2,6-Dichlorpyridin-3-yl)carbonyl]-3-ethoxyacrylsäureethylester (CAS 157373-27-8) und 3.43 g (26.7 mmol) 3-Chlorpyridin-2-amin in 30 ml DCM wurde mit 23.3 ml (134 mmol) DIPEA versetzt und für 4h bei RT gerührt. Anschließend wurden 2.64 g (19.1 mmol) Kaliumcarbonat zugegeben und für 3d unter Rückfluss erhitzt. Die Mischung wurde mit Dichlormethan verdünnt und zweimal mit 1M wässriger Salzsäure, einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde mit Ether verrührt, der Niederschlag abgesaugt, mit Ether nachgewaschen und im Hochvakuum getrocknet. Es wurden 4.14 g (60% d. Th., 100% Reinheit) der Titelverbindung erhalten.
LC-MS (Methode 2): Rt = 1.63 min; MS (ESIpos): m/z = 364 [M+H]+.
$^1$H-NMR (500 MHz, DMSO-d6): δ [ppm] = 8.92 (s, 1H), 8.67 (dd, 1H), 8.63 (d, 1H), 8.33 (dd, 1H), 7.76 (dd, 1H), 7.67 (d, 1H), 4.25 (q, 2H), 1.28 (t, 3H).

**Beispiel 18B**

7-Chlor-1-(3-chlorpyridin-2-yl)-4-oxo-1,4-dihydro-1,8-naphthyridin-3-carbonsäure

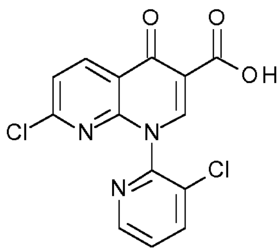

4.09 g (11.2 mmol) 7-Chlor-1-(3-chlorpyridin-2-yl)-4-oxo-1,4-dihydro-1,8-naphthyridin-3-carbonsäureethylester wurden in 33 ml Wasser vorgelegt, mit 33 ml 36 proz. wässriger Salzsäure und 33 ml THF versetzt und 4h bei 120°C nachgerührt. Das Reaktionsgemisch wurde auf RT abgekühlt und mit 220 ml Wasser versetzt. Der Niederschlag wurde abgesaugt, mit Wasser nachgewaschen und am Hochvakuum getrocknet. Es wurden 3.53 g (92% d. Th., 98% Reinheit) der Titelverbindung erhalten.
LC-MS (Methode 1): Rt = 0.90 min; MS (ESIpos): m/z = 336 [M+H]+.
$^1$H-NMR (400 MHz, DMSO-d6): δ [ppm] = 13.92 (br. s., 1H), 9.26 (s, 1H), 8.79 (d, 1H), 8.68 (dd, 1H), 8.34 (dd, 1H), 7.76-7.83 (m, 2H).

**Beispiel 18C**

7-Chlor-1-(3-chlorpyridin-2-yl)-4-oxo-N-[(2R)-1,1,1-trifluorbutan-2-yl]-1,4-dihydro-1,8-naphthyridin-3-carbonsäureamid

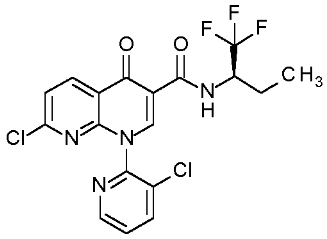

Gemäß allgemeiner Arbeitsvorschrift 1 wurden 800 mg (2.38 mmol) 7-Chlor-1-(3-chlorpyridin-2-yl)-4-oxo-1,4-dihydro-1,8-naphthyridin-3-carbonsäure (Beispiel 18B) mit 584 mg (3.57 mmol) (2R)-1,1,1-Trifluorbutan-2-amin-Hydrochlorid in Gegenwart von 1.09 g (2.86 mmol) HATU und 1.24 ml (7.14 mmol) DIPEA in 12 ml DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 827 mg (78% d. Th., 100% Reinheit) der Titelverbindung erhalten.
LC-MS (Methode 2): Rt = 2.19 min; MS (ESIpos): m/z = 445 [M+H]+.
$^1$H-NMR (400 MHz, DMSO-d6): δ [ppm] = 9.92 (d, 1H), 9.11 (d, 1H), 8.76 (dd, 1H), 8.68 (dt, 1H), 8.34 (dd, 1H), 7.73-7.81 (m, 2H), 4.72-4.84 (m, 1H), 1.83-1.96 (m, 1H), 1.62-1.75 (m, 1H), 0.98 (t, 3H).

**Beispiel 19A**

7-Chlor-1-(3-chlorpyridin-2-yl)-N-[(1R)-1-cyclopropyl-2,2,2-trifluorethyl]-4-oxo-1,4-dihydro-1,8-naphthyridin-3-carbonsäureamid

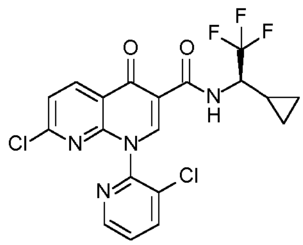

Gemäß allgemeiner Arbeitsvorschrift 1 wurden 150 mg (446 μmol) 7-Chlor-1-(3-chlorpyridin-2-yl)-4-oxo-1,4-dihydro-1,8-naphthyridin-3-carbonsäure (Beispiel 18B) mit 118 mg (669 μmol) (1R)-1-Cyclopropyl-2,2,2-trifluorethanamin-Hydrochlorid in Gegenwart von 204 mg (535 μmol) HATU und 311 μl (1.79 mmol) DIPEA in DMF zur Reaktion gebracht. Es wurde für 10 min nachgerührt und mit Wasser, Acetonitril und Ameisensäure verdünnt. Die Rohlösung wurde über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 167 mg (82% d. Th., 100% Reinheit) der Titelverbindung erhalten.
LC-MS (Methode 1): Rt = 1.15 min; MS (ESIpos): m/z = 457 [M+H]+.
$^1$H-NMR (400 MHz, DMSO-d6): δ [ppm] = 10.07 (dd, 1H), 9.11 (d, 1H), 8.77 (d, 1H), 8.67 (dd, 1H), 8.33 (dt, 1H), 7.73-7.81 (m, 2H), 4.40 (quint, 1H), 1.19-1.31 (m, 1H), 0.52-0.72 (m, 3H), 0.29-0.39 (m, 1H).

**Beispiel 20A**

7-Chlor-1-(3-fluorpyridin-2-yl)-4-oxo-1,4-dihydro-1,8-naphthyridin-3-carbonsäureethylester

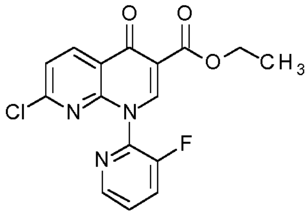

Eine Lösung aus 6.05 g (19.0 mmol) 2-[(2,6-Dichlorpyridin-3-yl)carbonyl]-3-ethoxyacrylsäureethylester (CAS 157373-27-8) und 2.98 g (26.6 mmol, 1.4 Äq.) 3-Fluorpyridin-2-amin in 30 ml DCM wurde mit 23.2 ml (133 mmol) DIPEA versetzt und für 4 h bei RT gerührt. Anschließend wurden 2.63 g (19.0 mmol) Kaliumcarbonat zugegeben und über Nacht unter Rückfluss erhitzt. Die Reaktionsmischung wurde mit 300 ml DCM verdünnt und zweimal mit 75 ml 1M wässriger Salzsäure gewaschen. Die organische Phase wurde über Natriumsulfat getrocknet, filtriert und das Lösungsmittel unter reduziertem Druck entfernt. Die erhaltene Suspension wurde mit 40 ml tert.-Butylmethylether verrührt, der Niederschlag abgesaugt, mit 10 ml tert.-Butylmethylether gewaschen und im Hochvakuum getrocknet. Es wurden 4.51 g (63% d. Th., 92% Reinheit) der Titelverbindung erhalten.
LC-MS (Methode 2): Rt = 1.57 min; MS (ESIpos): m/z = 348 [M+H]+.
$^1$H-NMR (400 MHz, DMSO-d6): $\delta$ [ppm] = 8.90 (s, 1H), 8.63 (d, 1H), 8.55 (d, 1H), 8.16-8.10 (m, 1H), 7.84-7.78 (m, 1H), 7.68 (d, 1H), 4.26 (q, 2H), 1.28 (t, 3H).

**Beispiel 20B**

7-Chlor-1-(3-fluorpyridin-2-yl)-4-oxo-1,4-dihydro-1,8-naphthyridin-3-carbonsäure

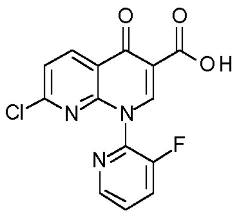

4.51 g (11.9 mmol, 92% Reinheit) 7-Chlor-1-(3-fluorpyridin-2-yl)-4-oxo-1,4-dihydro-1,8-naphthyridin-3-carbonsäureethylester wurden in 36.6 ml Wasser vorgelegt, mit 36.6 ml 36 proz. wässriger Salzsäure und 36.6 ml THF versetzt und 4h bei 120°C nachgerührt. Das Reaktionsgemisch wurde auf RT abgekühlt und mit 100 ml Wasser versetzt. Der Niederschlag wurde abgesaugt und am Hochvakuum getrocknet. Es wurden 3.84 g (98% d. Th., 97% Reinheit) der Titelverbindung erhalten.
LC-MS (Methode 2): Rt = 1.49 min; MS (ESIpos): m/z = 320 [M+H]+.
$^1$H-NMR (400 MHz, DMSO-d6): $\delta$ [ppm] = 13.9 (s, 1H), 9.18 (s, 1H), 8.79 (d, 1H), 8.56 (d, 1H), 8.19-8.12 (m, 1H), 7.87-7.79 (m, 2H).

**Beispiel 20C**

7-Chlor-N-[(1R)-1-cyclopropyl-2,2,2-trifluorethyl]-1-(3-fluorpyridin-2-yl)-4-oxo-1,4-dihydro-1,8-naphthyridin-3 -carbonsäureamid

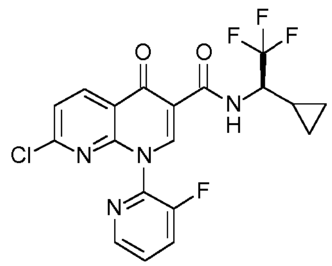

Gemäß allgemeiner Arbeitsvorschrift 1 wurden 150 mg (457 µmol) 7-Chlor-1-(3-fluorpyridin-2-yl)-4-oxo-1,4-dihydro-1,8-naphthyridin-3-carbonsäure (Beispiel 20B) mit 88.3 mg (503 µmol) (1R)-1-Cyclopropyl-2,2,2-trifluorethanamin-Hydrochlorid in Gegenwart von 174 mg (457 µmol) HATU und 239 µl (1.37 mmol) DIPEA in 4.5 ml DMF zur Reaktion gebracht. Nach wässriger Aufarbeitung wurde das Rohprodukt mittels Normalphasenchromatographie (Essigsäureethylester-Cyclohexan-Gradient) gereinigt und 103 mg (51% d. Th., 100% Reinheit) der Titelverbindung erhalten.
LC-MS (Methode 2): Rt = 2.13 min; MS (ESIpos): m/z = 441 [M+H]+.
$^{1}$H-NMR (400 MHz, DMSO-d6): δ [ppm] = 10.06 (d, 1H), 9.09 (s, 1H), 8.76 (d, 1H), 8.56 (d, 1H), 8.17-8.10 (m, 1H), 7.85-7.76 (m, 2H), 4.47-4.35 (m, 1H), 1.29-1.21 (m, 1H), 0.71-0.53 (m, 3H), 0.39-0.30 (m, 1H).

## Beispiel 21A

7-Chlor-1-(3-fluorpyridin-2-yl)-4-oxo-*N*-[(2*R*)-1,1,1-trifluorbutan-2-yl]-1,4-dihydro-1,8-naphthyridin-3-carbonsäureamid

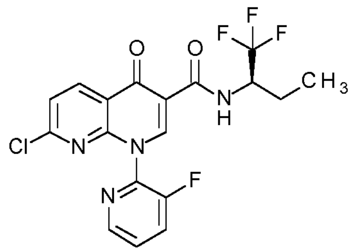

Gemäß allgemeiner Arbeitsvorschrift 1 wurden 1.00 g (3.05 mmol) 7-Chlor-1-(3-fluorpyridin-2-yl)-4-oxo-1,4-dihydro-1,8-naphthyridin-3-carbonsäure (Beispiel 20B) mit 648 mg (3.96 mmol) (2R)-1,1,1-Trifluorbutan-2-amin-Hydrochlorid in Gegenwart von 1.16 g (3.05 mmol) HATU und 1.59 ml (9.14 mmol) DIPEA in 30 ml DMF zur Reaktion gebracht. Nach wässriger Aufarbeitung wurde das Rohprodukt mittels Normalphasenchromatographie (Essigsäureethylester-Cyclohexan-Gradient) gereinigt und 670 mg (51% d. Th., 100% Reinheit) der Titelverbindung erhalten.
LC-MS (Methode 2): Rt = 2.13 min; MS (ESIpos): m/z = 429 [M+H]+.
$^{1}$H-NMR (400 MHz, DMSO-d6): δ [ppm] = 9.90 (d, 1H), 9.10 (s, 1H), 8.76 (d, 1H), 8.56 (d, 1H), 8.17-8.11 (m, 1H), 7.85-7.76 (m, 2H), 4.84-4.72 (m, 2H), 1.96-1.83 (m, 1H), 1.74-1.61 (m, 1H), 0.98 (t, 3H).

## Beispiel 22A

7-Chlor-1-(3,5-difluorpyridin-4-yl)-4-oxo-1,4-dihydro-1,8-naphthyridin-3-carbonsäureethylester

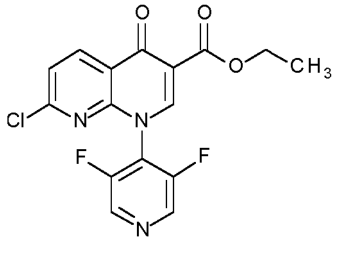

Eine Lösung aus 1.22 g (3.82 mmol) 2-[(2,6-Dichlorpyridin-3-yl)carbonyl]-3-ethoxyacrylsäureethylester (CAS 157373-27-8) und 696 mg (5.35 mmol) 3,5-Difluorpyridin-4-amin in 10 ml Dichlormethan wurde mit 4.7 ml (26.7 mmol) DIPEA versetzt und für 4h bei RT gerührt. Anschließend wurden 528 mg (3.82 mmol) Kaliumcarbonat zugegeben und über Nacht unter Rückfluss erhitzt. Die Mischung wurde mit 100 ml DCM verdünnt und dreimal mit 20 ml 1M wässriger Salzsäure gewaschen und einmal mit gesättigter Natriumchloridlösung ausgeschüttelt. Die organische Phase wurde über Magnesiumsulfat getrocknet, filtriert und das Lösungsmittel unter reduziertem Druck entfernt. Der Rückstand wurde über eine Kieselgelkartusche (Dichlormethan/Methanol) gereinigt. Die Lösemittel wurden im Vakuum verdampft und der Rückstand wurde direkt mittels präp. RP-HPLC gereinigt (Säule: Reprosil 250x30; 10μ, Fluss: 50 mL/min, Acetonitril/Wasser; 0.1% Trifluoressigsäure). Die Lösemittel wurden im Vakuum verdampft und der Rückstand im Hochvakuum getrocknet. Es wurden 203 mg (15% d. Th., 100 % Reinheit) der Titelverbindung erhalten.
LC-MS (Methode 2): Rt = 1.55 min; MS (ESIpos): m/z = 366 [M+H]+.
$^1$H-NMR (400 MHz, DMSO-d6): δ [ppm] = 8.99 (s, 1H), 8.91 (s, 2H), 8.63 (d, 1H), 7.70 (d, 1H), 4.25 (q, 2H), 1.27 (t, 3H).

**Beispiel 22B**

7-Chlor-1-(3,5-difluorpyridin-4-yl)-4-oxo-1,4-dihydro-1,8-naphthyridin-3-carbonsäure

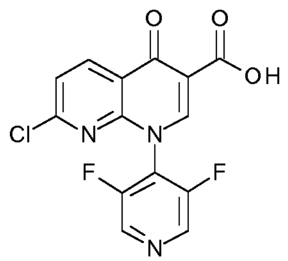

203 mg (555 μmol) 7-Chlor-1-(3,5-difluorpyridin-4-yl)-4-oxo-1,4-dihydro-1,8-naphthyridin-3-carbonsäureethylester wurden in 2.5 ml Wasser vorgelegt, mit 2.5 ml 36 proz. wässriger Salzsäure und 2.5 ml THF versetzt und 2h bei 120°C nachgerührt. Das Reaktionsgemisch wurde auf RT abgekühlt und mit 10 ml Wasser versetzt. Der Niederschlag wurde abgesaugt, mit Wasser nachgewaschen und am Hochvakuum getrocknet. Es wurden 169 mg (90% d. Th., 100% Reinheit) der Titelverbindung erhalten.
LC-MS (Methode 1): Rt = 0.84 min; MS (ESIpos): m/z = 338 [M+H]+.
$^1$H-NMR (400 MHz, DMSO-d6): δ [ppm] = 13.72 (s, 1H), 9.30 (s, 1H), 8.93 (s, 2H), 8.78 (d, 1H), 7.83 (d, 1H).

**Beispiel 22C**

7-Chlor-1-(3,5-difluorpyridin-4-yl)-4-oxo-*N*-[(2*S*)-1,1,1-trifluorbutan-2-yl]-1,4-dihydro-1,8-naphthyridin-3-carbonsäureamid

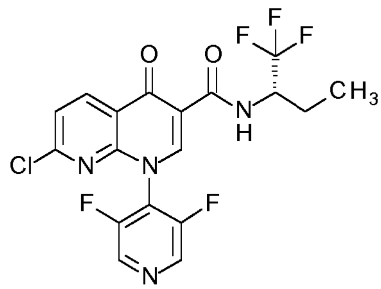

Gemäß allgemeiner Arbeitsvorschrift 1 wurden 147 mg (434 μmol) 7-Chlor-1-(3,5-difluorpyridin-4-yl)-4-oxo-1,4-dihydro-1,8-naphthyridin-3-carbonsäure (Beispiel 22B) mit 82.7 mg (651 μmol) (2S)-1,1,1-Trifluorbutan-2-amin in Gegenwart von 165 mg (434 μmol) HATU und 181 μl (1.04 mmol) DIPEA in 6 ml DMF zur Reaktion gebracht. Der

Ansatz wurde mit Wasser und Acetonitril verdünnt und mittels präparativer HPLC (Säule: Reprosil, 10 µm, 125*30 mm, Lösungsmittel: Wasser, Acetonitril, 0.1% Trifluoressigsäure) gereinigt und 111 mg (57% d. Th., 100% Reinheit) der Titelverbindung erhalten.
LC-MS (Methode 2): Rt = 2.11 min; MS (ESIpos): m/z = 447 [M+H]+.
$^1$H-NMR (400 MHz, DMSO-d6): δ [ppm] = 9.83 (d, 1H), 9.21 (s, 1H), 8.92 (s, 2H), 8.76 (d, 1H), 7.80 (d, 1H), 4.72-4.83 (m, 1H), 1.85-1.95 (m, 1H), 1.62-1.74 (m, 1H), 0.98 (t, 3H).

## Beispiel 23A

1-(3,5-Difluorpyridin-2-yl)-7-[(3*R*,4*R*)-3,4-dihydroxypyrrolidin-1-yl]-4-oxo-1,4-dihydro-1,8-naphthyridin-3 -carbonsäure-ethylester

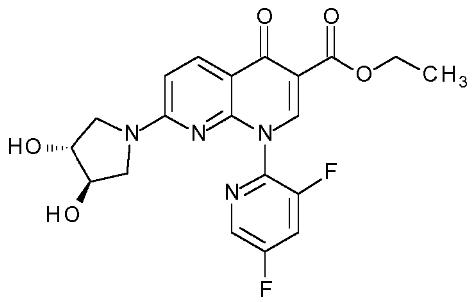

Gemäß allgemeiner Arbeitsvorschrift 3 wurden 1.00 g (2.73 mmol) 7-Chlor-1-(3,5-difluorpyridin-2-yl)-4-oxo-1,4-dihydro-1,8-naphthyridin-3-carbonsäureethylester (Beispiel 7A) mit 458 mg (3.28 mmol) (3R,4R)-Pyrrolidin-3,4-diol-Hydrochlorid in Gegenwart von 1.67 ml (9.57 mmol) DIPEA in 12.3 ml DMF zur Reaktion gebracht. Die Reaktion wurde durch Zugabe von Wasser, Acetonitril und Ameisensäure beendet, durch einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 580 mg (49% d. Th., 100% Reinheit) der Titelverbindung erhalten.
LC-MS (Methode 2): Rt = 1.10 min; MS (ESIpos): m/z = 433 [M+H]+.
$^1$H-NMR (500 MHz, DMSO-d6): δ [ppm] = 8.58-8.63 (m, 2H), 8.32 (td, 1H), 8.18 (d, 1H), 6.67 (d, 1H), 5.21-5.27 (m, 0.5H), 5.17 (d, 1H), 5.02-5.08 (m, 0.5H), 4.21 (q, 2H), 4.01-4.07 (m, 1H), 3.88-3.96 (m, 1H), 3.52-3.64 (m, 1H), 3.16-3.31 (m, 2H), 2.99-3.13 (m, 1H), 1.26 (t, 3H).

## Beispiel 23B

1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure

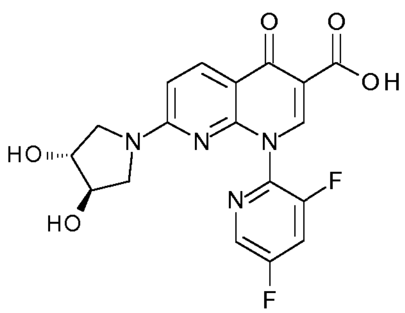

Variante A: 500 mg (1.16 mmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäureethylester wurden in 3.50 ml Wasser vorgelegt, mit 3.50 ml 36 proz. wässriger Salzsäure und 3.50 ml THF versetzt und 3h bei 120°C nachgerührt. Das Reaktionsgemisch wurde auf RT abgekühlt und mit 10 ml Wasser versetzt. Der Niederschlag wurde abgesaugt, mit Wasser nachgewaschen und am Hochvakuum getrocknet. Es wurden 372 mg (80% d. Th., 100% Reinheit) der Titelverbindung erhalten.

LC-MS (Methode 2): Rt = 0.99 min; MS (ESIpos): m/z = 405 [M+H]+.
$^1$H-NMR (500 MHz, DMSO-d6): δ [ppm] = 15.23 (br s, 1H), 8.94 (br d, 1H), 8.63 (br t, 1H), 8.33-8.39 (m, 1H), 8.30 (d, 1H), 6.86 (d, 1H), 4.99-5.41 (m, 2H), 4.06 (br s, 1H), 3.94 (br s, 1H), 3.59-3.69 (m, 1H), 3.36-3.41 (m, 1H), 3.24 (br. dd, 1H), 3.03-3.17 (m, 1H).

Variante B: Alternativ kann die Titelverbindung wie folgt hergestellt werden. Zu einer Lösung von 1.00 g (2.96 mmol) 7-Chlor-1-(3,5-difluorpyridin-2-yl)-4-oxo-1,4-dihydro-1,8-naphthyridin-3-carbonsäure (Beispiel 4B) und 496 mg (3.55 mmol) (3*R*,4*R*)-Pyrrolidin-3,4-diol-Hydrochlorid in 14.2 ml DMF wurde bei RT 1.81 ml (10.4 mmol) DIPEA gegeben. Es wurde über Nacht bei RT nachgerührt. Die Reaktionsmischung wurde auf Wasser, wässrige 1N Salzsäure und Essigsäureethylester gegeben. Der Feststoff wurde abgesaugt und am Hochvakuum getrocknet. Es wurden 1.04 g (87% d. Th., 100% Reinheit) der Titelverbindung erhalten.

**Beispiel 24A**

1-(3,5-Difluorpyridin-2-yl)-7-[(4*S*)-4-hydroxy-2-oxopyrrolidin-1-yl]-4-oxo-1,4-dihydro-1,8-naphthyridin-3-carbonsäure

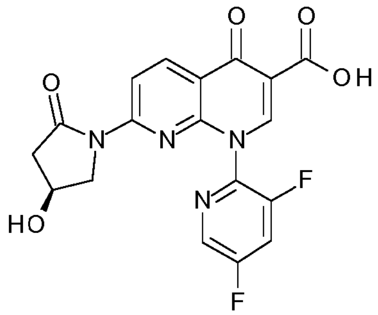

Gemäß allgemeiner Arbeitsvorschrift 2 wurden 1.58 g (4.68 mmol) 7-Chlor-1-(3,5-difluorpyridin-2-yl)-4-oxo-1,4-dihydro-1,8-naphthyridin-3-carbonsäure (Beispiel 4B) mit 568 mg (5.62 mmol) (4S)-4-Hydroxypyrrolidin-2-on in Gegenwart von 970 mg (7.02 mmol) Kaliumcarbonat, 210 mg (0.94 mmol) Palladiumacetat und 541 mg (0.94 mmol) Xantphos in 31.6 ml Dioxan bei 80°C zur Reaktion gebracht. Die Reaktionsmischung wurde mit Acetonitril verdünnt und filtriert. Der Rückstand aus dem Filter wurde mit warmen Acetonitril verrührt und erneut filtriert (Vorgang viermal wiederholt). Die Filtrate wurden vereinigt und fast vollständig eingeengt. Der Niederschlag wurde abgesaugt und im Hochvakuum getrocknet. Es wurden 1.36 g (72% d. Th., 100% Reinheit) der Titelverbindung erhalten.
LC-MS (Methode 2): Rt = 1.15 min; MS (ESIpos): m/z = 403 [M+H]+.
$^1$H-NMR (400 MHz, DMSO-d6): δ [ppm] = 14.42 (br. s., 1H), 9.15 (s, 1H), 8.75 (d, 1H), 8.68 (br. dd, 1H), 8.56-8.63 (m, 1H), 8.42 (td, 1H), 5.24-5.42 (m, 1H), 4.30 (br. s., 1H), 3.63-3.80 (m, 1H), 3.42-3.56 (m, 1H), 2.88-3.05 (m, 1H), 2.39 (br. dd, 1H).

**Beispiel 25A**

1-(3,5-Difluorpyridin-2-yl)-7-[3-(2-hydroxyethyl)-2-oxoimidazolidin-1-yl]-4-oxo-1,4-dihydro-1,8-naphthyridin-3-carbonsäure

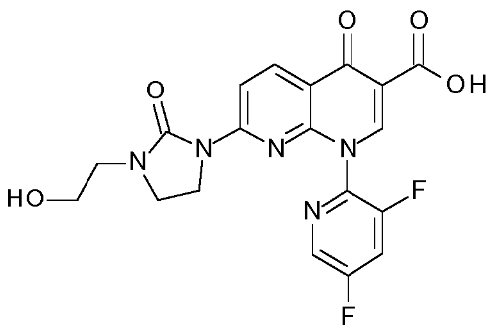

Gemäß allgemeiner Arbeitsvorschrift 2 wurden 250 mg (740 μmol) 7-Chlor-1-(3,5-difluorpyridin-2-yl)-4-oxo-1,4-dihydro-1,8-naphthyridin-3-carbonsäure (Beispiel 4B) mit 217 mg (888 μmol) 1-(2-{[tert.-Butyl(dimethyl)silyl]oxy}ethyl)imidazolidin-2-on (EP 1721905 A1, Ex. 43) in Gegenwart von 256 mg (1.85 mmol) Kaliumcarbonat, 33.2 mg (148 μmol) Palladiumacetat und 85.7 mg (148 μmol) Xantphos in 5 ml Dioxan für 90 min bei 90°C zur Reaktion gebracht. Das Reaktionsgemisch wurde mit 10 ml Dioxan und 10 ml 1N wässriger HCl versetzt und bei 50°C ca. 60 min. nachgerührt. Das Reaktionsgemisch wurde über einen Milliporefilter filtriert und auf Essigsäureethylester und Wasser gegeben. Die Phasen wurden getrennt und die wässrige Phase dreimal mit Essigsäureethylester extrahiert. Die vereinigten organischen Phasen wurden mit gesättigter wässriger Natriumchlorid-Lösung gewaschen, über Natriumsulfat getrocknet und das Lösungsmittel unter reduziertem Druck entfernt. Der Rückstand wurde aus Acetonitril kristallisiert, der Niederschlag abgesaugt, mit etwas kaltem Acetonitril nachgewaschen und am Hochvakuum getrocknet. Es wurden 269 mg (84% d. Th., 100% Reinheit) der Titelverbindung erhalten.
LC-MS (Methode 2): Rt = 1.18 min; MS (ESIpos): m/z = 432 [M+H]+.
$^{1}$H-NMR (400 MHz, DMSO-d6): δ [ppm] = 14.67 (s, 1H), 9.10 (s, 1H), 8.65 (d, 1H), 8.61 (d, 1H), 8.51 (d, 1H), 8.35 (ddd, 1H), 4.75 (t, 1H), 3.43-3.63 (m, 6H), 3.22-3.30 (m, 2H).

## Beispiel 26A

1-(2-{[tert.-Butyl(dimethyl)silyl]oxy}ethyl)tetrahydropyrimidin-2(1H)-on

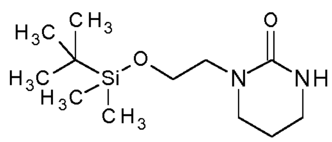

Zu einer Lösung von 600 mg (4.16 mmol) 1-(2-Hydroxyethyl)tetrahydropyrimidin-2(1H)-on (DE 1121617, Chem. Abstr. 1962, 56, 11601g) und 690 mg (4.58 mmol) tert.-Butyl(chlor)dimethylsilan in 4.2 ml DMF wurde bei 0°C 340 mg (4.99 mmol) Imidazol gegeben. Es wurde für 30 min bei 0°C und über Nacht bei RT nachgerührt. Anschließend wurden alle flüchtigen Bestandteile unter reduziertem Druck entfernt und der Rückstand mit 10 ml Wasser versetzt und dreimal mit 20 ml Essigsäureethylester extrahiert. Die vereinigten organischen Phasen wurden mit 30 ml gesättigter wässriger Natriumchlorid-Lösung gewaschen, mit Magnesiumsulfat getrocknet, filtriert und das Lösungsmittel unter reduziertem Druck entfernt. Es wurden 732 mg (68% d. Th., 100% Reinheit) der Titelverbindung erhalten.
LC-MS (Methode 2): Rt = 1.83 min; MS (ESIpos): m/z = 259 [M+H]+.
$^{1}$H-NMR (400 MHz, DMSO-d6): δ [ppm] = 6.11 (s, 1H), 3.63 (t, 2H), 3.30-3.21 (m, 4H), 3.11-3.04 (m, 2H), 1.80-1.72 (m, 2H), 0.86 (s, 9H), 0.03 (s, 6H).

## Beispiel 26B

1-(3,5-Difluorpyridin-2-yl)-7-[3-(2-hydroxyethyl)-2-oxotetrahydropyrimidin-1(2H)-yl]-4-oxo-1,4-dihydro-1,8-naphthyridin-3-carbonsäure

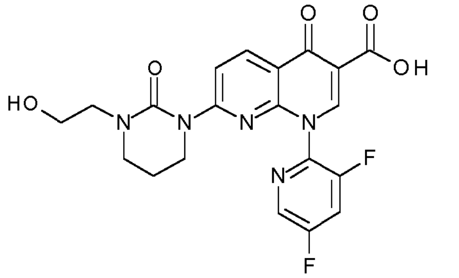

Gemäß allgemeiner Arbeitsvorschrift 2 wurden 250 mg (740 μmol) 7-Chlor-1-(3,5-difluorpyridin-2-yl)-4-oxo-1,4-dihydro-1,8-naphthyridin-3-carbonsäure (Beispiel 4B) mit 230 mg (888 μmol) 1-(2-{[tert.-Butyl(dimethyl)silyl]oxy}ethyl)tetrahydropyrimidin-2(1H)-on in Gegenwart von 256 mg (1.85 mmol) Kaliumcarbonat, 33.2 mg (148 μmol)

Palladiumacetat und 85.7 mg (148 µmol) Xantphos in 5 ml Dioxan für 90 min bei 90°C zur Reaktion gebracht. Das Reaktionsgemisch wurde mit 10 ml Dioxan und 10 ml 1N wässriger HCl versetzt und bei 50°C ca. 60 min. nachgerührt. Das Reaktionsgemisch wurde über einen Milliporefilter filtriert und auf Essigsäureethylester und Wasser gegeben. Die Phasen wurden getrennt und die wässrige Phase dreimal mit Essigsäureethylester extrahiert. Die vereinigten organischen Phasen wurden mit gesättigter wässriger Natriumchlorid-Lösung gewaschen, über Natriumsulfat getrocknet und das Lösungsmittel unter reduziertem Druck entfernt. Der Rückstand wurde aus Acetonitril kristallisiert, der Niederschlag abgesaugt, mit etwas kaltem Acetonitril nachgewaschen und am Hochvakuum getrocknet. Es wurden 219 mg (57% d. Th., 86% Reinheit) der Titelverbindung erhalten.
LC-MS (Methode 2): Rt = 1.20 min; MS (ESIpos): m/z = 446 [M+H]+.
$^1$H-NMR (400 MHz, DMSO-d6): δ [ppm] = 14.62 (s, 1H), 9.12 (s, 1H), 8.66 (d, 1H), 8.54 (d, 1H), 8.34-8.42 (m, 1H), 8.24 (d, 1H), 4.72 (t, 1H), 3.48-3.58 (m, 4H), 3.39 (br t, 4H), 1.85-1.97 (m, 2H).

**Beispiel 27A**

1-(3-Chlorpyridin-2-yl)-7-[(46)-4-hydroxy-2-oxopyrrolidin-1-yl]-4-oxo-1,4-dihydro-1,8-naphthyridin-3-carbonsäure

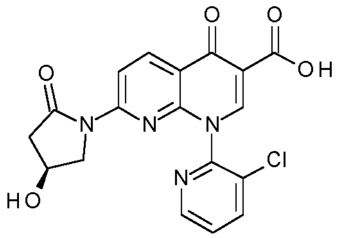

Gemäß allgemeiner Arbeitsvorschrift 2 wurden 1.50 g (4.46 mmol) 7-Chlor-1-(3-chlorpyridin-2-yl)-4-oxo-1,4-dihydro-1,8-naphthyridin-3-carbonsäure (Beispiel 18B) mit 541 mg (5.36 mmol) (4S)-4-Hydroxypyrrolidin-2-on in Gegenwart von 925 mg (6.69 mmol) Kaliumcarbonat, 200 mg (892 µmol) Palladiumacetat und 516 mg (892 µmol) Xantphos in 30 ml Dioxan für 3h bei 80°C zur Reaktion gebracht. Das Reaktionsgemisch wurde mit 1N wässriger Salzsäure versetzt und ausgiebig mit Essigsäureethylester extrahiert. Die vereinigten organischen Phasen wurden mit gesättigter wässriger Natriumchlorid-Lösung gewaschen, über Natriumsulfat getrocknet und das Lösungsmittel unter reduziertem Druck entfernt. Das Rohprodukt in wenig Essigsäureethylester wurde für 1h ruhen gelassen und anschließend der ausgefallene Feststoff abgesaugt und am Hochvakuum getrocknet. Es wurden 1.16 g (52% d. Th., 80% Reinheit) der Titelverbindung erhalten.
LC-MS (Methode 1): Rt = 0.63/0.65 min; MS (ESIpos): m/z = 401 [M+H]+.
$^1$H-NMR (400 MHz, DMSO-d6): δ [ppm] = 14.50 (br. s, 1H), 9.23 (br. d, 1H), 8.76 (d, 1H), 8.68 (ddd, 1H), 8.59 (dd, 1H), 8.35 (dt, 1H), 7.79 (ddd, 1H), 5.25-5.40 (m, 1H), 4.20-4.30 (m, 1H), 3.51-3.68 (m, 1H), 3.32-3.45 (m, 1H), 2.87-3.02 (m, 1H), 2.37 (dd, 1H).

**Beispiel 28A**

*N*-Benzyl-1,1,1,2,2-pentafluorbutan-3-amin (Racemat)

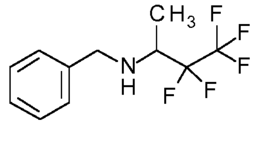

Zu einer Lösung von 2.00 g (12.2 mmol) 3,3,4,4,4-Pentafluorobutan-2-on in 10 ml Dichlormethan wurden bei 0°C 5.40 ml (18.3 mmol) Titantetraisopropoxid und 2.66 ml (24.4 mmol) Benzylamin gegeben. Es wurde 90 min bei RT nachgerührt bevor erneut auf 0°C abgekühlt wurde. Anschließend wurde 2.14 g (34.1 mmol) Natriumcyanoborhydrid, 36 ml Methanol und 3A Molsieb zugegeben. Es wurde auf RT erwärmt und 2d nachgerührt. Die Reaktionslösung wurde wenig mit Wasser und Essigsäureethylester versetzt und filtriert. Das Filtrat wurde zweimal mit gesättigter wässriger

NatriumhydrogencarbonatLösung und einmal mit gesättigter wässriger Natriumchlorid-Lösung gewaschen. Die organische Phase wurde über Natriumsulfat getrocknet, filtriert und das Lösungsmittel unter reduziertem Druck entfernt. Der Rückstand wurde zweimal mittels Normalphasenchromatographie (Essigsäureethylester/Cyclohexan 1/20) gereinigt und 1.65 g (48% d. Th., 91% Reinheit) der Titelverbindung erhalten.
LC-MS (Methode 5): Rt = 2.17 min; MS (ESIpos): m/z = 254 [M+H]+.
$^1$H-NMR (500 MHz, DMSO-d6): δ [ppm] = 7.28-7.36 (m, 4H), 7.20-7.27 (m, 1H), 3.83 (dd, 1H), 3.72 (dd, 1H), 3.22-3.30 (m, 1H), 2.43-2.48 (m, 1H), 1.20 (d, 3H).

**Beispiel 28B**

1,1,1,2,2-Pentafluorbutan-3 -amin-Hydrochlorid (Racemat)

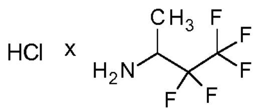

Zu einer Lösung von 1.50 g (5.92 mmol) *N*-Benzyl-1,1,1,2,2-pentafluorpentan-3-amin in 27.4 ml Methanol wurden 150 mg Palladium auf Kohle (10%) gegeben und für 6h bei Normaldruck und Raumtemperatur hydriert. Anschließend wurde die Reaktionsmischung durch einen Milliporefilter filtriert und das Lösungsmittel unter reduziertem Druck entfernt. Die Vorlage mit dem abdestillierten Lösungsmittel wurde anschließend in einen Kolben überführt und mit 4N Salzsäure in Dioxan versetzt und erneut eingeengt. Der Rückstand wurde mit Ether verrührt, der Niederschlag abgesaugt und im Hochvakuum getrocknet. Es wurden 456 mg (39% d. Th., 100% Reinheit) der Titelverbindung erhalten.
$^1$H-NMR (500 MHz, DMSO-d6): δ [ppm] = 9.21 (br. s, 3H), 4.40-4.29 (m, 1H), 1.41 (d, 3H).

**Beispiel 29A**

7-Chlor-1-(3,5-difluorpyridin-2-yl)-*N*-(1,1,1,3,3,3-hexafluor-2-methylpropan-2-yl)-4-oxo-1,4-dihydro-1,8-naphthyridin-3-carbonsäureamid

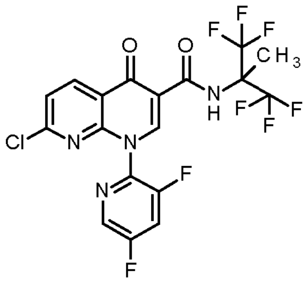

Zu einer Lösung aus 90.0 mg (267 μmol) 7-Chlor-1-(3,5-difluorpyridin-2-yl)-4-oxo-1,4-dihydro-1,8-naphthyridin-3-carbonsäure (Beispiel 7B), 58.1 mg (321 μmol) 1,1,1,3,3,3-Hexafluor-2-methylpropan-2-amin und 0.14 ml (0.80 mmol) DIPEA in 1 ml Essigsäureethylester wurden 0.47 ml (0.80 mmol) 2,4,6-Tripropyl-1,3,5,2,4,6-trioxatriphosphorinan-2,4,6-trioxid (T3P, 50% in DMF) zugetropft. Es wurde über das Wochenende bei 80°C nachgerührt. Die Reaktionslösung wurde eingeengt und der Rückstand in wenig Wasser, Acetonitril und Ameisensäure gelöst, über einen Milliporefilter filtriert und mittels präparativer HPLC (Acetonitril / Wasser/ 0.1% Ameisensäure) gereinigt. Es wurden 23.1 mg (17% d. Th., 100% Reinheit) der Titelverbindung erhalten.
LC-MS (Methode 2): $R_t$ = 2.35 min; MS (ESIpos): m/z = 501 [M+H]+.
$^1$H NMR (400 MHz, DMSO-d6): δ [ppm] = 10.97 (s, 1H), 9.13 (s, 1H), 8.79 (d, 1H), 8.67 (d, 1H), 8.44-8.37 (m, 1H), 7.79 (d, 1H), 2.08 (s, 1H).

**Beispiel 30A**

1-(3,5-Difluorpyridin-2-yl)-4-oxo-7-(2-oxoimidazolidin-1-yl)-1,4-dihydro-1,8-naphthyridin-3-carbonsäure

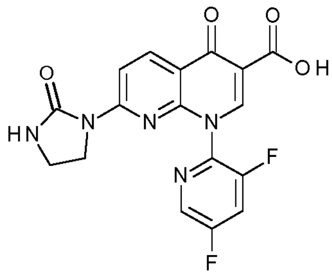

Gemäß allgemeiner Arbeitsvorschrift 2 wurden 800 mg (2.37 mmol) 7-Chlor-1-(3,5-difluorpyridin-2-yl)-4-oxo-1,4-dihydro-1,8-naphthyridin-3-carbonsäure (Beispiel 4B) mit 2.04 g (23.7 mmol) Imidazolidin-2-on in Gegenwart von 491 mg (3.55 mmol) Kaliumcarbonat, 106 mg (474 μmol) Palladiumacetat und 274 mg (474 μmol) Xantphos in 21.1 ml Dioxan über Nacht bei 80°C zur Reaktion gebracht. Anschließend wurde Dioxan abrotiert und zur Reaktionslösung Acetonitril gegeben. Es wurde über einen Filter filtriert. Das Filtrat wurde aufbewahrt, der Rückstand aus dem Filter in Acetonitril warm verrührt und erneut abfiltriert. Dieses Vorgehen wurde zweimal wiederholt. Es wurden 805 mg (68% d. Th., 78% Reinheit) der Titelverbindung erhalten.
LC-MS (Methode 2): Rt = 1.21 min; MS (ESIpos): m/z = 388 [M+H]+.

**Beispiel 31A**

7-({(2*R*)-2-[(*tert*.-Butoxycarbonyl)amino]propyl}amino)-1-(3,5-difluorpyridin-2-yl)-4-oxo-1,4-dihydro-1,8-naphthyridin-3-carbonsäureethylester

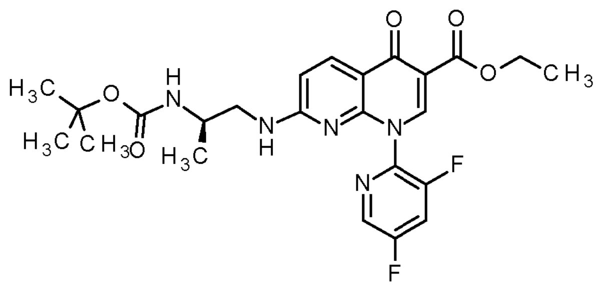

Die Verbindung aus Beispiel 4A (1.89 g, 5.17 mmol) wurde in 33 ml DMF vorgelegt, mit *tert*.-Butyl-[(2*R*)-1-aminopropan-2-yl]carbamat Hydrochlorid (1.31 g, 6.20 mmol) und *N,N*-Diisopropylethylamin (3.2 ml, 18 mmol) versetzt und bei 60°C über Nacht gerührt. Die Reaktionslösung wurde auf ca. 300 ml Wasser geben und mit 1 N Salzsäure auf pH = 5 gestellt. Der Niederschlag wurde abfiltriert, mit Wasser gewaschen und am Hochvakuum getrocknet. Das Rohprodukt wurde mittels Kieselgel gereinigt (Laufmittel: Cyclohexan/Ethylacetat = 2/1, anschließend Dichlormethan/Methanol = 20/1). Es wurden 1.58 g der Zielverbindung (53% d.Th., Reiheit 93%) erhalten.
LC-MS (Methode 2): $R_t$ = 1.70 min; MS (ESIpos): m/z = 504 [M+H]+

**Beispiel 31B**

7-{[(2*R*)-2-Aminopropyl]amino}-1-(3,5-difluorpyridin-2-yl)-4-oxo-1,4-dihydro-1,8-naphthyridin-3-carbonsäureethylester Trifluoracetat

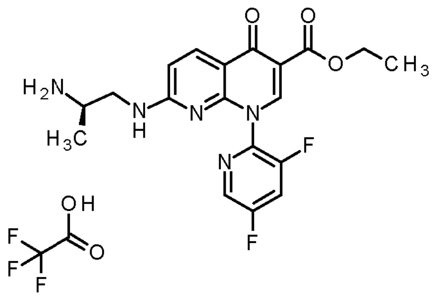

Die Verbindung aus Beispiel 31A (1.38 g, 2.74 mmol) wurde in Dichlormethan (80 ml) vorgelegt, unter Eiskühlung mit Trifluoressigsäure (4.2 ml, 55 mmol) versetzt und 4.5 h bei Raumtemperatur gerührt. Das Reaktionsgemisch wurde einrotiert und zweimal mit Toluol versetzt, eingedampft und anschließend im Hochvakuum getrocknet. Es wurden 2.18 g der Zielverbindung (88% d. Th., Reinheit 57%) erhalten. Das Rohprodukt wurde ohne weitere Aufreinigung weiter umgesetzt.
LC-MS (Methode 2): $R_t$ = 0.80 min; MS (ESIpos): m/z = 404 [M+H]+

**Beispiel 31C**

1-(3,5-Difluorpyridin-2-yl)-7-[(4*R*)-4-methyl-2-oxoimidazolidin-1-yl]-4-oxo-1,4-dihydro-1,8-naphthyridin-3-carbonsäure-ethylester

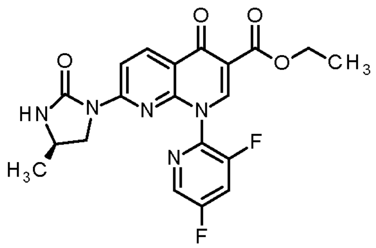

Unter Argon wurde die Verbindung aus Beispiel 31B (2.50 g, 57 % Reinheit, 2.75 mmol) in DMF (61 ml) gelöst, mit Kaliumcarbonat (381 mg, 2.75 mmol) und 1,1'-Carbonyldiimidazol (1.12 g, 6.89 mmol) versetzt und für 1.5 h bei Raumtemperatur gerührt. Die Reaktionslösung auf ca. 600 ml Wasser geben, mit 1 N Salzsäure sauer gestellt und anschließend dreimal mit Ethylacetat extrahiert. Die vereinigten organischen Phasen wurden einmal mit Wasser gewaschen, anschließend über Natriumsulfat getrocknet, filtriert und einrotiert. Es wurden 960 mg der Zielverbindung (70% d. Th., Reinheit 87%) erhalten.
LC-MS (Methode 2): $R_t$ = 1.38 min; MS (ESIpos): m/z = 430 [M+H]+
$^1$H-NMR (400 MHz, DMSO-$d_6$) $\delta$ [ppm]: 0.008 (1.58), 1.090 (1.23), 1.102 (1.26), 1.147 (1.29), 1.159 (1.19), 1.242 (0.90), 1.255 (3.35), 1.273 (6.25), 1.291 (2.93), 1.908 (0.48), 2.731 (13.52), 2.891 (16.00), 2.933 (0.66), 3.060 (0.42), 3.716 (0.82), 3.755 (0.71), 3.770 (0.60), 4.205 (0.65), 4.214 (1.15), 4.223 (0.87), 4.231 (2.82), 4.249 (2.63), 4.267 (0.80), 7.762 (1.77), 7.952 (2.11), 8.332 (1.63), 8.354 (2.25), 8.430 (2.46), 8.452 (1.55), 8.603 (0.53), 8.612 (0.92), 8.629 (1.49), 8.767 (2.11).

**Beispiel 31D**

1-(3,5-Difluorpyridin-2-yl)-7-[(4*R*)-4-methyl-2-oxoimidazolidin-1-yl]-4-oxo-1,4-dihydro-1,8-naphthyridin-3 -carbonsäure

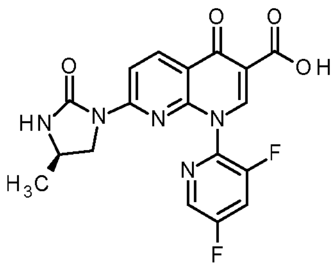

Die Verbindung aus Beispiel 31C (960 mg, 87 % Reinheit, 1.95 mmol) wurde in 5.9 ml THF suspendiert. Es wurden 5.9 ml Wasser und 18 ml konz. Salzsäure zugeben und für 3 h bei 110°C Badtemperatur gerührt. Der erhaltene Feststoff wurde nach dem Abkühlen abfiltriert, mit Wasser gewaschen und im Hochvakuum getrocknet. Es wurden 420 mg der Zielverbindung (54% d. Th., Reinheit 100%) erhalten.
LC-MS (Methode 1): $R_t$ = 0.73 min; MS (ESIpos): m/z = 402 [M+H]+
$^1$H-NMR (500 MHz, DMSO-$d_6$) δ [ppm]: -0.007 (1.58), 0.006 (1.20), 1.104 (4.83), 1.114 (4.85), 1.160 (5.01), 1.170 (4.83), 1.232 (0.69), 2.516 (1.88), 2.520 (1.64), 2.524 (1.25), 3.090 (1.26), 3.101 (1.27), 3.122 (0.49), 3.149 (1.26), 3.160 (1.27), 3.706 (0.56), 3.723 (1.92), 3.735 (2.25), 3.785 (2.31), 3.799 (1.78), 7.906 (6.55), 8.337 (0.72), 8.354 (1.27), 8.374 (1.08), 8.395 (1.21), 8.411 (0.66), 8.493 (3.64), 8.511 (5.36), 8.582 (16.00), 8.600 (10.62), 8.654 (5.90), 9.089 (5.35), 9.095 (5.25), 14.664 (1.73).

**Beispiel 32A**

7-Chlor-1-(3,5-difluorpyridin-2-yl)-4-oxo-N-(3,3,4,4,4-pentafluor-2-methylbutan-2-yl)-1,4-dihydro-1,8-naphthyridin-3-carboxamid

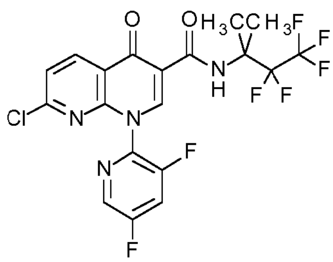

7-Chlor-1-(3,5-difluorpyridin-2-yl)-4-oxo-1,4-dihydro-1,8-naphthyridin-3-carbonsäure (800 mg, 95 % Reinheit, 2.25 mmol), 3,3,4,4,4-Pentafluor-2-methylbutan-2-aminhydrochlorid (1:1) (529 mg, 2.48 mmol) und *N*,*N*-Diisopropylethylamin (1.6 ml, 9.0 mmol) wurden in 23 ml Essigsäureethylacetat vorgelegt, T3P (50% in Essigsäurethylester, 2,4,6-Tripropyl-1,3,5,2,4,6-trioxatriphosphorinane-2,4,6-trioxide solution) (5.3 ml, 50 % Reinheit, 9.0 mmol) wurde hinzugefügt und über Nacht bei 80°C nachgerührt. Das Reaktionsgemisch wurde auf Wasser und Essigsäureethylester gegeben. Die Phasen wurden getrennt. Die wässrige Phase wurde noch dreimal mit Essigsäureethylester extrahiert. Die vereinigten organsichen Phasen wurden mit gesättigter, wässriger Natriumchloridlösung gewaschen, über Natriumsulfat getrocknet, filtriert und eingedampft. Der Rückstand wurde mittels Kieselgelchromatographie (Laufmittel: Cyclohexan/Essigsäureethylester 3:1) getrennt. Die produkthaltigen Fraktionen wurden vereinigt und eingedampft. Der Rückstand wurde am Hochvakuum getrocknet. Es wurden 1.1g der Zielverbindung (98 % d. Th., Reinheit 100%) erhalten.
LC-MS (Methode3): $R_t$ = 1.55 min; MS (ESIpos): m/z = 497 $[M+H]^+$
$^1$H-NMR (400 MHz, DMSO-d6) δ [ppm]: -0.008 (1.91), 0.008 (1.83), 1.157 (0.54), 1.175 (1.10), 1.193 (0.56), 1.398 (0.53), 1.703 (16.00), 1.989 (2.02), 4.021 (0.48), 4.039 (0.48), 7.757 (4.23), 7.778 (4.44), 8.372 (0.78), 8.378 (0.89), 8.395 (1.19), 8.399 (1.29), 8.416 (0.80), 8.422 (0.89), 8.661 (3.86), 8.667 (3.65), 8.745 (4.39), 8.767 (4.21), 9.066 (7.38), 10.122 (4.04).

**Beispiel 33A**

3,3,4,4,4-Pentafluor-N-[(1S)-1-phenylethyl]butan-2-imin

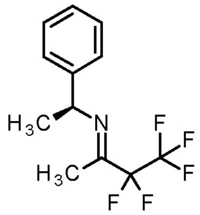

3,3,4,4,4-Pentafluorbutan-2-on (200 g, 1.23 mol) wurde in 6.4 L in Diethylether vorgelegt und auf -40°C abgekühlt. Dann wurden zügig (1S)-1-Phenylethanamin (160 ml, 1.2 mol) und Triethylamin (340 ml, 2.5 mol) zugesetzt und bei einer Innentemperatur von 0°C wurde anschließend langsam Titan(IV)chlorid (1 M in Toluol, 620 ml, 620 mmol) zugetropft. Anschließend wurde das Eisbad entfernt und das Gemisch wurde auf RT erwärmt. Anschließend wurde das Reaktionsgemisch für 1 h unter Rückfluss erhitzt und dann über Nacht bei RT nachgerührt. Das Reaktionsgemisch wurde mit Celite versetzt, 1 h nachgerührt, anschließend über Celite filtriert und gut mit Diethylether nachgewaschen. Das Filtrat wurde bei 25°C Wasserbadtemperatur eingedampft. Der Rückstand wurde mit Cyclohexan versetzt und erneut über Celite abfiltrierrt und mit Cyclohexan gewaschen. Das Filtrat wurde bei 25°C Wasserbadtemperatur eingedampft. Das Rohprodukt wurde ohne weitere Reinigung in die Folgestufe eingesetzt. Es wurden 289 g (88% d. Th.) der Titelverbindung erhalten.

**Beispiel 34A**

3,3,4,4,4-Pentafluor-N-[(1S)-1-phenylethyl]butan-2-amin-Hydrochlorid (enantiomerenrein)

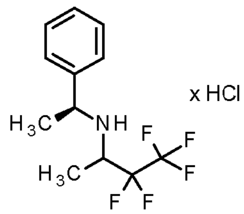

3,3,4,4,4-Pentafluor-N-[(1S)-1-phenylethyl]butan-2-imin (239 g, 901 mmol) wurde in 1.9 l Dichlormethan vorgelegt, dann wurden 420 ml DMF und Molsieb 3Å zugegeben und das Gemisch wurde 1 h bei RT gerührt. Das Reaktionsgemisch wurde dann auf -50°C abgekühlt und es wurde langsam Trichlorsilan (270 ml, 2.7 mol) zugetropft. Nach 30 min und bei einer Innentemperatur von -70°C bis -50°C wurde vorsichtig mit halbkonzentrierter Natriumhydroxid-Lösung gequencht, bis pH7 erreicht war. Es wurde Dichlormethan zugesetzt und die Phasen wurden getrennt. Die organische Phase wurde über Natriumsulfat getrocknet und anschließend wurden 2.2 l Chlorwasserstoff in Diethylether (2 M Lösung) zugesetzt und das Rohprodukt wurde im Vakuum eingedampft. Es wurden 192 g (70% d. Th.) der Titelverbindung erhalten.
LC-MS (Methode 1): $R_t$ = 1.22 min; MS (ESIpos): m/z = 268 $[M-HCl+H]^+$

**Beispiel 35A**

3,3,4,4,4-Pentafluorbutan-2-amin-Hydrochlorid (enantiomerenrein)

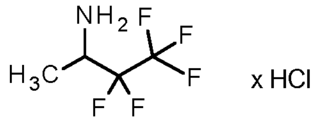

192 g (632 mmol) 3,3,4,4,4-Pentafluor-N-[(1S)-1-phenylethyl]butan-2-amin-Hydrochlorid (enantiomerenrein, aus Beispiel 34A) wurden in 1.2 l Ethanol gelöst und mit 19.2 g Palladium(II)hydroxid (20% auf Kohle) versetzt und anschließend über Nacht bei RT und Normaldruck hydriert. Der Niederschlag wurde abfiltriert, gut nachgewaschen und das Filtrat wurde vorsichtig eingedampft. Es wurden 117 g (93% d. Th.) der Titelverbindung erhalten.
$^1$H NMR (400 MHz, DMSO-d6): δ [ppm] = 9.29 (br. s, 3H), 4.22-4.44 (m, 1H), 1.42 (d, H).

**Beispiel 36A**

7-Chlor-1-(3,5-difluorpyridin-2-yl)-4-oxo-N-(3,3,4,4,4-pentafluorbutan-2-yl)-1,4-dihydro-1,8-naphthyridin-3-carboxamid(Enantiomerenrein)

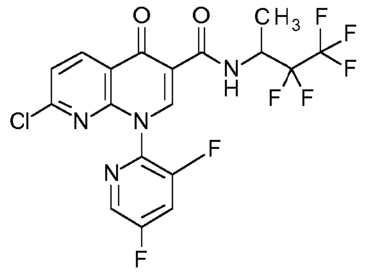

7-Chlor-1-(3,5-difluorpyridin-2-yl)-4-oxo-1,4-dihydro-1,8-naphthyridin-3-carbonsäure (800 mg, 95 % Reinheit, 2.25 mmol), 3,3,4,4,4-Pentafluorbutan-2-amin-Hydrochlorid (enantiomerenrein) und N,N-Diisopropylethylamin (1.6 ml, 9.0 mmol) wurden in 22 ml Essigsäureethylacetat vorgelegt, T3P (50% in Essigsäurethylester) 2,4,6-Tripropyl-1,3,5,2,4,6-trioxatriphosphorinane-2,4,6-trioxide solution (5.3 ml, 50 % Reinheit, 9.0 mmol) wurde hinzugefügt und über Nacht bei 80°C nachgerührt. Das Reaktionsgemisch wurde auf Wasser und Essigsäureethylester gegeben. Die Phasen wurden getrennt. Die wässrige Phase wurde noch dreimal mit Essigsäureethylester extrahiert. Die vereinigten organsichen Phasen wurden mit gesättigter, wässriger Natriumchloridlösung gewaschen, über Natriumsulfat getrocknet, filtriert und eingedampft. Der Rückstand wurde mittels Kieselgelchromatographie (Laufmittel: Cyclohexan/Essigsäureethylester 3:1) getrennt. Die produkthaltigen Fraktionen wurden vereinigtund eingedampft. Der Rückstand wurde am Hochvakuum getrocknet. Es wurden 1.1 g der Zielverbindung (100% d. Th., Reinheit 100%) erhalten.
LC-MS (Methode 3): $R_t$ = 1.50 min; MS (ESIpos): m/z = 483 $[M+H]^+$
$^1$H-NMR (400 MHz, DMSO-d6) δ [ppm]: -0.149 (0.75), -0.008 (9.15), 0.008 (5.98), 0.146 (0.74), 1.157 (1.80), 1.175 (3.54), 1.193 (1.86), 1.398 (4.14), 1.411 (12.90), 1.429 (12.48), 1.988 (6.38), 2.328 (0.75), 2.366 (0.74), 2.524 (3.41), 2.670 (0.83), 2.710 (0.77), 4.003 (0.52), 4.021 (1.51), 4.039 (1.49), 4.056 (0.50), 5.009 (0.77), 5.030 (1.24), 5.053 (1.49), 5.073 (1.45), 5.096 (1.20), 5.116 (0.66), 7.767 (11.03), 7.788 (11.45), 8.373 (1.93), 8.380 (2.11), 8.400 (3.33), 8.418 (1.88), 8.424 (1.93), 8.664 (9.19), 8.670 (8.42), 8.737 (12.44), 8.758 (11.90), 9.106 (16.00), 9.998 (5.24), 10.022 (4.95).

**Beispiel 37A**

7-Chlor-1-(3,5-difluorpyridin-2-yl)-N-(1,1,1,3,3,3-hexafluorpropan-2-yl)-4-oxo-1,4-dihydro-1,8-naphthyridin-3-carboxamid

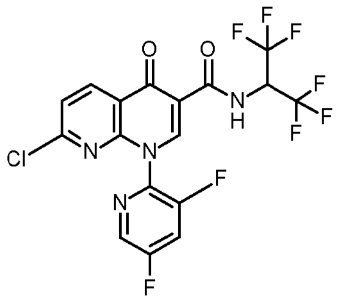

7-Chlor-1-(3,5-difluorpyridin-2-yl)-4-oxo-1,4-dihydro-1,8-naphthyridin-3-carbonsäure (800 mg, 2.37 mmol), 1,1,1,3,3,3-Hexafluorpropan-2-amin (435 mg, 2.61 mmol) und *N*,*N*-Diisopropylethylamin (1.2 ml, 7.1 mmol) wurden in 21 ml Essigsäureethylacetat vorgelegt, T3P (50% in Essigsäurethylester) 2,4,6-Tripropyl-1,3,5,2,4,6-trioxatriphosphorinane-2,4,6-trioxide solution (5.5 ml, 50 % Reinheit, 9.5 mmol) wurde hinzugefügt und über Nacht bei 80°C nachgerührt. Es wurden nochmals 1,1,1,3,3,3-Hexafluorpropan-2-amin (79 mg, 475 μmol) hinzugefügt und über Nacht bei 80°C nachgerührt. Das Reaktionsgemisch wurde auf Wasser und Essigsäureethylester gegeben. Die Phasen wurden getrennt. Die wässrige Phase wurde noch dreimal mit Essigsäureethylester extrahiert. Die vereinigten organsichen Phasen wurden mit gesättigter, wässriger Natriumchloridlösung gewaschen, über Natriumsulfat getrocknet, filtriert und eingedampft. Der Rückstand wurde mittels Kieselgelchromatographie (Laufmittel: Cyclohexan/Essigsäureethylester 3:1) getrennt. Die produkthaltigen Fraktionen wurden vereinigt undeingedampft.Die Substanz wurde aus Acetonitril umkristallisiert, der Feststoff abgesaugt und mit Acetonitril nachgewaschen und am Hochvakuum getrocknet. Es wurden 495 mg der Zielverbindung (43% d. Th., Reinheit 99%) erhalten.
LC-MS (§MCW-FT-MS-M1): $R_t$ = 2.28 min; MS (ESIpos): m/z = 487 $[M+H]^+$
$^1$H-NMR (400 MHz, DMSO-d6) δ [ppm]: -0.149 (0.46), -0.008 (4.04), 0.146 (0.49), 2.328 (0.57), 2.670 (0.62), 6.378 (0.45), 6.397 (1.11), 6.415 (1.54), 6.440 (1.62), 6.458 (1.07), 6.477 (0.41), 7.797 (9.34), 7.817 (9.78), 8.387 (1.78), 8.393 (2.08), 8.410 (2.89), 8.414 (3.15), 8.431 (1.88), 8.437 (2.04), 8.678 (8.72), 8.684 (8.40), 8.767 (9.84), 8.789 (9.45), 9.192 (16.00), 10.761 (4.75), 10.786 (4.59).

## Beipiel 38A

1-(3,5-Difluorpyridin-2-yl)-7-(2-oxa-6-azaspiro[3.3]hept-6-yl)-4-oxo-1,4-dihydro-1,8-naphthyridin-3-carbonsäure

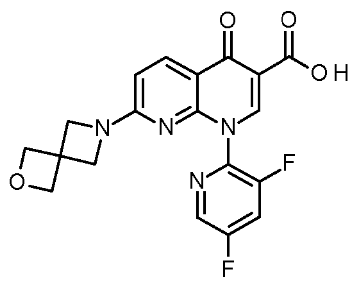

7-Chlor-1-(3,5-difluorpyridin-2-yl)-4-oxo-1,4-dihydro-1,8-naphthyridin-3-carbonsäure (300 mg, 888 μmol) wurde in 4.8 ml DMF vorgelegt, mit N,N-Diisopropylethylamin (1.5 ml, 8.9 mmol) und Ethandisäure-2-oxa-6-azaspiro[3.3]heptan (1:2) (192 mg, 666 μmol) bei Raumtemperatur versetzt. Die Reaktionslösung wurde über Nacht bei Raumtemperatur gerührt. Die Reaktionslösung wurde auf Wasser gegeben, mit 1 N Salzsäure angesäuert und der ausgefallene Niederschalg kurz ausgerührt. Der Feststoff wurde abfiltriert und im Hochvakuum getrocknet. Es wurden 302 mg der Zielverbindung (83% d. Th., Reinheit 98%) erhalten.
LC-MS (Methode 2): $R_t$ = 1.36 min; MS (ESIpos): m/z = 401 $[M+H]^+$
$^1$H-NMR (400 MHz, DMSO-d6) δ [ppm]: -0.008 (2.52), 0.008 (2.29), 2.524 (1.17), 3.994 (0.50), 4.308 (0.49), 4.663 (16.00), 6.659 (5.39), 6.681 (5.50), 8.288 (5.50), 8.310 (5.36), 8.318 (1.21), 8.324 (1.28), 8.341 (1.71), 8.345 (1.82), 8.362 (1.12), 8.369 (1.22), 8.619 (5.31), 8.626 (5.15), 8.932 (9.50), 15.150 (9.52).

**AUSFÜHRUNGSBEISPIELE:**

**Beispiel 1**

1-(3,5-Difluorpyridin-2-yl)-7-[(4*S*)-4-hydroxy-2-oxopyrrolidin-1-yl]-4-oxo-*N*-[(2*R*)-1,1,1-trifluorbutan-2-yl]-1,4-dihydro-1,8-naphthyridin-3-carbonsäureamid

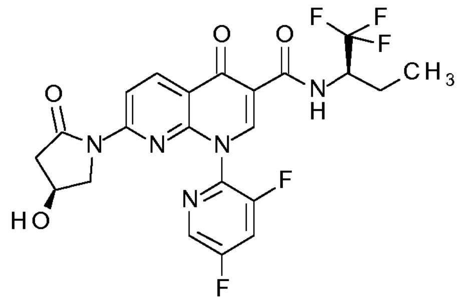

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 45.7 mg (280 μmol) (2R)-1,1,1-Trifluorbutan-2-amin-Hydrochlorid in Gegenwart von 85.1 mg (224 μmol) HATU und 0.13 ml (0.75 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 66.5 mg (70% d. Th., 100% Reinheit) der Titelverbindung erhalten.
LC-MS (Methode 2): Rt = 1.74 min; MS (ESIpos): m/z = 512 [M+H]+.
$^1$H-NMR (400 MHz, DMSO-d6): δ [ppm] = 10.12 (d, 1H), 9.06 (s, 1H), 8.71 (d, 1H), 8.67 (br. s, 1H), 8.54 (br. t, 1H), 8.39 (td, 1H), 5.21-5.40 (m, 1H), 4.70-4.84 (m, 1H), 4.29 (br. s., 1H), 3.64-3.80 (m, 1H), 3.44-3.58 (m, 1H), 2.87-3.03 (m, 1H), 2.32-2.43 (m, 1H), 1.83-1.96 (m, 1H), 1.59-1.73 (m, 1H), 0.93-1.03 (m, 3H).

**Beispiel 2**

1-(3,5-Difluorpyridin-2-yl)-7-[(4*S*)-4-hydroxy-2-oxopyrrolidin-1-yl]-4-oxo-*N*-[(2*S*)-1,1,1-trifluorbutan-2-yl]-1,4-dihydro-1,8-naphthyridin-3-carbonsäureamid

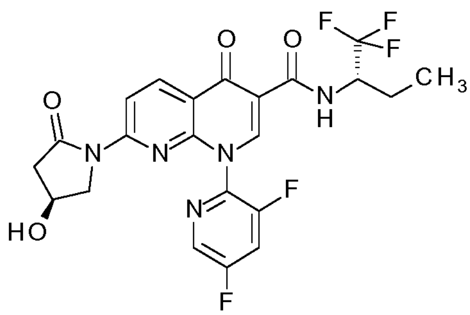

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 35.5 mg (280 μmol) (2S)-1,1,1-Trifluorbutan-2-amin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 77.2 mg (81% d. Th., 100% Reinheit) der Titelverbindung erhalten.
LC-MS (Methode 1): Rt = 0.93 min; MS (ESIpos): m/z = 512 [M+H]+.
$^1$H-NMR (400 MHz, DMSO-d6): δ [ppm] = 10.12 (d, 1H), 9.06 (s, 1H), 8.71 (d, 1H), 8.62-8.69 (m, 1H), 8.54 (br. t, 1H), 8.34-8.43 (m, 1H), 5.23-5.41 (m, 1H), 4.71-4.83 (m, 1H), 4.29 (br. s., 1H), 3.64-3.79 (m, 1H), 3.44-3.58 (m, 1H), 2.87-3.03

(m, 1H), 2.32-2.43 (m, 1H), 1.83-1.96 (m, 1H), 1.60-1.74 (m, 1H), 0.92-1.02 (m, 3H).

**Beispiel 3**

1-(3,5-Difluorpyridin-2-yl)-7-[(4S)-4-hydroxy-2-oxopyrrolidin-1-yl]-4-oxo-N-[(2S)-1,1,1-trifluorpropan-2-yl]-1,4-dihydro-1,8-naphthyridin-3-carbonsäureamid

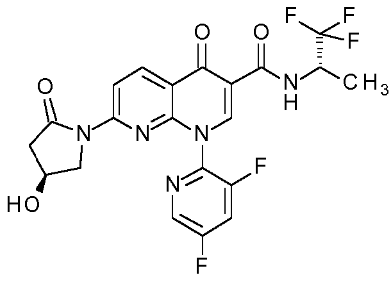

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 31.6 mg (280 μmol) (2S)-1,1,1-Trifluorpropan-2-amin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1%) Ameisensäure) gereinigt und 67.9 mg (73% d. Th., 100% Reinheit) der Titelverbindung erhalten.
LC-MS (Methode 2): Rt = 1.62 min; MS (ESIpos): m/z = 498 [M+H]+.
$^1$H-NMR (400 MHz, DMSO-d6): δ [ppm] = 10.17 (d, 1H), 9.05 (s, 1H), 8.70 (d, 1H), 8.62-8.68 (m, 1H), 8.54 (br. t, 1H), 8.39 (ddd, 1H), 5.22-5.41 (m, 1H), 4.87-4.99 (m, 1H), 4.29 (br. s., 1H), 3.64-3.78 (m, 1H), 3.44-3.57 (m, 1H), 2.87-3.03 (m, 1H), 2.32-2.43 (m, 1H), 1.40 (d, 3H).

**Beispiel 4**

*N*-[1-(2-Chlorphenyl)-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid (Diastereomerengemisch)

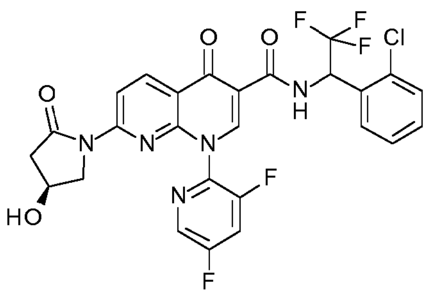

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 58.6 mg (280 μmol) 1-(2-Chlorphenyl)-2,2,2-trifluorethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 86.9 mg (78% d. Th., 100% Reinheit) der Titelverbindung erhalten.
LC-MS (Methode 2): Rt = 2.03 min; MS (ESIpos): m/z = 594 [M+H]+.
$^1$H-NMR (400 MHz, DMSO-d6): δ [ppm] = 11.22 (d, 1H), 9.06 (s, 1H), 8.75 (d, 1H), 8.62-8.71 (m, 1H), 8.51-8.60 (m, 1H), 8.39 (td, 1H), 7.44-7.70 (m, 4H), 6.47 (quintt, 1H), 5.20-5.42 (m, 1H), 4.29 (br. s, 1H), 3.61-3.80 (m, 1H), 3.42-3.58 (m, 1H), 2.85-3.05 (m, 1H), 2.29-2.45 (m, 1H).

67.0 mg der Titelverbindung (Diastereomerengemisch) wurde durch chirale HPLC in die Diastereomere getrennt (präparative HPLC: Säule Daicel Chiralpak AZ-H 5 μm 250x30 mm; Eluent: 40% Iso-Hexan, 60% Iso-Propanol; Temperatur: 23°C; Fluss: 40 ml/min; UV-Detektion: 220 nm.)
Man erhielt (in der Reihenfolge der Elution von der Säule) 18.2 mg Diastereomer 1 (Beispiel 5) (99% de), Rt = 6.21 min und 24.0 mg Diastereomer 2 (99% de), Rt = 10.47 min.
[Analytische HPLC: Säule Daicel Chiralpak AZ-3 3 μm 50x4.6 mm; Eluent: 50% Iso-Hexan, 50% Iso-propanol; Fluss: 1.0 ml/min; UV-Detektion: 220 nm].
Diastereomer 2 wurde zusätzlich mittels präparativer HPLC (Säule: Chromatorex C18, 10 μm, 125*40 mm, Lösungsmittel: Acetonitril, Wasser, 0.1% Ameisensäure) gereinigt und 11.4 mg (10% d. Th., 100% Reinheit) der Titelverbindung aus Beispiel 6 erhalten.

## Beispiel 5

*N*-[1-(2-Chlorphenyl)-2,2,2-trifluorethyl]-1-(3,5-difluorpyridin-2-yl)-7-[(4*S*)-4-hydroxy-2-oxopyrrolidin-1 -yl] -4-oxo-1,4-dihydro-1,8-naphthyridin-3 -carbonsäureamid (Diastereomer 1)

LC-MS (Methode 2): Rt = 2.03 min; MS (ESIpos): m/z = 594 [M+H]+.
$^1$H-NMR (400 MHz, DMSO-d6): δ [ppm] = 11.22 (d, 1H), 9.06 (br. s, 1H), 8.76 (d, 1H), 8.66 (br. s, 1H), 8.51-8.60 (m, 1H), 8.34-8.44 (m, 1H), 7.60-7.69 (m, 2H), 7.47-7.59 (m, 2H), 6.47 (quintt, 1H), 5.22-5.41 (m, 1H), 4.29 (br. s, 1H), 3.64-3.78 (m, 1H), 3.43-3.56 (m, 1H), 2.88-3.03 (m, 1H), 2.31-2.43 (m, 1H).

## Beispiel 6

*N*-[1-(2-Chlorphenyl)-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid (Diastereomer 2)

LC-MS (Methode 2): Rt = 2.02 min; MS (ESIpos): m/z = 594 [M+H]+.
$^1$H-NMR (400 MHz, DMSO-d6): δ [ppm] = 11.22 (d, 1H), 9.06 (s, 1H), 8.75 (d, 1H), 8.66 (dd, 1H), 8.56 (br. t, 1H), 8.39 (br. t, 1H), 7.59-7.69 (m, 2H), 7.47-7.59 (m, 2H), 6.47 (quint, 1H), 5.22-5.42 (m, 1H), 4.29 (br. s, 1H), 3.63-3.79 (m, 1H), 3.43-3.57 (m, 1H), 2.87-3.04 (m, 1H), 2.32-2.44 (m, 1H).

## Beispiel 7

*N*-[1-(3-Chlorphenyl)-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid (Diastereomerengemisch)

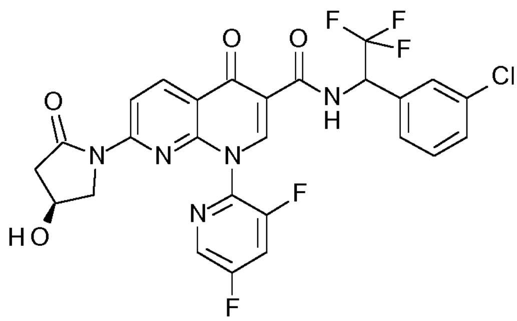

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 58.6 mg (280 μmol) 1-(3-Chlorphenyl)-2,2,2-trifluorethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 82.5 mg (75% d. Th., 100% Reinheit) der Titelverbindung erhalten.
LC-MS (Methode 2): Rt = 2.09 min; MS (ESIpos): m/z = 594 [M+H]+.
$^1$H-NMR (400 MHz, DMSO-d6): δ [ppm] = 11.11 (d, 1H), 9.06 (s, 1H), 8.77 (d, 1H), 8.62-8.71 (m, 1H), 8.56 (t, 1H), 8.39

(td, 1H), 7.69 (br. s, 1H), 7.47-7.61 (m, 3H), 6.21 (quint, 1H), 5.20-5.42 (m, 1H), 4.29 (br. s, 1H), 3.62-3.80 (m, 1H), 3.42-3.58 (m, 1H), 2.86-3.05 (m, 1H), 2.32-2.44 (m, 1H).

**Beispiel 8**

*N*-[1-(4-Chlorphenyl)-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid (Diastereomerengemisch)

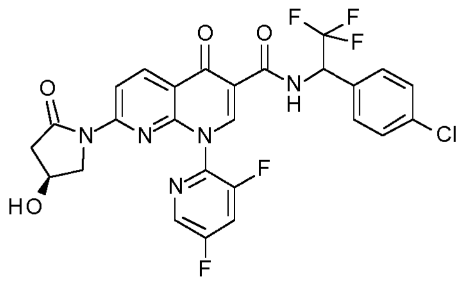

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 58.6 mg (280 μmol) 1-(4-Chlorphenyl)-2,2,2-trifluorethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 91.4 mg (83% d. Th., 100% Reinheit) der Titelverbindung erhalten.
LC-MS (Methode 1): Rt = 1.16 min; MS (ESIpos): m/z = 594 [M+H]+.
$^1$H-NMR (400 MHz, DMSO-d6): δ [ppm] = 11.10 (d, 1H), 9.06 (s, 1H), 8.76 (d, 1H), 8.62-8.70 (m, 1H), 8.56 (t, 1H), 8.32-8.43 (m, 1H), 7.51-7.66 (m, 4H), 6.12-6.24 (m, 1H), 5.21-5.42 (m, 1H), 4.29 (br. s, 1H), 3.63-3.79 (m, 1H), 3.43-3.57 (m, 1H), 2.86-3.04 (m, 1H), 2.32-2.44 (m, 1H).

**Beispiel 9**

*N*-[1-(2,6-Difluorphenyl)-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid (Diastereomerengemisch)

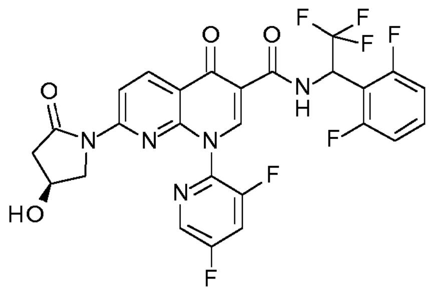

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 59.0 mg (280 μmol) 1-(2,6-Difluorphenyl)-2,2,2-trifluorethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 89.0 mg (80% d. Th., 100% Reinheit) der Titelverbindung erhalten.
LC-MS (Methode 1): Rt = 1.10 min; MS (ESIpos): m/z = 596 [M+H]+.
$^1$H-NMR (400 MHz, DMSO-d6): δ [ppm] = 11.29 (d, 1H), 9.08 (s, 1H), 8.75 (d, 1H), 8.62-8.70 (m, 1H), 8.50-8.59 (m, 1H), 8.34-8.44 (m, 1H), 7.64 (quint, 1H), 7.32 (t, 2H), 6.44 (quint, 1H), 5.21-5.42 (m, 1H), 4.29 (br. s, 1H), 3.62-3.80 (m,

1H), 3.42-3.57 (m, 1H), 2.86-3.04 (m, 1H), 2.31-2.44 (m, 1H).

**Beispiel 10**

1-(3,5-Difluorpyridin-2-yl)-7-[(4*S*)-4-hydroxy-2-oxopyrrolidin-1-yl]-4-oxo-*N*-[2,2,2-trifluor-1-(3-fluorphenyl)ethyl]-1,4-dihydro-1,8-naphthyridin-3-carbonsäureamid (Diastereomerengemisch)

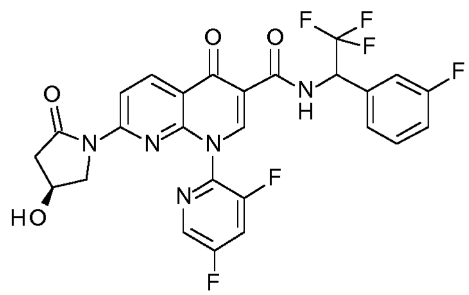

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 54.0 mg (280 μmol) 2,2,2-Trifluor-1-(3-fluorphenyl)ethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 80.5 mg (75% d. Th., 100% Reinheit) der Titelverbindung erhalten.
LC-MS (Methode 2): Rt = 1.97 min; MS (ESIpos): m/z = 578 [M+H]+.
$^1$H-NMR (400 MHz, DMSO-d6): δ [ppm] = 11.10 (d, 1H), 9.07 (s, 1H), 8.77 (d, 1H), 8.63-8.70 (m, 1H), 8.51-8.60 (m, 1H), 8.39 (td, 1H), 7.52-7.62 (m, 1H), 7.41-7.50 (m, 2H), 7.31 (br. t, 1H), 6.20 (quint, 1H), 5.21-5.42 (m, 1H), 4.29 (br. s, 1H), 3.63-3.80 (m, 1H), 3.43-3.58 (m, 1H), 2.86-3.04 (m, 1H), 2.31-2.45 (m, 1H).

**Beispiel 11**

1-(3,5-Difluorpyridin-2-yl)-7-[(4*S*)-4-hydroxy-2-oxopyrrolidin-1-yl]-4-oxo-*N*-[2,2,2-trifluor-1-(4-fluorphenyl)ethyl]-1,4-dihydro-1,8-naphthyridin-3-carbonsäureamid (Diastereomerengemisch)

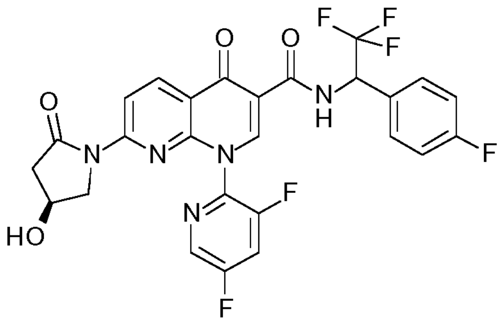

Gemäß allgemeiner Arbeitsvorschrift 1 wurden 75.0 mg (186 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 54.0 mg (280 μmol) 2,2,2-Trifluor-1-(4-fluorphenyl)ethanamin in Gegenwart von 85.1 mg (224 μmol) HATU und 0.10 ml (0.56 mmol) DIPEA in 1.2 ml DMF zur Reaktion gebracht. Das Rohprodukt wurde mittels präparativer HPLC (Säule: Chromatorex C18, 10 μm, 125*40 mm, Lösungsmittel: Acetonitril, Wasser, 0.1% Ameisensäure) gereinigt und 85.0 mg (79% d. Th., 100% Reinheit) der Titelverbindung erhalten.
LC-MS (Methode 1): Rt = 1.09 min; MS (ESIpos): m/z = 578 [M+H]+.
$^1$H-NMR (400 MHz, DMSO-d6): δ [ppm] = 11.09 (d, 1H), 9.06 (s, 1H), 8.76 (d, 1H), 8.62-8.70 (m, 1H), 8.51-8.60 (m,

1H), 8.39 (td, 1H), 7.61-7.69 (m, 2H), 7.30-7.39 (m, 2H), 6.16 (quint, 1H), 5.23-5.41 (m, 1H), 4.29 (br. s, 1H), 3.64-3.79 (m, 1H), 3.44-3.57 (m, 1H), 2.88-3.03 (m, 1H), 2.38 (dd, 1H).

71.0 mg der Titelverbindung (Diastereomerengemisch) wurde durch chirale HPLC in die Diastereomere getrennt (präparative HPLC: Säule Daicel Chiralpak OX-H 5 μm 250x20 mm; Eluent: 25% n-Heptan, 75% Iso-Propanol; Temperatur: 50°C; Fluss: 15 ml/min; UV-Detektion: 220 nm.)

Man erhielt (in der Reihenfolge der Elution von der Säule) 30.2 mg Diastereomer 1 (99% de) $R_t$ = 14.57 min und 30.2 mg Diastereomer 2 (99% de) $R_t$ = 19.05 min.

[Analytische HPLC: Säule Daicel Chiralpak OX-H 5 μm 250x20 mm; Eluent: 30% n-Heptan, 70% Iso-Propanol; Temperatur: 50°C; Fluss: 15 ml/min; UV-Detektion: 220 nm].

Diastereomer 1 wurde zusätzlich mittels präparativer HPLC (Acetonitril / Wasser/ 0.1 % Ameisensäure) gereinigt und 24.5 mg (23% d. Th., 99% Reinheit) der Titelverbindung aus Beispiel 12 erhalten.

Diastereomer 2 wurde zusätzlich mittels präparativer HPLC (Acetonitril / Wasser/ 0.1 % Ameisensäure) gereinigt und 24.6 mg (23% d. Th., 100% Reinheit) der Titelverbindung aus Beispiel 13 erhalten.

## Beispiel 12

1-(3,5-Difluorpyridin-2-yl)-7-[(4*S*)-4-hydroxy-2-oxopyrrolidin-1-yl]-4-oxo-*N*-[2,2,2-trifluor-1-(4-fluorphenyl)ethyl]-1,4-dihydro-1,8-naphthyridin-3-carbonsäureamid (Diastereomer 1)

LC-MS (Methode 2): $R_t$ = 1.92 min; MS (ESIpos): m/z = 578 [M+H]+.$^1$H NMR (400 MHz, DMSO-d6): δ [ppm] = 11.09 (d, 1H), 9.06 (s, 1H), 8.76 (d, 1H), 8.63-8.69 (m, 1H), 8.56 (br t, 1H), 8.35-8.42 (m, 1H), 7.59-7.71 (m, 2H), 7.28-7.40 (m, 2H), 6.16 (quin, 1H), 5.23-5.41 (m, 1H), 4.23-4.34 (m, 1H), 3.63-3.78 (m, 1H), 3.44-3.57 (m, 1H), 2.88-3.03 (m, 1H), 2.34-2.46 (m, 1H).

## Beispiel 13

1-(3,5-Difluorpyridin-2-yl)-7-[(4*S*)-4-hydroxy-2-oxopyrrolidin-1-yl]-4-oxo-*N*-[2,2,2-trifluor-1-(4-fluorphenyl)ethyl]-1,4-dihydro-1,8-naphthyridin-3-carbonsäureamid (Diastereomer 2)

LC-MS (Methode 2): $R_t$ = 1.90 min; MS (ESIpos): m/z = 578 [M+H]+.$^1$H NMR (400 MHz, DMSO-d6): δ [ppm] = 11.09 (d, 1H), 9.06 (d, 1H), 8.76 (d, 1H), 8.66 (d, 1H), 8.52-8.59 (m, 1H), 8.35-8.42 (m, 1H), 7.61-7.68 (m, 2H), 7.30-7.38 (m, 2H), 6.12-6.21 (m, 1H), 5.22-5.40 (m, 1H), 4.26-4.32 (m, 1H), 3.64-3.78 (m, 1H), 3.43-3.57 (m, 1H), 2.88-3.03 (m, 1H), 2.34-2.43 (m, 1H).

## Beispiel 14

*N*-[(1S)-1-Cyclopropyl-2,2,2-trifluorethyl]-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid

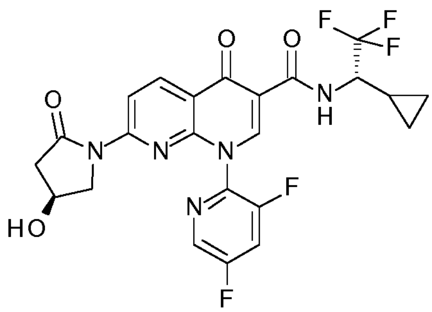

Gemäß allgemeiner Arbeitsvorschrift 2 wurden 100 mg (218 μmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 26.4 mg (262 μmol) (4S)-4-Hydroxypyrrolidin-2-on in Gegenwart von 45.2 mg (327 μmol) Kaliumcarbonat, 9.80 mg (44.0 μmol) Palladiumacetat und 25.2 mg (44.0 μmol) Xantphos in 2.1 ml Dioxan bei 80°C zur Reaktion gebracht. Die Mischung wurde dann auf RT abgekühlt, dann mit 100 mg N-Acetylcystein versetzt und 15 min nachgerührt. Die Mischung wurde mit 15 ml gesättigter wässriger Natriumhydrogencarbonat-Lösung und 20 ml Essigsäureethylester verdünnt und die Phasen

getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in wenig Acetonitril, Wasser und Ameisensäure gelöst, durch einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 74.6 mg (65% d. Th., 100% Reinheit) der Titelverbindung erhalten.
LC-MS (MCW_SQ-HSST3-Methode 1): Rt = 0.94 min; MS (ESIpos): m/z = 524 [M+H]+.
$^1$H-NMR (400 MHz, DMSO-d6): δ [ppm] = 10.26 (d, 1H), 9.05 (s, 1H), 8.72 (d, 1H), 8.63-8.69 (m, 1H), 8.54 (t, 1H), 8.34-8.43 (m, 1H), 5.22-5.41 (m, 1H), 4.42 (sxt, 1H), 4.29 (br. s, 1H), 3.64-3.79 (m, 1H), 3.44-3.58 (m, 1H), 2.86-3.04 (m, 1H), 2.38 (dd, 1H), 1.18-1.29 (m, 1H), 0.50-0.72 (m, 3H), 0.29-0.40 (m, 1H).

**Beispiel 15**

1-(3,5-Difluorpyridin-2-yl)-*N*-(1,1,1,3,3,3-hexafluorpropan-2-yl)-7-[(4*S*)-4-hydroxy-2-oxopyrrolidin-1-yl]-4-oxo-1,4-dihydro-1,8-naphthyridin-3-carbonsäureamid

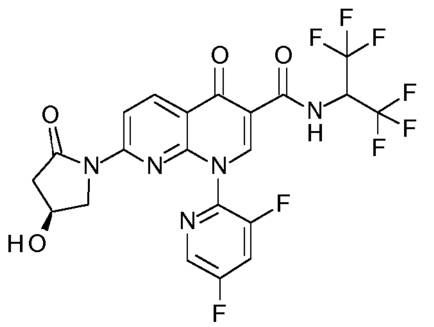

Gemäß allgemeiner Arbeitsvorschrift 2 wurden 18.6 g (38.2 mmol) 7-Chlor-1-(3,5-difluorpyridin-2-yl)-N-(1,1,1,3,3,3-hexafluorpropan-2-yl)-4-oxo-1,4-dihydro-1,8-naphthyridin-3-carbonsäureamid (Beispiel 10A) mit 4.64 g (45.9 mmol) (4S)-4-Hydroxypyrrolidin-2-on in Gegenwart von 7.92 g (57.3 mmol) Kaliumcarbonat, 1.72 g (7.64 mmol) Palladiumacetat und 4.42 g (7.64 mmol) Xantphos in 372 ml Dioxan bei 80°C zur Reaktion gebracht. Die Reaktionsmischung wurde auf Wasser gegossen, 15 min nachgerührt, der Niederschlag abgesaugt, mit Wasser nachgewaschen und am Hochvakuum getrocknet. Der Feststoff wurde in Acetonitril gelöst und mittels Normalphasenchromatographie (Essigsäureethylester-Cyclohexan Gradient) gereinigt. Das Lösungsmittel wurde unter reduziertem Druck entfernt und der Rückstand aus wenig Acetonitril umkristallisiert. Es wurden 13.3 g (63% d. Th., 100% Reinheit) der Titelverbindung erhalten.
LC-MS (Methode 2): Rt = 1.88 min; MS (ESIpos): m/z = 552 [M+H]+.
$^1$H-NMR (400 MHz, DMSO-d6): δ [ppm] = 11.04 (d, 1H), 9.14 (s, 1H), 8.74 (d, 1H), 8.66-8.70 (m, 1H), 8.52-8.60 (m, 1H), 8.40 (td, 1H), 6.33-6.45 (m, 1H), 5.24-5.41 (m, 1H), 4.30 (br. s, 1H), 3.64-3.79 (m, 1H), 3.44-3.57 (m, 1H), 2.88-3.04 (m, 1H), 2.33-2.44 (m, 1H).

**Beispiel 16**

*N*-(2,6-Dichlorphenyl)-1-(3,5-difluorpyridin-2-yl)-7-[(4*S*)-4-hydroxy-2-oxopyrrolidin-1-yl]-4-oxo-1,4-dihydro-1,8-naphthyridin-3-carbonsäureamid

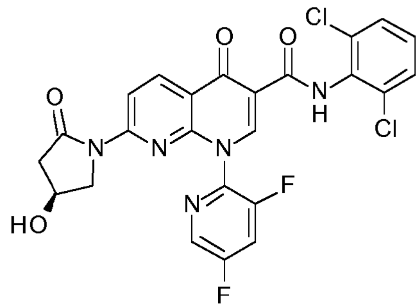

Gemäß allgemeiner Arbeitsvorschrift 2 wurden 60.0 mg (125 µmol) 7-Chlor-N-(2,6-dichlorphenyl)-1-(3,5-difluorpyridin-2-yl)-4-oxo-1,4-dihydro-1,8-naphthyridin-3-carbonsäureamid (Beispiel 11B) mit 15.1 mg (149 µmol) (4S)-4-Hydroxypyrrolidin-2-on in Gegenwart von 25.8 mg (187 µmol) Kaliumcarbonat, 5.60 mg (24.9 µmol) Palladiumacetat und 14.4 mg (24.9 µmol) Xantphos in 0.93 ml Dioxan bei 80°C zur Reaktion gebracht. Die Mischung wurde dann auf RT abgekühlt, dann mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in 3 ml Acetonitril und 1 ml Wasser gelöst und mittels präparativer HPLC (Säule: Chromatorex C18, 10 µm, 125*40 mm, Lösungsmittel: Acetonitril, Wasser, 0.1% Ameisensäure) gereinigt und 16.7 mg (25% d. Th., 100% Reinheit) der Titelverbindung erhalten.
LC-MS (Methode 2): Rt = 1.77 min; MS (ESIpos): m/z = 546 [M+H]+.
$^1$H-NMR (400 MHz, DMSO-d6): δ [ppm] = 11.59 (s, 1H), 9.13 (s, 1H), 8.77 (d, 1H), 8.63-8.71 (m, 1H), 8.53-8.61 (m, 1H), 8.34-8.45 (m, 1H), 7.60 (d, 2H), 7.34-7.44 (m, 1H), 5.22-5.44 (m, 1H), 4.30 (br. s, 1H), 3.64-3.82 (m, 1H), 3.45-3.60 (m, 1H), 2.87-3.06 (m, 1H), 2.39 (dd, 1H).

**Beispiel 17**

1-(3,5-Difluorpyridin-2-yl)-7-[(4S)-4-hydroxy-2-oxopyrrolidin-1-yl]-4-oxo-N-[1-(trifluormethoxy)-butan-2-yl]-1,4-dihydro-1,8-naphthyridin-3-carbonsäureamid (Diastereomerengemisch)

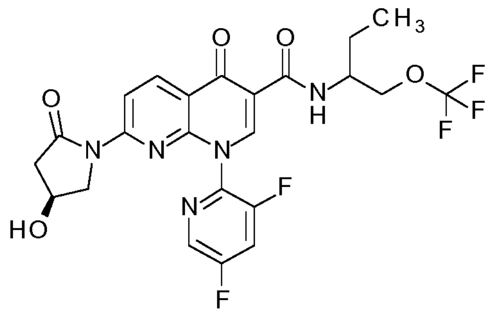

Gemäß allgemeiner Arbeitsvorschrift 2 wurden 148 mg (311 µmol) 7-Chlor-1-(3,5-difluorpyridin-2-yl)-4-oxo-N-[1-(trifluormethoxy)butan-2-yl]-1,4-dihydro-1,8-naphthyridin-3-carbonsäureamid (Beispiel 12A) mit 31.4 mg (311 µmol) (4S)-4-Hydroxypyrrolidin-2-on in Gegenwart von 64.5 mg (467 µmol) Kaliumcarbonat, 12.6 mg (56.0 µmol) Palladiumacetat und 64.8 mg (112 µmol) Xantphos in 2.6 ml Dioxan bei 80°C zur Reaktion gebracht. Die Reaktionsmischung wurde anschließend mit Essigsäureethylester verdünnt und mit gesättigter wässriger Natriumhydrogencarbonat gewaschen. Die organische Phase wurde über Magnesiumsulfat getrocknet, filtriert und das Lösungsmittel unter reduziertem Druck entfernt. Das Rohprodukt wurde in wenig Acetonitril, Wasser und Ameisensäure gelöst, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurden 42.0 mg (25% d. Th., 100% Reinheit) der Titelverbindung erhalten.
LC-MS (Methode 1): Rt = 1.00 min; MS (ESIpos): m/z = 542 [M+H]+.
$^1$H-NMR (400 MHz, DMSO-d6): δ [ppm] = 9.81 (d, 1H), 9.00 (s, 1H), 8.71 (d, 1H), 8.63-8.68 (m, 1H), 8.52 (t, 1H), 8.34-8.43 (m, 1H), 5.22-5.40 (m, 1H), 4.14-4.33 (m, 4H), 3.64-3.79 (m, 1H), 3.44-3.58 (m, 1H), 2.87-3.03 (m, 1H), 2.38 (dd, 1H), 1.55-1.75 (m, 2H), 0.95 (td, 3H).

42.0 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 40% Iso-Hexan, 60% Ethanol; Temperatur: 24°C; Fluss: 20 ml/min; UV-Detektion: 220 nm.)

Man erhielt (in der Reihenfolge der Elution von der Säule) 14.2 mg Diastereomer 1 (Beispiel 18) (99% de) Rt = 6.63 min und 15.1 mg Diastereomer 2 (Beispiel 19) (99% de) Rt = 9.45 min.

[Analytische HPLC: Säule Daicel Chiralpak OX-3 3 µm 50x4.6 mm; Eluent: 50% Iso-Hexan, 50% Ethanol; Fluss: 1.0 ml/min; UV-Detektion: 220nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 11.5 mg (6.8% d. Th., 100% Reinheit) der Titelverbindung aus Beispiel 18 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 11.3 mg (6.7% d. Th., 100% Reinheit) der Titelverbindung aus Beispiel 19 erhalten.

## Beispiel 18

1-(3,5-Difluorpyridin-2-yl)-7-[(4S)-4-hydroxy-2-oxopyrrolidin-1-yl]-4-oxo-N -[1-(trifluormethoxy)-butan-2-yl]-1,4-dihydro-1,8-naphthyridin-3-carbonsäureamid (Diastereomer 1)

LC-MS (Methode 2): Rt = 1.77 min; MS (ESIpos): m/z = 542 [M+H]+.

$^1$H-NMR (400 MHz, DMSO-d6): δ [ppm] = 9.81 (d, 1H), 9.00 (s, 1H), 8.71 (d, 1H), 8.63-8.68 (m, 1H), 8.52 (t, 1H), 8.38 (ddd, 1H), 5.21-5.41 (m, 1H), 4.14-4.33 (m, 4H), 3.64-3.79 (m, 1H), 3.44-3.58 (m, 1H), 2.86-3.03 (m, 1H), 2.34-2.43 (m, 1H), 1.54-1.75 (m, 2H), 0.95 (t, 3H).

## Beispiel 19

1-(3,5-Difluorpyridin-2-yl)-7-[(4S)-4-hydroxy-2-oxopyrrolidin-1-yl]-4-oxo-N -[1-(trifluormethoxy)-butan-2-yl]-1,4-dihydro-1,8-naphthyridin-3-carbonsäureamid (Diastereomer 2)

LC-MS (Methode 2): Rt = 1.76 min; MS (ESIpos): m/z = 542 [M+H]+.

$^1$H-NMR (500 MHz, DMSO-d6): δ [ppm] = 9.81 (d, 1H), 9.00 (s, 1H), 8.71 (d, 1H), 8.63-8.68 (m, 1H), 8.49-8.56 (m, 1H), 8.38 (td, 1H), 5.22-5.40 (m, 1H), 4.14-4.33 (m, 4H), 3.64-3.79 (m, 1H), 3.44-3.57 (m, 1H), 2.87-3.02 (m, 1H), 2.32-2.43 (m, 1H), 1.54-1.74 (m, 2H), 0.95 (td, 3H).

## Beispiel 20

1-(3-Chlor-5-fluorpyridin-2-yl)-7-[(4S)-4-hydroxy-2-oxopyrrolidin-1-yl]-4-oxo-N-[(2R)-1,1,1-trifluorbutan-2-yl]-1,4-dihydro-1,8-naphthyridin-3-carbonsäureamid

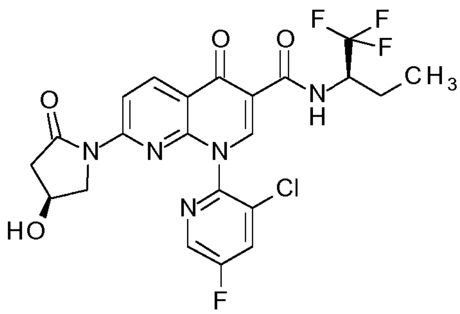

Gemäß allgemeiner Arbeitsvorschrift 2 wurden 60.0 mg (130 µmol) 7-Chlor-1-(3-chlor-5-fluorpyridin-2-yl)-4-oxo-N-[(2R)-1,1,1-trifluorbutan-2-yl]-1,4-dihydro-1,8-naphthyridin-3-carbonsäureamid (Beispiel 16C) mit 15.7 mg (155 µmol) (4S)-4-Hydroxypyrrolidin-2-on in Gegenwart von 26.9 mg (194 µmol) Kaliumcarbonat, 5.82 mg (26.0 µmol) Palladiumacetat und 15.0 mg (26.0 µmol) Xantphos in 1 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1%

Ameisensäure) gereinigt und 45.4 mg (66% d. Th., 100% Reinheit) der Titelverbindung erhalten.
LC-MS (Methode 1): Rt = 0.95 min; MS (ESIpos): m/z = 528 $[M]^+$.
$^1$H-NMR (500 MHz, DMSO-d6): δ [ppm] = 10.14 (d, 1H), 9.08 (d, 1H), 8.76 (dd, 1H), 8.71 (dd, 1H), 8.49-8.58 (m, 2H), 5.24-5.38 (m, 1H), 4.72-4.83 (m, 1H), 4.23-4.31 (m, 1H), 3.59-3.70 (m, 1H), 3.42 (dd, 1H), 2.87-3.00 (m, 1H), 2.37 (dd, 1H), 1.85-1.94 (m, 1H), 1.62-1.73 (m, 1H), 0.98 (t, 3H).

## Beispiel 21

1-(3-Chlor-5-fluorpyridin-2-yl)-N-[(1R)-1-cyclopropyl-2,2,2-trifluorethyl]-7-[(4S)-4-hydroxy-2-oxopyrrolidin-1-yl]-4-oxo-1,4-dihydro-1,8-naphthyridin-3-carbonsäureamid

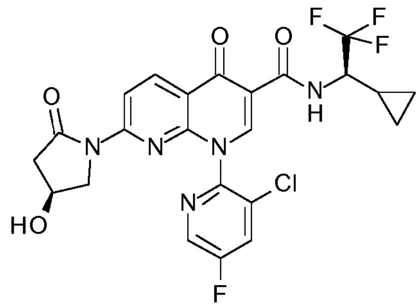

Gemäß allgemeiner Arbeitsvorschrift 2 wurden 60.0 mg (126 μmol) 7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid (Beispiel 17A) mit 15.3 mg (152 μmol) (4S)-4-Hydroxypyrrolidin-2-on in Gegenwart von 26.2 mg (189 μmol) Kaliumcarbonat, 5.67 mg (25.0 μmol) Palladiumacetat und 14.6 mg (25.0 μmol) Xantphos in 1 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt. Nach Umkristallisation aus Acetonitril wurden 30.1 mg (44% d. Th., 100% Reinheit) der Titelverbindung erhalten.
LC-MS (Methode 1): Rt = 0.97 min; MS (ESIpos): m/z = 540 [M+H]+.
$^1$H-NMR (400 MHz, DMSO-d6): δ [ppm] = 10.28 (dd, 1H), 9.07 (d, 1H), 8.69-8.79 (m, 2H), 8.49-8.59 (m, 2H), 5.23-5.38 (m, 1H), 4.36-4.49 (m, 1H), 4.22-4.31 (m, 1H), 3.64 (ddd, 1H), 3.43 (dd, 1H), 2.86-3.01 (m, 1H), 2.37 (dd, 1H), 1.18-1.29 (m, 1H), 0.50-0.72 (m, 3H), 0.30-0.40 (m, 1H).

## Beispiel 22

1-(3-Chlorpyridin-2-yl)-N-[(1R)-1-cyclopropyl-2,2,2-trifluorethyl]-7-[(4S)-4-hydroxy-2-oxopyrrolidin-1-yl]-4-oxo-1,4-dihydro-1,8-naphthyridin-3-carbonsäureamid

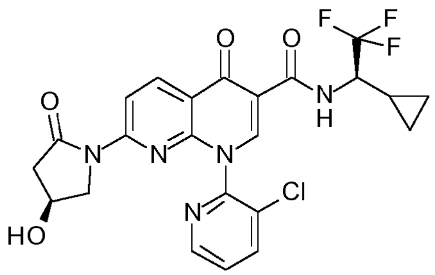

Gemäß allgemeiner Arbeitsvorschrift 2 wurden 60.0 mg (131 μmol) 7-Chlor-1-(3-chlorpyridin-2-yl)-N-[(1R)-1-cyclopropyl-2,2,2-trifluorethyl]-4-oxo-1,4-dihydro-1,8-naphthyridin-3-carbonsäureamid (Beispiel 19A) mit 15.9 mg (157 μmol) (4S)-4-Hydroxypyrrolidin-2-on in Gegenwart von 27.2 mg (197 μmol) Kaliumcarbonat, 5.89 mg (26.0 μmol) Palladiumacetat und 15.2 mg (26.0 μmol) Xantphos in 1 ml Dioxan bei 80°C zur Reaktion gebracht. Die Reaktionslösung

wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Nach Umkristallisation aus Acetonitril wurden 34.2 mg (50% d. Th., 100% Reinheit) der Titelverbindung erhalten.
LC-MS (Methode 1): Rt = 0.93 min; MS (ESIpos): m/z = 522 [M+H]+.
$^1$H-NMR (400 MHz, DMSO-d6): δ [ppm] = 10.30 (dd, 1H), 9.04 (d, 1H), 8.72 (d, 1H), 8.67 (ddd, 1H), 8.49-8.57 (m, 1H), 8.30-8.37 (m, 1H), 7.77 (ddd, 1H), 5.24-5.39 (m, 1H), 4.35-4.49 (m, 1H), 4.20-4.30 (m, 1H), 3.52-3.68 (m, 1H), 3.33-3.47 (m, 1H), 2.85-3.01 (m, 1H), 2.36 (dd, 1H), 1.18-1.29 (m, 1H), 0.50-0.72 (m, 3H), 0.30-0.40 (m, 1H).

## Beispiel 23

1-(3-Chlorpyridin-2-yl)-7-[(4S)-4-hydroxy-2-oxopyrrolidin-1-yl]-4-oxo-N-[2,2,2-trifluor-1-(3-fluorphenyl)ethyl]-1,4-dihydro-1,8-naphthyridin-3-carbonsäureamid (Diastereomerengemisch)

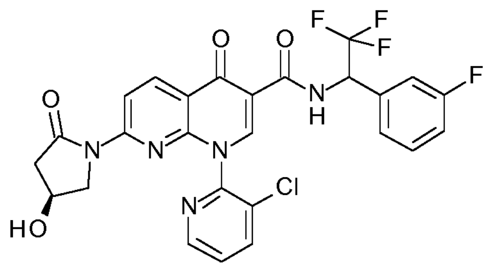

Gemäß allgemeiner Arbeitsvorschrift 1 wurden 75.0 mg (187 µmol) 1-(3-Chlorpyridin-2-yl)-7-[(4S)-4-hydroxy-2-oxopyrrolidin-1-yl]-4-oxo-1,4-dihydro-1,8-naphthyridin-3-carbonsäure (Beispiel 27A) mit 54.2 mg (281 µmol) 2,2,2-Trifluor-1-(3-fluorphenyl)ethanamin in Gegenwart von 85.4 mg (225 µmol) HATU und 98 µl (561 µmol) DIPEA in 1.2 ml DMF zur Reaktion gebracht. Die Reaktionslösung wurde anschließend mit wenig Acetonitril, Wasser und Ameisensäure versetzt, durch einen Milliporefilter filtriert und zweimal mittels präparativer HPLC (Säule: Chromatorex C18, 10 µm, 125*40 mm, Lösungsmittel: Acetonitril, Wasser, 0.1% Ameisensäure) gereinigt. Das Produkt wurde erneut mittels Normalphasenchromatographie (Essigsäureethylester) gereinigt und 38.6 mg (36% d. Th., 100% Reinheit) der Titelverbindung erhalten.
LC-MS (Methode 2): Rt = 1.90 min; MS (ESIpos): m/z = 576 [M+H]+.
$^1$H-NMR (400 MHz, DMSO-d6): δ [ppm] = 11.14 (d, 1H), 9.07 (dd, 1H), 8.78 (d, 1H), 8.62-8.71 (m, 1H), 8.50-8.59 (m, 1H), 8.29-8.37 (m, 1H), 7.73-7.81 (m, 1H), 7.52-7.62 (m, 1H), 7.42-7.50 (m, 2H), 7.27-7.36 (m, 1H), 6.15-6.26 (m, 1H), 5.24-5.38 (m, 1H), 4.20-4.29 (m, 1H), 3.51-3.69 (m, 1H), 3.33-3.47 (m, 1H), 2.85-3.01 (m, 1H), 2.36 (dd, 1H).

## Beispiel 24

1-(3,5-Difluorpyridin-4-yl)-7-[(4S)-4-hydroxy-2-oxopyrrolidin-1-yl]-4-oxo-N-[(2S)-1,1,1-trifluorbutan-2-yl]-1,4-dihydro-1,8-naphthyridin-3-carbonsäureamid

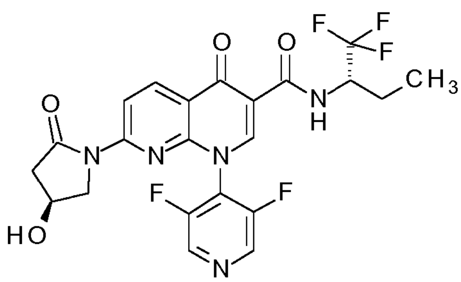

Gemäß allgemeiner Arbeitsvorschrift 2 wurden 40.0 mg (89.5 µmol) 7-Chlor-1-(3,5-difluorpyridin-4-yl)-4-oxo-N-[(2S)-1,1,1-trifluorbutan-2-yl]-1,4-dihydro-1,8-naphthyridin-3-carbonsäureamid (Beispiel 22C) mit 10.9 mg (107 µmol)

(4S)-4-Hydroxypyrrolidin-2-on in Gegenwart von 18.6 mg (134 μmol) Kaliumcarbonat, 4.02 mg (18.0 μmol) Palladiumacetat und 10.4 mg (18.0 μmol) Xantphos in 3 ml Dioxan bei 80°C zur Reaktion gebracht. Die Mischung wurde dann auf RT abgekühlt, dann mit Wasser und Acetonitril versetzt, filtriert und die Rohlösung mittels präparativer HPLC (Säule: Reprosil, 10 μm, 125*30 mm, Lösungsmittel: Wasser, Acetonitril, 0.1% Trifluoressigsäure-Gradient) gereinigt und 25.9 mg (54% d. Th., 96% Reinheit) der Titelverbindung erhalten.
LC-MS (Methode 2): Rt = 1.71 min; MS (ESIpos): m/z = 512 [M+H]+.
$^1$H-NMR (400 MHz, DMSO-d6): δ [ppm] = 10.07 (d, 1H), 9.15 (s, 1H), 8.91 (m, 2H), 8.73 (d, 1H), 8.55 (d, 1H), 4.71-4.83 (m, 1H), 4.26-4.31 (m, 1H), 3.68 (dd, 1H), 3.43-3.51 (m, 1H), 2.94 (dd, 1H), 2.32-2.43 (m, 1H), 1.84-1.95 (m, 1H), 1.61-1.73 (m, 1H), 0.98 (t, 3H).

**Beispiel 25**

N-[1-Cyclopropyl-2,2,2-trifluorethyl]-1-(3,5-difluorpyridin-2-yl)-7-[(3S)-3-hydroxy-2-oxopyrrolidin-1-yl]-4-oxo-1,4-dihydro-1,8-naphthyridin-3-carbonsäureamid (Diastereomerengemisch)

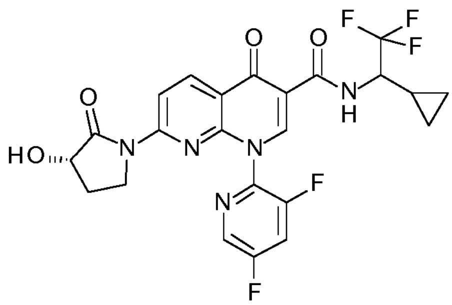

Gemäß allgemeiner Arbeitsvorschrift 2 wurden 50.0 mg (109 μmol) 7-Chlor-N-[1-cyclopropyl-2,2,2-trifluorethyl]-1-(3,5-difluorpyridin-2-yl)-4-oxo-1,4-dihydro-1,8-naphthyridin-3-carbonsäureamid (Beispiel 7A) mit 13.2 mg (131 μmol) (3S)-3-Hydroxypyrrolidin-2-on (CAS: 34368-52-0) in Gegenwart von 22.6 mg (163 μmol) Kaliumcarbonat, 4.89 mg (22.0 μmol) Palladiumacetat und 12.6 mg (22.0 μmol) Xantphos in 1 ml Dioxan bei 80°C zur Reaktion gebracht. Die Mischung wurde dann auf RT abgekühlt, dann mit Wasser und Acetonitril versetzt, filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt. Das Produkt wurde nochmals mittels Normalphasenchromatographie (Cyclohexan /Essigsäureethylester-Gradient) gereinigt und 20.4 mg (36% d. Th., 100% Reinheit) der Titelverbindung erhalten.
LC-MS (Methode 2): Rt = 1.77 min; MS (ESIpos): m/z = 524 [M+H]+.
$^1$H-NMR (400 MHz, DMSO-d6): δ [ppm] = 10.25 (d, 1H), 9.05 (s, 1H), 8.74 (d, 1H), 8.65 (d, 1H), 8.55 (t, 1H), 8.32-8.42 (m, 1H), 5.91 (dd, 1H), 4.31-4.49 (m, 2H), 3.54-3.69 (m, 1H), 3.33-3.45 (m, 1H), 2.26-2.38 (m, 1H), 1.70-1.87 (m, 1H), 1.18-1.29 (m, 1H), 0.50-0.71 (m, 3H), 0.28-0.40 (m, 1H).

**Beispiel 26**

N-[1-Cyclopropyl-2,2,2-trifluorethyl]-1-(3,5-difluorpyridin-2-yl)-7-[(3R)-3-hydroxy-2-oxopyrrolidin-1-yl]-4-oxo-1,4-dihydro-1,8-naphthyridin-3-carbonsäureamid (Diastereomerengemisch)

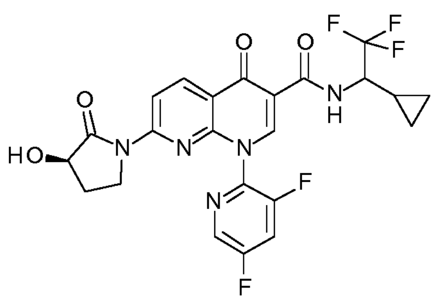

Gemäß allgemeiner Arbeitsvorschrift 2 wurden 50.0 mg (109 µmol) 7-Chlor-N-[1-cyclopropyl-2,2,2-trifluorethyl]-1-(3,5-difluorpyridin-2-yl)-4-oxo-1,4-dihydro-1,8-naphthyridin-3-carbonsäureamid (Beispiel 7A) mit 13.2 mg (131 µmol) (3R)-3-Hydroxypyrrolidin-2-on (CAS: 77510-50-0) in Gegenwart von 22.6 mg (163 µmol) Kaliumcarbonat, 4.89 mg (22.0 µmol) Palladiumacetat und 12.6 mg (22.0 µmol) Xantphos in 1 ml Dioxan bei 80°C zur Reaktion gebracht. Die Mischung wurde dann auf RT abgekühlt, dann mit Wasser und Acetonitril versetzt, filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt. Das Produkt wurde nochmals mittels Normalphasenchromatographie (Cyclohexan/Essigsäureethylester-Gradient) gereinigt und 18.9 mg (32% d. Th., 96% Reinheit) der Titelverbindung erhalten.
LC-MS (Methode 2): Rt = 1.77 min; MS (ESIpos): m/z = 524 [M+H]+.
$^1$H-NMR (400 MHz, DMSO-d6): δ [ppm] = 10.25 (d, 1H), 9.05 (s, 1H), 8.74 (d, 1H), 8.65 (d, 1H), 8.55 (t, 1H), 8.31-8.42 (m, 1H), 5.92 (dd, 1H), 4.32-4.48 (m, 2H), 3.54-3.69 (m, 1H), 3.33-3.44 (m, 1H), 2.26-2.37 (m, 1H), 1.69-1.86 (m, 1H), 1.20-1.26 (m, 1H), 0.50-0.72 (m, 3H), 0.25-0.42 (m, 1H).

**Beispiel 27**

N-[(1R)-1-Cyclopropyl-2,2,2-trifluorethyl]-1-(3,5-difluorpyridin-2-yl)-7-[4-methyl-2-oxopyrrolidin-1-yl]-4-oxo-1,4-dihydro-1,8-naphthyridin-3-carbonsäureamid (Diastereomerengemisch)

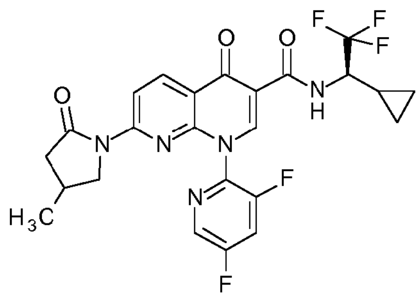

Gemäß allgemeiner Arbeitsvorschrift 2 wurden 150 mg (327 µmol) 7-Chlor-N-[(1R)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 8A) mit 35.8 mg (343 µmol, 95% Reinheit) 4-Methylpyrrolidin-2-on in Gegenwart von 67.8 mg (490 µmol) Kaliumcarbonat, 13.2 mg (59.0 µmol) Palladiumacetat und 68.1 mg (118 µmol) Xantphos in 2.9 ml Dioxan bei 80°C zur Reaktion gebracht. Die Mischung wurde dann auf RT abgekühlt, dann mit Wasser, Ameisensäure und Acetonitril versetzt, filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 43.2 mg (25% d. Th., 100% Reinheit) der Titelverbindung erhalten.
LC-MS (Methode 1): Rt = 1.15 min; MS (ESIpos): m/z = 522 [M+H]+.
$^1$H-NMR (400 MHz, DMSO-d6): δ [ppm] = 10.26 (d, 1H), 9.04 (s, 1H), 8.70 (d, 1H), 8.65 (d, 1H), 8.52 (d, 1H), 8.34-8.45 (m, 1H), 4.35-4.49 (m, 1H), 3.67-3.83 (m, 1H), 3.09-3.24 (m, 1H), 2.68-2.81 (m, 1H), 2.22-2.47 (m, 2H), 1.18-1.29 (m, 1H), 1.03 (dd, 3H), 0.51-0.72 (m, 3H), 0.29-0.40 (m, 1H).
43.2 mg der Titelverbindung (Diastereomerengemisch) wurde durch chirale HPLC in die Diastereomere getrennt (präparative HPLC: Säule Daicel Chiralpak ID 5 µm 20x250 mm; Eluent: 60% Iso-Hexan, 40% Ethanol; Temperatur: 30°C; Fluss: 15 ml/min; UV-Detektion: 220 nm.)
Man erhielt (in der Reihenfolge der Elution von der Säule) 17.0 mg (9.8% d. Th., 100% Reinheit) Diastereomer 1 (Beispiel 28) (99% de) Rt = 12.44 min, und 16.0 mg (9.3% d. Th., 100% Reinheit) Diastereomer 2 (Beispiel 29) (99% de) Rt = 14.26 min.
[Analytische HPLC: Säule Daicel Chiralpak ID 5 µm 250x4.6 mm; Eluent: 50% Iso-Hexan, 50% Ethanol; Temperatur: 30°C; Fluss: 1.0 ml/min; UV-Detektion: 220nm].

**Beispiel 28**

N-[(1R)-1-Cyclopropyl-2,2,2-trifluorethyl]-1-(3,5-difluorpyridin-2-yl)-7-[4-methyl-2-oxopyrrolidin-1-yl]-4-oxo-1,4-dihydro-1,8-naphthyridin-3-carbonsäureamid (Diastereomer 1)

LC-MS (Methode 1): Rt = 1.15 min; MS (ESIpos): m/z = 522 [M+H]+.
$^1$H-NMR (400 MHz, DMSO-d6): δ [ppm] = 10.26 (d, 1H), 9.04 (s, 1H), 8.70 (d, 1H), 8.65 (d, 1H), 8.52 (d, 1H),

8.33-8.44 (m, 1H), 4.42 (sxt, 1H), 3.67-3.83 (m, 1H), 3.09-3.24 (m, 1H), 2.68-2.81 (m, 1H), 2.22-2.48 (m, 2H), 1.18-1.29 (m, 1H), 1.04 (dd, 3H), 0.51-0.72 (m, 3H), 0.29-0.40 (m, 1H).

**Beispiel 29**

N-[(1R)-1-Cyclopropyl-2,2,2-trifluorethyl]-1-(3,5-difluorpyridin-2-yl)-7-[4-methyl-2-oxopyrrolidin-1-yl]-4-oxo-1,4-dihydro-1,8-naphthyridin-3-carbonsäureamid (Diastereomer 2)

LC-MS (Methode 1): Rt = 1.15 min; MS (ESIpos): m/z = 522 [M+H]+.

$^1$H-NMR (400 MHz, DMSO-d6): δ [ppm] = 10.26 (d, 1H), 9.04 (s, 1H), 8.70 (d, 1H), 8.65 (d, 1H), 8.52 (d, 1H), 8.33-8.45 (m, 1H), 4.35-4.49 (m, 1H), 3.67-3.83 (m, 1H), 3.09-3.25 (m, 1H), 2.68-2.81 (m, 1H), 2.22-2.47 (m, 2H), 1.18-1.30 (m, 1H), 1.03 (dd, 3H), 0.50-0.72 (m, 3H), 0.28-0.40 (m, 1H).

**Beispiel 30**

1-(3,5-Difluorpyridin-2-yl)-7-[4-methyl-2-oxopyrrolidin-1-yl]-4-oxo-N-[(2S)-1,1,1-trifluorbutan-2-yl]-1,4-dihydro-1,8-naphthyridin-3-carbonsäureamid (Diastereomerengemisch)

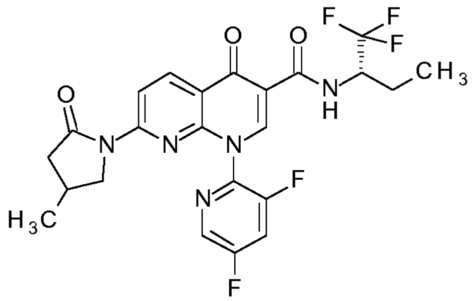

Gemäß allgemeiner Arbeitsvorschrift 2 wurden 150.0 mg (336 μmol) 7-Chlor-1-(3,5-difluorpyridin-2-yl)-4-oxo-N-[(2S)-1,1,1-trifluorbutan-2-yl]-1,4-dihydro-1,8-naphthyridin-3-carbonsäureamid (Beispiel 5A) mit 36.8 mg (353 μmol, 95% Reinheit) 4-Methylpyrrolidin-2-on in Gegenwart von 69.6 mg (504 μmol) Kaliumcarbonat, 13.6 mg (60.0 μmol) Palladiumacetat und 69.9 mg (121 μmol) Xantphos in 3 ml Dioxan bei 80°C zur Reaktion gebracht. Die Mischung wurde dann auf RT abgekühlt, dann mit Wasser, Ameisensäure und Acetonitril versetzt, filtriert und die Rohlösung zweimal mittels präparativer HPLC (Säule: Chromatorex C18, 10 μm, 125*40 mm, Lösungsmittel: Acetonitril, Wasser, 0.1% Ameisensäure) gereinigt und 39.2 mg (23% d. Th., 100% Reinheit) der Titelverbindung erhalten.
LC-MS (Methode 1): Rt = 1.20 min; MS (ESIpos): m/z = 510 [M+H]+.
$^1$H-NMR (500 MHz, DMSO-d6): δ [ppm] = 10.11 (d, 1H), 9.05 (s, 1H), 8.70 (d, 1H), 8.66 (d, 1H), 8.52 (d, 1H), 8.34-8.44 (m, 1H), 4.71-4.83 (m, 1H), 3.67-3.82 (m, 1H), 3.09-3.24 (m, 1H), 2.74 (td, 1H), 2.23-2.47 (m, 2H), 1.84-1.96 (m, 1H), 1.60-1.73 (m, 1H), 0.93-1.09 (m, 6H).

**Beispiel 31**

1-(3,5-Difluorpyridin-2-yl)-7-[7-hydroxy-6,6-dimethyl-5-oxo-4-azaspiro[2.4]hept-4-yl]-4-oxo-N-[(2S)-1,1,1-trifluorbutan-2-yl]-1,4-dihydro-1,8-naphthyridin-3-carbonsäureamid (Diastereomerengemisch)

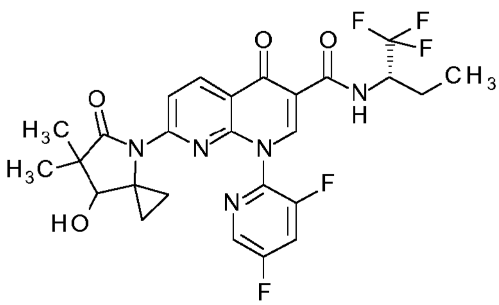

Gemäß allgemeiner Arbeitsvorschrift 2 wurden 36.0 mg (80.5 µmol) 7-Chlor-1-(3,5-difluorpyridin-2-yl)-4-oxo-N-[(2S)-1,1,1-trifluorbutan-2-yl]-1,4-dihydro-1,8-naphthyridin-3-carbonsäureamid (Beispiel 5A) mit 15.0 mg (96.7 µmol) 7-Hydroxy-6,6-dimethyl-4-azaspiro[2.4]heptan-5-on (Beispiel 1D) in Gegenwart von 16.7 mg (121 µmol) Kaliumcarbonat, 3.62 mg (16.1 µmol) Palladiumacetat und 9.32 mg (16.1 µmol) Xantphos in 0.6 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in Acetonitril, Wasser und Ameisensäure gelöst und mittels präparativer HPLC (Säule: Chromatorex C18, 10 µm, 125*40 mm, Lösungsmittel: Acetonitril, Wasser, 0.1% Ameisensäure) gereinigt und 12.5 mg (27% d. Th., 100% Reinheit) der Titelverbindung erhalten.
LC-MS (Methode 1): Rt = 1.06 min; MS (ESIpos): m/z = 566 [M+H]+.
1H-NMR (400 MHz, DMSO-d6): δ [ppm] = 10.07 (d, 1H), 9.07 (s, 1H), 8.65-8.75 (m, 2H), 8.43-8.53 (m, 1H), 8.14 (t, 1H), 5.36 (dd, 1H), 4.69-4.85 (m, 1H), 3.42-3.59 (m, 1H), 1.83-1.96 (m, 1H), 1.59-1.74 (m, 1H), 1.13-1.21 (m, 3H), 1.08 (s, 3H), 0.88-1.05 (m, 5H), 0.36-0.75 (m, 2H).

**Beispiel 32**

N-[(1R)-1-Cyclopropyl-2,2,2-trifluorethyl]-1-(3,5-difluorpyridin-2-yl)-7-[7-hydroxy-6,6-dimethyl-5-oxo-4-azaspiro[2.4]hept-4-yl]-4-oxo-1,4-dihydro-1,8-naphthyridin-3-carbonsäureamid (Diastereomerengemisch)

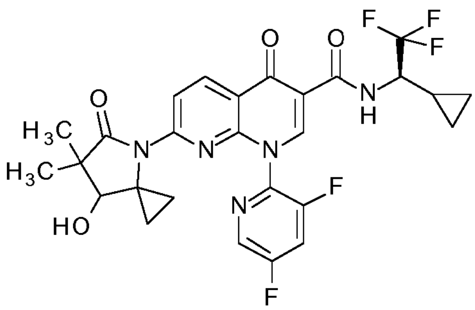

Gemäß allgemeiner Arbeitsvorschrift 2 wurden 24.6 mg (53.7 µmol) 7-Chlor-N-[(1R)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 8A) mit 10.0 mg (64.4 µmol) 7-Hydroxy-6,6-dimethyl-4-azaspiro[2.4]heptan-5-on (Beispiel 1D) in Gegenwart von 11.1 mg (80.5 µmol) Kaliumcarbonat, 2.14 mg (10.7 µmol) Palladiumacetat und 6.21 mg (10.7 µmol) Xantphos in 0.4 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in Acetonitril gelöst und mittels präparativer HPLC (Säule: Chromatorex C18, 10 µm, 125*40 mm, Lösungsmittel: Acetonitril, Wasser, 0.1% Ameisensäure) gereinigt und 5.20 mg (16% d. Th., 96% Reinheit) der Titelverbindung erhalten.
LC-MS (Methode 2): Rt = 2.03 min; MS (ESIpos): m/z = 578 [M+H]+.
$^1$H-NMR (400 MHz, DMSO-d6): δ [ppm] = 10.21 (dd, 1H), 9.06 (s, 1H), 8.65-8.75 (m, 2H), 8.42-8.52 (m, 1H), 8.14 (t,

1H), 5.36 (dd, 1H), 4.41 (sxt, 1H), 3.42-3.58 (m, 1H), 1.19-1.29 (m, 1H), 1.17 (d, 3H), 1.08 (s, 3H), 0.95-1.06 (m, 2H), 0.31-0.73 (m, 6H).

**Beispiel 33**

N-[(1S)-1-Cyclopropyl-2,2,2-trifluorethyl]-1-(3,5-difluorpyridin-2-yl)-7-[7-hydroxy-6,6-dimethyl-5-oxo-4-azaspiro[2.4]hept-4-yl]-4-oxo-1,4-dihydro-1,8-naphthyridin-3-carbonsäureamid (Diastereomerengemisch)

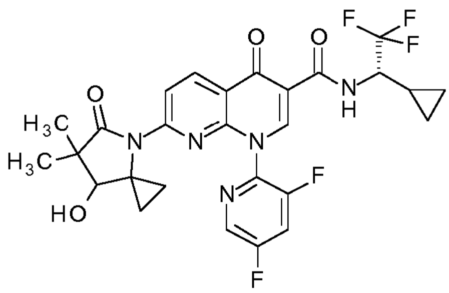

Gemäß allgemeiner Arbeitsvorschrift 2 wurden 30.0 mg (65.4 µmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 12.2 mg (78.5 µmol) 7-Hydroxy-6,6-dimethyl-4-azaspiro[2.4]heptan-5-on (Beispiel 1D) in Gegenwart von 13.6 mg (98.1 µmol) Kaliumcarbonat, 2.94 mg (13.1 µmol) Palladiumacetat und 7.57 mg (13.1 µmol) Xantphos in 0.5 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in Acetonitril, Wasser und Ameisensäure gelöst, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 20.4 mg (52% d. Th., 97% Reinheit) der Titelverbindung erhalten.
LC-MS (Methode 2): Rt = 2.03 min; MS (ESIpos): m/z = 578 [M+H]+.
$^{1}$H-NMR (400 MHz, DMSO-d6): δ [ppm] = 10.21 (br. dd, 1H), 9.06 (s, 1H), 8.65-8.75 (m, 2H), 8.43-8.53 (m, 1H), 8.14 (t, 1H), 5.36 (dd, 1H), 4.34-4.48 (m, 1H), 3.42-3.59 (m, 1H), 1.20-1.28 (m, 1H), 1.13-1.20 (m, 3H), 1.08 (s, 3H), 0.89-1.06 (m, 2H), 0.29-0.75 (m, 6H).

**Beispiel 34**

1-(3,5-Difluorpyridin-2-yl)-4-oxo-7-(2-oxopiperidin-1-yl)-N-[(2R)-1,1,1-trifluorbulan-2-yl]-1,4-dihydro-1,8-naphthyridin-3-carbonsäureamid

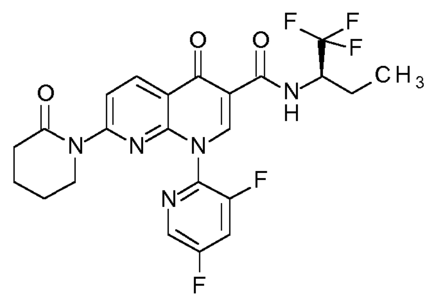

Gemäß allgemeiner Arbeitsvorschrift 2 wurden 25.0 mg (56.0 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 6.66 mg (67.0 µmol)

Piperidin-2-on in Gegenwart von 11.6 mg (84.0 µmol) Kaliumcarbonat, 2.51 mg (11.0 µmol) Palladiumacetat und 6.48 mg (11.0 µmol) Xantphos in 0.5 ml Dioxan bei 80°C zur Reaktion gebracht. Die Mischung wurde anschließend mit Acetonitril, Wasser und Ameisensäure verdünnt, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 15.9 mg (56% d. Th., 100% Reinheit) der Titelverbindung erhalten.
LC-MS (Methode 2): Rt = 2.06 min; MS (ESIpos): m/z = 510 [M+H]+.
$^{1}$H-NMR (400 MHz, DMSO-d6): δ [ppm] = 10.10 (d, 1H), 9.06 (s, 1H), 8.59-8.69 (m, 2H), 8.38 (ddd, 1H), 8.16 (d, 1H), 4.70-4.85 (m, 1H), 3.47-3.63 (m, 2H), 1.84-1.97 (m, 1H), 1.60-1.82 (m, 5H), 0.92-1.04 (m, 3H).

## Beispiel 35

N-[1-Cyclopropyl-2,2,2-trifluorethyl]-1-(3,5-difluorpyridin-2-yl)-7-[(3R,4S)-3,4-dihydroxypyrrolidin-1-yl]-4-oxo-1,4-dihydro-1,8-naphthyridin-3-carbonsäureamid (Diastereomerengemisch)

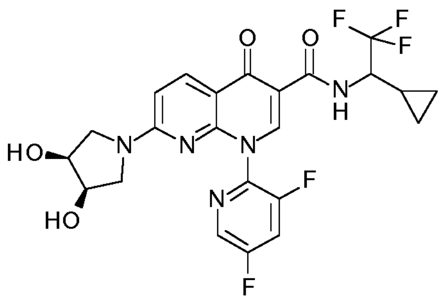

Gemäß allgemeiner Arbeitsvorschrift 3 wurden 50.0 mg (109 µmol) 7-Chlor-N-[1-cyclopropyl-2,2,2-trifluorethyl]-1-(3,5-difluorpyridin-2-yl)-4-oxo-1,4-dihydro-1,8-naphthyridin-3-carbonsäureamid (Beispiel 7A) mit 18.3 mg (131 µmol) (3R,4S)-Pyrrolidin-3,4-diol Hydrochlorid in Gegenwart von 66.0 µl (381 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 43 mg (75% d. Th., 100% Reinheit) der Titelverbindung erhalten.
LC-MS (Methode 1): Rt = 0.89 min; MS (ESIpos): m/z = 526 [M+H]+.
$^{1}$H-NMR (400 MHz, DMSO-d6): δ [ppm] = 10.55 (dd, 1H), 8.81 (s, 1H), 8.58-8.65 (m, 1H), 8.24-8.39 (m, 2H), 6.76 (d, 1H), 4.99-5.11 (m, 1H), 4.87-4.98 (m, 1H), 4.40 (sxt, 1H), 3.97-4.19 (m, 2H), 3.54-3.68 (m, 1H), 3.16-3.29 (m, 2H), 2.93-3.13 (m, 1H), 1.15-1.26 (m, 1H), 0.47-0.71 (m, 3H), 0.28-0.39 (m, 1H).

## Beispiel 36

N-[1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomerengemisch)

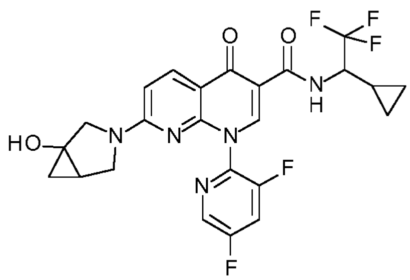

Gemäß allgemeiner Arbeitsvorschrift 3 wurden 60.0 mg (131 µmol) 7-Chlor-N-[1-cyclopropyl-2,2,2-trifluorethyl]-1-(3,5-difluorpyridin-2-yl)-4-oxo-1,4-dihydro-1,8-naphthyridin-3-carbonsäureamid (Beispiel 7A) mit 21.3 mg (157 µmol, 91% Reinheit) 3-Azabicyclo[3.1.0]hexan-1-ol-Hydrochlorid (Racemat) in Gegenwart von 79.7 µl (458 µmol) DTPEA in

0.6 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 48 mg (70% d. Th., 100% Reinheit) der Titelverbindung erhalten.
LC-MS (Methode 2): Rt = 1.90 min; MS (ESIpos): m/z = 522 [M+H]+.
$^1$H-NMR (400 MHz, DMSO-d6): δ [ppm] = 10.52 (dd, 1H), 8.82 (s, 1H), 8.61 (br. s, 1H), 8.24-8.43 (m, 2H), 6.68-6.85 (m, 1H), 5.91-6.12 (m, 1H), 4.39 (sxt, 1H), 3.34-3.95 (m, 3H), 3.05-3.27 (m, 1H), 1.49-1.71 (m, 1H), 1.15-1.26 (m, 1H), 0.97-1.10 (m, 1H), 0.27-0.70 (m, 5H).

**Beispiel 37**

N-[(1R)-1-Cyclopropyl-2,2,2-trifluorethyl]-1-(3,5-difluorpyridin-2-yl)-7-[1-hydroxy-3-azabicyclo-[3.1.0]hex-3-yl]-4-oxo-1,4-dihydro-1,8-naphthyridin-3-carbonsäureamid (Diastereomerengemisch)

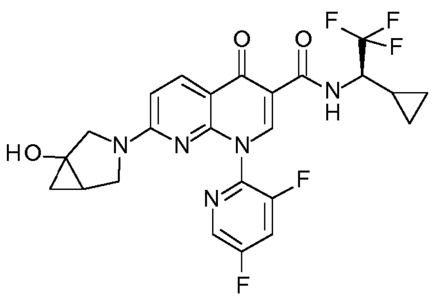

Gemäß allgemeiner Arbeitsvorschrift 3 wurden 89.0 mg (194 µmol) 7-Chlor-N-[(1R)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 8A) mit 34.7 mg (233 µmol, 91% Reinheit) 3-Azabicyclo[3.1.0]hexan-1-ol-Hydrochlorid (Racemat) in Gegenwart von 118 µl (679 µmol) DIPEA in 0.81 ml DMF zur Reaktion gebracht. Die Mischung wurde anschließend mit Acetonitril, Wasser und Ameisensäure verdünnt und die Rohlösung mittels präparativer HPLC (Säule: Chromatorex C18, 10 µm, 125*40 mm, Lösungsmittel: Acetonitril, Wasser, 0.1% Ameisensäure) gereinigt und 60 mg (59% d. Th., 100% Reinheit) der Titelverbindung erhalten.
LC-MS (Methode 1): Rt = 1.06 min; MS (ESIpos): m/z = 522 [M+H]+.
$^1$H-NMR (400 MHz, DMSO-d6): δ [ppm] = 10.52 (dd, 1H), 8.82 (s, 1H), 8.61 (br. s, 1H), 8.23-8.44 (m, 2H), 6.68-6.85 (m, 1H), 5.89-6.13 (m, 1H), 4.39 (sxt, 1H), 3.35-3.96 (m, 3H), 3.04-3.26 (m, 1H), 1.49-1.71 (m, 1H), 1.15-1.26 (m, 1H), 0.95-1.10 (m, 1H), 0.26-0.71 (m, 5H).

60.0 mg der Titelverbindung (Diastereomerengemisch) wurde durch chirale HPLC in die Diastereomere getrennt (präparative HPLC: Säule Daicel Chiralpak AS-H 5 µm 250x20 mm; Eluent: 80% Iso-Hexan, 20% Iso-Propanol + 0.2% DEA; Temperatur: 23°C; Fluss: 20 ml/min; UV-Detektion: 270 nm.)

Man erhielt (in der Reihenfolge der Elution von der Säule) 15.5 mg Diastereomer 1 (99% de) Rt = 7.52 min und 19.2 mg (96% de) Diastereomer 2 Rt = 11.03 min.

[Analytische HPLC: Säule Daicel Chiralpak AS-3 3 µm 50x4.6 mm; Eluent: 80% Iso-Hexan, 20% Iso-propanol + 0.2% DEA; Fluss: 1.0 ml/min; UV-Detektion: 220 nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 10.0 mg (10% d. Th., 100% Reinheit) der Titelverbindung aus Beispiel 38 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 9.5 mg (9% d. Th., 100% Reinheit) der Titelverbindung aus Beispiel 39 erhalten.

**Beispiel 38**

N-[(1R)-1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomer 1)

LC-MS (Methode 2): Rt = 1.90 min; MS (ESIpos): m/z = 522 [M+H]+.

$^1$H-NMR (400 MHz, DMSO-d6): δ [ppm] = 10.52 (dd, 1H), 8.82 (s, 1H), 8.61 (br. s, 1H), 8.24-8.43 (m, 2H),

6.68-6.85 (m, 1H), 5.90-6.12 (m, 1H), 4.33-4.46 (m, 1H), 3.37-3.97 (m, 3H), 3.06-3.26 (m, 1H), 1.50-1.70 (m, 1H), 1.16-1.26 (m, 1H), 0.97-1.11 (m, 1H), 0.28-0.70 (m, 5H).

**Beispiel 39**

N-[(1R)-1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomer 2)

LC-MS (Methode 2): Rt = 1.90 min; MS (ESIpos): m/z = 522 [M+H]+.

$^1$H-NMR (400 MHz, DMSO-d6): δ [ppm] = 10.52 (dd, 1H), 8.81 (s, 1H), 8.61 (br. s, 1H), 8.24-8.44 (m, 2H), 6.68-6.84 (m, 1H), 5.90-6.13 (m, 1H), 4.39 (sxt, 1H), 3.39-3.96 (m, 3H), 3.06-3.27 (m, 1H), 1.50-1.70 (m, 1H), 1.16-1.26 (m, 1H), 0.97-1.10 (m, 1H), 0.28-0.70 (m, 5H).

**Beispiel 40**

N-[1-Cyclopropyl-2,2,2-trifluorethyl]-1-(3,5-difluorpyridin-2-yl)-7-[(3R,4R)-3,4-dihydroxypyrrolidin-1-yl]-4-oxo-1,4-dihydro-1,8-naphthyridin-3-carbonsäureamid (Diastereomerengemisch)

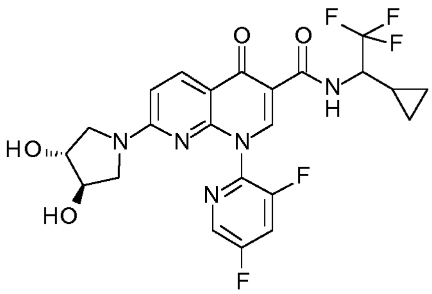

Gemäß allgemeiner Arbeitsvorschrift 3 wurden 50.0 mg (109 µmol) 7-Chlor-N-[1-cyclopropyl-2,2,2-trifluorethyl]-1-(3,5-difluorpyridin-2-yl)-4-oxo-1,4-dihydro-1,8-naphthyridin-3-carbonsäureamid (Beispiel 7A) mit 18.3 mg (131 µmol) (3R,4R)-Pyrrolidin-3,4-diol-Hydrochlorid in Gegenwart von 66.0 µl (381 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 38.3 mg (67% d. Th., 100% Reinheit) der Titelverbindung erhalten.
LC-MS (Methode 2): Rt = 1.58 min; MS (ESIpos): m/z = 526 [M+H]+.
$^1$H-NMR (400 MHz, DMSO-d6): δ [ppm] = 10.56 (dd, 1H), 8.81 (s, 1H), 8.61 (d, 1H), 8.24-8.38 (m, 2H), 6.78 (d, 1H), 5.03-5.31 (m, 2H), 4.40 (sxt, 1H), 3.88-4.09 (m, 2H), 3.62 (ddd, 1H), 3.33-3.41 (m, 1H), 3.01-3.27 (m, 2H), 1.16-1.27 (m, 1H), 0.47-0.70 (m, 3H), 0.28-0.39 (m, 1H).

**Beispiel 41**

1-(3,5-Difluorpyridin-2-yl)-7-[(3R,4S)-3,4-dihydroxypyrrolidin-1-yl]-4-oxo-N-[(2R)-1,1,1-trifluorbutan-2-yl]-1,4-dihydro-1,8-naphthyridin-3-carbonsäureamid

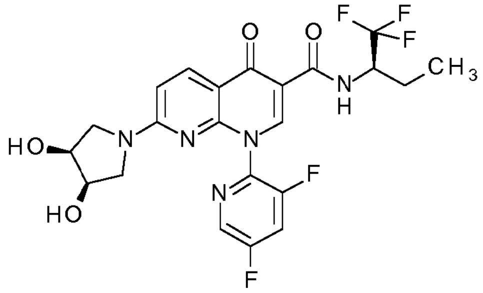

Gemäß allgemeiner Arbeitsvorschrift 3 wurden 50.0 mg (112 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 18.7 mg (134 µmol) (3R,4S)-Pyrrolidin-3,4-diol-Hydrochlorid in Gegenwart von 68.0 µl (392 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 51.9 mg (90% d. Th., 100% Reinheit) der Titelverbindung erhalten.
LC-MS (Methode 2): Rt = 1.59 min; MS (ESIpos): m/z = 514 [M+H]+.
$^1$H-NMR (400 MHz, DMSO-d6): δ [ppm] = 10.42 (d, 1H), 8.82 (s, 1H), 8.62 (br. s, 1H), 8.30-8.39 (m, 1H), 8.27 (d, 1H), 6.76 (d, 1H), 5.04 (dd, 1H), 4.93 (dd, 1H), 4.68-4.81 (m, 1H), 4.09-4.19 (m, 1H), 3.96-4.09 (m, 1H), 3.54-3.68 (m, 1H), 3.16-3.27 (m, 1H), 2.93-3.13 (m, 1H), 1.81-1.94 (m, 1H), 1.57-1.71 (m, 1H), 0.92-1.03 (m, 3H).

## Beispiel 42

1-(3,5-Difluorpyridin-2-yl)-7-[(3R,4R)-3,4-dihydroxypyrrolidin-1-yl]-4-oxo-N-[(2R)-1,1,1-trifluorbutan-2-yl]-1,4-dihydro-1,8-naphthyridin-3-carbonsäureamid

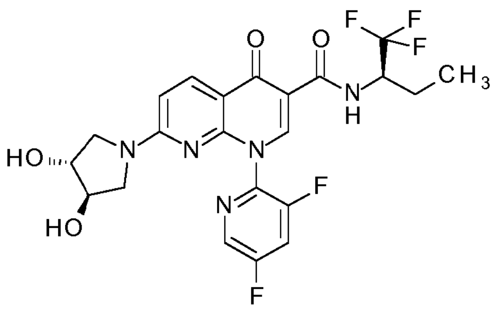

Gemäß allgemeiner Arbeitsvorschrift 3 wurden 50.0 mg (112 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 18.7 mg (134 µmo1) (3R,4R)-Pyrrolidin-3,4-diol-Hydrochlorid in Gegenwart von 68.0 µl (392 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 52.3 mg (91% d. Th., 100% Reinheit) der Titelverbindung erhalten.
LC-MS (Methode 2): Rt = 1.54 min; MS (ESIpos): m/z = 514 [M+H]+.
$^1$H-NMR (400 MHz, DMSO-d6): δ [ppm] = 10.43 (d, 1H), 8.82 (d, 1H), 8.62 (d, 1H), 8.30-8.37 (m, 1H), 8.28 (d, 1H), 6.78 (d, 1H), 5.01-5.32 (m, 2H), 4.66-4.83 (m, 1H), 3.86-4.11 (m, 2H), 3.55-3.70 (m, 1H), 3.33-3.40 (m, 1H), 3.19-3.29 (m, 1H), 3.01-3.18 (m, 1H), 1.81-1.96 (m, 1H), 1.56-1.73 (m, 1H), 0.97 (dd, 3H).

## Beispiel 43

1-(3,5-Difluorpyridin-2-yl)-7-[1-hydroxy-3-azabicyclo[3.1.0]hex-3-yl]-4-oxo-N-[(2R)-1,1,1-trifluorbutan-2-yl]-1,4-dihydro-1,8-naphthyridin-3-carbonsäureamid (Diastereomerengemisch)

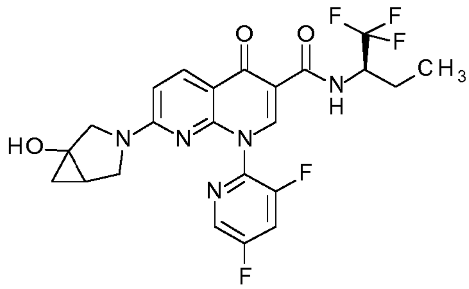

Gemäß allgemeiner Arbeitsvorschrift 3 wurden 50.0 mg (112 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 18.2 mg (134 µmol, 91% Reinheit) 3-Azabicyclo[3.1.0]hexan-1-ol-Hydrochlorid (Racemat) in Gegenwart von 66.0 µl (392 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 42.9 mg (75% d. Th., 100% Reinheit) der Titelverbindung erhalten.
LC-MS (Methode 2): Rt = 1.86 min; MS (ESIpos): m/z = 510 [M+H]+.
$^1$H-NMR (400 MHz, DMSO-d6): δ [ppm] = 10.38 (d, 1H), 8.82 (s, 1H), 8.62 (br. s, 1H), 8.23-8.44 (m, 2H), 6.67-6.84 (m, 1H), 5.90-6.13 (m, 1H), 4.67-4.82 (m, 1H), 3.35-3.96 (m, 3H), 3.04-3.27 (m, 1H), 1.81-1.94 (m, 1H), 1.50-1.71 (m, 2H), 0.90-1.10 (m, 4H), 0.35-0.50 (m, 1H).

31.0 mg der Titelverbindung (Diastereomerengemisch) wurde durch chirale HPLC in die Diastereomere getrennt (präparative HPLC: Säule Daicel Chiralcel OX-H 5 µm 250x20 mm; Eluent: 75% Iso-Hexan, 25% Iso-Propanol + 0.2% DEA; Temperatur: 45°C; Fluss: 15 ml/min; UV-Detektion: 220 nm.)

Man erhielt (in der Reihenfolge der Elution von der Säule) 14.0 mg Diastereomer 1 (99% de) Rt = 11.78 min und 14.0 mg (99% de) Diastereomer 2 Rt = 15.10 min.

[Analytische HPLC: Säule Daicel Chiralcel OX-H 5 µm 250x4.6 mm; Eluent: 75% Iso-Hexan, 25% Iso-propanol + 0.2% DEA; Temperatur: 45°C; Fluss: 1.0 ml/min; UV-Detektion: 235nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 9.4 mg (16% d. Th., 100% Reinheit) der Titelverbindung aus Beispiel 44 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 9.2 mg (16% d. Th., 100% Reinheit) der Titelverbindung aus Beispiel 45 erhalten.

## Beispiel 44

1-(3,5-Difluorpyridin-2-yl)-7-[1-hydroxy-3-azabicyclo[3.1.0]hex-3-yl]-4-oxo-N-[(2R)-1,1,1-trifluorbutan-2-yl]-1,4-dihydro-1,8-naphthyridin-3-carbonsäureamid (Diastereomer 1)

LC-MS (Methode 2): Rt = 1.88 min; MS (ESIpos): m/z = 510 [M+H]+.

$^1$H-NMR (400 MHz, DMSO-d6): δ [ppm] = 10.38 (d, 1H), 8.83 (s, 1H), 8.62 (br. s, 1H), 8.22-8.44 (m, 2H), 6.68-6.84 (m, 1H), 5.89-6.12 (m, 1H), 4.67-4.81 (m, 1H), 3.34-3.95 (m, 3H), 3.05-3.26 (m, 1H), 1.81-1.95 (m, 1H), 1.51-1.72 (m, 2H), 0.90-1.10 (m, 4H), 0.35-0.51 (m, 1H).

## Beispiel 45

1-(3,5-Difluorpyridin-2-yl)-7-[1-hydroxy-3-azabicyclo[3.1.0]hex-3-yl]-4-oxo-N-[(2R)-1,1,1-trifluorbutan-2-yl]-1,4-dihydro-1,8-naphthyridin-3-carbonsäureamid (Diastereomer 2)

LC-MS (Methode 2): Rt = 1.88 min; MS (ESIpos): m/z = 510 [M+H]+.

$^1$H-NMR (400 MHz, DMSO-d6): δ [ppm] = 10.38 (d, 1H), 8.82 (s, 1H), 8.62 (br. s, 1H), 8.23-8.44 (m, 2H), 6.68-6.85 (m, 1H), 5.90-6.13 (m, 1H), 4.66-4.82 (m, 1H), 3.39-3.95 (m, 3H), 3.07-3.20 (m, 1H), 1.81-1.94 (m, 1H), 1.50-1.72 (m, 2H), 0.89-1.10 (m, 4H), 0.35-0.50 (m, 1H).

**Beispiel 46**

1-(3,5-Difluorpyridin-2-yl)-7-[3-hydroxy-3-methylpyrrolidin-1-yl]-4-oxo-N-[(2R)-1,1,1-trifluorbutan-2-yl]-1,4-dihydro-1,8-naphthyridin-3-carbonsäureamid (Diastereomerengemisch)

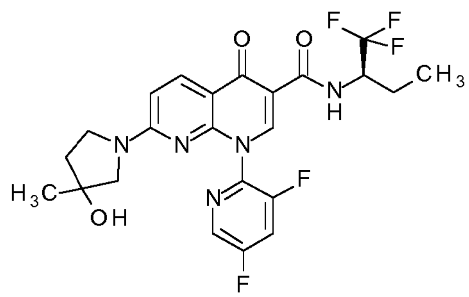

Gemäß allgemeiner Arbeitsvorschrift 3 wurden 50.0 mg (112 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 18.5 mg (134 µmol) 3-Methylpyrrolidin-3-ol-Hydrochlorid (Racemat) in Gegenwart von 68.0 µl (392 µmol) DIPEA in 0.5 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 49.4 mg (86% d. Th., 100% Reinheit) der Titelverbindung erhalten.
LC-MS (Methode 2): Rt = 1.89 min; MS (ESIpos): m/z = 512 [M+H]+.
$^1$H-NMR (400 MHz, DMSO-d6): δ [ppm] = 10.43 (d, 1H), 8.81 (s, 1H), 8.61 (br. s, 1H), 8.22-8.39 (m, 2H), 6.67-6.82 (m, 1H), 4.67-4.95 (m, 2H), 3.48-3.63 (m, 1H), 3.34-3.42 (m, 1H), 2.87-3.28 (m, 2H), 1.56-1.99 (m, 4H), 1.21-1.37 (m, 3H), 0.91-1.02 (m, 3H).

**Beispiel 47**

1-(3-Chlorpyridin-2-yl)-7-[1-hydroxy-3-azabicyclo[3.1.0]hex-3-yl]-4-oxo-N-[(2R)-1,1,1-trifluorbutan-2-yl]-1,4-dihydro-1,8-naphthyridin-3-carbonsäureamid (Diastereomerengemisch)

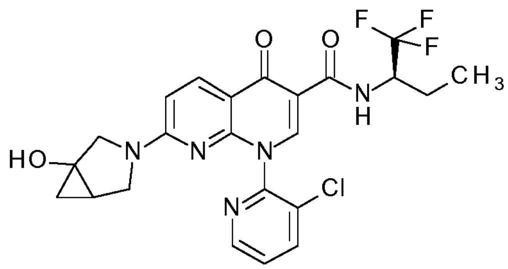

Gemäß allgemeiner Arbeitsvorschrift 3 wurden 100 mg (225 µmol) 7-Chlor-1-(3-chlorpyridin-2-yl)-4-oxo-N-[(2R)-1,1,1-trifluorbutan-2-yl]-1,4-dihydro-1,8-naphthyridin-3-carbonsäureamid (Beispiel 18C) mit 36.5 mg (270 µmol, 91% Reinheit) 3-Azabicyclo[3.1.0]hexan-1-ol-Hydrochlorid (Racemat) in Gegenwart von 137 µl (786 µmol) DIPEA in 1 ml DMF zur Reaktion gebracht. Die Mischung wurde anschließend mit Acetonitril, Wasser und Ameisensäure verdünnt und die Rohlösung mittels präparativer HPLC (Säule: Chromatorex C18, 10 µm, 125*40 mm, Lösungsmittel: Acetonitril, Wasser, 0.1% Ameisensäure) gereinigt und 90.8 mg (80% d. Th., 100% Reinheit) der Titelverbindung erhalten.
LC-MS (Methode 1): Rt = 0.98 min; MS (ESIpos): m/z = 508 [M+H]+.
$^1$H-NMR (400 MHz, DMSO-d6): δ [ppm] = 10.43 (d, 1H), 8.78 (d, 1H), 8.59-8.67 (m, 1H), 8.28 (br. d, 2H), 7.67-7.77 (m, 1H), 6.67-6.82 (m, 1H), 5.87-6.10 (m, 1H), 4.67-4.80 (m, 1H), 3.58-3.94 (m, 1H), 3.36-3.57 (m, 2H), 2.94-3.19 (m, 1H), 1.81-1.94 (m, 1H), 1.47-1.71 (m, 2H), 0.97 (t, 4H), 0.39 (t, 1H).

**Beispiel 48**

1-(3-Chlor-5-fluorpyridin-2-yl)-7-[1-hydroxy-3-azabicyclo[3.1.0]hex-3-yl]-4-oxo-N-[(2R)-1,1,1-trifluorbutan-2-yl]-1,4-dihydro-1,8-naphthyridin-3-carbonsäureamid (Diastereomerengemisch)

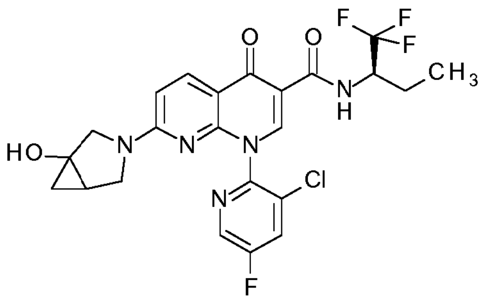

Gemäß allgemeiner Arbeitsvorschrift 3 wurden 70.0 mg (151 µmol) 7-Chlor-1-(3-chlor-5-fluorpyridin-2-yl)-4-oxo-N-[(2R)-1,1,1-trifluorbutan-2-yl]-1,4-dihydro-1,8-naphthyridin-3-carbonsäureamid (Beispiel 16C) mit 24.6 mg (181 µmol, 91% Reinheit) 3-Azabicyclo[3.1.0]hexan-1-ol-Hydrochlorid (Racemat) in Gegenwart von 92.0 µl (529 µmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 60.5 mg (76% d. Th., 100% Reinheit) der Titelverbindung erhalten.
LC-MS (Methode 1): Rt = 1.01 min; MS (ESIpos): m/z = 526 [M+H]+.
$^{1}$H-NMR (500 MHz, DMSO-d6): δ [ppm] = 10.42 (d, 1H), 8.82 (d, 1H), 8.68-8.76 (m, 1H), 8.45-8.57 (m, 1H), 8.27 (d, 1H), 6.68-6.81 (m, 1H), 5.89-6.11 (m, 1H), 4.68-4.80 (m, 1H), 3.59-3.93 (m, 1H), 3.35-3.58 (m, 2H), 3.02-3.21 (m, 1H), 1.83-1.93 (m, 1H), 1.49-1.70 (m, 2H), 0.94-1.07 (m, 4H), 0.40 (br. t, 1H).

**Beispiel 49**

1-(3-Chlor-5-fluorpyridin-2-yl)-7-[3-hydroxy-3-methylpyrrolidin-1-yl]-4-oxo-N-[(2R)-1,1,1-trifluorbutan-2-yl]-1,4-dihydro-1,8-naphthyridin-3-carbonsäureamid (Diastereomerengemisch)

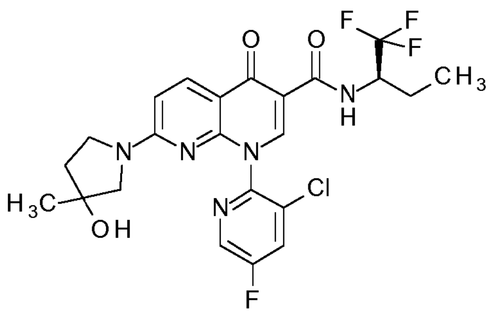

Gemäß allgemeiner Arbeitsvorschrift 3 wurden 70.0 mg (151 µmol) 7-Chlor-1-(3-chlor-5-fluorpyridin-2-yl)-4-oxo-N-[(2R)-1,1,1-trifluorbutan-2-yl]-1,4-dihydro-1,8-naphthyridin-3-carbonsäureamid (Beispiel 16C) mit 25.0 mg (181 µmol) 3-Methylpyrrolidin-3-ol-Hydrochlorid (Racemat) in Gegenwart von 92.0 µl (529 µmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 71.6 mg (90% d. Th., 100% Reinheit) der Titelverbindung erhalten.
LC-MS (Methode 1): Rt = 1.02 min; MS (ESIpos): m/z = 528 [M+H]+.
$^{1}$H-NMR (500 MHz, DMSO-d6): δ [ppm] = 10.46 (d, 1H), 8.80 (br. s, 1H), 8.68-8.76 (m, 1H), 8.45-8.55 (m, 1H), 8.27 (t, 1H), 6.66-6.79 (m, 1H), 4.67-4.91 (m, 2H), 3.48-3.59 (m, 1H), 2.84-3.40 (m, 3H), 1.58-1.96 (m, 4H), 1.19-1.36 (m, 3H),

0.97 (t, 3H).

**Beispiel 50**

1-(3-Chlor-5-fluorpyridin-2-yl)-N-[(1R)-1-cyclopropyl-2,2,2-trifluorethyl]-7-[(3R,4S)-3,4-dihydroxypyrrolidin-1-yl]-4-oxo-1,4-dihydro-1,8-naphthyridin-3-carbonsäureamid

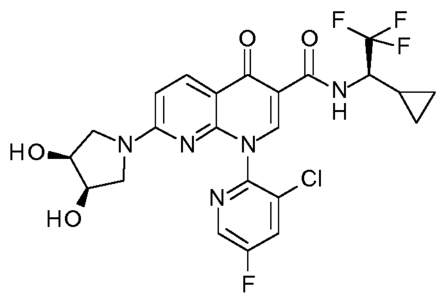

Gemäß allgemeiner Arbeitsvorschrift 3 wurden 40.0 mg (84.2 µmol) 7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid (Beispiel 17A) mit 14.1 mg (101 µmol) (3R,4S)-Pyrrolidin-3,4-diol-Hydrochlorid in Gegenwart von 51.0 µl (295 µmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 40.2 mg (88% d. Th., 100% Reinheit) der Titelverbindung erhalten.
LC-MS (Methode 2): Rt = 1.70 min; MS (ESIpos): m/z = 542 [M+H]+.
$^1$H-NMR (400 MHz, DMSO-d6): δ [ppm] = 10.58 (dd, 1H), 8.76-8.84 (m, 1H), 8.71 (dd, 1H), 8.50 (d, 1H), 8.27 (d, 1H), 6.74 (d, 1H), 4.84-5.10 (m, 2H), 4.31-4.47 (m, 1H), 3.92-4.19 (m, 2H), 3.50-3.67 (m, 1H), 3.08-3.28 (m, 2H), 2.87-3.06 (m, 1H), 1.13-1.27 (m, 1H), 0.46-0.71 (m, 3H), 0.27-0.39 (m, 1H).

**Beispiel 51**

1-(3-Chlor-5-fluorpyridin-2-yl)-N-[(1R)-1-cyclopropyl-2,2,2-trifluorethyl]-7-[(3R,4R)-3,4-dihydroxypyrrolidin-1-yl]-4-oxo-1,4-dihydro-1,8-naphthyridin-3-carbonsäureamid

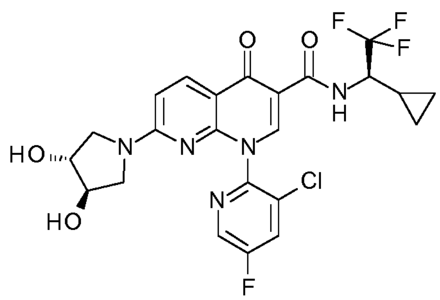

Gemäß allgemeiner Arbeitsvorschrift 3 wurden 40.0 mg (84.2 µmol) 7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid (Beispiel 17A) mit 14.1 mg (101 µmol) (3R,4R)-Pyrrolidin-3,4-diol-Hydrochlorid in Gegenwart von 51.0 µl (295 µmol) DIPEA in 0.4 ml DMF zur Reaktion gebracht. Die Mischung wurde anschließend mit Acetonitril, Wasser und Ameisensäure verdünnt und die Rohlösung mittels präparativer HPLC (Säule: Chromatorex C18, 10 µm, 125*40 mm, Lösungsmittel: Acetonitril, Wasser, 0.1% Ameisensäure) gereinigt und 39.4 mg (86% d. Th., 100% Reinheit) der Titelverbindung erhalten.
LC-MS (Methode 2): Rt = 1.65 min; MS (ESIpos): m/z = 542 [M+H]+.
$^1$H-NMR (400 MHz, DMSO-d6): δ [ppm] = 10.59 (dd, 1H), 8.80 (d, 1H), 8.71 (t, 1H), 8.50 (dt, 1H), 8.28 (d, 1H), 6.76 (d,

1H), 5.04-5.29 (m, 2H), 4.33-4.46 (m, 1H), 4.04 (br. s, 1H), 3.91 (br. s, 1H), 3.61 (td, 1H), 3.12-3.39 (m, 2H), 3.03 (dd, 1H), 1.14-1.27 (m, 1H), 0.46-0.71 (m, 3H), 0.29-0.39 (m, 1H).

**Beispiel 52**

1-(3-Chlor-5-fluorpyridin-2-yl)-N-[(1R)-1-cyclopropyl-2,2,2-trifluorethyl]-7-[1-hydroxy-3-azabicyclo[3.1.0]hex-3-yl]-4-oxo-1,4-dihydro-1,8-naphthyridin-3-carbonsäureamid (Diastereomerengemisch)

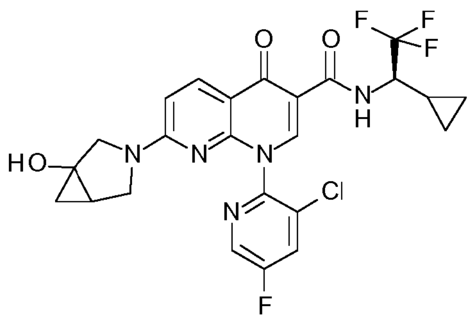

Gemäß allgemeiner Arbeitsvorschrift 3 wurden 70.0 mg (147 µmol) 7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid (Beispiel 17A) mit 24.0 mg (177 µmol, 91% Reinheit) 3-Azabicyclo[3.1.0]hexan-1-ol-Hydrochlorid (Racemat) in Gegenwart von 90.0 µl (516 µmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 62.4 mg (78% d. Th., 99% Reinheit) der Titelverbindung erhalten.
LC-MS (Methode 2): Rt = 1.96 min; MS (ESIpos): m/z = 538 [M+H]+.
$^1$H-NMR (400 MHz, DMSO-d6): δ [ppm] = 10.55 (dd, 1H), 8.81 (d, 1H), 8.72 (br. s, 1H), 8.44-8.58 (m, 1H), 8.28 (d, 1H), 6.68-6.82 (m, 1H), 5.89-6.11 (m, 1H), 4.32-4.45 (m, 1H), 3.59-3.94 (m, 1H), 3.38-3.56 (m, 1.5H), 2.99-3.28 (m, 1.5H), 1.49-1.70 (m, 1H), 1.15-1.26 (m, 1H), 0.96-1.08 (m, 1H), 0.48-0.70 (m, 3H), 0.29-0.44 (m, 2H).

**Beispiel 53**

1-(3-Chlor-5-fluorpyridin-2-yl)-N-[(1R)-1-cyclopropyl-2,2,2-trifluorethyl]-7-[3-hydroxy-3-methylpyrrolidin-1-yl]-4-oxo-1,4-dihydro-1,8-naphthyridin-3-carbonsäureamid (Diastereomerengemisch)

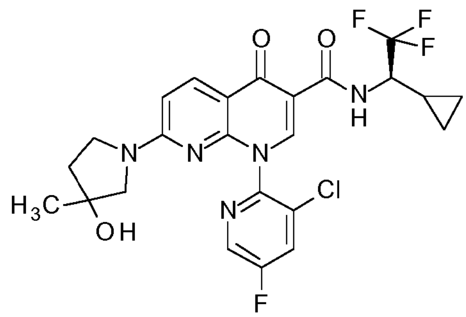

Gemäß allgemeiner Arbeitsvorschrift 3 wurden 70.0 mg (147 µmol) 7-Chlor-1-(3-chlor-5-fluorpyridin-2-yl)-N-[(1R)-1-cyclopropyl-2,2,2-trifluorethyl]-4-oxo-1,4-dihydro-1,8-naphthyridin-3-carbonsäureamid (Beispiel 17A) mit 24.3 mg (177 µmol) 3-Methylpyrrolidin-3-ol-Hydrochlorid (Racemat) in Gegenwart von 90.0 µl (516 µmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 71.7 mg (90% d. Th., 100% Reinheit) der Titelverbindung erhalten.

LC-MS (Methode 2): Rt = 1.98 min; MS (ESIpos): m/z = 540 [M+H]+.
$^1$H-NMR (400 MHz, DMSO-d6): δ [ppm] = 10.60 (dd, 1H), 8.79 (br. s, 1H), 8.66-8.75 (m, 1H), 8.49 (t, 1H), 8.27 (t, 1H), 6.65-6.80 (m, 1H), 4.73-4.91 (m, 1H), 4.33-4.45 (m, 1H), 3.48-3.60 (m, 1H), 3.28-3.44 (m, 1H), 2.84-3.27 (m, 2H), 1.71-1.96 (m, 2H), 1.15-1.36 (m, 4H), 0.46-0.71 (m, 3H), 0.29-0.39 (m, 1H).

**Beispiel 54**

1-(3-Chlorpyridin-2-yl)-N-[(1R)-1-cyclopropyl-2,2,2-trifluorethyl]-7-[1-hydroxy-3-azabicyclo[3.1.0]-hex-3-yl]-4-oxo-1,4-dihydro-1,8-naphthyridin-3-carbonsäureamid (Diastereomerengemisch)

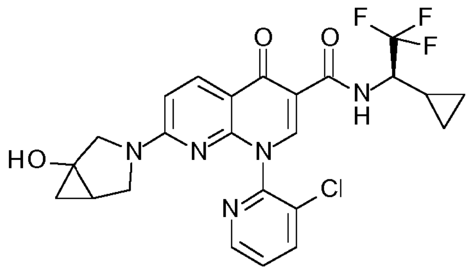

Gemäß allgemeiner Arbeitsvorschrift 3 wurden 70.0 mg (153 μmol) 7-Chlor-1-(3-chlorpyridin-2-yl)-N-[(1R)-1-cyclopropyl-2,2,2-trifluorethyl]-4-oxo-1,4-dihydro-1,8-naphthyridin-3-carbonsäureamid (Beispiel 19A) mit 24.9 mg (184 μmol, 91% Reinheit) 3-Azabicyclo[3.1.0]hexan-1-ol-Hydrochlorid (Racemat) in Gegenwart von 93.0 μl (536 μmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 61.5 mg (77% d. Th., 100% Reinheit) der Titelverbindung erhalten.
LC-MS (Methode 1): Rt = 0.99 min; MS (ESIpos): m/z = 520 [M+H]+.
$^1$H-NMR (400 MHz, DMSO-d6): δ [ppm] = 10.56 (dd, 1H), 8.77 (d, 1H), 8.63 (d, 1H), 8.28 (d, 2H), 7.66-7.77 (m, 1H), 6.66-6.82 (m, 1H), 5.86-6.11 (m, 1H), 4.32-4.46 (m, 1H), 3.57-3.94 (m, 1H), 3.38-3.55 (m, 1H), 2.90-3.29 (m, 2H), 1.46-1.69 (m, 1H), 1.13-1.26 (m, 1H), 0.95-1.10 (m, 1H), 0.47-0.71 (m, 3H), 0.28-0.45 (m, 2H).

**Beispiel 55**

1-(3,5-Difluorpyridin-2-yl)-7-[(3R,4S)-3,4-dihydroxypyrrolidin-1-yl]-N-(1,1,1,3,3,3-hexafluorpropan-2-yl)-4-oxo-1,4-dihydro-1,8-naphthyridin-3-carbonsäureamid

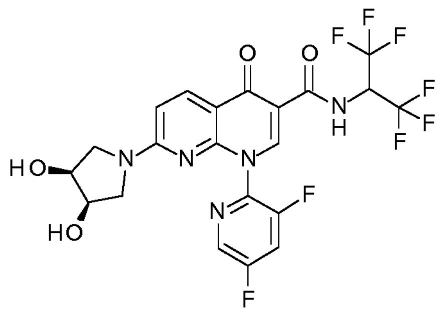

Gemäß allgemeiner Arbeitsvorschrift 3 wurden 40.0 mg (82.2 μmol) 7-Chlor-1-(3,5-difluorpyridin-2-yl)-N-(1,1,1,3,3,3-hexafluorpropan-2-yl)-4-oxo-1,4-dihydro-1,8-naphthyridin-3-carbonsäureamid (Beispiel 10A) mit 13.8 mg (98.6 μmol) (3R,4S)-Pyrrolidin-3,4-diol-Hydrochlorid in Gegenwart von 50.0 μl (288 μmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 35.6 mg (78% d. Th., 100% Reinheit) der Titelverbindung erhalten.
LC-MS (Methode 2): Rt = 1.76 min; MS (ESIpos): m/z = 554 [M+H]+.
$^1$H-NMR (400 MHz, DMSO-d6): δ [ppm] = 11.40 (d, 1H), 8.91 (s, 1H), 8.59-8.66 (m, 1H), 8.25-8.41 (m, 2H), 6.78 (d, 1H), 6.25-6.40 (m, 1H), 4.87-5.12 (m, 2H), 3.96-4.20 (m, 2H), 3.55-3.69 (m, 1H), 3.16-3.28 (m, 1H), 2.92-3.13 (m, 1H).

**Beispiel 56**

1-(3,5-Difluorpyridin-2-yl)-7-[(3R,4R)-3,4-dihydroxypyrrolidin-1-yl]-N-(1,1,1,3,3,3-hexafluorpropan-2-yl)-4-oxo-1,4-dihydro-1,8-naphthyridin-3-carbonsäureamid

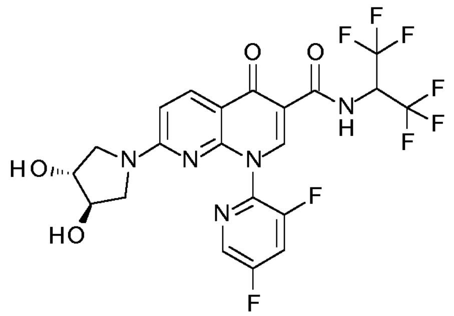

Gemäß allgemeiner Arbeitsvorschrift 3 wurden 40.0 mg (82.2 µmol) 7-Chlor-1-(3,5-difluorpyridin-2-yl)-N-(1,1,1,3,3,3-hexafluorpropan-2-yl)-4-oxo-1,4-dihydro-1,8-naphthyridin-3-carbonsäureamid (Beispiel 10A) mit 13.8 mg (98.6 µmol) (3R,4R)-Pyrrolidin-3,4-diol-Hydrochlorid in Gegenwart von 50.0 µl (288 µmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 39.3 mg (86% d. Th., 100% Reinheit) der Titelverbindung erhalten.
LC-MS (Methode 2): Rt = 1.71 min; MS (ESIpos): m/z = 554 [M+H]+.
$^1$H-NMR (400 MHz, DMSO-d6): δ [ppm] = 11.41 (d, 1H), 8.91 (d, 1H), 8.63 (d, 1H), 8.25-8.40 (m, 2H), 6.81 (d, 1H), 6.26-6.39 (m, 1H), 5.04-5.32 (m, 2H), 3.88-4.09 (m, 2H), 3.56-3.69 (m, 1H), 3.33-3.42 (m, 1H), 3.02-3.29 (m, 2H).

**Beispiel 57**

1-(3,5-Difluorpyridin-2-yl)-N-(1,1,1,3,3,3-hexafluorpropan-2-yl)-7-[1-hydroxy-3-azabicyclo[3.1.0]hex-3-yl]-4-oxo-1,4-dihydro-1,8-naphthyridin-3-carbonsäureamid (Racemat)

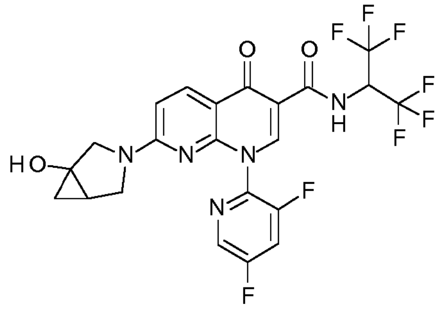

Gemäß allgemeiner Arbeitsvorschrift 3 wurden 70.0 mg (144 µmol) 7-Chlor-1-(3,5-difluorpyridin-2-yl)-N-(1,1,1,3,3,3-hexafluorpropan-2-yl)-4-oxo-1,4-dihydro-1,8-naphthyridin-3-carbonsäureamid (Beispiel 10A) mit 23.4 mg (173 µmol, 91% Reinheit) 3-Azabicyclo[3.1.0]hexan-1-ol-Hydrochlorid (Racemat) in Gegenwart von 88.0 µl (503 µmol) DIPEA in 0.7 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm 125*40 mm, Lösungsmittel: Acetonitril, Wasser, 0.1% Ameisensäure) gereinigt und 56.1 mg (71% d. Th., 100% Reinheit) der Titelverbindung erhalten.

LC-MS (Methode 2): Rt = 2.03 min; MS (ESIpos): m/z = 550 [M+H]+.

$^1$H-NMR (400 MHz, DMSO-d6): δ [ppm] = 11.36 (d, 1H), 8.92 (s, 1H), 8.63 (br. s, 1H), 8.25-8.46 (m, 2H), 6.71-6.87 (m, 1H), 6.26-6.40 (m, 1H), 5.91-6.13 (m, 1H), 3.39-3.98 (m, 3H), 3.06-3.27 (m, 1H), 1.50-1.71 (m, 1H), 0.97-1.11 (m, 1H), 0.36-0.51 (m, 1H).

43.0 mg der Titelverbindung (Diastereomerengemisch) wurde durch chirale HPLC in die Diastereomere getrennt (präparative HPLC: Säule Daicel Chiralpak IA 5 µm 250x30 mm; Eluent: 80% Iso-Hexan, 20% Ethanol; Temperatur: 23°C; Fluss: 20 ml/min; UV-Detektion: 220 nm.)

Man erhielt (in der Reihenfolge der Elution von der Säule) 17.2 mg Diastereomer 1 (99% de) Rt = 7.50 min und 18.2 mg (99% de) Diastereomer 2 Rt = 10.30 min.

[Analytische HPLC: Säule Daicel Chiralpak IA-3 3 µm 50x4.6 mm; Eluent: 80% Iso-Hexan, 20% Ethanol; Fluss: 1.0 ml/min; UV-Detektion: 220nm].

Diastereomer 1 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 11.0 mg (14% d. Th., 100% Reinheit) der Titelverbindung aus Beispiel 58 erhalten.

Diastereomer 2 wurde zusätzlich mittels präparativer HPLC (Säule: Chromatorex C18, 10 µm, 125*40 mm, Lösungsmittel: Acetonitril, Wasser, 0.1% Ameisensäure) gereinigt und 15.0 mg (19% d. Th., 100% Reinheit) der Titelverbindung aus Beispiel 59 erhalten.

## Beispiel 58

1-(3,5-Difluorpyridin-2-yl)-N-(1,1,1,3,3,3-hexafluorpropan-2-yl)-7-[1-hydroxy-3-azabicyclo[3.1.0]hex-3-yl]-4-oxo-1,4-dihydro-1,8-naphthyridin-3-carbonsäureamid (Diastereomer 1)

LC-MS (Methode 1): Rt = 1.07 min; MS (ESIpos): m/z = 550 [M+H]+.

$^1$H-NMR (400 MHz, DMSO-d6): δ [ppm] = 11.36 (d, 1H), 8.92 (s, 1H), 8.58-8.68 (m, 1H), 8.25-8.44 (m, 2H), 6.72-6.86 (m, 1H), 6.27-6.39 (m, 1H), 5.92-6.12 (m, 1H), 3.38-3.95 (m, 3H), 3.07-3.25 (m, 1H), 1.51-1.70 (m, 1H), 0.97-1.11 (m, 1H), 0.37-0.50 (m, 1H).

## Beispiel 59

1-(3,5-Difluorpyridin-2-yl)-N-(1,1,1,3,3,3-hexafluorpropan-2-yl)-7-[1-hydroxy-3-azabicyclo[3.1.0]hex-3-yl]-4-oxo-1,4-dihydro-1,8-naphthyridin-3-carbonsäureamid (Diastereomer 2)

LC-MS (Methode 2): Rt = 2.02 min; MS (ESIpos): m/z = 550 [M+H]+.

$^1$H-NMR (400 MHz, DMSO-d6): δ [ppm] = 11.36 (d, 1H), 8.92 (s, 1H), 8.63 (br. s, 1H), 8.25-8.45 (m, 2H), 6.71-6.87 (m, 1H), 6.26-6.40 (m, 1H), 5.91-6.14 (m, 1H), 3.40-3.97 (m, 3H), 3.07-3.26 (m, 1H), 1.50-1.71 (m, 1H), 0.96-1.11 (m, 1H), 0.36-0.51 (m, 1H).

## Beispiel 60

1-(3,5-Difluorpyridin-2-yl)-7-[(3S,4S)-3,4-dihydroxypyrrolidin-1-yl]-N-(1,1,1,3,3,3-hexafluorpropan-2-yl)-4-oxo-1,4-dihydro-1,8-naphthyridin-3-carbonsäureamid

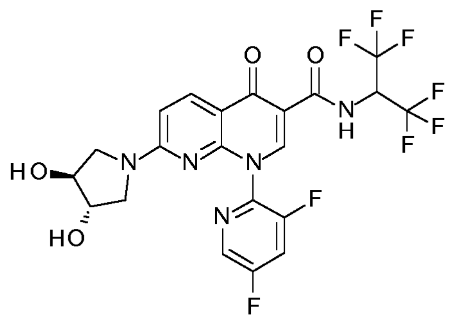

Gemäß allgemeiner Arbeitsvorschrift 3 wurden 40.0 mg (82.2 µmol) 7-Chlor-1-(3,5-difluorpyridin-2-yl)-N-(1,1,1,3,3,3-hexafluorpropan-2-yl)-4-oxo-1,4-dihydro-1,8-naphthyridin-3-carbonsäureamid (Beispiel 10A) mit 10.2 mg (98.6 µmol) (3S,4S)-Pyrrolidin-3,4-diol-Hydrochlorid in Gegenwart von 50.0 µl (288 µmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 38.3 mg (84% d. Th., 100% Reinheit) der Titelverbindung erhalten.
LC-MS (Methode 2): Rt = 1.71 min; MS (ESIpos): m/z = 554 [M+H]+.
$^1$H-NMR (400 MHz, DMSO-d6): δ [ppm] = 11.41 (d, 1H), 8.91 (d, 1H), 8.63 (d, 1H), 8.25-8.40 (m, 2H), 6.81 (d, 1H), 6.25-6.39 (m, 1H), 5.04-5.33 (m, 2H), 3.88-4.10 (m, 2H), 3.63 (ddd, 1H), 3.34-3.40 (m, 1H), 3.19-3.29 (m, 1H), 3.01-3.18 (m, 1H).

**Beispiel 61**

N-[(1S)-1-Cyclopropyl-2,2,2-trifluorethyl]-1-(3,5-difluorpyridin-2-yl)-7-[(3S,4S)-3,4-dihydroxypyrrolidin-1-yl]-4-oxo-14-dihydro-1,8-naphthyridin-3-carbonsäureamid

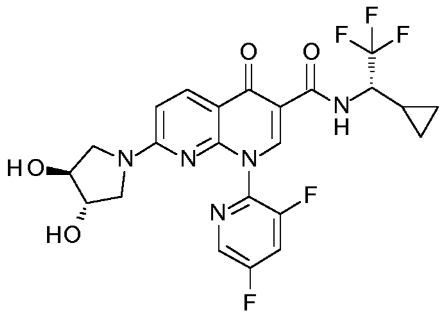

Gemäß allgemeiner Arbeitsvorschrift 3 wurden 20.0 mg (43.6 µmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 7.30 mg (52.0 µmol) (3S,4S)-Pyrrolidin-3,4-diol-Hydrochlorid in Gegenwart von 27.0 µl (153 µmol) DIPEA in 0.2 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 11.7 mg (51% d. Th., 100% Reinheit) der Titelverbindung erhalten.
LC-MS (Methode 2): Rt = 1.59 min; MS (ESIpos): m/z = 526 [M+H]+.
$^1$H-NMR (500 MHz, DMSO-d6): δ [ppm] = 10.56 (dd, 1H), 8.81 (d, 1H), 8.61 (d, 1H), 8.30-8.37 (m, 1H), 8.28 (d, 1H), 6.78 (d, 1H), 5.02-5.32 (m, 2H), 4.35-4.46 (m, 1H), 4.05 (br. s, 1H), 3.93 (br. s, 1H), 3.62 (ddd, 1H), 3.33-3.39 (m, 1H), 3.19-3.28 (m, 1H), 3.01-3.18 (m, 1H), 1.16-1.26 (m, 1H), 0.47-0.71 (m, 3H), 0.29-0.39 (m, 1H).

**Beispiel 62**

N-[(1S)-1-Cyclopropyl-2,2,2-trifluorethyl]-1-(3,5-difluorpyridin-2-yl)-7-[(3R,4S)-3,4-dihydroxypyrrolidin-1-yl]-4-oxo-1,4-dihydro-1,8-naphthyridin-3-carbonsäureamid

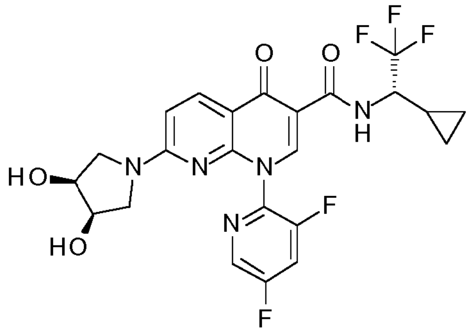

Gemäß allgemeiner Arbeitsvorschrift 3 wurden 20.0 mg (43.6 μmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 7.30 mg (52.0 μmol) (3R,4S)-Pyrrolidin-3,4-diol-Hydrochlorid in Gegenwart von 27.0 μl (153 μmol) DIPEA in 0.2 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 15.9 mg (69% d. Th., 100% Reinheit) der Titelverbindung erhalten.
LC-MS (Methode 2): Rt = 1.64 min; MS (ESIpos): m/z = 526 [M+H]+.
$^1$H-NMR (500 MHz, DMSO-d6): δ [ppm] = 10.55 (dd, 1H), 8.81 (s, 1H), 8.56-8.66 (m, 1H), 8.30-8.38 (m, 1H), 8.28 (d, 1H), 6.76 (d, 1H), 4.86-5.11 (m, 2H), 4.40 (sxt, 1H), 4.09-4.20 (m, 1H), 3.96-4.08 (m, 1H), 3.54-3.68 (m, 1H), 3.16-3.27 (m, 1H), 2.94-3.12 (m, 1H), 1.16-1.27 (m, 1H), 0.47-0.70 (m, 3H), 0.28-0.39 (m, 1H).

## Beispiel 63

N-[(1S)-1-Cyclopropyl-2,2,2-trifluorethyl]-1-(3,5-difluorpyridin-2-yl)-7-[(3R,4R)-3,4-dihydroxypyrrolidin-1-yl]-4-oxo-1,4-dihydro-1,8-naphthyridin-3-carbonsäureamid

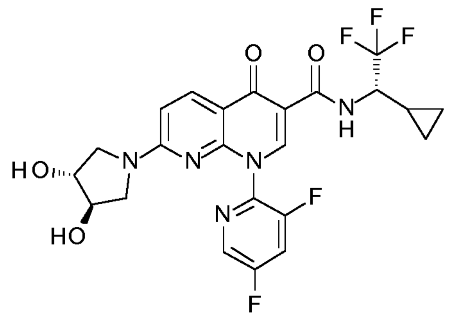

Gemäß allgemeiner Arbeitsvorschrift 3 wurden 20.0 mg (43.6 μmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 7.30 mg (52.0 μmol) (3R,4R)-Pyrrolidin-3,4-diol-Hydrochlorid in Gegenwart von 27.0 μl (153 μmol) DIPEA in 0.2 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 18.1 mg (79% d. Th., 100% Reinheit) der Titelverbindung erhalten.
LC-MS (Methode 1): Rt = 0.86 min; MS (ESIpos): m/z = 526 [M+H]+.
$^1$H-NMR (400 MHz, DMSO-d6): δ [ppm] = 10.56 (dd, 1H), 8.81 (d, 1H), 8.61 (d, 1H), 8.30-8.37 (m, 1H), 8.28 (d, 1H), 6.78 (d, 1H), 5.04-5.30 (m, 2H), 4.40 (sxt, 1H), 4.05 (br. s, 1H), 3.88-3.98 (m, 1H), 3.56-3.68 (m, 1H), 3.32-3.40 (m, 1H), 3.19-3.29 (m, 1H), 3.02-3.18 (m, 1H), 1.16-1.26 (m, 1H), 0.48-0.70 (m, 3H), 0.29-0.40 (m, 1H).

**Beispiel 64**

1-(3,5-Difluorpyridin-2-yl)-4-oxo-7-(2-oxoimidazolidin-1-yl)-N-[(2S)-1,1,1-trifluorbutan-2-yl]-1,4-dihydro-1,8-naphthyridin-3-carbonsäureamid

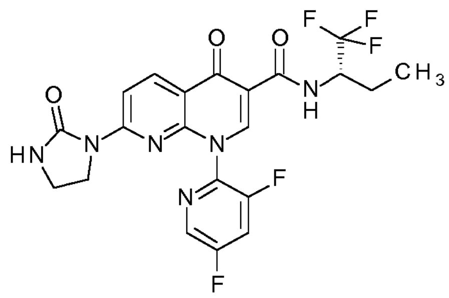

Gemäß allgemeiner Arbeitsvorschrift 2 wurden 60.0 mg (134 µmol) 7-Chlor-1-(3,5-difluorpyridin-2-yl)-4-oxo-N-[(2S)-1,1,1-trifluorbutan-2-yl]-1,4-dihydro-1,8-naphthyridin-3-carbonsäureamid (Beispiel 5A) mit 57.8 mg (671 µmol) Imidazolidin-2-on in Gegenwart von 65.6 mg (201 µmol) Cäsiumcarbonat, 1.51 mg (6.72 µmol) Palladiumacetat und 7.77 mg (13.4 µmol) Xantphos in 5 ml Dioxan bei 80°C zur Reaktion gebracht. Die Mischung wurde dann auf RT abgekühlt und mit 2 ml Chloroform und 0.5 ml gesättigter wässriger Natriumhydrogencarbonat-Lösung gewaschen. Die organische Phase wurde über Magnesiumsulfat getrocknet, filtriert und das Lösungsmittel unter reduziertem Druck entfernt. Das Rohprodukt wurde in etwas Wasser, Acetonitril und Ameisensäure gelöst und mittels präparativer HPLC (Säule: Chromatorex C18, 10 µm, 125*40 mm, Lösungsmittel: Acetonitril, Wasser, 0.1% Ameisensäure) gereinigt und 19.8 mg (30% d. Th., 100% Reinheit) der Titelverbindung erhalten.
LC-MS (Methode 1): Rt = 0.95 min; MS (ESIpos): m/z = 497 [M+H]+.
$^1$H-NMR (400 MHz, DMSO-d6): δ [ppm] = 10.21 (d, 1H), 8.99 (s, 1H), 8.64 (d, 1H), 8.56 (d, 1H), 8.44 (d, 1H), 8.33 (ddd, 1H), 7.67 (s, 1H), 4.69-4.84 (m, 1H), 3.52-3.72 (m, 2H), 3.33-3.43 (m, 2H), 1.82-1.96 (m, 1H), 1.59-1.74 (m, 1H), 0.92-1.03 (m, 3H).

**Beispiel 65**

N-[(1R)-1-Cyclopropyl-2,2,2-trifluorethyl]-1-(3,5-difluorpyridin-2-yl)-4-oxo-7-(2-oxoimidazolidin-1-yl)-1,4-dihydro-1,8-naphthyridin-3-carbonsäureamid

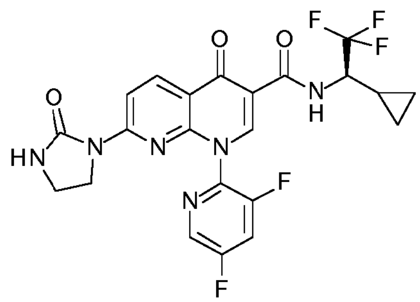

Gemäß allgemeiner Arbeitsvorschrift 2 wurden 60.0 mg (131 µmol) 7-Chlor-N-[(1R)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 8A) mit 56.3 mg (654 µmol) Imidazolidin-2-on in Gegenwart von 63.9 mg (196 µmol) Cäsiumcarbonat, 1.47 mg (6.54 µmol) Palladiumacetat und 7.57 mg (13.1 µmol) Xantphos in 5 ml Dioxan bei 100°C zur Reaktion gebracht. Die Mischung wurde dann auf RT abgekühlt und eingeengt. Das Rohprodukt wurde in etwas Wasser, Acetonitril und Ameisensäure gelöst und mittels präparativer HPLC (Säule: Chromatorex C18, 10 µm, 125*40 mm, Lösungsmittel: Acetonitril, Wasser, 0.1% Ameisensäure) gereinigt und 30.3 mg (46% d. Th., 100% Reinheit) der Titelverbindung erhalten.
LC-MS (Methode 2): Rt = 1.82 min; MS (ESIpos): m/z = 509 [M+H]+.

$^1$H-NMR (400 MHz, DMSO-d6): δ [ppm] = 10.34 (d, 1H), 8.98 (s, 1H), 8.63 (d, 1H), 8.56 (d, 1H), 8.44 (d, 1H), 8.28-8.37 (m, 1H), 7.67 (s, 1H), 4.35-4.49 (m, 1H), 3.53-3.71 (m, 2H), 3.33-3.42 (m, 2H), 1.17-1.28 (m, 1H), 0.49-0.72 (m, 3H), 0.29-0.41 (m, 1H).

**Beispiel 66**

N-(2,6-Dichlorphenyl)-1-(3,5-difluorpyridin-2-yl)-7-[(3R,4S)-3,4-dihydroxypyrrolidin-1-yl]-4-oxo-1,4-dihydro-1,8-naphthyridin-3-carbonsäureamid

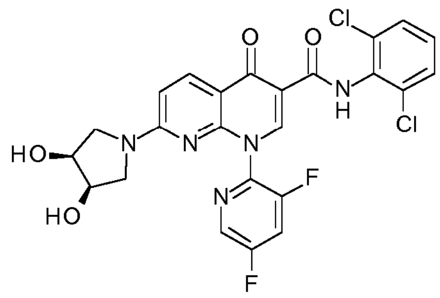

Gemäß allgemeiner Arbeitsvorschrift 2 wurden 60.0 mg (125 µmol) 7-Chlor-N-(2,6-dichlorphenyl)-1-(3,5-difluorpyridin-2-yl)-4-oxo-1,4-dihydro-1,8-naphthyridin-3-carbonsäureamid (Beispiel 11B) mit 20.9 mg (149 µmol) (3R,4S)-Pyrrolidin-3,4-diol-Hydrochlorid in Gegenwart von 25.8 mg (187 µmol) Kaliumcarbonat, 5.59 mg (24.9 µmol) Palladiumacetat und 14.4 mg (24.9 µmol) Xantphos in 0.9 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in 3 ml Acetonitril und 1 ml DMSO gelöst, mittels präparativer HPLC (Säule: Chromatorex C18, 10 µm 125*40 mm, Lösungsmittel: Acetonitril, Wasser, 0.1% Ameisensäure) gereinigt und 7.10 mg (10% d. Th., 100% Reinheit) der Titelverbindung erhalten.
LC-MS (Methode 1): Rt = 0.88 min; MS (ESIpos): m/z = 548 [M+H]+.
$^1$H-NMR (400 MHz, DMSO-d6): δ [ppm] = 11.92 (s, 1H), 8.89 (s, 1H), 8.62 (br. s, 1H), 8.33 (d, 2H), 7.59 (d, 2H), 7.34-7.41 (m, 1H), 6.78 (d, 1H), 4.89-5.13 (m, 2H), 3.98-4.20 (m, 2H), 3.48-3.69 (m, 2H), 2.96-3.21 (m, 2H).

**Beispiel 67**

N-(2,6-Dichlorphenyl)-1-(3,5-difluorpyridin-2-yl)-7-[(3R,4R)-3,4-dihydroxypyrrolidin-1-yl]-4-oxo-1,4-dihydro-1,8-naphthyridin-3-carbonsäureamid

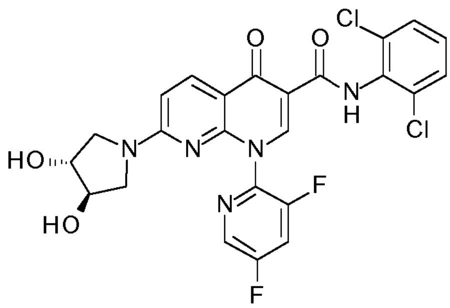

Gemäß allgemeiner Arbeitsvorschrift 2 wurden 60.0 mg (125 µmol) 7-Chlor-N-(2,6-dichlorphenyl)-1-(3,5-difluorpyridin-2-yl)-4-oxo-1,4-dihydro-1,8-naphthyridin-3-carbonsäureamid (Beispiel 11B) mit 20.9 mg (149 µmol) (3R,4R)-Pyrrolidin-3,4-diol-Hydrochlorid in Gegenwart von 25.8 mg (187 µmol) Kaliumcarbonat, 5.59 mg (24.9 µmol)

Palladiumacetat und 14.4 mg (24.9 μmol) Xantphos in 0.9 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in 3 ml Acetonitril und 1 ml DMSO gelöst, mittels präparativer HPLC (Säule: Chromatorex C18, 10 μm, 125*40 mm, Lösungsmittel: Acetonitril, Wasser, 0.1% Ameisensäure) gereinigt und 6.70 mg (10% d. Th., 100% Reinheit) der Titelverbindung erhalten.
LC-MS (Methode 2): Rt = 1.58 min; MS (ESIpos): m/z = 548 [M+H]+.
$^1$H-NMR (400 MHz, DMSO-d6): δ [ppm] = 11.94 (s, 1H), 8.89 (d, 1H), 8.62 (d, 1H), 8.34 (d, 2H), 7.59 (d, 2H), 7.34-7.41 (m, 1H), 6.80 (d, 1H), 5.05-5.32 (m, 2H), 3.90-4.10 (m, 2H), 3.58-3.70 (m, 1H), 3.36-3.41 (m, 1H), 3.04-3.27 (m, 2H).

**Beispiel 68**

N-(2,6-Dichlorphenyl)-1-(3,5-difluorpyridin-2-yl)-7-[(3S,4S)-3,4-dihydroxypyrrolidin-1-yl]-4-oxo-1,4-dihydro-1,8-naphthyridin-3-carbonsäureamid

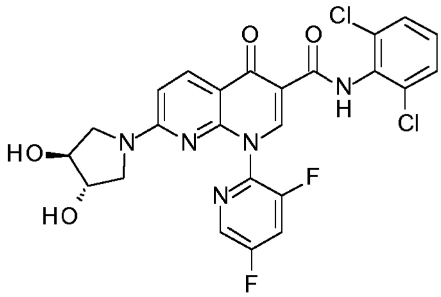

Gemäß allgemeiner Arbeitsvorschrift 3 wurden 35.9 mg (74.5 μmol) 7-Chlor-N-(2,6-dichlorphenyl)-1-(3,5-difluorpyridin-2-yl)-4-oxo-1,4-dihydro-1,8-naphthyridin-3-carbonsäureamid (Beispiel 11B) mit 12.5 mg (89.4 μmol) (3S,4S)-Pyrrolidin-3,4-diol-Hydrochlorid in Gegenwart von 45.0 μl (261 μmol) DIPEA in 0.37 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 19.6 mg (48% d. Th., 100% Reinheit) der Titelverbindung erhalten.
LC-MS (Methode 1): Rt = 0.89 min; MS (ESIpos): m/z = 548 [M+H]+.
$^1$H-NMR (400 MHz, DMSO-d6): δ [ppm] = 11.94 (s, 1H), 8.89 (d, 1H), 8.62 (d, 1H), 8.34 (d, 2H), 7.59 (d, 2H), 7.38 (dd, 1H), 6.80 (d, 1H), 5.04-5.33 (m, 2H), 3.90-4.13 (m, 2H), 3.57-3.71 (m, 1H), 3.36-3.43 (m, 1H), 3.04-3.28 (m, 2H).

**Beispiel 69**

N-(2,6-Dichlorphenyl)-1-(3,5-difluorpyridin-2-yl)-7-[3-hydroxy-3-methylpyrrolidin-1-yl]-4-oxo-1,4-dihydro-1,8-naphthyridin-3-carbonsäureamid (Racemat)

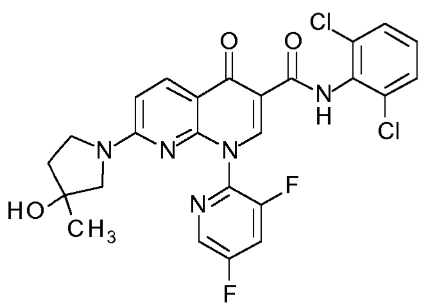

Gemäß allgemeiner Arbeitsvorschrift 2 wurden 100 mg (208 μmol) 7-Chlor-N-(2,6-dichlorphenyl)-1-(3,5-difluorpyridin-2-yl)-4-oxo-1,4-dihydro-1,8-naphthyridin-3-carbonsäureamid (Beispiel 11B) mit 34.3 mg (249 μmol) 3-Methylpyrrolidin-3-ol-Hydrochlorid (Racemat) in Gegenwart von 43.0 mg (311 μmol) Kaliumcarbonat, 9.32 mg (41.5 μmol) Palladiumacetat und 24.0 mg (41.5 μmol) Xantphos in 1.6 ml Dioxan bei 80°C zur Reaktion gebracht. Die Mischung wurde dann auf RT abgekühlt, dann mit 1M wässriger Salzsäure angesäuert, mit 100 mg N-Acetylcystein versetzt und 15 min nachgerührt. Die Mischung wurde dann mit 15 ml gesättigter wässriger Natriumhydrogencarbonat-Lösung und 20 ml Essigsäureethylester verdünnt und die Phasen getrennt. Die wässrige Phase wurde dreimal mit Essigsäureethylester extrahiert. Die vereinigten organischen Phasen wurden über Magnesiumsulfat getrocknet, filtriert und das Lösungsmittel unter reduziertem Druck entfernt. Das Rohprodukt wurde in 3 ml Acetonitril und 1 ml DMSO gelöst und zweimal mittels präparativer HPLC (Säule: Chromatorex C18, 10 μm, 125*40 mm, Lösungsmittel: Acetonitril, Wasser, 0.1% Ameisensäure) gereinigt. Anschließend wurden die Produktfraktionen vereinigt, eingeengt und das Rohprodukt nochmals mittels Normalphasenchromatographie (Essigsäureethylester-Cyclohexan Gradient) gereinigt und 16.9 mg (15% d. Th., 100% Reinheit) der Titelverbindung erhalten.
LC-MS (Methode 1): Rt = 1.04 min; MS (ESIpos): m/z = 546 [M+H]+.
$^1$H-NMR (400 MHz, DMSO-d6): δ [ppm] = 11.94 (s, 1H), 8.88 (s, 1H), 8.61 (br. d, 1H), 8.33 (t, 2H), 7.59 (d, 2H), 7.33-7.43 (m, 1H), 6.70-6.85 (m, 1H), 4.74-4.96 (m, 1H), 3.51-3.64 (m, 1H), 3.36-3.45 (m, 1H), 2.93-3.25 (m, 1H), 1.68-2.00 (m, 2H), 1.12-1.38 (m, 4H).

## Beispiel 70

7-[(2,2-Difluorethyl)amino]-1-(3,5-difluorpyridin-2-yl)-4-oxo-N-[(2R)-1,1,1-trifluorbutan-2-yl]-1,4-dihydro-1,8-naphthyridin-3-carbonsäureamid

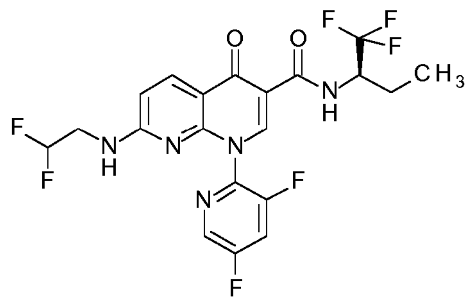

Gemäß allgemeiner Arbeitsvorschrift 3 wurden 25.0 mg (56.0 μmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 5.44 mg (67.0 μmol) 2,2-Difluorethanamin in Gegenwart von 34.0 μl (196 μmol) DIPEA in 0.4 ml DMF über Nacht zur Reaktion gebracht. Anschließend wurden 2,2-Difluorethanamin und DIPEA nachgegeben und 3h bei 50°C nachgerührt. Das Rohprodukt wurde mit Acetonitril, Wasser und Ameisensäure gelöst, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 21.9 mg (80% d. Th., 100% Reinheit) der Titelverbindung erhalten.
LC-MS (Methode 2): Rt = 1.96 min; MS (ESIpos): m/z = 492 [M+H]+.
$^1$H-NMR (400 MHz, DMSO-d6): δ [ppm] = 10.38 (d, 1H), 8.85 (s, 1H), 8.63 (d, 1H), 8.30-8.44 (m, 2H), 8.26 (d, 1H), 6.82 (d, 1H), 5.62-6.00 (m, 1H), 4.67-4.83 (m, 1H), 3.36-3.55 (m, 2H), 1.81-1.95 (m, 1H), 1.56-1.72 (m, 1H), 0.91-1.03 (m, 3H).

## Beispiel 71

1-(3,5-Difluorpyridin-2-yl)-7-(morpholin-4-yl)-4-oxo-N-[(2R)-1,1,1-trifluorbutan-2-yl]-1,4-dihydro-1,8-naphthyridin-3 -carbonsäureamid

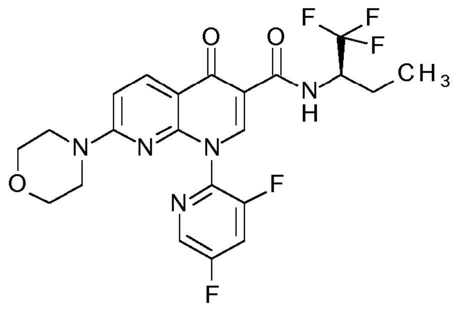

Gemäß allgemeiner Arbeitsvorschrift 3 wurden 25.0 mg (56.0 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 6.0 µL (67.0 µmol) Morpholin in Gegenwart von 34.0 µl (196 µmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 22.9 mg (80% d. Th., 97% Reinheit) der Titelverbindung erhalten.
LC-MS (Methode 2): Rt = 1.98 min; MS (ESIpos): m/z = 498 [M+H]+.
$^1$H-NMR (400 MHz, DMSO-d6): δ [ppm] = 10.35 (d, 1H), 8.84 (s, 1H), 8.62 (d, 1H), 8.25-8.38 (m, 2H), 7.13 (d, 1H), 4.66-4.83 (m, 1H), 3.54-3.66 (m, 4H), 3.40-3.53 (m, 4H), 1.81-1.96 (m, 1H), 1.56-1.72 (m, 1H), 0.89-1.03 (m, 3H).

## Beispiel 72

7-(3,3-Difluorpiperidin-1-yl)-1-(3,5-difluorpyridin-2-yl)-4-oxo-N-[(2R)-1,1,1-trifluorbutan-2-yl]-1,4-dihydro-1,8-naphthyridin-3-carbonsäureamid

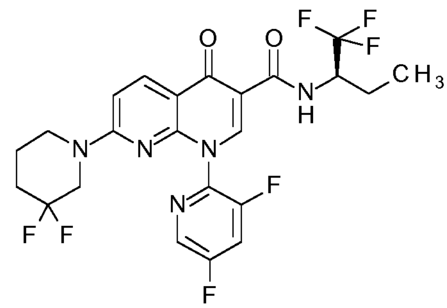

Gemäß allgemeiner Arbeitsvorschrift 3 wurden 25.0 mg (56.0 µmol) 7-Chlor-1-(3,5-difluorpyridin-2-yl)-4-oxo-N-[(2R)-1,1,1-trifluorbutan-2-yl]-1,4-dihydro-1,8-naphthyridin-3-carbonsäureamid (Beispiel 4C) mit 10.6 mg (67.1 µmol) 3,3-Difluorpiperidin-Hydrochlorid in Gegenwart von 34.0 µl (196 µmol) DIPEA in 0.4 ml DMF zur Reaktion gebracht. Die Mischung wurde anschließend mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 27.0 mg (91% d. Th., 100% Reinheit) der Titelverbindung erhalten.
LC-MS (Methode 2): Rt = 2.16 min; MS (ESIpos): m/z = 532 [M+H]+.
$^1$H-NMR (400 MHz, DMSO-d6): δ [ppm] = 10.33 (d, 1H), 8.86 (s, 1H), 8.64 (d, 1H), 8.28-8.44 (m, 2H), 7.26 (d, 1H), 4.67-4.83 (m, 1H), 3.78-3.99 (m, 2H), 3.50-3.63 (m, 2H), 1.99-2.14 (m, 2H), 1.82-1.95 (m, 1H), 1.57-1.72 (m, 3H), 0.91-1.04 (m, 3H).

## Beispiel 73

1-(3,5-Difluorpyridin-2-yl)-7-[(4S)-4-hydroxy-2-oxopyrrolidin-1-yl]-4-oxo-N-[(2R)-1,1,1-trifluor-3,3-dimethylbutan-2-yl]-1,4-dihydro-1,8-naphthyridin-3-carbonsäureamid

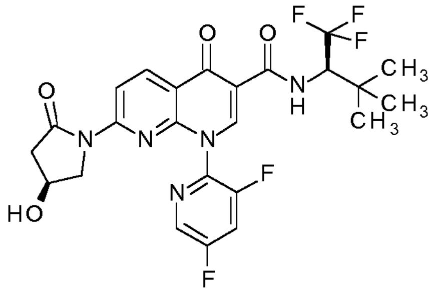

Gemäß allgemeiner Arbeitsvorschrift 1 wurden 70.0 mg (174 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 32.4 mg (209 µmol) (2R)-1,1,1-Trifluor-3,3-dimethylbutan-2-amin in Gegenwart von 79.4mg (209 µmol) HATU und 91.0 µl (522 µmol) DIPEA in 0.7 ml DMF zur Reaktion gebracht. Das Reaktionsgemisch wurde dann mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 76.7 mg (82% d. Th., 100% Reinheit) der Titelverbindung erhalten.
LC-MS (Methode 2): Rt = 1.96 min; MS (ESIpos): m/z = 540 [M+H]+.
$^1$H-NMR (500 MHz, DMSO-d6): δ [ppm] = 10.48 (br. d, 1H), 9.07 (s, 1H), 8.74 (d, 1H), 8.66 (br. s, 1H), 8.54 (br. dd, 1H), 8.39 (ddd, 1H), 5.23-5.40 (m, 1H), 4.66 (quint, 1H), 4.30 (br. s, 1H), 3.65-3.78 (m, 1H), 3.45-3.57 (m, 1H), 2.89- 3.02 (m, 1H), 2.34-2.43 (m, 1H), 1.10 (br. s, 9H).

**Beispiel 74**

1-(3,5-Difluorpyridin-2-yl)-7-[(4S)-4-hydroxy-2-oxopyrrolidin-1-yl]-4-oxo-N-[(2R)-1,1,1-trifluor-4-methylpentan-2-yl]-1,4-dihydro-1,8-naphthyridin-3-carbonsäureamid

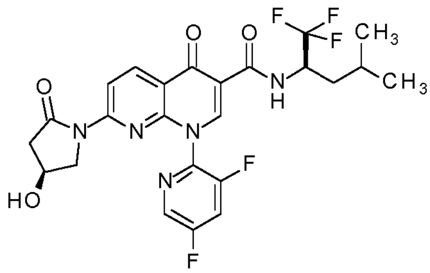

Gemäß allgemeiner Arbeitsvorschrift 1 wurden 70.0 mg (174 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 32.4 mg (209 µmol) (2R)-1,1,1-Trifluor-4-methylpentan-2-amin in Gegenwart von 79.4 mg (209 µmol) HATU und 91.0 µl (522 µmol) DIPEA in 0.7 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 78.0 mg (83% d. Th., 100% Reinheit) der Titelverbindung erhalten.
LC-MS (Methode 2): Rt = 2.01 min; MS (ESIpos): m/z = 540 [M+H]+.
$^1$H-NMR (500 MHz, DMSO-d6): δ [ppm] = 10.10 (br. d, 1H), 9.06 (s, 1H), 8.65-8.72 (m, 2H), 8.51-8.57 (m, 1H), 8.39 (ddd, 1H), 5.24-5.40 (m, 1H), 4.81-4.90 (m, 1H), 4.29 (br. s, 1H), 3.64-3.78 (m, 1H), 3.46-3.56 (m, 1H), 2.89-3.02 (m, 1H), 2.34-2.43 (m, 1H), 1.65-1.74 (m, 2H), 1.54-1.64 (m, 1H), 0.95 (br. dd, 3H), 0.90 (br. t, 3H).

**Beispiel 75**

1-(3,5-Difluorpyridin-2-yl)-7-[(4S)-4-hydroxy-2-oxopyrrolidin-1-yl]-4-oxo-N-[(2S)-1,1,1-trifluor-4-methylpentan-2-yl]-1,4-dihydro-1,8-naphthyridin-3-carbonsäureamid

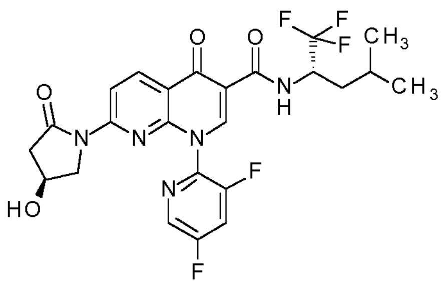

Gemäß allgemeiner Arbeitsvorschrift 1 wurden 70.0 mg (174 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 32.4 mg (209 µmol) (2S)-1,1,1-Trifluor-4-methylpentan-2-amin in Gegenwart von 79.4 mg (209 µmol) HATU und 91.0 µl (522 µmol) DIPEA in 0.7 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 77.1 mg (82% d. Th., 100% Reinheit) der Titelverbindung erhalten.
LC-MS (Methode 2): Rt = 2.02 min; MS (ESIpos): m/z = 540 [M+H]+.
$^1$H-NMR (500 MHz, DMSO-d6): δ [ppm] = 10.10 (br. dd, 1H), 9.06 (s, 1H), 8.71 (d, 1H), 8.66 (br. dd, 1H), 8.54 (br. dd, 1H), 8.36-8.41 (m, 1H), 5.23-5.40 (m, 1H), 4.81-4.90 (m, 1H), 4.29 (br. s, 1H), 3.65-3.78 (m, 1H), 3.45-3.56 (m, 1H), 2.89-3.02 (m, 1H), 2.34-2.43 (m, 1H), 1.65-1.74 (m, 2H), 1.54-1.62 (m, 1H), 0.95 (br. dd, 3H), 0.89 (br. t, 3H).

## Beispiel 76

1-(3,5-Difluorpyridin-2-yl)-7-[(4S)-4-hydroxy-2-oxopyrrolidin-1-yl]-4-oxo-N-[3,3,4,4,4-pentafluorbutan-2-yl]-1,4-dihydro-1,8-naphthyridin-3-carbonsäureamid (Diastereomerengemisch)

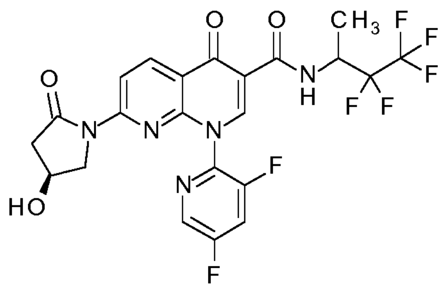

Gemäß allgemeiner Arbeitsvorschrift 1 wurden 125.0 mg (311 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 74.4 mg (373 µmol) (2S)-3,3,4,4,4-Pentafluorbutan-2-amin-Hydrochlorid (Beispiel 28B, Racemat) in Gegenwart von 142 mg (373 µmol) HATU und 0.22 ml (1.24 mmol) DIPEA in 1.25 ml DMF zur Reaktion gebracht. Das Reaktionsgemisch wurde mit Acetonitril, Wasser und Ameisensäure verdünnt, über einen Milliporefilter filtriert und die Rohlösungmittels präparativer HPLC (Säule: Chromatorex C18, 10 µm, 125*40 mm, Lösungsmittel: Acetonitril, Wasser, 0.1% Ameisensäure) gereinigt und 119 mg (70% d. Th., 100% Reinheit) der Titelverbindung erhalten.
LC-MS (Methode 1): Rt = 0.99 min; MS (ESIpos): m/z = 548 [M+H]+.
$^1$H-NMR (400 MHz, DMSO-d6): δ [ppm] = 10.24 (d, 1H), 9.06 (s, 1H), 8.71 (d, 1H), 8.64-8.68 (m, 1H), 8.50-8.58 (m, 1H), 8.35-8.42 (m, 1H), 5.23-5.40 (m, 1H), 4.98-5.12 (m, 1H), 4.29 (br. s, 1H), 3.64-3.78 (m, 1H), 3.44-3.56 (m, 1H), 2.88-3.02 (m, 1H), 2.33-2.43 (m, 1H), 1.42 (br. d, 3H).
107 mg der Titelverbindung (Diastereomerengemisch) wurde durch chirale HPLC in die Diastereomere getrennt (präparative HPLC: Säule Daicel Chiralpak OX-H 5 µm 250x20 mm; Eluent: 50% Iso-Hexan; 50% Isopropanol; Temperatur: 50°C; Fluss: 15 ml/min; UV-Detektion: 220 nm.)
Man erhielt (in der Reihenfolge der Elution von der Säule) 37 mg (22% d. Th., 100% Reinheit) aus Beispiel 77 (Diastereomer 1, 99% de) $R_t$ = 5.29 min und 42 mg (25% d. Th., 100% Reinheit) aus Beispiel 78 (Diastereomer 2, 99% de) $R_t$ = 6.21 min.
[Analytische HPLC: Säule Daicel Chiralpak OX-H 5 µm 250x4.6 mm; Eluent: 50% Iso-Hexan, 50% Isopropanol;

Temperatur: 50°C; Fluss: 1.0 ml/min; UV-Detektion: 220nm].

**Beispiel 77**

1-(3,5-Difluorpyridin-2-yl)-7-[(4S)-4-hydroxy-2-oxopyrrolidin-l-yl]-4-oxo-N-[3,3,4,4,4-pentafluorbutan-2-yl]-1,4-dihydro-1,8-naphthyridin-3-carbonsäureamid (Diastereomer 1)

LC-MS (Methode 2): $R_t$ = 1.78 min; MS (ESIpos): m/z = 548 [M+H]+.

$^1$H NMR (400 MHz, DMSO-d6): δ [ppm] = 10.24 (br d, 1H), 9.04-9.07 (m, 1H), 8.71 (d, 1H), 8.64-8.68 (m, 1H), 8.54 (br t, 1H), 8.35-8.42 (m, 1H), 5.22-5.39 (m, 1H), 4.98-5.12 (m, 1H), 4.26-4.32 (m, 1H), 3.65-3.78 (m, 1H), 3.44-3.56 (m, 1H), 2.89-3.02 (m, 1H), 2.33-2.42 (m, 1H), 1.42 (br d, 3H).

**Beispiel 78**

1-(3,5-Difluorpyridin-2-yl)-7-[(4S)-4-hydroxy-2-oxopyrrolidin-1-yl]-4-oxo-N-[3,3,4,4,4-pentafluorbutan-2-yl]-1,4-dihydro-1,8-naphthyridin-3-carbonsäureamid (Diastereomer 2)

LC-MS (Methode 2): $R_t$ = 1.77 min; MS (ESIpos): m/z = 548 [M+H]+.

$^1$H NMR (400 MHz, DMSO-d6): δ [ppm] = 10.24 (d, 1H), 9.06 (s, 1H), 8.71 (d, 1H), 8.64-8.68 (m, 1H), 8.54 (br t, 1H), 8.36-8.42 (m, 1H), 5.24-5.41 (m, 1H), 4.99-5.13 (m, 1H), 4.26-4.32 (m, 1H), 3.64-3.79 (m, 1H), 3.44-3.57 (m, 1H), 2.87-3.02 (m, 1H), 2.34-2.43 (m, 1H), 1.41 (br d, 3H).

**Beispiel 79**

1-(3,5-Difluorpyridin-2-yl)-7-[(4S)-4-hydroxy-2-oxopyrrolidin-1-yl]-4-oxo-N-[1-(trifluormethyl)cyclopentyl]-1,4-dihydro-1,8-naphthyridin-3-carbonsäureamid

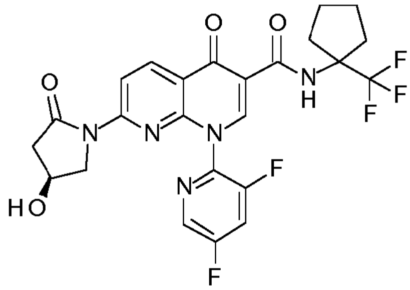

Gemäß allgemeiner Arbeitsvorschrift 1 wurden 70.0 mg (174 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 39.6 mg (209μmol) 1-(Trifluormethyl)cyclopentanamin-Hydrochlorid in Gegenwart von 79.4 mg (209 μmol) HATU und 121 μl (696 μmol) DIPEA in 0.7 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 77.8 mg (83% d. Th., 100% Reinheit) der Titelverbindung erhalten.
LC-MS (Methode 2): Rt = 1.92 min; MS (ESIpos): m/z = 538 [M+H]+.
$^1$H-NMR (500 MHz, DMSO-d6): δ [ppm] = 10.20 (s, 1H), 9.01 (s, 1H), 8.70 (d, 1H), 8.64-8.68 (m, 1H), 8.50-8.56 (m, 1H), 8.35-8.41 (m, 1H), 5.23-5.40 (m, 1H), 4.29 (br. s, 1H), 3.64-3.78 (m, 1H), 3.45-3.56 (m, 1H), 2.88-3.02 (m, 1H), 2.33-2.45 (m, 3H), 2.02-2.11 (m, 2H), 1.70-1.85 (m, 4H).

**Beispiel 80**

1-(3,5-Difluorpyridin-2-yl)-7-[(4S)-4-hydroxy-2-oxopyrrolidin-1-yl]-4-oxo-N-(4,4,4-trifluor-2-methylbutan-2-yl)-1,4-dihydro-1,8-naphthyridin-3-carbonsäureamid

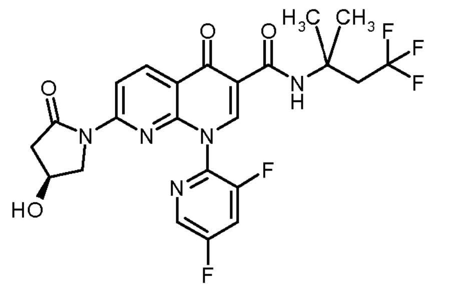

Gemäß allgemeiner Arbeitsvorschrift 1 wurden 50.0 mg (124 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 26.5 mg (149 μmol) 4,4,4-Trifluor-2-methylbutan-2-amin-Hydrochlorid in Gegenwart von 56.7 mg (149 μmol) HATU und 65.0 μl (373 μmol) DIPEA in 0.48 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 33.8 mg (52% d. Th., 100% Reinheit) der Titelverbindung erhalten.
LC-MS (Methode 2): Rt = 1.76 min; MS (ESIpos): m/z = 526 [M+H]+.
$^{1}$H-NMR (400 MHz, DMSO-d6): δ [ppm] = 9.90 (s, 1H), 8.98 (s, 1H), 8.69 (d, 1H), 8.64-8.67 (m, 1H), 8.49-8.56 (m, 1H), 8.35-8.41 (m, 1H), 5.37 (br. d, 0.5H), 5.25 (br. d, 0.5H), 4.26-4.32 (m, 1H), 3.65-3.78 (m, 1H), 3.44-3.57 (m, 1H), 2.88-3.02 (m, 3H), 2.34-2.43 (m, 1H), 1.50 (s, 6H).

## Beispiel 81

1-(3,5-Difluorpyridin-2-yl)-7-[(3R,4R)-3,4-dihydroxypyrrolidin-1-yl]-4-oxo-N-[(2R)-1,1,1-trifluor-4-methylpentan-2-yl]-1,4-dihydro-1,8-naphthyridin-3-carbonsäureamid

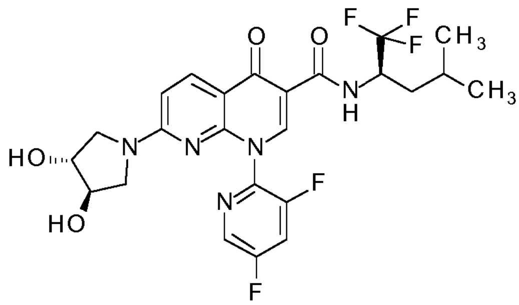

Gemäß allgemeiner Arbeitsvorschrift 1 wurden 30.0 mg (74.2 μmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 13.8 mg (89.0 μmol) (2R)-1,1,1-Trifluor-4-methylpentan-2-amin in Gegenwart von 33.9 mg (89.0 μmol) HATU und 39.0 μl (223 μmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösungmittels präparativer HPLC (Säule: Chromatorex C18, 10 μm, 125*40 mm, Lösungsmittel: Acetonitril, Wasser, 0.1% Ameisensäure) gereinigt und 27.6 mg (69% d. Th., 100% Reinheit) der Titelverbindung erhalten.
LC-MS (Methode 2): Rt = 1.80 min; MS (ESIpos): m/z = 542 [M+H]+.
$^{1}$H-NMR (400 MHz, DMSO-d6): δ [ppm] = 10.41 (dd, 1H), 8.82 (br. s, 1H), 8.61 (d, 1H), 8.29-8.36 (m, 1H), 8.27 (d, 1H), 6.78 (d, 1H), 5.27 (d, 0.5H), 5.19 (t, 1H), 5.07 (d, 0.5H), 4.77-4.89 (m, 1H), 4.05 (br. s, 1H), 3.93 (br. s, 1H), 3.57-3.67 (m, 1H), 3.33-3.39 (m, 1H), 3.19-3.29 (m, 1H), 3.03-3.17 (m, 1H), 1.62-1.72 (m, 2H), 1.52-1.62 (m, 1H), 0.86-0.97 (m, 6H).

**Beispiel 82**

1-(3,5-Difluorpyridin-2-yl)-7-[(3R,4R)-3,4-dihydroxypyrrolidin-1-yl]-4-oxo-N-[(2S)-1,1,1-trifluor-4-methylpentan-2-yl]-1,4-dihydro-1,8-naphthyridin-3-carbonsäureamid

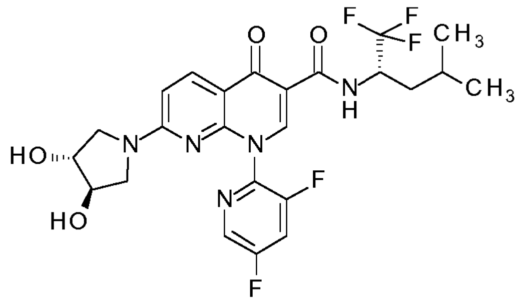

Gemäß allgemeiner Arbeitsvorschrift 1 wurden 30.0 mg (74.2 µmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 13.8 mg (89.0 µmol) (2S)-1,1,1-Trifluor-4-methylpentan-2-amin in Gegenwart von 33.9 mg (89.0 µmol) HATU und 39.0 µl (223 µmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 26.4 mg (66% d. Th., 100% Reinheit) der Titelverbindung erhalten.
LC-MS (Methode 2): Rt = 1.80 min; MS (ESIpos): m/z = 542 [M+H]+.
$^1$H-NMR (400 MHz, DMSO-d6): δ [ppm] = 10.42 (dd, 1H), 8.82 (d, 1H), 8.61 (br. t, 1H), 8.29-8.36 (m, 1H), 8.27 (d, 1H), 6.78 (d, 1H), 4.99-5.36 (m, 2H), 4.78-4.89 (m, 1H), 4.02-4.09 (m, 1H), 3.89-3.97 (m, 1H), 3.48-3.70 (m, 1H), 3.19-3.39 (m, 1H, unter dem Wasser Signal), 3.02-3.17 (m, 1H), 1.62-1.72 (m, 2H), 1.52-1.61 (m, 1H), 1.03 (br. d, 1H), 0.86-0.98 (m, 6H).

**Beispiel 83**

N-[1-(2-Chlorphenyl)-2,2,2-trifluorethyl]-1-(3,5-difluorpyridin-2-yl)-7-[(3R,4R)-3,4-dihydroxypyrrolidin-1-yl]-4-oxo-1,4-dihydro-1,8-naphthyridin-3-carbonsäureamid (Diastereomerengemisch)

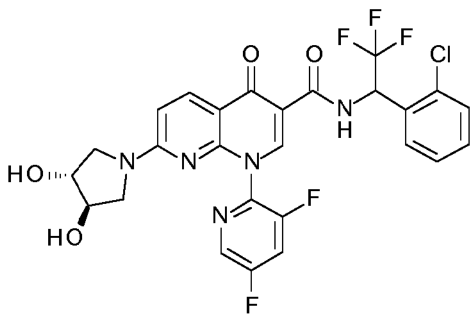

Gemäß allgemeiner Arbeitsvorschrift 1 wurden 30.0 mg (74.2 µmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 18.7 mg (89.0 µmol) (1S)-1-(2-Chlorphenyl)-2,2,2-trifluorethanamin in Gegenwart von 33.9 mg (89.0 µmol) HATU und 39.0 µl (223 µmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösungmittels präparativer HPLC (Säule: Chromatorex C18, 10 µm, 125*40 mm, Lösungsmittel: Acetonitril, Wasser, 0.1% Ameisensäure) gereinigt und 32.5 mg (74% d. Th., 100% Reinheit) der Titelverbindung erhalten.
LC-MS (Methode 2): Rt = 1.88 min; MS (ESIpos): m/z = 596 [M+H]+.

$^1$H-NMR (400 MHz, DMSO-d6): δ [ppm] = 11.52-11.58 (m, 1H), 8.79-8.86 (m, 1H), 8.57-8.65 (m, 1H), 8.26-8.38 (m, 2H), 7.45-7.67 (m, 4H), 6.80 (d, 1H), 6.40-6.50 (m, 1H), 5.24-5.30 (m, 0.5H), 5.15-5.23 (m, 1H), 5.03-5.10 (m, 0.5H), 4.05 (br. s, 1H), 3.93 (br. s, 1H), 3.56-3.68 (m, 1H), 3.34-3.40 (m, 1H), 3.19-3.28 (m, 1H), 3.01-3.18 (m, 1H).

28.7 mg der Titelverbindung (Diastereomerengemisch) wurde durch chirale HPLC in die Diastereomere getrennt (präparative HPLC: Säule Daicel Chiralcel OX-H 5 μm 250x20 mm; Eluent: 30% *n*-Heptan, 70% Ethanol; Temperatur: 25°C; Fluss: 15 ml/min; UV-Detektion: 210 nm.)

Man erhielt in der Reihenfolge der Elution von der Säule 13.8 mg (31% d. Th., 100% Reiheit) aus Beispiel 84 (99% de) $R_t$ = 1.19 min und 14.4 mg (33% d. Th., 100% Reiheit) aus Beispiel 85 (99% de) $R_t$ = 3.14 min.

[Analytische HPLC: Säule Daicel Chiralpak OX-3 3 μm 50x4.6 mm; Eluent: 50% *n*-Heptan, 50% Ethanol; Temperatur: 23°C; Fluss: 1.0 ml/min; UV-Detektion: 220nm].

## Beispiel 84

*N*-[1-(2-Chlorphenyl)-2,2,2-trifluorethyl]-1-(3,5-difluorpyridin-2-yl)-7-[(3*R*,4*R*)- 3,4-dihydroxypyrrolidin-1-yl]-4-oxo-1,4-dihydro-1,8-naphthyridin-3-carbonsäureamid (Diastereomer 1)

LC-MS (Methode 2): $R_t$ = 1.86 min; MS (ESIpos): m/z = 596 [M+H]+.

$^1$H NMR (400 MHz, DMSO-d6): δ [ppm] = 11.53-11.58 (m, 1H), 8.83 (s, 1H), 8.61 (dd, 1H), 8.30-8.37 (m, 2H), 7.47-7.65 (m, 4H), 6.80 (d, 1H), 6.40-6.49 (m, 1H), 5.04-5.30 (m, 2H), 4.03-4.08 (m, 1H), 3.90-3.95 (m, 1H), 3.57-3.67 (m, 1H), 3.33-3.39 (m, 1H), 3.18-3.29 (m, 1H), 3.03-3.17 (m, 1H).

## Beispiel 85

*N*-[1-(2-Chlorphenyl)-2,2,2-trifluorethyl]-1-(3,5-difluorpyridin-2-yl)-7-[(3*R*,4*R*)- 3,4-dihydroxypyrrolidin-1-yl]-4-oxo-1,4-dihydro-1,8-naphthyridin-3-carbonsäureamid (Diastereomer 2)

LC-MS (Methode 2): $R_t$ = 1.86 min; MS (ESIpos): m/z = 596 [M+H]+.

$^1$H NMR (400 MHz, DMSO-d6): δ [ppm] = 11.56 (d, 1H), 8.83 (d, 1H), 8.61 (dd, 1H), 8.30-8.36 (m, 2H), 7.48-7.65 (m, 4H), 6.80 (d, 1H), 6.41-6.49 (m, 1H), 5.28 (d, 0.5H), 5.19 (d, 1H), 5.06 (d, 0.5H), 4.03-4.07 (m, 1H), 3.90-3.96 (m, 1H), 3.57-3.67 (m, 1H), 3.33-3.40 (m, 1H), 3.20-3.30 (m, 1H), 3.02-3.16 (m, 1H).

## Beispiel 86

1-(3,5-Difluorpyridin-2-yl)-7-[(3R,4R)-3,4-dihydroxypyrrolidin-1-yl]-4-oxo-N-[(2R)-1,1,1-trifluor-3,3-dimethylbutan-2-yl]-1,4-dihydro-1,8-naphthyridin-3-carbonsäureamid

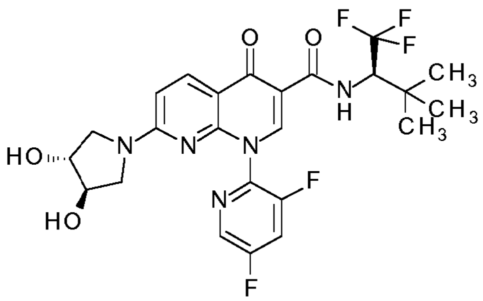

Gemäß allgemeiner Arbeitsvorschrift 1 wurden 30.0 mg (74.2 μmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 13.8 mg (89.0 μmol) (2R)-1,1,1-Trifluor-3,3-dimethylbutan-2-amin in Gegenwart von 33.9 mg (89.0 μmol) HATU und 39.0 μl (223 μmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 26.5 mg (66% d. Th., 100% Reinheit) der Titelverbindung erhalten.
LC-MS (Methode 2): Rt = 1.74 min; MS (ESIpos): m/z = 542 [M+H]+.
$^1$H-NMR (400 MHz, DMSO-d6): δ [ppm] = 10.77 (dd, 1H), 8.83 (d, 1H), 8.61 (d, 1H), 8.29-8.36 (m, 2H), 6.78 (d, 1H),

5.24-5.31 (m, 0.5H), 5.20 (br. s, 1H), 5.04-5.13 (m, 0.5H), 4.58-4.68 (m, 1H), 4.05 (br. s, 1H), 3.93 (br. s, 1H), 3.56-3.68 (m, 1H), 3.35-3.41 (m, 1H), 3.19-3.28 (m, 1H), 3.03-3.17 (m, 1H), 1.09 (br. s, 9H).

**Beispiel 87**

1-(3,5-Difluorpyridin-2-yl)-7-[(3R,4R)-3,4-dihydroxypyrrolidin-1-yl]-4-oxo-N-[1-(trifluormethyl)-cyclopentyl]-1,4-dihydro-1,8-naphthyridin-3-carbonsäureamid

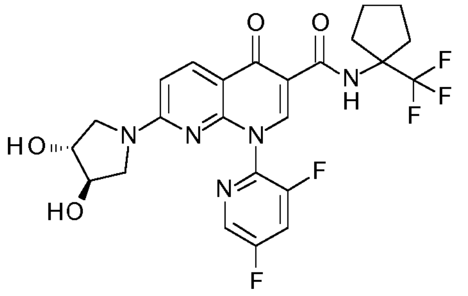

Gemäß allgemeiner Arbeitsvorschrift 1 wurden 30.0 mg (74.2 μmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 16.9 mg (89.0 μmol) 1-(Trifluormethyl)cyclopentanamin-Hydrochlorid in Gegenwart von 33.9 mg (89.0 μmol) HATU und 39.0 μl (223 μmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösung zweimal mittels präparativer HPLC (Säule: Chromatorex C18, 10 μm, 125*40 mm, Lösungsmittel: Acetonitril, Wasser, 0.1% Ameisensäure) gereinigt und 18.3 mg (46% d. Th., 100% Reinheit) der Titelverbindung erhalten.
LC-MS (Methode 2): Rt = 1.72 min; MS (ESIpos): m/z = 540 [M+H]+.
1H NMR (400 MHz, DMSO-d6): δ [ppm] = 10.50 (s, 1H), 8.77 (d, 1H), 8.61 (d, 1H), 8.33 (td, 1H), 8.27 (d, 1H), 6.77 (d, 1H), 5.27 (d, 0.5H), 5.16-5.21 (m, 1H), 5.06 (d, 0.5H), 4.05 (br. s, 1H), 3.90-3.95 (m, 1H), 3.57-3.66 (m, 1H), 3.33-3.38 (m, 1H), 3.18-3.29 (m, 1H), 3.03-3.17 (m, 1H), 2.35-2.44 (m, 2H), 2.00-2.09 (m, 2H), 1.69-1.85 (m, 4H).

**Beispiel 88**

1-(3,5-Difluorpyridin-2-yl)-7-[(3R,4R)-3,4-dihydroxypyrrolidin-1-yl]-4-oxo-N-[3,3,4,4,4-pentafluorbutan-2-yl]-1,4-dihydro-1,8-naphthyridin-3-carbonsäureamid (Diastereomerengemisch)

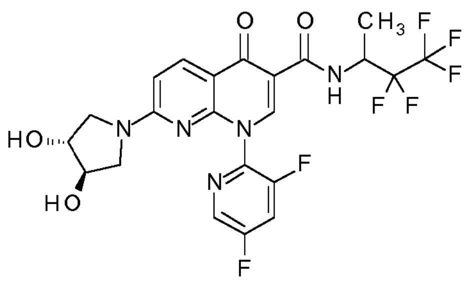

Gemäß allgemeiner Arbeitsvorschrift 1 wurden 30.0 mg (74.2 μmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 17.8 mg (89.0 μmol) (2S)-3,3,4,4,4-Pentafluorbutan-2-amin-Hydrochlorid (Beispiel 28B, Racemat) in Gegenwart von 33.9 mg (89.0 μmol) HATU und 52.0 μl (297 μmol) DIPEA in 0.3 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 34.3 mg (84% d. Th., 100% Reinheit) der Titelverbindung erhalten.
LC-MS (Methode 2): Rt = 1.63 min; MS (ESIpos): m/z = 550 [M+H]+.

$^1$H-NMR (400 MHz, DMSO-d6): δ [ppm] = 10.55 (br. d, 1H), 8.82 (br. t, 1H), 8.59-8.64 (m, 1H), 8.29-8.37 (m, 1H), 8.27 (d, 1H), 6.77 (d, 1H), 5.23-5.30 (m, 0.5H), 5.15-5.23 (m, 1H), 5.06-5.10 (m, 0.5H), 4.96-5.06 (m, 1H), 4.05 (br. s, 1H), 3.93 (br. s, 1H), 3.56-3.68 (m, 1H), 3.33-3.40 (m, 1H), 3.19-3.29 (m, 1H), 3.02-3.18 (m, 1H), 1.39 (br. d, 3H).

17.0 mg der Titelverbindung (Diastereomerengemisch) wurde durch chirale HPLC in die Diastereomere getrennt (präparative HPLC: Säule Daicel Chiralcel OX-H 5 μm 250x20 mm; Eluent: 70% *n*-Heptan, 30% Ethanol + 0.2% DEA; Temperatur: 25°C; Fluss: 15 ml/min; UV-Detektion: 210 nm.)

Man erhielt (in der Reihenfolge der Elution von der Säule) 6.20 mg (15% d. Th., 100% Reinheit) Diastereomer 1 (Beispiel 89) (99% de) Rt = 6.13 min und 7.30 mg (18% d. Th., 100% Reinheit) Diastereomer 2 (Beispiel 90) (99% de) Rt = 7.91 min.

[Analytische HPLC: Säule Daicel Chiralpak OX-3 3 μm 50x4.6 mm; Eluent: 80% *n*-Heptan, 20% Ethanol + 0.2% DEA; Fluss: 1.0 ml/min; UV-Detektion: 220nm].

## Beispiel 89

1-(3,5-Difluorpyridin-2-yl)-7-[(3R,4R)-3,4-dihydroxypyrrolidin-1-yl]-4-oxo-N-[3,3,4,4,4-pentafluorbutan-2-yl]-1,4-dihydro-1,8-naphthyridin-3-carbonsäureamid (Diastereomer 1)

LC-MS (Methode 1): Rt = 0.90 min; MS (ESIpos): m/z = 550 [M+H]+.

$^1$H-NMR (400 MHz, DMSO-d6): δ [ppm] = 10.56 (d, 1H), 8.82 (d, 1H), 8.61 (d, 1H), 8.29-8.36 (m, 1H), 8.27 (d, 1H), 6.77 (d, 1H), 5.25-5.31 (m, 0.5H), 5.19 (d, 1H), 4.96-5.10 (m, 1.5H), 4.05 (br. s, 1H), 3.93 (br. s, 1H), 3.56-3.67 (m, 1H), 3.34-3.43 (m, 1H), 3.19-3.30 (m, 1H), 3.02-3.17 (m, 1H), 1.40 (br. d, 3H).

## Beispiel 90

1-(3,5-Difluorpyridin-2-yl)-7-[(3R,4R)-3,4-dihydroxypyrrolidin-1-yl]-4-oxo-N-[3,3,4,4,4-pentafluorbutan-2-yl]-1,4-dihydro-1,8-naphthyridin-3-carbonsäureamid (Diastereomer 2)

LC-MS (Methode 1): Rt = 0.90 min; MS (ESIpos): m/z = 550 [M+H]+.

$^1$H-NMR (400 MHz, DMSO-d6): δ [ppm] = 10.56 (br. d, 1H), 8.82 (s, 1H), 8.61 (t, 1H), 8.30-8.36 (m, 1H), 8.27 (d, 1H), 6.78 (d, 1H), 5.23-5.31 (m, 0.5H), 5.16-5.23 (m, 1H), 4.95-5.11 (m, 1.5H), 4.05 (br. s, 1H), 3.89-3.96 (m, 1H), 3.56-3.68 (m, 1H), 3.34-3.43 (m, 1H), 3.18-3.29 (m, 1H), 3.03-3.17 (m, 1H), 1.39 (br. d, 3H).

## Beispiel 91

1-(3,5-Difluorpyridin-2-yl)-7-[(3R,4R)-3,4-dihydroxypyrrolidin-1-yl]-4-oxo-N-(4,4,4-trifluor-2-methylbutan-2-yl)-1,4-dihydro-1,8-naphthyridin-3-carbonsäureamid

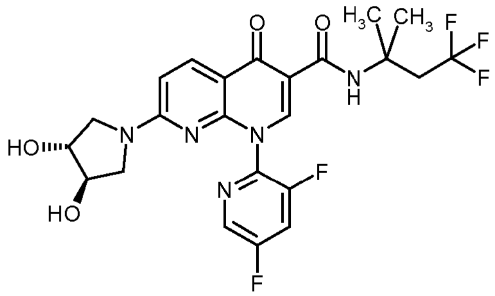

Gemäß allgemeiner Arbeitsvorschrift 1 wurden 50.0 mg (124 μmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 26.4 mg (148 μmol) 4,4,4-Trifluor-2-methylbutan-2-amin-Hydrochlorid in Gegenwart von 56.4 mg (148 μmol) HATU und 65.0 μl (371 μmol) DIPEA in 0.5 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 36.9 mg (57% d. Th., 100% Reinheit) der Titelverbindung erhalten.
LC-MS (Methode 2): Rt = 1.58 min; MS (ESIpos): m/z = 528 [M+H]+.

$^1$H-NMR (400 MHz, DMSO-d6): δ [ppm] = 10.16 (br. s, 1H), 8.73 (d, 1H), 8.61 (d, 1H), 8.29-8.35 (m, 1H), 8.26 (d, 1H), 6.75 (d, 1H), 5.01-5.33 (m, 2H), 4.00-4.09 (m, 1H), 3.87-3.96 (m, 1H), 3.56-3.66 (m, 1H), 3.35-3.45 (m, 1H), 3.02-3.23 (m, 2H), 2.89-3.01 (m, 2H), 1.48 (br. s, 6H).

**Beispiel 92**

1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomerengemisch)

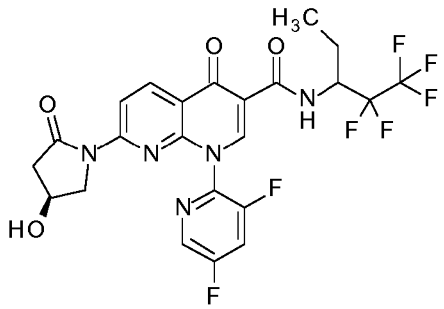

Gemäß allgemeiner Arbeitsvorschrift 1 wurden 100 mg (249 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 63.7 mg (298 μmol) (3S)-1,1,1,2,2-Pentafluorpentan-3-amin-Hydrochlorid (Beispiel 14B, Racemat) in Gegenwart von 113 mg (298 μmol) HATU und 173 μl (994 μmol) DIPEA in 1 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 97.4 mg (70% d. Th., 100% Reinheit) der Titelverbindung erhalten.
LC-MS (Methode 2): Rt = 1.88 min; MS (ESIpos): m/z = 562 [M+H]+.
$^1$H-NMR (400 MHz, DMSO-d6): δ [ppm] = 10.16 (d, 1H), 9.06 (s, 1H), 8.72 (d, 1H), 8.63-8.69 (m, 1H), 8.51-8.58 (m, 1H), 8.39 (ddd, 1H), 5.31-5.41 (m, 0.5H), 5.26 (br. s, 0.5H), 4.83-4.97 (m, 1H), 4.29 (br. s, 1H), 3.65-3.79 (m, 1H), 3.44-3.57 (m, 1H), 2.88-3.03 (m, 1H), 2.32-2.44 (m, 1H), 1.88-1.99 (m, 1H), 1.62-1.74 (m, 1H), 0.94-1.01 (m, 3H).

83.0 mg der Titelverbindung (Diastereomerengemisch) wurde durch chirale HPLC in die Diastereomere getrennt (präparative HPLC: Säule Daicel Chiralpak IE 5 μm 250x20 mm; Eluent: 70% *n*-Heptan, 30% Iso-Propanol; Temperatur: 35°C; Fluss: 15 ml/min; UV-Detektion: 220 nm.)

Man erhielt (in der Reihenfolge der Elution von der Säule) 34.0 mg (24% d. Th. ,100% Reinheit) Diastereomer 1 (Beispiel 93) (98.9% de) Rt = 9.56 min und 37.0 mg (27% d. Th., 100% Reinheit) Diastereomer 2 (Beispiel 94) (95.8% de) Rt = 13.40 min.

[Analytische HPLC: Säule Daicel Chiralpak IE 5 μm 250x4.6 mm; Eluent: 60% Iso-Hexan, 40% Iso-Propanol; Temperatur: 35°C; Fluss: 1.0 ml/min; UV-Detektion: 220nm].

**Beispiel 93**

1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomer 1)

LC-MS (Methode 2): Rt = 1.90 min; MS (ESIpos): m/z = 562 [M+H]+.

$^1$H-NMR (400 MHz, DMSO-d6): δ [ppm] = 10.16 (d, 1H), 9.06 (s, 1H), 8.72 (d, 1H), 8.63-8.68 (m, 1H), 8.54 (br. t, 1H), 8.39 (ddd, 1H), 5.38 (br. d, 0.5H), 5.26 (br. d, 0.5H), 4.83-4.97 (m, 1H), 4.29 (br. s, 1H), 3.63-3.78 (m, 1H), 3.43-3.57 (m, 1H), 2.87-3.03 (m, 1H), 2.34-2.44 (m, 1H), 1.88-1.99 (m, 1H), 1.62-1.74 (m, 1H), 0.92-1.01 (m, 3H).

**Beispiel 94**

1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomer 2)

LC-MS (Methode 2): Rt = 1.90 min; MS (ESIpos): m/z = 562 [M+H]+.

¹H-NMR (400 MHz, DMSO-d6): δ [ppm] = 10.16 (br. d, 1H), 9.06 (br. d, 1H), 8.72 (d, 1H), 8.66 (br. s, 1H), 8.54 (br. t, 1H), 8.39 (br. t, 1H), 5.38 (br. d, 0.5H), 5.26 (br. d, 0.5H), 4.83-4.97 (m, 1H), 4.26-4.32 (m, 1H), 3.64-3.79 (m, 1H), 3.45-3.57 (m, 1H), 2.88-3.03 (m, 1H), 2.34-2.43 (m, 1H), 1.88-1.99 (m, 1H), 1.62-1.74 (m, 1H), 0.93-1.02 (m, 3H).

## Beispiel 95

1-(3,5-Difluorpyridin-2-yl)-7-[(3R,4R)-3,4-dihydroxypyrrolidin-1-yl]-4-oxo-N-[1,1,1,2,2-pentafluorpentan-3-yl]-1,4-dihydro-1,8-naphthyridin-3-carbonsäureamid (Diastereomerengemisch)

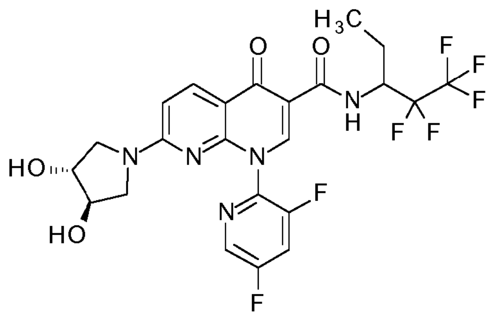

Gemäß allgemeiner Arbeitsvorschrift 3 wurden 50.0 mg (101 μmol) 7-Chlor-1-(3,5-difluorpyridin-2-yl)-4-oxo-N-[(3S)-1,1,1,2,2-pentafluorpentan-3-yl]-1,4-dihydro-1,8-naphthyridin-3-carbonsäureamid (Beispiel 15A, Racemat) mit 12.5 mg (121 μmol) (3R,4R)-Pyrrolidin-3,4-diol-Hydrochlorid in Gegenwart von 61.0 μl (352 μmol) DIPEA in 0.5 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 51.3 mg (90% d. Th., 100% Reinheit) der Titelverbindung erhalten.
LC-MS (Methode 1): Rt = 0.97 min; MS (ESIpos): m/z = 564 [M+H]+.
¹H-NMR (400 MHz, DMSO-d6): δ [ppm] = 10.47 (br. d, 1H), 8.81 (br. s, 1H), 8.61 (br. s, 1H), 8.22-8.41 (m, 2H), 6.78 (br. d, 1H), 5.02-5.33 (m, 2H), 4.79-4.98 (m, 1H), 4.00-4.12 (m, 1H), 3.86-3.98 (m, 1H), 3.55-3.69 (m, 1H), 3.00-3.26 (m, 2H), 1.83-2.01 (m, 1H), 1.57-1.75 (m, 1H), 0.87-1.06 (m, 3H).

37.0 mg der Titelverbindung (Diastereomerengemisch) wurde durch chirale HPLC in die Diastereomere getrennt (präparative HPLC: Säule Daicel Chiralcel OX-H 5 μm 250x20 mm; Eluent: 80% *n*-Heptan, 20% Ethanol; Temperatur: 35°C; Fluss: 15 ml/min; UV-Detektion: 220 nm.)

Man erhielt (in der Reihenfolge der Elution von der Säule) 16.0 mg (28% d. Th., 100% Reinheit) aus Beispiel 96 (99.9% de) $R_t$ = 5.45 min und 16.0 mg (28% d. Th., 100% Reinheit) aus Beispiel 97 (99.9% de) $R_t$ = 6.39 min.

[Analytische HPLC: Säule Daicel Chiralcel OX-H 5 μm 250x4.6 mm; Eluent: 80% Iso-Hexan, 20% Ethanol; Temperatur: 30°C; Fluss: 1.0 ml/min; UV-Detektion: 220nm].

## Beispiel 96

1-(3,5-Difluorpyridin-2-yl)-7-[(3*R*,4*R*)-3,4-dihydroxypyrrolidin-1-yl]-4-oxo-*N*-[1,1,1,2,2-pentafluorpentan-3-yl]-1,4-dihydro-1,8-naphthyridin-3-carbonsäureamid (Diastereomer 1)

LC-MS (Methode 2): $R_t$ = 1.72 min; MS (ESIpos): m/z = 564 [M+H]+.

¹H NMR (400 MHz, DMSO-d6): δ [ppm] = 10.47 (d, 1H), 8.82 (d, 1H), 8.61 (d, 1H), 8.30-8.36 (m, 1H), 8.28 (d, 1H), 6.78 (d, 1H), 5.28 (br d, 0.5H), 5.15-5.23 (m, 1H), 5.07 (br d, 0.5H), 4.80-4.94 (m, 1H), 4.00-4.08 (m, 1H), 3.87-3.97 (m, 1H), 3.55-3.67 (m, 1H), 3.33-3.40 (m, 1H), 3.19-3.29 (m, 1H), 3.03-3.17 (m, 1H), 1.86-1.98 (m, 1H), 1.59-1.72 (m, 1H), 0.93-1.00 (m, 3H).

## Beispiel 97

1-(3,5-Difluorpyridin-2-yl)-7-[(3*R*,4*R*)-3,4-dihydroxypyrrolidin-1-yl]-4-oxo-*N*-[1,1,1,2,2-pentafluorpentan-3-yl]-1,4-dihydro-1,8-naphthyridin-3-carbonsäureamid (Diastereomer 2)

LC-MS (MCW-FT-MS-M1-Methode 2): $R_t$ = 1.72 min; MS (ESIpos): m/z = 564 [M+H]+.

$^1$H NMR (400 MHz, DMSO-d6): δ [ppm] = 10.47 (d, 1H), 8.82 (s, 1H), 8.61 (t, 1H), 8.30-8.37 (m, 1H), 8.28 (d, 1H), 6.78 (d, 1H), 5.28 (br d, 0.5H), 5.19 (br t, 1H), 5.07 (br d, 0.5H), 4.80-4.94 (m, 1H), 4.05 (br s, 1H), 3.93 (br s, 1H), 3.56-3.68 (m, 1H), 3.33-3.39 (m, 1H), 3.19-3.29 (m, 1H), 3.02-3.18 (m, 1H), 1.86-1.98 (m, 1H), 1.59-1.72 (m, 1H), 0.91-1.01 (m, 3H).

**Beispiel 98**

1-(3,5-Difluorpyridin-2-yl)-7-[(4S)-4-hydroxy-2-oxopyrrolidin-1-yl]-4-oxo-N-[(2S)-1,1,1-trifluor-3,3-dimethylbutan-2-yl]-1,4-dihydro-1,8-naphthyridin-3-carbonsäureamid

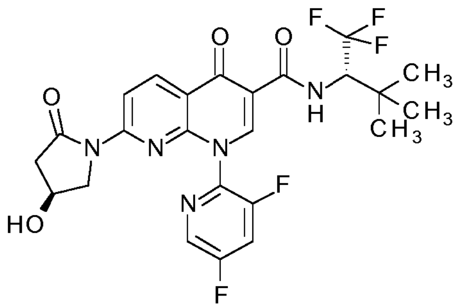

Gemäß allgemeiner Arbeitsvorschrift 1 wurden 60.0 mg (149 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 27.8mg (179 μmol) (2S)-1,1,1-Trifluor-3,3-dimethylbutan-2-amin in Gegenwart von 68.0 mg (179 μmol) HATU und 104 μl (597 μmol) DIPEA in 0.6 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 47.5 mg (59% d. Th., 100% Reinheit) der Titelverbindung erhalten.
LC-MS (Methode 2): Rt = 1.96 min; MS (ESIpos): m/z = 540 [M+H]+.
$^1$H-NMR (400 MHz, DMSO-d6): δ [ppm] = 10.48 (br. dd, 1H), 9.07 (s, 1H), 8.74 (d, 1H), 8.64-8.69 (m, 1H), 8.54 (t, 1H), 8.36-8.42 (m, 1H), 5.38 (br. d, 0.5H), 5.26 (br. d, 0.5H), 4.66 (quint, 1H), 4.26-4.32 (m, 1H), 3.65-3.78 (m, 1H), 3.46-3.57 (m, 1H), 2.89-3.02 (m, 1H), 2.33-2.44 (m, 1H), 1.10 (s, 9H).

**Beispiel 99**

1-(3,5-Difluorpyridin-2-yl)-7-[(3R,4R)-3,4-dihydroxypyrrolidin-1-yl]-4-oxo-N-[(2S)-1,1,1-trifluor-3,3-dimethylbutan-2-yl]-1,4-dihydro-1,8-naphthyridin-3-carbonsäureamid

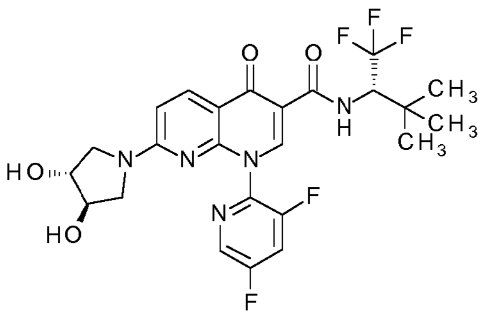

Gemäß allgemeiner Arbeitsvorschrift 3 wurden 80.0 mg (168 μmol) 7-Chlor-1-(3,5-difluorpyridin-2-yl)-4-oxo-N-[(2S)-1,1,1-trifluor-3,3-dimethylbutan-2-yl]-1,4-dihydro-1,8-naphthyridin-3-carbonsäureamid (Beispiel 13A) mit 28.2 mg (202 μmol) (3R,4R)-Pyrrolidin-3,4-diol-Hydrochlorid in Gegenwart von 103 μl (590 μmol) DIPEA in 0.8 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungs-

mittel: Acetonitril, Wasser, 0.1% Ameisensäure) gereinigt und 85.3 mg (93% d. Th., 100% Reinheit) der Titelverbindung erhalten.
LC-MS (Methode 2): Rt = 1.76 min; MS (ESIpos): m/z = 542 [M+H]+.
$^1$H-NMR (400 MHz, DMSO-d6): δ [ppm] = 10.77 (dd, 1H), 8.83 (d, 1H), 8.61 (t, 1H), 8.28-8.36 (m, 2H), 6.78 (d, 1H), 5.28 (br. d, 0.5H), 5.20 (d, 1H), 5.07 (br. d, 0.5H), 4.63 (quintt, 1H), 4.05 (br. s, 1H), 3.93 (br. s, 1H), 3.56-3.68 (m, 1H), 3.33-3.40 (m, 1H), 3.19-3.30 (m, 1H), 3.03-3.18 (m, 1H), 1.09 (br. s, 9H).

**Beispiel 100**

1-(3,5-Difluorpyridin-2-yl)-4-oxo-7-(2-oxoimidazolidin-1-yl)-N-[(2S)-1,1,1-trifluor-3,3-dimethylbutan-2-yl]-1,4-dihydro-1,8-naphthyridin-3-carbonsäureamid

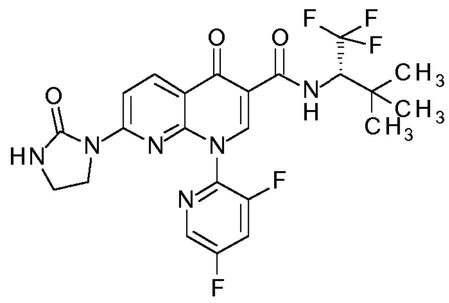

Gemäß allgemeiner Arbeitsvorschrift 2 wurden 80.0 mg (168 μmol) 7-Chlor-1-(3,5-difluorpyridin-2-yl)-4-oxo-N-[(2S)-1,1,1-trifluor-3,3-dimethylbutan-2-yl]-1,4-dihydro-1,8-naphthyridin-3-carbonsäureamid (Beispiel 13A) mit 145 mg (1.69 mmol) Imidazolidin-2-on in Gegenwart von 34.9 mg (253 μmol) Kaliumcarbonat, 7.57 mg (34.0 μmol) Palladiumacetat und 19.5 mg (34.0 μmol) Xantphos in 1.6 ml Dioxan bei 80°C zur Reaktion gebracht. Es wurde in Acetonitril, Wasser und Ameisensäure verdünnt, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 40.5 mg (46% d. Th., 100% Reinheit) der Titelverbindung erhalten.
LC-MS (Methode 2): Rt = 1.97 min; MS (ESIpos): m/z = 525 [M+H]+.
$^1$H-NMR (400 MHz, DMSO-d6): δ [ppm] = 10.49-10.67 (m, 1H), 9.00 (br. s, 1H), 8.52-8.71 (m, 2H), 8.40-8.51 (m, 1H), 8.25-8.40 (m, 1H), 7.67 (br. s, 1H), 4.57-4.74 (m, 1H), 3.54-3.71 (m, 2H), 1.10 (br. s, 9H).

**Beispiel 101**

N-(Bicyclo[1.1.1]pent-1-yl)-1-(3,5-difluorpyridin-2-yl)-7-[(4S)-4-hydroxy-2-oxopyrrolidin-1-yl]-4-oxo-1,4-dihydro-1,8-naphthyridin-3-carbonsäureamid

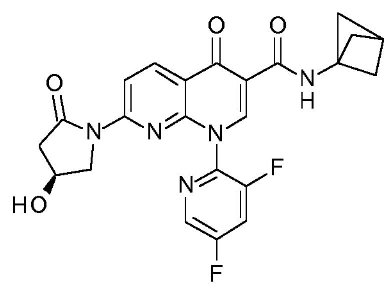

Gemäß allgemeiner Arbeitsvorschrift 1 wurden 60.0 mg (149 μmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 21.4 mg (179 μmol) Bicyclo[1.1.1]pentan-1-amin-Hydrochlorid in Gegenwart von 68.0 mg (179 μmol) HATU und 78.0 μl (447 μmol) DIPEA in 0.6 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40

mm, Lösungsmittel: Acetonitril, Wasser, 0.1% Ameisensäure) gereinigt und 39.7 mg (57% d. Th., 100% Reinheit) der Titelverbindung erhalten.
LC-MS (Methode 2): Rt = 1.62 min; MS (ESIpos): m/z = 468 [M+H]+.
$^1$H-NMR (400 MHz, DMSO-d6): δ [ppm] = 10.02 (s, 1H), 8.95 (s, 1H), 8.65-8.69 (m, 2H), 8.49-8.55 (m, 1H), 8.36-8.42 (m, 1H), 5.35-5.39 (m, 0.5H), 5.24-5.27 (m, 0.5H), 4.26-4.31 (m, 1H), 3.64-3.77 (m, 1H), 3.44-3.55 (m, 1H), 2.88-3.01 (m, 1H), 2.32-2.42 (m, 1H), 2.11 (s, 6H).

**Beispiel 102**

1-(3,5-Difluorpyridin-2-yl)-N-(3-fluorbicyclo[1.1.1]pent-1-yl)-7-[(4S)-4-hydroxy-2-oxopyrrolidin-1-yl]-4-oxo-1,4-dihydro-1,8-naphthyridin-3-carbonsäureamid

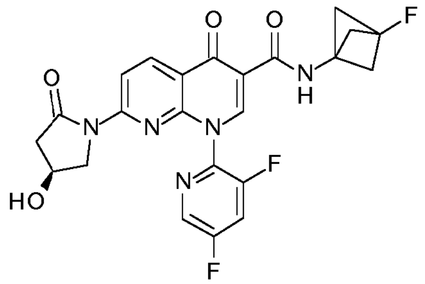

Gemäß allgemeiner Arbeitsvorschrift 1 wurden 60.0 mg (149 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 27.4 mg (179 µmol, 90% Reinheit) 3-Fluorbicyclo[1.1.1]pentan-1-amin-Hydrochlorid in Gegenwart von 68.0 mg (179 µmol) HATU und 78.0 µl (447 µmol) DIPEA in 0.6 ml DMF zur Reaktion gebracht. Das Reaktionsgemisch wurde mit Acetonitril, Wasser und Ameisensäure verdünnt, über einen Milliporefilter filtriert und die Rohlösung zweimal mittels präparativer HPLC (Säule: Chromatorex C18, 10 µm, 125*40 mm, Lösungsmittel: Acetonitril, Wasser, 0.1% Ameisensäure) gereinigt und 9.00 mg (12% d. Th., 100% Reinheit) der Titelverbindung erhalten.
LC-MS (Methode 2): Rt = 1.57 min; MS (ESIpos): m/z = 486 [M+H]+.
$^1$H-NMR (400 MHz, DMSO-d6): δ [ppm] = 10.13 (s, 1H), 8.97 (s, 1H), 8.64-8.69 (m, 2H), 8.49-8.57 (m, 1H), 8.36-8.42 (m, 1H), 5.34-5.40 (m, 0.5H), 5.23-5.28 (m, 0.5H), 4.18-4.33 (m, 1H), 3.43-3.79 (m, 3H), 2.88-3.01 (m, 1H), 2.46 (d, 6H).

**Beispiel 103**

N-(1,3-Difluor-2-methylpropan-2-yl)-1-(3,5-difluorpyridin-2-yl)-7-[(3R,4R)-3,4-dihydroxypyrrolidin-1-yl]-4-oxo-1,4-dihydro-1,8-naphthyridin-3-carbonsäureamid

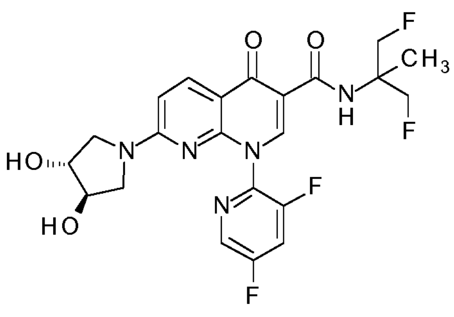

Gemäß allgemeiner Arbeitsvorschrift 1 wurden 30.0 mg (74.2 µmol) 1-(3,5-Difluorpyridin-2-yl)-7-[(3R,4R)-3,4-dihydroxypyrrolidin-1-yl]-4-oxo-1,4-dihydro-1,8-naphthyridin-3-carbonsäure (Beispiel 23B) mit 13.0 mg (89.0 µmol) 1,3-Difluor-2-methylpropan-2-amin-Hydrochlorid in Gegenwart von 33.9 mg (89.0 µmol) HATU und 39.0 µl (223 µmol) DIPEA in 0.3 ml DMF zur Reaktion gebracht. Das Reaktionsgemisch wurde mit Acetonitril, Wasser und Ameisensäure

verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 28.6 mg (78% d. Th., 100% Reinheit) der Titelverbindung erhalten.
LC-MS (Methode 2): Rt = 1.37 min; MS (ESIpos): m/z = 496 [M+H]+.
$^1$H-NMR (400 MHz, DMSO-d6): δ [ppm] = 10.35 (s, 1H), 8.75 (d, 1H), 8.61 (d, 1H), 8.30-8.36 (m, 1H), 8.27 (d, 1H), 6.76 (d, 1H), 5.26 (br. d, 0.5H), 5.15-5.22 (m, 1H), 5.06 (br. d, 0.5H), 4.69-4.78 (m, 2H), 4.57-4.66 (m, 2H), 4.05 (br. s, 1H), 3.92 (br. s, 1H), 3.55-3.67 (m, 1H), 3.33-3.38 (m, 1H), 3.18-3.29 (m, 1H), 3.02-3.17 (m, 1H), 1.40-1.45 (m, 3H).

**Beispiel 104**

N-[(1S)-1-Cyclopropyl-2,2,2-trifluorethyl]-1-(3,5-difluorpyridin-2-yl)-7-[4-hydroxy-3-methyl-2-oxopyrrolidin-1-yl]-4-oxo-1,4-dihydro-1,8-naphthyridin-3-carbonsäureamid (racemisches Diastereomerengemisch)

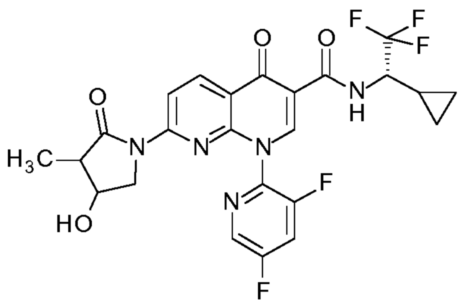

Gemäß allgemeiner Arbeitsvorschrift 2 wurden 50.0 mg (109 µmol) 7-Chlor-N-[(1S)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 15.1 mg (131 µmol) 4-Hydroxy-3-methylpyrrolidin-2-on (Beispiel 2D) in Gegenwart von 22.6 mg (163 µmol) Kaliumcarbonat, 4.89 mg (22.0 µmol) Palladiumacetat und 12.6 mg (22.0 µmol) Xantphos in 1 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurde aus Acetonitril umkristallisiert und 19.5 mg (33% d. Th., 100% Reinheit) der Titelverbindung erhalten.
LC-MS (Methode 1): Rt = 1.01 min; MS (ESIpos): m/z = 538 [M+H]+.
$^1$H-NMR (400 MHz, DMSO-d6): δ [ppm] = 10.23-10.30 (m, 1H), 9.03-9.06 (m, 1H), 8.71 (d, 1H), 8.65-8.69 (m, 1H), 8.50-8.59 (m, 1H), 8.36-8.43 (m, 1H), 5.43-5.54 (m, 0.25H), 5.10-5.33 (m, 0.75H), 4.37-4.47 (m, 1H), 4.19 (br. q, 0.75H), 3.75-3.98 (m, 0.5H), 3.48-3.69 (m, 1.5H), 3.22-3.28 (m, 0.25H), 2.77-2.92 (m, 0.75H), 1.05-1.28 (m, 4H), 0.52-0.70 (m, 3H), 0.30-0.39 (m, 1H).

13.0 mg der Titelverbindung (Diastereomerengemisch) wurde durch chirale HPLC in die Diastereomere getrennt (präparative HPLC: Säule Daicel Chiralcel OZ-H 5 µm 250x20 mm; Eluent: 80% *n*-Heptan, 20% Ethanol; Temperatur: 23°C; Fluss: 20 ml/min; UV-Detektion: 258 nm.)

Man erhielt (in der Reihenfolge der Elution von der Säule) 1.70 mg (2.9% d. Th., 100% Reinheit) Diastereomer 1 (Racemat; Beispiel 105) (55% de, rac) Rt = 11.29 min, 1.30 mg (2.2% d. Th., 100% Reinheit) Diastereomer 2 (Enantiomer A; Beispiel 106) (95.6% de) Rt = 14.43 min und 3.10 mg (5.2% d. Th., 100% Reinheit) Diastereomer 2 (Enantiomer B; Beispiel 107) (99.9% de) Rt = 29.03 min.

[Analytische HPLC: Säule Daicel Chiralpak OZ-3 3 µm 50x4.6 mm; Eluent: 80% *n*-Heptan, 20% Ethanol; Fluss: 1.0 ml/min; UV-Detektion: 220nm].

**Beispiel 105**

N-[(1S)-1-Cyclopropyl-2,2,2-trifluorethyl]-1-(3,5-difluorpyridin-2-yl)-7-[4-hydroxy-3-methyl-2-oxopyrrolidin-1-yl]-4-oxo-1,4-dihydro-1,8-naphthyridin-3-carbonsäureamid (Diastereomer 1, Racemat)

LC-MS (Methode 2): Rt = 1.92 min; MS (ESIpos): m/z = 538 [M+H]+.

1H NMR (400 MHz, DMSO-d6): δ [ppm] = 10.13-10.32 (m, 1H), 9.02-9.08 (m, 1H), 8.71 (d, 1H), 8.67 (d, 1H), 8.48-8.59 (m, 1H), 8.36-8.44 (m, 1H), 5.45-5.53 (m, 1H), 4.37-4.46 (m, 1H), 3.44-4.04 (m, 2H), 1.06-1.31 (m, 6H), 0.51-0.71 (m, 3H), 0.31-0.39 (m, 1H).

**Beispiel 106**

N-[(1S)-1-Cyclopropyl-2,2,2-trifluorethyl]-1-(3,5-difluorpyridin-2-yl)-7-[4-hydroxy-3-methyl-2-oxopyrrolidin-1-yl]-4-oxo-1,4-dihydro-1,8-naphthyridin-3-carbonsäureamid (Diastereomer 2, Enantiomer A)

LC-MS (Methode 2): Rt = 1.91 min; MS (ESIpos): m/z = 538 [M+H]+.

$^1$H NMR (400 MHz, DMSO-d6): δ [ppm] = 10.26 (d, 1H), 9.04 (s, 1H), 8.71 (d, 1H), 8.65-8.69 (m, 1H), 8.51-8.59 (m, 1H), 8.36-8.42 (m, 1H), 5.29 (br. d, 0.5H), 5.15 (br. d, 0.5H), 4.37-4.48 (m, 1H), 4.19 (br. q, 1H), 3.46-3.68 (m, 2H), 2.77-2.92 (m, 1H), 1.05-1.30 (m, 4H), 0.51-0.71 (m, 3H), 0.30-0.39 (m, 1H).

**Beispiel 107**

N-[(1S)-1-Cyclopropyl-2,2,2-trifluorethyl]-1-(3,5-difluorpyridin-2-yl)-7-[4-hydroxy-3-methyl-2-oxopyrrolidin-1-yl]-4-oxo-1,4-dihydro-1,8-naphthyridin-3-carbonsäureamid (Diastereomer 2, Enantiomer B)

LC-MS (Methode 2): Rt = 1.91 min; MS (ESIpos): m/z = 538 [M+H]+.

$^1$H-NMR (400 MHz, DMSO-d6): δ [ppm] = 10.18-10.32 (m, 1H), 9.04 (s, 1H), 8.69-8.73 (m, 1H), 8.67 (br s, 1H), 8.49-8.60 (m, 1H), 8.35-8.43 (m, 1H), 5.11-5.54 (m, 1H), 4.38-4.46 (m, 1H), 4.13-4.23 (m, 1H), 3.45-3.70 (m, 1H), 2.75-2.94 (m, 1H), 1.04-1.32 (m, 5H), 0.47-0.75 (m, 3H), 0.27-0.43 (m, 1H).

**Beispiel 108**

1-(3,5-Difluorpyridin-2-yl)-7-[4-hydroxy-3-methyl-2-oxopyrrolidin-1-yl]-4-oxo-N-[(2S)-1,1,1-trifluor-butan-2-yl]-1,4-dihydro-1,8-naphthyridin-3-carbonsäureamid (racemisches Diastereomerengemisch)

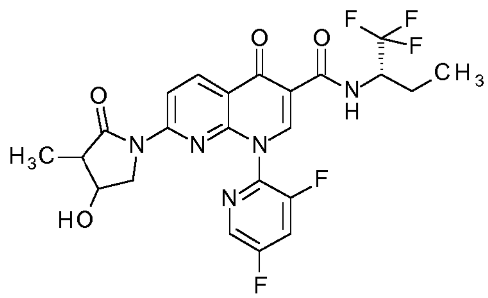

Gemäß allgemeiner Arbeitsvorschrift 2 wurden 50.0 mg (112 μmol) 7-Chlor-1-(3,5-difluorpyridin-2-yl)-4-oxo-*N*-[(2S)-1,1,1-trifluorbutan-2-yl]-1,4-dihydro-1,8-naphthyridin-3-carbonsäureamid (Beispiel 5A) mit 15.5 mg (134 μmol) 4-Hydroxy-3-methylpyrrolidin-2-on (Beispiel 2D) in Gegenwart von 23.2 mg (168 μmol) Kaliumcarbonat, 5.03 mg (22.0 μmol) Palladiumacetat und 13.0 mg (22.0 μmol) Xantphos in 1 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt. Es wurde aus Acetonitril umkristallisiert und 23.7 mg (40% d. Th., 100% Reinheit) der Titelverbindung erhalten.
LC-MS (Methode 1): Rt = 0.99 min; MS (ESIpos): m/z = 526 [M+H]+.
$^1$H-NMR 400 MHz, DMSO-d6): δ [ppm] = 10.09-10.15 (m, 1H), 9.02-9.09 (m, 1H), 8.63-8.74 (m, 2H), 8.50-8.59 (m, 1H), 8.35-8.45 (m, 1H), 5.44-5.53 (m, 0.25H), 5.10-5.32 (m, 0.75H), 4.71-4.83 (m, 1H), 4.19 (br. q, 0.75H), 3.74-3.98 (m, 0.5H), 3.48-3.68 (m, 1.5H), 3.20-3.28 (m, 0.25H), 2.76-2.92 (m, 0.75H), 1.84-1.95 (m, 1H), 1.61-1.73 (m, 1H), 1.05-1.18 (m, 3H), 0.94-1.02 (m, 3H).

17.0 mg der Titelverbindung (Diastereomerengemisch) wurde durch chirale HPLC in die Diastereomere getrennt (präparative HPLC: Säule Daicel IA 250x20 mm; Eluent: 80% *n*-Heptan, 20% Ethanol; Temperatur: 23°C; Fluss: 20 ml/min; UV-Detektion: 220 nm.)

Man erhielt (in der Reihenfolge der Elution von der Säule) 3.30 mg (5.6% d. Th., 100% Reinheit) Diastereomer 1 (Racemat; Beispiel 109) (99.9 de, rac) $R_t$ = 10.37/10.83 min, 4.10 mg (6.9% d. Th., 100% Reinheit) Diastereomer 2 (Enantiomer A; Beispiel 110) (86.46% de) $R_t$ = 11.78 min und 4.50 mg (7.6% d. Th., 100% Reinheit) Diastereomer 2 (Enantiomer B; Beispiel 111) (99.9% de) $R_t$ = 13.61 min.

[Analytische HPLC: Säule Daicel Chiralpak IA-3 3 µm 50x4.6 mm; Eluent: 80% Iso-Hexan, 20% Ethanol; Fluss: 1.0 ml/min; UV-Detektion: 220nm].

**Beispiel 109**

1-(3,5-Difluorpyridin-2-yl)-7-[4-hydroxy-3-methyl-2-oxopyrrolidin-1-yl]-4-oxo-N-[(2S)-1,1,1-trifluor-butan-2-yl]-1,4-dihydro-1,8-naphthyridin-3-carbonsäureamid (Diastereomer 1, Racemat)

LC-MS (Methode 2): Rt = 1.89 min; MS (ESIpos): m/z = 526 [M+H]+.

$^1$H-NMR (400 MHz, DMSO-d6): δ [ppm] = 10.11 (d, 1H), 9.06 (s, 1H), 8.71 (d, 1H), 8.67 (d, 1H), 8.52 (d, 1H), 8.37-8.44 (m, 1H), 5.44-5.54 (m, 1H), 4.72-4.82 (m, 1H), 3.86-3.98 (m, 1H), 3.74-3.85 (m, 1H), 3.22-3.29 (m, 1H), 1.84-1.95 (m, 1H), 1.60-1.72 (m, 1H), 1.11-1.19 (m, 3H), 0.94-1.02 (m, 3H).

**Beispiel 110**

1-(3,5-Difluorpyridin-2-yl)-7-[4-hydroxy-3-methyl-2-oxopyrrolidin-1-yl]-4-oxo-N-[(2S)-1,1,1-trifluorbutan-2-yl]-1,4-dihydro-1,8-naphthyridin-3-carbonsäureamid (Diastereomer 2, Enantiomer A)

LC-MS (Methode 2): Rt = 1.88 min; MS (ESIpos): m/z = 526 [M+H]+.

$^1$H-NMR (400 MHz, DMSO-d6): δ [ppm] = 10.12 (d, 1H), 9.05 (s, 1H), 8.71 (d, 1H), 8.67 (br. s, 1H), 8.56 (br. t, 1H), 8.39 (ddd, 1H), 5.29 (br. d, 0.5H), 5.16 (br. d, 0.5H), 4.72-4.83 (m, 1H), 4.17-4.22 (m, 1H), 3.47-3.71 (m, 2H), 2.76-2.92 (m, 1H), 1.84-1.95 (m, 1H), 1.61-1.73 (m, 1H), 1.05-1.12 (m, 3H), 0.94-1.03 (m, 3H).

**Beispiel 111**

1-(3,5-Difluorpyridin-2-yl)-7-[4-hydroxy-3-methyl-2-oxopyrrolidin-1-yl]-4-oxo-N-[(2S)-1,1,1-trifluorbutan-2-yl]-1,4-dihydro-1,8-naphthyridin-3-carbonsäureamid (Diastereomer 2, Enantiomer B)

LC-MS (Methode 2): Rt = 1.88 min; MS (ESIpos): m/z = 526 [M+H]+.

$^1$H-NMR (400 MHz, DMSO-d6): δ [ppm] = 10.12 (br. d, 1H), 9.05 (s, 1H), 8.71 (d, 1H), 8.65-8.69 (m, 1H), 8.52-8.58 (m, 1H), 8.36-8.42 (m, 1H), 5.29 (br. d, 0.5H), 5.15 (br. d, 0.5H), 4.72-4.84 (m, 1H), 4.19 (q, 1H), 3.48-3.68 (m, 2H), 2.76-2.93 (m, 1H), 1.85-1.95 (m, 1H), 1.62-1.73 (m, 1H), 1.05-1.11 (m, 3H), 0.94-1.02 (m, 3H).

**Beispiel 112**

1-(3,5-Difluorpyridin-2-yl)-7-[4-hydroxy-3-methyl-2-oxopyrrolidin-1-yl]-4-oxo-N-[(2S)-1,1,1-trifluor-propan-2-yl]-1,4-dihydro-1,8-naphthyridin-3-carbonsäureamid (racemisches Diastereomerengemisch)

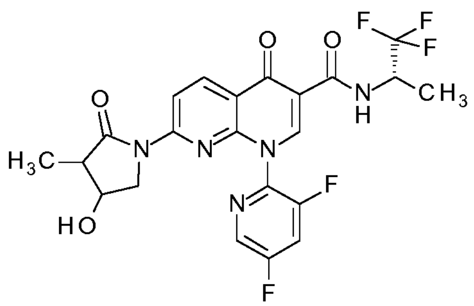

Gemäß allgemeiner Arbeitsvorschrift 2 wurden 50.0 mg (116 µmol) 7-Chlor-1-(3,5-difluorpyridin-2-yl)-4-oxo-*N*-[(2S)-1,1,1-trifluorpropan-2-yl]-1,4-dihydro-1,8-naphthyridin-3-carbonsäureamid (Beispiel 6A) mit 16.0 mg (139 µmol) 4-Hydroxy-3-methylpyrrolidin-2-on (Beispiel 2D) in Gegenwart von 24.0 mg (173 µmol) Kaliumcarbonat, 5.19 mg (23.0 µmol) Palladiumacetat und 13.4 mg (23.0 µmol) Xantphos in 1 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurde aus Acetonitril umkristallisiert und 7.90 mg (13% d. Th., 100% Reinheit) der

Titelverbindung erhalten.
LC-MS (Methode 1): Rt = 0.93 min; MS (ESIpos): m/z = 512 [M+H]+.
$^1$H-NMR (400 MHz, DMSO-d6): δ [ppm] = 10.14-10.20 (m, 1H), 9.03-9.06 (m, 1H), 8.64-8.72 (m, 2H), 8.50-8.58 (m, 1H), 8.36-8.43 (m, 1H), 5.45-5.53 (m, 0.25H), 5.13-5.31 (m, 0.75H), 4.87-4.98 (m, 1H), 4.19 (br. q, 0.75H), 3.88-3.97 (m, 0.25H), 3.74-3.85 (m, 0.25H), 3.47-3.68 (m, 1.50H), 3.21-3.28 (m, 0.25H), 2.76-2.92 (m, 0.75H), 1.40 (br. d, 3H), 1.05-1.18 (m, 3H).

**Beispiel 113**

1-(3,5-Difluorpyridin-2-yl)-7-[7-hydroxy-6-methyl-4-oxo-5-azaspiro[2.4]hept-5-yl]-4-oxo-N-[(2S)-1,1,1-trifluorbutan-2-yl]-1,4-dihydro-1,8-naphthyridin-3-carbonsäureamid (Diastereomerengemisch)

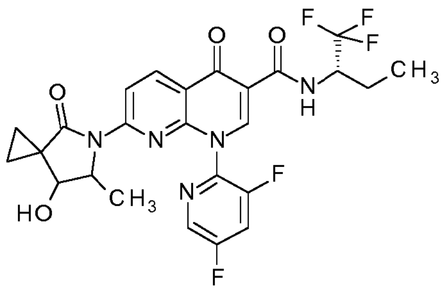

Gemäß allgemeiner Arbeitsvorschrift 2 wurden 100 mg (224 µmol) 7-Chlor-1-(3,5-difluorpyridin-2-yl)-4-oxo-*N*-[(2*S*)-1,1,1-trifluorbutan-2-yl]-1,4-dihydro-1,8-naphthyridin-3-carbonsäureamid (Beispiel 5A) mit 37.9 mg (269 µmol) 7-Hydroxy-6-methyl-5-azaspiro[2.4]heptan-4-on (Beispiel 3E) in Gegenwart von 46.4 mg (336 µmol) Kaliumcarbonat, 10.1 mg (45.0 µmol) Palladiumacetat und 25.9 mg (45.0 µmol) Xantphos in 2 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurde aus Acetonitril umkristallisiert und 57.1 mg (46% d. Th., 100% Reinheit) der Titelverbindung erhalten.
LC-MS (Methode 1): Rt = 1.08 min; MS (ESIpos): m/z = 552 [M+H]+.
$^1$H-NMR (400 MHz, DMSO-d6): δ [ppm] = 10.12 (d, 1H), 9.07 (d, 1H), 8.66-8.71 (m, 2H), 8.40-8.51 (m, 2H), 5.29-5.33 (m, 1H), 4.72-4.83 (m, 1H), 4.31-4.40 (m, 1H), 4.16-4.27 (m, 1H), 1.84-1.95 (m, 1H), 1.61-1.73 (m, 1H), 1.17-1.26 (m, 1H), 1.03-1.17 (m, 3H), 0.94-1.02 (m, 3H), 0.79-0.88 (m, 3H).

**Beispiel 114**

N-[(1R)-1-Cyclopropyl-2,2,2-trifluorethyl]-1-(3,5-difluorpyridin-2-yl)-7-[7-hydroxy-6-methyl-4-oxo-5-azaspiro[2.4]hept-5-yl]-4-oxo-1,4-dihydro-1,8-naphthyridin-3-carbonsäureamid (Diastereomerengemisch)

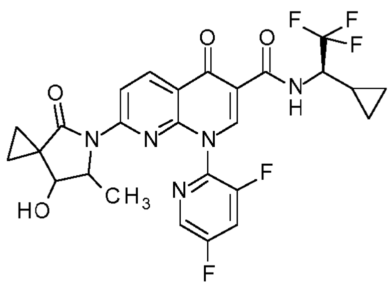

Gemäß allgemeiner Arbeitsvorschrift 2 wurden 100 mg (218 µmol) 7-Chlor-*N*-[(1*R*)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 8A) mit 36.9 mg (262 µmol) 7-Hydroxy-6-methyl-5-azaspiro[2.4]heptan-4-on (Beispiel 3E) in Gegenwart von 45.2 mg (327 µmol) Kaliumcarbonat, 9.79 mg (44.0 µmol) Palladiumacetat und 25.2 mg (44.0 µmol) Xantphos in 2 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurde aus Acetonitril umkristallisiert und 71.9 mg (59% d. Th., 100% Reinheit) der Titelverbindung erhalten.
LC-MS (Methode 2): Rt = 2.08 min; MS (ESIpos): m/z = 564 [M+H]+.
$^1$H-NMR (400 MHz, DMSO-d6): δ [ppm] = 10.26 (d, 1H), 9.07 (d, 1H), 8.66-8.71 (m, 2H), 8.39-8.51 (m, 2H), 5.29-5.34 (m, 1H), 4.31-4.46 (m, 2H), 4.15-4.27 (m, 1H), 1.18-1.28 (m, 2H), 1.02-1.17 (m, 3H), 0.78-0.87 (m, 3H), 0.52-0.70 (m, 3H), 0.30-0.39 (m, 1H).

**Beispiel 115**

N-[(1S)-1-Cyclopropyl-2,2,2-trifluorethyl]-1-(3,5-difluorpyridin-2-yl)-7-[7-hydroxy-6-methyl-4-oxo-5-azaspiro[2.4]hept-5-yl]-4-oxo-1,4-dihydro-1,8-naphthyridin-3-carbonsäureamid (Diastereomerengemisch)

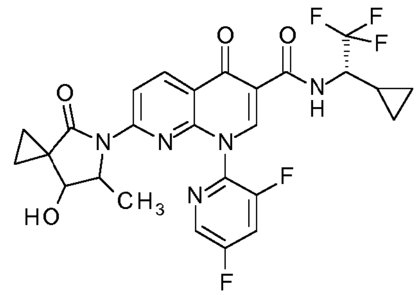

Gemäß allgemeiner Arbeitsvorschrift 2 wurden 100 mg (218 µmol) 7-Chlor-*N*-[(1*S*)-1-cyclopropyl-2,2,2-trifluorethyl]-1-(3,5-difluorpyridin-2-yl)-4-oxo-1,4-dihydro-1,8-naphthyridin-3-carbonsäureamid (Beispiel 9A) mit 36.9 mg (262 µmol) 7-Hydroxy-6-methyl-5-azaspiro[2.4]heptan-4-on (Beispiel 3E) in Gegenwart von 45.2 mg (327 µmol) Kaliumcarbonat, 9.79 mg (44.0 µmol) Palladiumacetat und 25.2 mg (44.0 µmol) Xantphos in 2 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt. Es wurde aus Acetonitril umkristallisiert und 43.9 mg (36% d. Th., 100% Reinheit) der Titelverbindung erhalten.
LC-MS (Methode 2): Rt = 2.08 min; MS (ESIpos): m/z = 564 [M+H]+.
$^1$H-NMR (400 MHz, DMSO-d6): δ [ppm] = 10.26 (d, 1H), 9.07 (d, 1H), 8.65-8.72 (m, 2H), 8.39-8.52 (m, 2H), 5.31 (br s, 1H), 4.31-4.46 (m, 2H), 4.16-4.27 (m, 1H), 1.18-1.28 (m, 2H), 1.05-1.18 (m, 3H), 0.77-0.90 (m, 3H), 0.52-0.70 (m, 3H), 0.31-0.39 (m, 1H).

**Beispiel 116**

1-(3,5-Difluorpyridin-2-yl)-7-[7-hydroxy-6-methyl-4-oxo-5-azaspiro[2.4]hept-5-yl]-4-oxo-N-[(2S)-1,1,1-trifluorpropan-2-yl]-1,4-dihydro-1,8-naphthyridin-3-carbonsäureamid (Diastereomerengemisch)

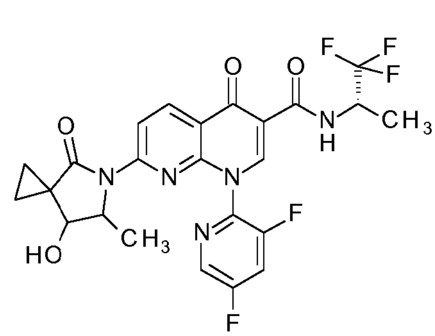

Gemäß allgemeiner Arbeitsvorschrift 2 wurden 100 mg (231 μmol) 7-Chlor-1-(3,5-difluorpyridin-2-yl)-4-oxo-*N*-[(2*S*)-1,1,1-trifluorpropan-2-yl]-1,4-dihydro-1,8-naphthyridin-3-carbonsäureamid (Beispiel 6A) mit 39.1 mg (277 μmol) 7-Hydroxy-6-methyl-5-azaspiro[2.4]heptan-4-on (Beispiel 3E) in Gegenwart von 47.9 mg (347 μmol) Kaliumcarbonat, 10.4 mg (46.0 μmol) Palladiumacetat und 26.7 mg (46.0 μmol) Xantphos in 2 ml Dioxan bei 80°C zur Reaktion gebracht. Die Reaktionslösung wurde anschließend mit wenig Acetonitril, Wasser und Ameisensäure versetzt, durch einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt. Es wurde aus Acetonitril umkristallisiert und 80.5 mg (65% d. Th., 100% Reinheit) der Titelverbindung erhalten.
LC-MS (Methode 1): Rt = 1.02 min; MS (ESIpos): m/z = 538 [M+H]+.
$^1$H-NMR (400 MHz, DMSO-d6): δ [ppm] = 10.17 (d, 1H), 9.07 (d, 1H), 8.63-8.72 (m, 2H), 8.39-8.51 (m, 2H), 5.29-5.33 (m, 1H), 4.87-4.98 (m, 1H), 4.31-4.40 (m, 1H), 4.15-4.27 (m, 1H), 1.40 (d, 3H), 1.18-1.26 (m, 1H), 1.03-1.18 (m, 3H), 0.76-0.88 (m, 3H).

## Beispiel 117

1-(3,5-Difluorpyridin-2-yl)-7-[3-hydroxypiperidin-1-yl]-4-oxo-N-[(2R)-1,1,1-trifluorbutan-2-yl]-1,4-dihydro-1,8-naphthyridin-3-carbonsäureamid (Diastereomerengemisch)

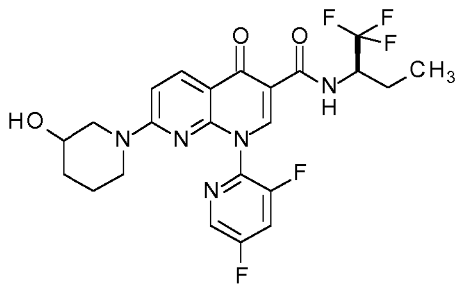

Gemäß allgemeiner Arbeitsvorschrift 3 wurden 25.0 mg (56.0 μmol) 7-Chlor-1-(3,5-difluorpyridin-2-yl)-4-oxo-*N*-[(2*R*)-1,1,1-trifluorbutan-2-yl]-1,4-dihydro-1,8-naphthyridin-3-carbonsäureamid (Beispiel 4C) mit 6.79 mg (67.1 μmol) Piperidin-3-ol (Racemat) in Gegenwart von 34.0 μl (196 μmol) DIPEA in 0.28 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 25.5 mg (89% d. Th., 100% Reinheit) der Titelverbindung erhalten.
LC-MS (Methode 2): Rt = 1.85 min; MS (ESIpos): m/z = 512 [M+H]+.
$^1$H-NMR (500 MHz, DMSO-d6): δ [ppm] = 10.39 (d, 1H), 8.81 (br s, 1H), 8.62 (br d, 1H), 8.28-8.36 (m, 1H), 8.25 (dd, 1H), 7.10 (d, 1H), 4.81-4.86 (m, 1H), 4.69-4.79 (m, 1H), 3.58-3.90 (m, 2H), 3.38-3.53 (m, 1H), 2.89-3.25 (m, 2H), 1.79-1.92 (m, 2H), 1.59-1.70 (m, 2H), 1.26-1.46 (m, 2H), 0.96 (br dd, 3H).

## Beispiel 118

1-(3,5-Difluorpyridin-4-yl)-7-[(3R,4R)-3,4-dihydroxypyrrolidin-1-yl]-4-oxo-N-[(2S)-1,1,1-trifluorbutan-2-yl]-1,4-dihydro-1,8-naphthyridin-3-carbonsäureamid

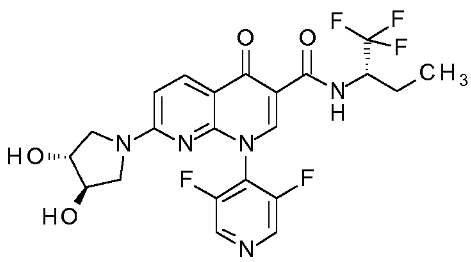

Gemäß allgemeiner Arbeitsvorschrift 3 wurden 40.0 mg (89.5 µmol) 7-Chlor-1-(3,5-difluorpyridin-4-yl)-4-oxo-N-[(2S)-1,1,1-trifluorbutan-2-yl]-1,4-dihydro-1,8-naphthyridin-3-carbonsäureamid (Beispiel 22C) mit 15.0 mg (107 µmol) (3R,4R)-Pyrrolidin-3,4-diol-Hydrochlorid in Gegenwart von 55.0 µl (313 µmol) DIPEA in 3 ml DMF zur Reaktion gebracht. Das Reaktionsgemisch wurde mit Wasser und Acetonitril verdünnt, filtriert und mittels präparativer HPLC (Wasser / Acetonitril-Gradient 0.1% Trifluressigsäure-Gradient) gereinigt und 37.8 mg (82% d. Th., 100% Reinheit) der Titelverbindung erhalten.
LC-MS (Methode 2): Rt = 1.53 min; MS (ESIpos): m/z = 514 [M+H]+.
$^1$H-NMR (400 MHz, DMSO-d6): δ [ppm] = 10.39 (d, 1H), 8.91 (s, 1H), 8.86 (d, 2H), 8.28 (d, 1H), 6.80 (d, 1H), 4.64-4.85 (m, 1H), 3.99-4.11 (m, 1H), 3.88-3.95 (m, 1H), 3.60-3.64 (m, 3H), 3.30-3.46 (m, 1H), 3.16-3.30 (m, 1H), 3.03-3.06 (m, 1H), 1.83-1.93 (m, 1H), 1.59-1.70 (m, 1H), 0.97 (t, 3H).

**Beispiel 119**

1-(3,5-Difluorpyridin-2-yl)-7-[3-(2-hydroxyethyl)-2-oxoimidazolidin-1-yl]-4-oxo-N-[(2S)-1,1,1-trifluorbutan-2-yl]-1,4-dihydro-1,8-naphthyridin-3-carbonsäureamid

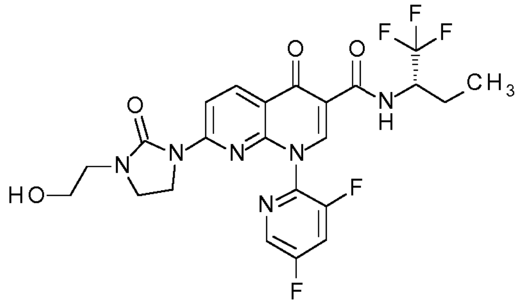

Gemäß allgemeiner Arbeitsvorschrift 1 wurden 40.0 mg (92.7 µmol) 1-(3,5-Difluorpyridin-2-yl)-7-[3-(2-hydroxyethyl)-2-oxoimidazolidin-1-yl]-4-oxo-1,4-dihydro-1,8-naphthyridin-3-carbonsäure (Beispiel 25A) mit 18.2 mg (111 µmol) (2*S*)-1,1,1-Trifluorbutan-2-amin-Hydrochlorid in Gegenwart von 42.3 mg (111 µmol) HATU und 65.0 µl (371 µmol) DIPEA in 0.6 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 36.8 mg (73% d. Th., 100% Reinheit) der Titelverbindung erhalten.
LC-MS (Methode 2): Rt = 1.73 min; MS (ESIpos): m/z = 541 [M+H]+.
$^1$H-NMR (400 MHz, DMSO-d6): δ [ppm] = 10.21 (d, 1H), 8.99 (s, 1H), 8.64 (br. d, 1H), 8.57 (d, 1H), 8.44 (d, 1H), 8.33 (ddd, 1H), 4.71-4.83 (m, 2H), 3.43-3.63 (m, 6H), 3.21-3.30 (m, 2H), 1.84-1.95 (m, 1H), 1.60-1.72 (m, 1H), 0.94-1.01 (m, 3H).

**Beispiel 120**

1-(3,5-Difluorpyridin-2-yl)-7-[3-(2-hydroxyethyl)-2-oxotetrahydropyrimidin-1(2H)-yl]-4-oxo-N-[(2S)-1,1,1-trifluorbutan-2-yl]-1,4-dihydro-1,8-naphthyridin-3-carbonsäureamid

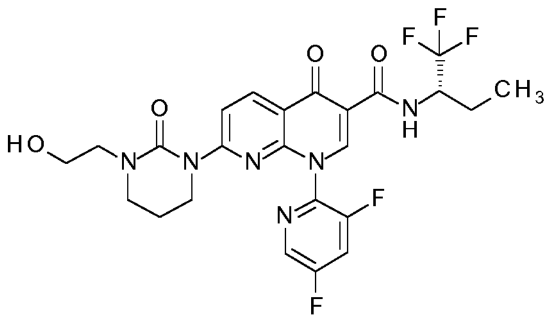

Gemäß allgemeiner Arbeitsvorschrift 1 wurden 40.0 mg (89.8 µmol, 86% Reinheit) 1-(3,5-Difluorpyridin-2-yl)-7-[3-(2-hydroxyethyl)-2-oxotetrahydropyrimidin-1(2H)-yl]-4-oxo-1,4-dihydro-1,8-naphthyridin-3-carbonsäure (Beispiel 26B) mit 17.6 mg (108 µmol) (2*S*)-1,1,1-Trifluorbutan-2-amin-Hydrochlorid in Gegenwart von 41.0 mg (108 µmol) HATU und 63.0 µl (359 µmol) DIPEA in 0.6 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 37.1 mg (75% d. Th., 100% Reinheit) der Titelverbindung erhalten.
LC-MS (Methode 1): Rt = 0.98 min; MS (ESIpos): m/z = 555 [M+H]+.
$^1$H-NMR (400 MHz, DMSO-d6): δ [ppm] = 10.18 (d, 1H), 9.01 (s, 1H), 8.65 (d, 1H), 8.50 (d, 1H), 8.36 (ddd, 1H), 8.17 (d, 1H), 4.73-4.82 (m, 1H), 4.71 (t, 1H), 3.48-3.60 (m, 4H), 3.35-3.42 (m, 4H), 1.85-1.96 (m, 3H), 1.60-1.72 (m, 1H), 0.94-1.02 (m, 3H).

## Beispiel 121

N-[(1R)-1-Cyclopropyl-2,2,2-trifluorethyl]-1-(3,5-difluorpyridin-2-yl)-7-[3-(2-hydroxyethyl)-2-oxoimidazolidin-1-yl]-4-oxo-1,4-dihydro-1,8-naphthyridin-3-carbonsäureamid

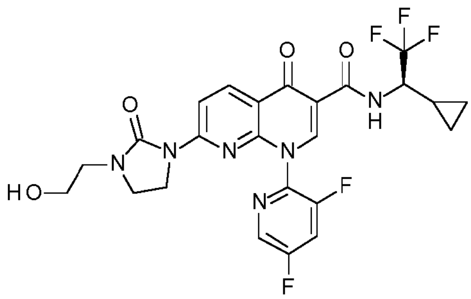

Gemäß allgemeiner Arbeitsvorschrift 1 wurden 40.0 mg (92.7 µmol) 1-(3,5-Difluorpyridin-2-yl)-7-[3-(2-hydroxyethyl)-2-oxoimidazolidin-1-yl]-4-oxo-1,4-dihydro-1,8-naphthyridin-3-carbonsäure (Beispiel 25A) mit 19.5 mg (111 µmol) (1*R*)-1-Cyclopropyl-2,2,2-trifluorethanamin-Hydrochlorid in Gegenwart von 42.3 mg (111 µmol) HATU und 65.0 µl (371 µmol) DIPEA in 0.6 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 32.3 mg (63% d. Th., 100% Reinheit) der Titelverbindung erhalten.
LC-MS (Methode 2): Rt = 1.77 min; MS (ESIpos): m/z = 553 [M+H]+.
$^1$H-NMR (400 MHz, DMSO-d6): δ [ppm] = 10.34 (br d, 1H), 8.98 (s, 1H), 8.64 (d, 1H), 8.57 (d, 1H), 8.45 (d, 1H), 8.33 (ddd, 1H), 4.75 (t, 1H), 4.37-4.47 (m, 1H), 3.44-3.62 (m, 6H), 3.21-3.30 (m, 2H), 1.19-1.27 (m, 1H), 0.51-0.70 (m, 3H), 0.30-0.39 (m, 1H).

**Beispiel 122**

N-[(1R)-1-Cyclopropyl-2,2,2-trifluorethyl]-1-(3,5-difluorpyridin-2-yl)-7-[3-(2-hydroxyethyl)-2-oxotetrahydropyrimidin-1(2H)-yl]-4-oxo-1,4-dihydro-1,8-naphthyridin-3-carbonsäureamid

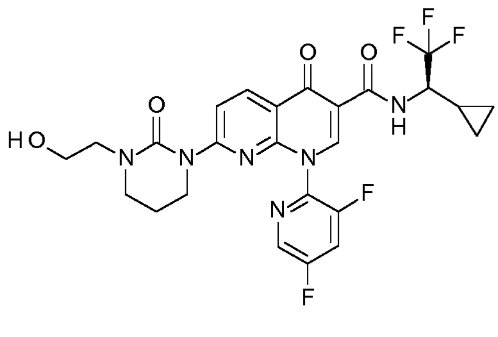

Gemäß allgemeiner Arbeitsvorschrift 1 wurden 40.0 mg (89.8 μmol, 86% Reinheit) 1-(3,5-Difluorpyridin-2-yl)-7-[3-(2-hydroxyethyl)-2-oxotetrahydropyrimidin-1(2*H*)-yl]-4-oxo-1,4-dihydro-1,8-naphthyridin-3-carbonsäure (Beispiel 26B) mit 18.9 mg (108 μmol) (1*R*)-1-Cyclopropyl-2,2,2-trifluorethanamin-Hydrochlorid in Gegenwart von 41.0 mg (108 μmol) HATU und 63.0 μl (359 μmol) DIPEA in 0.6 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 37.3 mg (73% d. Th., 100% Reinheit) der Titelverbindung erhalten.
LC-MS (Methode 1): Rt = 1.00 min; MS (ESIpos): m/z = 567 [M+H]+.
$^1$H-NMR (400 MHz, DMSO-d6): δ [ppm] = 10.33 (d, 1H), 9.00 (s, 1H), 8.65 (d, 1H), 8.51 (d, 1H), 8.36 (ddd, 1H), 8.17 (d, 1H), 4.71 (t, 1H), 4.36-4.47 (m, 1H), 3.48-3.58 (m, 4H), 3.36-3.42 (m, 4H), 1.87-1.96 (m, 2H), 1.18-1.28 (m, 1H), 0.51-0.70 (m, 3H), 0.31-0.39 (m, 1H).

**Beispiel 123**

N-[(1S)-1-Cyclopropyl-2,2,2-trifluorethyl]-1-(3,5-difluorpyridin-2-yl)-7-[3-(2-hydroxyethyl)-2-oxotetrahydropyrimidin-1(2H)-yl]-4-oxo-1,4-dihydro-1,8-naphthyridin-3-carbonsäureamid

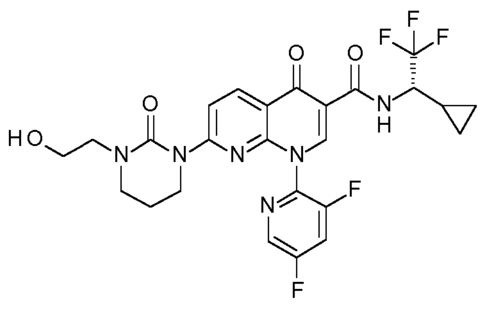

Gemäß allgemeiner Arbeitsvorschrift 1 wurden 30.0 mg (67.4 μmol, 86% Reinheit) 1-(3,5-Difluorpyridin-2-yl)-7-[3-(2-hydroxyethyl)-2-oxotetrahydropyrimidin-1(2*H*)-yl]-4-oxo-1,4-dihydro-1,8-naphthyridin-3-carbonsäure (Beispiel 26B) mit 14.2mg (81.0 μmol) (1*S*)-1-Cyclopropyl-2,2,2-trifluorethanamin-Hydrochlorid in Gegenwart von 30.7 mg (81.0 μmol) HATU und 47.0 μl (269 μmol) DIPEA in 0.5 ml DMF zur Reaktion gebracht. Das Reaktionsgemisch wurde mit Wasser, Acetonitril und Ameisensäure verdünnt, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 26.5 mg (69% d. Th., 100% Reinheit) der Titelverbindung erhalten.
LC-MS (Methode 1): Rt = 1.00 min; MS (ESIpos): m/z = 567 [M+H]+.
$^1$H-NMR (400 MHz, DMSO-d6): δ [ppm] = 10.33 (d, 1H), 9.00 (s, 1H), 8.65 (d, 1H), 8.51 (d, 1H), 8.36 (ddd, 1H), 8.17 (d, 1H), 4.71 (t, 1H), 4.35-4.50 (m, 1H), 3.47-3.60 (m, 4H), 3.35-3.42 (m, 4H), 1.86-1.96 (m, 2H), 1.18-1.28 (m, 1H),

0.52-0.70 (m, 3H), 0.30-0.39 (m, 1H).

**Beispiel 124**

1-(3,5-Difluorpyridin-2-yl)-7-[3-(2-hydroxyethyl)-2-oxoimidazolidin-1-yl]-4-oxo-N-[(2S)-1,1,1-trifluor-3,3-dimethylbutan-2-yl]-1,4-dihydro-1,8-naphthyridin-3-carbonsäureamid

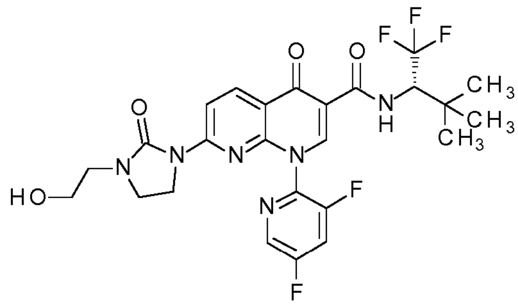

Gemäß allgemeiner Arbeitsvorschrift 2 wurden 60.0 mg (126 μmol) 7-Chlor-1-(3,5-difluorpyridin-2-yl)-4-oxo-*N*-[(2*S*)-1,1,1-trifluor-3,3-dimethylbutan-2-yl]-1,4-dihydro-1,8-naphthyridin-3-carbonsäureamid (Beispiel 13A) mit 37.1 mg (152 μmol) 1-(2-{[*tert*.-Butyl(dimethyl)silyl]oxy}ethyl)imidazolidin-2-on (EP 1721905 A1, Ex. 43) in Gegenwart von 43.7 mg (316 μmol) Kaliumcarbonat, 5.67 mg (25.0 μmol) Palladiumacetat und 14.6 mg (25.0 μmol) Xantphos in 1.6 ml Dioxan für 90 min bei 90°C zur Reaktion gebracht. Das Rohprodukt wurde mittels präparativer HPLC teilweise aufgereinigt. Anschließend wurde das Rohprodukt in 5 ml Dioxan aufgenommen und mit 5 ml IN wässriger Salzsäure versetzt und 1h bei 40°C nachgerührt. Es wurden alle flüchtigen Komponenten unter reduziertem Druck entfernt und der Rückstand in etwas DMSO, Wasser, Acetonitril und Ameisensäure gelöst, über einen Milliporefilter filtriert und mittels präparativer HPLC (Säule: Chromatorex C18, 10 μm, 125*40 mm, Lösungsmittel: Acetonitril, Wasser, 0.1% Ameisensäure) gereinigt und 17.4 mg (24% d. Th., 100% Reinheit) der Titelverbindung erhalten.
LC-MS (Methode 2): Rt = 1.93 min; MS (ESIpos): m/z = 569 [M+H]+.
$^1$H-NMR (400 MHz, DMSO-d6): δ [ppm] = 10.57 (br. dd, 1H), 9.00 (s, 1H), 8.64 (br. d, 1H), 8.60 (d, 1H), 8.45 (d, 1H), 8.29-8.37 (m, 1H), 4.75 (t, 1H), 4.65 (quint, 1H), 3.40-3.70 (m, 7H), 3.21-3.28 (m, 1H), 1.10 (br. s, 9H).

**Beispiel 125**

1-(3,5-Difluorpyridin-2-yl)-7-[3-(2-hydroxyethyl)-2-oxotetrahydropyrimidin-1(2H)-yl]-4-oxo-N-[(2S)-1,1,1-trifluor-3,3-dimethylbutan-2-yl]-1,4-dihydro-1,8-naphthyridin-3-carbonsäureamid

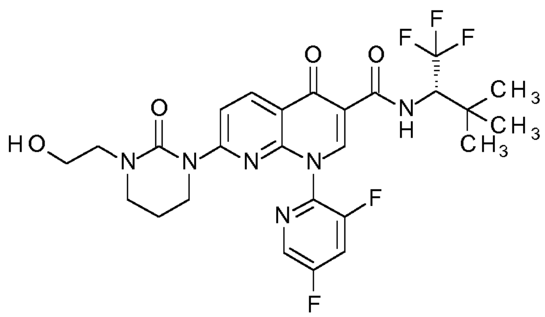

Gemäß allgemeiner Arbeitsvorschrift 2 wurden 60.0 mg (126 μmol) 7-Chlor-1-(3,5-difluorpyridin-2-yl)-4-oxo-*N*-[(2*S*)-1,1,1-trifluor-3,3-dimethylbutan-2-yl]-1,4-dihydro-1,8-naphthyridin-3-carbonsäureamid (Beispiel 13A) mit 39.2 mg (152 μmol) 1-(2-{[*tert*.-Butyl(dimethyl)silyl]oxy}ethyl)tetrahydropyrimidin-2(1*H*)-on (Beispiel 26A) in Gegenwart von 43.7 mg (316 μmol) Kaliumcarbonat, 5.67 mg (25.0 μmol) Palladiumacetat und 14.6 mg (25.0 μmol) Xantphos in 1.6 ml Dioxan für 90 min bei 90°C zur Reaktion gebracht. Das Rohprodukt wurde mittels präparativer HPLC teilweise aufgereinigt. Anschließend wurde das Rohprodukt in 5 ml Dioxan aufgenommen und mit 5 ml 1N wässriger Salzsäure

versetzt und 1h bei 40°C nachgerührt. Es wurden alle flüchtigen Komponenten unter reduziertem Druck entfernt und der Rückstand in etwas DMSO, Wasser, Acetonitril und Ameisensäure gelöst, über einen Milliporefilter filtriert und mittels präparativer HPLC (Säule: Chromatorex C18, 10 µm, 125*40 mm, Lösungsmittel: Acetonitril, Wasser, 0.1% Ameisensäure) gereinigt und 37.7 mg (51% d. Th., 100% Reinheit) der Titelverbindung erhalten.
LC-MS (Methode 2): $R_t$ = 1.93 min; MS (ESIpos): m/z = 583 [M+H]+.
$^1$H-NMR (400 MHz, DMSO-d6): δ [ppm] = 10.54 (br. d, 1H), 9.02 (s, 1H), 8.62-8.67 (m, 1H), 8.53 (d, 1H), 8.32-8.40 (m, 1H), 8.18 (d, 1H), 4.71 (t, 1H), 4.60-4.69 (m, 1H), 3.48-3.60 (m, 4H), 3.36-3.42 (m, 4H), 1.86-1.97 (m, 2H), 1.10 (s, 9H).

**Beispiel 126**

1-(3,5-Difluorpyridin-2-yl)-7-[3-(2-hydroxyethyl)-2-oxoimidazolidin-1-yl]-4-oxo-N-[(2R)-1,1,1-trifluor-3,3-dimethylbutan-2-yl]-1,4-dihydro-1,8-naphthyridin-3-carbonsäureamid

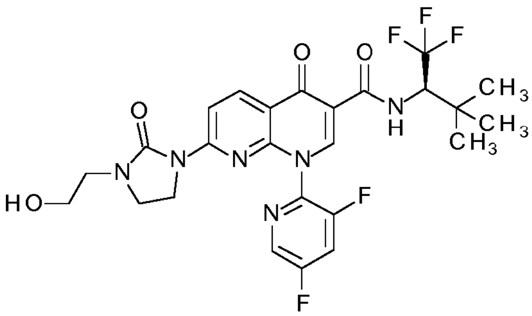

Gemäß allgemeiner Arbeitsvorschrift 1 wurden 40.0 mg (92.7 µmol) 1-(3,5-Difluorpyridin-2-yl)-7-[3-(2-hydroxyethyl)-2-oxoimidazolidin-1-yl]-4-oxo-1,4-dihydro-1,8-naphthyridin-3-carbonsäure (Beispiel 25A) mit 17.3 mg (111 µmol) (2*R*)-1,1,1-Trifluor-3,3-dimethylbutan-2-amin in Gegenwart von 42.3 mg (111 µmol) HATU und 48.0 µl (278 µmol) DIPEA in 0.6 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 µm, 125*40 mm, Lösungsmittel: Acetonitril, Wasser, 0.1% Ameisensäure) gereinigt und 37.3 mg (71% d. Th., 100% Reinheit) der Titelverbindung erhalten.
LC-MS (Methode 2): Rt = 1.93 min; MS (ESIpos): m/z = 569 [M+H]+.
$^1$HNMR (400 MHz, DMSO-d6): δ [ppm] = 10.57 (br dd, 1H), 9.00 (s, 1H), 8.64 (br d, 1H), 8.60 (d, 1H), 8.45 (d, 1H), 8.29-8.36 (m, 1H), 4.75 (t, 1H), 4.65 (quintt, 1H), 3.44-3.63 (m, 6H), 3.22-3.30 (m, 2H), 1.10 (s, 9H).

**Beispiel 127**

1-(3,5-Difluorpyridin-2-yl)-7-[3-(2-hydroxyethyl)-2-oxotetrahydropyrimidin-1(2H)-yl]-4-oxo-N-[(2R)-1,1,1-trifluor-3,3-dimethylbutan-2-yl]-1,4-dihydro-1,8-naphthyridin-3-carbonsäureamid

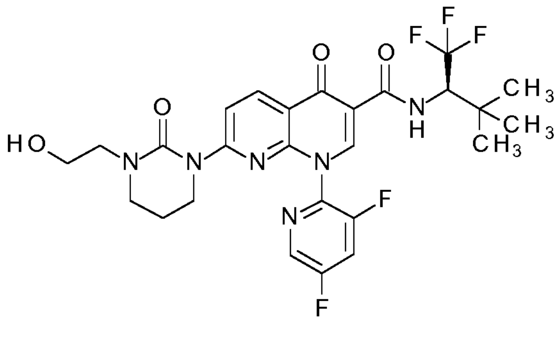

Gemäß allgemeiner Arbeitsvorschrift 1 wurden 40.0 mg (89.8 µmol, 86% Reinheit) 1-(3,5-Difluorpyridin-2-yl)-7-[3-(2-hydroxyethyl)-2-oxotetrahydropyrimidin-1(2*H*)-yl]-4-oxo-1,4-dihydro-1,8-naphthyridin-3-carbonsäure (Beispiel

26B) mit 16.7 mg (108 μmol) (2*R*)-1,1,1-Trifluor-3,3-dimethylbutan-2-amin in Gegenwart von 41.0 mg (108 μmol) HATU und 47.0 μl (269 μmol) DIPEA in 0.6 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Säule: Chromatorex C18, 10 μm, 125*40 mm, Lösungsmittel: Acetonitril, Wasser, 0.1% Ameisensäure) gereinigt und 36.0 mg (66% d. Th., 96% Reinheit) der Titelverbindung erhalten.
LC-MS (Methode 1): Rt = 1.08 min; MS (ESIpos): m/z = 583 [M+H]+.
$^{1}$H-NMR (400 MHz, DMSO-d6): δ [ppm] = 10.55 (br. d, 1H), 9.02 (s, 1H), 8.65 (d, 1H), 8.53 (d, 1H), 8.36 (ddd, 1H), 8.18 (d, 1H), 4.71 (t, 1H), 4.61-4.69 (m, 1H), 3.48-3.60 (m, 4H), 3.35-3.43 (m, 4H), 1.86-1.97 (m, 2H), 1.10 (s, 9H).

## Beispiel 128

1-(3,5-Difluorpyridin-4-yl)-4-oxo-7-(2-oxoimidazolidin-1-yl)-N-[(2S)-1,1,1-trifluorbutan-2-yl]-1,4-dihydro-1,8-naphthyridin-3-carbonsäureamid

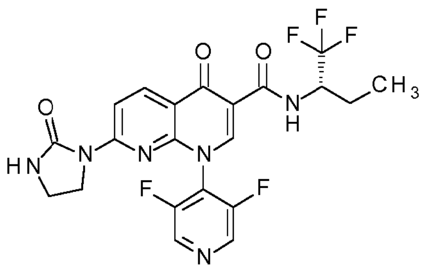

Unter Argon, wurden 18.6 mg (134 μmol) Kaliumcarbonat, 1.00 mg (4 μmol) Palladiumacetat und 5.18 mg (8.95 μmol) 9,9-Dimethyl-4,5-bis-(diphenylphosphino)-xanthen in 3 ml entgastem Dioxan vorgelegt. Der Ansatz wurde 10 min bei RT gerührt und dann 40.0 mg (89.5 μmol) 7-Chlor-1-(3,5-difluorpyridin-4-yl)-4-oxo-*N*-[(2*S*)-1,1,1-trifluorbutan-2-yl]-1,4-dihydro-1,8-naphthyridin-3-carbonsäureamid (Beispiel 22C) und 77.1 mg (895 μmol) Imidazolidin-2-on zugegeben. Es wurde 4h bei 80°C gerührt, dann mit Wasser und Acetonitril versetzt, filtriert und direkt mittels präparative RP-HPLC gereinigt (Säule: Reprosil 125x30; 10μ, Fluss: 50 mL/min, MeCN/Wasser; 0.1 % TFA). Die Lösemittel wurden im Vakuum verdampft und der Rückstand im Hochvakuum getrocknet. Es wurden 21.3 mg (48% d. Th., 100 % Reinheit) der Titelverbindung erhalten.
LC-MS (Methode 2): $R_t$ = 1.75 min; MS (ESIpos): m/z = 497 [M+H]+
$^{1}$H NMR (400 MHz, DMSO-$d_6$) δ [ppm] = 10.16 (d, 1H) 9.08 (s, 1H) 8.88 (s, 2H) 8.57 (d, 1H) 8.45 (d, 1H) 7.68 (s, 1H) 4.71-4.82 (m, 1H) 3.52-3.63 (m, 2H) 3.33-3.42 (m, 2H) 1.84-1.95 (m, 1H) 1.61-1.73 (m, 1H) 0.98 (t, 3H).

## Beispiel 129

1-(3,5-Difluorpyridin-2-yl)-7-[(3S)-3-fluorpyrrolidin-1-yl]-4-oxo-N-[(2S)-1,1,1-trifluorbutan-2-yl]-1,4-dihydro-1,8-naphthyridin-3-carbonsäureamid

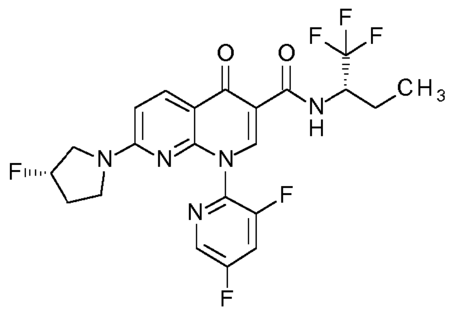

Gemäß allgemeiner Arbeitsvorschrift 3 wurden 25.0 mg (56.0 μmol) 7-Chlor-1-(3,5-difluorpyridin-2-yl)-4-oxo-*N*-[[2*S*)-1,1,1-trifluorbutan-2-yl]-1,4-dihydro-1,8-naphthyridin-3-carbonsäureamid (Beispiel 5A) mit 7.73 mg (62.0 μmol)

(3*S*)-3-Fluorpyrrolidin-Hydrochlorid in Gegenwart von 34 μl (0.20 mmol) DIPEA in 0.5 ml DMF über Nacht zur Reaktion gebracht. Anschließend wurde mit 1M wässriger Salzsäure auf pH 1 gestellt, mit 1 ml Acetonitril verdünnt und die Rohlösung mittels präparativer HPLC [Säule: Chromatorex C18,10 μm, 125*30 mm, LM: Acetonitril / 0.05% Ameisensäure -Gradient; (0 bis 2.5 min. 10% Acetonitril bis 23 min. 90% Acetonitril und weitere 2 min 90% Acetonitril)] gereinigt. Es wurden 21.2 mg (75% d. Th., 99% Reinheit) der Titelverbindung erhalten.
LC-MS (Methode 2): $R_t$ = 2.09 min; MS (ESIpos): m/z = 500 [M+H]+.
$^1$H NMR (400 MHz, DMSO-d6): δ [ppm] = 10.41 (br. s, 1H), 8.84 (s, 1H), 8.62 (s, 1H), 8.32 (br. s, 2H), 6.83 (br. s, 1H), 5.61-5.19 (m, 1H), 4.83-4.65 (m, 1H), 3.91-2.00 (m, 6H, zum Teil unter Lösungsmittel Signalen), 1.91-1.85 (m, 1H), 1.73-1.54 (m, 1H), 0.96 (br. s, 3H).

**Beispiel 130**

1-(3,5-Difluorpyridin-2-yl)-7-(dimethylamino)-4-oxo-N-[(2S)-1,1,1-trifluorbutan-2-yl]-1,4-dihydro-1,8-naphthyridin-3 -carbonsäureamid

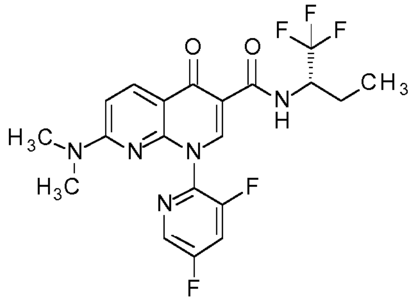

Gemäß allgemeiner Arbeitsvorschrift 3 wurden 25.0 mg (56.0 μmol) 7-Chlor-1-(3,5-difluorpyridin-2-yl)-4-oxo-*N*-[(2*S*)-1,1,1-trifluorbutan-2-yl]-1,4-dihydro-1,8-naphthyridin-3-carbonsäureamid (Beispiel 5A) mit 31 μl (62 μmol) Dimethylamin (2M in THF) in Gegenwart von 34 μl (0.20 mmol) DIPEA in 0.5 ml DMF über Nacht zur Reaktion gebracht. Anschließend wurde mit 1M wässriger Salzsäure auf pH 1 gestellt, mit 2 ml Acetonitril und 1 ml Wasser versetzt, zentrifugiert, dekantiert und der Niederschlag mit 3 ml Diethylether verrührt. Anschließend wurde der verbliebene Niederschlag am Hochvakuum getrocknet, mittels Normalphasenchromatographie (Essigsäureethylester-Cyclohexan Gradient) gereinigt und 10.3 mg (40% d. Th., 99% Reinheit) der Titelverbindung erhalten.
LC-MS (Methode 2): Rt = 2.08 min; MS (ESIpos): m/z = 456 [M+H]+.
$^1$HNMR (400 MHz, DMSO-d6): δ [ppm] = 10.42 (d, 1H), 8.82 (s, 1H), 8.63-8.61 (m, 1H), 8.36-8.27 (m, 2H), 6.96 (d, 1H), 4.81-4.68 (m, 1H), 2.96 (br. s, 6H), 1.94-1.82 (m, 1H), 1.71-1.57 (m, 1H), 1.00-0.93 (m, 3H).

**Beispiel 131**

1-(3,5-Difluorpyridin-2-yl)-7-[3-hydroxy-3-(trifluormethyl)azetidin-1-yl]-4-oxo-N-[(2S)-1,1,1-trifluorbutan-2-yl]-1,4-dihydro-1,8-naphthyridin-3-carbonsäureamid

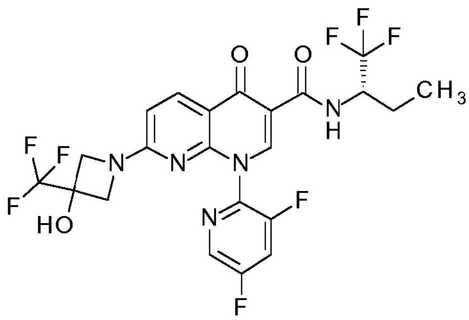

Gemäß allgemeiner Arbeitsvorschrift 3 wurden 25.0 mg (56 µmol) 7-Chlor-1-(3,5-difluorpyridin-2-yl)-4-oxo-*N*-[(2*S*)-1,1,1-trifluorbutan-2-yl]-1,4-dihydro-1,8-naphthyridin-3-carbonsäureamid (Beispiel 5A) mit 10.9 mg (62.0 µmol) 3-(Trifluormethyl)azetidin-3-ol-Hydrochlorid in Gegenwart von 34 µl (0.20 mmol) DIPEA in 0.5 ml DMF über Nacht zur Reaktion gebracht. Anschließend wurde mit 1M wässriger Salzsäure auf pH 1 gestellt, mit 1 ml Acetonitril verdünnt und die Rohlösung mittels präparativer HPLC [Säule: Chromatorex C18,10 µm, 125*30 mm, LM: Acetonitril / 0.05% Ameisensäure-Gradient; (0 bis 2.5 min. 10% Acetonitril bis 23 min. 90% Acetonitril und weitere 2 min 90% Acetonitril)] gereinigt. Es wurden 26.4 mg (85% d. Th., 99% Reinheit) der Titelverbindung erhalten.
LC-MS (Methode 1): Rt = 1.10 min; MS (ESIpos): m/z = 552 [M+H]+.
$^1$HNMR (400 MHz, DMSO-d6): δ [ppm] = 10.32 (br. d, 1H), 8.87 (s, 1H), 8.61 (s, 1H), 8.44-8.23 (m, 2H), 7.47 (s, 1H), 6.76 (d, 1H), 4.83-4.68 (m, 1H), 4.43-3.71 (br. m, 4H), 1.95-1.79 (m, 1H), 1.72-1.56 (m, 1H), 0.96 (br. s, 3H).

**Beispiel 132**

1-(3,5-Difluorpyridin-2-yl)-7-(3-fluorazetidin-1-yl)-4-oxo-N-[(2S)-1,1,1-trifluorbutan-2-yl]-1,4-dihydro-1,8-naphthyridin-3-carbonsäureamid

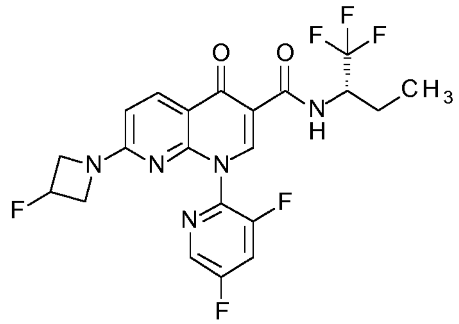

Gemäß allgemeiner Arbeitsvorschrift 3 wurden 25.0 mg (56.0 µmol) 7-Chlor-1-(3,5-difluorpyridin-2-yl)-4-oxo-*N*-[(2*S*)-1,1,1-trifluorbutan-2-yl]-1,4-dihydro-1,8-naphthyridin-3-carbonsäureamid (Beispiel 5A) mit 6.87 mg (62.0 µmol) 3-Fluorazetidin-Hydrochlorid in Gegenwart von 34 µl (0.20 mmol) DIPEA in 0.5 ml DMF über Nacht zur Reaktion gebracht. Anschließend wurde mit 1M wässriger Salzsäure auf pH 1 gestellt, mit 0.5 ml Acetonitril verdünnt und die Rohlösung mittels präparativer HPLC [Säule: Chromatorex C18,10 µm, 125*30 mm, LM: Acetonitril / 0.05% Ameisensäure -Gradient; (0 bis 2.5 min. 10% Acetonitril bis 23 min. 90% Acetonitril und weitere 2 min 90% Acetonitril)] gereinigt. Es wurden 24.3 mg (89% d. Th., 100% Reinheit) der Titelverbindung erhalten.
LC-MS (Methode 1): Rt = 1.09 min; MS (ESIpos): m/z = 486 [M+H]+.
$^1$HNMR (400 MHz, DMSO-d6): δ [ppm] = 10.35 (d, 1H), 8.85 (s, 1H), 8.62-8.59 (m, 1H), 8.35-8.27 (m, 2H), 6.68 (d, 1H), 5.55-5.37 (m, 1H), 4.80-4.69 (m, 1H), 4.43-3.85 (br. m, 4H), 1.93-1.83 (m, 1H), 1.69-1.59 (m, 1H), 1.00-0.92 (m, 3H).

**Beispiel 133**

1-(3,5-Difluorpyridin-2-yl)-7-(3-hydroxyazetidin-1-yl)-4-oxo-N-[(2S)-1,1,1-trifluorbutan-2-yl]-1,4-dihydro-1,8-naphthyridin-3-carbonsäureamid

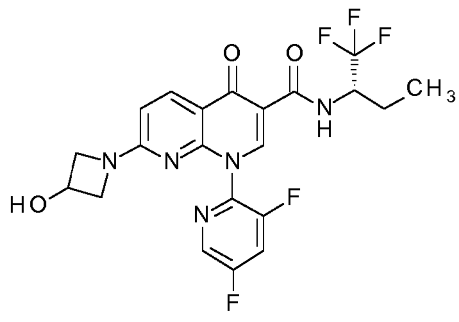

Gemäß allgemeiner Arbeitsvorschrift 3 wurden 25.0 mg (56.0 μmol) 7-Chlor-1-(3,5-difluorpyridin-2-yl)-4-oxo-*N*-[(2*S*)-1,1,1-trifluorbutan-2-yl]-1,4-dihydro-1,8-naphthyridin-3-carbonsäureamid (Beispiel 5A) mit 6.74 mg (62.0 μmol) Azetidin-3-ol-Hydrochlorid in Gegenwart von 34 μl (0.20 mmol) DIPEA in 0.5 ml DMF über Nacht zur Reaktion gebracht. Anschließend wurde mit 1M wässriger Salzsäure auf pH 1 gestellt, mit 0.5 ml Acetonitril verdünnt und die Rohlösung mittels präparativer HPLC [Säule: Chromatorex C18, 10 μm, 125*30 mm, LM: Acetonitril / 0.05% Ameisensäure-Gradient; (0 bis 3 min 10% Acetonitril bis 35 min 90% Acetonitril und weitere 3 min 90% Acetonitril)] gereinigt. Es wurden 21.2 mg (78% d. Th., 99% Reinheit) der Titelverbindung erhalten.
LC-MS (Methode 2): Rt = 1.75 min; MS (ESIpos): m/z = 484 [M+H]+.
$^1$HNMR (400 MHz, DMSO-d6): δ [ppm] = 10.39 (d, 1H), 8.82 (s, 1H), 8.62-8.59 (m, 1H), 8.34-8.25 (m, 2H), 6.61 (d, 1H), 5.75 (d, 1H), 4.80-4.69 (m, 1H), 4.58-4.50 (m, 1H), 4.35-3.43 (br. m, 4H), 1.93-1.83 (m, 1H), 1.70-1.58 (m, 1H), 1.00-0.91 (m, 3H).

**Beispiel 134**

1-(3-Fluorpyridin-2-yl)-7-[1-hydroxy-3-azabicyclo[3.1.0]hex-3-yl]-4-oxo-N-[(2R)-1,1,1-trifluorobutan-2-yl]-1,4-dihydro-1,8-naphthyridin-3-carbonsäureamid (Diastereomerengemisch)

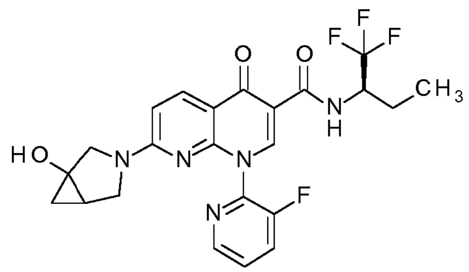

Gemäß allgemeiner Arbeitsvorschrift 3 wurden 100 mg (23.3 μmol) 7-Chlor-1-(3-fluorpyridin-2-yl)-4-oxo-*N*-((*R*)-1,1,1-trifluorobutan-2-yl)-1,4-dihydro-1,8-naphthyridin-3-carbonsäureamid (Beispiel 21A) mit 38.2 mg (257 μmol, 91% Reinheit) 3-Azabicyclo[3.1.0]hexan-1-ol-Hydrochlorid (Racemat) in Gegenwart von 142 μl (816 μmol) DIPEA in 2.2 ml DMF über Nacht zur Reaktion gebracht. Anschließend wurde mit 0.5 ml Acetonitril verdünnt und die Rohlösung mittels präparativer HPLC [Säule: Chromatorex C18, 10 μm, 125*30 mm, LM: Acetonitril / 0.05% Ameisensäure-Gradient; (0 bis 3 min 10% Acetonitril bis 35 min 90% Acetonitril und weitere 3 min 90% Acetonitril)] gereinigt. Es wurden 64.9 mg (56% d. Th., 99% Reinheit) der Titelverbindung erhalten.
LC-MS (Methode 2): Rt = 1.82 min; MS (ESIpos): m/z = 492 [M+H]+.
$^1$HNMR (400 MHz, DMSO-d6): δ [ppm] = 10.40 (d, 1H), 8.82 (s, 1H), 8.52 (br. s, 1H), 8.28 (d, 1H), 8.14-8.01 (m, 1H), 7.82-7.70 (m, 1H), 6.84-6.67 (m, 1H), 6.12-5.86 (m, 1H), 4.82-4.67 (m, 1H), 3.97-3.00 (br. m, 4H, zum Teil unter dem Wasser Peak), 1.93-1.81 (m, 1H), 1.71-1.46 (m, 2H), 1.10-0.85 (m, 4H), 0.50-0.34 (m, 1H).

**Beispiel 135**

N-[(1R)-1-cyclopropyl-2,2,2-trifluorethyl]-1-(3-fluorpyridin-2-yl)-7-[(1S)-1-hydroxy-3-azabicyclo[3.1.0]hex-3-yl]-4-oxo-1,4-dihydro-1,8-naphthyridin-3-carbonsäureamid (Diastereomerengemisch)

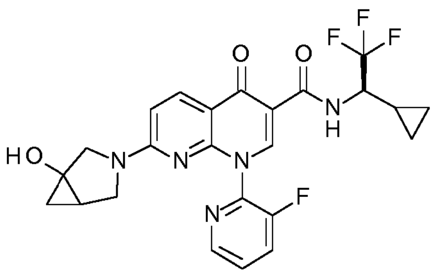

Gemäß allgemeiner Arbeitsvorschrift 3 wurden 50.0 mg (113 μmol) 7-Chlor-N-[(R)-1-cyclopropyl-2,2,2-trifluorethyl]-1-(3-fluoropyridin-2-yl)-4-oxo-1,4-dihydro-1,8-naphthyridin-3-carbonsäureamid (Beispiel 20C) mit 18.6 mg (125 μmol, 91% Reinheit) 3-Azabicyclo[3.1.0]hexan-1-ol-Hydrochlorid (Racemat) in Gegenwart von 69.0 μl (397 μmol) DIPEA in 1 ml DMF über Nacht zur Reaktion gebracht. Anschließend wurde mit 0.5 ml Acetonitril verdünnt und die Rohlösung mittels präparativer HPLC [Säule: Chromatorex C18, 10 μm, 125*30 mm, LM: Acetonitril / 0.05% Ameisensäure-Gradient; (0 bis 3 min 10% Acetonitril bis 35 min 90% Acetonitril und weitere 3 min 90% Acetonitril)] gereinigt. Es wurden 37.2 mg (65% d. Th., 100% Reinheit) der Titelverbindung erhalten.
LC-MS (Methode 1): Rt = 0.98 min; MS (ESIpos): m/z = 504 [M+H]+.
$^1$HNMR (400 MHz, DMSO-d6): δ [ppm] = 10.54 (d, 1H), 8.81 (s, 1H), 8.51 (br. s, 1H), 8.29 (d, 1H), 8.15-8.02 (m, 1H), 7.82-7.71 (m, 1H), 6.84-6.68 (m, 1H), 6.12-5.87 (m, 1H), 4.46-4.33 (m, 1H), 3.95-3.01 (br. m, 4H, zum Teil unter dem Wasser Peak), 1.69-1.48 (m, 1H), 1.26-1.15 (m, 1 H), 1.10-0.95 (m, 1H), 0.70-0.28 (m, 5H).

35 mg der Titelverbindung (Diastereomerengemisch) wurde durch chirale HPLC in die Diastereomere getrennt (präparative HPLC: Säule Daicel Chiralcel AS-H 5 μm 250x20 mm; Eluent: 35% Iso-Propanol, 65% Iso-Hexan; Temperatur: 45°C; Fluss: 15 ml/min; UV-Detektion: 220 nm.)

Man erhielt (in der Reihenfolge der Elution von der Säule) 13 mg Diastereomer 1 (99% de) Rt = 5.04 min und 15 mg (96%de) Diastereomer 2 Rt = 6.15 min.

[Analytische HPLC:Säule Chiralcel AS-H 5 μm 250x4.6 mm; Eluent: 35% Iso-Propanol, 65% Iso-Hexan; Temperatur: 40°C; Fluss: 1.0 ml/min; UV-Detektion: 220 nm].

Diastereomer 1 wurde zusätzlich mittels präparativer HPLC (Säule: Chromatorex C18, 10 μm, 125*30 mm, Lösungsmittel: Acetonitril / 0.05% Ameisensäure-Gradient; (0 bis 3 min 10% Acetonitril bis 35 min 90% Acetonitril und weitere 3 min 90% Acetonitril) gereinigt und 10.6 mg (18% d. Th., 99% Reinheit) der Titelverbindung aus Beispiel 136 erhalten.

Diastereomer 2 wurde zusätzlich mittels präparativer HPLC (Säule: Chromatorex C18, 10 μm, 125*30 mm, Lösungsmittel: Acetonitril / 0.05% Ameisensäure-Gradient; (0 bis 3 min 10% Acetonitril bis 35 min 90% Acetonitril und weitere 3 min 90% Acetonitril) gereinigt und 11.8 mg (20% d. Th., 99% Reinheit) der Titelverbindung aus Beispiel 137 erhalten.

## Beispiel 136

N-[(1R)-1-cyclopropyl-2,2,2-trifluorethyl]-1-(3-fluorpyridin-2-yl)-7-[(1S)-1-hydroxy-3-azabicyclo[3.1.0]hex-3-yl]-4-oxo-1,4-dihydro-1,8-naphthyridin-3-carbonsäureamid (Diastereomer 1)

LC-MS (Methode 2): Rt = 1.85 min; MS (ESIpos): m/z = 504 [M+H]+.

$^1$HNMR (400 MHz, DMSO-d6): δ [ppm] = 10.53 (d, 1H), 8.81 (s, 1H), 8.51 (br. s, 1H), 8.29 (d, 1H), 8.14-8.01 (m, 1H), 7.82-7.71 (m, 1H), 6.82-6.68 (m, 1H), 6.11-5.89 (m, 1H), 4.46-4.33 (m, 1H), 3.95-3.02 (br. m, 4H, zum Teil unter dem Wasser Peak), 1.69-1.48 (m, 1H), 1.26-1.14 (m, 1 H), 1.10-0.95 (m, 1H), 0.71-0.26 (m, 5H).

## Beispiel 137

N-[(1R)-1-cyclopropyl-2,2,2-trifluorethyl]-1-(3-fluorpyridin-2-yl)-7-[(1S)-1-hydroxy-3-azabicyclo[3.1.0]hex-3-yl]-4-oxo-1,4-dihydro-1,8-naphthyridin-3-carbonsäureamid (Diastereomer 2)

LC-MS (Methode 2): Rt = 1.84 min; MS (ESIpos): m/z = 504 [M+H]+.

$^1$HNMR (400 MHz, DMSO-d6): δ [ppm] = 10.54 (d, 1H), 8.81 (s, 1H), 8.51 (br. s, 1H), 8.29 (d, 1H), 8.14-8.03 (m, 1H), 7.82-7.72 (m, 1H), 6.83-6.69 (m, 1H), 6.11-5.90 (m, 1H), 4.46-4.34 (m, 1H), 3.95-3.02 (br. m, 4H, zum Teil unter dem Wasser Peak), 1.68-1.49 (m, 1H), 1.25-1.16 (m, 1H), 1.10-0.95 (m, 1H), 0.70-0.27 (m, 5H).

## Beispiel 138

N-[(1R)-1-Cyclopropyl-2,2,2-trifluorethyl]-1-(3,5-difluorpyridin-2-yl)-7-[(4S)-4-hydroxy-2-oxopyrrolidin-1-yl]-4-oxo-14-dihydro-1,8-naphthyridin-3-carbonsäureamid

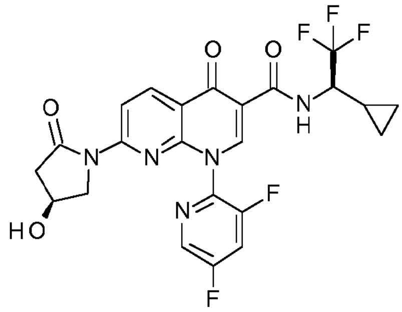

Gemäß allgemeiner Arbeitsvorschrift 1 wurden 50.0 mg (124 µmol) 1-(3,5-Difluorpyridin-2-yl)-7-[(4S)-4-hydroxy-2-oxopyrrolidin-1-yl]-4-oxo-1,4-dihydro-1,8-naphthyridin-3-carbonsäure (Beispiel 24A) mit 32.7 mg (186 µmol) (1R)-1-Cyclopropyl-2,2,2-trifluorethanamin-Hydrochlorid in Gegenwart von 47.3 mg (124 µmol) HATU und 52 µl (0.30 mmol) DIPEA in 1.3 ml DMF zur Reaktion gebracht. Anschließend wurde mit 1M wässriger Salzsäure auf pH 1 eingestellt und nach wässriger Aufarbeitung das Rohprodukt mittels Normalphasenchromatographie (Essigsäureethylester-Cyclohexan Gradient) gereinigt. Es wurden 48.4 mg (74% d. Th., 100% Reinheit) der Titelverbindung erhalten.
LC-MS (Methode 1): Rt = 1.01 min; MS (ESIpos): m/z = 524 [M+H]+.
$^1$HNMR (400 MHz, DMSO-d6): δ [ppm] = 10.26 (d, 1H), 9.05 (s, 1H), 8.72 (d, 1H), 8.67 (br. s, 1H), 8.59-8.51 (m, 1H), 8.43-8.35 (m, 1H), 5.41-5.22 (m, 1H), 4.49-4.36 (m, 1H), 4.33-4.26 (m, 1H), 3.79-3.64 (m, 1H), 3.58-3.44 (m, 1H), 3.03-2.87 (m, 1H), 2.44-2.33 (m, 1H), 1.30-1.18 (m, 1H), 0.71-0.51 (m, 3H), 0.40-0.29 (m, 1H).

## Beispiel 139

N-[(1R)-1-Cyclopropyl-2,2,2-trifluorethyl]-1-(3-fluorpyridin-2-yl)-7-[(4S)-4-hydroxy-2-oxopyrrolidin-1-yl]-4-oxo-1,4-dihydro-1,8-naphthyridin-3-carbonsäureamid

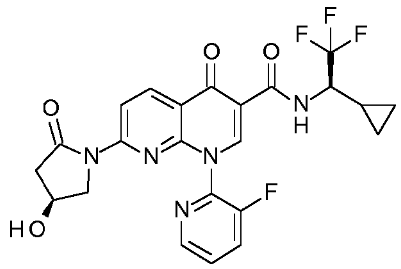

Gemäß allgemeiner Arbeitsvorschrift 2 wurden 50.0 mg (113 µmol) 7-Chlor-*N*-[(1*R*)-1-cyclopropyl-2,2,2-trifluorethyl]-1-(3-fluorpyridin-2-yl)-4-oxo-1,4-dihydro-1,8-naphthyridin-3-carbonsäureamid (Beispiel 20C) und 12.6 mg (125 µmol) (*S*)-4-Hydroxypyrrolidin in Gegenwart von 23.5 mg (170 µmol) Kaliumcarbonat, 2.55 mg (11.0 µmol) Palladiumacetat, 13.3 mg (23.0 µmol) Xantphos in 1.5 ml Dioxan bei 110°C zur Reaktion gebracht. Anschließend wurde das Lösungsmittel unter reduziertem Druck entfernt. Der Rückstand wurde in 3 ml Acetonitril und 0.5 ml Wasser gelöst und mittels präparativer HPLC (Säule: Chromatorex C18, 10 µm, 125*30 mm, Lösungsmittel: Acetonitril / 0.05% Ameisensäure-Gradient; (0 bis 3 min 10% Acetonitril bis 35 min 90% Acetonitril und weitere 13 min 90% Acetonitril) gereinigt. Es wurden 22.2 mg (38% d.Th., 98% Reinheit) der Titelverbindung erhalten.
LC-MS (Methode 2): $R_t$ = 1.69 min; MS (ESIpos): m/z = 506 [M+H]+.
$^1$HNMR (400 MHz, DMSO-d6): δ [ppm] = 10.27 (d, 1H), 9.05 (s, 1H), 8.72 (d, 1H), 8.58-8.51 (m, 2H), 8.15-8.08 (m, 1H), 7.85-7.79 (m, 1H), 5.40-5.23 (m, 1H), 4.50-4.36 (m, 1H), 4.32-4.23 (m, 1H), 3.79-3.40 (m, 2H), 3.03-2.86 (m, 1H), 2.43-2.32 (m, 1H), 1.30-1.18 (m, 1H), 0.72-0.51 (m, 3H), 0.42-0.30 (m, 1H).

## Beispiel 140

1-(3-Fluorpyridin-2-yl)-7-[(4S)-4-hydroxy-2-oxopyrrolidin-1-yl]-4-oxo-N-[(2R)-1,1,1-trifluorbutan-2-yl]-1,4-dihydro-1,8-naphthyridin-3-carbonsäureamid

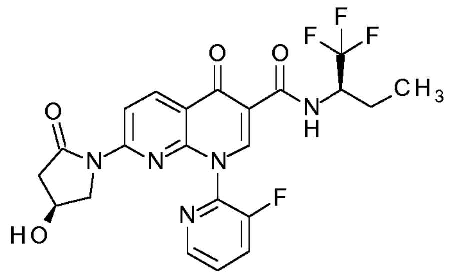

Gemäß allgemeiner Arbeitsvorschrift 2 wurden 35.0 mg (82.0 µmol) 7-Chlor-1-(3-fluorpyridin-2-yl)-4-oxo-*N*-[(2*R*)-1,1,1-trifluorobutan-2-yl]-1,4-dihydro-1,8-naphthyridin-3-carbonsäureamid (Beispiel 21A) und 8.25 mg (82.0 µmol) (*S*)-4-Hydroxypyrrolidin in Gegenwart von 39.9 mg (122 µmol) Cäsiumcarbonat, 3.30 mg (15.0 µmol) Palladiumacetat, 17.0 mg (29.0 µmol) Xantphos in 1.6 ml Dioxan zur Reaktion gebracht. Anschließend wurde das Lösungsmittel unter reduziertem Druck entfernt. Der Rückstand wurde in 3 ml Acetonitril und 0.5 ml Wasser gelöst und mittels präparativer HPLC (Säule: Chromatorex C18, 10 µm, 125*30 mm, Lösungsmittel: Acetonitril / 0.05% Ameisensäure-Gradient; (0 bis 3 min 10% Acetonitril bis 34 min 60% Acetonitril, innerhalb von 1 min auf 90% Acetonitril und weitere 10 min 90% Acetonitril) gereinigt. Es wurden 8.40 mg (21% d.Th., 99% Reinheit) der Titelverbindung erhalten.
LC-MS (Methode 1): $R_t$ = 0.97 min; MS (ESIpos): m/z = 494 [M+H]+.
$^1$HNMR (400 MHz, DMSO-d6): δ [ppm] = 10.14 (d, 1H), 9.06 (s, 1H), 8.72 (d, 1H), 8.58-8.51 (m, 2H), 8.16-8.09 (m, 1H), 7.85-7.79 (m, 1H), 5.42-5.23 (m, 1H), 4.84-4.68 (m, 1H), 4.31-4.23 (m, 1H), 3.79-3.40 (m, 2H), 3.03-2.86 (m, 1H), 2.43-2.30 (m, 1H), 1.96-1.81 (m, 1H), 1.74-1.59 (m, 1H), 0.98 (br. s, 3H).

**Beispiel 141**

1-(3,5-Difluorpyridin-2-yl)-7-[(4*R*)-4-methyl-2-oxoimidazolidin-1-yl]-4-oxo-N-[1-(trifluormethoxy)butan-2-yl]-1,4-dihydro-1,8-naphthyridin-3-carbonsäureamid (Diastereomerengemisch)

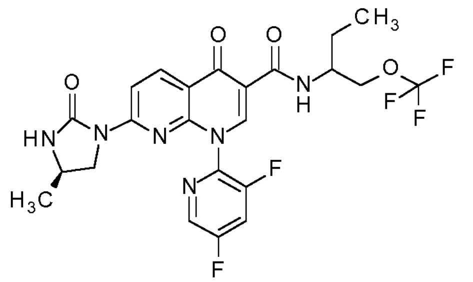

Die Verbindung aus Beispiel 31D (40.0 mg, 99.7 µmol) wurde in 990 µl DMF gelöst, mit HATU (45.5 mg, 120 µmol) und *N,N*-Diisopropylethylamin (69 µl, 400 µmol) versetzt und 15 min bei Raumtemperatur gerührt. Anschließend wurde mit *rac*-1-(Trifluormethoxy)butan-2-amin Hydrochlorid (23.2 mg, 120 µmol) versetzt und für 2.5 h bei Raumtemperatur gerührt. Die Reaktionslösung wurde mit Wasser verdünnt, mit 1 N Salzsäure angesäuert und zweimal mit Essigsäureethylester extrahiert. Die vereinigten organischen Phasen wurden einmal mit Wasser gewaschen, über Natriumsulfat getrocknet, filtriert und einrotiert. Der Rückstand wurde mittels Dickschichtchromatographie gereinigt (Laufmittel: Dichlormethan/Methanol = 10/1). Es wurden 36 mg der Zielverbindung (66% d. Th., Reinheit 98%) erhalten.
LC-MS (Methode 2): $R_t$ = 1.89 min; MS (ESIpos): m/z = 541 [M+H]+
$^1$H-NMR (400 MHz, DMSO-$d_6$) δ [ppm]: -0.149 (1.25), -0.008 (16.00), 0.008 (8.99), 0.146 (1.14), 0.933 (5.79), 0.945 (9.60), 0.951 (9.57), 0.962 (5.23), 1.099 (5.82), 1.111 (5.82), 1.156 (6.07), 1.168 (5.43), 1.235 (0.67), 1.566 (0.97), 1.584 (1.61), 1.600 (2.14), 1.618 (2.20), 1.638 (1.59), 1.661 (1.92), 1.673 (2.17), 1.693 (2.09), 2.328 (1.42), 2.366 (1.03), 2.519 (7.68), 2.524 (6.90), 2.670 (1.36), 2.710 (0.92), 3.092 (1.47), 3.105 (1.56), 3.150 (1.67), 3.163 (1.53), 3.723 (3.06), 3.736 (3.31), 3.779 (2.89), 3.797 (1.98), 4.197 (7.71), 5.754 (2.34), 7.812 (8.93), 8.322 (1.61), 8.343 (1.70), 8.365 (1.59), 8.384 (1.03), 8.407 (6.73), 8.430 (8.82), 8.531 (1.70), 8.539 (12.41), 8.553 (1.34), 8.562 (9.04), 8.636 (6.46), 8.931 (8.60), 9.877 (3.81), 9.896 (3.45).

In Analogie zu Beispiel 141 wurden die in Tabelle 1 gezeigten Beispielverbindungen hergestellt, indem 1-(3,5-Difluorpyridin-2-yl)-7-[(4R)-4-methyl-2-oxoimidazolidin-1-yl]-4-oxo-1,4-dihydro-1,8-naphthyridin-3-carbonsäure (Beispiel 31D) mit den entsprechenden Aminen (bzw. deren Salzen; 1.2 Äquivalente) unter den beschriebenen Reaktionsbedingungen (2.5 h bei Raumtemperatur) umgesetzt wurde.

Tabelle 1:

| Bsp. | IUPAC-Name Struktur Eingesetztes Amin Ausbeute | LC-MS Methode Retentionszeit Detektierte Masse NMR-Daten |
|---|---|---|
| **142** | N-[(1S)-1-Cyclopropyl-2,2,2-trifluorethyl]-1-(3,5-difluorpyridin-2-yl)-7-[(4R)-4-methyl-2-oxoimidazolidin-1-yl]-4-oxo-1,4-dihydro-1,8-naphthyridin-3-carboxamid 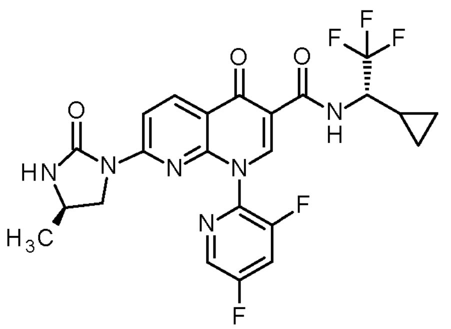 (1S)-1-Cyclopropyl-2,2,2-trifluorethanaminhydrochlorid (73% d. Th.) | LC-MS (Methode 2): $R_t$ = 1.89 min m/z = 523 $[M+H]^+$ $^1$H-NMR (400 MHz, DMSO-$d_6$) $\delta$ [ppm]: -0.149 (1.18), -0.008 (9.38), 0.008 (8.84), 0.146 (1.08), 0.348 (2.59), 0.552 (3.29), 0.656 (2.72), 1.099 (6.22), 1.111 (6.45), 1.157 (6.96), 1.169 (6.62), 1.198 (1.61), 1.206 (2.08), 1.219 (3.50), 1.238 (3.73), 1.251 (1.88), 2.328 (1.85), 2.367 (1.34), 2.524 (5.82), 2.670 (1.78), 2.710 (1.41), 3.096 (1.61), 3.109 (1.71), 3.152 (1.88), 3.165 (1.82), 3.724 (3.43), 3.740 (3.76), 3.778 (3.16), 4.390 (1.71), 4.411 (3.13), 4.432 (3.06), 4.452 (1.65), 7.830 (10.79), 8.306 (0.94), 8.328 (1.82), 8.346 (2.02), 8.368 (1.88), 8.428 (8.13), 8.450 (11.29), 8.549 (16.00), 8.572 (11.19), 8.639 (8.13), 8.978 (6.89), 10.334 (3.93), 10.356 (3.83). |
| **143** | 1-(3,5-Difluorpyridin-2-yl)-7-[(4R)-4-methyl-2-oxoimidazolidin-1-yl]-4-oxo-N-[(2S)-1,1,1-trifluorbutan-2-yl]-1,4-dihydro-1,8-naphthyridin-3-carboxamid 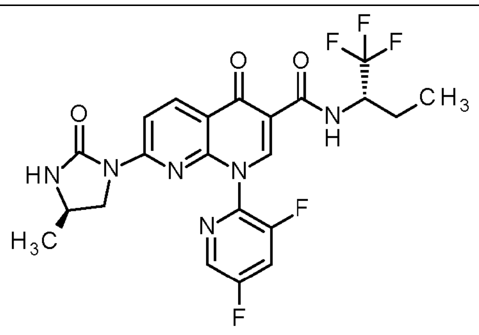 (2S)-1,1,1-Trifluorbutan-2-aminhydrochlorid (75% d. Th.) | LC-MS (Methode 2): $R_t$ = 1.86 min m/z = 511 $[M+H]^+$ $^1$H-NMR (400 MHz, DMSO-$d_6$) $\delta$ [ppm]: -0.149 (0.89), -0.008 (8.18), 0.008 (7.53), 0.146 (0.92), 0.969 (9.50), 0.983 (9.98), 1.000 (4.34), 1.100 (6.07), 1.112 (6.45), 1.158 (6.91), 1.170 (6.53), 1.622 (1.16), 1.640 (1.67), 1.656 (1.97), 1.682 (1.97), 1.700 (1.43), 1.863 (1.51), 1.882 (1.86), 1.891 (2.08), 1.908 (1.62), 1.925 (1.13), 2.328 (1.40), 2.367 (1.05), 2.524 (4.83), 2.670 (1.51), 2.711 (1.16), 3.097 (1.59), 3.110 (1.70), 3.152 (1.83), 3.166 (1.81), 3.725 (3.35), 3.740 (3.75), 3.781 (3.21), 3.799 (2.37), 4.763 (2.02), 4.783 (1.92), 7.829 (10.55), 8.308 (0.89), 8.329 (1.75), 8.352 (1.94), 8.372 (1.83), 8.391 (0.92), 8.427 (7.80), 8.449 (11.14), 8.546 (16.00), 8.568 (11.22), 8.642 (6.26), 8.988 (6.72), 10.196 (5.83), 10.220 (5.67). |

(fortgesetzt)

| Bsp. | IUPAC-Name Struktur Eingesetztes Amin Ausbeute | LC-MS Methode Retentionszeit Detektierte Masse NMR-Daten |
|---|---|---|
| **144** | N-(1,1-Difluor-2-methylpropan-2-yl)-1-(3,5-difluorpyridin-2-yl)-7-[(4R)-4-methyl-2-oxoimidazolidin-1-yl]-4-oxo-1,4-dihydro-1,8-naphthyridin-3-carboxamid 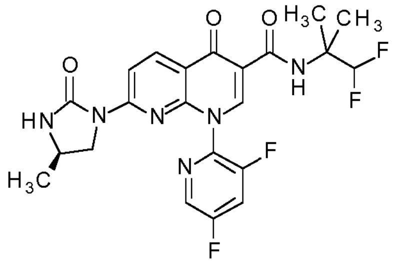 1,1-Difluor-2-methylpropan-2-aminhydrochlorid (67% d. Th.) | LC-MS (Methode 2): $R_t$ = 1.80 min m/z = 493 $[M+H]^+$ <br> $^1$H-NMR (400MHz, DMSO-$d_6$) $\delta$ [ppm]: -0.008 (3.23), 0.008 (3.30), 1.108 (1.47), 1.154 (1.51), 1.166 (1.42), 1.450 (16.00), 2.328 (0.49), 2.524 (1.59), 2.670 (0.50), 3.102 (0.40), 3.146 (0.42), 3.733 (0.82), 3.774 (0.76), 6.292 (0.98), 6.434 (1.81), 6.577 (0.80), 7.815 (2.53), 8.323 (0.42), 8.346 (0.46), 8.411 (2.13), 8.433 (2.87), 8.532 (4.37), 8.555 (3.00), 8.635 (1.98), 8.918 (2.96), 10.119 (3.31). |

**Beispiel 145**

1-(3,5-Difluorpyridin-2-yl)-4-oxo-7-(2-oxoimidazolidin-1-yl)-*N*-(4,4,4-trifluor-2-methylbutan-2-yl)-1,4-dihydro-1,8-naphthyridin-3 -carbonsäureamid

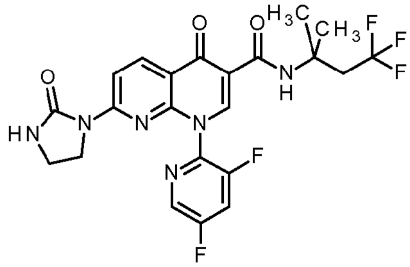

Gemäß allgemeiner Arbeitsvorschrift 1 wurden 50.0 mg (100 µmol, 78% Reinheit) 1-(3,5-Difluorpyridin-2-yl)-4-oxo-7-(2-oxoimidazolidin-1-yl)-1,4-dihydro-1,8-naphthyridin-3-carbonsäure (Beispiel 30A) mit 21.4 mg (121 µmol) 4,4,4-Trifluor-2-Methylbutan-2-amin Hydrochlorid in Gegenwart von 45.8 mg (121 µmol) HATU und 52 µl (300 µmol) DIPEA in 0.39 ml DMF zur Reaktion gebracht. Das Reaktionsgemisch wurde mit Wasser, Acetonitril und Ameisensäure verdünnt, über einen Milliporefilter filtriert und mittels präparativer HPLC (Acetonitril / Wasser/ 0.1 % Ameisensäure) gereinigt und 15.3 mg (30% d. Th., 100% Reinheit) der Titelverbindung erhalten.
LC-MS (Methode 2): $R_t$ = 1.83 min; MS (ESIpos): m/z = 511 [M+H]+.
$^1$H NMR (400 MHz, DMSO-d6): $\delta$ [ppm] = 9.98 (s, 1H), 8.91 (s, 1H), 8.63 (d, 1H), 8.54 (d, 1H), 8.42 (d, 1H), 8.32 (ddd, 1H), 7.65 (br s, 1H), 3.54-3.70 (m, 2H), 3.32-3.41 (m, 2H), 2.90-3.02 (m, 2H), 1.50 (s, 6H).

**Beispiel 146**

*N*-Cyclobutyl-1-(3,5-difluorpyridin-2-yl)-4-oxo-7-(2-oxoimidazolidin-1-yl)-1,4-dihydro-1,8-naphthyridin-3 -carbonsäureamid

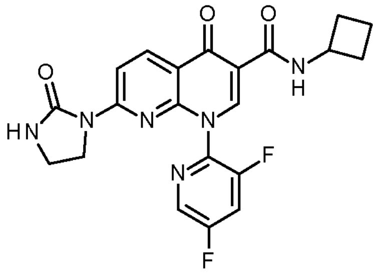

Gemäß allgemeiner Arbeitsvorschrift 1 wurden 25.0 mg (64.5 µmol, 78% Reinheit) 1-(3,5-Difluorpyridin-2-yl)-4-oxo-7-(2-oxoimidazolidin-1-yl)-1,4-dihydro-1,8-naphthyridin-3-carbonsäure (Beispiel 30A) mit 8.33 mg (77.5 µmol) Cyclobutanamin Hydrochlorid in Gegenwart von 29.5 mg (77.5 µmol) HATU und 34 µl (190 µmol) DIPEA in 0.25 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Acetonitril / Wasser/ 0.1 % Ameisensäure) gereinigt und 5.50 mg (19% d. Th., 100% Reinheit) der Titelverbindung erhalten.
LC-MS (Methode 2): $R_t$ = 1.53 min; MS (ESIpos): m/z = 441 [M+H]+.
$^1$H NMR (400 MHz, DMSO-d6): δ [ppm] = 9.94 (d, 1H), 8.89 (s, 1H), 8.63 (d, 1H), 8.54 (d, 1H), 8.41 (d, 1H), 8.32 (td, 1H), 7.60-7.66 (m, 1H), 4.39-4.48 (m, 1H), 3.53-3.69 (m, 2H), 3.32-3.40 (m, 2H), 2.26-2.33 (m, 2H), 1.93-2.03 (m, 2H), 1.66-1.78 (m, 2H).

## Beispiel 147

*N*-[1-(2-Chlorphenyl)-2,2,2-trifluorethyl]-1-(3,5-difluorpyridin-2-yl)-4-oxo-7-(2-oxoimidazolidin-1-yl)-1,4-dihydro-1,8-naphthyridin-3-carbonsäureamid (Racemat)

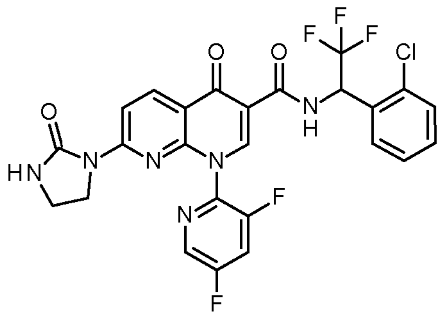

Gemäß allgemeiner Arbeitsvorschrift 1 wurden 50.0 mg (99.4 µmol, 78% Reinheit) 1-(3,5-Difluorpyridin-2-yl)-4-oxo-7-(2-oxoimidazolidin-1-yl)-1,4-dihydro-1,8-naphthyridin-3-carbonsäure (Beispiel 30A) mit 62.5 mg (298 µmol) 1-(2-Chlorphenyl)-2,2,2-trifluorethanamin in Gegenwart von 45.4 mg (119 µmol) HATU und 52 µl (300 µmol) DIPEA in 0.38 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Acetonitril / Wasser/ 0.1 % Ameisensäure) gereinigt und 17.2 mg (30% d. Th., 100% Reinheit) der Titelverbindung erhalten.
LC-MS (Methode 2): $R_t$ = 2.07 min; MS (ESIpos): m/z = 579 [M+H]+.
$^1$H NMR (400 MHz, DMSO-d6): δ [ppm] = 11.32 (d, 1H), 9.00 (s, 1H), 8.63 (d, 1H), 8.60 (d, 1H), 8.46 (d, 1H), 8.29-8.36 (m, 1H), 7.48-7.69 (m, 5H), 6.42-6.51 (m, 1H), 3.53-3.70 (m, 2H), 3.33-3.43 (m, 2H).

15.5 mg der Titelverbindung (Racemat) wurde durch chirale HPLC in die Enantiomere getrennt (präparative HPLC: Säule YMC Chiralart Amylose 5 µm 250x30 mm; Eluent: 50% *n*-Heptan, 50% Iso-Propanol; Temperatur: 35°C; Fluss: 30 ml/min; UV-Detektion: 220 nm.)

Man erhielt (in der Reihenfolge der Elution von der Säule) 4.00 mg Enantiomer A (99% ee) Rt = 8.67 min und 6.00 mg Enantiomer B (98% ee) Rt = 13.68 min.

[Analytische HPLC: Säule YMC Chiralart Amylose 5 µm 250x4.6 mm; Eluent: 50% Iso-Hexan, 50% Iso-propanol; Temperatur: 35°C; Fluss: 1.0 ml/min; UV-Detektion: 220 nm].

Enantiomer A wurde zusätzlich mittels präparativer HPLC (Acetonitril, Wasser, 0.1% Ameisensäure) gereinigt und 3.50 mg (6% d. Th., 100% Reinheit) der Titelverbindung aus Beispiel 148 erhalten.

Enantiomer B wurde zusätzlich mittels präparativer HPLC (Acetonitril, Wasser, 0.1% Ameisensäure) gereinigt

und 3.70 mg (6.5% d. Th., 100% Reinheit) der Titelverbindung aus Beispiel 149 erhalten.

**Beispiel 148**

*N*-[1-(2-Chlorphenyl)-2,2,2-trifluorethyl]-1-(3,5-difluorpyridin-2-yl)-4-oxo-7-(2-oxoimidazolidin-1-yl)-1,4-dihydro-1,8-naphthyridin-3-carboxamid (Enantiomer A)

LC-MS (Methode 1): $R_t$ = 1.10 min; MS (ESIpos): m/z = 579 [M+H]+
$^1$H-NMR (500 MHz, DMSO-d6): δ [ppm] = 11.32 (br d, 1 H), 9.00 (s, 1 H), 8.61 - 8.66 (m, 1 H), 8.60 (d, 1 H), 8.46 (d, 1 H), 8.30 - 8.36 (m, 1 H), 7.68 (s, 1 H), 7.59 - 7.67 (m, 2 H), 7.48 - 7.59 (m, 2 H), 6.43 - 6.50 (m, 1 H), 3.54 - 3.69 (m, 2 H), 3.32 - 3.40 (m, 2 H).

**Beispiel 149**

*N*-[1-(2-Chlorphenyl)-2,2,2-trifluorethyl]-1-(3,5-difluorpyridin-2-yl)-4-oxo-7-(2-oxoimidazolidin-1-yl)-1,4-dihydro-1,8-naphthyridin-3-carboxamid (Enantiomer B)

LC-MS (Methode 1): $R_t$ = 1.10 min; MS (ESIpos): m/z = 579 [M+H]+
$^1$H-NMR (400 MHz, DMSO-d6): δ [ppm] = 11.32 (br d, 1 H), 9.00 (s, 1 H), 8.62 - 8.67 (m, 1 H), 8.60 (d, 1 H), 8.46 (d, 1 H), 8.29 - 8.38 (m, 1 H), 8.34 (br d, 1 H), 7.68 (s, 1 H), 7.59 - 7.67 (m, 2 H), 7.47 - 7.59 (m, 2 H), 6.39 - 6.51 (m, 1 H), 3.53 - 3.71 (m, 2 H), 3.34 - 3.40 (m, 2 H).

**Beispiel 150**

1-(3,5-Difluorpyridin-2-yl)-4-oxo-7-(2-oxoimidazolidin-1-yl)-*N*-[1-(trifluormethyl)cyclobutyl]-1,4-dihydro-1,8-naphthyridin-3-carbonsäureamid

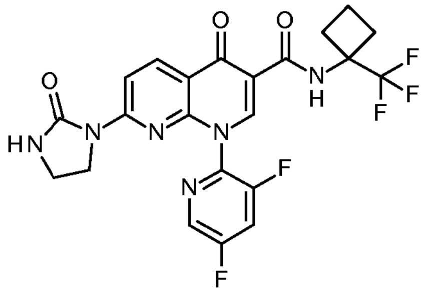

Gemäß allgemeiner Arbeitsvorschrift 1 wurden 50.0 mg (99.4 μmol, 78% Reinheit) 1-(3,5-Difluorpyridin-2-yl)-4-oxo-7-(2-oxoimidazolidin-1-yl)-1,4-dihydro-1,8-naphthyridin-3-carbonsäure (Beispiel 30A) mit 41.5 mg (298 μmol) 1-(Trifluormethyl)cyclobutanamin in Gegenwart von 45.4 mg (119 μmol) HATU und 52 μl (300 μmol) DIPEA in 0.38 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Acetonitril / Wasser/ 0.1 % Ameisensäure) gereinigt und 15.9 mg (31% d. Th., 100% Reinheit) der Titelverbindung erhalten.
LC-MS (Methode 2): $R_t$ = 1.82 min; MS (ESIpos): m/z = 509 [M+H]+.
$^1$H NMR (400 MHz, DMSO-d6): δ [ppm] = 10.32 (s, 1H), 8.94 (s, 1H), 8.64 (d, 1H), 8.55 (d, 1H), 8.43 (d, 1H), 8.29-8.36 (m, 1H), 7.66 (s, 1H), 3.53-3.71 (m, 2H), 3.35-3.43 (m, 2H), 2.55-2.64 (m, 2H), 1.89-2.09 (m, 2H).

**Beispiel 151**

1-(3,5-Difluorpyridin-2-yl)-7-[(4*S*)-4-hydroxy- 2-oxopyrrolidin-1-yl]-4-oxo-*N*-[1-(trifluormethyl)cyclobutyl]-1,4-dihydro-1,8-naphthyridin-3-carbonsäureamid

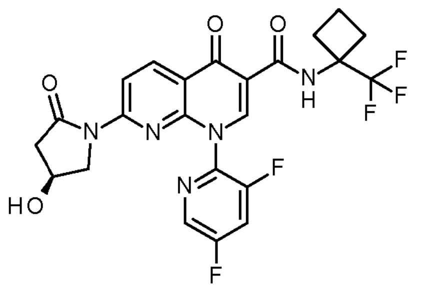

Gemäß allgemeiner Arbeitsvorschrift 1 wurden 50.0 mg (124 µmol, 95% Reinheit) 1-(3,5-Difluorpyridin-2-yl)-7-[(4*S*)-4-hydroxy-2-oxopyrrolidin-1-yl]-4-oxo-1,4-dihydro-1,8-naphthyridin-3-carbonsäure (Beispiel 24A) mit 20.7 mg (149 µmol) 1-(Trifluormethyl)cyclobutanamin in Gegenwart von 56.7 mg (149 µmol) HATU und 65 µl (370 µmol) DIPEA in 0.48 ml DMF zur Reaktion gebracht. Das Reaktionsgemisch wurde mit Acetonitril, Wasser und Ameisensäure verdünnt, über einen Milliporefilter filtriert und die Rohlösung mittels präparativer HPLC (Acetonitril / Wasser/ 0.1 % Ameisensäure) gereinigt und 48.0 mg (74% d. Th., 100% Reinheit) der Titelverbindung erhalten.
LC-MS (Methode 2): $R_t$ = 1.76 min; MS (ESIpos): m/z = 524 [M+H]+.
$^1$H NMR (400 MHz, DMSO-d6): δ [ppm] = 10.22 (s, 1H), 9.01 (s, 1H), 8.71 (d, 1H), 8.65-8.69 (m, 1H), 8.51-8.57 (m, 1H), 8.39 (td, 1H), 5.24-5.40 (m, 1H), 4.26-4.32 (m, 1H), 3.65-3.78 (m, 1H), 3.45-3.56 (m, 1H), 2.88-3.02 (m, 1H), 2.55-2.65 (m, 4H), 2.34-2.42 (m, 1H), 1.91-2.10 (m, 2H).

**Beispiel 152**

1-(3,5-Difluorpyridin-2-yl)-7-[(3*R*,4*R*)-3,4-dihydroxypyrrolidin-1-yl]-*N*-(1,1,1,3,3,3-hexafluor-2-methylpropan-2-yl)-4-oxo-1,4-dihydro-1,8-naphthyridin-3-carbonsäureamid

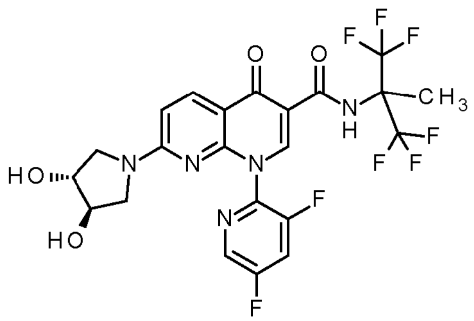

Gemäß allgemeiner Arbeitsvorschrift 3 wurden 23.2 mg (46.3 µmol) 7-Chlor-1-(3,5-difluorpyridin-2-yl)-*N*-(1,1,1,3,3,3-hexafluor-2-methylpropan-2-yl)-4-oxo-1,4-dihydro-1,8-naphthyridin-3-carbonsäureamid (Beispiel 29A) mit 7.76 mg (55.6 µmol) (3*R*,4*R*)-Pyrrolidin-3,4-diol-Hydrochlorid in Gegenwart von 24 µl (140 µmol) DIPEA in 0.19 ml DMF zur Reaktion gebracht. Es wurde mit Wasser, Acetonitril und Ameisensäure verdünnt, über einen Milliporefilter filtriert und mittels präparativer HPLC (Acetonitril / Wasser/ 0.1 % Ameisensäure) gereinigt und 13.4 mg (51% d. Th., 100% Reinheit) der Titelverbindung erhalten.
LC-MS (Methode 2): $R_t$ = 1.79 min; MS (ESIpos): m/z = 568 [M+H]+.
$^1$H NMR (400 MHz, DMSO-d6): δ [ppm] = 11.61 (s, 1H), 8.83 (d, 1H), 8.62 (d, 1H), 8.32-8.37 (m, 1H), 8.30 (d, 1H), 6.79 (d, 1H), 5.05-5.30 (m, 2H), 4.02-4.08 (m, 1H), 3.90-3.95 (m, 1H), 3.57-3.68 (m, 1H), 3.33-3.39 (m, 1H), 3.18-3.28 (m, 1H), 3.03-3.16 (m, 1H), 2.06 (br s, 3H).

**Beispiel 153**

N-[(2-Cyclopropyl-1,1,1-trifluorpropan-2-yl]-1-(3,5-difluorpyridin-2-yl)-7-[(3*R*,4*R*)-3,4-dihydroxypyrrolidin-1-yl]-4-oxo-1,4-dihydro-1,8-naphthyridin-3-carboxamid (Diastereomerengemisch)

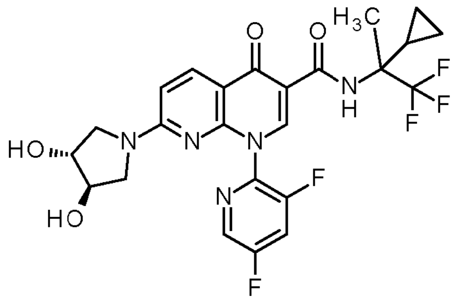

Gemäß AAV1 wurden 50.0 mg (124 µmol) 1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure mit 22.7 mg (148 µmol) 2-Cyclopropyl-1,1,1-trifluorpropan-2-amin in Gegenwart von 56.4 mg (148 µmol) HATU und 65 µl (370 µmol) DIPEA in 500 µl DMF zur Reaktion gebracht. Das Rohprodukt wurde zweimal mittels präparativer HPLC (Acetonitril / Wasser/ 0.1 % Ameisensäure) gereinigt. Es wurden 30.7 mg (46% d. Th., 100% Reinheit) der Titelverbindung erhalten.
LC-MS (Methode 1): $R_t$ = 0.91 min; MS (ESIpos): m/z = 540 [M+H]+
$^1$H-NMR (400 MHz, DMSO-d6): δ [ppm] = 10.38 (d, 1 H), 8.75 (d, 1 H), 8.61 (d, 1 H), 8.30 - 8.36 (m, 1 H), 8.28 (d, 1 H), 6.76 (d, 1 H), 5.25 - 5.29 (m, 0.50 H), 5.17 - 5.21 (m, 1 H), 5.05 - 5.08 (m, 0.50 H), 4.02 - 4.07 (m, 1 H), 3.90 - 3.95 (m, 1 H), 3.57 - 3.66 (m, 1 H), 3.32 - 3.38 (m, 1 H), 3.19 - 3.25 (m, 0.50 H), 3.10 - 3.18 (m, 0.50 H), 3.03 - 3.08 (m, 0.50 H), 1.60 (s, 3 H), 1.37 - 1.45 (m, 1 H), 0.65 - 0.72 (m, 1 H), 0.54 - 0.60 (m, 2 H), 0.45 - 0.52 (m, 1H).

**Beispiel 154**

1-(3,5-Difluorpyridin-2-yl)-7-[(4*S*)-4-hydroxy-2-oxopyrrolidin-1-yl]-4-oxo-*N*-[1,1,1-trifluor-3-methylbutan-2-yl]-1,4-dihydro-1,8-naphthyridin-3-carboxamid (Diastereomerengemisch)

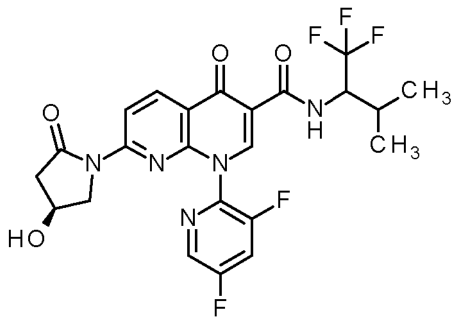

Gemäß AAV1 wurden 80.0 mg (199 µmol) 1-(3,5-Difluorpyridin-2-yl)-7-[(4*S*)-4-hydroxy-2-oxopyrrolidin-1-yl]-4-oxo-1,4-dihydro-1,8-naphthyridin-3-carbonsäure mit 33.7 mg (239 µmol) 1,1,1-Trifluor-3-methylbutan-2-amin in Gegenwart von 90.7 mg (239 µmol) HATU und 100 µl (600 µmol) DIPEA in 1.4 ml DMF zur Reaktion gebracht. Das Rohprodukt wurde mittels präparativer HPLC (Acetonitril / Wasser/ 0.1 % Ameisensäure) gereinigt. Es wurden 72.9 mg (67% d. Th., 96% Reinheit) der Titelverbindung erhalten.
LC-MS (Methode 2): $R_t$ = 1.85 min; MS (ESIpos): m/z = 526 [M+H]+
$^1$H-NMR (400 MHz, DMSO-d6): δ [ppm] = 10.33 (br d, 1 H), 9.07 (s, 1 H), 8.74 (d, 1 H), 8.63 - 8.70 (m, 1 H), 8.55 (br t, 1 H), 8.35 - 8.43 (m, 1 H), 5.38 (br d, 0.5 H), 5.26 (br d, 0.5 H), 4.75 - 4.85 (m, 1 H), 4.29 (br s, 1 H), 3.65 - 3.78 (m, 1 H), 3.45 - 3.57 (m, 1 H), 2.88 - 3.03 (m, 1 H), 2.34 - 2.43 (m, 1 H), 2.22 - 2.31 (m, 1 H), 1.04 (br d, 3 H), 0.94 - 1.00 (m, 3 H).

**Beispiel 155**

1-(3,5-Difluorpyridin-2-yl)-7-[(3*R*,4*R*)-3,4-dihydroxypyrrolidin-1-yl]-4-oxo-*N*-[1-(trifluormethyl)cyclobutyl]-1,4-dihydro-1,8-naphthyridin-3-carboxamid

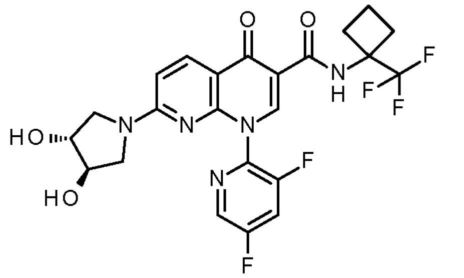

Gemäß AAV1 wurden 60.0 mg (148 μmol) 1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure mit 31.3 mg (178 μmol) 1-(Trifluormethyl)cyclobutanamin Hydrochlorid in Gegenwart von 67.7 mg (178 μmol) HATU und 90 μl (520 μmol) DIPEA in 660 μl DMF zur Reaktion gebracht. Das Rohprodukt wurde mittels präparativer HPLC (Acetonitril / Wasser/ 0.1 % Ameisensäure) gereinigt. Es wurden 59.8 mg (77% d. Th., 100% Reinheit) der Titelverbindung erhalten.
LC-MS (Methode 2): $R_t$ = 1.58 min; MS (ESIpos): m/z = 526 [M+H]+
$^1$H-NMR (400 MHz, DMSO-d6): δ [ppm] = 10.54 (s, 1 H), 8.77 (d, 1 H), 8.62 (d, 1 H), 8.33 (td, 1 H), 8.27 (d, 1 H), 6.77 (d, 1 H), 5.27 (br d, 0.50 H), 5.19 (br s, 1 H), 5.07 (br d, 0.50 H), 4.05 (br s, 1 H), 3.93 (br s, 1 H), 3.57 - 3.67 (m, 1 H), 3.33 - 3.38 (m, 1 H), 3.19 - 3.26 (m, 0.50 H), 3.03 - 3.17 (m, 1 H), 2.54 - 2.63 (m, 3 H), 1.90 - 2.06 (m, 2 H).

**Beispiel 156**

1-(3,5-Difluorpyridin-2-yl)-4-oxo-7-(2-oxoimidazolidin-1-yl)-*N*-[(2*R*)-1,1,1-trifluor-4-methylpentan-2-yl]-1,4-dihydro-1,8-naphthyridin-3-carboxamid

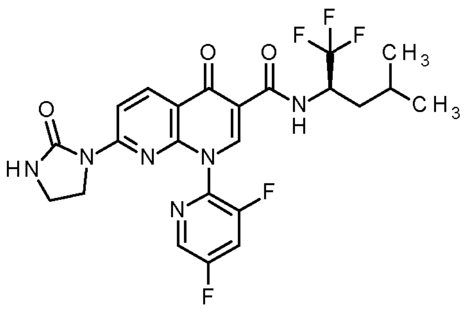

Gemäß AAV1 wurden 50.0 mg (129 μmol) 1-(3,5-Difluorpyridin-2-yl)-4-oxo-7-(2-oxoimidazolidin-1-yl)-1,4-dihydro-1,8-naphthyridin-3-carbonsäure mit 40.1 mg (258 μmol) (2*R*)-1,1,1-Trifluor-4-methylpentan-2-amin in Gegenwart von 98.2 mg (258 μmol) HATU und 90 μl (520 μmol) DIPEA in 750 μl DMF zur Reaktion gebracht. Das Rohprodukt wurde mittels präparativer HPLC (Acetonitril / Wasser/ 0.1 % Ameisensäure) gereinigt. Es wurden 18.9 mg (28% d. Th., 99% Reinheit) der Titelverbindung erhalten.
LC-MS (Methode 2): $R_t$ = 2.05 min; MS (ESIpos): m/z = 525 [M+H]+
$^1$H-NMR (400 MHz, DMSO-d6): δ [ppm] = 10.16 - 10.23 (m, 1 H), 8.99 (s, 1 H), 8.63 (br d, 1 H), 8.55 (d, 1 H), 8.44 (d, 1 H), 8.29 - 8.36 (m, 1 H), 7.67 (s, 1 H), 4.80 - 4.91 (m, 1 H), 3.54 - 3.71 (m, 2 H), 3.33-3.42 (m, 2 H), 1.54 - 1.74 (m, 3 H), 0.95 (br d, 3 H), 0.89 (br t, 3 H).

**Beispiel 157**

1-(3,5-Difluorpyridin-2-yl)-4-oxo-7-(2-oxoimidazolidin-1-yl)-*N*-[(2*S*)-1,1,1-trifluor-4-methylpentan-2-yl]-1,4-dihydro-1,8-naphthyridin-3-carboxamid

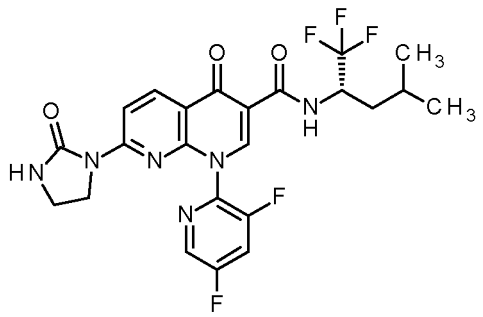

Gemäß AAV1 wurden 50.0 mg (129 μmol) 1-(3,5-Difluorpyridin-2-yl)-4-oxo-7-(2-oxoimidazolidin-1-yl)-1,4-dihydro-1,8-naphthyridin-3-carbonsäure mit 40.0 mg (258 μmol) (2*S*)-1,1,1-Trifluor-4-methylpentan-2-amin in Gegenwart von 98.2 mg (258 μmol) HATU und 90 μl (520 μmol) DIPEA in 750 μl DMF zur Reaktion gebracht. Das Rohprodukt wurde mittels präparativer HPLC (Acetonitril / Wasser/ 0.1 % Ameisensäure) gereinigt. Es wurden 19.2 mg (28% d. Th., 100% Reinheit) der Titelverbindung erhalten.
LC-MS (Methode 2): $R_t$ = 2.05 min; MS (ESIpos): m/z = 525 [M+H]+
$^1$H-NMR (400 MHz, DMSO-d6): δ [ppm] = 10.20 (br dd, 1 H), 8.99 (s, 1 H), 8.63 (d, 1 H), 8.55 (d, 1 H), 8.44 (d, 1 H), 8.30 - 8.36 (m, 1 H), 7.67 (s, 1 H), 4.79 - 4.91 (m, 1 H), 3.54 - 3.70 (m, 2 H), 3.33 - 3.41 (m, 2 H), 1.63 - 1.74 (m, 2 H), 1.54 - 1.63 (m, 1 H), 0.93 - 0.98 (m, 3 H), 0.89 (br t, 3 H).

**Beispiel 158**

1-(3,5-Difluorpyridin-2-yl)-*N*-(1,1,1,3,3,3-hexafluorpropan-2-yl)-4-oxo-7-(2-oxoimidazolidin-1-yl)-1,4-dihydro-1,8-naphthyridin-3 -carboxamid

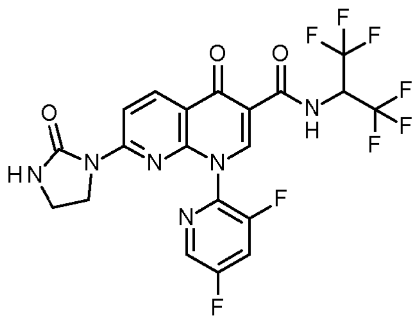

Gemäß AAV2 wurden 70.0 mg (144 μmol) 7-Chlor-1-(3,5-difluorpyridin-2-yl)-*N*-(1,1,1,3,3,3-hexafluorpropan-2-yl)-4-oxo-1,4-dihydro-1,8-naphthyridin-3-carboxamid mit 124 mg (1.44 mmol) Imida-zolidin-2-on in Gegenwart von 29.8 mg (216 μmol) Kaliumcarbonat, 6.46 mg (28.8 μmol) Palladiumacetat und 16.6 mg (28.8 μmol) Xantphos in 1.4 ml 1,4-Dioxan zur Reaktion gebracht. Das Rohprodukt wurde mittels präparativer HPLC (Acetonitril / Wasser/ 0.1 % Ameisensäure) gereinigt. Die Substanz wurde abschließend aus Acetonitril umkristallisiert, abgesaugt, mit etwas Acetonitril nachgewaschen, nachgetrocknet und nochmals mittels präparativer HPLC (Acetonitril / Wasser/ 0.1% Ameisensäure) gereinigt. Es wurden 32.7 mg (40% d. Th., 95% Reinheit) der Titelverbindung erhalten.
LC-MS (Methode 2): $R_t$ = 1.93 min; MS (ESIpos): m/z = 537 [M+H]+
$^1$H-NMR (400 MHz, DMSO-d6): δ [ppm] = 11.14 (d, 1 H), 9.08 (s, 1 H), 8.65 (d, 1 H), 8.58 (d, 1 H), 8.46 (d, 1 H), 8.31 - 8.37 (m, 1 H), 7.69 (s, 1 H), 6.32 - 6.42 (m, 1 H), 3.54 - 3.70 (m, 2 H), 3.33 - 3.41 (m, 2 H).

**Beispiel 159**

1-(3,5-Difluorpyridin-2-yl)-*N*-[1,1,1,4,4,4-hexafluorbutan-2-yl]-7-[(4*S*)-4-hydroxy-2-oxopyrrolidin-1-yl]-4-oxo-1,4-dihydro-1,8-naphthyridin-3-carboxamid (Diastereomerengemisch)

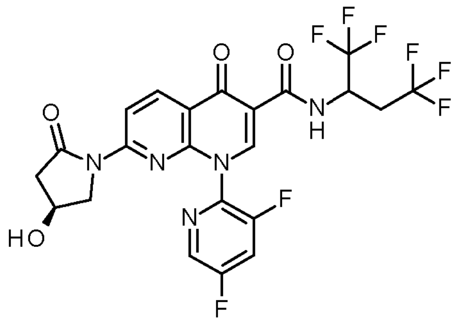

Gemäß AAV1 wurden 40.0 mg (99.4 μmol) 1-(3,5-Difluorpyridin-2-yl)-7-[(4*S*)-4-hydroxy-2-oxopyrrolidin-1-yl]-4-oxo-1,4-dihydro-1,8-naphthyridin-3-carbonsäure mit 30.3 mg (139 μmol) 1,1,1,4,4,4-Hexafluorbutan-2-amin Hydrochlorid in Gegenwart von 45.4 mg (119 μmol) HATU und 52 μl (300 μmol) DIPEA in 370 μl DMF zur Reaktion gebracht. Das Rohprodukt wurde mittels präparativer HPLC (Acetonitril / Wasser/ 0.1 % Ameisensäure) gereinigt. Es wurden 37.0 mg (63% d. Th., 95% Reinheit) der Titelverbindung erhalten.
LC-MS (Methode 2): $R_t$ = 1.74 min; MS (ESIpos): m/z = 566 [M+H]+
$^1$H-NMR (400 MHz, DMSO-d6): δ [ppm] = 10.37 (d, 1 H), 9.07 (s, 1 H), 8.71 (d, 1 H), 8.64 - 8.68 (m, 1 H), 8.55 (br t, 1 H), 8.36 - 8.42 (m, 1 H), 5.38 (br d, 0.50 H), 5.23 - 5.34 (m, 1.50 H), 4.29 (br s, 1 H), 3.62 - 3.78 (m, 1 H), 3.44 - 3.57 (m, 1 H), 3.10 - 3.22 (m, 1 H), 2.88 - 3.03 (m, 2 H), 2.34 - 2.43 (m, 1 H).

**Beispiel 160**

1-(3,5-Difluorpyridin-2-yl)-7-[(3*R*,4*R*)-3,4-dihydroxypyrrolidin-1-yl]-*N*-[1,1,1,4,4,4-hexafluorbutan-2-yl]-4-oxo-1,4-dihydro-1,8-naphthyridin-3-carboxamid (Diastereomerengemisch)

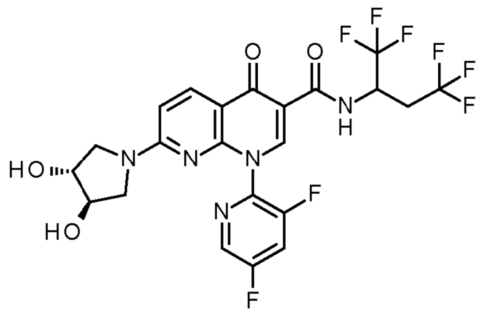

Gemäß AAV1 wurden 40.0 mg (98.9 μmol) 1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure mit 30.1 mg (138 μmol) 1,1,1,4,4,4-Hexafluorbutan-2-amin Hydrochlorid in Gegenwart von 45.1 mg (119 μmol) HATU und 52 μl (300 μmol) DIPEA in 370 μl DMF zur Reaktion gebracht. Das Rohprodukt wurde mittels präparativer HPLC (Acetonitril / Wasser/ 0.1 % Ameisensäure) gereinigt. Es wurden 22.1 mg (37% d. Th., 95% Reinheit) der Titelverbindung erhalten.
LC-MS (Methode 2): $R_t$ = 1.57 min; MS (ESIpos): m/z = 568 [M+H]+
$^1$H-NMR (400 MHz, DMSO-d6): δ [ppm] = 10.70 (d, 1 H), 8.83 (t, 1 H), 8.61 (d, 1 H), 8.30 - 8.36 (m, 1 H), 8.27 (d, 1 H), 8.20 (s, 1 H), 6.78 (d, 1 H), 4.99 - 5.33 (m, 3 H), 4.02 - 4.09 (m, 1 H), 3.88 - 3.97 (m, 1 H), 3.56 - 3.67 (m, 1 H), 3.02 - 3.19 (m, 3 H), 2.90 - 3.01 (m, 1 H).

**Beispiel 161**

1-(3,5-Difluorpyridin-2-yl)-4-oxo-7-(2-oxoimidazolidin-1-yl)-*N*-[3,3,4,4,4-pentafluorbutan-2-yl]-1,4-dihydro-1,8-naphthyridin-3 -carboxamid (Racemat)

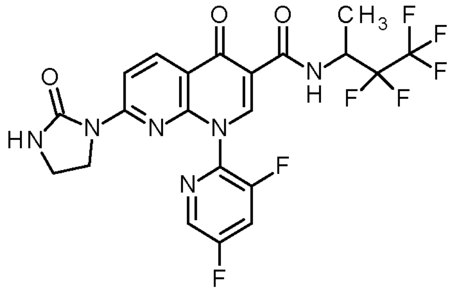

Gemäß AAV1 wurden 220 mg (568 µmol) 1-(3,5-Difluorpyridin-2-yl)-4-oxo-7-(2-oxoimidazolidin-1-yl)-1,4-dihydro-1,8-naphthyridin-3-carbonsäure mit 147 mg (738 µmol) 3,3,4,4,4-Pentafluorbutan-2-amin Hydrochlorid in Gegenwart von 302 mg (795 µmol) HATU und 430 µl (2.40 mmol) DIPEA in 5.1 ml DMF zur Reaktion gebracht. Das Rohprodukt wurde mittels präparativer HPLC (Acetonitril / Wasser/ 0.1 % Ameisensäure) gereinigt. Es wurden 165 mg (55% d. Th., 100% Reinheit nach LC-MS) der Titelverbindung erhalten.
LC-MS (Methode 1): $R_t$ = 0.98 min; MS (ESIpos): m/z = 533 [M+H]+
$^1$H-NMR (400 MHz, DMSO-d6): δ [ppm] = 10.34 (d, 1 H), 8.99 (s, 1 H), 8.63 (d, 1 H), 8.55 (d, 1 H), 8.44 (d, 1 H), 8.36-8.30 (m, 1 H), 7.67 (br s, 1 H), 6.53 (br. dd, 1H), 5.10-4.99 (m, 1 H), 4.65-4.49 (m, 1H), 3.69-3.55 (m, 2 H), 3.40-3.33 (m, 2 H), 1.41 (br d, 3 H), 1.25-1.21 (m, 3H).

(Für die Titelverbindung ist kein $EC_{50}$ berichtet, da die Reinheit nach $^1$H-NMR kleiner 50% ist.)

165 mg der Titelverbindung (Racemat) wurde durch chirale HPLC in die Enantiomere getrennt (präparative HPLC: Säule YMC Chiralart Cellulose SC 5 µm 250x20 mm; Eluent: 50% *n*-Heptan, 50% Ethanol + 0.2% DEA; Temperatur: 35°C; Fluss: 15 ml/min; UV-Detektion: 265 nm.)

Man erhielt (in der Reihenfolge der Elution von der Säule) 48.0 mg Enantiomer 1 (99% ee) Rt = 8.52 min und 50.0 mg Enantiomer 2 (97.4% ee) Rt = 9.48 min.

[Analytische HPLC: Säule Daicel Chiralpak IC 5 µm 250x4.6 mm; Eluent: 50% Iso-Hexan, 50% Ethanol + 0.2% DEA; Temperatur: 35°C; Fluss: 1.0 ml/min; UV-Detektion: 265 nm].

Enantiomer 1 wurde zusätzlich mittels präparativer HPLC (Acetonitril / Wasser/ 0.1 % Ameisensäure) gereinigt und 41.0 mg (14% d. Th., 100% Reinheit) der Titelverbindung aus Beispiel 162 erhalten.

Enantiomer 2 wurde zusätzlich mittels präparativer HPLC (Acetonitril / Wasser/ 0.1 % Ameisensäure) gereinigt und 42.0 mg (14% d. Th., 100% Reinheit) der Titelverbindung aus Beispiel 163 erhalten.

## Beispiel 162

1-(3,5-Difluorpyridin-2-yl)-4-oxo-7-(2-oxoimidazolidin-1-yl)-*N*-[3,3,4,4,4-pentafluorbutan-2-yl]-1,4-dihydro-1,8-naphthyridin-3-carboxamid (Enantiomer 1)

LC-MS (Methode 2): $R_t$ = 1.85 min; MS (ESIpos): m/z = 533 [M+H]+

$^1$H NMR (400 MHz, DMSO-$d_6$): δ ppm = 10.34 (br d, 1 H), 8.99 (br d, 1 H), 8.63 (d, 1 H), 8.55 (d, 1 H), 8.44 (d, 1 H), 8.33 (br t, 1 H), 7.67 (s, 1 H), 4.98 - 5.11 (m, 1 H), 3.54 - 3.70 (m, 2 H), 3.33 - 3.41 (m, 2 H), 1.41 (br d, 3 H).

## Beispiel 163

1-(3,5-Difluorpyridin-2-yl)-4-oxo-7-(2-oxoimidazolidin-1-yl)-*N*-[3,3,4,4,4-pentafluorbutan-2-yl]-1,4-dihydro-1,8-naphthyridin-3-carboxamid (Enantiomer 2)

LC-MS (Methode 2): $R_t$ = 1.85 min; MS (ESIpos): m/z = 533 [M+H]+

$^1$H NMR (400 MHz, DMSO-$d_6$): δ ppm = 10.34 (br d, 1 H), 8.99 (br d, 1 H), 8.63 (d, 1 H), 8.55 (d, 1 H), 8.43 (d, 1 H), 8.33 (br t, 1 H), 7.67 (s, 1 H), 4.97 - 5.11 (m, 1 H), 3.53 - 3.70 (m, 2 H), 3.32 - 3.41 (m, 2 H), 1.41 (br d, 3 H).

## Beispiel 164

1-(3,5-Difluorpyridin-2-yl)-4-oxo-7-(2-oxoimidazolidin-1-yl)-*N*-[1,1,1,2,2-pentafluorpentan-3-yl]-1,4-dihydro-1,8-naphthyridin-3 -carboxamid (Racemat)

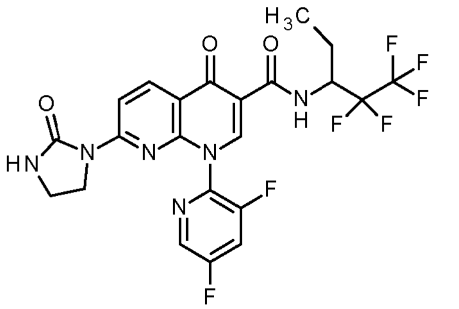

Gemäß AAV1 wurden 213 mg (550 µmol) 1-(3,5-Difluorpyridin-2-yl)-4-oxo-7-(2-oxoimidazolidin-1-yl)-1,4-dihydro-1,8-naphthyridin-3-carbonsäure mit 153 mg (715 µmol) 1,1,1,2,2-Pentafluorpentan-3-amin Hydrochlorid in Gegenwart von 293 mg (770 µmol) HATU und 410 µl (2.4 mmol) DIPEA in 4.9 ml DMF zur Reaktion gebracht. Das Rohprodukt wurde mittels präparativer HPLC (Säule: Acetonitril / Wasser/ 0.1 % Ameisensäure) gereinigt. Die Substanz wurde aus Acetonitril und DMF umkristallisiert, der Niederschlag abgesaugt, mit etwas Acetonitril nachgewaschen und nachgetrocknet. Die Substanz wurde abschließend mittels präparativer HPLC (Acetonitril / Wasser/ 0.1 % Ameisensäure) gereinigt. Es wurden 117 mg (39% d. Th., 96% Reinheit) der Titelverbindung erhalten.

LC-MS (Methode 1): $R_t$ = 1.03 min; MS (ESIpos): m/z = 547 [M+H]+

$^1$H-NMR (400 MHz, DMSO-d6): δ [ppm] = 10.26 (d, 1 H), 8.97 - 9.02 (m, 1 H), 8.63 (d, 1 H), 8.56 (d, 1 H), 8.44 (d, 1 H), 8.30 - 8.36 (m, 1 H), 7.67 (s, 1 H), 4.83 - 4.96 (m, 1 H), 3.54 - 3.70 (m, 2 H), 3.33 - 3.41 (m, 2 H), 1.88 - 1.98 (m, 1 H), 1.62 - 1.73 (m, 1 H), 0.94 - 1.00 (m, 3 H).

117 mg 1-(3,5-Difluorpyridin-2-yl)-4-oxo-7-(2-oxoimidazolidin-1-yl)-*N*-[1,1,1,2,2-pentafluorpentan-3-yl]-1,4-dihydro-1,8-naphthyridin-3-carboxamid (Racemat) wurde durch chirale HPLC in die Enantiomere getrennt (präparative HPLC: Säule YMC Chiralart Cellulose SB, 5 µm, 250 x 20 mm; Eluent: 50% *n*-Heptan/ 50% Isopropanol + 0.2% DEA; Fluss 15 ml/min; Temperatur: 30°C, Detektion: 220 nm).
Man erhielt (in der Reihenfolge der Elution von der Säule) 55.0 mg Enantiomer A (>99% ee) $R_t$ = 5.65 min und 57.0 mg Enantiomer B (96.4% ee) $R_t$ = 6.44 min. [HPLC: Säule YMC Cellulose SB, 1ml/min; 5 µm, 250 x 4.6 mm; Eluent: 50% *n*-Heptan / 50% Isopropanol + 0.2% DEA; Detektion: 265 nm].
Enantiomer A wurde zusätzlich mittels präparativer HPLC (Säule: Acetonitril / Wasser / 0.1 % Ameisensäure) gereinigt und 42.0 mg (100% Reinheit) der Titelverbindung aus Beispiel 165 erhalten.
Enantiomer B wurde zusätzlich mittels präparativer HPLC (Säule: Acetonitril / Wasser / 0.1 % Ameisensäure) gereinigt und 40.0 mg (100% Reinheit) der Titelverbindung aus Beispiel 166 erhalten.

## Beispiel 165

1-(3,5-Difluorpyridin-2-yl)-4-oxo-7-(2-oxoimidazolidin-1-yl)-*N*-[1,1,1,2,2-pentafluorpentan-3-yl]-1,4-dihydro-1,8-naphthyridin-3-carboxamid (Enantiomer A)

LC-MS (Methode 2): $R_t$ = 1.98 min; MS (ESIpos): m/z = 547 $[M+H]^+$

$^1$H-NMR (400 MHz, DMSO-d6) δ [ppm]: -0.149 (0.60), -0.008 (5.43), 0.008 (5.16), 0.146 (0.60), 0.948 (4.38), 0.965 (10.46), 0.977 (11.06), 0.995 (5.03), 1.637 (1.34), 1.655 (1.87), 1.663 (1.71), 1.671 (2.27), 1.681 (2.11), 1.690 (1.91), 1.698 (2.23), 1.716 (1.58), 1.734 (0.45), 1.931 (2.00), 2.329 (0.71), 2.367 (0.69), 2.524 (2.58), 2.671 (0.80), 2.711 (0.80), 3.342 (5.10), 3.361 (5.16), 3.383 (3.27), 3.552 (1.00), 3.578 (2.96), 3.595 (2.98), 3.618 (1.89), 3.632 (2.18), 3.655 (3.16), 3.672 (2.47), 3.698 (0.71), 4.854 (1.31), 4.880 (1.74), 4.906 (1.76), 4.931 (1.29), 7.671 (11.17), 8.310 (2.27), 8.331 (4.25), 8.354 (2.23), 8.429 (11.53), 8.452 (16.00), 8.549 (15.98), 8.572 (11.33), 8.630 (10.19), 8.636 (9.75), 8.995 (7.23), 8.999 (7.61), 10.247 (6.25), 10.272 (6.05).

## Beispiel 166

1-(3,5-Difluorpyridin-2-yl)-4-oxo-7-(2-oxoimidazolidin-1-yl)-*N*-[1,1,1,2,2-pentafluorpentan-3-yl]-1,4-dihydro-1,8-naphthyridin-3-carboxamid (Enantiomer B)

LC-MS (Methode 2): $R_t$ = 1.99 min; MS (ESIpos): m/z = 547 $[M+H]^+$

$^1$H-NMR (400 MHz, DMSO-d6) δ [ppm]: -0.008 (2.79), 0.008 (2.51), 0.948 (4.46), 0.966 (10.56), 0.977 (11.21), 0.995 (5.10), 1.637 (1.35), 1.655 (1.92), 1.663 (1.73), 1.671 (2.32), 1.681 (2.12), 1.690 (1.92), 1.698 (2.24), 1.716 (1.61), 1.734 (0.47), 1.933 (2.02), 2.074 (1.14), 2.329 (0.49), 2.368 (0.47), 2.671 (0.61), 2.711 (0.51), 3.342 (5.10), 3.361 (5.24), 3.384 (3.36), 3.553 (0.96), 3.578 (3.06), 3.595 (3.04), 3.618 (1.92), 3.633 (2.24), 3.655 (3.22), 3.673 (2.51), 3.699 (0.71),

4.855 (1.33), 4.880 (1.79), 4.906 (1.77), 4.932 (1.32), 7.671 (11.39), 8.310 (2.30), 8.330 (4.28), 8.354 (2.24), 8.429 (11.43), 8.452 (16.00), 8.549 (15.71), 8.572 (11.19), 8.630 (10.19), 8.636 (9.78), 8.995 (7.19), 9.000 (7.58), 10.247 (6.36), 10.272 (6.16).

## Beispiel 167

*N*-[(1*S*)-1-Cyclopropyl-2,2,2-trifluorethyl]-1-(3,5-difluorpyridin-2-yl)-7-[3-(2-hydroxyethyl)-2-oxoimidazolidin-1-yl]-4-oxo-1,4-dihydro-1,8-naphthyridin-3-carbonsäureamid

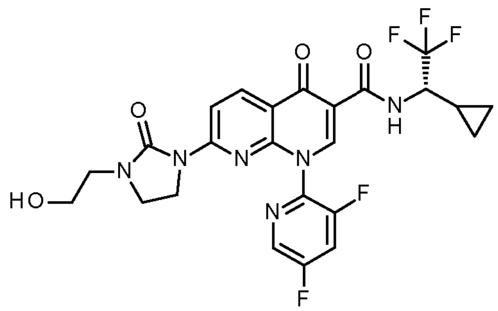

Gemäß AAV 1 wurden 40.0 mg (92.7 μmol, 100% Reinheit) 1-(3,5-Difluorpyridin-2-yl)-7-[3-(2-hydroxyethyl)-2-oxoimidazolidin-1-yl]-4-oxo-1,4-dihydro-1,8-naphthyridin-3-carbonsäure (Beispiel 25A) mit 19.5 mg (111 μmol) (1*S*)-1-Cyclopropyl-2,2,2-trifluorethanamin Hydrochlorid in Gegenwart von 42.3 mg (111 μmol) HATU und 65.0 μl (371 μmol) DIPEA in 0.6 ml DMF zur Reaktion gebracht.

Das Reaktionsgemisch wurde mit Wasser, Acetonitril und Ameisensäure verdünnt, über einen Milliporefilter filtriert und mittels präparativer HPLC (Acetonitril / Wasser/ 0.1 % Ameisensäure) gereinigt und 35.3 mg (69% d. Th., 100% Reinheit) der Titelverbindung erhalten.

LC-MS (Methode 2): $R_t$ = 1.77 min; MS (ESIpos): m/z = 553 [M+H]+.

$^1$H-NMR (400 MHz, DMSO-d6): δ [ppm] = 10.34 (br d, 1H), 8.98 (s, 1H), 8.64 (d, 1H), 8.57 (d, 1H), 8.45 (d, 1H), 8.33 (ddd, 1H), 4.75 (t, 1H), 4.42 (sxt, 1H), 3.44-3.62 (m, 6H), 3.22-3.30 (m, 2H), 1.19-1.27 (m, 1H), 0.51-0.70 (m, 3H), 0.30-0.39 (m, 1H).

## Beispiel 168

*N*-Cyclobutyl-1-(3,5-difluorpyridin-2-yl)-7-[(3*R*,4*R*)-3,4-dihydroxypyrrolidin-1-yl]-4-oxo-1,4-dihydro-1,8-naphthyridin-3-carboxamid

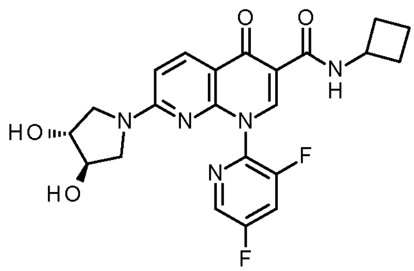

Gemäß AAV1 wurden 20.7 mg (51.2 μmol) 1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure mit 6.61 mg (61.4 μmol) Cyclobutanamin Hydrochlorid in Gegenwart von 23.4 mg (61.4 μmol) HATU und 27 μl (150 μmol) DIPEA in 200 μl DMF zur Reaktion gebracht. Das Rohprodukt wurde zweimal mittels präparativer HPLC (Säule: Acetonitril / Wasser / 0.1 % Ameisensäure) gereinigt. Es wurden 7.90 mg (34% d. Th., 100% Reinheit) der Titelverbindung erhalten.

LC-MS (Methode 1): $R_t$ = 0.71 min; MS (ESIpos): m/z = 458 $[M+H]^+$

$^1$H-NMR (400 MHz, DMSO-d6) δ [ppm]: -0.149 (2.35), 0.146 (2.30), 0.948 (0.81), 0.956 (1.15), 0.973 (1.10), 1.235 (0.86),

1.658 (1.63), 1.677 (3.40), 1.683 (3.21), 1.703 (6.66), 1.721 (7.95), 1.728 (6.37), 1.744 (4.55), 1.943 (4.74), 1.965 (6.56), 1.987 (4.41), 2.073 (6.18), 2.086 (2.54), 2.281 (7.86), 2.291 (6.90), 2.300 (7.33), 2.319 (3.45), 2.327 (3.50), 2.367 (1.05), 2.670 (1.72), 2.710 (1.05), 3.033 (2.49), 3.065 (3.11), 3.111 (1.87), 3.144 (3.21), 3.208 (2.87), 3.582 (2.40), 3.610 (3.35), 3.637 (1.82), 3.924 (6.61), 4.049 (6.85), 4.379 (0.67), 4.398 (2.83), 4.419 (5.41), 4.439 (5.32), 4.459 (2.78), 4.479 (0.72), 5.063 (1.72), 5.187 (3.02), 5.266 (1.68), 6.739 (14.04), 6.761 (14.42), 8.252 (15.19), 8.274 (16.00), 8.302 (3.45), 8.324 (6.04), 8.341 (3.21), 8.346 (3.31), 8.607 (13.56), 8.613 (13.08), 8.710 (9.39), 8.716 (10.78), 10.110 (9.39), 10.129 (9.20).

**Beispiel 169**

1-(3,5-Difluorpyridin-2-yl)-4-oxo-7-(2-oxoimidazolidin-1-yl)-*N*-[l,1,1-trifluor-3-methylbutan-2-yl]-1,4-dihydro-1,8-naphthyridin-3-carboxamid (Racemat)

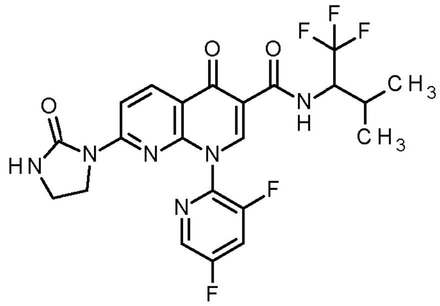

Gemäß AAV1 wurden 80.0 mg (207 µmol) 1-(3,5-Difluorpyridin-2-yl)-4-oxo-7-(2-oxoimidazolidin-1-yl)-1,4-dihydro-1,8-naphthyridin-3-carbonsäure mit 58.3 mg (413 µmol) 1,1,1-Trifluor-3-methylbutan-2-amin in Gegenwart von 157 mg (413 µmol) HATU und 140 µl (830 µmol) DIPEA in 1.4 ml DMF zur Reaktion gebracht. Das Rohprodukt wurde mittels präparativer HPLC (Säule: Acetonitril / Wasser / 0.1% Ameisensäure) gereinigt. Es wurden 33.7 mg (32% d. Th., 100% Reinheit) der Titelverbindung erhalten.

LC-MS (Methode 2): $R_t$ = 1.90 min; MS (ESIpos): m/z = 511 $[M+H]^+$

$^1H$-NMR (400 MHz, DMSO-d6) δ [ppm]: 0.889 (9.48), 0.899 (9.69), 0.906 (10.59), 0.915 (8.34), 0.959 (8.58), 0.972 (13.09), 0.986 (8.58), 1.032 (12.36), 1.048 (12.28), 2.074 (3.55), 2.093 (0.98), 2.109 (0.62), 2.237 (1.99), 2.254 (2.67), 2.263 (2.63), 2.281 (1.86), 2.328 (0.79), 2.367 (0.45), 2.671 (0.73), 3.342 (5.03), 3.362 (4.86), 3.385 (2.99), 3.582 (2.72), 3.598 (2.78), 3.622 (1.84), 3.635 (2.12), 3.656 (2.99), 3.674 (2.29), 4.254 (0.77), 4.266 (0.81), 4.278 (1.07), 4.288 (0.92), 4.301 (0.79), 4.768 (1.63), 4.777 (1.80), 4.791 (2.42), 4.800 (2.48), 4.813 (1.73), 4.823 (1.62), 6.568 (0.79), 6.584 (1.41), 6.609 (1.26), 7.673 (9.80), 8.307 (2.08), 8.313 (2.40), 8.334 (4.13), 8.351 (2.31), 8.357 (2.37), 8.432 (8.13), 8.455 (11.06), 8.574 (10.57), 8.597 (8.06), 8.637 (8.19), 8.642 (7.94), 9.002 (16.00), 10.408 (3.08), 10.426 (3.12).

**Beispiel 170**

*N*(3,3-Difluor-1-methylcyclobutyl)-1-(3,5-difluorpyridin-2-yl)-7-[(3*R*,4*R*)-3,4-dihydroxypyrrolidin-1-yl]-4-oxo-1,4-dihydro-1,8-naphthyridin-3-carboxamid

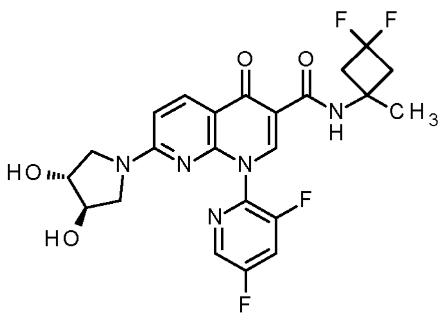

Gemäß AAV1 wurden 60.0 mg (148 µmol) 1-(3,5-Difluorpyridin-2-yl)-7-[(3*R*,4*R*)-3,4-dihydroxypyrrolidin-1-yl]-4-oxo-1,4-dihydro-1,8-naphthyridin-3-carbonsäure mit 28.1 mg (178 μmol) 3,3-Difluor-l-methylcyclobutanamin Hydrochlorid in Gegenwart von 67.7 mg (178 μmol) HATU und 90 μl (520 μmol) DIPEA in 660 μl DMF zur Reaktion gebracht. Das Rohprodukt wurde mittels präparativer HPLC (Säule: Acetonitril / Wasser / 0.1 % Ameisensäure) gereinigt. Es wurden 45.5 mg (60% d. Th., 100% Reinheit) der Titelverbindung erhalten.
LC-MS (Methode 2): $R_t$ = 1.43 min; MS (ESIpos): m/z = 508 $[M+H]^+$
$^1$H-NMR (400 MHz, DMSO-d6) δ [ppm]: 1.555 (16.00), 2.073 (3.51), 2.328 (0.71), 2.665 (1.27), 2.697 (1.92), 2.721 (1.91), 2.753 (0.98), 2.977 (1.00), 3.013 (2.65), 3.047 (2.62), 3.147 (1.17), 3.330 (5.13), 3.613 (0.99), 3.925 (1.94), 4.051 (1.96), 5.207 (0.48), 6.746 (4.28), 6.769 (4.23), 8.216 (1.05), 8.250 (5.12), 8.272 (4.71), 8.309 (1.01), 8.331 (1.78), 8.348 (0.94), 8.612 (4.56), 8.618 (4.23), 8.735 (3.84), 8.741 (4.17), 10.275 (5.29).

**Beispiel 171**

*N*-(3,3-Difluor-1-methylcyclobutyl)-1-(3,5-difluorpyridin-2-yl)-4-oxo-7-(2-oxoimidazolidin-1-yl)-1,4-dihydro-1,8-naphthyridin-3-carboxamid

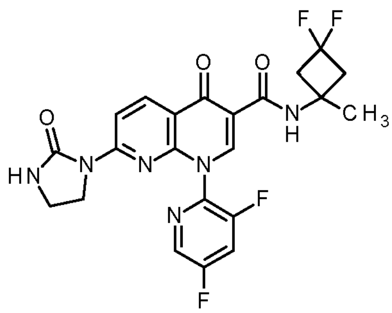

Gemäß AAV1 wurden 50.0 mg (129 μmol) 1-(3,5-Difluorpyridin-2-yl)-4-oxo-7-(2-oxoimidazolidin-1-yl)-1,4-dihydro-1,8-naphthyridin-3-carbonsäure mit 40.7 mg (258 μmol) 3,3-Difluor-1-methylcyclobutanamin Hydrochlorid in Gegenwart von 98.2 mg (258 μmol) HATU und 90 μl (520 μmol) DIPEA in 750 μl DMF zur Reaktion gebracht. Das Rohprodukt wurde mittels präparativer HPLC (Säule: Acetonitril / Wasser / 0.1 % Ameisensäure) gereinigt. Es wurden 17.6 mg (26% d. Th., 95% Reinheit) der Titelverbindung erhalten.
LC-MS (Methode 2): $R_t$ = 1.69 min; MS (ESIpos): m/z = 491 $[M+H]^+$
$^1$H-NMR (400 MHz, DMSO-d6) δ [ppm]: -0.008 (2.82), 0.008 (2.53), 1.382 (16.00), 1.567 (12.75), 2.073 (2.14), 2.328 (0.62), 2.446 (1.00), 2.465 (1.20), 2.670 (0.81), 2.679 (0.92), 2.712 (1.75), 2.737 (1.51), 2.770 (0.79), 2.821 (1.05), 2.856 (2.83), 2.893 (2.63), 2.929 (0.84), 2.989 (0.73), 3.023 (2.00), 3.059 (1.79), 3.093 (0.58), 3.356 (1.59), 3.378 (0.96), 3.573 (0.83), 3.589 (0.88), 3.625 (0.66), 3.648 (0.94), 3.664 (0.71), 6.237 (2.55), 7.649 (3.22), 8.301 (0.82), 8.307 (0.95), 8.328 (1.39), 8.345 (0.82), 8.351 (0.90), 8.410 (3.70), 8.432 (5.00), 8.527 (5.13), 8.549 (3.54), 8.631 (4.29), 8.638 (4.07), 8.911 (8.53), 10.081 (4.04).

**Beispiel 172**

*N*-(3,3-Difluor-1-methylcyclobutyl)-1-(3,5-difluorpyridin-2-yl)-7-[(4*S*)-4-hydroxy-2-oxopyrrolidin-1-yl]-4-oxo-1,4-dihydro-1,8-naphthyridin-3-carboxamid

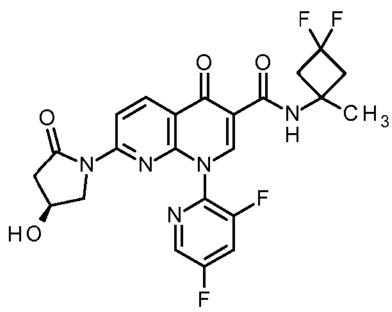

Gemäß AAV1 wurden 50.0 mg (124 μmol) 1-(3,5-Difluorpyridin-2-yl)-7-[(4*S*)-4-hydroxy-2-oxopyrrolidin-1-yl]-4-oxo-1,4-dihydro-1,8-naphthyridin-3-carbonsäure mit 23.5 mg (149 μmol) 3,3-Difluor-l-methylcyclobutanamin Hydrochlorid in Gegenwart von 56.7 mg (149 μmol) HATU und 87 μl (500 μmol) DIPEA in 750 μl DMF zur Reaktion gebracht. Das Rohprodukt wurde mittels präparativer HPLC (Säule: Acetonitril / Wasser / 0.1 % Ameisensäure) gereinigt. Es wurden 37.7 mg (60% d. Th., 100% Reinheit) der Titelverbindung erhalten.
LC-MS (Methode 2): $R_t$ = 1.63 min; MS (ESIpos): m/z = 506 $[M+H]^+$
$^1$H-NMR (400 MHz, DMSO-d6) δ [ppm]: -0.008 (0.93), 0.008 (0.87), 1.572 (16.00), 2.074 (0.87), 2.344 (0.67), 2.368 (0.78), 2.387 (0.79), 2.411 (0.73), 2.524 (0.72), 2.685 (0.81), 2.721 (1.81), 2.744 (1.82), 2.755 (1.25), 2.778 (0.94), 2.888 (0.46), 2.902 (0.49), 2.931 (0.46), 2.950 (0.65), 2.967 (0.53), 2.991 (1.16), 3.025 (2.48), 3.060 (2.21), 3.095 (0.69), 3.460 (0.60), 3.490 (0.77), 3.519 (0.57), 3.549 (0.74), 3.657 (0.48), 3.668 (0.56), 3.687 (0.42), 3.699 (0.40), 3.728 (0.53), 3.738 (0.55), 3.757 (0.44), 4.292 (1.37), 5.253 (0.96), 5.260 (0.96), 5.373 (0.92), 5.382 (0.88), 8.361 (0.81), 8.367 (0.92), 8.388 (1.53), 8.406 (0.85), 8.412 (0.89), 8.504 (0.74), 8.525 (1.66), 8.547 (1.03), 8.664 (2.25), 8.681 (4.96), 8.704 (3.86), 8.977 (5.82), 9.999 (4.93).

## Beispiel 173

1-(3,5-Difluorpyridin-2-yl)-7-[(2R)-2-(hydroxymethyl)piperidin-1-yl]-4-oxo-N-(3,3,4,4,4-pentafluor-2-methylbutan-2-yl)-1,4-dihydro-1,8-naphthyridin-3-carboxamid

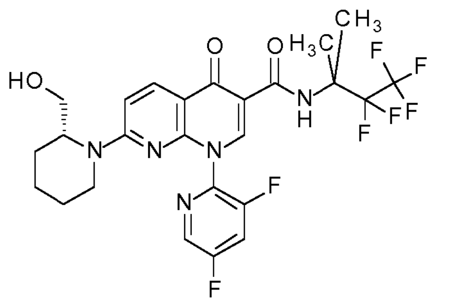

Es wurde 7-Chlor-1-(3,5-difluorpyridin-2-yl)-4-oxo-*N*-(3,3,4,4,4-pentafluor-2-methylbutan-2-yl)-1,4-dihydro-1,8-naphthyridin-3-carboxamid (50.0 mg, 101 μmol) in 1.1 ml DMF gelöst, (2R)-Piperidin-2-ylmethanol (12.8 mg, 111 μmol) und N,N-Diisopropylethylamin (61 μl, 350 μmol) hinzugefügt und für 18 h bei 55°C nachgerührt. Es wurde nochmals (2R)-Piperidin-2-ylmethanol (11.6 mg, 101 μmol) und N,N-Diisopropylethylamin (18 μl, 100 μmol) hinzugefügt und über Nacht bei 55°C nachgerührt. Das Reaktionsgemisch wurde abgekühlt und mittels präparativer HPLC (Säule: Acetonitril / Wasser / 0.1 % Ameisensäure) gereinigt. Es wurden 40.8 mg (70% d. Th., 99% Reinheit) der Titelverbindung erhalten.
LC-MS (Methode 3): $R_t$ = 2.17 min; MS (ESIpos): m/z = 576 $[M+H]^+$
$^1$H-NMR (400 MHz, DMSO-d6) δ [ppm]: -0.008 (1.61), 0.008 (1.62), 1.510 (1.42), 1.547 (1.15), 1.577 (0.84), 1.612 (0.70), 1.682 (16.00), 1.778 (1.06), 1.810 (0.94), 3.503 (0.99), 3.516 (0.93), 4.049 (0.81), 4.081 (0.77), 4.225 (0.92), 4.690 (1.00), 7.075 (3.25), 7.099 (3.39), 8.231 (2.79), 8.254 (2.91), 8.278 (0.55), 8.298 (0.60), 8.322 (0.60), 8.602 (4.13), 8.608 (4.02), 8.756 (6.43), 10.638 (5.18).

## Beispiel 174

1-(3,5-Difluorpyridin-2-yl)-7-[4-hydroxy-4-(hydroxymethyl)piperidin-1-yl]-4-oxo-N-(3,3,4,4,4-pentafluor-2-methylbutan-2-yl)-1,4-dihydro-1,8-naphthyridin-3-carboxamid

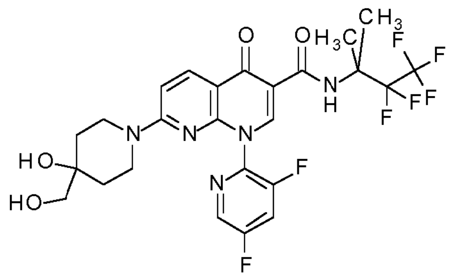

Es wurde 7-Chlor-1-(3,5-difluorpyridin-2-yl)-4-oxo-*N*-(3,3,4,4,4-pentafluor-2-methylbutan-2-yl)-1,4-dihydro-1,8-naphthyridin-3-carboxamid (50.0 mg, 101 μmol) in 1.1 ml DMF gelöst, 4-(Hydroxymethyl)piperidin-4-olhydrochlorid (1:1) (19.5 mg, 95 % Reinheit, 111 μmol) und N,N-Diisopropylethylamin (79 μl, 450 μmol) hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 49 mg (82% d. Th., 100% Reinheit) der Titelverbindung erhalten.
LC-MS (Methode 3): $R_t$ = 1.82 min; MS (ESIpos): m/z = 592 $[M+H]^+$
$^1$H-NMR (400 MHz, DMSO-d6) δ [ppm]: 1.337 (1.29), 1.371 (1.16), 1.469 (0.99), 1.492 (1.08), 1.520 (0.53), 1.681 (16.00), 2.328 (0.48), 2.670 (0.47), 3.148 (4.54), 3.162 (4.61), 3.915 (1.39), 3.942 (1.25), 4.319 (5.79), 4.554 (1.11), 4.568 (2.37), 4.583 (1.07), 7.111 (3.27), 7.134 (3.41), 8.249 (4.45), 8.272 (4.15), 8.313 (0.90), 8.319 (1.07), 8.340 (1.60), 8.358 (0.95), 8.364 (1.03), 8.611 (4.88), 8.617 (4.69), 8.764 (10.14), 10.621 (5.45).

## Beispiel 175

1-(3,5-Difluorpyridin-2-yl)-7-[(2R)-2-(hydroxymethyl)pyrrolidin-1-yl]-4-oxo-*N*-(3,3,4,4,4-pentafluorbutan-2-yl)-1,4-dihydro-1,8-naphthyridin-3-carboxamid (Enantiomerenrein)

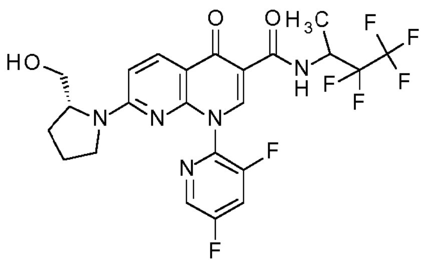

Es wurde 7-Chlor-1-(3,5-difluorpyridin-2-yl)-4-oxo-*N*-[3,3,4,4,4-pentafluorbutan-2-yl]-1,4-dihydro-1,8-naphthyridin-3-carboxamid (50.0 mg, 104 μmol) in 1.1 ml DMF gelöst, (2R)-Pyrrolidin-2-ylmethanol (11 μl, 99 % Reinheit, 110 μmol) und N,N-Diisopropylethylamin (63 μl, 360 μmol) hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 52 mg (92% d. Th., 100% Reinheit) der Titelverbindung erhalten.
LC-MS (Methode 3): $R_t$ = 1.98 min; MS (ESIpos): m/z = 548 $[M+H]^+$
$^1$H-NMR (400 MHz, DMSO-d6) δ [ppm]: 1.387 (15.87), 1.404 (16.00), 1.953 (3.83), 2.328 (1.78), 2.366 (0.61), 2.670 (1.96), 2.710 (0.61), 3.015 (1.22), 3.167 (1.04), 3.487 (1.17), 3.652 (1.04), 3.768 (0.87), 4.019 (0.70), 4.383 (1.00), 4.562 (1.22), 4.973 (1.09), 4.993 (1.78), 5.018 (2.04), 5.039 (2.04), 5.063 (1.74), 5.082 (0.91), 6.742 (1.96), 6.762 (2.04), 6.914 (0.91), 8.258 (4.52), 8.279 (5.17), 8.305 (2.65), 8.591 (13.61), 8.597 (13.48), 8.824 (9.26), 10.541 (4.70), 10.564 (4.61).

## Beispiel 176

1-(3,5-Difluorpyridin-2-yl)-7-[(2R)-2-(hydroxymethyl)piperidin-1-yl]-4-oxo-N-(3,3,4,4,4-pentafluorbutan-2-yl)-1,4-dihydro-1,8-naphthyridin-3-carboxamid (enantiomerenrein)

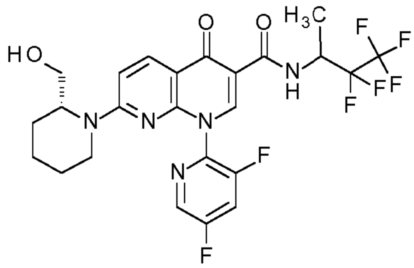

Es wurde 7-Chlor-1-(3,5-difluorpyridin-2-yl)-4-oxo-N-[3,3,4,4,4-pentafluorbutan-2-yl]-1,4-dihydro-1,8-naphthyridin-3-carboxamid (50.0 mg, 104 µmol) in 1.1 ml DMF gelöst, (2R)-Piperidin-2-ylmethanol (13.1 mg, 114 µmol) und N,N-Diisopropylethylamin (63 µl, 360 µmol) hinzugefügt und für 18 h bei 55°C nachgerührt. Es wurde nochmals (2R)-Piperidin-2-ylmethanol (11.9 mg, 104 µmol) und N,N-Diisopropylethylamin (18 µl, 100 µmol) hinzugefügt und über Nacht bei 55°C nachgerührt. Das Reaktionsgemisch wurde abgekühlt und mittels präparativer HPLC (Säule: Acetonitril / Wasser / 0.1 % Ameisensäure) gereinigt. Es wurden 39 mg (66% d. Th., 99% Reinheit) der Titelverbindung erhalten.
LC-MS (Methode 3): $R_t$ = 2.07 min; MS (ESIpos): m/z = 562 $[M+H]^+$
$^1$H-NMR (400 MHz, DMSO-d6) δ [ppm]: -0.149 (0.39), 0.146 (0.41), 1.314 (1.37), 1.342 (1.52), 1.387 (16.00), 1.404 (16.00), 1.504 (4.80), 1.549 (3.76), 1.579 (2.65), 1.614 (2.21), 1.647 (1.48), 1.780 (3.39), 1.809 (3.03), 2.328 (0.69), 2.670 (0.79), 2.831 (1.18), 3.503 (3.17), 3.519 (3.00), 4.048 (2.68), 4.079 (2.54), 4.228 (3.12), 4.694 (3.09), 4.971 (0.92), 4.992 (1.65), 5.015 (1.95), 5.036 (1.95), 5.060 (1.67), 5.080 (0.88), 7.086 (9.59), 7.109 (9.99), 8.223 (8.59), 8.246 (8.73), 8.273 (1.76), 8.303 (1.51), 8.330 (1.73), 8.348 (0.91), 8.608 (9.36), 8.807 (12.83), 10.531 (7.24), 10.555 (6.98).

**Beispiel 177**

1-(3,5-Difluorpyridin-2-yl)-7-[4-hydroxy-4-(hydroxymethyl)piperidin-1-yl]-4-oxo-N-(3,3,4,4,4-pentafluorbutan-2-yl)-1,4-dihydro-1,8-naphthyridin-3-carboxamid (enantiomerenrein)

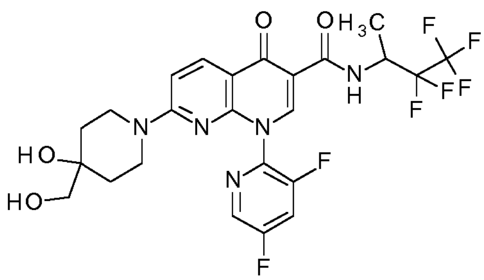

Es wurde 7-Chlor-1-(3,5-difluorpyridin-2-yl)-4-oxo-N-[3,3,4,4,4-pentafluorbutan-2-yl]-1,4-dihydro-1,8-naphthyridin-3-carboxamid (50.0 mg, 104 µmol) in 1.1 ml DMF gelöst, 4-(Hydroxymethyl)piperidin-4-olhydrochlorid (1:1) (20.1 mg, 95 % Reinheit, 114 µmol) und N,N-Diisopropylethylamin (63 µl, 360 µmol) hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurde nochmals mittels präparativer HPLC (Säule: Acetonitril / Wasser / 0.1 % Ameisensäure) gereinigt. Die produkthaltigen Fraktionen wurden vereinigt, eingedampft und aus Wasser/Acetonitril lyophilisiert. Es wurden 17.2 mg (28% d. Th., 99% Reinheit) der Titelverbindung erhalten.
LC-MS (Methode 3): $R_t$ = 1.72 min; MS (ESIpos): m/z = 578 $[M+H]^+$
$^1$H-NMR (400 MHz, DMSO-d6) δ [ppm]: -0.149 (0.79), -0.008 (9.63), 0.008 (6.77), 0.146 (0.79), 0.936 (0.62), 0.953 (0.62), 1.340 (3.98), 1.386 (14.49), 1.403 (13.31), 1.459 (2.99), 1.492 (3.19), 1.524 (1.38), 2.328 (1.25), 2.670 (1.28), 3.149 (13.21), 3.163 (13.17), 3.917 (4.24), 3.941 (3.68), 4.322 (16.00), 4.555 (3.32), 4.569 (6.67), 4.583 (3.02), 4.971 (0.89), 4.994 (1.51), 5.013 (1.71), 5.037 (1.68), 5.060 (1.41), 5.081 (0.72), 7.121 (9.03), 7.144 (9.17), 8.242 (12.42), 8.264 (11.33), 8.319 (2.27), 8.341 (3.81), 8.363 (2.07), 8.613 (9.03), 8.619 (8.51), 8.808 (6.51), 8.816 (6.97), 10.516 (5.03), 10.540 (4.86).

**Beispiel 178**

1-(3,5-Difluorpyridin-2-yl)-N-(1,1,1,3,3,3-hexafluorpropan-2-yl)-7-[(2R)-2-(hydroxymethyl)piperidin-1-yl]-4-oxo-1,4-dihydro-1,8-naphthyridin-3-carboxamid

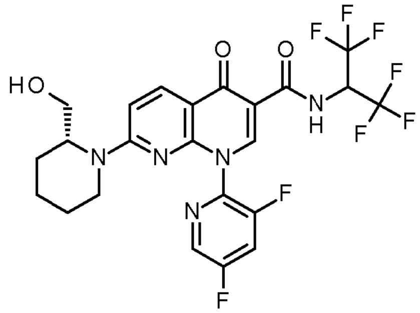

Es wurde 7-Chlor-1-(3,5-difluorpyridin-2-yl)-N-(1,1,1,3,3,3-hexafluorpropan-2-yl)-4-oxo-1,4-dihydro-1,8-naphthyridin-3-carboxamid (50.0 mg, 103 µmol) in 1 ml DMF gelöst, (2R)-Piperidin-2-ylmethanol (13.0 mg, 113 µmol) und N,N-Diisopropylethylamin (63 µl, 360 µmol) hinzugefügt und über das Wochenende bei 55°C nachgerührt. Das Reaktionsgemisch wurde abgekühlt und mittels präparativer HPLC (Säule: Acetonitril / Wasser / 0.1 % Ameisensäure) gereinigt. Die produkthaltigen Fraktionen wurden vereinigt, eingedampft und aus Wasser/Acetonitril lyophilisiert. Es wurden 33.4 mg (57% d. Th., 99% Reinheit) der Titelverbindung erhalten.
LC-MS (Methode 3): $R_t$ = 2.13 min; MS (ESIpos): m/z = 566 $[M+H]^+$
$^1$H-NMR (400 MHz, DMSO-d6) δ [ppm]: -0.149 (0.80), -0.008 (7.72), 0.008 (8.00), 0.146 (0.82), 1.315 (1.38), 1.505 (5.30), 1.551 (3.89), 1.581 (2.75), 1.617 (2.23), 1.782 (3.63), 1.810 (3.26), 2.328 (1.02), 2.671 (1.17), 2.871 (1.17), 3.508 (3.74), 3.522 (3.57), 4.057 (2.83), 4.089 (2.79), 4.239 (3.26), 4.702 (3.52), 6.276 (0.76), 6.294 (1.97), 6.319 (2.79), 6.337 (2.96), 6.355 (1.95), 7.115 (11.76), 7.139 (12.41), 8.247 (10.66), 8.270 (10.94), 8.295 (1.77), 8.316 (2.14), 8.342 (2.23), 8.361 (1.21), 8.619 (16.00), 8.626 (15.72), 8.900 (15.11), 11.382 (9.10), 11.407 (8.71).

**Beispiel 179**

1-(3,5-Difluorpyridin-2-yl)-N-(1,1,1,3,3,3-hexafluorpropan-2-yl)-7-[4-hydroxy-4-hydroxymethyl)piperidin-1-yl]-4-oxo-1,4-dihydro-1,8-naphthyridin-3-carboxamid

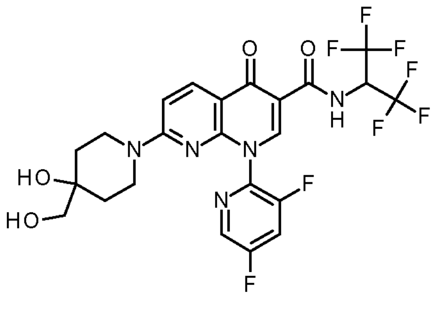

Es wurde 7-Chlor-1-(3,5-difluorpyridin-2-yl)-N-(1,1,1,3,3,3-hexafluorpropan-2-yl)-4-oxo-1,4-dihydro-1,8-naphthyridin-3-carboxamid (50.0 mg, 103 µmol) in 1 ml DMF gelöst, 4-(Hydroxymethyl)piperidin-4-olhydrochlorid (1:1) (19.9 mg, 95 % Reinheit, 113 µmol) und N,N-Diisopropylethylamin (81 µl, 460 µmol) hinzugefügt und über das Wochenende bei 55°C nachgerührt. Das Reaktionsgemisch wurde abgekühlt und mittels präparativer HPLC (Säule: Acetonitril / Wasser / 0.1 % Ameisensäure) gereinigt. Die produkthaltigen Fraktionen wurden vereinigt, eingedampft und aus Wasser/Acetonitril lyophilisiert. Es wurden 31.5 mg (52% d. Th., 99% Reinheit) der Titelverbindung erhalten.
LC-MS (Methode 3): $R_t$ = 1.80 min; MS (ESIpos): m/z = 582 $[M+H]^+$
$^1$H-NMR (400 MHz, DMSO-d6) δ [ppm]: -0.149 (0.70), 0.146 (0.72), 1.350 (2.52), 1.382 (2.54), 1.475 (2.14), 1.499 (2.32), 1.529 (1.10), 2.329 (0.85), 2.368 (0.66), 2.671 (0.98), 2.711 (0.72), 3.153 (8.93), 3.167 (9.21), 3.931 (3.22), 3.961 (2.80),

4.334 (10.99), 4.563 (2.14), 4.577 (4.38), 4.591 (2.14), 6.296 (1.09), 6.320 (1.64), 6.338 (1.71), 6.357 (1.16), 7.150 (6.04), 7.173 (6.32), 8.266 (7.77), 8.288 (7.29), 8.332 (1.71), 8.337 (2.01), 8.359 (3.41), 8.376 (1.84), 8.382 (2.01), 8.628 (8.38), 8.634 (8.49), 8.907 (16.00), 11.365 (5.14), 11.390 (4.93).

**Beispiel 180**

1-(3,5-Difluorpyridin-2-yl)-N-(1,1,1,3,3,3-hexafluorpropan-2-yl)-7-[(2R)-2-(hydroxymethyl)pyrrolidin-1-yl]-4-oxo-1,4-dihydro-1,8-naphthyridin-3-carboxamid

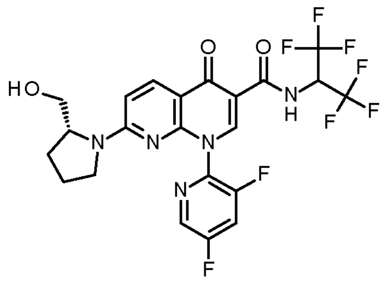

Es wurde 7-Chlor-1-(3,5-difluorpyridin-2-yl)-*N*-(1,1,1,3,3,3-hexafluorpropan-2-yl)-4-oxo-1,4-dihydro-1,8-naphthyridin-3-carboxamid (50.0 mg, 103 μmol) in 1 ml DMF gelöst, (2R)-Pyrrolidin-2-ylmethanol (11 μl, 99 % Reinheit, 110 μmol) und N,N-Diisopropylethylamin (63 μl, 360 μmol) hinzugefügt und über das Wochenende bei 55°C nachgerührt. Das Reaktionsgemisch wurde abgekühlt und mittels präparativer HPLC (Säule: Acetonitril / Wasser / 0.1 % Ameisensäure) gereinigt. Die produkthaltigen Fraktionen wurden vereinigt, eingedampft und aus Wasser/Acetonitril lyophilisiert. Es wurden 34 mg (59% d. Th., 99% Reinheit) der Titelverbindung erhalten.
$^1$H-NMR (400 MHz, DMSO-d6) δ [ppm]: -0.149 (1.69), -0.008 (15.45), 0.008 (16.00), 0.146 (1.69), 1.958 (3.28), 2.328 (1.31), 2.366 (0.55), 2.670 (1.47), 2.710 (0.60), 3.011 (1.09), 3.046 (0.98), 3.158 (0.98), 3.521 (1.04), 3.654 (0.87), 3.774 (0.82), 4.039 (0.55), 4.392 (1.04), 4.570 (1.20), 4.881 (0.60), 4.946 (0.44), 6.295 (1.58), 6.314 (2.29), 6.338 (2.40), 6.356 (1.58), 6.772 (1.86), 6.792 (2.02), 6.961 (0.87), 8.234 (0.93), 8.286 (3.93), 8.309 (3.93), 8.606 (10.87), 8.612 (10.48), 8.921 (3.60), 11.391 (3.71), 11.415 (3.71).

**Beipiel 181**

1-(3,5-Difluorpyridin-2-yl)-7-(2-oxa-6-azaspiro[3.3]hept-6-yl)-4-oxo-N-[3,3,4,4,4-pentafluorbutan-2-yl]-1,4-dihydro-1,8-naphthyridin-3-carboxamid (enantiomerenrein)

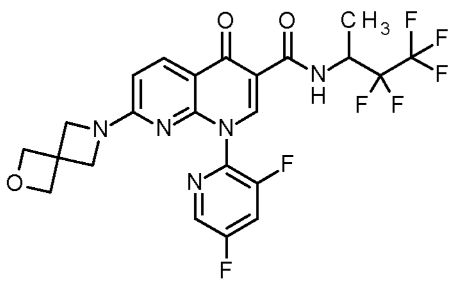

1-(3,5-Difluorpyridin-2-yl)-7-(2-oxa-6-azaspiro[3.3]hept-6-yl)-4-oxo-1,4-dihydro-1,8-naphthyridin-3-carbonsäure (150 mg, 375 μmol) wurde in 2.8 ml DMF vorgelegt, mit HATU (171 mg, 450 μmol) und N,N-Diisopropylethylamin (290 μl, 1.7 mmol) versetzt und 3,3,4,4,4-Pentafluorbutan-2-aminhydrochlorid (89.7 mg, 450 μmol) wurde hinzugeben. Die Mischung wurde über zwei Nächte bei Raumtemperatur gerührt. Die Reaktionslösung wurde mit Wasser versetzt und dreimal mit Ethylacetat extrahiert und eingedampft. Das Gemisch wurde mit Acetonitril, Wasser und TFA versetzt. Es fiel dabei ein Feststoff aus. Die Suspension wurde in Ethylacetat gelöst und zweimal mit Wasser und zweimal mit Natriumhydrogencarbonat-Lösung gewaschen. Die vereinigten wässrigen Phasen wurden mit Ethylacetat reextrahiert. Das Gemisch wurde eingedampft und der Rückstand wurde mittels Kieselgelchromatographie gereinigt (Laufmittel:

Dichlormethan/Cyclohexan/Methanol 100:50:1 nach 100:20:1). Es wurden 158 mg der Zielverbindung (76 % d. Th., Reinheit 98 %) erhalten.
LC-MS (Methode 1): $R_t$ = 1.03 min; MS (ESIpos): m/z = 546 $[M+H]^+$
$^1$H-NMR (400 MHz, DMSO-d6) δ [ppm]: -0.149 (0.75), -0.008 (5.81), 0.008 (6.07), 0.147 (0.70), 1.381 (4.23), 1.398 (6.18), 2.328 (1.23), 2.366 (0.60), 2.670 (1.26), 2.710 (0.60), 4.109 (0.68), 4.660 (16.00), 5.009 (0.57), 6.590 (4.12), 6.612 (4.16), 8.263 (4.53), 8.285 (4.60), 8.315 (1.25), 8.603 (3.34), 8.609 (3.30), 8.812 (2.31), 8.820 (2.58), 10.491 (1.92), 10.514 (1.89).

**Beipiel 182**

1-(3,5-Difluorpyridin-2-yl)-7-(2-oxa-6-azaspiro[3.3]hept-6-yl)-4-oxo-N-(3,3,4,4,4-pentafluor-2-methylbutan-2-yl)-1,4-dihydro-1,8-naphthyridin-3-carboxamid

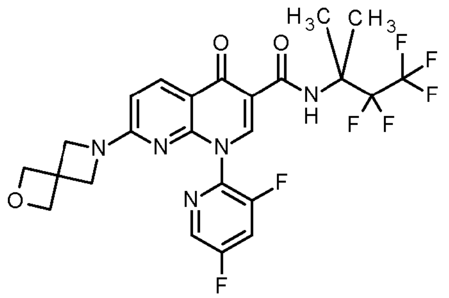

1-(3,5-Difluorpyridin-2-yl)-7-(2-oxa-6-azaspiro[3.3]hept-6-yl)-4-oxo-1,4-dihydro-1,8-naphthyridin-3-carbonsäure (150 mg, 374 μmol) wurde in 2.8 ml DMF vorgelegt, mit HATU (171 mg, 449 μmol) und N,N-Diisopropylethylamin (290 μl, 1.7 mmol) versetzt und 3,3,4,4,4-Pentafluor-2-methylbutan-2-amin-Hydrochlorid (95.9 mg, 449 μmol) wurde hinzugeben. Die Reaktion wurde über zwei Nächte bei Raumtemperatur gerührt. Die Reaktionslösung wurde mit Ethylacetat versetzt und zweimal mit Wasser extrahiert. Die vereinigten wässrigen Phasen wurden zweimal mit Ethylacetat reextrahiert. Die vereinigten organischen Phasen wurden über Natriumsulfat getrocknet, fitriert und eingedampft. Der Rückstand wurde mittels Kieselgelchromatographie gereinigt (Laufmittel: Dichlormethan/Cyklohexan/Methanol: 100:50:1 nach 100:20:1). Es wurden 187 mg der Zielverbindung über (87% d. Th., Reinheit 97%) erhalten.
LC-MS (Methode 1): $R_t$ = 1.12 min; MS (ESIpos): m/z = 560 $[M+H]^+$
$^1$H-NMR (400 MHz, DMSO-d6) δ [ppm]: -0.149 (0.47), -0.008 (3.55), 0.008 (3.46), 0.146 (0.42), 1.398 (5.78), 1.676 (15.55), 2.323 (0.64), 2.328 (0.91), 2.332 (0.66), 2.366 (0.55), 2.523 (2.42), 2.665 (0.74), 2.670 (1.02), 2.674 (0.72), 2.710 (0.59), 4.112 (0.62), 4.659 (16.00), 6.581 (4.53), 6.603 (4.59), 8.272 (4.78), 8.288 (1.00), 8.294 (5.46), 8.310 (1.34), 8.315 (1.42), 8.332 (0.89), 8.338 (0.96), 8.600 (4.29), 8.607 (4.12), 8.767 (8.03), 10.600 (5.01).

**Beipiel 183**

7-[3,3-Bis(hydroxymethyl)azetidin-1-yl]-1-(3,5-difluorpyridin-2-yl)-4-oxo-N -[3,3,4,4,4-pentafluorbutan-2-yl]-1,4-dihydro-1,8-naphthyridin-3-carboxamid (enantiomerenrein)

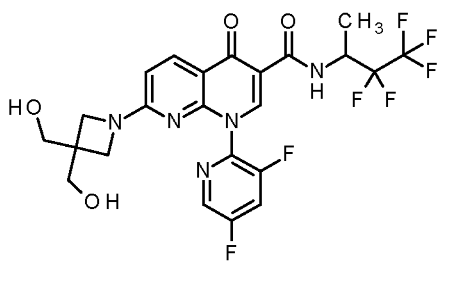

1-(3,5-Difluorpyridin-2-yl)-7-(2-oxa-6-azaspiro[3.3]hept-6-yl)-4-oxo-N-[3,3,4,4,4-pentafluorbutan-2-yl]-1,4-dihydro-1,8-naphthyridin-3-carboxamid (enantiomerenrein) (152 mg, 98 % Reinheit, 273 μmol) wurde in Trifluoressigsäure (1.7 ml, 22 mmol) vorgelegt, mit 1.7 ml Wasser und 1.7 ml Acetonitril versetzt und zwei Tage bei Raumtemperatur gerührt. Die Reaktionslösung wurde mittels präparativer HPLC gereinigt (RP18 Säule, Laufmittel: Acetonitril/Wasser-Gradient unter Zusatz von 0.1% TFA). Die Produktfraktionen wurden einrotiert. Der Rückstand wurde in Dichlormethan aufgenommen und zweimal mit gesättigter, wässriger Natriumhydrogencarbonatlösung gewaschen. Die vereinigten wässrigen Phasen wurde mit Dichlormethan reextrahiert. Die vereinigten organischen Phasen wurden über Natriumsulfat getrocknet, filtriert und eingedampft. Es wurden 86 mg der Zielverbindung (55% d. Th., Reinheit 99%) erhalten.
LC-MS (Methode 2): $R_t$ = 1.66 min; MS (ESIpos): m/z = 564 $[M+H]^+$
$^1$H-NMR (400 MHz, DMSO-d6) δ [ppm]: -0.149 (1.11), -0.008 (8.96), 0.008 (8.69), 0.146 (1.11), 1.146 (0.49), 1.234 (1.29), 1.383 (13.86), 1.400 (13.86), 2.328 (2.54), 2.366 (1.16), 2.670 (2.76), 2.710 (1.20), 3.473 (14.08), 3.803 (2.54), 4.831 (3.57), 5.013 (1.78), 5.033 (1.83), 5.056 (1.60), 6.585 (14.13), 6.607 (14.04), 8.238 (16.00), 8.260 (15.06), 8.286 (2.23), 8.309 (4.06), 8.330 (2.14), 8.594 (11.81), 8.601 (11.59), 8.795 (7.44), 8.803 (8.38), 10.527 (6.11), 10.551 (5.84).

**Beipiel 184**

7-[3,3- Bis(hydroxymethyl)azetidin-1-yl]-1-(3,5-difluorpyridin-2-yl)-4-oxo-N-(3,3,4,4,4-pentafluor-2-methylbutan-2-yl)-1,4-dihydro-1,8-naphthyridin-3-carboxamid

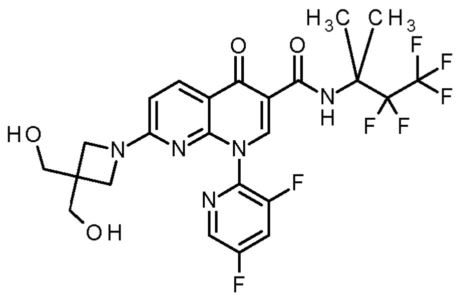

1-(3,5-Difluorpyridin-2-yl)-7-(2-oxa-6-azaspiro[3.3]hept-6-yl)-4-oxo-N-(3,3,4,4,4-pentafluor-2-methylbutan-2-yl)-1,4-dihydro-1,8-naphthyridin-3-carboxamid (181 mg, 324 μmol) wurde in 2 ml Trifluoressigsäure vorgelegt, mit 2 ml Wasser und 2 ml Acetonitril versetzt und zwei Tage bei Raumtemperatur gerührt. Die Reaktionslösung wurde mittels präparativer HPLC gereinigt (RP18 Säule, Laufmittel: Acetonitril/Wasser-Gradient unter Zusatz von 0.1% TFA). Die Produktfraktionen wurden einrotiert. Der Rückstand wurde in Dichlormethan aufgenommen und zweimal mit gesättigter, wässriger Natriumhydrogencarbonatlösung gewaschen. Die vereinigten wässrigen Phasen wurden mit Dichlormethan reextrahiert. Die vereinigten organischen Phasen wurden über Natriumsulfat getrocknet, filtriert und einrotiert. Es wurden 133 mg der Zielverbindung (70% d. Th., Reinheit 99%) erhalten.
LC-MS (Methode 2): $R_t$ = 1.79 min; MS (ESIpos): m/z = 578 $[M+H]^+$
$^1$H-NMR (400 MHz, DMSO-d6) δ [ppm]: -0.149 (0.64), -0.008 (5.13), 0.008 (4.91), 0.146 (0.62), 1.678 (16.00), 2.328 (1.15), 2.366 (0.44), 2.670 (1.10), 2.710 (0.44), 3.471 (4.27), 3.801 (0.80), 4.839 (1.11), 5.754 (2.74), 6.574 (4.42), 6.597 (4.42), 8.246 (5.02), 8.267 (4.71), 8.281 (0.99), 8.287 (1.08), 8.308 (1.53), 8.325 (0.95), 8.331 (1.06), 8.592 (4.93), 8.598 (4.75), 8.750 (10.54), 10.635 (5.19).

**Beispiel 185**

7-[4,4-Bis(hydroxymethyl)piperidin-1-yl]-1-(3,5-difluorpyridin-2-yl)-4-oxo-*N*-[3,3,4,4,4-pentafluorbutan-2-yl]-1,4-dihydro-1,8-naphthyridin-3-carboxamid (enantiomerenrein)

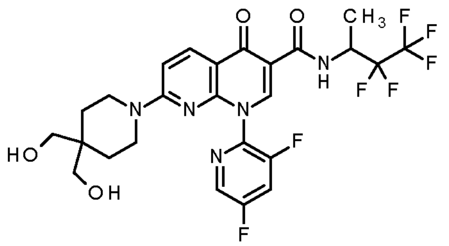

Es wurde 7-Chlor-1-(3,5-difluorpyridin-2-yl)-4-oxo-*N*-[3,3,4,4,4-pentafluorbutan-2-yl]-1,4-dihydro-1,8-naphthyridin-3-carboxamid (enantiomerenrein) (50.0 mg, 104 μmol) in 1.1 ml DMF gelöst, Piperidin-4,4-diyldimethanol-Hydrochlorid (21.8 mg, 95% Reinheit, 114 μmol) und *N*,*N*-Diisopropylethylamin (81 μl, 470 μmol) wurden hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 50.0 mg (81% d. Th., 99% Reinheit) der Titelverbindung erhalten.
LC-MS (Methode 3): $R_t$ = 1.75 min; MS (ESIpos): m/z = 592 $[M+H]^+$
$^1$H-NMR (400 MHz, DMSO-d6) δ [ppm]: -0.008 (1.25), 0.008 (1.21), 1.345 (6.70), 1.386 (7.98), 1.403 (7.69), 2.328 (0.52), 2.666 (0.43), 2.671 (0.61), 3.280 (15.22), 3.293 (16.00), 3.483 (4.39), 4.405 (4.66), 4.418 (10.58), 4.432 (4.62), 4.972 (0.45), 4.992 (0.83), 5.015 (0.96), 5.037 (0.96), 5.060 (0.85), 5.081 (0.45), 7.074 (5.67), 7.097 (5.78), 8.236 (7.58), 8.259 (7.08), 8.315 (1.21), 8.337 (2.22), 8.359 (1.23), 8.612 (5.44), 8.617 (5.26), 8.804 (3.86), 8.813 (4.35), 10.525 (2.98), 10.549 (2.89).

**Beispiel 186**

7-[4,4-Bis(hydroxymethyl)piperidin-1-yl]-1-(3,5-difluorpyridin-2-yl)-N-(1,1,1 ,3,3,3-hexafluorpropan-2-yl)-4-oxo-1,4-dihydro-1,8-naphthyridin-3-carboxamid

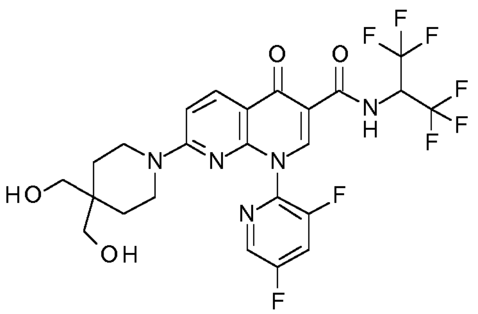

Es wurde 7-Chlor-1-(3,5-difluorpyridin-2-yl)-*N*-(1,1,1,3,3,3-hexafluorpropan-2-yl)-4-oxo-1,4-dihydro-1,8-naphthyridin-3-carboxamid (50.0 mg, 103 μmol) in 1.0 ml DMF gelöst, Piperidin-4,4-diyldimethanol-Hydrochlorid (21.6 mg, 95% Reinheit, 113 μmol) und *N*,*N* Diisopropylethylamin (81 μl, 460 μmol) wurden hinzugefügt und für 80 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 43.1 mg (70% d. Th., 99% Reinheit) der Titelverbindung erhalten.
LC-MS (Methode 3): $R_t$ = 1.84 min; MS (ESIpos): m/z = 596 $[M+H]^+$
$^1$H-NMR (400 MHz, DMSO-d6) δ [ppm]: -0.008 (0.57), 0.008 (0.57), 1.352 (0.65), 3.282 (1.64), 3.295 (1.74), 3.312 (16.00), 3.492 (0.44), 4.409 (0.50), 4.422 (1.13), 4.436 (0.49), 7.102 (0.61), 7.126 (0.62), 8.260 (0.81), 8.283 (0.76), 8.626 (0.89), 8.632 (0.84), 8.903 (1.73), 11.374 (0.48), 11.399 (0.45).

**Beispiel 187**

7-[4,4-Bis(hydroxymethyl)piperidin-1-yl]-1-(3,5-difluorpyridin-2-yl)-4-oxo- N-(3,3,4,4,4-pentafluor-2-methylbutan-2-yl)-1,4-dihydro-1,8-naphthyridin-3-carboxamid

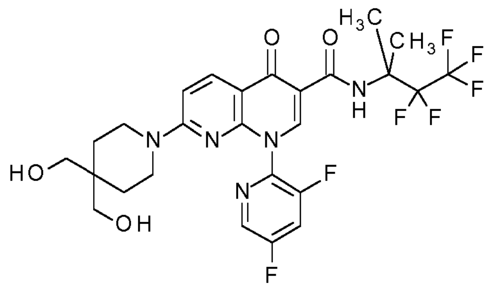

Es wurde 7-Chlor-1-(3,5-difluorpyridin-2-yl)-4-oxo-N-(3,3,4,4,4-pentafluor-2-methylbutan-2-yl)-1,4-dihydro-1,8-naphthyridin-3-carboxamid (50.0 mg, 101 µmol) in 1.1 ml DMF gelöst, Piperidin-4,4-diyldimethanol-Hydrochlorid (21.2 mg, 95% Reinheit, 111 µmol) und *N*,*N* Diisopropylethylamin (79 µl, 450 µmol) wurden hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 49.1 mg (80% d. Th., 99% Reinheit) der Titelverbindung erhalten.
LC-MS (Methode 3): $R_t$ = 1.85 min; MS (ESIpos): m/z = 606 $[M+H]^+$
$^1$H-NMR (400 MHz, DMSO-d6) δ [ppm]: 1.344 (0.75), 1.681 (2.97), 3.279 (1.60), 3.292 (1.71), 3.312 (16.00), 3.481 (0.49), 4.405 (0.45), 4.418 (0.99), 4.432 (0.45), 7.064 (0.61), 7.086 (0.63), 8.244 (0.82), 8.266 (0.77), 8.609 (0.90), 8.615 (0.87), 8.760 (1.82), 10.629 (1.02).

**Beispiel 188**

1-(3,5-Difluorpyridin-2-yl)-7-[(2R)-2-(hydroxymethyl)pyrrolidin-1-yl]-4-oxo-N-(3,3,4,4,4-pentafluor-2-methylbutan-2-yl)-1,4-dihydro-1,8-naphthyridin-3-carboxamid

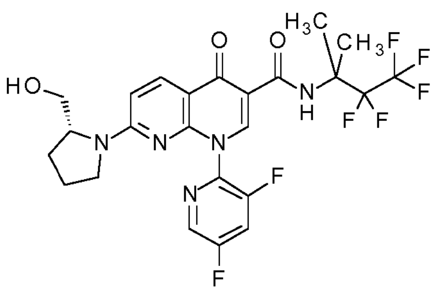

Es wurde 7-Chlor-1-(3,5-difluorpyridin-2-yl)-4-oxo-N-(3,3,4,4,4-pentafluor-2-methylbutan-2-yl)-1,4-dihydro-1,8-naphthyridin-3-carboxamid (50.0 mg, 101 µmol) in 1.1 ml DMF gelöst, (2*R*)-Pyrrolidin-2-ylmethanol (11 µl, 99% Reinheit, 110 µmol) und *N*,*N* Diisopropylethylamin (61 µl, 350 µmol) wurden hinzugefügt und für 18 h bei 55°C nachgerührt. Das Reaktionsgemisch wurde abgekühlt und mittels präparativer HPLC (Säule: Acetonitril / Wasser / 0.1 % Ameisensäure) gereinigt. Es wurden 49.9 mg (88% d. Th., 99% Reinheit) der Titelverbindung erhalten.
LC-MS (Methode 3): $R_t$ = 2.07 min; MS (ESIpos): m/z = 562 $[M+H]^+$
$^1$H-NMR (400 MHz, DMSO-d6) δ [ppm]: 1.683 (16.00), 1.873 (0.93), 1.953 (1.11), 2.328 (0.49), 2.670 (0.50), 6.731 (0.53), 6.754 (0.59), 8.264 (1.43), 8.287 (1.55), 8.590 (4.26), 8.596 (4.12), 8.767 (2.37), 10.649 (2.98).

**B. BEWERTUNG DER PHARMAKOLOGISCHEN WIRKSAMKEIT**

Die pharmakologische Aktivität der erfindungsgemäßen Verbindungen kann durch *in vitro*- und *in vivo*-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

**Abkürzungen und Akronyme:**

| | |
|---|---|
| $B_{Max}$ | Anzahl spezifischer Bindungstellen des Radioliganden |
| CAFTY | calcium free tyrode |
| CHO | chinese hamster ovary |
| CRE | cAMP-responsive element |
| DMEM | Dulbecco's modified eagle medium |
| DMSO | Dimethylsulfoxid |
| FCS | fötales Kälberserum |
| FRET | fluorescence resonance energy transfer |
| GIRK1/4 | G-protein-coupled inward rectifier potassium channel, member 1/4 |
| HEPES | Hydroxyethylpiperazin-Ethansulfonsäure |
| HTRF | homogeneous time resolved fluorescence |
| $K_d$ | Gleichgewichtsdissoziationskonstante |
| $K_i$ | Gleichgewichtsinhibitor-Konstante |
| $k_{off}$ | Dissoziationsrate |
| $k_{on}$ | Assoziationsrate |
| nM | nano-molar |
| MEM | minimum essential medium |
| μL | Mikro-Liter |
| μM | mikro-molar |
| mL | milli-Liter |
| mM | milli-molar |
| mtClytin | mitochondriales Clytin |
| min | Minuten |
| NMS | N-Me-Scopolamin |
| PAM | positiv allosterischer Modulator |
| PEI | Polyethylenimin |
| Pen/Strep | Penicillin/Streptomycin |
| sec | Sekunden |

•

## B-1. Funktioneller M2-GIRK1/4-Aktivierungstest

Sowohl die Aktivierung des M2-Rezeptors durch orthosterische Agonisten allein als auch die allosterische Verstärkung der Orthoster-induzierten Aktivierung durch positiv allosterische Modulatoren (PAMs) kann mittels eines zellbasierten funktionellen GIRK1/4-Aktivitätstest bestimmt werden. Die Bindung von orthosterischen Agonisten (endogener Ligand: Acetylcholin) an den M2-Rezeptor führt zu einer Rezeptoraktivierung bzw. Konformationsänderung des Rezeptors im Sinne einer Gleichgewichtsverschiebung zugunsten der aktiven Rezeptorkonformation. Die Bindung der orthosterischen Agonisten an den M2-Rezeptor und damit dessen Aktivierung kann durch positive allosterische Modulatoren, welche nicht an die orthosterische Bindungsstelle der Agonisten, sondern an eine separate allosterische Bindungsstelle binden, verstärkt werden.

Die Agonisten-induzierte Konformationsänderung des M2-Rezeptors hat eine Gai-Proteinaktivierung zur Folge. Die Aktivierung der Ga-Untereinheit wiederum führt zu einer Dissoziation und damit Freisetzung der Gβγ-Untereinheiten von der Ga-Untereinheit und der Aktivierung separater nachgeschalteter Signaltransduktionskaskaden. Der freigesetzte heterodimere Gβγ-Komplex bindet an den GIRK1/4 Kalium-Kanal und induziert eine ligandengesteuerte Kanalaktivierung bzw. -öffnung (Reuveny et al., Nature, July 1994, 370, 143-146). Unter physiologischen Bedingungen kommt es dann zu einem selektiven Ausstrom von Kalium aus der Zelle entlang des elektrochemischen Gradienten. Der Export positiver Ladung führt zu einer Erniedrigung des Transmembranpotentials und damit zu einer Hyperpolarisation der Zelle. Das Ausmaß der Hyperpolarisation kann daher als Maß für die Aktivierung des M2-Rezeptors angesehen werden.

Als Testzelle dient eine rekombinante CHO-DUKX-Zelllinie, welche mit cDNA kodierend für den humanen M2-Rezeptor sowie mit cDNA kodierend für beide GIRK1/4-Untereinheiten stabil transfiziert wurde (CHO-DUKX-M2-GIRK). Die Bestimmung des Transmembranpotentials bzw. der relativen Änderungen des Transmembranpotentials in Abhängigkeit von Substanzzugabe bzw. M2-Aktivierung erfolgt mittels eines spannungssensitiven Farbstoffes (FLIPR Membrane Potential Assay Kit Blue, Molecular Devices # R8034) und der Messung der Zellfluoreszenz unter Verwendung eines proprietären Fluoreszenz-Imaging-Messgerätes.

### B-1.1. Bestimmung der allosterischen Potenz der Testsubstanzen ($EC_{50}$-Wert)

Die Testsubstanzen werden in Dimethylsulfoxid (DMSO) mit einer Konzentration von 10 mM gelöst und für

eine 10 Punkt-Dosis-Wirkungsanalyse in Schritten von 1:3.16 seriell in DMSO verdünnt. Entsprechend der gewünschten Testkonzentrationen werden die Substanzen in Beladungspuffer (Zusammensetzung: 0,6 ml FLIPR Membrane Potential Assay Kit Blue (10 mg/ml), 0,6 ml Brilliant Black (10 mg/ml), 2 mM $CaCl_2$ und 2 mM KCl ad 50 ml Natrium-Gluconat-Tyrode (PAA, #T21-155)) vorverdünnt.

Die in MEM alpha-Medium (supplementiert mit 10% FCS, 2% Glutamax, 1mg/ml Geniticin) kultivierten Reporter-Zellen wurden mit 2000 Zellen (Messung nach 48 h) bzw. 4000 Zellen (Messung nach 24 h) in 30 μl pro 384 well in μCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 μl Beladungspuffer pro 384 Well). Anschließend erfolgte in einer ersten Messung die Bestimmung der Fluoreszenz für das Ruhe-Transmembranpotential für einen Zeitraum von 5 sec. Hiernach erfolgte die Zugabe von je 10 μl der in Beladungspuffer verdünnten Testsubstanzen, gefolgt von einer zweiten Messung zur Bestimmung des Transmembranpotentials für einen Zeitraum von 50 sec. Schließlich wurden die Zellen mit 10 μl Agonistenlösung (Acetylcholin gelöst in Beladungspuffer) versetzt. Acetylcholin wurde mit der dem $EC_{20}$-Wert entsprechenden Konzentration eingesetzt, die in einem Vortest bestimmt wurde. Die M2-vermittelte GIRK1/4-Aktivierung bzw. Hyperpolarisation wurde dann in einer dritten Messung über einen Zeitraum von 60 sec verfolgt. Der $EC_{50}$-Wert (Maß der allosterischen Potenz der Testverbindung) und die Effizienz (Maß der Verstärkung des Acetylcholin-Effektes bei einer $EC_{20}$-Acetylcholin-Konzentration) wurden mit Hilfe einer 4-Parameter-Logistik-Funktion (Hill-Funktion) ermittelt.

### B-1.2. Bestimmung der positiven Kooperativität (α-Faktor)

Die Testsubstanzen wurden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen wurden die Substanzen in Beladungspuffer (s.o.) vorverdünnt.

Die in MEM alpha-Medium (supplementiert mit 10% FCS, 2% Glutamax, 1mg/ml Geniticin) kultivierten Reporter-Zellen werden mit 2000 Zellen (Messung nach 48 h) bzw. 4000 Zellen (Messung nach 24 h) in 30 μl pro 384 well in μCLEAR/schwarz Greiner-Zellkulturplatten (#781092) ausgesät und 24 h bzw. 48 h bei 37°C inkubiert. Das Aussaatmedium bestand aus MEM alpha-Medium (supplementiert mit 5% FCS, 2% Glutamax, kein Geniticin).

Für die jeweilige Messung wurde das Medium entfernt und die Zellen für mindestens 6 min bei Raumtemperatur mit dem spannungssensitiven Farbstoff beladen (30 μl Beladungspuffer pro 384 well). Anschließend erfolgt in einer ersten Messung die Bestimmung des Ruhe-Transembranpotentials für einen Zeitraum von 5 sec in 1 sec-Inkrementen. Hiernach erfolgt die Zugabe von je 10 μl der in Beladungspuffer verdünnten Testsubstanzen, gefolgt von einer zweiten Messung zur Bestimmung des Transmembranpotentials für einen Zeitraum von 50 sec in 1 sec-Inkrementen.

Schließlich werden die Zellen mit 10 μl Agonistenlösung (Acetylcholin gelöst in Beladungspuffer) versetzt. Im Gegensatz zur $EC_{50}$-Bestimmung der Testsubstanzen (siehe B-1.1) wird dabei aber nicht nur eine Acetylcholin-Konzentration eingesetzt, sondern jede Konzentration der Testsubstanz wird mit einer Acetylcholin-8 Punkt-Dosis-Wirkungskurve kombiniert. Für die Acetylcholin-Verdünnungsreihe wird der Agonist beginnend mit einer maximalen Endkonzentration von 3 μM in Schritten von 1:3.16 seriell in Beladungspuffer entsprechend der gewünschten Endkonzentrationen vorverdünnt. Die M2-vermittelte GIRK1/4-Aktivierung bzw. Hyperpolarisation wird dann in einer dritten Messung über einen Zeitraum von 60 sec in 1 sec-Inkrementen verfolgt. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert. Der ermittelte α-Faktor ist dabei ein Maß für die Stärke und Richtung des allosterischen Effektes. α- > 1 spiegeln eine Erniedrigung des $EC_{50}$-Wertes bzw. eine Erhöhung der Potenz des Agonisten (Acetylcholin) in Anwesenheit von Alloster wider und bedeuten damit eine positive Kooperativität zwischen Orthoster (Acetylcholin) und Alloster (Testsubstanz). Eine positive Kooperativität ist das Kennzeichen eines positiven allosterischen Modulators. Umgekehrt sind α -Werte < 1 bezeichnend für eine negative Kooperativität zwischen Orthoster und Alloster und kennzeichnen damit negative allosterische Modulatoren. α-Werte = 1 bedeuten keine Kooperativität zwischen Orthoster und Alloster, d.h. die Bindungsaffinitäten von Orthoster und Alloster an den Rezeptor beeinflussen sich nicht wechselseitig. Je größer der Betrag des α-Wertes ist, umso größer ist das Ausmaß der Kooperativität zwischen Orthoster und Alloster.

In der folgenden Tabelle 2 sind für individuelle Ausführungsbeispiele die so ermittelten $EC_{50}$- und Effizienz-Werte sowie die α-Werte aus diesem Assay aufgeführt (zum Teil als Mittelwerte aus mehreren unabhängigen Einzelbestimmungen):

**Tabelle 2**

| Bsp.Nr. | Rezeptoraktivität $EC_{50}$ [μmol/L] | Effizienz [%] | Kooperativität (alpha- Faktor) |
|---|---|---|---|
| 1 | 0.073 | 97.71 | 46 |

(fortgesetzt)

| Bsp.Nr. | Rezeptoraktivität $EC_{50}$ [μmol/L] | Effizienz [%] | Kooperativität (alpha- Faktor) |
|---|---|---|---|
| 2 | 0.092 | 97.50 | 35 |
| 3 | 0.240 | 97.75 | |
| 4 | 0.014 | 98.25 | 63 |
| 5 | 0.031 | 98.25 | 63 |
| 6 | 0.014 | 95.00 | |
| 7 | 0.022 | 100.00 | |
| 8 | 0.083 | 100.00 | |
| 9 | 0.020 | 94.50 | |
| 10 | 0.025 | 95.50 | |
| 11 | 0.037 | 99.50 | |
| 12 | 0.055 | 100.00 | |
| 13 | 0.130 | 99.50 | |
| 14 | 0.087 | 98.50 | 50 |
| 15 | 0.043 | 96.17 | 63 |
| 16 | 0.048 | 99.00 | 40 |
| 17 | 0.111 | 88.00 | 24 |
| 18 | 0.067 | 87.00 | 34 |
| 19 | 0.086 | 91.50 | 38 |
| 20 | 0.330 | 87.00 | |
| 21 | 0.195 | 98.50 | |
| 22 | 0.575 | 85.00 | |
| 23 | 0.145 | 92.00 | |
| 24 | 0.560 | 87.00 | |
| 25 | 0.210 | 98.00 | |
| 26 | 0.200 | 94.50 | |
| 27 | 0.036 | 79.00 | 10 |
| 28 | 0.019 | 100.00 | 17 |
| 29 | 0.059 | 98.00 | |
| 30 | 0.064 | 99.00 | 14 |
| 31 | 0.645 | 88.50 | |
| 32 | 0.400 | 89.50 | |
| 33 | 0.505 | 83.00 | |
| 34 | 0.880 | 82.00 | |
| 35 | 0.170 | 99.50 | |
| 36 | 0.022 | 100.00 | |
| 37 | 0.021 | 99.00 | |
| 38 | 0.018 | 98.00 | |
| 39 | 0.051 | 99.00 | |

(fortgesetzt)

| Bsp.Nr. | Rezeptoraktivität $EC_{50}$ [μmol/L] | Effizienz [%] | Kooperativität (alpha- Faktor) |
|---|---|---|---|
| 40 | 0.160 | 100.00 | |
| 41 | 0.330 | 100.00 | |
| 42 | 0.295 | 100.00 | |
| 43 | 0.065 | 100.00 | |
| 44 | 0.031 | 98.50 | |
| 45 | 0.057 | 96.00 | |
| 46 | 0.385 | 97.00 | |
| 47 | 0.640 | 89.00 | |
| 48 | 0.074 | 92.50 | |
| 49 | 0.560 | 97.00 | |
| 50 | 0.730 | 92.00 | |
| 51 | 0.527 | 84.33 | |
| 52 | 0.048 | 90.50 | |
| 53 | 0.555 | 96.50 | |
| 54 | 0.350 | 94.67 | |
| 55 | 0.110 | 100.00 | 79 |
| 56 | 0.034 | 100.00 | 68 |
| 57 | 0.010 | 94.00 | |
| 58 | 0.011 | 96.00 | |
| 59 | 0.023 | 99.00 | |
| 60 | 0.275 | 100.00 | |
| 61 | 0.795 | 100.00 | |
| 62 | 0.220 | 100.00 | |
| 63 | 0.170 | 100.00 | |
| 64 | 0.154 | 91.33 | 32 |
| 65 | 0.087 | 100.00 | |
| 66 | 0.310 | 92.50 | |
| 67 | 0.053 | 97.00 | 36 |
| 68 | 0.195 | 96.00 | |
| 69 | 0.365 | 92.00 | |
| 70 | 0.220 | 91.00 | |
| 71 | 0.360 | 97.50 | |
| 72 | 0.045 | 90.00 | |
| 73 | 0.010 | 100.00 | 68 |
| 74 | 0.014 | 100.00 | |
| 75 | 0.019 | 100.00 | |
| 76 | 0.035 | 94.00 | |
| 77 | 0.260 | 100.00 | |

(fortgesetzt)

| Bsp.Nr. | Rezeptoraktivität $EC_{50}$ [µmol/L] | Effizienz [%] | Kooperativität (alpha- Faktor) |
|---|---|---|---|
| 78 | 0.044 | 91.50 | |
| 79 | 0.054 | 100.00 | |
| 80 | 0.120 | 100.00 | |
| 81 | 0.015 | 100.00 | |
| 82 | 0.028 | 100.00 | |
| 83 | 0.017 | 100.00 | |
| 84 | 0.020 | 100.00 | 57 |
| 85 | 0.085 | 99.00 | |
| 86 | 0.019 | 100.00 | |
| 87 | 0.063 | 100.00 | |
| 88 | 0.059 | 99.50 | |
| 89 | 0.275 | 98.00 | |
| 90 | 0.024 | 100.00 | 41 |
| 91 | 0.280 | 99.00 | |
| 92 | 0.017 | 100.00 | |
| 93 | 0.014 | 98.00 | 41 |
| 94 | 0.032 | 100.00 | |
| 95 | 0.026 | 100.00 | |
| 96 | 0.068 | 100.00 | |
| 97 | 0.038 | 96.50 | 53 |
| 98 | 0.023 | 100.00 | 56 |
| 99 | 0.038 | 100.00 | |
| 100 | 0.026 | 100.00 | |
| 101 | 0.585 | 78.50 | |
| 102 | 1.065 | 89.00 | |
| 103 | 0.305 | 100.00 | |
| 104 | 0.250 | 98.50 | |
| 105 | 0.098 | 100.00 | |
| 106 | 0.165 | 91.50 | |
| 107 | 0.097 | 98.00 | |
| 108 | 0.185 | 84.00 | |
| 109 | 0.135 | 96.00 | |
| 110 | 0.190 | 94.50 | |
| 111 | 0.100 | 99.00 | |
| 112 | 0.525 | 98.00 | |
| 113 | 0.145 | 97.00 | |
| 114 | 0.111 | 96.50 | |
| 115 | 0.150 | 89.00 | |

(fortgesetzt)

| Bsp.Nr. | Rezeptoraktivität $EC_{50}$ [$\mu$mol/L] | Effizienz [%] | Kooperativität (alpha- Faktor) |
|---|---|---|---|
| 116 | 0.335 | 96.00 | |
| 117 | 0.400 | 94.50 | |
| 118 | 0.500 | 100.00 | |
| 119 | 0.790 | 92.00 | |
| 120 | 0.400 | 100.00 | |
| 121 | 0.320 | 99.00 | |
| 122 | 0.220 | 92.00 | |
| 123 | 0.240 | 98.00 | |
| 124 | 0.130 | 97.00 | |
| 125 | 0.073 | 97.00 | |
| 126 | 0.087 | 98.00 | |
| 127 | 0.050 | 96.00 | |
| 128 | 0.520 | 89.00 | |
| 129 | 0.021 | 96.50 | |
| 130 | 0.053 | 93.00 | |
| 131 | 0.108 | 95.50 | |
| 132 | 0.325 | 98.00 | |
| 133 | 0.315 | 92.00 | |
| 134 | 0.105 | 100.00 | |
| 135 | 0.097 | 100.00 | |
| 136 | 0.064 | 97.50 | |
| 137 | 0.110 | 99.00 | |
| 138 | 0.070 | 98.50 | 61 |
| 139 | 0.230 | 100.00 | |
| 140 | 0.253 | 92.00 | 47 |
| 141 | 0.018 | 95.00 | |
| 142 | 0.047 | 94.50 | |
| 143 | 0.090 | 90.00 | |
| 144 | 0.150 | 92.00 | |
| 145 | 0.435 | 98.50 | |
| 146 | 0.910 | 96.00 | |
| 147 | 0.055 | 98.50 | |
| 148 | 0.066 | 100.00 | |
| 149 | 0.032 | 100.00 | |
| 150 | 0.375 | 88.00 | |
| 151 | 0.290 | 99.00 | |
| 152 | 0.103 | 99.50 | |
| 153 | 0.200 | 100.00 | |

(fortgesetzt)

| Bsp.Nr. | Rezeptoraktivität $EC_{50}$ [µmol/L] | Effizienz [%] | Kooperativität (alpha- Faktor) |
|---|---|---|---|
| 154 | 0.089 | 100.00 | |
| 155 | 0.360 | 100.00 | |
| 156 | 0.036 | 100.00 | |
| 157 | 0.050 | 100.00 | |
| 158 | 0.115 | 90.50 | |
| 159 | 0.094 | 100.00 | |
| 160 | 0.155 | 90.50 | |
| 161 | 0.070 | 100.00 | |
| 162 | 0.024 | 96.00 | |
| 163 | 0.120 | 100.00 | |
| 164 | 0.014 | 100.00 | |
| 165 | 0.013 | 84.00 | 30 |
| 166 | 0.030 | 81.00 | |
| 167 | 0.420 | 92.00 | |
| 168 | 2.850 | 84.50 | |
| 169 | 0.120 | 97.50 | |
| 170 | 2.250 | 86.00 | |
| 171 | 3.100 | 90.50 | |
| 172 | 1.600 | 98.00 | |
| 173 | 0.088 | 100.00 | |
| 174 | 0.083 | 100.00 | |
| 175 | 0.060 | 98.50 | |
| 176 | 0.028 | 100.00 | |
| 177 | 0.038 | 100.00 | |
| 178 | 0.056 | 100.00 | |
| 179 | 0.095 | 100.00 | |
| 180 | 0.089 | 100.00 | |
| 181 | 0.220 | 100.00 | |
| 182 | 0.410 | 100.00 | |
| 183 | 0.053 | 100.00 | |
| 184 | 0.130 | 100.00 | |
| 185 | 0.121 | 100.00 | |
| 186 | 0.110 | 100.00 | |
| 187 | 0.265 | 97.50 | |
| 188 | 0.118 | 100.00 | |

**B-2. Funktioneller Ca2+-Freisetzungstest mittels M2-Gα16-Reporterzellen**

Eine potentiell agonistische wie auch die positiv allosterische Wirkung der Testsubstanzen auf den M2-Rezeptor

kann anhand eines funktionellen $Ca^{2+}$-Freisetzungstests bestimmt werden. Die Aktivierung des M2-Rezeptors durch Bindung orthosterischer Agonisten (Acetylcholin) oder anderer Substanzen mit einem agonistischen Effekt führt zu einer Konformationsänderung des Rezeptors, welche im endogenen Zustand eine Gai-Protein-Aktivierung zur Folge hat. Durch Kopplung des M2-Rezeptors an das exogen exprimierte promiskuitive Gaq-Protein $G\alpha16$ kommt es jedoch zu einer $G\alpha16$-Proteinaktivierung nach Aktivierung des M2-Rezeptors, welche - über eine nachgeschaltete Signaltransduktionskaskade - eine intrazelluläre $Ca^{2+}$-Freisetzung bedingt. Das Ausmaß der intrazellulären $Ca^{2+}$-Mobilisierung kann daher in diesem Fall als Maß für die Aktivierung des M2-Rezeptors angesehen werden.

Als Testzelle dient eine rekombinante CHO-Zelllinie, welche mit cDNA kodierend für den humanen M2-Rezeptor und das $G\alpha16$-Protein sowie mit cDNA kodierend für das mitochondrial exprimierte Photoprotein Clytin (mtClytin) stabil transfiziert wurde (CHO mtClytin $G\alpha16$ M2). Die Bestimmung der intrazellulären $Ca^{2+}$-Freisetzung in Abhängigkeit von Substanzzugabe bzw. M2-Aktivierung erfolgt mittels eines $Ca^{2+}$-sensitiven Farbstoffes (Fluo-8) und der Messung der Zellfluoreszenz unter Verwendung eines $FLIPR^{TETRA}$-Instrumentes (Molecular Devices).

**B-2.1. Agonismus-Assay**

Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Entsprechend der gewünschten Testkonzentrationen werden die Substanzen in Fluo-8-Puffer (Zusammensetzung pro 100 ml: 500 µl Probenecid, 2 ml Brilliant Black (20 mg/ml), 440 µl Fluo-8, 2 mM $CaCl_2$ ad 100 ml CAFTY-Tyrode (130 mM NaCl, 5 mM KCl, 20 mM HEPES, 1 mM $MgCl_2$, 5 mM NaHCOs, pH 7.4)) vorverdünnt.

Die in MEM alpha-Medium (supplementiert mit 10% FCS, 2% Glutamax) kultivierten Reporter-Zellen wurden mit 3000 Zellen in 30 µl-Aussaatmedium pro 384 Well in µCLEAR/schwarz Greiner-Zellkulturplatten (#781092) ausgesät und 24 h bei 37°C inkubiert. Das Aussaatmedium besteht aus MEM alpha-Medium (supplementiert mit 5% FCS, 2% Glutamax). Für die jeweilige Messung wird das Medium entfernt und die Zellen nach Zugabe von je 20 µl Fluo-8-Puffer pro 384 Well für 1h 37°C im Brutschrank inkubiert. Nach Zugabe von je 10 µl pro 384 Well der vorverdünnten Testsubstanzen erfolgt die Messung der Zellfluoreszenz für einen Zeitraum von 5 min in 1 sec-Inkrementen. Das relative Maß der maximalen Aktivierung des M2-Rezeptors durch die jeweiligen Testsubstanzen wird durch Normierung des Testsignals auf das der $E_{Max}$-Konzentration von Acetylcholin (3/µM) entsprechende Signal berechnet.

**B-2.2. Bestimmung des positiv allosterischen Modulator-Effektes**

Um die positive Kooperativität der Testsubstanzen in Bezug auf die Acetylcholin-vermittelte M2-Rezeptoraktivierung bestimmen zu können, wird anschließend Referenzagonist (Acetylcholin) für eine vollständige Dosis-Wirkungsanalyse hinzugefügt. Hierzu wird Acetylcholin beginnend mit einer maximalen finalen Konzentration von 1 µM in Schritten von 1:3.16 seriell in Fluo-8-Puffer verdünnt. Nach Zugabe von je 10 µl Agonistenlösung pro 384 Well erfolgt wiederum die Messung der Zellfluoreszenz für einen Zeitraum von 5 min in 1 sec-Inkrementen. Dabei wird dieselbe Assay-Platte verwendet wie unmittelbar zuvor für den M2-Agonismus-Assay. Die Verschiebung der Acetylcholin-Dosis-Wirkungskurve in Anwesenheit zunehmender Konzentrationen der Testsubstanz wird mittels GraphPad PRISM (Allosteric $EC_{50}$ shift) analysiert und quantifiziert (s.o.).

**B-3. Selektivitätstest gegenüber humanen muskarinischen Acetylcholin-Rezeptoren**

Eine potentiell agonistische Wirkung wie auch positiv allosterische Wirkung der Testsubstanzen auf anderen humanen muskarinischen Acetylcholin-Rezeptoren kann in einem in funktionellen $Ca^{2+}$-Freisetzungstests bestimmt werden (Eurofins; GPCRProfiler® Services in agonistic and allosteric mode for Mx Receptors; cat#: HTS600GPCR).

Als Testzellen dienen mit dem jeweiligen Rezeptor transfizierten Chem-1- oder Chem-4-Zelllinien (ChemiScreen™ M1 Calcium-Optimized FLIPR Cell Lines, Eurofins; M1: HTS044C; ChemiScreen™ Calcium-Optimized Stable Cell Line Human Recombinant M2 Muscarininc Acetylcholine Receptor, Eurofins; M2: HTS115C; ChemiScreen™ Human Recombinant M3 Muscarinic Acetylcholine Receptor Calcium-Optimized Stable Cell Line, Eurofins; M3: HTS116C; ChemiScreen™ Human Recombinant M4 Muscarinic Acetylcholine Receptor Calcium-Optimized Stable Cell Line, Eurofins; M4: HTS117C; ChemiScreen™ M5 Calcium-Optimized FLIPR Cell Lines, Eurofins; M5: HTS075C). Der Substanztest wird mit einem $FLIPR^{TETRA}$-Instrument (Molecular Devices) durchgeführt.

**B-3.1. Agonismus-Assay**

Um einen potentiellen agonistischen Effekt der Testsubstanzen zu ermitteln, werden die jeweiligen Testsubstanzen mit einer finalen Testkonzentration von 10 µM oder 1 µM zugefügt. Die $Ca^{2+}$-Freisetzung bzw. Zellfluoreszenz wird über einen Zeitraum von 180 sec gemessen. Als Positivkontrolle zur Normierung des Substanzeffektes auf die

Rezeptoraktivierung wird eine dem $E_{Max}$-Wert entsprechende Konzentration von Acetylcholin eingesetzt.

Nach Beendigung des Agonismus-Assay wird die Assay-Platte bei 25°C für 7 min inkubiert. Nach der Inkubationsperiode wird der positiv allosterische Modulator-Assay initialisiert.

### B-3.2. Allosterischer Modulator-Assay

Um eine positiv oder negativ allosterische Wirkung der Testsubstanzen auf andere humane muskarinische Acetylcholinrezeptoren sowie den M2-Rezeptor selbst zu untersuchen, wird jede Substanzkonzentration mit einer Acetylcholin-8 Punkt-Dosis-Wirkungskurve kombiniert. Nach Zugabe von Agonistenlösung erfolgt wiederum die Messung der Zellfluoreszenz für einen Zeitraum von 180 sec. Die Verschiebung der Acetylcholin-Dosis-Wirkungskurve (maximale Verschiebung des $EC_{50}$-Wertes von Acetylcholin) wird mittels GraphPad PRISM (Sigmoidal dose-response (variable slope) - $EC_{50}$) analysiert und quantifiziert. Abschließend werden Quotienten der allosterischen Verschiebung für den M2-Rezeptor und M4-Rezeptor gebildet, die ihrerseits als Maß für die jeweilige Selektivität fungieren.

### B-4. In vitro M2 PAM Gi-Assay

Für die Charakterisierung von Testsubstanzen auf positiv allosterische Modulation des humanen M2 Rezeptors wird die Carbachol induzierte Hemmung des cAMP Anstieg durch Forskolin in rekombinant M2 Rezeptor-exprimierenden CHO Zellen gemessen, die zusätzlich ein Luciferase Gen unter der Kontrolle eine cAMP responsiven Elementes (CRE) exprimieren: 3000 Zellen in 25 µl Vollmedium (DMEM F12 PAN Medium, 10 % FCS, 1.35 mM Na-Pyruvat, 20 mM Hepes, 4mM Glutamax, 2 % Natrium Bicarbonat, 1 % Pen/Strep, 1 % 100x non-essential amino acids) werden je Loch einer 384 Multititerplatte (Greiner, TC Platte, schwarz mit klarem Boden) ausgesät und bei 37°C, 5 % $CO_2$ für 24 Stunden inkubiert. Vor der Messung wird das Medium durch 30 µl Messmedium (Optimem) ersetzt und bei 37°C, 5 % $CO_2$ für 10 Minuten inkubiert. Die Testsubstanz wird in DMSO in verschiedenen Konzentrationen (Startkonzentration 10 mM, Verdünnungsfaktor 3.16) als Dosiswirkungskurve vorbereitet und 1:50 mit calciumfreier Tyrode, 2 mM $CaCl_2$, 0.01% BSA vorverdünnt. 10 µl der vorverdünnten Substanzlösung werden zu den Zellen gegeben und bei 37°C, 5 % $CO_2$ für 10 Minuten inkubiert. Der M2 Rezeptor wird durch Zugabe von 10 µl Carbachol in verschiedenen Konzentrationen in calciumfreier Tyrode, 2 mM $CaCl_2$ aktiviert und bei 37°C, 5 % $CO_2$ für 5 Minuten inkubiert. Die Adenylylcyclase wird durch Zugabe von 10 µl 1 µM (finale Konzentration) Forskolin in calciumfreier Tyrode, 2 mM $CaCl_2$ aktiviert und bei 37°C, 5 % $CO_2$ für 5 Stunden inkubiert. Nach Entfernen des Zellüberstandes und Zugabe von 20 µl Luci/Triton Puffer (1:1) wurde die Lumineszenz in einem Luminometer für 60 Sekunden bestimmt.

Calciumfreie Tyrode: 130 mM NaCl, 5 mM KCl, 20 mM HEPES, 1 mM $MgCl_2$, 4.8 mM $NaHCO_3$, pH 7.4 Luci/Triton Puffer (1:1): Luci-Puffer (20mM Tricine, pH 7.8, 2,67 mM Magnesiumsulfat, 0.1 mM EDTA, 4 mM DTT, 270 µM Coenzym A, 470 µM D-Luciferin, 530 µM ATP) 1:1 mit Triton-Puffer (25 mM Tris wässr. Salzsäure, pH 7.8, 25 mM $Na_2HPO_4$, 2mM Dithiothreitol, 3 % Triton X-100, 10 % Glycerin) gemischt.

Der $EC_{50}$-Wert wurde mit Hilfe einer 4-Parameter-Logistik-Funktion (Hill-Funktion) ermittelt.

### B-5. Kompetitiver FRET-Bindungstest für humane M2- und M4-Rezeptoren

Die direkte Bindung der Testsubstanzen an den M2-Rezeptor sowie die Verstärkung der Bindung (Erhöhung der Affinität) des natürlichen Agonisten Acetylcholin an den M2-Rezeptor in Anwesenheit der Testsubstanzen (positiv allosterischer Effekt) wird mittels eines FRET-basierten Bindungsassays (HTRF Tag-lite® Bindungsassay, Cisbio) bestimmt. Zur Kontrolle der Selektivität wird die Bindung der Testsubstanzen an den strukturverwandten M4-Rezeptor analog untersucht. Der HTRF Tag-lite® Assay ist ein homogener Bindungsassay und beruht auf der kompetitiven Bindung eines fluoreszenten Liganden (Sonde) und der unmarkierten Testsubstanz an den Rezeptor, welcher auf lebenden Zellen exprimiert ist. Der Rezeptor ist seinerseits mit einem fluoreszenten Donor-Farbstoff (Terbium-Kryptat) derivatisiert, so dass nach Anregung des Donor-Farbstoffes ein FRET-Signal zwischen Rezeptor und Sonde (Akzeptor) entsteht, wenn die Sonde an den Rezeptor gebunden ist. Als Akzeptor-Sonde wurde ein Telenzepin-Derivat eingesetzt, welches mit einem HTRF Fluoreszenz-Farbstoff konjugiert ist (red ligand; L0040RED). Die Sonde bindet daher in der konservierten orthosterischen Bindungsstelle sowohl des M2- als auch des M4-Rezeptors. Die allosterische Bindungsstelle des M2-Rezeptors wurde röntgenkristallographisch charakterisiert und wird direkt oberhalb der orthosterischen Bindungstasche postuliert (Kruse et al., Nature, 2013, 504, 101-106). Sowohl die Bindung von unmarkierten orthosterischen Agonisten (Acetylcholin) an die orthosterische Bindungsstelle als auch die Bindung von allosterischen Modulatoren (Testsubstanzen) an die allosterische Bindungsstelle führt daher zu einer konzentrationsabhängigen kompetitiven Verdrängung der Sonde und damit einer Abnahme des FRET-basierten Fluoreszenzsignals.

Alle Bindungstests werden auf weißen 384 Mikrotiterplatten (small-volume) in einem Gesamtvolumen von 20 µl durchgeführt. Die HTRF-Messungen werden mit einem PHERAstar-Instrument (BMG Labtech) vorgenommen. Für den muskarinischen M2- oder M4-Rezeptor-Bindungstest werden SNAPed-M2-exprimierende Zellen (C1TT1M2) oder

SNAPed-M4-exprimierende Zellen (C1TT1M4) verwendet, welche mit einem Donor-Fluorophor (Lumi4Tb; CELLCUST) markiert wurden. Die Zellen werden mit der Akzeptor-Sonde in Tag-lite Bindungspuffer (LABMED) in Anwesenheit von Testsubstanz bzw. Acetylcholin inkubiert. Anschließend wird das Fluoreszenz-Signal bei Wellenlängen von 665 nm und 620 nm gemessen und der HTRF-Quotient (Signal bei 665 nm / Signal bei 620 nm) bestimmt. Das relative spezifische Signal wird durch Subtraktion des HTRF-Quotienten der Negativkontrolle (nur Tag-lite Puffer ohne Sonde) ermittelt.

**B-5.1. Bindung der Testsubstanzen**

Um die Bindung der Testsubstanzen an den M2- oder M4-Rezeptor in Abwesenheit von orthosterischem Agonisten zu bestimmen, wird eine Dosis-Wirkungsanalyse der Testsubstanzen im kompetitiven Format des M2-Tag-lite®- oder M4-Tag-lite®-Bindungsassays vorgenommen. Die Testsubstanzen werden in DMSO mit einer Konzentration von 10 mM gelöst und für eine Dosis-Wirkungsanalyse in Schritten von 1:3.16 seriell in DMSO verdünnt. Die maximale Testkonzentration entspricht 10 μM. Die molare Konzentration der Testsubstanz, die eine halbmaximale Reduktion des HTRF-Signals in Bezug auf das maximale und verbleibende HTRF-Signal bei höchster Substanzkonzentration bewirkte ($EC_{50}$-Wert der Bindung), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

**B-5.2. Bindung der Testsubstanzen im allosterischen Modus**

Um die allosterische Modulation des M2-Rezeptors durch die Testverbindungen zu untersuchen, wird zum einen eine Dosis-Wirkungsanalyse der Testsubstanzen im kompetitiven Format des M2-Tag-lite®- oder M4-Tag-lite®-Bindungsassays in Anwesenheit einer dem $EC_{20}$-Wert entsprechenden Konzentration von Acetylcholin vorgenommen, welche in einer separaten 11 Punkt-Acetylcholin-Dosis-Wirkungsanalyse bestimmt wird (3 μM). Die Testsubstanzen werden in DMSO mit einer Konzentration von 10 mM gelöst und für eine 10 Punkt-Dosis-Wirkungsanalyse in Schritten von 1:3.16 seriell in DMSO verdünnt. Die maximale Testkonzentration entspricht 10 μM. Die molare Konzentration der Testsubstanz, die eine halbmaximale Reduktion des HTRF-Signals in Bezug auf das maximale und verbleibende HTRF-Signal bei höchster Substanzkonzentration in Anwesenheit einer dem EC20-Wert entsprechenden Acetylcholin-Konzentration bewirkt ($EC_{50}$-Wert der Bindung ), wird mittels GraphPad PRISM (Sigmoidal dose response) ermittelt. Zugleich wird die Stärke des Kompetitionseffektes bestimmt, indem die maximale Abnahme des spezifischen HTRF-Signals bei höchster Substanzkonzentration berechnet wird (% max. Kompetition).

Um die Verstärkung der Bindung von Acetylcholin an den M2- oder M4-Rezeptor zu untersuchen, wurde zusätzlich zum anderen eine 11 Punkt-Dosis-Wirkungsanalyse von Acetylcholin im kompetitiven Format des M2-Tag-lite®- oder M4-Tag-lite®-Bindungsassays in Abwesenheit oder in Anwesenheit von 1 μM oder 10 μM Testsubstanzvorgenommen. Die Verschiebung der Acetylcholin-Dosis-Wirkungskurve (maximale Verschiebung des $EC_{50}$-Wertes von Acetylcholin) wurde mittels GraphPad PRISM (Sigmoidal dose-response) analysiert und quantifiziert.

**B-6. Radioligand-Bindungstests für humane M2-Rezeptoren**

Der allosterische Wirkmechanismus der Testsubstanzen kann mittels verschiedener Radioligand-Bindungstests näher untersucht und quantifiziert werden. Die Bindung des Allosters an die allosterische Bindungsstelle des M2-Rezeptors hat im Falle einer positiven Kooperativität eine Erhöhung der Bindungaffinität des orthosterischen Liganden für den M2-Rezeptor zur Folge. Die Erhöhung der Bindungsaffinität des orthosterischen Liganden durch den Alloster im ternären Komplex bestehend aus Orthoster, Alloster und M2-Rezeptor wiederum ist auf eine Modulation der Bindungskinetiken des Orthosters zurückzuführen. Dabei kann der Alloster die Assoziations- und/oder Dissoziationsgeschwindigkeit des Orthosters am M2-Rezeptor verändern. Eine Erniedrigung der Dissoziationsgeschwindigkeit spiegelt dabei eine Stabilisierung des ternären Komplexes wieder und geht daher auch mit einer Erniedrigung der Dissoziationskonstante des orthosterischen Liganden unter Gleichgewichtsbedingungen einher (Lazareno, Determination of Allosteric Interactions Using Radioligand-Binding Techniques in Methods in Molecular Biology, vol. 259, Receptor Signal Transduction Protocols, 2nd ed.; Kostenis and Mohr, Trends Pharmacol. Sci. 1996, 17(8), 280-283).

**B-6.1. $^{3}$H-Oxotremorin-M Radioligand-Bindungstest unter Gleichgewichtsbedingungen**

Um den Einfluss der Testsubstanzen auf die Bindungsaffinität von orthosterischen Agonisten für den M2-Rezeptor zu überprüfen und zu quantifizieren, kann ein Radioligand-Bindungstest unter Gleichgewichtsbedingungen durchgeführt werden. Dabei wird die Bindung des radioaktiv markierten M2-Rezeptor-Agonisten $^{3}$H-Oxotremorin-M an den M2-Rezeptor für unterschiedliche $^{3}$H-Oxotremorin-M-Konzentrationen im Bindungsgleichgewicht gemessen (Croy et al., Mol. Pharmacol. 2014, 86, 106-115). Basierend auf der spezifisch gebundenen Menge von radioaktivem Agonisten

an den M2-Rezeptor in Abhängigkeit von der Agonisten-Konzentration (graphisch dargestellt als sog. Langmuir-Isotherme) kann dann zum einen die Gleichgewichtsdissoziationskonstante $K_d$ des Agonisten als quantitatives Maß seiner Bindungsaffinität für den M2-Rezeptor sowie zum anderen die Konzentration bzw. Anzahl spezifischer Bindungstellen des Radioliganden (Agonisten) $B_{max}$ in Abwesenheit oder Anwesenheit unterschiedlicher Konzentrationen der Testsubstanzen (positiven allosterischen Modulatoren) berechnet werden (Hulme and Trevethick, Brit. J. Pharmacol. 2010, 161, 1219-1237).

Der Radioligandenbindungsassay für den M2 Rezeptor (Euroscreen, FAST-0261B) wird mittels $^3$H-markiertem Oxotremorin-M (NET671) als Agonisten durchgeführt. Die Agonistenbindung an den M2-Rezeptor wird auf 96 Mikrotiterplatten (Master Block, Greiner, 786201) in Bindungspuffer (Natrium-/Kalium-Phosphat-Pufer, pH 7,4) in Triplikaten vorgenommen. Hierzu werden pro Ansatz M2-Membranextrakte (20 µg Protein / 96 Well) mit verschiedenen Konzentrationen von radioaktiv markiertem Agonisten (0,2 - 100 nM) allein oder in Anwesenheit von 1 µM oder 10 µM Testsubstanz oder Bindungspuffer allein in einem Gesamtvolumen von 0,1 mL für 60 min bei 37°C inkubiert. Die nichtspezifische Bindung von $^3$H-markiertem Oxotremorin-M an die Membran wird durch Co-Inkubation mit N-Me-Scopolamin (NMS), einem orthosterischen Antagonisten des M2-Rezeptors, in 200-fachem Überschuss bestimmt. Anschließend werden die Proben zum Stoppen der Bindungsreaktion über GF/C Filter (Perkin Elmer, 6005174), welche zuvor mit 0,5 %-iger Polyethylenimin- (PEI-) Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 µL Microscint 20 Szintillationslösung (Packard) pro Ansatz zugegeben. Die Proben werden dann für 15 min auf einem Orbitalschüttler inkubiert, bevor die Messung der Radioaktivität mittels eines TopCount™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $K_d$-Wert sowie der $B_{max}$-Wert von $^3$H-Oxotremorin-M für den M2-Rezeptor werden mit Hilfe eines "one-site" spezifischen Bindungsmodells bestimmt (Croy et al., Mol. Pharmacol. 2014, 86, 106-115).

**B-6.2. $^3$H-NMS Radioligand-Verdrängungstest unter Gleichgewichtsbedingungen**

Um den Einfluss der Testsubstanzen auf die Bindungsaffinität von orthosterischen Agonisten für den M2-Rezeptor weitergehend zu überprüfen und zu quantifizieren, wird zusätzlich ein kompetitiver Radioligand-Bindungstest unter Gleichgewichtsbedingungen durchgeführt. Hierbei wird die Bindung des antagonistischen Radioliganden $^3$H-N-Me-Scopolamin ($^3$H-NMS) an den M2-Rezeptor in Abwesenheit oder Anwesenheit unterschiedlicher Konzentrationen des nicht radioaktiv markierten Agonisten Oxotremorin-M bestimmt (Croy et al., Mol. Pharmacol. 2014, 86, 106-115; Schober et al., Mol. Pharmacol. 2014, 86, 116-123). Die radioaktiv markierte Sonde (Antagonist) und der unmarkierte Agonist kompetieren um die Bindung an die orthosterische Bindungsstelle des M2-Rezeptors. Die Fähigkeit zur Verdrängung der radioaktiv markierten Sonde dient daher als Maß für die Bindungsaffinität des Agonisten für den Rezeptor und kann gemäß der Cheng-Prusoff-Gleichung als Gleichgewichtsinhibitor-Konstante ($K_i$-Wert) quantifiziert werden (Cheng and Prusoff, Biochem. Pharmacol. 1973, 22(23), 3099-3108). Um den allosterischen Effekt der Testsubstanzen weitergehend zu untersuchen, wird der Einfluss der Testsubstanzen auf den $K_i$-Wert von Oxotremorin-M bestimmt.

Der Antagonisten-Inhibitionsbindungsassay für den M2-Rezeptor (Euroscreen, FAST-0261B) wird auf 96 Mikrotiterplatten (Master Block, Greiner, 786201) in Bindungspuffer (50 mM Tris-Puffer pH 7.4, 1 mM EDTA, 10 µg/ml Saponin) mit $^3$H-NMS als M2 Rezeptor-Antagonisten durchgeführt. Zur Einstellung des Bindungsgleichewichts werden pro Ansatz M2-Membranextrakte (20 µg Protein / 96 Well) mit einer definierten Konzentration von radioaktiv markiertem Antagonisten (0,5 nM) allein oder in Anwesenheit von unterschiedlichen Konzentrationen von unmarkierten Agonisten (Oxotremorin-M; 0,001 nM bis 1 mM) mit oder ohne 1 µM oder 10 µM Testsubstanz oder Bindungspuffer allein in einem Gesamtvolumen von 0,1 mL für 2 h bei 25°C inkubiert. Die nichtspezifische Bindung von $^3$H-markiertem NMS an die Membran wird durch Co-Inkubation mit nicht radioaktiv markiertem Acetylcholin in 200-fachem Überschuss bestimmt. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 µL Microscint 20 Szintillationslösung (Packard) pro Ansatz zugegeben. Die Proben wurden dann für 15 min auf einem Orbitalschüttler inkubiert, bevor die Messung der Radioaktivität mittels eines TopCount™-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Die $K_i$-Werte in An- oder Abwesenheit von Testsubstanz werden mit Hilfe der Cheng-Prusoff-Gleichung quantifiziert (Cheng and Prusoff, Biochem. Pharmacol. 1973, 22(23), 3099-3108). Dabei werden die $IC_{50}$-Werte der Sub-

stanzen gemäß einer vier Parameter enthaltenden logistischen Gleichung ermittelt und der $K_d$-Wert von NMS in einem Radioligand-Bindungstest unter Gleichgewichtsbedingungen ermittelt (Schober et al., Mol. Pharmacol. 2014, 86, 116-123).

**B-6.3. $^3$H-Oxotremorin-M Dissoziationskinetiktest**

Mithilfe eines kinetischen Radioligand-Bindungstests kann die Kinetik der Dissoziation des radioaktiv markierten Agonisten $^3$H-Oxotremorin-M für den M2-Rezeptor in An- oder Abwesenheit von Testsubstanz untersucht werden. Hierdurch kann der Einfluss der allosterischen Aktivität der Testsubstanzen auf die Dissoziationskonstante ($k_{off}$-Rate) des M2-Agonisten bestimmt und so der Mechanismus der Allosterie der Testsubstanzen weitergehend charakterisiert werden (Lazareno, Determination of Allosteric Interactions Using Radioligand-Binding Techniques in Methods in Molecular Biology, vol. 259, Receptor Signal Transduction Protocols, 2nd ed.; Schrage et al., Biochem. Pharmacol., 2014, 90, 307-319.).

Der Radioliganden Dissoziationsbindungsassay für den M2 Rezeptor (Euroscreen, FAST-0261B) wird mit $^3$H-markiertem Oxotremorin-M (NET671) als Agonisten durchgeführt. Die Bindungsreaktion wird in Bindungspuffer (Natrium-/Kalium-Phosphat-Pufer, pH 7,4) auf 96 Mikrotiterplatten (Master Block, Greiner, 786201) vorgenommen. Hierzu werden pro Ansatz M2-Membranextrakte (20 $\mu$g Protein / 96 Well) mit einer definierten Konzentration von radioaktiv markiertem Agonisten (9,65 nM) allein oder in Anwesenheit von 1 $\mu$M oder 10 $\mu$M Testsubstanz oder Bindungspuffer allein für 60 min bei 37°C vorinkubiert. Anschließend wird NMS in 200-fachem Überschuss zu unterschiedlichen Zeitpunkten hinzugefügt (ein Zeitpunkt pro Ansatz) und die Ansätze in einem Gesamtvolumen von 0,1 mL bei 37°C inkubiert. Anschließend werden die Proben zum Stoppen der Bindungsreaktion über GF/C Filter (Perkin Elmer, 6005174), welche zuvor mit 0,5 %-iger PEI-Lösung benetzt wurden, für 2 h bei Raumtemperatur filtriert. Die Filter werden sechsmal mit je 0,5 mL eiskaltem Waschpuffer (10 mM Natrium-/Kalium-Phosphat-Puffer, pH 7.4) gewaschen und anschließend je 50 $\mu$L Microscint 20 Szintillationslösung (Packard) pro Ansatz zugegeben. Die Proben werden dann für 15 min auf einem Orbitalschüttler inkubiert, bevor die Messung der Radioaktivität mittels eines TopCountTM-Gerätes (1 min / Well) vorgenommen wird.

Die Testsubstanzen werden in DMSO mit einer Konzentration von 10 mM gelöst und entsprechend der finalen Testkonzentration weiter in DMSO verdünnt, um eine 100-fache Verdünnung der eingesetzten DMSO-Lösung in Bindungspuffer zu erhalten.

Der $k_{off}$-Wert wurde mit Hilfe eines "one phase" exponentiellen Zerfallsmodells der Dissoziation bestimmt (Hulme and Trevethick, Brit. J. Pharmacol. 2010, 161, 1219-1237; Kostenis and Mohr, Trends Pharmacol. Sci. 1996, 17(8), 280-283).

**B-6.4. $^3$H-M2-PAM Bindungstest**

Die Bindungsaffinität der Testsubstanzen für den human M2 Rezeptor kann direkt bestimmt werden mithilfe einer radioaktiv markierten Testsubstanz als Sonde. Hierzu wurpositiv de eine allosterische Testsubstanz mittels Tritiierung radioaktiv markiert ($^3$H-M2-PAM).

Mittels eines Radioligandenbindungstests unter Gleichgewichtsbedingungen können zum einen die Gleichgewichtsdissoziationskonstante $K_d$ der positiv allosterischen Testsubstanz ($^3$H-M2-PAM) als quantitatives Maß seiner Bindungsaffinität für den M2-Rezeptor sowie zum anderen die Anzahl spezifischer Bindungstellen des Radioliganden $B_{max}$ in Abwesenheit oder Anwesenheit eines orthosterischen Agonisten (Acetlycholin) bestimmt werden (Hulme and Trevethick, Brit. J. Pharmacol. 2010, 161, 1219-1237; Schober et al., Mol. Pharmacol. 2014, 86, 116-123). Für den $^3$H-M2-PAM-Gleichgewichtsbindungs-Test werden M2 Rezeptor-Zellmembranpräparationen (CHO-S / hM2, 200 $\mu$g) in Inkubationspuffer (10 mM Tris/HCl pH 7,4, 2 mM $MgCl_2$, 120 mM NaCl, Protease Inhibitoren, 0,3 % BSA) zusammen mit jeweils verschiedenen Konzentrationen des allosterischen Radioliganden **$^3$H-M2-PAM** (0,5 - 4000 nM) in Abwesenheit oder Anwesenheit von Acetylcholin (100 $\mu$M) für 1 h bei 4°C inkubiert. Die unspezifische Bindung wird bestimmt durch die Zugabe eines Überschusses von nicht-radioaktiv markiertem allosterischen Liganden (M2-PAM) (10 $\mu$M). Zum Stoppen der Bindungsreaktion werden die Proben über ein Brandel Filter-System filtriert und mit Stop-Buffer gewaschen (50 mM Tris/HCl pH 7,4, 500 mM NaCl, 0,3 % BSA). Die Filter wurden zuvor mit 0,3 %-iger PEI-Lösung benetzt. Der Kd- und Bmax-Wert des allosterischen Radioliganden werden basierend auf einem "one-site" spezifischen Bindungsmodell ermittelt (GraphPad Prism).

Mittels eines kompetitiven $^3$H-M2-PAM -Bindungstests kann die Affinität unmarkierter allosterischer Testsubstanzen für die Bindungstelle des Radioliganden **$^3$H-M2-PAM** an den M2 Rezeptor ermittelt werden. (Croy et al., Mol. Pharmacol. 2014, 86, 106-115; Schober et al., Mol. Pharmacol. 2014, 86, 116-123). Die radioaktiv markierte Sonde $^3$H-M2-PAM) und die unmarkierte allosterische Testsubstanz kompetieren um die Bindung an die allosterische Bindungsstelle des M2-Rezeptors. Die Fähigkeit zur Verdrängung der radioaktiv markierten Sonde dient daher als Maß für die allosterische Bindungsaffinität der Testsubstanzen für den Rezeptor und kann gemäß der Cheng-Prusoff-Gleichung als

Gleichgewichtsinhibitor-Konstante (Ki-Wert) quantifiziert werden (Cheng and Prusoff, Biochem. Pharmacol. 1973, 22(23), 3099-3108). Die Verdrängung der radioaktiv markierten allosterischen Sonde wird dabei in An- oder Abwesenheit von orthosterischem Agonisten (Acetylcholin) bestimmt. Der $^3$H-M2-PAM Kompetitionsbindungs-Test wird analog zum oben beschriebenen $^3$H-M2-PAM Bindungstest unter Gleichgewichtsbedingungen durchgeführt. Dabei werden die M2 Rezeptor enthaltenen Membranpräparationen mit 1 nM $^3$H-M2-PAM und verschiedenen Konzentrationen von unmarkierter Testsubstanz in Ab- oder Anwesenheit von Acetylcholin (100 μM) inkubiert. Die K-Werte in An- oder Abwesenheit von Acetylcholin werden mit Hilfe der Cheng-Prusoff-Gleichung ermittelt (Cheng and Prusoff, Biochem. Pharmacol. 1973, 22(23), 3099-3108).

## B-7. Effekte der Testsubstanzen auf Acetylcholin vermittelte GIRK1/4-Kanalströme in primären atrialen Kardiomyozyten der Ratte

Die Substanztestung erfolgt gemäß eines in der Literatur beschriebenen Patch-Clamp Protokolls zur elektrophysiologischen Messung Acetylcholin-induzierter GIRK1/4-Membranströme in nativen atrialen Myozyten der Ratte (Cheng and Prusoff, Biochem. Pharmacol. 1973, 22(23), 3099-3108 , siehe z.B. Beckmann and Rinne et al., Cell. Physiol. Biochem. 2008, 21, 259-268).

Zunächst wird eine Acetylcholin Dosis-Wirkungskurve in Abwesenheit von Testsubstanz (DMSO-Kontrolle) für die GIRK1/4-Aktivität bestimmt, indem Testlösungen mit ansteigender Acetylcholin-Konzentration perfundiert und die resultierenden Membranströme gemessen werden. Die Messung der Membranströme bzw. Änderung der Membranströme für eine gegebene ACh-Konzentration erfolgt dabei für ca. 10 bis 20 Sekunden. Nach Applikation der maximalen ACh-Konzentration innerhalb einer DWK-Reihe erfolgt die Perfusion einer Lösung mit Atropin (10 μM) gefolgt von einem Auswaschen der Substanzlösungen, um die M2-Selektivität und Reversibilität der M2-Aktivierung sicherzustellen. Die Änderungen der Membranströme werden entsprechend aufgenommen. Dabei wird für jede Acetylcholin-Konzentration der jeweils gemessene Membranstrom auf den maximalen Acetylcholin-induzierten Membranstrom normiert (I/IMax). Eine Acetylcholin Dosis-Wirkungskurve umfasst dabei fünf verschiedene Konzentrationen (InM, 10 nM, 100 nM, 1 μM, 10 μM). Der $EC_{50}$-Wert wird mit Hilfe einer 4-Parameter-Logistik-Funktion (Hill-Funktion) ermittelt.

Um den allosterischen Effekt der Testsubstanzen auf den M2 Rezeptor zu ermitteln, wird die Acetylcholin Dosis-Wirkungskurve für den GIRK1/4-Membranstrom in Anwesenheit einer konstanten Konzentration der jeweiligen Testsubstanz (z.B. 1 μM) bestimmt. Hierzu wird nach Präinkubation der Zelle mit der Testsubstanz für ca. 20 Sekunden und Messung der Membranströme eine Testlösung, welche dieselbe Substanzkonzentration und eine definierte ACh-Konzentrationen enthielt, für ca. 10 bis 20 Sekunden perfundiert und die Membranströme gemessen. Nach Applikation der maximalen Acetylcholin-Konzentration innerhalb einer Messreihe erfolgt wiederum die Perfusion einer Lösung mit Atropin (10 μM), um die M2-Selektivität des Substanzeffektes zu kontrollieren. Der $EC_{50}$-Wert in Anwesenheit von Testsubstanz wird analog mit Hilfe einer 4-Parameter-Logistik-Funktion (Hill-Funktion) ermittelt (s.o.).

Die Verschiebung der Acetylcholin Dosis-Wirkungskurve wird anhand der Veränderung des $EC_{50}$-Wertes für Acetylcholin in Ab- oder Anwesenheit der Testsubstanz ermittelt und quantifiziert.

## B-8. Effekte der Testsubstanzen auf das isolierte perfundierte Rattenherz

Männliche Wistar-Ratten (Stamm: HsdCpb:WU) mit einem Körpergewicht von 200-250g werden mit Narcoren (100 mg/kg) narkotisiert. Nach Eröffnen des Thorax wird das Herz frei präpariert, ausgeschnitten und durch Kanulieren der Aorta an eine Langendorff-Apparatur angeschlossen. Das Herz wird retrograd mit einer Krebs-Henseleit-Pufferlösung (begast mit 95% $O_2$ und 5% $CO_2$, pH 7,4, 35°C) mit folgender Zusammensetzung in mmol/1: NaCl 118; KCl 3; $NaHCO_3$ 22; $KH_2PO_4$ 1,2; Magnesiumsulfat 1,2; $CaCl_2$ 1,8; Glucose 10; Na-Pyruvat 2 mit 9 ml/min flußkonstant perfundiert. Für die Erfassung der Kontraktionskraft des Herzens wird durch eine Öffnung im linken Herzohr an einem PE-Schlauch befestigter und mit Wasser gefüllter Ballon aus dünner Kunststofffolie in den linken Ventrikel eingeführt. Der Ballon wird mit einem Druckaufnehmer verbunden. Der enddiastolische Druck wird auf 5-10 mmHg über das Ballonvolumen eingestellt. Die Daten werden von einem Brückenverstärker verstärkt und von einem Computer mit der LabChart Software (ADInstruments) registriert.

Um die allosterische Wirkung der Testsubstanzen zu untersuchen, werden die Herzen mit Zusatz von 300 nmol/l der Testsubstanz perfundiert. Nach 15 Min wird Carbachol kumulativ in ansteigenden Konzentrationen der Perfusionslösung hinzugefügt. Daraus resultierende Senkung der Herzfrequenz wird als Dosis-Wirkungs-Kurve mit Effekten auf Herzen verglichen, die mit Lösungsmittel statt Testsubstanz behandelt wurden. Die Verschiebung der Carbachol-Dosis-Wirkungskurve wird mittels GraphPad PRISM (sigmoidal dose-response) analysiert und quantifiziert.

## B-9. Effekte der Testsubstanzen auf die Herzfrequenz in narkotisierten Ratten

Männliche Ratten des Stamm (WI) WU Br des Züchters Charles River werden initial mit einer 4-5%-igen

Isofluran-Inhalation ca. 3 min narkotisiert. Danach erfolgt eine Erhaltungsnarkose mit einer 1,5 %-igen Isofluran-Inhalation. Dafür werden die narkotisierten Tiere dorsal auf einer beheizten Operationsplatte fixiert. Über visuelle Kontrolle und Zwischenzehenreflex wird die Narkosetiefe überprüft.

Für die Applikation der Testsubstanz wird ein i.v. Zugang an der Vena jugularis angelegt. Im Folgenden wird ein Hautschnitt in Längsrichtung von kaudal nach kranial durchgeführt und sowohl die Halsmuskulatur als auch die Speicheldrüsen durchtrennt. Die rechte Arteria carotis communis wird dargestellt und sowohl proximal als auch distal werden Blutsperren angelegt. Mit Mikroinstrumentarium wird ein TIP- Katheter (1.2F) in das Gefäß eingebracht um den arteriellen Druck und die Herzfrequenz zu messen.

Zunächst werden beide Parameter für 10 min im Basalzustand ohne Substanzgabe erfasst. Die zu untersuchenden Substanzen werden in geeigneten Lösungsmittelgemischen gelöst und anschliessend in verschiedenen Dosierungen jeweils einer Gruppe von Tieren über die Vena jugalaris über eine Infusionspumpe über 5 Min verabreicht. Eine Lösungsmittel-behandelte Gruppe wird als Kontrolle unter den gleichen Versuchsbedingungen eingesetzt. Die Erfassung des arteriellen Blutdruckes sowie der Herzfrequenz unter Substanzgabe erfolgt für 20 min. Die Daten werden mit dem PowerLab-System (ADinstruments) registriert und mit dem Programm LabChart (ADinstruments) ausgewertet.

## B-10. Radiotelemetrische Messung von Blutdruck und Herzfrequenz an wachen Ratten

Für die im Folgenden beschriebenen Messungen an wachen Ratten wird ein im Handel erhältliches Telemetriesystem der Firma Data Sciences International DSI, USA, eingesetzt. Das System besteht aus 3 Hauptkomponenten: (1) Implantierbare Sender (PhysioTel® Telemetrietransmitter), (2) Empfänger (PhysioTel® Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Ratten (Wistar Unilever/WU oder Spontaneous Hypertensive Rat/SHR) mit einem Körpergewicht von > 200 g durchgeführt. Die Versuchstiere werden nach Senderimplantation einzeln in Makrolon®-Käfigen Typ III gehalten. Sie haben freien Zugang zu Standardfutter und Wasser. Der Tag/Nacht-Rhythmus im Versuchslabor wird durch Wechsel der Raumbeleuchtung eingestellt.

Senderimplantation:

Die eingesetzten Telemetriesender (z.B. PA-C40, HD-S10, DSI) werden den Versuchstieren mindestens 14 Tage vor dem ersten Versuchseinsatz unter aseptischen Bedingungen chirurgisch implantiert. Zur Implantation werden die nüchternen Tiere mit Isofluran narkotisiert (IsoF1o®, Abbott, Einleitung 5%, Erhaltung 2%) und an der Bauchseite weiträumig rasiert und desinfiziert. Nach Eröffnung des Bauchraumes entlang der Linea alba wird der flüssigkeitsgefüllte Messkatheter des Systems oberhalb der Bifurkation nach cranial in die Aorta descendens eingesetzt und mit Gewebekleber (Vetbond™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Ursocyclin® 10%, 60 mg/kg s.c., 0.06 ml/100 g Körpergewicht, Serumwerk Bernburg AG, Deutschland) sowie ein Analgetikum (Rimadyl®, 4 mg/kg s.c., Pfizer, Deutschland) verabreicht.

Substanzen und Lösungen:

Wenn nicht anders beschrieben, werden die zu untersuchenden Substanzen jeweils einer Gruppe von Tieren (M= 6) oral verabreicht. Entsprechend einem Applikationsvolumen von 2 ml/kg Körpergewicht werden die Testsubstanzen in geeigneten Lösungsmittelgemischen gelöst. Eine Lösungsmittel-behandelte Gruppe von Tieren wird als Kontrolle eingesetzt.

Versuchsab lauf:

Die Telemetrie-Messeinrichtung ist für 24 Tiere konfiguriert. Den in der Anlage lebenden instrumentierten Ratten ist jeweils eine eigene Empfangsantenne zugeordnet (RPC-1 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI oder Ponemah, DSI) online erfasst und entsprechend aufgearbeitet werden. Im Standardablauf werden über je 10 Sekunden Dauer gemessen: (1) systolischer Blutdruck (SBP), (2) diastolischer Blutdruck (DBP), (3) arterieller Mitteldruck (MAP), (4) Herzfrequenz (HR) und (5) Aktivität (ACT). Diese Parameter werden im Anschluss an die Applikation über 24 Stunden gemessen. Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference

Monitor, APR-1, DSI) korrigiert.

Auswertung:

Nach Versuchsende werden die erhobenen Einzeldaten mit der Analysis-Software (Dataquest™ A.R.T. 4.1 Analysis oder Ponemah, DSI) sortiert. Als Leerwert wird der Zeitpunkt 2 Stunden vor Substanz-Applikation angenommen. Die Daten werden über eine voreinstellbare Zeit durch Mittelwertbestimmung geglättet (30 Minuten-Mittelwert).

**B-11. Effekte der Testsubstanzen auf die Herzfrequenz in anästhesierten Hunden**

Männliche oder weibliche Mischlingshunde (Mongrels, Marshall BioResources, USA) mit einem Gewicht zwischen 20 und 30 Kg werden mit Pentobarbital (30 mg/kg iv, Narcoren®, Merial, Deutschland) annarkotisiert. Pancuroniumchlorid (Pancuronium-Actavis®, Actavis, Deutschland, 1 mg/Tier iv) dient dabei zusätzlich als Muskelrelaxans. Die Hunde werden intubiert und mit einem Sauerstoff-Raumluft-Gemisch (40/60%) beatmet (etwa 5-6L/min). Die Beatmung erfolgt mit einem Beatmungsgerät der Firma GE Healthcare (Avance), welches gleichzeitig als Narkoseüberwachung dient (CO2-Analysator). Die Narkose wird aufrechterhalten durch eine ständige Infusion von Pentobarbital (50µg/kg/min); als Analgetikum dient Fentanyl (10µg/kg/h). Eine Alternative zu Pentobarbital besteht in der Verwendung von Isofluran (1-2Vol%).

Der Hund wird folgendermaßen instrumentiert:

- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem Drucksensor (Braun Melsungen, Melsungen, Deutschland) zur Messung des systemischen Blutdrucks
- Einführen eines NaCl-gefüllten Venenkatheter (Vygon, Deutschland) in die V.femoralis zur Infusion von Prüfsubstanzen oder zur Blutentnahme.
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Anbringen einer Ultraschall-Flussmess-Sonde (Transsonic Systems, Ithaka, USA) an der Aorta descendens zur Messung des Aortenflusses
- Anbringen einer Ultraschall-Flussmess-Sonde (Transsonic Systems, Ithaka, USA) an der linken A. coronaria zur Messung des Koronarflusses.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

**B-12. Effekte der Testsubstanzen auf die Herzfrequenz und Herzfrequenzvariabilität in gesunden, wachen Hunden**

Zur Charakterisierung von Prüfsubstanzen hinsichtlich ihrer Wirkung auf die Herzfrequenz, Herzfrequenzvariabilität (HRV) und Blutdruck werden telemetrische Messungen in gesunden, männlichen Beagle-Hunden durchgeführt.

Unter Isofluran-Narkose wird den Tieren zunächst ein Telemetriesender (Modell L21, Firma Data Sciences International, USA) implantiert. Dabei werden nach linksseitiger Thorakotomie Drucksensoren in der Aorta und im linken Ventrikel platziert. Zur Registrierung eines Elektrokardiogramms (EKG) werden weiterhin Elektroden auf dem Herzen platziert. Die Tiere werden anschließend zur Wundheilung unter antibiotischer (Clindamycin, Zoetis, Deutschland) und analgetischer (Fentanyl, Janssen, Deutschland) Nachsorge zurück in den Stall verbracht. Mittels im Tierstall installierter Antennen werden die Blutdruck- und EKG-Signale an einen Datenakquisitionscomputer weitergeleitet und durch eine Analysesoftware (Ponemah, Data Sciences International, USA) ausgewertet. Die Telemetrie-Anlage ermöglicht eine kontinuierliche Erfassung von Blutdrücken und EKG-Signalen in wachen Tieren. Technische Details können der Dokumentation der Herstellerfirma (Data Sciences International, USA) entnommen werden.

Die zu untersuchenden Substanzen werden den gesunden Hunden mittels einer Gelatine-Kapsel in geeigneten Lösungsmittelgemischen oral verabreicht. Eine Vehikel-behandelte Gruppe von Tieren wird als Kontrolle eingesetzt. Die telemetrische Messung wird vor Substanzgabe gestartet und über einen Zeitraum von mehreren Stunden aufgezeichnet. Die graphische Darstellung der zeitlichen Verläufe der durch Mittelwertbestimmung geglätteten Daten erfolgt mit Hilfe der GraphPadPrism Software (GraphPad, USA). Zur Analyse der HRV werden die EKG-Daten einer frequenzbezogenen Herzfrequenzvariabilitäts-Analyse ("frequency-domain") unterzogen. Dazu werden die R-R-Intervalle der aufgezeichneten EKG s verwendet. Daten außerhalb des zuvor definierten Bereiches von 0,2s - 1,5s werden von der Analyse ausgeschlossen. Die ausgeschlossenen Daten werden durch Werte die durch lineare Interpolation gewonnen wurden ersetzt. Diese Daten werden durch Spline-Interpolation in gleichabständige Unterstützungspunkte konvertiert. Zur Analyse der Herzfrequenzvariabilität werden die Daten weiterhin in 30 s Schritten zu Paketen von 300s Länge unterteilt. Für jedes Datenpaket wird eine Fourier-Transformation kalkuliert. Daraus wird weiterhin die Leistung in drei Frequenzbändern (vlf=0.0033 - 0.04 1/s; lf=0.04 - 0.15 1/s; hf=0.15 - 0.5 1/s) kalkuliert. Zur Charakterisierung der Prüfsubstanz wird die Gesamtleistung (Summe aller drei Frequenzbänder) zur HRV- Analyse herangezogen.

## Patentansprüche

**1.** Verbindung der Formel (I)

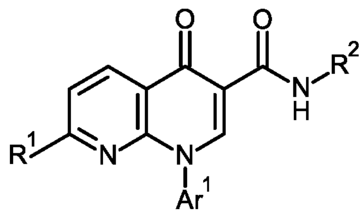

(I),

in welcher

$R^1$ für $NR^3R^4$ steht,
worin

$R^3$ Wasserstoff, Methyl, $(C_2\text{-}C_4)$-Alkyl oder $(C_3\text{-}C_6)$-Cycloalkyl bedeutet,
wobei $(C_2\text{-}C_4)$-Alkyl mit Hydroxy oder bis zu dreifach mit Fluor substituiert sein kann

und

$R^4$ $(C_1\text{-}C_6)$-Alkyl, $(C_3\text{-}C_6)$-Cycloalkyl, 3- bis 6-gliedriges gesättigtes Heterocyclyl oder $(C_1\text{-}C_4)$-Alkylsulfonyl bedeutet,
wobei $(C_1\text{-}C_6)$-Alkyl, $(C_3\text{-}C_6)$-Cycloalkyl und 3- bis 6-gliedriges gesättigtes Heterocyclyl, bis zu dreifach, gleich oder verschieden, mit Methyl, Difluormethyl, Trifluormethyl, Hydroxy, Hydroxycarbonyl, Oxo, Methoxy, Difluormethoxy, Trifluormethoxy, Cyano und weiterhin bis zu vierfach mit Fluor, substituiert sein können,

oder

$R^3$ und $R^4$ bilden zusammen mit dem Stickstoffatom, an das sie gebunden sind, einen gesättigten oder partiell ungesättigten, 3- bis 6-gliedrigen monocyclischen oder 6- bis 10-gliedrigen bicyclischen Heterocyclus, der ein oder zwei weitere, gleiche oder verschiedene Heteroatome aus der Reihe N, O, S, SO und/oder

$SO_2$ als Ringglieder enthalten kann,
wobei der 3- bis 6-gliedrige monocyclische und der 6- bis 10-gliedrige bicyclische Heterocyclus jeweils mit 1 bis 5 Substituenten unabhängig voneinander ausgewählt aus der Gruppe ($C_1$-$C_4$)-Alkyl, Difluormethyl, Trifluormethyl, Hydroxy, Hydroxycarbonyl, Oxo, ($C_1$-$C_3$)-Alkoxy, Difluormethoxy, Trifluormethoxy, Cyano, ($C_1$-$C_3$)-Alkoxycarbonyl, Aminocarbonyl, Mono-($C_1$-$C_3$)-alkylaminocarbonyloxy, -NHC(=O)$R^{14A}$, -$CH_2$NHC(=O)$R^{14B}$, und weiterhin bis zu vierfach mit Fluor, substituiert sein können, worin

$R^{14A}$ und $R^{14B}$ unabhängig voneinander ($C_1$-$C_3$)-Alkyl oder Cyclopropyl darstellen,

und
worin ($C_1$-$C_4$)-Alkyl ein- oder zweifach, gleich oder verschieden, mit Hydroxy, ($C_1$-$C_3$)-Alkoxy, und bis zu vierfach mit Fluor, substituiert sein kann,

$R^2$ für eine Gruppe der Formel

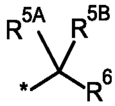
,
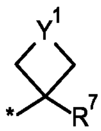
oder
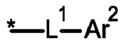

steht, worin

* die Verknüpfungsstelle mit dem N-Atom der Amid-Gruppierung markiert,
$R^{5A}$ Wasserstoff oder ($C_1$-$C_4$)-Alkyl bedeutet,
$R^{5B}$ Wasserstoff, ($C_1$-$C_4$)-Alkyl, Cyclopropyl, Monofluormethyl, Difluormethyl, Trifluormethyl, Pentafluorethyl, Methoxymethyl oder Trifluormethoxymethyl bedeutet,
$R^6$ bis zu fünffach mit Fluor substituiertes ($C_1$-$C_4$)-Alkyl oder bis zu vierfach mit Fluor substituiertes ($C_3$-$C_5$)-Cycloalkyl bedeutet,
$Y^1$ -$(CH_2)_k$-, -$CF_2$-, -O-$CH_2$-, -$CH_2$-O- oder -$CH_2$-O-$CH_2$- bedeutet,
worin

k für 0, 1, 2 oder 3 steht,

$R^7$ Wasserstoff, bis zu fünffach mit Fluor substituiertes ($C_1$-$C_2$)-Alkyl oder Trifluormethoxymethyl bedeutet,
$L^1$ eine Bindung oder eine Gruppe der Formel -C($R^{8A}R^{8B}$)-(C($R^{9A}R^{9B}$))$_m$- bedeutet,
worin

m 0 oder 1darstellt,
$R^{8A}$ Wasserstoff oder Methyl darstellt,
$R^{8B}$ Wasserstoff, Methyl, Trifluormethyl, Pentafluorethyl oder Trifluormethoxymethyl darstellt,
$R^{9A}$ und $R^{9B}$ unabhängig voneinander Wasserstoff oder Methyl darstellen,

$Ar^2$ Phenyl bedeutet,
wobei Phenyl ein- bis dreifach, gleich oder verschieden, mit Fluor, Chlor, ($C_1$-$C_3$)-Alkyl, Difluormethoxymethyl, Trifluormethoxymethyl und/oder Trifluormethyl substituiert sein kann,

oder
für einen 5- bis 10-gliedrigen bicyclischen oder tricyclischen Carbocyclus steht,
wobei der 5- bis 10-gliedrige bicyclische oder tricyclische Carbocyclus bis zu dreifach, gleich oder verschieden, mit ($C_1$-$C_3$)-Alkyl, Trifluormethyl, und weiterhin bis zu vierfach mit Fluor substituiert sein kann,
$Ar^1$ für einen über ein Ring-Kohlenstoffatom angebundenen Pyridinring steht, wobei der Pyridinring ein oder zweifach mit Fluor, Chlor, Cyano, Methyl oder Trifluormethyl substituiert sein kann,

sowie ihre *N*-Oxide, Salze, Solvate, Salze der *N*-Oxide und Solvate der *N*-Oxide und Salze.

**2.** Verbindung der Formel (I) nach Anspruch 1,
in welcher

$R^1$ für $NR^3R^4$ steht,
worin

$R^3$ Wasserstoff, Methyl oder ($C_2$-$C_4$)-Alkyl bedeutet, und
$R^4$ bis zu vierfach mit Fluor substituiertes ($C_1$-$C_6$)-Alkyl bedeutet,
wobei ($C_1$-$C_6$)-Alkyl mit Oxo substituiert sein kann,

oder

$R^3$ und $R^4$ zusammen mit dem Stickstoffatom, an das sie gebunden sind, einen gesättigten 4- bis 6-gliedrigen monocyclischen oder 6- bis 9-gliedrigen bicyclischen Heterocyclus bilden, der ein oder zwei weitere, gleiche oder verschiedene Heteroatome aus der Reihe N und/oder O als Ringglieder enthalten kann,
wobei der 4- bis 6-gliedrige monocyclische und der 6- bis 9-gliedrige bicyclische Heterocyclus jeweils mit 1 bis 5 Substituenten unabhängig voneinander ausgewählt aus der Gruppe ($C_1$-$C_4$)-Alkyl, Difluormethyl, Trifluormethyl, Hydroxy, Oxo, ($C_1$-$C_3$)-Alkoxy, Difluormethoxy, Trifluormethoxy und weiterhin bis zu vierfach mit Fluor, substituiert sein können,
worin ($C_1$-$C_4$)-Alkyl ein- oder zweifach, gleich oder verschieden, mit Hydroxy, ($C_1$-$C_3$)-Alkoxy, und bis zu vierfach mit Fluor, substituiert sein kann,

$R^2$ für eine Gruppe der Formel

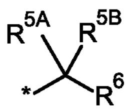 , 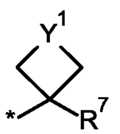 , 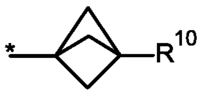 oder 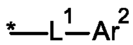

steht, worin

* die Verknüpfungsstelle mit dem N-Atom der Amid-Gruppierung markiert,
$R^{5A}$ Wasserstoff oder ($C_1$-$C_4$)-Alkyl bedeutet,
$R^{5B}$ Methyl, Monofluormethyl, Difluormethyl, Trifluormethyl, Pentafluorethyl oder Trifluormethoxymethyl bedeutet, und
$R^6$ bis zu fünffach mit Fluor substituiertes ($C_1$-$C_4$)-Alkyl oder bis zu vierfach mit Fluor substituiertes ($C_3$-$C_5$)-Cycloalkyl bedeutet,
$Y^1$ -$(CH_2)_k$- bedeutet,
worin

k für 1 oder 2 steht,

$R^7$ bis zu fünffach mit Fluor substituiertes ($C_1$-$C_2$)-Alkyl bedeutet,
$R^{10}$ Wasserstoff, Fluor oder Trifluormethyl bedeutet,
$L^1$ eine Bindung oder eine Gruppe der Formel -$CR^{8A}R^{8B}$- bedeutet,
worin

$R^{8A}$ Wasserstoff darstellt,
$R^{8B}$ Methyl, Trifluormethyl, Pentafluorethyl oder Trifluormethoxymethyl darstellt,

$Ar^2$ Phenyl bedeutet,
wobei Phenyl ein- bis dreifach, gleich oder verschieden, mit Fluor, Chlor, ($C_1$-$C_3$)-Alkyl, und/oder Trifluormethyl substituiert sein kann,

$Ar^1$ für einen über ein Ring-Kohlenstoffatom angebundenen Pyridinring steht,
wobei der Pyridinring ein oder zweifach mit Fluor, Chlor, Cyano, Methyl oder Trifluormethyl substituiert sein kann,

sowie ihre *N*-Oxide, Salze, Solvate, Salze der *N*-Oxide und Solvate der *N*-Oxide und Salze.

**3.** Verbindung der Formel (I) nach Anspruch 1 oder 2,

in welcher

$R^1$ für $NR^3R^4$ steht,
worin

$R^3$ Wasserstoff oder Methyl bedeutet, und
R4 Methyl oder 2-Fluorethyl bedeutet,

oder
für einen über ein Stickstoff-Atom gebundenen Heterocyclus der Formel

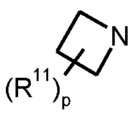 , 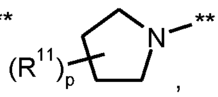 , 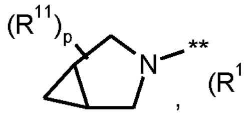 , 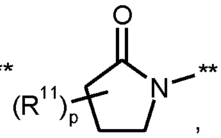 ,

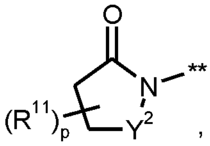 , 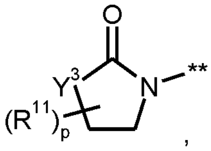 , 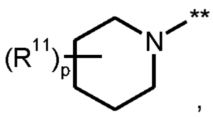 ,

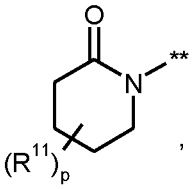 , 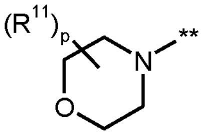 oder 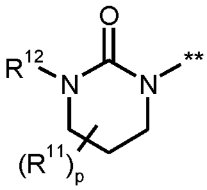

steht, worin

** die Verknüpfungsstelle mit dem Rest des Moleküls markiert,
$Y^2$ eine Gruppe der Formel

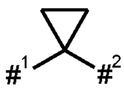

darstellt, worin

$\#^1$ die Verknüpfungsstelle mit dem N-Atom des Pyrrolidinonrings markiert, und
$\#^2$ die Verknüpfungsstelle mit dem C-Atom des Pyrrolidinonrings markiert,

$Y^3$ -N($R^{12}$)- oder eine Gruppe der Formel

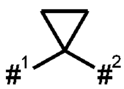

darstellt, worin

$\#^1$ und $\#^2$ jeweils die Verknüpfungsstelle mit dem C-Atom des Pyrrolidinonrings markieren,

$R^{11}$ Fluor, ($C_1$-$C_4$)-Alkyl, Difluormethyl, Trifluormethyl, Hydroxy, Hydroxymethyl, Methoxy, Difluormethoxy oder Trifluormethoxy darstellt,

p die Zahl 0, 1, 2, 3 oder 4 darstellt,
wobei im Fall, dass die Substituenten $R^{11}$ mehrfach auftreten, deren Bedeutungen jeweils gleich oder verschieden sein können,
$R^{12}$ Wasserstoff oder 2-Hydroxyethyl darstellt,

$R^2$ für eine Gruppe der Formel

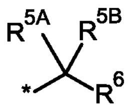 , 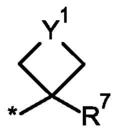 , 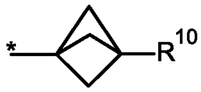 oder 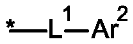

steht, worin

* die Verknüpfungsstelle mit dem N-Atom der Amid-Gruppierung markiert,
$R^{5A}$ Wasserstoff oder Methyl bedeutet,
$R^{5B}$ Methyl, Monofluormethyl, Difluormethyl, Trifluormethyl, Pentafluorethyl oder Trifluormethoxymethyl bedeutet, und
$R^6$ Methyl, Ethyl, Trifluormethyl, 2,2,2-Trifluorethyl, tert-Butyl, iso-Butyl oder Cyclopropyl bedeutet,
$Y^1$ $-(CH_2)_k-$ bedeutet,
worin

k für 1 oder 2 steht,

$R^7$ Trifluormethyl bedeutet,
$R^{10}$ Wasserstoff, Fluor oder Trifluormethyl bedeutet,
$L^1$ eine Bindung oder eine Gruppe der Formel $-CR^{8A}R^{8B}-$ bedeutet,
worin

$R^{8A}$ Wasserstoff darstellt,
$R^{8B}$ Trifluormethyl darstellt,

$Ar^2$ Phenyl bedeutet,
wobei Phenyl ein- oder zweifach, gleich oder verschieden, mit Fluor und/oder Chlor substituiert sein kann,

$Ar^1$ für eine Gruppe der Formel

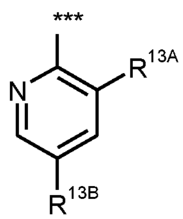 oder 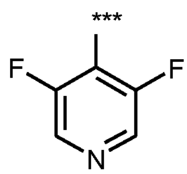

steht, worin

*** die Verknüpfungsstelle mit dem N-Atom markiert,
$R^{13A}$ für Fluor oder Chlor steht,
$R^{13B}$ für Fluor oder Wasserstoff steht,

sowie ihre Salze, Solvate und Solvate der Salze.

**4.** Verbindung der Formel (I) nach einem der Ansprüche 1 bis 3,
in welcher

$R^1$ für einen über ein Stickstoff-Atom gebundenen Heterocyclus der Formel

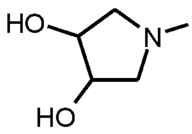, 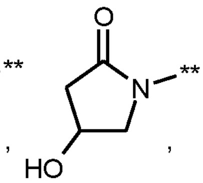, 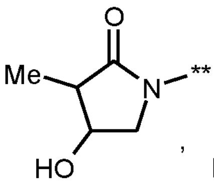, 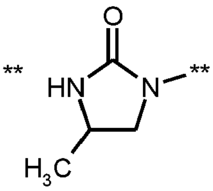 oder 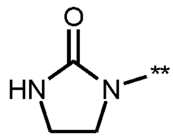

steht, worin

** die Verknüpfungsstelle mit dem Rest des Moleküls markiert,

$R^2$ für eine Gruppe der Formel

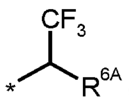, 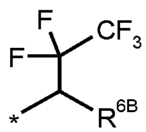, 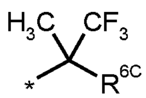, 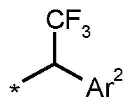 oder 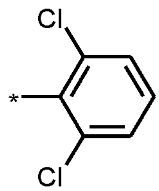

steht, worin

* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl oder Ethyl bedeutet,
$R^{6C}$ Trifluormethyl oder Cyclopropyl bedeutet,
$Ar^2$ für eine Gruppe der Formel

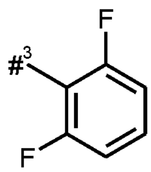 oder 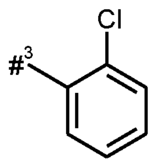

steht, worin

$\#^3$ jeweils die Verknüpfungsstelle markiert,

$Ar^1$ für eine Gruppe der Formel

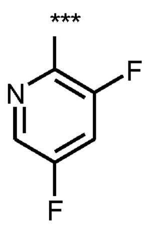

steht, worin

*** die Verknüpfungsstelle mit dem N-Atom markiert,

sowie ihre Salze, Solvate und Solvate der Salze.

**5.** Verbindung der Formel (I) nach Anspruch 1 oder 2,

$R^1$ für einen über ein Stickstoff-Atom gebundenen Heterocyclus der Formel

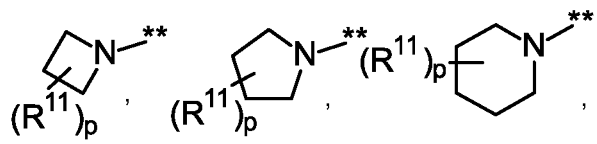

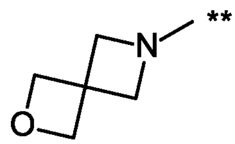

steht, worin

** die Verknüpfungsstelle mit dem Rest des Moleküls markiert,
$R^{11}$ Fluor, $(C_1\text{-}C_4)$-Alkyl, Difluormethyl, Trifluormethyl, Hydroxy, Hydroxymethyl, Methoxy, Difluormethoxy oder Trifluormethoxy darstellt,
p die Zahl 0, 1, 2, 3 oder 4 darstellt,
wobei im Fall, dass die Substituenten $R^{11}$ mehrfach auftreten, deren Bedeutungen jeweils gleich oder verschieden sein können,

$R^2$ für eine Gruppe der Formel

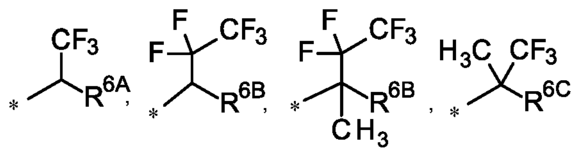

steht, worin

* die Verknüpfungsstelle mit dem N-Atom der Amid-Gruppierung markiert,
$R^{6A}$ Trifluormethyl, Ethyl, tert-Butyl, Isobutyl oder Cyclopropyl bedeutet,
$R^{6B}$ Methyl, Ethyl, tert-Butyl oder Cyclopropyl bedeutet,
$R^{6C}$ Trifluormethyl oder Cyclopropyl bedeutet,

$Ar^1$ für eine Gruppe der Formel

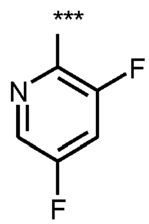

steht, worin

*** die Verknüpfungsstelle mit dem N-Atom markiert,

sowie ihre Salze, Solvate und Solvate der Salze.

**6.** Verbindung der Formel (I) nach Anspruch 5,

$R^1$ für einen über ein Stickstoff-Atom gebundenen Heterocyclus der Formel

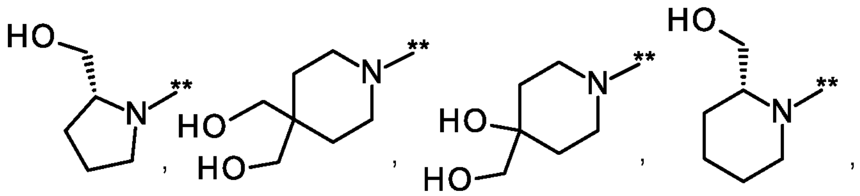

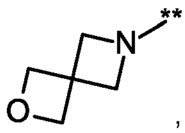 , 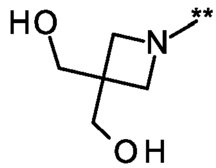

steht, worin

** die Verknüpfungsstelle mit dem Rest des Moleküls markiert,

$R^2$ für eine Gruppe der Formel

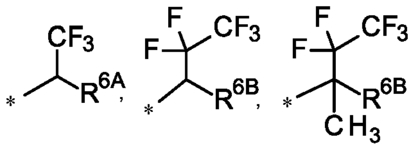

steht, worin

* die Verknüpfungsstelle mit dem N-Atom der Amid-Gruppierung markiert,
$R^{6A}$ Trifluormethyl bedeutet,
$R^{6B}$ Methyl bedeutet,

$Ar^1$ für eine Gruppe der Formel

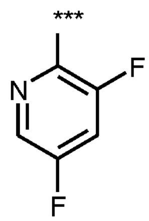

steht, worin

*** die Verknüpfungsstelle mit dem N-Atom markiert,

sowie ihre Salze, Solvate und Solvate der Salze.

**7.** Verbindung nach Anspruch 1, ausgewählt aus N-[(1R)-1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomerengemisch) der Formel

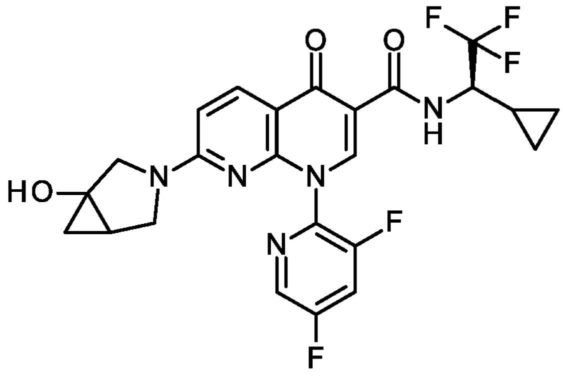

dem N-[(1R)-1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomer 1) und dem N-[(1R)-1-Cyclopropyl-2,2,2-trifluorethyl]-1-(3,5-difluorpyridin-2-yl)-7-[1-hydroxy-3-azabicyclo[3.1.0]hex-3-yl]-4-oxo-1,4-dihydro-1,8-naphthyridin-3-carbonsäureamid (Diastereomer 2).

**8.** Verbindung nach Anspruch 1, ausgewählt aus 1-(3,5-Difluorpyridin-2-yl)-N-(1,1,1,3,3,3-hexafluorpropan-2-yl)-7-[1-hydroxy-3-azabicyclo[3.1,0]hex-3-yl]-4-oxo-1,4-dihydro-1,8-naphthyridin-3-carbonsäureamid (Racemat) der Formel

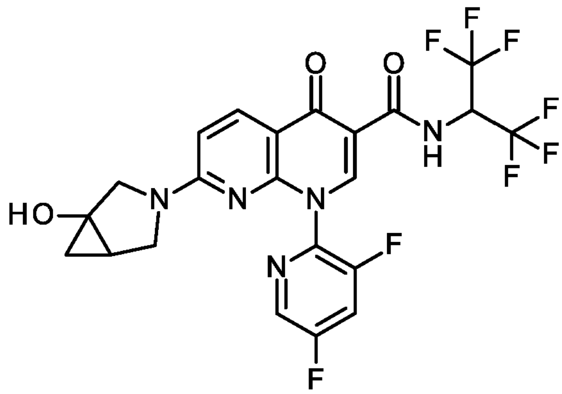

dem 1-(3,5-Difluorpyridin-2-yl)-N-(1,1,1,3,3,3-hexafluorpropan-2-yl)-7-[1-hydroxy-3-azabicyclo-[3.1.0]hex-3-yl]-4-oxo-1,4-dihydro-1,8-naphthyridin-3-carbonsäureamid (Diastereomer 1) und dem 1-(3,5-Difluorpyridin-2-yl)-N-(1,1,1,3,3,3-hexafluorpropan-2-yl)-7-[1-hydroxy-3-azabicyclo-[3.1.0]hex-3-yl]-4-oxo-1,4-dihydro-1,8-naphthyridin-3-carbonsäureamid (Diastereomer 2).

**9.** Verbindung nach Anspruch 1,1-(3,5-Difluorpyridin-2-yl)-7-[(4S)-4-hydroxy-2-oxopyrrolidin-1-yl]-4-oxo-N-[(2R)-1,1,1-trifluor-4-methylpentan-2-yl]-1,4-dihydro-1,8-naphthyridin-3-carbonsäureamid der Formel

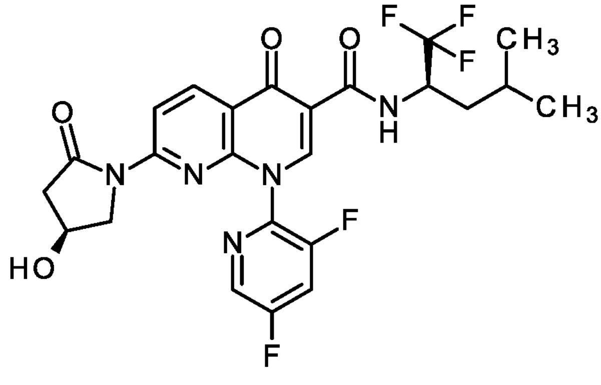

**10.** Verbindung nach Anspruch 1,1-(3,5-Difluorpyridin-2-yl)-7-[(4S)-4-hydroxy-2-oxopyrrolidin-1-yl]-4-oxo-N-[(2R)-1,1,1-trifluor-3,3-dimethylbutan-2-yl]-1,4-dihydro-1,8-naphthyridin-3-carbonsäureamid der Formel

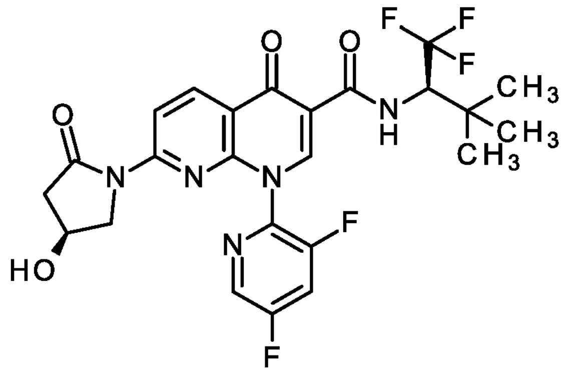

**11.** Verbindung nach Anspruch 1, 1-(3,5-Difluorpyridin-2-yl)-7-[(3R4R)-3,4-dihydroxypyrrolidin-1-yl]-4-oxo-N-[(2R)-1,1,1-trifluor-4-methylpentan-2-yl]-1,4-dihydro-1,8-naphthyridin-3-carbonsäureamid der Formel

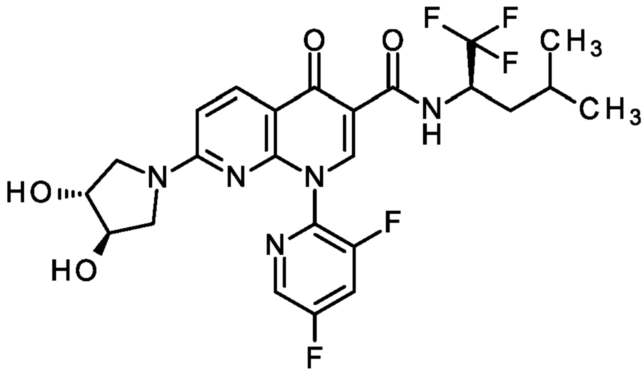

**12.** Verbindung nach Anspruch 1,1-(3,5-Difluorpyridin-2-yl)-7-[(3R4R)-3,4-dihydroxypyrrolidin-1-yl]-4-oxo-N-[(2R)-1,1,1-trifluor-3,3-dimethylbutan-2-yl]-1,4-dihydro-1,8-naphthyridin-3-carbonsäureamid der Formel

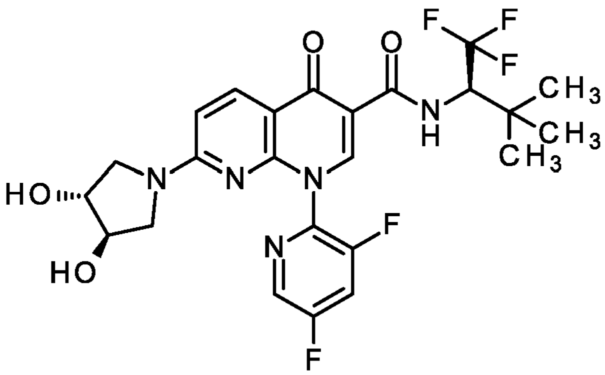

**13.** Verbindung nach Anspruch 1, ausgewählt aus 1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomerengemisch) der Formel

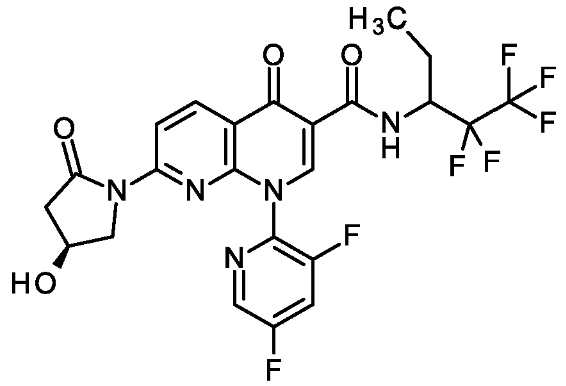

dem 1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomer 1) und dem 1-(3,5-Difluorpyridin-2-yl)-7-[(4S)-4-hydroxy-2-oxopyrrolidin-1-yl]-4-oxo-N-[1,1,1,2,2-pentafluorpentan-3-yl]-1,4-dihydro-1,8-naphthyridin-3-carbonsäureamid (Diastereomer 2).

**14.** Verfahren zur Herstellung von Verbindungen der Formel (I) wie in einem der Ansprüche 1 bis 13 definiert, **dadurch gekennzeichnet, dass** man

[A] eine Verbindung der Formel (II)

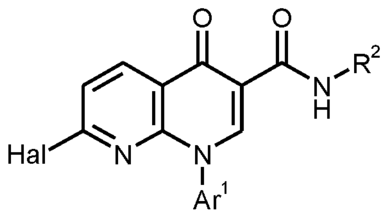

(II),

in welcher $R^2$ und $Ar^1$ die in den Ansprüchen 1 bis 6 angegebenen Bedeutungen haben und
Hal für Fluor, Chlor, Brom oder Iod, bevorzugt für Chlor, steht,

mit einer Verbindung der Formel (III)

$R^1$- H (III),

in welcher $R^1$ die oben angegebene Bedeutung hat,
zum Carbonsäureamid der Formel (I)

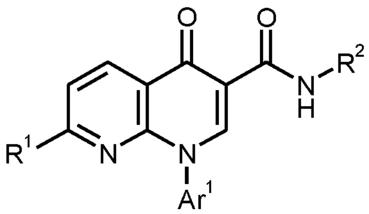

(I),

in welcher $R^1$, $R^2$ und $Ar^1$ die in den Ansprüchen 1 bis 6 angegebenen Bedeutungen haben, umsetzt,
oder
[B] eine Verbindung der Formel (IV)

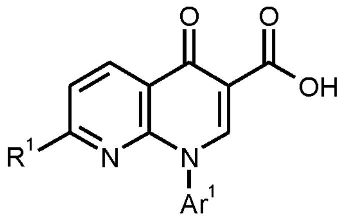

(IV),

in welcher $R^1$ und $Ar^1$ die in den Ansprüchen 1 bis 6 angegebenen Bedeutungen haben, mit einer Verbindung der Formel (V)

$R^2$-$NH_2$ (V),

in welcher $R^2$ die oben angegebene Bedeutung hat, zum Carbonsäureamid der Formel (I)

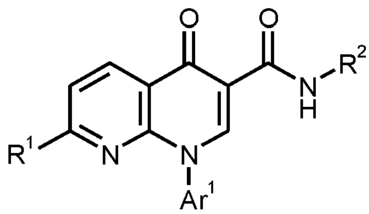

(I),

in welcher $R^1$, $R^2$ und $Ar^1$ die in den Ansprüchen 1 bis 6 angegebenen Bedeutungen haben, umsetzt, und gegebenenfalls die so erhaltenen Verbindungen der Formel (I) in ihre Enantiomere und/oder Diastereomere trennt und/oder mit den entsprechenden (*i*) Lösungsmitteln und/oder *(ii)* Basen oder Säuren in ihre Solvate, Salze und/oder Solvate der Salze überführt.

**15.** Verbindung der Formel (II)

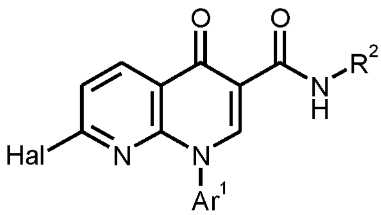

(II),

in welcher $R^2$ und $Ar^1$ die in den Ansprüchen 1 bis 6 für Verbindungen der Formel (I) angegebenen Bedeutungen haben und Hal für Fluor, Chlor, Brom oder Iod, bevorzugt für Chlor, steht.

**16.** Verbindung der Formel (IV)

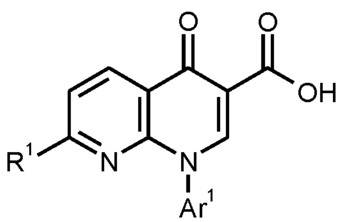

(IV),

in welcher $R^1$ und $Ar^1$ die in den Ansprüchen 1 bis 6 für Verbindungen der Formel (I) angegebenen Bedeutungen haben.

**17.** Verwendung einer Verbindung der Formel (II)

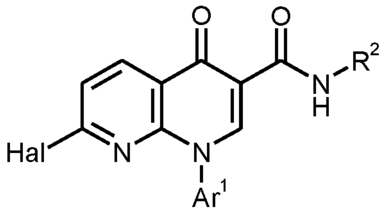

(II),

in welcher $R^2$ und $Ar^1$ die in den Ansprüchen 1 bis 6 für Verbindungen der Formel (I) angegebenen Bedeutungen haben
und
Hal für Fluor, Chlor, Brom oder Iod, bevorzugt für Chlor, steht.

oder
einer Verbindung der Formel (IV)

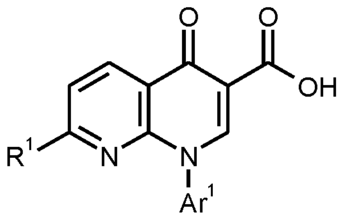

(IV),

in welcher $R^1$ und $Ar^1$ die in den Ansprüchen 1 bis 6 für Verbindungen der Formel (I) angegebenen Bedeutungen haben,
zur Herstellung einer Verbindung der Formel (I) gemäß einem der Ansprüche 1 bis 13.

**18.** Verbindung, wie in einem der Ansprüche 1 bis 13 definiert, zur Verwendung bei der Behandlung und/oder Prophylaxe von Krankheiten.

**19.** Verbindung der Formel (I), wie in einem der Ansprüche 1 bis 13 definiert, zur Verwendung in einen Verfahren zur Behandlung und/oder Prophylaxe von Herzinsuffizienz, koronarer Herzerkrankung, atrialen und ventrikulären Arrhythmien, Niereninsuffizienz und Nephropathien.

**20.** Arzneimittel enthaltend eine Verbindung, wie in einem der Ansprüche 1 bis 13 definiert, in Kombination mit einem oder mehreren weiteren Wirkstoffen ausgewählt aus der Gruppe bestehend aus den Blutdruck senkende Wirkstoffe, antiarrhythmische Wirkstoffe, Vasopressin-Rezeptor-Antagonisten, PDE 5-Inhibitoren, Thrombozytenaggregationshemmer, sGC-Aktivatoren und sGC-Stimulatoren.

**21.** Arzneimittel enthaltend eine Verbindung, wie in einem der Ansprüche 1 bis 13 definiert, in Kombination mit einem inerten, nichttoxischen, pharmazeutisch geeigneten Hilfsstoff.

**22.** Arzneimittel nach Anspruch 20 oder 21 zur Verwendung bei der Behandlung und/oder Prophylaxe von Herzinsuffizienz, koronarer Herzerkrankung, atrialen und ventrikulären Arrhythmien, Niereninsuffizienz und Nephropathien.

## Claims

**1.** Compound of the formula (I)

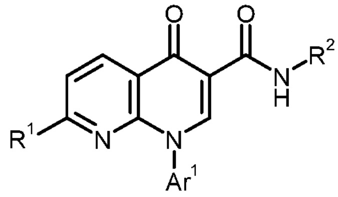

(I),

in which

$R^1$ represents $NR^3R^4$,
in which

$R^3$ represents hydrogen, methyl, $(C_2-C_4)$ -alkyl or $(C_3-C_6)$ -cycloalkyl,

where $(C_2-C_4)$-alkyl may be substituted by hydroxy or up to trisubstituted by fluorine
and

$R^4$ represents $(C_1-C_6)$-alkyl, $(C_3-C_6)$ -cycloalkyl, 3- to 6-membered saturated heterocyclyl or $(C_1-C_4)$-alkylsulfonyl,
where $(C_1-C_6)$-alkyl, $(C_3-C_6)$ -cycloalkyl and 3-to 6-membered saturated heterocyclyl may be up to trisubstituted by identical or different substituents from the group consisting of methyl, difluoromethyl, trifluoromethyl, hydroxy, hydroxycarbonyl, oxo, methoxy, difluoromethoxy, trifluoromethoxy and cyano and furthermore up to tetrasubstituted by fluorine,

or

$R^3$ and $R^4$ together with the nitrogen atom to which they are attached form a saturated or partially unsaturated 3- to 6-membered monocyclic or 6- to 10-membered bicyclic heterocycle which may contain one or two further identical or different heteroatoms from the group consisting of N, O, S, SO and/or $SO_2$ as ring members,

where the 3- to 6-membered monocyclic and the 6-to 10-membered bicyclic heterocycle may each be substituted by 1 to 5 substituents independently selected from the group of $(C_1-C_4)$-alkyl, difluoromethyl, trifluoromethyl, hydroxy, hydroxycarbonyl, oxo, $(C_1-C_3)$-alkoxy, difluoromethoxy, trifluoromethoxy, cyano, $(C_1-C_3)$-alkoxycarbonyl, aminocarbonyl, mono-$(C_1-C_3)$-alkylaminocarbonyloxy,-NHC(=O)$R^{14A}$ and
-$CH_2$NHC (=O)$R^{14B}$, and additionally up to tetrasubstituted by fluorine, in which

$R^{14A}$ and $R^{14B}$ independently represent $(C_1-C_3)$-alkyl or cyclopropyl,
and
where $(C_1-C_4)$-alkyl may be mono- or disubstituted by identical or different substituents from the group consisting of hydroxy and $(C_1-C_3)$-alkoxy, and up to tetrasubstituted by fluorine,

$R^2$ represents a group of the formula

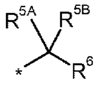

,

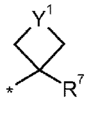

or

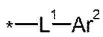

in which

* marks the point of attachment to the nitrogen atom of the amide moiety,

$R^{5A}$ represents hydrogen or $(C_1-C_4)$-alkyl,
$R^{5B}$ represents hydrogen, $(C_1-C_4)$-alkyl, cyclopropyl, monofluoromethyl, difluoromethyl, trifluoromethyl, pentafluoroethyl, methoxymethyl or trifluoromethoxymethyl,
$R^6$ represents $(C_1-C_4)$-alkyl which is up to pentasubstituted by fluorine, or $(C_3-C_5)$-cycloalkyl which is

up to tetrasubstituted by fluorine,
$Y^1$ is $-(CH_2)_k-$, $-CF_2-$, $-O-CH_2-$, $-CH_2-O-$ or $-CH_2-O-CH_2-$,

in which

k represents 0, 1, 2 or 3,
$R^7$ represents hydrogen, $(C_1-C_2)$-alkyl which is up to pentasubstituted by fluorine, or trifluoromethoxymethyl,

$L^1$ represents a bond or a group of the formula $-C(R^{8A}R^{8B}) - (C(R^{9A}R^{9B}))_m-$,
in which

m represents 0 or 1,
$R^{8A}$ represents hydrogen or methyl,

$R^{8B}$ represents hydrogen, methyl, trifluoromethyl, pentafluoroethyl or trifluoromethoxymethyl,

$R^{9A}$ and $R^{9B}$ independently represent hydrogen or methyl,
$Ar^2$ represents phenyl,
where phenyl may be mono- to trisubstituted by identical or different substituents from the group consisting of fluorine, chlorine, $(C_1-C_3)$-alkyl, difluoromethoxymethyl, trifluoromethoxymethyl and/or trifluoromethyl,

or
represents a 5- to 10-membered bicyclic or tricyclic carbocycle,
where the 5- to 10-membered bicyclic or tricyclic carbocycle may be up to trisubstituted, identically or differently, by $(C_1-C_3)$-alkyl and trifluoromethyl, and additionally up to tetrasubstituted by fluorine,
$Ar^1$ represents a pyridine ring which is attached via a ring carbon atom,
where the pyridine ring may be mono- or disubstituted by fluorine, chlorine, cyano, methyl or trifluoromethyl,

and the *N*-oxides, salts, solvates, salts of the *N*-oxides and solvates of the *N*-oxides and salts thereof.

**2.** Compound of the formula (I) according to Claim 1, in which

$R^1$ represents $NR^3R^4$,
in which

$R^3$ represents hydrogen, methyl or $(C_2-C_4)$ -alkyl, and
$R^4$ represents $(C_1-C_6)$-alkyl which is up to tetrasubstituted by fluorine,
where $(C_1-C_6)$-alkyl may be substituted by oxo,

or

$R^3$ and $R^4$ together with the nitrogen atom to which they are attached form a saturated 4- to 6-membered monocyclic or 6- to 9-membered bicyclic heterocycle which may contain one or two further identical or different heteroatoms from the group consisting of N and/or O as ring members,

where the 4- to 6-membered monocyclic and 6- to 9-membered bicyclic heterocycle may each be substituted by 1 to 5 substituents independently selected from the group of $(C_1-C_4)$-alkyl, difluoromethyl, trifluoromethyl, hydroxy, oxo, $(C_1-C_3)$-alkoxy, difluoromethoxy, trifluoromethoxy, and furthermore up to tetrasubstituted by fluorine,
where $(C_1-C_4)$-alkyl may be mono- or disubstituted by identical or different substituents from the group consisting of hydroxy and $(C_1-C_3)$-alkoxy, and up to tetrasubstituted by fluorine,
$R^2$ represents a group of the formula

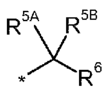 , 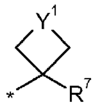 , 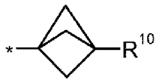 or 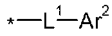

in which

* marks the point of attachment to the nitrogen atom of the amide moiety,
$R^{5A}$ represents hydrogen or $(C_1-C_4)$-alkyl,
$R^{5B}$ represents methyl, monofluoromethyl, difluoromethyl, trifluoromethyl, pentafluoroethyl or trifluoromethoxymethyl, and
$R^6$ represents $(C_1-C_4)$-alkyl which is up to pentasubstituted by fluorine, or $(C_3-C_5)$-cycloalkyl which is up to tetrasubstituted by fluorine,
$Y^1$ represents - $(CH_2)_k$-,

in which

k represents 1 or 2,
$R^7$ represents $(C_1-C_2)$ -alkyl which is up to pentasubstituted by fluorine,
$R^{10}$ represents hydrogen, fluorine or trifluoromethyl,
$L^1$ represents a bond or a group of the formula $-CR^{8A}R^{8B}-$,
in which

$R^{8A}$ represents hydrogen,
$R^{8B}$ represents methyl, trifluoromethyl, pentafluoroethyl or trifluoromethoxymethyl,

$Ar^2$ represents phenyl,
where phenyl may be mono- to trisubstituted by identical or different substituents from the group consisting of fluorine, chlorine, $(C_1-C_3)$-alkyl and/or trifluoromethyl,

$Ar^1$ represents a pyridine ring which is attached via a ring carbon atom,
where the pyridine ring may be mono- or disubstituted by fluorine, chlorine, cyano, methyl or trifluoromethyl,

and the *N*-oxides, salts, solvates, salts of the *N*-oxides and solvates of the *N*-oxides and salts thereof.

**3.** Compound of the formula (I) according to Claim 1 or 2,
in which

$R^1$ represents $NR^3R^4$,
in which

$R^3$ represents hydrogen or methyl, and
$R^4$ represents methyl or 2-fluoroethyl,

or
represents a heterocycle, attached via a nitrogen atom, of the formula

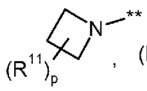 , 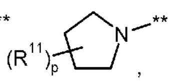 , 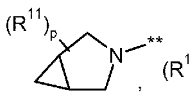 , 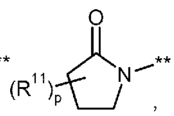 ,

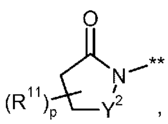 , 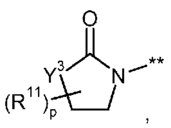 , 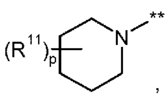 ,

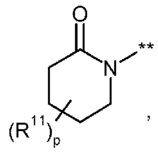 , 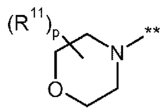 or 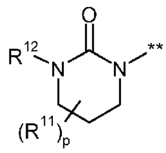

in which

** marks the point of attachment to the remainder of the molecule,
$Y^2$ represents a group of the formula

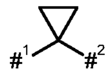

in which

#[1] marks the point of attachment to the nitrogen atom of the pyrrolidinone ring, and
#[2] marks the point of attachment to the carbon atom of the pyrrolidinone ring, and
$Y^3$ represents $-N(R^{12})-$ or a group of the formula

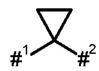

in which

#[1] and #[2] each mark the point of attachment to the carbon atom of the pyrrolidinone ring,

$R^{11}$ represents fluorine, $(C_1-C_4)$-alkyl, difluoromethyl, trifluoromethyl, hydroxy, hydroxymethyl, methoxy, difluoromethoxy or trifluoromethoxy,
p represents the number 0, 1, 2, 3 or 4, where, in the case that the substituents $R^{11}$ occur more than once, their meanings may in each case be identical or different,
$R^{12}$ represents hydrogen or 2-hydroxyethyl,

$R^2$ represents a group of the formula

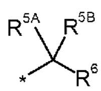 , 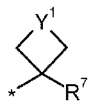 , 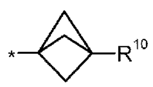 or $*-L^1-Ar^2$

in which

* marks the point of attachment to the nitrogen atom of the amide moiety,
$R^{5A}$ is hydrogen or methyl,
$R^{5B}$ represents methyl, monofluoromethyl, difluoromethyl, trifluoromethyl, pentafluoroethyl or trifluoromethoxymethyl, and
$R^6$ represents methyl, ethyl, trifluoromethyl, 2,2,2-trifluoroethyl, tert-butyl, isobutyl or cyclopropyl,
$Y^1$ represents $-(CH_2)_k-$,
in which
k represents 1 or 2,
$R^7$ represents trifluoromethyl,
$R^{10}$ represents hydrogen, fluorine or trifluoromethyl,
$L^1$ represents a bond or a group of the formula $-CR^{8A}R^{8B}-$, in which

$R^{8A}$ represents hydrogen,

$R^{8B}$ represents trifluoromethyl,

$Ar^2$ represents phenyl,
where phenyl may be mono- or disubstituted by identical or different substituents from the group consisting of fluorine and/or chlorine,

$Ar^1$ represents a group of the formula

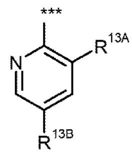 or 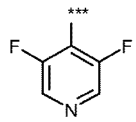

in which

*** marks the point of attachment to the nitrogen atom,
$R^{13A}$ represents fluorine or chlorine,
$R^{13B}$ represents fluorine or hydrogen,

and the salts, solvates and solvates of the salts thereof.

**4.** Compound of the formula (I) according to any of Claims 1 to 3,
in which

$R^1$ represents a heterocycle, attached via a nitrogen atom, of the formula

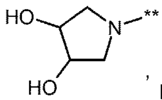, 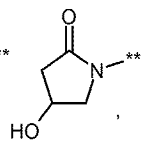, 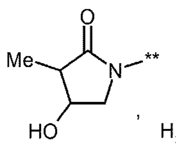, 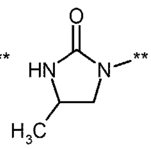 or 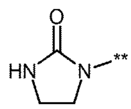

in which

** marks the point of attachment to the remainder of the molecule,

$R^2$ represents a group of the formula

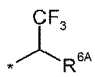, 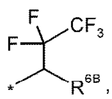, 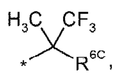, 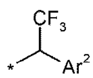 or 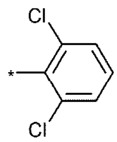

in which

* marks the point of attachment to the nitrogen atom of the amide moiety,
$R^{6A}$ represents trifluoromethyl, ethyl, tert-butyl, isobutyl or cyclopropyl,
$R^{6B}$ represents methyl or ethyl,
$R^{6C}$ represents trifluoromethyl or cyclopropyl,
$Ar^2$ represents a group of the formula

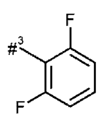 or 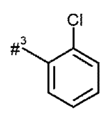

in which

#[3] in each case marks the bonding site

$Ar^1$ represents a group of the formula

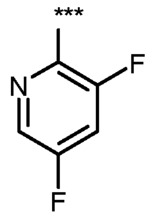

in which

*** marks the point of attachment to the nitrogen atom,

and the salts, solvates and solvates of the salts thereof.

**5.** Compound of the formula (I) according to Claim 1 or 2,
$R^1$ represents a heterocycle, attached via a nitrogen atom, of the formula

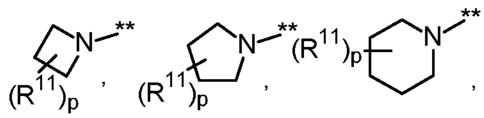

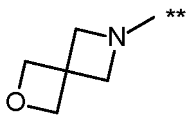

in which

** marks the point of attachment to the remainder of the molecule,
$R^{11}$ represents fluorine, ($C_1$-$C_4$)-alkyl, difluoromethyl, trifluoromethyl, hydroxy, hydroxymethyl, methoxy, difluoromethoxy or trifluoromethoxy,
p represents the number 0, 1, 2, 3 or 4,
where, in the case that the substituents $R^{11}$ occur more than once, their meanings may in each case be identical or different,

$R^2$ represents a group of the formula

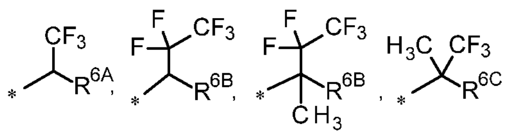

in which

* marks the point of attachment to the nitrogen atom of the amide moiety,
$R^{6A}$ represents trifluoromethyl, ethyl, tert-butyl, isobutyl or cyclopropyl,
$R^{6B}$ represents methyl, ethyl, tert-butyl or cyclopropyl,
$R^{6C}$ represents trifluoromethyl or cyclopropyl,

$Ar^1$ represents a group of the formula

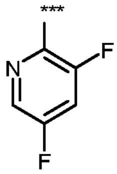

in which

*** marks the point of attachment to the nitrogen atom,

and the salts, solvates and solvates of the salts thereof.

**6.** Compound of the formula (I) according to Claim 5, $R^1$ represents a heterocycle, attached via a nitrogen atom, of the formula

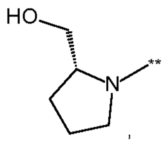 , 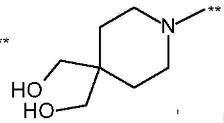 , 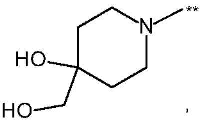 , 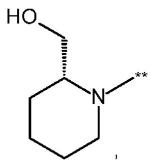 ,

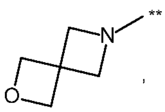 , 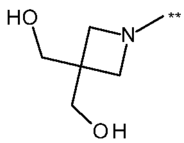

in which

** marks the point of attachment to the remainder of the molecule,
$R^2$ represents a group of the formula

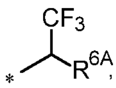 , 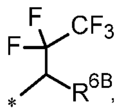 , 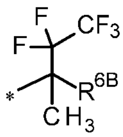

in which

* marks the point of attachment to the nitrogen atom of the amide moiety,
$R^{6A}$ represents trifluoromethyl,
$R^{6B}$ represents methyl,

$Ar^1$ represents a group of the formula

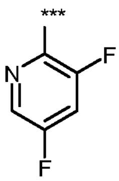

in which

*** marks the point of attachment to the nitrogen atom,

and the salts, solvates and solvates of the salts thereof.

7. Compound according to Claim 1, selected from N-[(1R)-1-cylopropyl-2,2,2-trifluoroethyl]-1-(3,5-difluoropyridin-2-yl)-7-[1-hydroxy-3-azabicyclo[3.1.0]hex-3-yl]-4-oxo-1,4-dihydro-1,8-naphthyridine-3-carboxamide (diastereomer mixture) of the formula

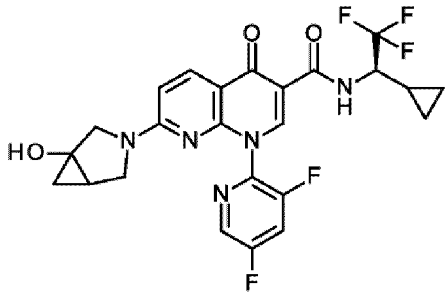

N-[(1R)-1-cylopropyl-2,2,2-trifluoroethyl]-1-(3,5-difluoropyridin-2-yl)-7-[1-hydroxy-3-azabicyclo[3.1.0]hex-3-yl]-4-oxo-1,4-dihydro-1,8-naphthyridine-3-carboxamide (diastereomer 1) and N-[(1R)-1-cylopropyl-2,2,2-trifluoroethyl]-1-(3,5-difluoropyridin-2-yl)-7-[1-hydroxy-3-azabicyclo[3.1.0]hex-3-yl]-4-oxo-1,4-dihydro-1,8-naphthyridine-3-carboxamide (diastereomer 2).

8. Compound according to Claim 1, selected from 1-(3,5-difluoropyridin-2-yl)-N-(1,1,1,3,3,3-hexafluoropropan-2-yl)-7-[1-hydroxy-3-azabicyclo[3.1.0]hex-3-yl]-4-oxo-1,4-dihydro-1,8-naphthyridine-3-carboxamide (racemate) of the formula

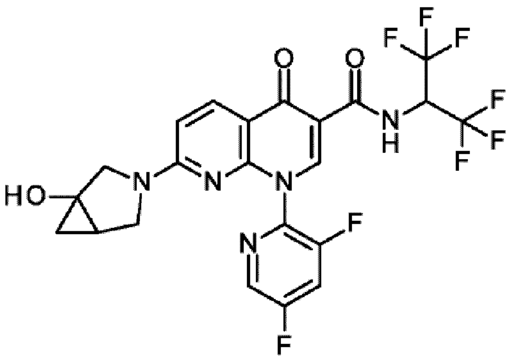

1-(3,5-difluoropyridin-2-yl)-N-(1,1,1,3,3,3-hexafluoropropan-2-yl)-7-[1-hydroxy-3-azabicyclo[3.1.0]hex-3-yl]-4-oxo-1,4-dihydro-1,8-naphthyridine-3-carboxamide (diastereomer 1) and 1-(3,5-difluoropyridin-2-yl)-N-(1,1,1,3,3,3-hexafluoropropan-2-yl)-7-[1-hydroxy-3-azabicyclo[3.1.0]hex-3-yl]-4-oxo-1,4-dihydro-1,8-naphthyridine-3-carboxamide (diastereomer 2).

9. Compound according to Claim 1, 1-(3,5-difluoropyridin-2-yl)-7-[(4S)-4-hydroxy-2-oxopyrrolidin-1-yl]-4-oxo-N-[(2R)-1,1,1-trifluoro-4-methylpentan-2-yl]-1,4-dihydro-1,8-naphthyridine-3-carboxamide of the formula

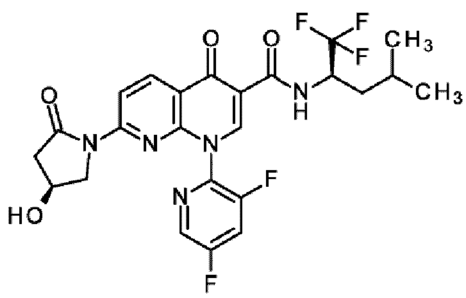

**10.** Compound according to Claim 1, 1-(3,5-difluoropyridin-2-yl)-7-[(4S)-4-hydroxy-2-oxopyrrolidin-1-yl]-4-oxo-N-[(2R)-1,1,1-trifluoro-3,3-dimethylbutan-2-yl]-1,4-dihydro-1,8-naphthyridine-3-carboxamide of the formula

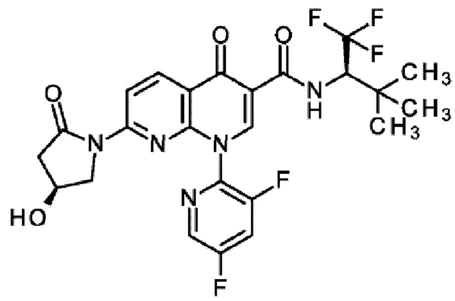

**11.** Compound according to Claim 1, 1-(3,5-difluoropyridin-2-yl)-7-[(3R,4R)-3,4-dihydroxypyrrolidin-1-yl]-4-oxo-N-[(2R)-1,1,1-trifluoro-4-methylpentan-2-yl]-1,4-dihydro-1,8-naphthyridine-3-carboxamide of the formula

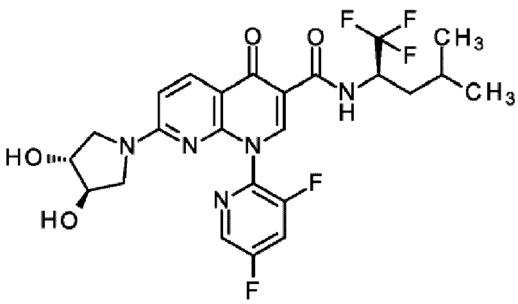

**12.** Compound according to Claim 1, 1-(3,5-difluoropyridin-2-yl)-7-[(3R,4R)-3,4-dihydroxypyrrolidin-1-yl]-4-oxo-N-[(2R)-1,1,1-trifluoro-3,3-dimethylbutan-2-yl]-1,4-dihydro-1,8-naphthyridine-3-carboxamide of the formula

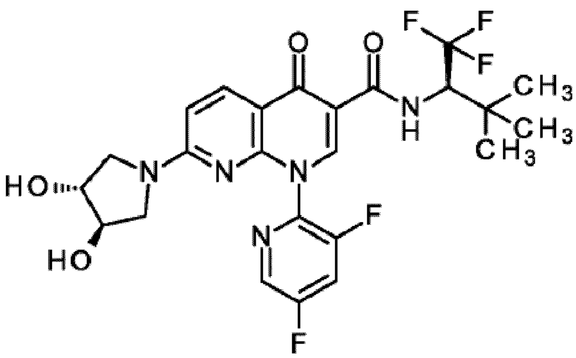

**13.** Compound according to Claim 1, selected from 1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphthyridine-3-carboxamide (diastereomer mixture) of the formula

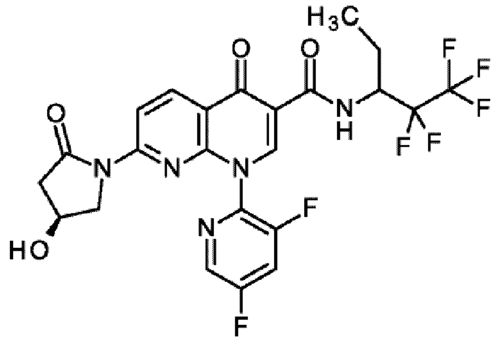

1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphthyridine-3-carboxamide (diastereomer 1) and 1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphthyridine-3-carboxamide (diastereomer 2).

**14.** Process for preparing compounds of the formula (I) as defined in any of Claims 1 to 13, **characterized in that**

[A] a compound of the formula (II)

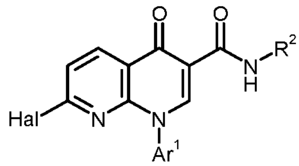

(II),

in which $R^2$ and $Ar^1$ have the meanings given in Claims 1 to 6
and
Hal represents fluorine, chlorine, bromine or iodine, preferably chlorine,

is reacted with a compound of the formula (III)

$R^1$-H (III),

in which $R^1$ has the meaning given above,
to give the carboxamide of the formula (I)

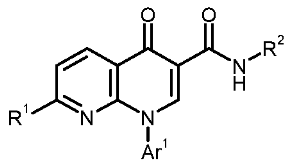

(I),

in which $R^1$, $R^2$ and $Ar^1$ have the meanings given in Claims 1 to 6,
or
[B] a compound of the formula (IV)

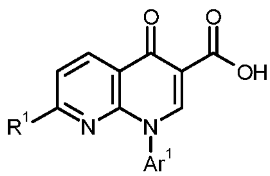

(IV),

in which $R^1$ and $Ar^1$ have the meanings given in Claims 1 to 6

is reacted with a compound of the formula (V)

$R^2$-$NH_2$ (V),

in which $R^2$ has the meaning given above,
to give the carboxamide of the formula (I)

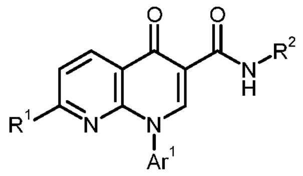

(I),

in which $R^1$, $R^2$ and $Ar^1$ have the meanings given in Claims 1 to 6,
and, if appropriate, the compounds of the formula (I) thus obtained are separated into their enantiomers and/or diastereomers and/or converted with the appropriate (i) solvents and/or (ii) bases or acids to their solvates, salts and/or solvates of the salts.

**15.** Compound of the formula (II)

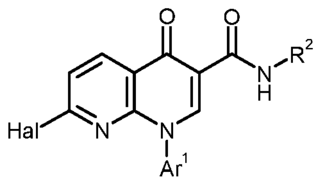

(II),

in which $R^2$ and $Ar^1$ have the meanings given in Claims 1 to 6 for compounds of the formula (I)
and
Hal represents fluorine, chlorine, bromine or iodine, preferably chlorine.

**16.** Compound of the formula (IV)

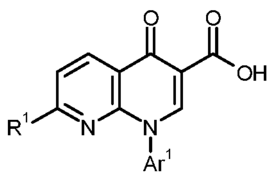

(IV),

in which $R^1$ and $Ar^1$ have the meanings given in Claims 1 to 6 for compounds of the formula (I).

**17.** Use of a compound of the formula (II)

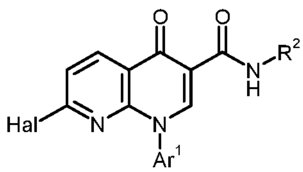

(II),

in which $R^2$ and $Ar^1$ have the meanings given in Claims 1 to 6 for compounds of the formula (I) and
Hal represents fluorine, chlorine, bromine or iodine, preferably chlorine.

or
of a compound of the formula (IV)

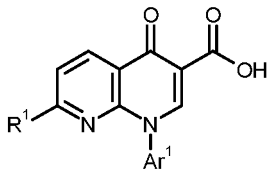

(IV),

in which $R^1$ and $Ar^1$ have the meanings given in Claims 1 to 6 for compounds of the formula (I), for preparation of a compound of the formula (I) according to any of Claims 1 to 13.

**18.** Compound as defined in any of Claims 1 to 13 for use in the treatment and/or prophylaxis of diseases.

**19.** Compound of the formula (I) as defined in any of Claims 1 to 13 for use in a method of treatment and/or prophylaxis of heart failure, coronary heart disease, atrial and ventricular arrhythmia, renal failure and nephropathy.

**20.** Medicament comprising a compound as defined in any of Claims 1 to 13 in combination with one or more further active ingredients selected from the group consisting of active hypotensive ingredients, active antiarrhythmic ingredients, vasopressin receptor antagonists, PDE 5 inhibitors, platelet aggregation inhibitors, sGC activators and sGC stimulators.

**21.** Medicament comprising a compound as defined in any of Claims 1 to 13 in combination with an inert, nontoxic, pharmaceutically suitable excipient.

**22.** Medicament according to Claim 20 or 21 for use in the treatment and/or prophylaxis of heart failure, coronary heart disease, atrial and ventricular arrhythmia, renal failure and nephropathy.

**Revendications**

**1.** Composé de la formule (I) :

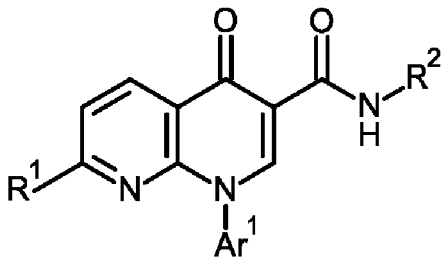

(I),

dans lequel

$R^1$ représente $NR^3R^4$,
où

$R^3$ signifie hydrogène, méthyle, alkyle en ($C_2$-$C_4$) ou cycloalkyle en ($C_3$-$C_6$),
l'alkyle en ($C_2$-$C_4$) pouvant être substitué par hydroxy ou jusqu'à trois fois par fluor,
et
$R^4$ signifie alkyle en ($C_1$-$C_6$), cycloalkyle en ($C_3$-$C_6$), hétérocyclyle saturé de 3 à 6 chaînons ou alkylsulfonyle en ($C_1$-$C_4$),
l'alkyle en ($C_1$-$C_6$), le cycloalkyle en ($C_3$-$C_6$) et l'hétérocyclyle saturé de 3 à 6 chaînons pouvant être substitués jusqu'à trois fois, de manière identique ou différente, par méthyle, difluorométhyle, trifluorométhyle, hydroxy, hydroxycarbonyle, oxo, méthoxy, difluorométhoxy, trifluorométhoxy, cyano et en outre jusqu'à quatre fois par fluor,
ou
$R^3$ et $R^4$ forment ensemble avec l'atome d'azote, auquel ils sont reliés, un hétérocycle saturé ou partiellement insaturé, monocyclique de 3 à 6 chaînons, ou bicyclique de 6 à 10 chaînons, qui peut contenir un ou deux

hétéroatomes supplémentaires, identiques ou différents, de la série constituée par N, O, S, SO et/ou $SO_2$ en tant qu'éléments de cycle,

l'hétérocycle monocyclique de 3 à 6 chaînons et l'hétérocycle bicyclique de 6 à 10 chaînons pouvant à chaque fois être substitués par 1 à 5 substituants choisis indépendamment les uns des autres dans le groupe constitué par alkyle en ($C_1$-$C_4$), difluorométhyle, trifluorométhyle, hydroxy, hydroxycarbonyle, oxo, alcoxy en ($C_1$-$C_3$), difluorométhoxy, trifluorométhoxy, cyano, alcoxycarbonyle en ($C_1$-$C_3$), aminocarbonyle, mono-alkylaminocarbonyloxy en ($C_1$-$C_3$), -NHC (=O)$R^{14A}$, - $CH_2$NHC (=O)$R^{14B}$, et en outre jusqu'à quatre fois par fluor, où
$R^{14A}$ et $R^{14B}$ représentent indépendamment l'un de l'autre alkyle en ($C_1$-$C_3$)ou cyclopropyle, et où l'alkyle en ($C_1$-$C_4$)peut être substitué une ou deux fois, de manière identique ou différente, avec hydroxy, alcoxy en ($C_1$-$C_3$), et jusqu'à quatre fois par fluor,

$R^2$ représente un groupe de la formule :

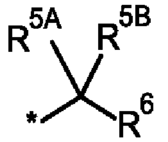
,
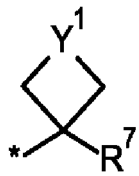

ou

*-$L^1$-$Ar^2$

où

* marque l'emplacement de liaison avec l'atome N du groupement amide,
$R^{5A}$ signifie hydrogène ou alkyle en ($C_1$-$C_4$),
$R^{5B}$ signifie hydrogène, alkyle en ($C_1$-$C_4$), cyclopropyle, monofluorométhyle, difluorométhyle, trifluorométhyle, pentafluoroéthyle, méthoxyméthyle ou trifluorométhoxyméthyle,
$R^6$ signifie alkyle en ($C_1$-$C_4$)substitué jusqu'à cinq fois par fluor ou cycloalkyle en ($C_3$-$C_5$) substitué jusqu'à quatre fois par fluor,
$Y^1$ signifie - $(CH_2)_k$-, -$CF_2$-, -O-$CH_2$-, -$CH_2$-O- ou - $CH_2$-O-$CH_2$-,

où
k représente 0, 1, 2 ou 3,

$R^7$ signifie hydrogène, alkyle en ($C_1$-$C_2$) substitué jusqu'à cinq fois par fluor ou trifluorométhoxyméthyle,
$L^1$ signifie une liaison ou un groupe de la formule -C($R^{8A}R^{8B}$)-$(C(R^{9A}R^{9B}))_m$-,

où
m représente 0 ou 1,
$R^{8A}$ représente hydrogène ou méthyle,
$R^{8B}$ représente hydrogène, méthyle, trifluorométhyle, pentafluoroéthyle ou trifluorométhoxyméthyle,
$R^{9A}$ et $R^{9B}$ représentent indépendamment l'un de l'autre hydrogène ou méthyle,

$Ar^2$ signifie phényle,
le phényle pouvant être substitué une à trois fois, de manière identique ou différente, par fluor, chlore, alkyle en ($C_1$-$C_3$), difluorométhoxyméthyle, trifluorométhoxyméthyle et/ou trifluorométhyle,
ou
représente un carbocycle bicyclique ou tricyclique de 5 à 10 chaînons,
le carbocycle bicyclique ou tricyclique de 5 à 10 chaînons pouvant être substitué jusqu'à trois fois, de manière identique ou différente, par alkyle en ($C_1$-$C_3$), trifluorométhyle, et en outre jusqu'à quatre fois par fluor,

$Ar^1$ représente un cycle pyridine relié par l'intermédiaire d'un atome de carbone de cycle,

le cycle pyridine pouvant être substitué une ou deux fois par fluor, chlore, cyano, méthyle ou trifluorométhyle, ainsi que ses *N*-oxydes, sels, solvates, sels des N-oxydes et solvates des *N*-oxydes et sels.

**2.** Composé de la formule (I) selon la revendication 1, dans lequel

$R^1$ représente $NR^3R^4$,
où

$R^3$ représente hydrogène, méthyle ou alkyle en ($C_2$-$C_4$), et
$R^4$ signifie alkyle en ($C_1$-$C_6$) substitué jusqu'à quatre fois par fluor,
l'alkyle en ($C_1$-$C_6$) pouvant être substitué par oxo,
ou
$R^3$ et $R^4$ forment ensemble avec l'atome d'azote, auquel ils sont reliés, un hétérocycle saturé, monocyclique de 4 à 6 chaînons, ou bicyclique de 6 à 9 chaînons, qui peut contenir un ou deux hétéroatomes supplémentaires, identiques ou différents, de la série constituée par N et/ou O en tant qu'éléments de cycle,

l'hétérocycle monocyclique de 4 à 6 chaînons et l'hétérocycle bicyclique de 6 à 9 chaînons pouvant à chaque fois être substitués par 1 à 5 substituants choisis indépendamment les uns des autres dans le groupe constitué par alkyle en ($C_1$-$C_4$), difluorométhyle, trifluorométhyle, hydroxy, oxo, alcoxy en ($C_1$-$C_3$), difluorométhoxy, trifluorométhoxy, et en outre jusqu'à quatre fois par fluor,
où l'alkyle en ($C_1$-$C_4$)peut être substitué une ou deux fois, de manière identique ou différente, par hydroxy, alcoxy en ($C_1$-$C_3$), et jusqu'à quatre fois par fluor,

$R^2$ représente un groupe de la formule :

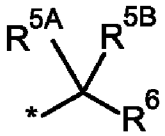 , 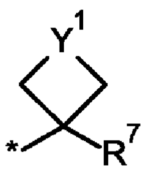 , 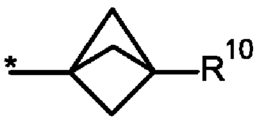 ou 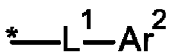

où

* marque l'emplacement de liaison avec l'atome N du groupement amide,
$R^{5A}$ signifie hydrogène ou alkyle en ($C_1$-$C_4$),
$R^{5B}$ signifie méthyle, monofluorométhyle, difluorométhyle, trifluorométhyle, pentafluoroéthyle ou trifluorométhoxyméthyle, et
$R^6$ signifie alkyle en ($C_1$-$C_4$)substitué jusqu'à cinq fois par fluor ou cycloalkyle en ($C_3$-$C_5$) substitué jusqu'à quatre fois par fluor,
$Y^1$ signifie $-(CH_2)_k-$,

où
k représente 1 ou 2,

$R^7$ signifie alkyle en ($C_1$-$C_2$) substitué jusqu'à cinq fois par fluor,
$R^{10}$ signifie hydrogène, fluor ou trifluorométhyle,
$L^1$ signifie une liaison ou un groupe de la formule $-CR^{8A}R^{8B}-$,

où
$R^{8A}$ représente hydrogène,
$R^{8B}$ représente méthyle, trifluorométhyle, pentafluoroéthyle ou trifluorométhoxyméthyle,

$Ar^2$ signifie phényle,
le phényle pouvant être substitué une à trois fois, de manière identique ou différente, par fluor, chlore, alkyle en ($C_1$-$C_3$)et/ou trifluorométhyle,

Ar[1] représente un cycle pyridine relié par l'intermédiaire d'un atome de carbone de cycle, le cycle pyridine pouvant être substitué une ou deux fois par fluor, chlore, cyano, méthyle ou trifluorométhyle, ainsi que ses *N*-oxydes, sels, solvates, sels des N-oxydes et solvates des *N*-oxydes et sels.

**3.** Composé de la formule (I) selon la revendication 1 ou 2, dans lequel

$R^1$ représente $NR^3R^4$, où

$R^3$ signifie hydrogène ou méthyle, et
$R^4$ signifie méthyle ou 2-fluoroéthyle,

ou
un hétérocycle relié par l'intermédiaire d'un atome d'azote de la formule :

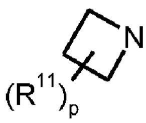 , 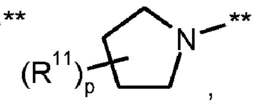 , 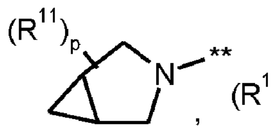 , 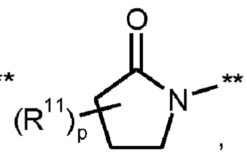 ,

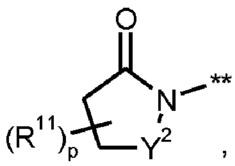 , 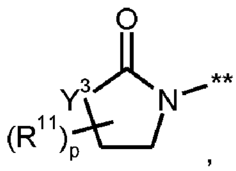 , 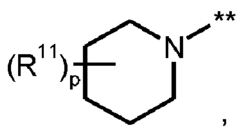 ,

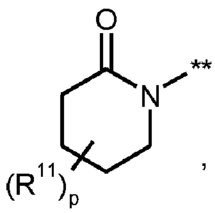 , 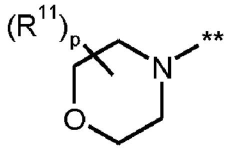 ou 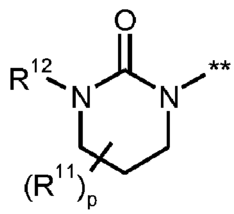

où

** marque l'emplacement de liaison avec le reste de la molécule,
$Y^2$ représente un groupe de la formule :

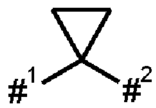

où

$\#^1$ marque l'emplacement de liaison avec l'atome N du cycle pyrrolidinone, et
$\#^2$ marque l'emplacement de liaison avec l'atome C du cycle pyrrolidinone,

$Y^3$ représente $-N(R^{12})-$ ou un groupe de la formule :

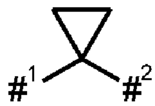

où

$\#^1$ et $\#^2$ marquent chacun l'emplacement de liaison avec l'atome C du cycle pyrrolidinone,
$R^{11}$ représente fluor, alkyle en ($C_1$-$C_4$), difluorométhyle, trifluorométhyle, hydroxy, hydroxyméthyle, méthoxy, difluorométhoxy ou trifluorométhoxy,
p représente le nombre 0, 1, 2, 3 ou 4,
dans le cas où les substituants $R^{11}$ occurrent à plusieurs reprises, leurs significations pouvant à chaque fois être identiques ou différentes,
$R^{12}$ représente hydrogène ou 2-hydroxyéthyle,

$R^2$ représente un groupe de la formule :

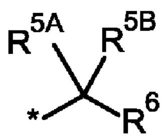 , 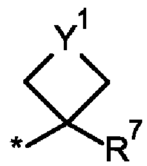 , 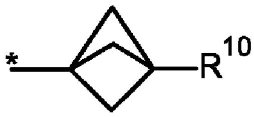 ou *—$L^1$—$Ar^2$

où

* marque l'emplacement de liaison avec l'atome N du groupement amide,
$R^{5A}$ signifie hydrogène ou méthyle,
$R^{5B}$ signifie méthyle, monofluorométhyle, difluorométhyle, trifluorométhyle, pentafluoroéthyle ou trifluorométhoxyméthyle, et
$R^6$ signifie méthyle, éthyle, trifluorométhyle, 2,2,2-trifluoroéthyle, tert-butyle, iso-butyle ou cyclopropyle,
$Y^1$ signifie $-(CH_2)_k-$,

où
k représente 1 ou 2,

$R^7$ signifie trifluorométhyle,
$R^{10}$ signifie hydrogène, fluor ou trifluorométhyle,
$L^1$ signifie une liaison ou un groupe de la formule $-CR^{8A}R^{8B}-$,

où
$R^{8A}$ représente hydrogène,
$R^{8B}$ représente trifluorométhyle,

$Ar^2$ signifie phényle,
le phényle pouvant être substitué une ou deux fois, de manière identique ou différente, par fluor et/ou chlore,

$Ar^1$ représente un groupe de la formule :

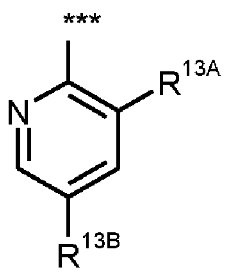 ou 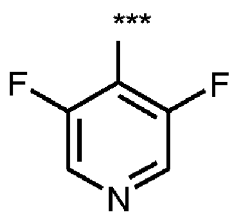

où

*** marque l'emplacement de liaison avec l'atome N,
$R^{13A}$ représente fluor ou chlore,
$R^{13B}$ représente fluor ou hydrogène,

ainsi que ses sels, solvates et solvates des sels.

**4.** Composé de la formule (I) selon l'une quelconque des revendications 1 à 3, dans lequel

$R^1$ représente un hétérocycle relié par l'intermédiaire d'un atome d'azote de la formule :

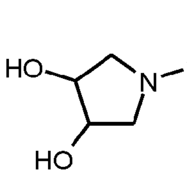 , 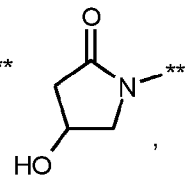 , 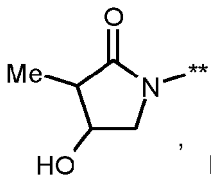 , 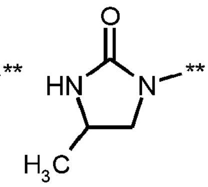 ou 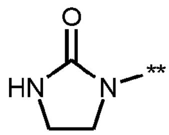

où
** marque l'emplacement de liaison avec le reste de la molécule,
$R^2$ représente un groupe de la formule :

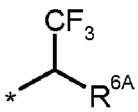 , 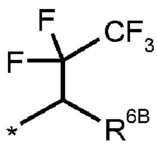 , 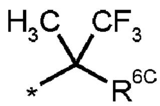 , 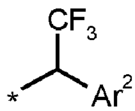 ou 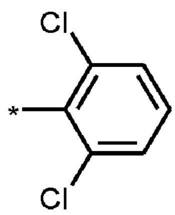

où

* marque l'emplacement de liaison avec l'atome N du groupement amide,
$R^{6A}$ signifie trifluorométhyle, éthyle, tert-butyle, isobutyle ou cyclopropyle,
$R^{6B}$ signifie méthyle ou éthyle,
$R^{6C}$ signifie trifluorométhyle ou cyclopropyle,
$Ar^2$ signifie un groupe de la formule :

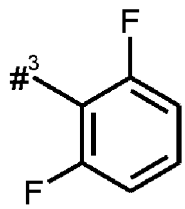 ou 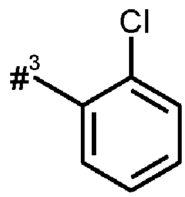

où
$\#^3$ marque à chaque fois l'emplacement de liaison,

$Ar^1$ représente un groupe de la formule :

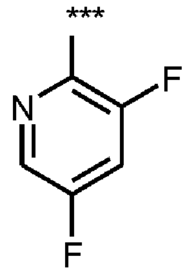

où
*** marque l'emplacement de liaison avec l'atome N,
ainsi que ses sels, solvates et solvates des sels.

**5.** Composé de la formule (I) selon la revendication 1 ou 2,

$R^1$ représente un hétérocycle relié par l'intermédiaire d'un atome d'azote de la formule :

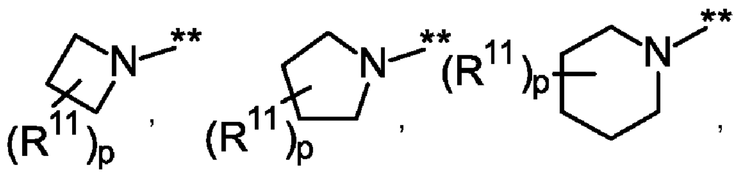

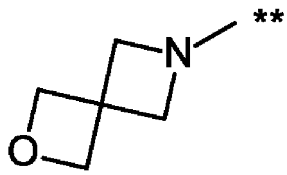

où

** marque l'emplacement de liaison avec le reste de la molécule,
$R^{11}$ représente fluor, alkyle en ($C_1$-$C_4$), difluorométhyle, trifluorométhyle, hydroxy, hydroxyméthyle, méthoxy, difluorométhoxy ou trifluorométhoxy,
p représente le nombre 0, 1, 2, 3 ou 4,
dans le cas où les substituants $R^{11}$ occurrent à plusieurs reprises, leurs significations pouvant à chaque fois être identiques ou différentes,

$R^2$ représente un groupe de la formule :

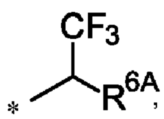 , 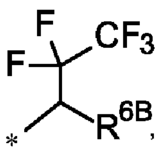 , 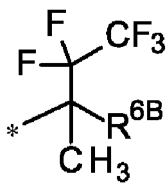 , 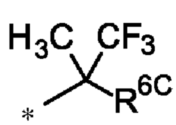

où

* marque l'emplacement de liaison avec l'atome N du groupement amide,
$R^{6A}$ signifie trifluorométhyle, éthyle, tert-butyle, isobutyle ou cyclopropyle,
$R^{6B}$ signifie méthyle, éthyle, tert-butyle ou cyclopropyle,
$R^{6C}$ signifie trifluorométhyle ou cyclopropyle,

$Ar^1$ représente un groupe de la formule :

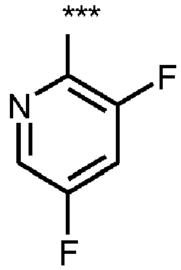

où
*** marque l'emplacement de liaison avec l'atome N,
ainsi que ses sels, solvates et solvates des sels.

**6.** Composé de la formule (I) selon la revendication 5,

$R^1$ représente un hétérocycle relié par l'intermédiaire d'un atome d'azote de la formule :

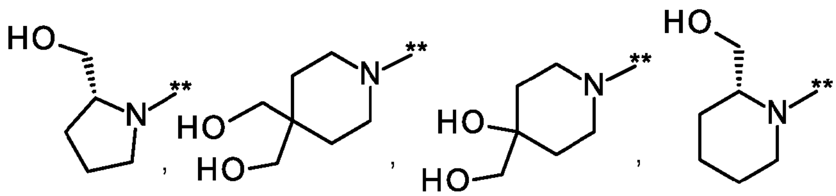

, , , ,

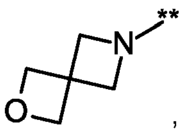

,

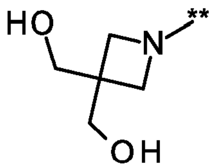

où
** marque l'emplacement de liaison avec le reste de la molécule,
$R^2$ représente un groupe de la formule :

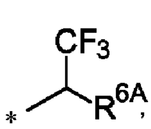

,

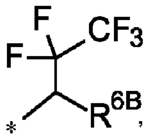

,

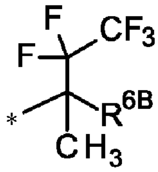

où

* marque l'emplacement de liaison avec l'atome N du groupement amide,
$R^{6A}$ signifie trifluorométhyle,
$R^{6B}$ signifie méthyle,

$Ar^1$ signifie un groupe de la formule :

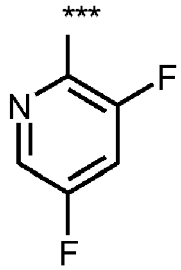

où
*** marque l'emplacement de liaison avec l'atome N,
ainsi que ses sels, solvates et solvates des sels.

**7.** Composé selon la revendication 1, choisi parmi l'amide de l'acide N-[(1R)-1-cyclopropyl-2,2,2-trifluoréthyl]-1-(3,5-difluoropyridin-2-yl)-7-[1-hydroxy-3-azabicyclo[3.1.0]hex-3-yl]-4-oxo-1,4-dihydro-1,8-naphtyridine-3-carboxylique (mélange de diastéréomères) de la formule :

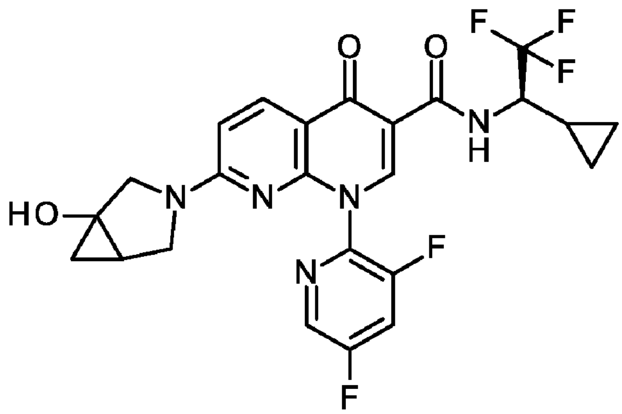

l'amide de l'acide N-([1R)-1-cyclopropyl-2,2,2-trifluoroéthyl]-1-(3,5-difluoropyridin-2-yl)-7-[1-hydroxy-3-azabicyclo[3.1.0]hex-3-yl]-4-oxo-1,4-dihydro-1,8-naphtyridine-3-carboxylique (diastéréomère 1) et l'amide de l'acide N-[(1R)-1-cyclopropyl-2,2,2-trifluoroéthyl]-1-(3,5-difluoropyridin-2-yl)-7-[1-hydroxy-3-azabicyclo[3.1.0]hex-3-yl]-4-oxo-1,4-dihydro-1,8-naphtyridine-3-carboxylique (diastéréomère 2).

**8.** Composé selon la revendication 1, choisi parmi l'amide de l'acide 1-(3,5-difluoropyridin-2-yl)-N-(1,1,1,3,3,3-hexafluoropropan-2-yl)-7-[1-hydroxy-3-azabicyclo[3.1.0]hex-3-yl]-4-oxo-1,4-dihydro-1,8-naphtyridine-3-carboxylique (racémat) de la formule :

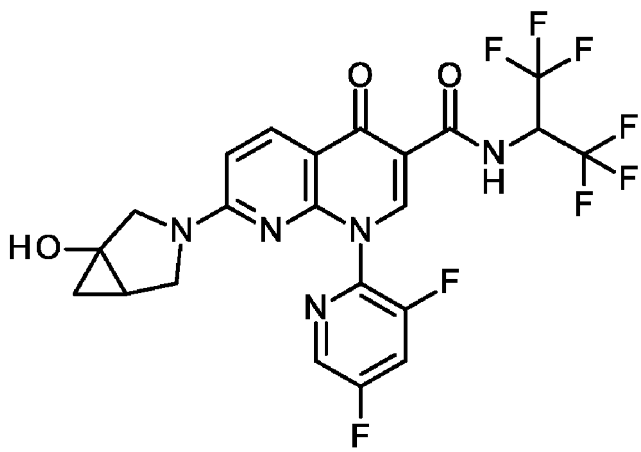

l'amide de l'acide 1-(3,5-difluoropyridin-2-yl)-N-(1,1,1,3,3,3-hexafluoropropan-2-yl)-7-[1-hydroxy-3-azabicyclo-[3.1.0]hex-3-yl]-4-oxo-1,4-dihydro-1,8-naphtyridine-3-carboxylique (diastéréomère 1) et l'amide de l'acide 1-(3,5-difluoropyridin-2-yl)-N-(1,1,1,3,3,3-hexafluoropropan-2-yl)-7-[1-hydroxy-3-azabicyclo-[3.1.0]hex-3-yl]-4-oxo-1,4-dihydro-1,8-naphtyridine-3-carboxylique (diastéréomère 2).

**9.** Composé selon la revendication 1, amide de l'acide 1-(3,5-difluoropyridin-2-yl)-7-[(4S)-4-hydroxy-2-oxopyrrolidin-1-yl]-4-oxo-N-[(2R)-1,1,1-trifluoro-4-méthylpentan-2-yl]-1,4-dihydro-1,8-naphtyridine-3-carboxylique de la formule :

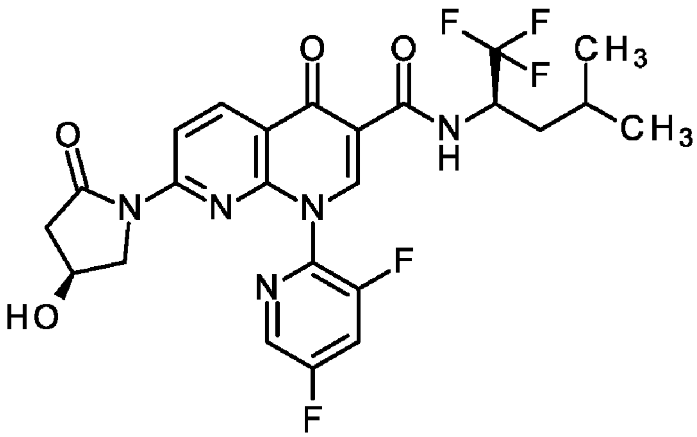

.

**10.** Composé selon la revendication 1, amide de l'acide 1-(3,5-difluoropyridin-2-yl)-7-[(4S)-4-hydroxy-2-oxopyrrolidin-1-yl]-4-oxo-N-[(2R)-1,1,1-trifluoro-3,3-diméthylbutan-2-yl]-1,4-dihydro-1,8-naphtyridine-3-carboxylique de la formule :

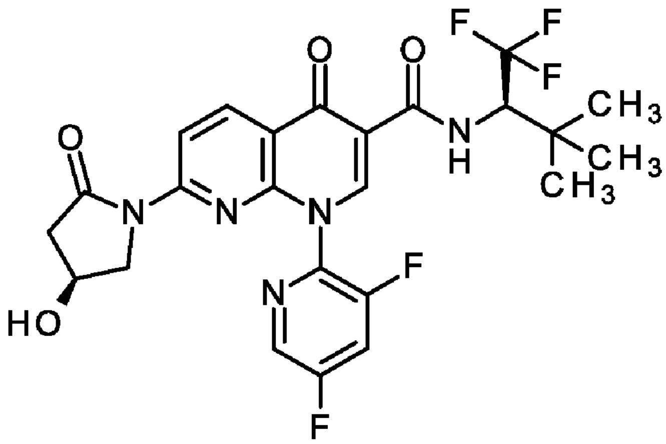

.

**11.** Composé selon la revendication 1, amide de l'acide 1-(3,5-difluoropyridin-2-yl)-7-[(3R,4R)-3,4-dihydroxypyrrolidin-1-yl]-4-oxo-N-[(2R)-1,1,1-trifluoro-4-méthylpentan-2-yl]-1,4-dihydro-1,8-naphtyridine-3-carboxylique de la formule :

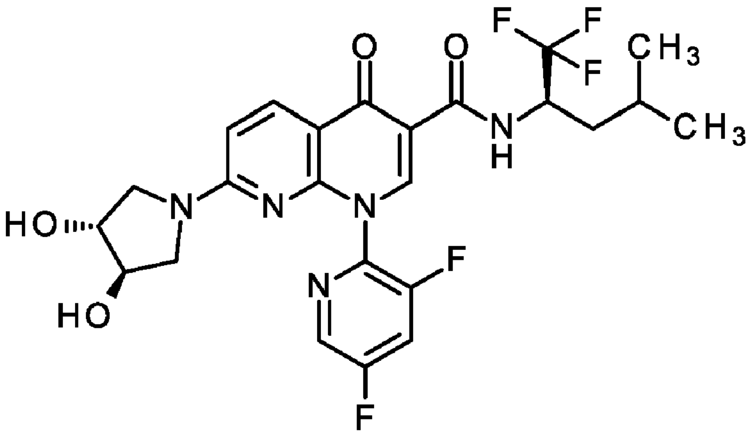

.

**12.** Composé selon la revendication 1, amide de l'acide 1-(3,5-difluoropyridin-2-yl)-7-[(3R,4R)-3,4-dihydroxypyrrolidin-1-yl]-4-oxo-N-[(2R)-1,1,1-trifluoro-3,3-diméthylbutan-2-yl]-1,4-dihydro-1,8-naphtyridine-3-carboxylique de la formule :

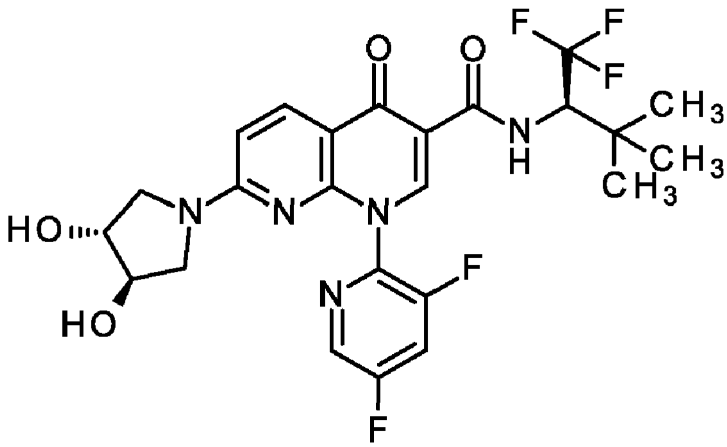

.

**13.** Composé selon la revendication 1, choisi parmi l'amide de l'acide 1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphtyridine-3-carboxylique (mélange de diastéréomères) de la formule :

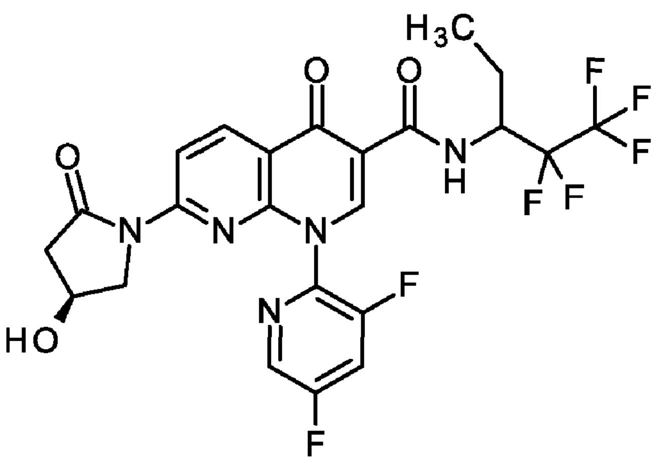

l'amide de l'acide 1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphtyridine-3-carboxylique (diastéréomère 1) et l'amide de l'acide 1-(3,5-difluoropyridin-2-yl)-7-[(4S)-4-hydroxy-2-oxopyrrolidin-1-yl]-4-oxo-N-[1,1,1,2,2-pentafluoropentan-3-yl]-1,4-dihydro-1,8-naphtyridine-3-carboxylique (diastéréomère 2).

**14.** Procédé de fabrication de composés de la formule (I) tels que définis dans l'une quelconque des revendications 1 à 13, **caractérisé en ce que**

[A] un composé de la formule (II) :

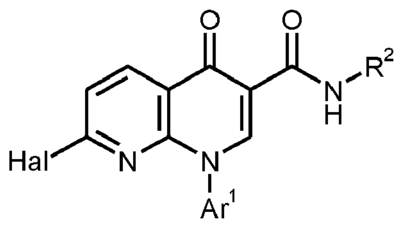

(II),

dans laquelle $R^2$ et $Ar^1$ ont les significations indiquées dans les revendications 1 à 6
et
Hal représente fluor, chlore, brome ou iode, de préférence chlore,

est mis en réaction avec un composé de la formule (III) :

$R^1$-H (III)

dans laquelle $R^1$ a la signification indiquée précédemment,
pour former l'amide d'acide carboxylique de la formule (I) :

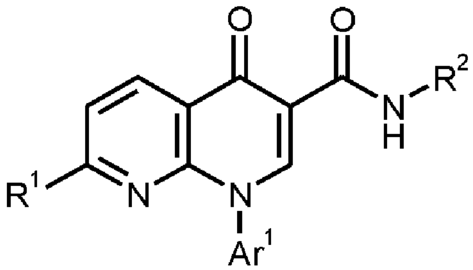

(I),

dans laquelle $R^1$, $R^2$ et $Ar^1$ ont les significations indiquées dans les revendications 1 à 6,
ou
[B] un composé de la formule (IV) :

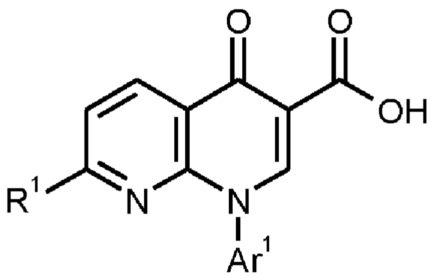

(IV),

dans laquelle $R^1$ et $Ar^1$ ont les significations indiquées dans les revendications 1 à 6,
est mis en réaction avec un composé de la formule (V) :

$R^2$-$NH_2$ (V),

dans laquelle $R^2$ a la signification indiquée précédemment,
pour former l'amide d'acide carboxylique de la formule (I) :

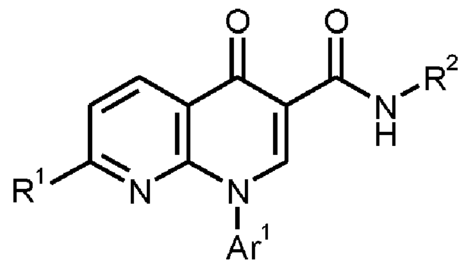

(I),

dans laquelle $R^1$, $R^2$ et $Ar^1$ ont les significations indiquées dans les revendications 1 à 6,
et les composés de la formule (I) ainsi obtenus sont éventuellement séparés en leurs énantiomères et/ou diastéréomères et/ou transformés avec (i) les solvants et/ou (ii) les bases ou acides correspondants en leurs solvates, sels et/ou solvates des sels.

**15.** Composé de la formule (II) :

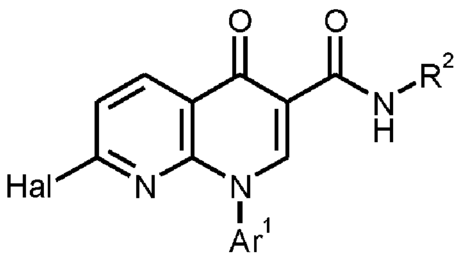

(II),

dans laquelle $R^2$ et $Ar^1$ ont les significations indiquées dans les revendications 1 à 6 pour des composés de la formule (I)
et
Hal représente fluor, chlore, brome ou iode, de préférence chlore.

**16.** Composé de la formule (IV) :

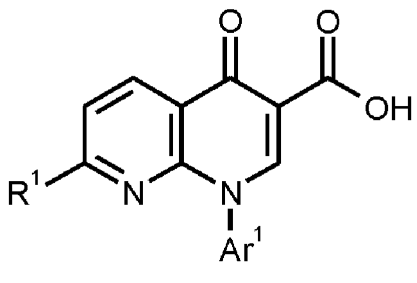

(IV),

dans laquelle $R^1$ et $Ar^1$ ont les significations indiquées dans les revendications 1 à 6 pour des composés de la formule (I).

**17.** Utilisation d'un composé de la formule (II) :

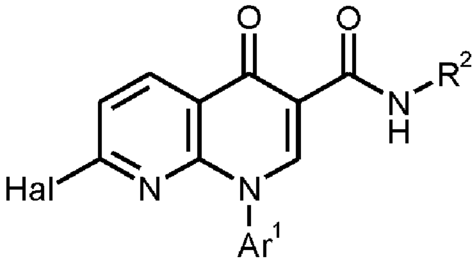

(II),

dans laquelle $R^2$ et $Ar^1$ ont les significations indiquées dans les revendications 1 à 6 pour des composés de la formule (I)
et
Hal représente fluor, chlore, brome ou iode, de préférence chlore,

ou
d'un composé de la formule (IV) :

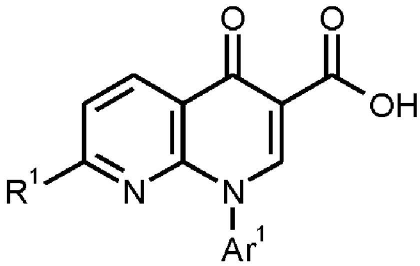

(IV),

dans laquelle $R^1$ et $Ar^1$ ont les significations indiquées dans les revendications 1 à 6 pour des composés de la formule (I),
pour la fabrication d'un composé de la formule (I) selon l'une quelconque des revendications 1 à 13.

**18.** Composé, tel que défini dans l'une quelconque des revendications 1 à 13, destiné à une utilisation dans le traitement et/ou la prophylaxie de maladies.

**19.** Composé de la formule (I), tel que défini dans l'une quelconque des revendications 1 à 13, destiné à une utilisation dans un procédé pour le traitement et/ou la prophylaxie de l'insuffisance cardiaque, de la maladie cardiaque coronarienne, des arythmies atriales et ventriculaires, de l'insuffisance rénale et des néphropathies.

**20.** Médicament contenant un composé, tel que défini dans l'une quelconque des revendications 1 à 13, en combinaison avec un ou plusieurs agents actifs supplémentaires choisis dans le groupe constitué par les agents actifs abaissant la tension artérielle, les agents actifs antiarythmiques, les antagonistes de récepteurs de vasopressine, les inhibiteurs de PDE 5, les inhibiteurs de l'agrégation des thrombocytes, les activateurs de sGC et les stimulateurs de sGC.

**21.** Médicament contenant un composé, tel que défini dans l'une quelconque des revendications 1 à 13, en combinaison avec un adjuvant inerte, non toxique, pharmaceutiquement approprié.

**22.** Médicament selon la revendication 20 ou 21 destiné à une utilisation dans le traitement et/ou la prophylaxie de l'insuffisance cardiaque, de la maladie cardiaque coronarienne, des arythmies atriales et ventriculaires, de l'insuffisance rénale et des néphropathies.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0945435 B1
- WO 2002085886 A2
- WO 2003050107 A1
- WO 2005026145 A2
- WO 2005026165 A1
- WO 2005049602 A1
- EP 1650192 A1
- WO 2005009971 A1
- JP 2005012561 B
- WO 2015189560 A1
- WO 2016081464 A1
- US 3966781 A, J. G. Atkinson
- WO 2012112363 A, K. Kassahun
- EP 0607825 A1
- WO 2010105770 A
- WO 2011104322 A
- WO 2016071212 A
- WO 0006568 A
- WO 0006569 A
- WO 0242301 A
- WO 03095451 A
- WO 2011147809 A
- WO 2012004258 A
- WO 2012028647 A
- WO 2012059549 A
- WO 0119355 A
- WO 0119776 A
- WO 0119778 A
- WO 0119780 A
- WO 02070462 A
- WO 02070510 A
- EP 1721905 A1
- DE 1121617


### In der Beschreibung aufgeführte Nicht-Patentliteratur


- **SCHRÄGE et al.** *Biochem. Pharmacol.,* 2014, vol. 90 (3), 307-319
- **NEUBIG et al.** *Pharmacol Rev,* 2003, vol. 55, 597-606
- **KRUSE et al.** *Mol Pharmacol.,* 2013, vol. 84 (4), 528-540
- **GREGORY et al.** *Current Neuropharmacol,* 2007, vol. 5 (3), 157-167
- **CLARK ; MITCHELSON.** *Br. J. Pharmac,* 1976, vol. 58, 323-331
- **CONN et al.** *Nat. Rev. Drug Disc.,* 2009, vol. 8, 41-54
- **CONN et al.** *Nat. Rev. Drug. Disc.,* 2014, vol. 13, 692-708
- **CHRISTOPOULOS.** *Mol. Pharmacol.,* 2014, vol. 86, 463-478
- **WANG et al.** *J. Pharmacol. Exp. Therap.,* 2009, vol. 331, 340-348
- **KRUSE et al.** *Nature,* 2013, vol. 504, 101-106
- **CROY et al.** *Molecular Pharmacology,* Juli 2014, vol. 86 (1), 106-115
- **SCHOBER et al.** *Molecular Pharmacology,* Juli 2014, vol. 86 (1), 116-123
- **HE et al.** *Br. J. Pharmacol.,* 2014
- **RANPURIA et al.** *Nephrol Dial Transplant,* 2008, vol. 23 (2), 444-4499
- **VINIK et al.** *Diabet Med,* 2011, vol. 28 (6), 643-651
- **SYKORA et al.** *Stroke,* 2009, vol. 40 (12), 678-682
- **HALARIS et al.** *Mod Trends Pharmacopsychiatri,* 2013, vol. 28, 144-161
- **RASH ; AGUIRRE-CAMACHO.** *Atten Defic Hyperact Disord,* 2012, vol. 4 (4), 167-177
- **DE FERRARI.** *J. Cardiovasc. Transl. Res.,* 2014, vol. 7 (3), 310-320
- **DE FERRARI.** *J. Cardiovasc. Transl. Res,* 2014, vol. 7 (3), 310-320
- **DE FERRARI GM et al.** *Eur. Heart J.,* 2011, vol. 32, 847-855
- **PREMCHAND RK et al.** *J. Card. Fail.,* 2014, vol. 20 (11), 808-816
- **ZANNAD et al.** *Eur. Heart J,* 2015, vol. 36 (7), 425-433
- **GOLD et al.** *JAm Coll Cardiol,* 29. Marz 2016, (16), 0735-1097
- **CHEN et al.** *Circ. Res,* 2014, vol. 114 (9), 1500-1515
- **MAISEL ; STEVENSON.** *Am. J. Cardiol.,* 2003, vol. 91, 2D-8D
- **LEVALTER T.** *Fortbildungsprogramm Pharmazie,* 2011, vol. 5, 106-127
- **LEONG-SIT ; TANG.** *Curr. Opin. Cardiol,* 2015

- **SCAMMELLS et al.** *ACS Chem. Neurosci,* 2013, vol. 4 (7), 1026-1048
- **MISTRY et al.** *J. Med. Chem.,* 2013, vol. 56, 5151-5172
- Isotopic Compositions of the Elements 1997. *Pure Appl. Chem,* 1998, vol. 70 (1), 217-235
- **H. J. LEIS et al.** *Curr. Org. Chem,* 1998, vol. 2, 131
- **ESAKI et al.** *Tetrahedron,* 2006, vol. 62, 10954
- **ESAKI et al.** *Chem. Eur. J.,* 2007, vol. 13, 4052
- **J. R. MORANDI et al.** *J. Org. Chem.,* 1969, vol. 34 (6), 1889
- **N. H. KHAN.** *J. Am. Chem. Soc.,* 1952, vol. 74 (12), 3018
- **S. CHANDRASEKHAR et al.** *Tetrahedron,* 2011, vol. 52, 3865
- **HANZLIK et al.** *J. Org. Chem.,* 1990, vol. 55, 3992-3997
- **R. P. HANZLIK et al.** *Biochem. Biophys. Res. Commun.,* 1989, vol. 160, 844
- **P. J. REIDER et al.** *J. Org. Chem.,* 1987, vol. 52, 3326-3334
- **M. JARMAN et al.** *Carcinogenesis,* 1993, vol. 16 (4), 683-688
- **J. ATZRODT et al.** *Angew. Chem., Int. Ed.,* 2007, vol. 46, 7744
- **K. MATOISHI et al.** *J. Chem. Soc, Chem. Commun.,* 2000, 1519-1520
- **A. STREITWIESER et al.** *J. Am. Chem. Soc.,* 1963, vol. 85, 2759
- **C. L. PERRIN et al.** *J. Am. Chem. Soc.,* 2007, vol. 129, 4490
- **C. L. PERRIN et al.** *J. Am. Chem. Soc.,* 2003, vol. 125, 15008
- **C. L. PERRIN.** *Advances in Physical Organic Chemistry,* vol. 44, 144
- **C. L. PERRIN et al.** *J. Am. Chem. Soc.,* 2005, vol. 127, 9641
- **B. TESTA et al.** *Int. J. Pharm.,* 1984, vol. 19 (3), 271
- **D. J. KUSHNER et al.** *Can. J. Physiol. Pharmacol,* 1999, vol. 77, 79
- **A. E. MUTLIB et al.** *Toxicol. Appl. Pharmacol,* 2000, vol. 169, 102
- **A. M. SHARMA et al.** *Chem. Res.Toxicol,* 2013, vol. 26, 410
- **UETRECHT et al.** *Chemical Research in Toxicology,* 2008, vol. 21 (9), 1862
- **A. J. MORALES et al.** *Abstract 285, The 15th North American Meeting of the International Society of Xenobiotics,* 16. Oktober 2008
- **C. J. WENTHUR et al.** *J. Med. Chem,* 2013, vol. 56, 5208
- **F. SCHNEIDER et al.** *Arzneim. Forsch. Drug. Res,* 2006, vol. 56, 295
- **F. MALTAIS et al.** *J. Med. Chem.,* 2009, vol. 52, 7993
- **REUVENY et al.** *Nature,* Juli 1994, vol. 370, 143-146
- *Methods in Molecular Biology,* vol. 259
- Receptor Signal Transduction Protocols
- **KOSTENIS ; MOHR.** *Trends Pharmacol. Sci.,* 1996, vol. 17 (8), 280-283
- **CROY et al.** *Mol. Pharmacol.,* 2014, vol. 86, 106-115
- **HULME ; TREVETHICK.** *Brit. J. Pharmacol.,* 2010, vol. 161, 1219-1237
- **SCHOBER et al.** *Mol. Pharmacol.,* 2014, vol. 86, 116-123
- **CHENG ; PRUSOFF.** *Biochem. Pharmacol.,* 1973, vol. 22 (23), 3099-3108
- **LAZARENO.** Determination of Allosteric Interactions Using Radioligand-Binding Techniques. *Methods in Molecular Biology,* vol. 259
- **SCHRAGE et al.** *Biochem. Pharmacol.,* 2014, vol. 90, 307-319
- **BECKMANN ; RINNE et al.** *Cell. Physiol. Biochem.,* 2008, vol. 21, 259-268